(19) Europäisches Patentamt
European Patent Office
Office européen des brevets

(11) EP 2 442 666 B1

(12) # EUROPEAN PATENT SPECIFICATION

(45) Date of publication and mention of the grant of the patent:
**08.04.2015 Bulletin 2015/15**

(21) Application number: **10730624.3**

(22) Date of filing: **14.06.2010**

(51) Int Cl.:
*A23L 1/30* $^{(2006.01)}$ *A23K 1/00* $^{(2006.01)}$
*A23K 1/16* $^{(2006.01)}$ *A23K 1/18* $^{(2006.01)}$
*A21D 2/16* $^{(2006.01)}$ *A23L 1/29* $^{(2006.01)}$
*C11B 1/10* $^{(2006.01)}$ *C12N 9/00* $^{(2006.01)}$
*C12N 9/10* $^{(2006.01)}$ *C12N 9/02* $^{(2006.01)}$
*C12N 15/81* $^{(2006.01)}$ *C12P 7/64* $^{(2006.01)}$

(86) International application number:
**PCT/US2010/038539**

(87) International publication number:
**WO 2010/147907 (23.12.2010 Gazette 2010/51)**

(54) **HIGH EICOSAPENTAENOIC ACID OILS FROM IMPROVED OPTIMIZED STRAINS OF YARROWIA LIPOLYTICA**

EICOSAPENTAENSÄURE REICHE ÖLE AUS VERBESSERTEN UND OPTIMIERTEN STÄMMEN VON YARROWIA LIPOLYTICA

HUILE EN TAUX ÉLEVÉ D'ACIDE EICOSAPENTAÉNOÏQUE DE SOUCHES AMELIORÉES ET OPTIMISÉES DE YARROWIA LIPOLYTICA

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **16.06.2009 US 187368 P**

(43) Date of publication of application:
**25.04.2012 Bulletin 2012/17**

(73) Proprietor: **E. I. du Pont de Nemours and Company**
**Wilmington, DE 19898 (US)**

(72) Inventors:
- **HONG, Seung-Pyo**
  **Hockessin, Delaware 19707 (US)**
- **SHARPE, Pamela, L.**
  **Wilmington, Delaware 19808 (US)**
- **XUE, Zhixiong**
  **Chadds Ford, Pennsylvania 19317-9728 (US)**
- **YADAV, Narendra, S.**
  **Wilmington, Delaware 19803 (US)**
- **ZHU, Qun**
  **West Chester, Pennsylvania 19382 (US)**

(74) Representative: **Dzieglewska, Hanna Eva**
**Dehns**
**St Bride's House**
**10 Salisbury Square**
**London EC4Y 8JD (GB)**

(56) References cited:
**WO-A1-2010/080388 US-A1- 2006 110 806**
**US-A1- 2008 254 191 US-A1- 2009 093 543**
**US-A1- 2009 325 265**

- **IBANEZ GONZALEZ M J ET AL: "Adsorption equilibria of fatty acids between methanol/water and reversed-phase chromatographic adsorbents", JOURNAL OF THE AMERICAN OIL CHEMISTS' SOCIETY, vol. 78, no. 3, March 2001 (2001-03), pages 277-285, XP002609150, ISSN: 0003-021X**
- **ROBLES MEDINA A ET AL: "Lipase-catalyzed esterification of glycerol and polyunsaturated fatty acids from fish and microalgae oils", JOURNAL OF BIOTECHNOLOGY, ELSEVIER SCIENCE PUBLISHERS, AMSTERDAM, NL LNKD- DOI:10.1016/S0168-1656(99)00091-7, vol. 70, no. 1-3, 30 April 1999 (1999-04-30), pages 379-391, XP004173418, ISSN: 0168-1656**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 442 666 B1

- **MEDINA A R ET AL: "CONCENTRATION AND PURIFICATION OF STEARIDONIC, EICOSAPENTAENOIC, AND DOCOSAHEXAENOIC ACIDS FROM COD LIVER OIL AND THE MARINE MICROALGA ISOCHRYSIS GALBANA", JOURNAL OF THE AMERICAN OIL CHEMISTS' SOCIETY, SPRINGER, BERLIN, DE, vol. 5, no. 72, 1 January 1995 (1995-01-01), pages 575-583, XP001071204, ISSN: 0003-021X, DOI: DOI:10.1007/BF02638859**
- **MOLINA GRIMA E ET AL: "Gram-scale purification of eicosapentaenoic acid (EPA, 20: 5n-3) from wet Phaeodactylum tricornutum UTEX 640 biomass", JOURNAL OF APPLIED PHYCOLOGY, vol. 8, no. 4-5, 1996, pages 359-367, & 7TH INTERNATIONAL CONFERENCE OF THE INTERNATIONAL ASSOCIATION OF APPLIED ALGOLOGY; KNYSNA, SOUTH AFRICA; APRIL 16-19, 1996 ISSN: 0921-8971**


Remarks:

The file contains technical information submitted after the application was filed and not included in this specification

SEQUENCE LISTING

<110> E.I. duPont de Nemours & Company, Inc.
Zhu, Quinn

<120> HIGH EICOSAPENTAENOIC ACID OILS FROM IMPROVED OPTIMIZED STRAINS OF YARROWIA LIPOLYTICA

<130> CL4714

<150> 61/187366 <151> 2009-06-16

<150> 61/187368 <151> 2009-06-16

<150> 61/187359 <151> 2009-06-16

<160> 156

<170> PatentIn version 3.5

<210> 1
<211> 24
<212> PRT
<213> Artificial Sequence

<220>
<223> Multizyme linker

<300>
<302> MULTIZYMES AND THEIR USE IN MAKING POLYUNSATURATED FATTY ACIDS

<310> WO 2008/124048
<311> 2008-04-03
<312> 2008-10-16
<313> (1)..(24)

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(24)

<400> 1

```
Gly Ala Gly Pro Ala Arg Pro Ala Gly Leu Pro Pro Ala Thr Tyr Tyr
1               5                   10                  15

Asp Ser Leu Ala Val Met Gly Ser
            20
```

<210> 2
<211> 19
<212> PRT
<213> Artificial Sequence

<220>
<223> Multizyme linker

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(19)

<300>
<302> MULTIZYMES AND THEIR USE IN MAKING POLYUNSATURATED FATTY ACIDS

<310> WO 2008/124048
<311> 2008-04-03
<312> 2008-10-16
<313> (1)..(19)

<400> 2

```
Gly Pro Ala Arg Pro Ala Gly Leu Pro Pro Ala Thr Tyr Tyr Asp Ser
1               5                   10                  15


Leu Ala Val
```

<210> 3
<211> 18
<212> PRT
<213> Artificial Sequence

<220>
<223> Multizyme linker

<300>
<302> MULTIZYMES AND THEIR USE IN MAKING POLYUNSATURATED FATTY ACIDS

<310> WO 2008/124048
<311> 2008-04-03
<312> 2008-10-16
<313> (1)..(18)

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(18)

<400> 3

```
Pro Ala Arg Pro Ala Gly Leu Pro Pro Ala Thr Tyr Tyr Asp Ser Leu
1               5                   10                  15


Ala Val
```

<210> 4
<211> 18
<212> PRT
<213> Artificial Sequence

<220>
<223> Multizyme linker

<300>
<302> MULTIZYMES AND THEIR USE IN MAKING POLYUNSATURATED FATTY ACIDS

<310> WO 2008/124048
<311> 2008-04-03

<312> 2008-10-16
<313> (1)..(18)

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(18)

<400> 4

```
Pro Thr Arg Pro Ala Gly Pro Pro Pro Ala Thr Tyr Tyr Asp Ser Leu
1               5                   10                  15

Ala Val
```

<210> 5
<211> 33
<212> PRT
<213> Artificial Sequence

<220>
<223> Multizyme linker

<300>
<302> MULTIZYMES AND THEIR USE IN MAKING POLYUNSATURATED FATTY ACIDS

<310> WO 2008/124048
<311> 2008-04-03
<312> 2008-10-16
<313> (1)..(33)

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(33)

<400> 5

```
Pro Gly Gly Pro Gly Lys Pro Ser Glu Ile Ala Ser Leu Pro Pro Pro
1               5                   10                  15

Ile Arg Pro Val Gly Asn Pro Pro Ala Ala Tyr Tyr Asp Ala Leu Ala
            20                  25                  30

Thr
```

<210> 6
<211> 22
<212> PRT

<213> Artificial Sequence

<220>
<223> Multizyme linker

<300>
<302> MULTIZYMES AND THEIR USE IN MAKING POLYUNSATURATED FATTY ACIDS

<310> WO 2008/124048
<311> 2008-04-03
<312> 2008-10-16
<313> (1)..(22)

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(22)

<400> 6

```
Pro Ala Arg Pro Ala Gly Leu Pro Pro Ala Thr Tyr Tyr Asp Ser Leu
1               5                   10                  15

Ala Val Ser Gly Arg Thr
            20
```

<210> 7
<211> 36
<212> PRT
<213> Artificial Sequence

<220>
<223> Multizyme linker

<300>
<302> MULTIZYMES AND THEIR USE IN MAKING POLYUNSATURATED FATTY ACIDS

<310> WO 2008/124048
<311> 2008-04-03
<312> 2008-10-16
<313> (1)..(36)

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(36)

<400> 7

**Pro Gly Gly Pro Gly Lys Pro Ser Glu Ile Ala Ser Leu Pro Pro Pro**
**1 5 10 15**

**Ile Arg Pro Val Gly Asn Pro Pro Ala Ala Tyr Tyr Asp Ala Leu Ala**
**20 25 30**

**Thr Gly Arg Thr**
**35**

<210> 8
<211> 2112
<212> DNA
<213> Artificial Sequence

<220>
<223> Multizyme: EgD9ES/EgD8M gene fusion

<220>
<221> CDS
<222> (1)..(2112)
<223> Multizyme: EgD9ES/EgD8M gene fusion

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(2112)

<300>
<302> MULTIZYMES AND THEIR USE IN MAKING POLYUNSATURATED FATTY ACIDS

<310> WO 2008/124048
<311> 2008-04-03
<312> 2008-10-16
<313> (1)..(2112)

<400> 8

```
atg gag gtc gtg aac gaa atc gtc tcc att ggc cag gag gtt ctt ccc        48
Met Glu Val Val Asn Glu Ile Val Ser Ile Gly Gln Glu Val Leu Pro
1               5                   10                  15

aag gtc gac tat gct cag ctc tgg tct gat gcc tcg cac tgc gag gtg        96
Lys Val Asp Tyr Ala Gln Leu Trp Ser Asp Ala Ser His Cys Glu Val
            20                  25                  30

ctg tac ctc tcc atc gcc ttc gtc atc ctg aag ttc acc ctt ggt cct       144
Leu Tyr Leu Ser Ile Ala Phe Val Ile Leu Lys Phe Thr Leu Gly Pro
        35                  40                  45

ctc gga ccc aag ggt cag tct cga atg aag ttt gtg ttc acc aac tac       192
Leu Gly Pro Lys Gly Gln Ser Arg Met Lys Phe Val Phe Thr Asn Tyr
    50                  55                  60

aac ctg ctc atg tcc atc tac tcg ctg ggc tcc ttc ctc tct atg gcc       240
Asn Leu Leu Met Ser Ile Tyr Ser Leu Gly Ser Phe Leu Ser Met Ala
65                  70                  75                  80

tac gcc atg tac acc att ggt gtc atg tcc gac aac tgc gag aag gct       288
Tyr Ala Met Tyr Thr Ile Gly Val Met Ser Asp Asn Cys Glu Lys Ala
                85                  90                  95

ttc gac aac aat gtc ttc cga atc acc act cag ctg ttc tac ctc agc       336
Phe Asp Asn Asn Val Phe Arg Ile Thr Thr Gln Leu Phe Tyr Leu Ser
            100                 105                 110

aag ttc ctc gag tac att gac tcc ttc tat ctg ccc ctc atg ggc aag       384
Lys Phe Leu Glu Tyr Ile Asp Ser Phe Tyr Leu Pro Leu Met Gly Lys
        115                 120                 125

cct ctg acc tgg ttg cag ttc ttt cac cat ctc gga gct cct atg gac       432
Pro Leu Thr Trp Leu Gln Phe Phe His His Leu Gly Ala Pro Met Asp
    130                 135                 140

atg tgg ctg ttc tac aac tac cga aac gaa gcc gtt tgg atc ttt gtg       480
Met Trp Leu Phe Tyr Asn Tyr Arg Asn Glu Ala Val Trp Ile Phe Val
145                 150                 155                 160

ctg ctc aac ggc ttc att cac tgg atc atg tac ggc tac tat tgg acc       528
```

```
Leu Leu Asn Gly Phe Ile His Trp Ile Met Tyr Gly Tyr Tyr Trp Thr
                165                 170                 175

cga ctg atc aag ctc aag ttc cct atg ccc aag tcc ctg att act tct        576
Arg Leu Ile Lys Leu Lys Phe Pro Met Pro Lys Ser Leu Ile Thr Ser
            180                 185                 190

atg cag atc att cag ttc aac gtt ggc ttc tac atc gtc tgg aag tac        624
Met Gln Ile Ile Gln Phe Asn Val Gly Phe Tyr Ile Val Trp Lys Tyr
        195                 200                 205

cgg aac att ccc tgc tac cga caa gat gga atg aga atg ttt ggc tgg        672
Arg Asn Ile Pro Cys Tyr Arg Gln Asp Gly Met Arg Met Phe Gly Trp
    210                 215                 220

ttt ttc aac tac ttc tac gtt ggt act gtc ctg tgt ctg ttc ctc aac        720
Phe Phe Asn Tyr Phe Tyr Val Gly Thr Val Leu Cys Leu Phe Leu Asn
225                 230                 235                 240

ttc tac gtg cag acc tac atc gtc cga aag cac aag gga gcc aaa aag        768
Phe Tyr Val Gln Thr Tyr Ile Val Arg Lys His Lys Gly Ala Lys Lys
                245                 250                 255

att cag ggc gcc ggt ccc gct cga cct gcc gga ctt cct ccc gct acc        816
Ile Gln Gly Ala Gly Pro Ala Arg Pro Ala Gly Leu Pro Pro Ala Thr
            260                 265                 270

tac tac gac tct ctg gcc gtc atg gga tcc gtg aag gct tct cga cag        864
Tyr Tyr Asp Ser Leu Ala Val Met Gly Ser Val Lys Ala Ser Arg Gln
        275                 280                 285

gct ctg ccc ctc gtc atc gac gga aag gtg tac gac gtc tcc gct tgg        912
Ala Leu Pro Leu Val Ile Asp Gly Lys Val Tyr Asp Val Ser Ala Trp
    290                 295                 300

gtg aac ttc cac cct ggt gga gct gaa atc att gag aac tac cag gga        960
Val Asn Phe His Pro Gly Gly Ala Glu Ile Ile Glu Asn Tyr Gln Gly
305                 310                 315                 320

cga gat gct act gac gcc ttc atg gtt atg cac tct cag gaa gcc ttc       1008
Arg Asp Ala Thr Asp Ala Phe Met Val Met His Ser Gln Glu Ala Phe
                325                 330                 335

gac aag ctc aag cga atg ccc aag atc aac cag gct tcc gag ctg cct       1056
Asp Lys Leu Lys Arg Met Pro Lys Ile Asn Gln Ala Ser Glu Leu Pro
            340                 345                 350

ccc cag gct gcc gtc aac gaa gct cag gag gat ttc cga aag ctc cga       1104
Pro Gln Ala Ala Val Asn Glu Ala Gln Glu Asp Phe Arg Lys Leu Arg
        355                 360                 365

gaa gag ctg atc gcc act ggc atg ttt gac gcc tct ccc ctc tgg tac       1152
Glu Glu Leu Ile Ala Thr Gly Met Phe Asp Ala Ser Pro Leu Trp Tyr
    370                 375                 380

tcg tac aag atc ttg acc acc ctg ggt ctt ggc gtg ctt gcc ttc ttc       1200
Ser Tyr Lys Ile Leu Thr Thr Leu Gly Leu Gly Val Leu Ala Phe Phe
385                 390                 395                 400

atg ctg gtc cag tac cac ctg tac ttc att ggt gct ctc gtg ctc ggt       1248
Met Leu Val Gln Tyr His Leu Tyr Phe Ile Gly Ala Leu Val Leu Gly
                405                 410                 415

atg cac tac cag caa atg gga tgg ctg tct cat gac atc tgc cac cac       1296
Met His Tyr Gln Gln Met Gly Trp Leu Ser His Asp Ile Cys His His
```

```
            420                 425                 430

cag acc ttc aag aac cga aac tgg aat aac gtc ctg ggt ctg gtc ttt        1344
Gln Thr Phe Lys Asn Arg Asn Trp Asn Asn Val Leu Gly Leu Val Phe
        435                 440                 445

ggc aac gga ctc cag ggc ttc tcc gtg acc tgg tgg aag gac aga cac        1392
Gly Asn Gly Leu Gln Gly Phe Ser Val Thr Trp Trp Lys Asp Arg His
    450                 455                 460

aac gcc cat cat tct gct acc aac gtt cag ggt cac gat ccc gac att        1440
Asn Ala His His Ser Ala Thr Asn Val Gln Gly His Asp Pro Asp Ile
465                 470                 475                 480

gat aac ctg cct ctg ctc gcc tgg tcc gag gac gat gtc act cga gct        1488
Asp Asn Leu Pro Leu Leu Ala Trp Ser Glu Asp Asp Val Thr Arg Ala
                485                 490                 495

tct ccc atc tcc cga aag ctc att cag ttc caa cag tac tat ttc ctg        1536
Ser Pro Ile Ser Arg Lys Leu Ile Gln Phe Gln Gln Tyr Tyr Phe Leu
            500                 505                 510

gtc atc tgt att ctc ctg cga ttc atc tgg tgt ttc cag tct gtg ctg        1584
Val Ile Cys Ile Leu Leu Arg Phe Ile Trp Cys Phe Gln Ser Val Leu
        515                 520                 525

acc gtt cga tcc ctc aag gac cga gac aac cag ttc tac cga tct cag        1632
Thr Val Arg Ser Leu Lys Asp Arg Asp Asn Gln Phe Tyr Arg Ser Gln
    530                 535                 540

tac aag aaa gag gcc att gga ctc gct ctg cac tgg act ctc aag acc        1680
Tyr Lys Lys Glu Ala Ile Gly Leu Ala Leu His Trp Thr Leu Lys Thr
545                 550                 555                 560

ctg ttc cac ctc ttc ttt atg ccc tcc atc ctg acc tcg atg ctg gtg        1728
Leu Phe His Leu Phe Phe Met Pro Ser Ile Leu Thr Ser Met Leu Val
                565                 570                 575

ttc ttt gtt tcc gag ctc gtc ggt ggc ttc gga att gcc atc gtg gtc        1776
Phe Phe Val Ser Glu Leu Val Gly Gly Phe Gly Ile Ala Ile Val Val
            580                 585                 590

ttc atg aac cac tac cct ctg gag aag atc ggt gat tcc gtc tgg gac        1824
Phe Met Asn His Tyr Pro Leu Glu Lys Ile Gly Asp Ser Val Trp Asp
        595                 600                 605

gga cat ggc ttc tct gtg ggt cag atc cat gag acc atg aac att cga        1872
Gly His Gly Phe Ser Val Gly Gln Ile His Glu Thr Met Asn Ile Arg
    610                 615                 620

cga ggc atc att act gac tgg ttc ttt gga ggc ctg aac tac cag atc        1920
Arg Gly Ile Ile Thr Asp Trp Phe Phe Gly Gly Leu Asn Tyr Gln Ile
625                 630                 635                 640

gag cac cat ctc tgg ccc acc ctg cct cga cac aac ctc act gcc gtt        1968
Glu His His Leu Trp Pro Thr Leu Pro Arg His Asn Leu Thr Ala Val
                645                 650                 655

tcc tac cag gtg gaa cag ctg tgc cag aag cac aac ctc ccc tac cga        2016
Ser Tyr Gln Val Glu Gln Leu Cys Gln Lys His Asn Leu Pro Tyr Arg
            660                 665                 670

aac cct ctg ccc cat gaa ggt ctc gtc atc ctg ctc cga tac ctg tcc        2064
Asn Pro Leu Pro His Glu Gly Leu Val Ile Leu Leu Arg Tyr Leu Ser
        675                 680                 685
```

```
cag ttc gct cga atg gcc gag aag cag ccc ggt gcc aag gct cag taa        2112
Gln Phe Ala Arg Met Ala Glu Lys Gln Pro Gly Ala Lys Ala Gln
    690                 695                 700
```

<210> 9
<211> 703
<212> PRT
<213> Artificial Sequence

<220>
<223> Synthetic Construct

<400> 9

```
Met Glu Val Val Asn Glu Ile Val Ser Ile Gly Gln Glu Val Leu Pro
1               5                   10                  15

Lys Val Asp Tyr Ala Gln Leu Trp Ser Asp Ala Ser His Cys Glu Val
            20                  25                  30

Leu Tyr Leu Ser Ile Ala Phe Val Ile Leu Lys Phe Thr Leu Gly Pro
        35                  40                  45

Leu Gly Pro Lys Gly Gln Ser Arg Met Lys Phe Val Phe Thr Asn Tyr
    50                  55                  60

Asn Leu Leu Met Ser Ile Tyr Ser Leu Gly Ser Phe Leu Ser Met Ala
65                  70                  75                  80

Tyr Ala Met Tyr Thr Ile Gly Val Met Ser Asp Asn Cys Glu Lys Ala
                85                  90                  95

Phe Asp Asn Asn Val Phe Arg Ile Thr Thr Gln Leu Phe Tyr Leu Ser
            100                 105                 110

Lys Phe Leu Glu Tyr Ile Asp Ser Phe Tyr Leu Pro Leu Met Gly Lys
        115                 120                 125

Pro Leu Thr Trp Leu Gln Phe Phe His His Leu Gly Ala Pro Met Asp
    130                 135                 140

Met Trp Leu Phe Tyr Asn Tyr Arg Asn Glu Ala Val Trp Ile Phe Val
145                 150                 155                 160

Leu Leu Asn Gly Phe Ile His Trp Ile Met Tyr Gly Tyr Tyr Trp Thr
                165                 170                 175

Arg Leu Ile Lys Leu Lys Phe Pro Met Pro Lys Ser Leu Ile Thr Ser
            180                 185                 190

Met Gln Ile Ile Gln Phe Asn Val Gly Phe Tyr Ile Val Trp Lys Tyr
```

```
        195                 200                 205

Arg Asn Ile Pro Cys Tyr Arg Gln Asp Gly Met Arg Met Phe Gly Trp
    210                 215                 220

Phe Phe Asn Tyr Phe Tyr Val Gly Thr Val Leu Cys Leu Phe Leu Asn
225                 230                 235                 240

Phe Tyr Val Gln Thr Tyr Ile Val Arg Lys His Lys Gly Ala Lys Lys
                245                 250                 255

Ile Gln Gly Ala Gly Pro Ala Arg Pro Ala Gly Leu Pro Pro Ala Thr
            260                 265                 270

Tyr Tyr Asp Ser Leu Ala Val Met Gly Ser Val Lys Ala Ser Arg Gln
        275                 280                 285

Ala Leu Pro Leu Val Ile Asp Gly Lys Val Tyr Asp Val Ser Ala Trp
    290                 295                 300

Val Asn Phe His Pro Gly Gly Ala Glu Ile Ile Glu Asn Tyr Gln Gly
305                 310                 315                 320

Arg Asp Ala Thr Asp Ala Phe Met Val Met His Ser Gln Glu Ala Phe
                325                 330                 335

Asp Lys Leu Lys Arg Met Pro Lys Ile Asn Gln Ala Ser Glu Leu Pro
            340                 345                 350

Pro Gln Ala Ala Val Asn Glu Ala Gln Glu Asp Phe Arg Lys Leu Arg
        355                 360                 365

Glu Glu Leu Ile Ala Thr Gly Met Phe Asp Ala Ser Pro Leu Trp Tyr
    370                 375                 380

Ser Tyr Lys Ile Leu Thr Thr Leu Gly Leu Gly Val Leu Ala Phe Phe
385                 390                 395                 400

Met Leu Val Gln Tyr His Leu Tyr Phe Ile Gly Ala Leu Val Leu Gly
                405                 410                 415

Met His Tyr Gln Gln Met Gly Trp Leu Ser His Asp Ile Cys His His
            420                 425                 430

Gln Thr Phe Lys Asn Arg Asn Trp Asn Asn Val Leu Gly Leu Val Phe
        435                 440                 445

Gly Asn Gly Leu Gln Gly Phe Ser Val Thr Trp Trp Lys Asp Arg His
    450                 455                 460
```

```
Asn Ala His His Ser Ala Thr Asn Val Gln Gly His Asp Pro Asp Ile
465                 470                 475                 480

Asp Asn Leu Pro Leu Leu Ala Trp Ser Glu Asp Asp Val Thr Arg Ala
                485                 490                 495

Ser Pro Ile Ser Arg Lys Leu Ile Gln Phe Gln Gln Tyr Tyr Phe Leu
            500                 505                 510

Val Ile Cys Ile Leu Leu Arg Phe Ile Trp Cys Phe Gln Ser Val Leu
        515                 520                 525

Thr Val Arg Ser Leu Lys Asp Arg Asp Asn Gln Phe Tyr Arg Ser Gln
    530                 535                 540

Tyr Lys Lys Glu Ala Ile Gly Leu Ala Leu His Trp Thr Leu Lys Thr
545                 550                 555                 560

Leu Phe His Leu Phe Phe Met Pro Ser Ile Leu Thr Ser Met Leu Val
                565                 570                 575

Phe Phe Val Ser Glu Leu Val Gly Gly Phe Gly Ile Ala Ile Val Val
            580                 585                 590

Phe Met Asn His Tyr Pro Leu Glu Lys Ile Gly Asp Ser Val Trp Asp
        595                 600                 605

Gly His Gly Phe Ser Val Gly Gln Ile His Glu Thr Met Asn Ile Arg
    610                 615                 620

Arg Gly Ile Ile Thr Asp Trp Phe Phe Gly Gly Leu Asn Tyr Gln Ile
625                 630                 635                 640

Glu His His Leu Trp Pro Thr Leu Pro Arg His Asn Leu Thr Ala Val
                645                 650                 655

Ser Tyr Gln Val Glu Gln Leu Cys Gln Lys His Asn Leu Pro Tyr Arg
            660                 665                 670

Asn Pro Leu Pro His Glu Gly Leu Val Ile Leu Leu Arg Tyr Leu Ser
        675                 680                 685

Gln Phe Ala Arg Met Ala Glu Lys Gln Pro Gly Ala Lys Ala Gln
    690                 695                 700
```

<210> 10
<211> 2109

<212> DNA
<213> Artificial Sequence

<220>
<223> Multizyme: EaD9ES/EaD8S gene fusion

<220>
<221> CDS
<222> (1) .. (2109)
<223> Multizyme: EaD9ES/EaD8S gene fusion

<300>
<302> MULTIZYMES AND THEIR USE IN MAKING POLYUNSATURATED FATTY ACIDS

<310> WO 2008/124048
<311> 2008-04-03
<312> 2008-10-16
<313> (1) .. (2109)

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1) .. (2109)

<400> 10

```
atg gag gct gcc aag gag ctg gtc tcc atc gtc cag gag gaa ctt ccc        48
Met Glu Ala Ala Lys Glu Leu Val Ser Ile Val Gln Glu Glu Leu Pro
1               5                   10                  15

aag gtg gac tac gcc cag ctc tgg cag gac gcc tcc tct tgc gag gtt        96
Lys Val Asp Tyr Ala Gln Leu Trp Gln Asp Ala Ser Ser Cys Glu Val
            20                  25                  30

ctg tac ctc tcg gtc gct ttc gtg gcc atc aag ttc atg ctt cga cct       144
Leu Tyr Leu Ser Val Ala Phe Val Ala Ile Lys Phe Met Leu Arg Pro
        35                  40                  45

ctg gac ctc aag cga caa gcc acc ctc aaa aag ctg ttc acc gca tac       192
Leu Asp Leu Lys Arg Gln Ala Thr Leu Lys Lys Leu Phe Thr Ala Tyr
    50                  55                  60

aac ttt ctc atg tcc atc tac tcg ttc ggc tcc ttc ctg gcg atg gcc       240
Asn Phe Leu Met Ser Ile Tyr Ser Phe Gly Ser Phe Leu Ala Met Ala
65                  70                  75                  80

tac gct ctc tct gtc act ggt att ctt tcc ggc gat tgt gag act gcc       288
Tyr Ala Leu Ser Val Thr Gly Ile Leu Ser Gly Asp Cys Glu Thr Ala
                85                  90                  95

ttc aac aat gac gtg ttc cga atc acc act cag ctg ttc tac ctc agc       336
Phe Asn Asn Asp Val Phe Arg Ile Thr Thr Gln Leu Phe Tyr Leu Ser
            100                 105                 110

aag ttc gtc gag tac atc gac tcc ttc tac ctt ccc ctc atg gac aag       384
Lys Phe Val Glu Tyr Ile Asp Ser Phe Tyr Leu Pro Leu Met Asp Lys
        115                 120                 125

ccc ttg tcg ttt ctg cag ttc ttt cac cat ctc gga gct ccc atc gac       432
Pro Leu Ser Phe Leu Gln Phe Phe His His Leu Gly Ala Pro Ile Asp
    130                 135                 140

atg tgg ctg ttc tac aag tat cga aac gaa ggc gtc tgg atc ttt gtt       480
Met Trp Leu Phe Tyr Lys Tyr Arg Asn Glu Gly Val Trp Ile Phe Val
145                 150                 155                 160
```

```
ctg ctc aac ggc ttc att cac tgg atc atg tac ggt tac tat tgg acg      528
Leu Leu Asn Gly Phe Ile His Trp Ile Met Tyr Gly Tyr Tyr Trp Thr
                165                 170                 175

cga ctc atc aag ctg aac ttc cct atg ccc aag aac ctc att acc tcc      576
Arg Leu Ile Lys Leu Asn Phe Pro Met Pro Lys Asn Leu Ile Thr Ser
            180                 185                 190

atg caa att atc cag ttc aac gtc gga ttc tac atc gtc tgg aag tac      624
Met Gln Ile Ile Gln Phe Asn Val Gly Phe Tyr Ile Val Trp Lys Tyr
        195                 200                 205

cga aac gtg ccc tgc tac cgg cag gac ggt atg cga atg ttt gcc tgg      672
Arg Asn Val Pro Cys Tyr Arg Gln Asp Gly Met Arg Met Phe Ala Trp
    210                 215                 220

atc ttc aac tac tgg tat gtc ggc acg gtg ctg ctt ctg ttc ctc aac      720
Ile Phe Asn Tyr Trp Tyr Val Gly Thr Val Leu Leu Leu Phe Leu Asn
225                 230                 235                 240

ttc tac gtc cag acc tac att cgg aag cct cga aag aac cga ggc aaa      768
Phe Tyr Val Gln Thr Tyr Ile Arg Lys Pro Arg Lys Asn Arg Gly Lys
                245                 250                 255

aag gag ggc gcc ggt ccc gct cga cct gcc gga ctt cct ccc gct acc      816
Lys Glu Gly Ala Gly Pro Ala Arg Pro Ala Gly Leu Pro Pro Ala Thr
            260                 265                 270

tac tac gac tct ctg gcc gtc atg gga tcc atg gtc aag cga ccc gct      864
Tyr Tyr Asp Ser Leu Ala Val Met Gly Ser Met Val Lys Arg Pro Ala
        275                 280                 285

ctg cct ctc acc gtg gac ggt gtc acc tac gac gtt tct gcc tgg ctc      912
Leu Pro Leu Thr Val Asp Gly Val Thr Tyr Asp Val Ser Ala Trp Leu
    290                 295                 300

aac cac cat ccc gga ggt gcc gac att atc gag aac tac cga ggt cgg      960
Asn His His Pro Gly Gly Ala Asp Ile Ile Glu Asn Tyr Arg Gly Arg
305                 310                 315                 320

gat gct acc gac gtc ttc atg gtt atg cac tcc gag aac gcc gtg tcc     1008
Asp Ala Thr Asp Val Phe Met Val Met His Ser Glu Asn Ala Val Ser
                325                 330                 335

aaa ctc aga cga atg ccc atc atg gaa cct tcc tct ccc ctg act cca     1056
Lys Leu Arg Arg Met Pro Ile Met Glu Pro Ser Ser Pro Leu Thr Pro
            340                 345                 350

aca cct ccc aag cca aac tcc gac gaa cct cag gag gat ttc cga aag     1104
Thr Pro Pro Lys Pro Asn Ser Asp Glu Pro Gln Glu Asp Phe Arg Lys
        355                 360                 365

ctg cga gac gag ctc att gct gca ggc atg ttc gat gcc tct ccc atg     1152
Leu Arg Asp Glu Leu Ile Ala Ala Gly Met Phe Asp Ala Ser Pro Met
    370                 375                 380

tgg tac gct tac aag acc ctg tcg act ctc gga ctg ggt gtc ctt gcc     1200
Trp Tyr Ala Tyr Lys Thr Leu Ser Thr Leu Gly Leu Gly Val Leu Ala
385                 390                 395                 400

gtg ctg ttg atg acc cag tgg cac tgg tac ctg gtt ggt gct atc gtc     1248
Val Leu Leu Met Thr Gln Trp His Trp Tyr Leu Val Gly Ala Ile Val
                405                 410                 415
```

```
ctc ggc att cac ttt caa cag atg gga tgg ctc tcg cac gac att tgc      1296
Leu Gly Ile His Phe Gln Gln Met Gly Trp Leu Ser His Asp Ile Cys
            420                 425                 430

cat cac cag ctg ttc aag gac cga tcc atc aac aat gcc att ggc ctg      1344
His His Gln Leu Phe Lys Asp Arg Ser Ile Asn Asn Ala Ile Gly Leu
        435                 440                 445

ctc ttc gga aac gtg ctt cag ggc ttt tct gtc act tgg tgg aag gac      1392
Leu Phe Gly Asn Val Leu Gln Gly Phe Ser Val Thr Trp Trp Lys Asp
    450                 455                 460

cga cac aac gct cat cac tcc gcc acc aac gtg cag ggt cac gat ccc      1440
Arg His Asn Ala His His Ser Ala Thr Asn Val Gln Gly His Asp Pro
465                 470                 475                 480

gac atc gac aac ctg cct ctc ctg gcg tgg tcc aag gag gac gtc gag      1488
Asp Ile Asp Asn Leu Pro Leu Leu Ala Trp Ser Lys Glu Asp Val Glu
                485                 490                 495

cga gct ggc ccg ttt tct cga cgg atg atc aag tac caa cag tat tac      1536
Arg Ala Gly Pro Phe Ser Arg Arg Met Ile Lys Tyr Gln Gln Tyr Tyr
            500                 505                 510

ttc ttt ttc atc tgt gcc ctt ctg cga ttc atc tgg tgc ttt cag tcc      1584
Phe Phe Phe Ile Cys Ala Leu Leu Arg Phe Ile Trp Cys Phe Gln Ser
        515                 520                 525

att cat act gcc acg ggt ctc aag gat cga agc aat cag tac tat cga      1632
Ile His Thr Ala Thr Gly Leu Lys Asp Arg Ser Asn Gln Tyr Tyr Arg
    530                 535                 540

aga cag tac gag aag gag tcc gtc ggt ctg gca ctc cac tgg ggt ctc      1680
Arg Gln Tyr Glu Lys Glu Ser Val Gly Leu Ala Leu His Trp Gly Leu
545                 550                 555                 560

aag gcc ttg ttc tac tat ttc tac atg ccc tcg ttt ctc acc gga ctc      1728
Lys Ala Leu Phe Tyr Tyr Phe Tyr Met Pro Ser Phe Leu Thr Gly Leu
                565                 570                 575

atg gtg ttc ttt gtc tcc gag ctg ctt ggt ggc ttc gga att gcc atc      1776
Met Val Phe Phe Val Ser Glu Leu Leu Gly Gly Phe Gly Ile Ala Ile
            580                 585                 590

gtt gtc ttc atg aac cac tac cct ctg gag aag att cag gac tcc gtg      1824
Val Val Phe Met Asn His Tyr Pro Leu Glu Lys Ile Gln Asp Ser Val
        595                 600                 605

tgg gat ggt cat ggc ttc tgt gct gga cag att cac gag acc atg aac      1872
Trp Asp Gly His Gly Phe Cys Ala Gly Gln Ile His Glu Thr Met Asn
    610                 615                 620

gtt cag cga ggc ctc gtc aca gac tgg ttt ttc ggt ggc ctc aac tac      1920
Val Gln Arg Gly Leu Val Thr Asp Trp Phe Phe Gly Gly Leu Asn Tyr
625                 630                 635                 640

cag atc gaa cat cac ctg tgg cct act ctt ccc aga cac aac ctc acc      1968
Gln Ile Glu His His Leu Trp Pro Thr Leu Pro Arg His Asn Leu Thr
                645                 650                 655

gct gcc tcc atc aaa gtg gag cag ctg tgc aag aag cac aac ctg ccc      2016
Ala Ala Ser Ile Lys Val Glu Gln Leu Cys Lys Lys His Asn Leu Pro
            660                 665                 670

tac cga tct cct ccc atg ctc gaa ggt gtc ggc att ctt atc tcc tac      2064
```

```
Tyr Arg Ser Pro Pro Met Leu Glu Gly Val Gly Ile Leu Ile Ser Tyr
        675                 680                 685

ctg ggc acc ttc gct cga atg gtt gcc aag gca gac aag gcc taa          2109
Leu Gly Thr Phe Ala Arg Met Val Ala Lys Ala Asp Lys Ala
    690                 695                 700
```

<210> 11
<211> 702
<212> PRT
<213> Artificial Sequence

<220>
<223> Synthetic Construct

<400> 11

```
Met Glu Ala Ala Lys Glu Leu Val Ser Ile Val Gln Glu Glu Leu Pro
1               5                   10                  15

Lys Val Asp Tyr Ala Gln Leu Trp Gln Asp Ala Ser Ser Cys Glu Val
            20                  25                  30

Leu Tyr Leu Ser Val Ala Phe Val Ala Ile Lys Phe Met Leu Arg Pro
        35                  40                  45

Leu Asp Leu Lys Arg Gln Ala Thr Leu Lys Lys Leu Phe Thr Ala Tyr
    50                  55                  60

Asn Phe Leu Met Ser Ile Tyr Ser Phe Gly Ser Phe Leu Ala Met Ala
65                  70                  75                  80

Tyr Ala Leu Ser Val Thr Gly Ile Leu Ser Gly Asp Cys Glu Thr Ala
                85                  90                  95

Phe Asn Asn Asp Val Phe Arg Ile Thr Thr Gln Leu Phe Tyr Leu Ser
            100                 105                 110

Lys Phe Val Glu Tyr Ile Asp Ser Phe Tyr Leu Pro Leu Met Asp Lys
        115                 120                 125

Pro Leu Ser Phe Leu Gln Phe Phe His His Leu Gly Ala Pro Ile Asp
    130                 135                 140

Met Trp Leu Phe Tyr Lys Tyr Arg Asn Glu Gly Val Trp Ile Phe Val
145                 150                 155                 160

Leu Leu Asn Gly Phe Ile His Trp Ile Met Tyr Gly Tyr Tyr Trp Thr
                165                 170                 175

Arg Leu Ile Lys Leu Asn Phe Pro Met Pro Lys Asn Leu Ile Thr Ser
            180                 185                 190
```

Met Gln Ile Ile Gln Phe Asn Val Gly Phe Tyr Ile Val Trp Lys Tyr
195 200 205

Arg Asn Val Pro Cys Tyr Arg Gln Asp Gly Met Arg Met Phe Ala Trp
210 215 220

Ile Phe Asn Tyr Trp Tyr Val Gly Thr Val Leu Leu Leu Phe Leu Asn
225 230 235 240

Phe Tyr Val Gln Thr Tyr Ile Arg Lys Pro Arg Lys Asn Arg Gly Lys
245 250 255

Lys Glu Gly Ala Gly Pro Ala Arg Pro Ala Gly Leu Pro Pro Ala Thr
260 265 270

Tyr Tyr Asp Ser Leu Ala Val Met Gly Ser Met Val Lys Arg Pro Ala
275 280 285

Leu Pro Leu Thr Val Asp Gly Val Thr Tyr Asp Val Ser Ala Trp Leu
290 295 300

Asn His His Pro Gly Gly Ala Asp Ile Ile Glu Asn Tyr Arg Gly Arg
305 310 315 320

Asp Ala Thr Asp Val Phe Met Val Met His Ser Glu Asn Ala Val Ser
325 330 335

Lys Leu Arg Arg Met Pro Ile Met Glu Pro Ser Ser Pro Leu Thr Pro
340 345 350

Thr Pro Pro Lys Pro Asn Ser Asp Glu Pro Gln Glu Asp Phe Arg Lys
355 360 365

Leu Arg Asp Glu Leu Ile Ala Ala Gly Met Phe Asp Ala Ser Pro Met
370 375 380

Trp Tyr Ala Tyr Lys Thr Leu Ser Thr Leu Gly Leu Gly Val Leu Ala
385 390 395 400

Val Leu Leu Met Thr Gln Trp His Trp Tyr Leu Val Gly Ala Ile Val
405 410 415

Leu Gly Ile His Phe Gln Gln Met Gly Trp Leu Ser His Asp Ile Cys
420 425 430

His His Gln Leu Phe Lys Asp Arg Ser Ile Asn Asn Ala Ile Gly Leu
435 440 445

```
Leu Phe Gly Asn Val Leu Gln Gly Phe Ser Val Thr Trp Trp Lys Asp
    450                 455                 460

Arg His Asn Ala His His Ser Ala Thr Asn Val Gln Gly His Asp Pro
465                 470                 475                 480

Asp Ile Asp Asn Leu Pro Leu Leu Ala Trp Ser Lys Glu Asp Val Glu
                485                 490                 495

Arg Ala Gly Pro Phe Ser Arg Arg Met Ile Lys Tyr Gln Gln Tyr Tyr
            500                 505                 510

Phe Phe Phe Ile Cys Ala Leu Leu Arg Phe Ile Trp Cys Phe Gln Ser
        515                 520                 525

Ile His Thr Ala Thr Gly Leu Lys Asp Arg Ser Asn Gln Tyr Tyr Arg
    530                 535                 540

Arg Gln Tyr Glu Lys Glu Ser Val Gly Leu Ala Leu His Trp Gly Leu
545                 550                 555                 560

Lys Ala Leu Phe Tyr Tyr Phe Tyr Met Pro Ser Phe Leu Thr Gly Leu
                565                 570                 575

Met Val Phe Phe Val Ser Glu Leu Leu Gly Gly Phe Gly Ile Ala Ile
            580                 585                 590

Val Val Phe Met Asn His Tyr Pro Leu Glu Lys Ile Gln Asp Ser Val
        595                 600                 605

Trp Asp Gly His Gly Phe Cys Ala Gly Gln Ile His Glu Thr Met Asn
    610                 615                 620

Val Gln Arg Gly Leu Val Thr Asp Trp Phe Phe Gly Gly Leu Asn Tyr
625                 630                 635                 640

Gln Ile Glu His His Leu Trp Pro Thr Leu Pro Arg His Asn Leu Thr
                645                 650                 655

Ala Ala Ser Ile Lys Val Glu Gln Leu Cys Lys Lys His Asn Leu Pro
            660                 665                 670

Tyr Arg Ser Pro Pro Met Leu Glu Gly Val Gly Ile Leu Ile Ser Tyr
        675                 680                 685

Leu Gly Thr Phe Ala Arg Met Val Ala Lys Ala Asp Lys Ala
    690                 695                 700
```

<210> 12
<211> 2127
<212> DNA
<213> Artificial Sequence

<220>
<223> Multizyme: E389D9eS/EgD8M gene fusion

<220>
<221> CDS
<222> (1)..(2127)
<223> Multizyme: E389D9eS/EgD8M gene fusion

<300>
<302> MULTIZYMES AND THEIR USE IN MAKING POLYUNSATURATED FATTY ACIDS

<310> US-2008-0254191-A1
<311> 2008-04-03
<312> 2008-10-16
<313> (1)..(2127)

<300>
<302> MULTIZYMES AND THEIR USE IN MAKING POLYUNSATURATED FATTY ACIDS

<310> WO 2008/124048
<311> 2008-04-03
<312> 2008-10-16
<313> (1)..(2127)

<400> 12

```
atg gct gcc gtc atc gag gtg gcc aac gag ttc gtc gct atc act gcc        48
Met Ala Ala Val Ile Glu Val Ala Asn Glu Phe Val Ala Ile Thr Ala
1               5                   10                  15

gag acc ctt ccc aag gtg gac tat cag cga ctc tgg cga gac atc tac        96
Glu Thr Leu Pro Lys Val Asp Tyr Gln Arg Leu Trp Arg Asp Ile Tyr
            20                  25                  30

tcc tgc gag ctc ctg tac ttc tcc att gct ttc gtc atc ctc aag ttt       144
Ser Cys Glu Leu Leu Tyr Phe Ser Ile Ala Phe Val Ile Leu Lys Phe
        35                  40                  45

acc ctt ggc gag ctc tcg gat tct ggc aaa aag att ctg cga gtg ctg       192
Thr Leu Gly Glu Leu Ser Asp Ser Gly Lys Lys Ile Leu Arg Val Leu
    50                  55                  60

ttc aag tgg tac aac ctc ttc atg tcc gtc ttt tcg ctg gtg tcc ttc       240
Phe Lys Trp Tyr Asn Leu Phe Met Ser Val Phe Ser Leu Val Ser Phe
65                  70                  75                  80

ctc tgt atg ggt tac gcc atc tac acc gtt gga ctg tac tcc aac gaa       288
Leu Cys Met Gly Tyr Ala Ile Tyr Thr Val Gly Leu Tyr Ser Asn Glu
                85                  90                  95

tgc gac aga gct ttc gac aac agc ttg ttc cga ttt gcc acc aag gtc       336
Cys Asp Arg Ala Phe Asp Asn Ser Leu Phe Arg Phe Ala Thr Lys Val
            100                 105                 110

ttc tac tat tcc aag ttt ctg gag tac atc gac tct ttc tac ctt ccc       384
Phe Tyr Tyr Ser Lys Phe Leu Glu Tyr Ile Asp Ser Phe Tyr Leu Pro
        115                 120                 125

ctc atg gcc aag cct ctg tcc ttt ctg cag ttc ttt cat cac ttg gga       432
Leu Met Ala Lys Pro Leu Ser Phe Leu Gln Phe Phe His His Leu Gly
    130                 135                 140

gct cct atg gac atg tgg ctc ttc gtg cag tac tct ggc gaa tcc att       480
```

```
Ala Pro Met Asp Met Trp Leu Phe Val Gln Tyr Ser Gly Glu Ser Ile
145                 150                 155                 160

tgg atc ttt gtg ttc ctg aac gga ttc att cac ttt gtc atg tac ggc        528
Trp Ile Phe Val Phe Leu Asn Gly Phe Ile His Phe Val Met Tyr Gly
                165                 170                 175

tac tat tgg aca cgg ctg atg aag ttc aac ttt ccc atg ccc aag cag        576
Tyr Tyr Trp Thr Arg Leu Met Lys Phe Asn Phe Pro Met Pro Lys Gln
            180                 185                 190

ctc att acc gca atg cag atc acc cag ttc aac gtt ggc ttc tac ctc        624
Leu Ile Thr Ala Met Gln Ile Thr Gln Phe Asn Val Gly Phe Tyr Leu
        195                 200                 205

gtg tgg tgg tac aag gac att ccc tgt tac cga aag gat ccc atg cga        672
Val Trp Trp Tyr Lys Asp Ile Pro Cys Tyr Arg Lys Asp Pro Met Arg
    210                 215                 220

atg ctg gcc tgg atc ttc aac tac tgg tac gtc ggt acc gtt ctt ctg        720
Met Leu Ala Trp Ile Phe Asn Tyr Trp Tyr Val Gly Thr Val Leu Leu
225                 230                 235                 240

ctc ttc atc aac ttc ttt gtc aag tcc tac gtg ttt ccc aag cct aag        768
Leu Phe Ile Asn Phe Phe Val Lys Ser Tyr Val Phe Pro Lys Pro Lys
                245                 250                 255

act gcc gac aaa aag gtc cag ggc gcc ggt ccc gct cga cct gcc gga        816
Thr Ala Asp Lys Lys Val Gln Gly Ala Gly Pro Ala Arg Pro Ala Gly
            260                 265                 270

ctt cct ccc gct acc tac tac gac tct ctg gcc gtc atg gga tcc gtg        864
Leu Pro Pro Ala Thr Tyr Tyr Asp Ser Leu Ala Val Met Gly Ser Val
        275                 280                 285

aag gct tct cga cag gct ctg ccc ctc gtc atc gac gga aag gtg tac        912
Lys Ala Ser Arg Gln Ala Leu Pro Leu Val Ile Asp Gly Lys Val Tyr
    290                 295                 300

gac gtc tcc gct tgg gtg aac ttc cac cct ggt gga gct gaa atc att        960
Asp Val Ser Ala Trp Val Asn Phe His Pro Gly Gly Ala Glu Ile Ile
305                 310                 315                 320

gag aac tac cag gga cga gat gct act gac gcc ttc atg gtt atg cac       1008
Glu Asn Tyr Gln Gly Arg Asp Ala Thr Asp Ala Phe Met Val Met His
                325                 330                 335

tct cag gaa gcc ttc gac aag ctc aag cga atg ccc aag atc aac cag       1056
Ser Gln Glu Ala Phe Asp Lys Leu Lys Arg Met Pro Lys Ile Asn Gln
            340                 345                 350

gct tcc gag ctg cct ccc cag gct gcc gtc aac gaa gct cag gag gat       1104
Ala Ser Glu Leu Pro Pro Gln Ala Ala Val Asn Glu Ala Gln Glu Asp
        355                 360                 365

ttc cga aag ctc cga gaa gag ctg atc gcc act ggc atg ttt gac gcc       1152
Phe Arg Lys Leu Arg Glu Glu Leu Ile Ala Thr Gly Met Phe Asp Ala
    370                 375                 380

tct ccc ctc tgg tac tcg tac aag atc ttg acc acc ctg ggt ctt ggc       1200
Ser Pro Leu Trp Tyr Ser Tyr Lys Ile Leu Thr Thr Leu Gly Leu Gly
385                 390                 395                 400

gtg ctt gcc ttc ttc atg ctg gtc cag tac cac ctg tac ttc att ggt       1248
Val Leu Ala Phe Phe Met Leu Val Gln Tyr His Leu Tyr Phe Ile Gly
```

```
                405                 410                 415

gct ctc gtg ctc ggt atg cac tac cag caa atg gga tgg ctg tct cat      1296
Ala Leu Val Leu Gly Met His Tyr Gln Gln Met Gly Trp Leu Ser His
            420                 425                 430

gac atc tgc cac cac cag acc ttc aag aac cga aac tgg aat aac gtc      1344
Asp Ile Cys His His Gln Thr Phe Lys Asn Arg Asn Trp Asn Asn Val
        435                 440                 445

ctg ggt ctg gtc ttt ggc aac gga ctc cag ggc ttc tcc gtg acc tgg      1392
Leu Gly Leu Val Phe Gly Asn Gly Leu Gln Gly Phe Ser Val Thr Trp
    450                 455                 460

tgg aag gac aga cac aac gcc cat cat tct gct acc aac gtt cag ggt      1440
Trp Lys Asp Arg His Asn Ala His His Ser Ala Thr Asn Val Gln Gly
465                 470                 475                 480

cac gat ccc gac att gat aac ctg cct ctg ctc gcc tgg tcc gag gac      1488
His Asp Pro Asp Ile Asp Asn Leu Pro Leu Leu Ala Trp Ser Glu Asp
                485                 490                 495

gat gtc act cga gct tct ccc atc tcc cga aag ctc att cag ttc caa      1536
Asp Val Thr Arg Ala Ser Pro Ile Ser Arg Lys Leu Ile Gln Phe Gln
            500                 505                 510

cag tac tat ttc ctg gtc atc tgt att ctc ctg cga ttc atc tgg tgt      1584
Gln Tyr Tyr Phe Leu Val Ile Cys Ile Leu Leu Arg Phe Ile Trp Cys
        515                 520                 525

ttc cag tct gtg ctg acc gtt cga tcc ctc aag gac cga gac aac cag      1632
Phe Gln Ser Val Leu Thr Val Arg Ser Leu Lys Asp Arg Asp Asn Gln
    530                 535                 540

ttc tac cga tct cag tac aag aaa gag gcc att gga ctc gct ctg cac      1680
Phe Tyr Arg Ser Gln Tyr Lys Lys Glu Ala Ile Gly Leu Ala Leu His
545                 550                 555                 560

tgg act ctc aag acc ctg ttc cac ctc ttc ttt atg ccc tcc atc ctg      1728
Trp Thr Leu Lys Thr Leu Phe His Leu Phe Phe Met Pro Ser Ile Leu
                565                 570                 575

acc tcg atg ctg gtg ttc ttt gtt tcc gag ctc gtc ggt ggc ttc gga      1776
Thr Ser Met Leu Val Phe Phe Val Ser Glu Leu Val Gly Gly Phe Gly
            580                 585                 590

att gcc atc gtg gtc ttc atg aac cac tac cct ctg gag aag atc ggt      1824
Ile Ala Ile Val Val Phe Met Asn His Tyr Pro Leu Glu Lys Ile Gly
        595                 600                 605

gat tcc gtc tgg gac gga cat ggc ttc tct gtg ggt cag atc cat gag      1872
Asp Ser Val Trp Asp Gly His Gly Phe Ser Val Gly Gln Ile His Glu
    610                 615                 620

acc atg aac att cga cga ggc atc att act gac tgg ttc ttt gga ggc      1920
Thr Met Asn Ile Arg Arg Gly Ile Ile Thr Asp Trp Phe Phe Gly Gly
625                 630                 635                 640

ctg aac tac cag atc gag cac cat ctc tgg ccc acc ctg cct cga cac      1968
Leu Asn Tyr Gln Ile Glu His His Leu Trp Pro Thr Leu Pro Arg His
                645                 650                 655

aac ctc act gcc gtt tcc tac cag gtg gaa cag ctg tgc cag aag cac      2016
Asn Leu Thr Ala Val Ser Tyr Gln Val Glu Gln Leu Cys Gln Lys His
            660                 665                 670
```

```
aac ctc ccc tac cga aac cct ctg ccc cat gaa ggt ctc gtc atc ctg       2064
Asn Leu Pro Tyr Arg Asn Pro Leu Pro His Glu Gly Leu Val Ile Leu
        675                 680                 685

ctc cga tac ctg tcc cag ttc gct cga atg gcc gag aag cag ccc ggt       2112
Leu Arg Tyr Leu Ser Gln Phe Ala Arg Met Ala Glu Lys Gln Pro Gly
    690                 695                 700

gcc aag gct cag taa                                                   2127
Ala Lys Ala Gln
705
```

<210> 13
<211> 708
<212> PRT
<213> Artificial Sequence

<220>
<223> Synthetic Construct

<400> 13

```
Met Ala Ala Val Ile Glu Val Ala Asn Glu Phe Val Ala Ile Thr Ala
1               5                   10                  15

Glu Thr Leu Pro Lys Val Asp Tyr Gln Arg Leu Trp Arg Asp Ile Tyr
            20                  25                  30

Ser Cys Glu Leu Leu Tyr Phe Ser Ile Ala Phe Val Ile Leu Lys Phe
        35                  40                  45

Thr Leu Gly Glu Leu Ser Asp Ser Gly Lys Lys Ile Leu Arg Val Leu
    50                  55                  60

Phe Lys Trp Tyr Asn Leu Phe Met Ser Val Phe Ser Leu Val Ser Phe
65                  70                  75                  80

Leu Cys Met Gly Tyr Ala Ile Tyr Thr Val Gly Leu Tyr Ser Asn Glu
                85                  90                  95

Cys Asp Arg Ala Phe Asp Asn Ser Leu Phe Arg Phe Ala Thr Lys Val
            100                 105                 110

Phe Tyr Tyr Ser Lys Phe Leu Glu Tyr Ile Asp Ser Phe Tyr Leu Pro
        115                 120                 125

Leu Met Ala Lys Pro Leu Ser Phe Leu Gln Phe Phe His His Leu Gly
    130                 135                 140

Ala Pro Met Asp Met Trp Leu Phe Val Gln Tyr Ser Gly Glu Ser Ile
145                 150                 155                 160

Trp Ile Phe Val Phe Leu Asn Gly Phe Ile His Phe Val Met Tyr Gly
```

```
            165                 170                 175

Tyr Tyr Trp Thr Arg Leu Met Lys Phe Asn Phe Pro Met Pro Lys Gln
        180                 185                 190

Leu Ile Thr Ala Met Gln Ile Thr Gln Phe Asn Val Gly Phe Tyr Leu
    195                 200                 205

Val Trp Trp Tyr Lys Asp Ile Pro Cys Tyr Arg Lys Asp Pro Met Arg
210                 215                 220

Met Leu Ala Trp Ile Phe Asn Tyr Trp Tyr Val Gly Thr Val Leu Leu
225                 230                 235                 240

Leu Phe Ile Asn Phe Phe Val Lys Ser Tyr Val Phe Pro Lys Pro Lys
            245                 250                 255

Thr Ala Asp Lys Lys Val Gln Gly Ala Gly Pro Ala Arg Pro Ala Gly
        260                 265                 270

Leu Pro Pro Ala Thr Tyr Tyr Asp Ser Leu Ala Val Met Gly Ser Val
    275                 280                 285

Lys Ala Ser Arg Gln Ala Leu Pro Leu Val Ile Asp Gly Lys Val Tyr
290                 295                 300

Asp Val Ser Ala Trp Val Asn Phe His Pro Gly Gly Ala Glu Ile Ile
305                 310                 315                 320

Glu Asn Tyr Gln Gly Arg Asp Ala Thr Asp Ala Phe Met Val Met His
            325                 330                 335

Ser Gln Glu Ala Phe Asp Lys Leu Lys Arg Met Pro Lys Ile Asn Gln
        340                 345                 350

Ala Ser Glu Leu Pro Pro Gln Ala Ala Val Asn Glu Ala Gln Glu Asp
    355                 360                 365

Phe Arg Lys Leu Arg Glu Glu Leu Ile Ala Thr Gly Met Phe Asp Ala
370                 375                 380

Ser Pro Leu Trp Tyr Ser Tyr Lys Ile Leu Thr Thr Leu Gly Leu Gly
385                 390                 395                 400

Val Leu Ala Phe Phe Met Leu Val Gln Tyr His Leu Tyr Phe Ile Gly
            405                 410                 415

Ala Leu Val Leu Gly Met His Tyr Gln Gln Met Gly Trp Leu Ser His
        420                 425                 430
```

Asp Ile Cys His His Gln Thr Phe Lys Asn Arg Asn Trp Asn Asn Val
435 440 445

Leu Gly Leu Val Phe Gly Asn Gly Leu Gln Gly Phe Ser Val Thr Trp
450 455 460

Trp Lys Asp Arg His Asn Ala His His Ser Ala Thr Asn Val Gln Gly
465 470 475 480

His Asp Pro Asp Ile Asp Asn Leu Pro Leu Leu Ala Trp Ser Glu Asp
485 490 495

Asp Val Thr Arg Ala Ser Pro Ile Ser Arg Lys Leu Ile Gln Phe Gln
500 505 510

Gln Tyr Tyr Phe Leu Val Ile Cys Ile Leu Leu Arg Phe Ile Trp Cys
515 520 525

Phe Gln Ser Val Leu Thr Val Arg Ser Leu Lys Asp Arg Asp Asn Gln
530 535 540

Phe Tyr Arg Ser Gln Tyr Lys Lys Glu Ala Ile Gly Leu Ala Leu His
545 550 555 560

Trp Thr Leu Lys Thr Leu Phe His Leu Phe Phe Met Pro Ser Ile Leu
565 570 575

Thr Ser Met Leu Val Phe Phe Val Ser Glu Leu Val Gly Gly Phe Gly
580 585 590

Ile Ala Ile Val Val Phe Met Asn His Tyr Pro Leu Glu Lys Ile Gly
595 600 605

Asp Ser Val Trp Asp Gly His Gly Phe Ser Val Gly Gln Ile His Glu
610 615 620

Thr Met Asn Ile Arg Arg Gly Ile Ile Thr Asp Trp Phe Phe Gly Gly
625 630 635 640

Leu Asn Tyr Gln Ile Glu His His Leu Trp Pro Thr Leu Pro Arg His
645 650 655

Asn Leu Thr Ala Val Ser Tyr Gln Val Glu Gln Leu Cys Gln Lys His
660 665 670

Asn Leu Pro Tyr Arg Asn Pro Leu Pro His Glu Gly Leu Val Ile Leu
675 680 685

Leu Arg Tyr Leu Ser Gln Phe Ala Arg Met Ala Glu Lys Gln Pro Gly
690 695 700

Ala Lys Ala Gln
705

<210> 14
<211> 1860
<212> DNA
<213> Saccharomyces cerevisiae

<220>
<221> CDS
<222> (1)..(1860)
<223> GenBank Accession No. NP_014818; "YOR175C"

<300>
<302> Genes encoding a novel type of lysophophatidylcholine acyltransferases and their use to increase triacylglycerol production and/or modify fatty acid composition

<310> US-2008-0145867-A1
<311> 2007-06-15
<312> 2008-06-19
<313> (1)..(1860)

<300>
<302> Genes encoding a novel type of lysophophatidylcholine acyltransferases and their use to increase triacylglycerol production and/or modify fatty acid composition

<310> WO 2008/076377
<311> 2007-12-13
<312> 2008-06-26
<313> (1)..(1860)

<300>
<302> USE OF A CLASS OF GENES ENCODING LYSOPHOSPHOLIPID ACYL TRANSFERASES FOR APPLICATION IN AGRICULTURE, BIOTECHNOLOGY AND MEDICINE

<310> WO 2009/001315
<311> 2008-06-25
<312> 2008-12-31
<313> (1)..(1860)

<400> 14

```
atg tac aat cct gtg gac gct gtt tta aca aag ata att acc aac tat        48
Met Tyr Asn Pro Val Asp Ala Val Leu Thr Lys Ile Ile Thr Asn Tyr
1               5                   10                  15

ggg att gat agt ttt aca ctg cga tat gct atc tgc tta ttg gga tcg        96
Gly Ile Asp Ser Phe Thr Leu Arg Tyr Ala Ile Cys Leu Leu Gly Ser
            20                  25                  30

ttc cca ctg aat gct att ttg aag aga att ccc gag aag cgt ata ggt       144
Phe Pro Leu Asn Ala Ile Leu Lys Arg Ile Pro Glu Lys Arg Ile Gly
        35                  40                  45

tta aaa tgt tgt ttt atc att tct atg tcg atg ttt tac tta ttc ggt       192
Leu Lys Cys Cys Phe Ile Ile Ser Met Ser Met Phe Tyr Leu Phe Gly
    50                  55                  60

gtg ctg aat cta gta agt gga ttc agg acc ctg ttt att agt acc atg       240
```

```
Val Leu Asn Leu Val Ser Gly Phe Arg Thr Leu Phe Ile Ser Thr Met
65                  70                  75                  80

ttt act tac ttg atc tca aga ttt tac cgt tcc aag ttt atg cca cac        288
Phe Thr Tyr Leu Ile Ser Arg Phe Tyr Arg Ser Lys Phe Met Pro His
                85                  90                  95

ttg aat ttc atg ttt gtt atg ggt cat ttg gca ata aat cat ata cac        336
Leu Asn Phe Met Phe Val Met Gly His Leu Ala Ile Asn His Ile His
            100                 105                 110

gcc caa ttc ctt aac gaa cag act caa act acc gtt gac att aca agt        384
Ala Gln Phe Leu Asn Glu Gln Thr Gln Thr Thr Val Asp Ile Thr Ser
        115                 120                 125

tca caa atg gtt tta gcc atg aaa cta act tct ttt gca tgg tcg tac        432
Ser Gln Met Val Leu Ala Met Lys Leu Thr Ser Phe Ala Trp Ser Tyr
    130                 135                 140

tat gat ggt tca tgc act agc gaa agc gat ttc aaa gat ttg act gag        480
Tyr Asp Gly Ser Cys Thr Ser Glu Ser Asp Phe Lys Asp Leu Thr Glu
145                 150                 155                 160

cat caa aaa tct cgt gct gtc aga ggt cat cca ccc tta tta aag ttc        528
His Gln Lys Ser Arg Ala Val Arg Gly His Pro Pro Leu Leu Lys Phe
                165                 170                 175

ctg gca tat gca ttt ttc tat tca acg ttg cta act ggc cca agt ttc        576
Leu Ala Tyr Ala Phe Phe Tyr Ser Thr Leu Leu Thr Gly Pro Ser Phe
            180                 185                 190

gat tat gcc gat ttt gac agc tgg ttg aat tgt gag atg ttc cgt gac        624
Asp Tyr Ala Asp Phe Asp Ser Trp Leu Asn Cys Glu Met Phe Arg Asp
        195                 200                 205

ttg cct gaa agc aaa aag cct atg aga aga cac cac cct ggt gaa aga        672
Leu Pro Glu Ser Lys Lys Pro Met Arg Arg His His Pro Gly Glu Arg
    210                 215                 220

aga cag att cca aag aat ggt aaa ctt gca tta tgg aaa gtt gtt caa        720
Arg Gln Ile Pro Lys Asn Gly Lys Leu Ala Leu Trp Lys Val Val Gln
225                 230                 235                 240

ggt ctt gct tgg atg att tta agt aca cta gga atg aag cac ttc ccc        768
Gly Leu Ala Trp Met Ile Leu Ser Thr Leu Gly Met Lys His Phe Pro
                245                 250                 255

gta aaa tac gtt ttg gac aaa gat ggc ttc cca acg aga tct ttt ata        816
Val Lys Tyr Val Leu Asp Lys Asp Gly Phe Pro Thr Arg Ser Phe Ile
            260                 265                 270

ttc aga atc cat tac tta ttc ttg ctt ggt ttc atc cat aga ttc aag        864
Phe Arg Ile His Tyr Leu Phe Leu Leu Gly Phe Ile His Arg Phe Lys
        275                 280                 285

tac tac gct gcc tgg act att tcg gaa gga tct tgt att ttg tgc ggt        912
Tyr Tyr Ala Ala Trp Thr Ile Ser Glu Gly Ser Cys Ile Leu Cys Gly
    290                 295                 300

ttg ggt tat aat ggt tat gat tca aag aca caa aag atc aga tgg gat        960
Leu Gly Tyr Asn Gly Tyr Asp Ser Lys Thr Gln Lys Ile Arg Trp Asp
305                 310                 315                 320

cgt gtc aga aat att gac att tgg acc gta gaa acg gcg cag aat acg       1008
Arg Val Arg Asn Ile Asp Ile Trp Thr Val Glu Thr Ala Gln Asn Thr
```

```
            325                 330                 335

cgt gaa atg ttg gaa gca tgg aat atg aat act aac aag tgg cta aaa      1056
Arg Glu Met Leu Glu Ala Trp Asn Met Asn Thr Asn Lys Trp Leu Lys
            340                 345                 350

tac tct gtt tat tta cgt gtc aca aag aag ggc aaa aaa cct ggt ttc      1104
Tyr Ser Val Tyr Leu Arg Val Thr Lys Lys Gly Lys Lys Pro Gly Phe
        355                 360                 365

cgc tca act ttg ttt act ttc cta act tcc gca ttt tgg cat ggt acc      1152
Arg Ser Thr Leu Phe Thr Phe Leu Thr Ser Ala Phe Trp His Gly Thr
    370                 375                 380

aga cct ggg tac tat ctg act ttt gcg aca ggg gct ttg tac caa aca      1200
Arg Pro Gly Tyr Tyr Leu Thr Phe Ala Thr Gly Ala Leu Tyr Gln Thr
385                 390                 395                 400

tgt ggt aaa atc tac aga cgc aat ttt aga cca att ttc ttg cga gaa      1248
Cys Gly Lys Ile Tyr Arg Arg Asn Phe Arg Pro Ile Phe Leu Arg Glu
                405                 410                 415

gat ggt gtc act cct ttg cct tct aaa aaa atc tac gat tta gtt ggc      1296
Asp Gly Val Thr Pro Leu Pro Ser Lys Lys Ile Tyr Asp Leu Val Gly
            420                 425                 430

ata tat gca att aaa cta gca ttt ggt tac atg gtg caa cca ttt att      1344
Ile Tyr Ala Ile Lys Leu Ala Phe Gly Tyr Met Val Gln Pro Phe Ile
        435                 440                 445

atc ctt gat ttg aag cca tct tta atg gta tgg ggc tct gtt tat ttc      1392
Ile Leu Asp Leu Lys Pro Ser Leu Met Val Trp Gly Ser Val Tyr Phe
    450                 455                 460

tat gtt cat att att gtt gct ttc tca ttt ttc cta ttc aga gga cca      1440
Tyr Val His Ile Ile Val Ala Phe Ser Phe Phe Leu Phe Arg Gly Pro
465                 470                 475                 480

tat gct aaa caa gtt act gaa ttt ttt aaa tcc aaa caa cct aaa gaa      1488
Tyr Ala Lys Gln Val Thr Glu Phe Phe Lys Ser Lys Gln Pro Lys Glu
                485                 490                 495

ata ttc att aga aaa caa aag aag ttg gaa aaa gat att tct gca agc      1536
Ile Phe Ile Arg Lys Gln Lys Lys Leu Glu Lys Asp Ile Ser Ala Ser
            500                 505                 510

tct cca aac ttg ggt ggt ata ttg aag gca aag att gaa cat gaa aag      1584
Ser Pro Asn Leu Gly Gly Ile Leu Lys Ala Lys Ile Glu His Glu Lys
        515                 520                 525

gga aag aca gca gaa gaa gaa gaa atg aac tta ggt att cca cca att      1632
Gly Lys Thr Ala Glu Glu Glu Glu Met Asn Leu Gly Ile Pro Pro Ile
    530                 535                 540

gag tta gaa aag tgg gac aat gct aag gaa gat tgg gaa gat ttc tgc      1680
Glu Leu Glu Lys Trp Asp Asn Ala Lys Glu Asp Trp Glu Asp Phe Cys
545                 550                 555                 560

aaa gat tac aaa gaa tgg aga aat aaa aat ggt ctt gaa ata gaa gag      1728
Lys Asp Tyr Lys Glu Trp Arg Asn Lys Asn Gly Leu Glu Ile Glu Glu
                565                 570                 575

gaa aac ctt tct aaa gct ttt gaa aga ttc aag cag gaa ttt tct aac      1776
Glu Asn Leu Ser Lys Ala Phe Glu Arg Phe Lys Gln Glu Phe Ser Asn
            580                 585                 590
```

```
gct gca agt gga tca ggt gaa cgt gtg aga aaa atg agt ttt agt ggt       1824
Ala Ala Ser Gly Ser Gly Glu Arg Val Arg Lys Met Ser Phe Ser Gly
        595                 600                 605

tac tca cca aag cct att tca aaa aag gaa gag tag                       1860
Tyr Ser Pro Lys Pro Ile Ser Lys Lys Glu Glu
    610                 615
```

<210> 15
<211> 619
<212> PRT
<213> Saccharomyces cerevisiae

<400> 15

```
Met Tyr Asn Pro Val Asp Ala Val Leu Thr Lys Ile Ile Thr Asn Tyr
1               5                   10                  15

Gly Ile Asp Ser Phe Thr Leu Arg Tyr Ala Ile Cys Leu Leu Gly Ser
            20                  25                  30

Phe Pro Leu Asn Ala Ile Leu Lys Arg Ile Pro Glu Lys Arg Ile Gly
        35                  40                  45

Leu Lys Cys Cys Phe Ile Ile Ser Met Ser Met Phe Tyr Leu Phe Gly
    50                  55                  60

Val Leu Asn Leu Val Ser Gly Phe Arg Thr Leu Phe Ile Ser Thr Met
65                  70                  75                  80

Phe Thr Tyr Leu Ile Ser Arg Phe Tyr Arg Ser Lys Phe Met Pro His
                85                  90                  95

Leu Asn Phe Met Phe Val Met Gly His Leu Ala Ile Asn His Ile His
            100                 105                 110

Ala Gln Phe Leu Asn Glu Gln Thr Gln Thr Thr Val Asp Ile Thr Ser
        115                 120                 125

Ser Gln Met Val Leu Ala Met Lys Leu Thr Ser Phe Ala Trp Ser Tyr
    130                 135                 140

Tyr Asp Gly Ser Cys Thr Ser Glu Ser Asp Phe Lys Asp Leu Thr Glu
145                 150                 155                 160

His Gln Lys Ser Arg Ala Val Arg Gly His Pro Pro Leu Leu Lys Phe
                165                 170                 175

Leu Ala Tyr Ala Phe Phe Tyr Ser Thr Leu Leu Thr Gly Pro Ser Phe
            180                 185                 190
```

```
Asp Tyr Ala Asp Phe Asp Ser Trp Leu Asn Cys Glu Met Phe Arg Asp
        195                 200                 205

Leu Pro Glu Ser Lys Lys Pro Met Arg Arg His His Pro Gly Glu Arg
    210                 215                 220

Arg Gln Ile Pro Lys Asn Gly Lys Leu Ala Leu Trp Lys Val Val Gln
225                 230                 235                 240

Gly Leu Ala Trp Met Ile Leu Ser Thr Leu Gly Met Lys His Phe Pro
                245                 250                 255

Val Lys Tyr Val Leu Asp Lys Asp Gly Phe Pro Thr Arg Ser Phe Ile
            260                 265                 270

Phe Arg Ile His Tyr Leu Phe Leu Leu Gly Phe Ile His Arg Phe Lys
        275                 280                 285

Tyr Tyr Ala Ala Trp Thr Ile Ser Glu Gly Ser Cys Ile Leu Cys Gly
    290                 295                 300

Leu Gly Tyr Asn Gly Tyr Asp Ser Lys Thr Gln Lys Ile Arg Trp Asp
305                 310                 315                 320

Arg Val Arg Asn Ile Asp Ile Trp Thr Val Glu Thr Ala Gln Asn Thr
                325                 330                 335

Arg Glu Met Leu Glu Ala Trp Asn Met Asn Thr Asn Lys Trp Leu Lys
            340                 345                 350

Tyr Ser Val Tyr Leu Arg Val Thr Lys Lys Gly Lys Lys Pro Gly Phe
        355                 360                 365

Arg Ser Thr Leu Phe Thr Phe Leu Thr Ser Ala Phe Trp His Gly Thr
    370                 375                 380

Arg Pro Gly Tyr Tyr Leu Thr Phe Ala Thr Gly Ala Leu Tyr Gln Thr
385                 390                 395                 400

Cys Gly Lys Ile Tyr Arg Arg Asn Phe Arg Pro Ile Phe Leu Arg Glu
                405                 410                 415

Asp Gly Val Thr Pro Leu Pro Ser Lys Lys Ile Tyr Asp Leu Val Gly
            420                 425                 430

Ile Tyr Ala Ile Lys Leu Ala Phe Gly Tyr Met Val Gln Pro Phe Ile
        435                 440                 445

Ile Leu Asp Leu Lys Pro Ser Leu Met Val Trp Gly Ser Val Tyr Phe
```

```
    450                 455                 460

Tyr Val His Ile Ile Val Ala Phe Ser Phe Phe Leu Phe Arg Gly Pro
465                 470                 475                 480

Tyr Ala Lys Gln Val Thr Glu Phe Phe Lys Ser Lys Gln Pro Lys Glu
                485                 490                 495

Ile Phe Ile Arg Lys Gln Lys Lys Leu Glu Lys Asp Ile Ser Ala Ser
            500                 505                 510

Ser Pro Asn Leu Gly Gly Ile Leu Lys Ala Lys Ile Glu His Glu Lys
        515                 520                 525

Gly Lys Thr Ala Glu Glu Glu Glu Met Asn Leu Gly Ile Pro Pro Ile
    530                 535                 540

Glu Leu Glu Lys Trp Asp Asn Ala Lys Glu Asp Trp Glu Asp Phe Cys
545                 550                 555                 560

Lys Asp Tyr Lys Glu Trp Arg Asn Lys Asn Gly Leu Glu Ile Glu Glu
                565                 570                 575

Glu Asn Leu Ser Lys Ala Phe Glu Arg Phe Lys Gln Glu Phe Ser Asn
            580                 585                 590

Ala Ala Ser Gly Ser Gly Glu Arg Val Arg Lys Met Ser Phe Ser Gly
        595                 600                 605

Tyr Ser Pro Lys Pro Ile Ser Lys Lys Glu Glu
    610                 615
```

<210> 16
<211> 1539
<212> DNA
<213> Yarrowia lipolytica

<220>
<221> CDS
<222> (1)..(1539)
<223> GenBank Accession No. XP_505624; "YALI0F19514p"

<400> 16

```
atg gcc ttt cca tgg gca gat aag tgg gca gcc gat gcg tct gca tct        48
Met Ala Phe Pro Trp Ala Asp Lys Trp Ala Ala Asp Ala Ser Ala Ser
1               5                   10                  15

aca ggg ctg cct ccg gac ctc ctc aag att gca ttc act ctg gtc atg        96
Thr Gly Leu Pro Pro Asp Leu Leu Lys Ile Ala Phe Thr Leu Val Met
            20                  25                  30

tct tat ccg ctg agt tct ctc atg aaa cgg ctg cca gat gac gcc aaa       144
Ser Tyr Pro Leu Ser Ser Leu Met Lys Arg Leu Pro Asp Asp Ala Lys
```

```
            35                  40                  45

aac ctc aag atc atc tat atc atc tcc gtg tcc atc ttc tac atg gtg      192
Asn Leu Lys Ile Ile Tyr Ile Ile Ser Val Ser Ile Phe Tyr Met Val
    50                  55                  60

ggt gtc ttc tcc ctc tat ggc gga gct gcc act ctg ctc ttc tcc tca      240
Gly Val Phe Ser Leu Tyr Gly Gly Ala Ala Thr Leu Leu Phe Ser Ser
65                  70                  75                  80

atg ggt acc ttc ttc atc acc caa tgg aag agc cct tac atg ccc tgg      288
Met Gly Thr Phe Phe Ile Thr Gln Trp Lys Ser Pro Tyr Met Pro Trp
                85                  90                  95

gtc aat ttt ggt ttt gtc atg acc cat ctc ttc gtc aat cac ctg cgt      336
Val Asn Phe Gly Phe Val Met Thr His Leu Phe Val Asn His Leu Arg
            100                 105                 110

tcg cag ttt ttc ccc gaa aca tac gac ccc aat gtc att gac atc acc      384
Ser Gln Phe Phe Pro Glu Thr Tyr Asp Pro Asn Val Ile Asp Ile Thr
        115                 120                 125

gga gca cag atg gtt ctg tgt atg aag cta tcg tct ttt gga tgg aac      432
Gly Ala Gln Met Val Leu Cys Met Lys Leu Ser Ser Phe Gly Trp Asn
    130                 135                 140

gtc tac gat gga tgg cag att gag aag ggt gag cag ctc agc gag ttc      480
Val Tyr Asp Gly Trp Gln Ile Glu Lys Gly Glu Gln Leu Ser Glu Phe
145                 150                 155                 160

cag act aaa agg gct gtt ctc aag cac ccc agt ctt atg gac ttc cta      528
Gln Thr Lys Arg Ala Val Leu Lys His Pro Ser Leu Met Asp Phe Leu
                165                 170                 175

gct ttt gtg ttc tac ttc cct tcc att ctg aca ggt cct tct tac gac      576
Ala Phe Val Phe Tyr Phe Pro Ser Ile Leu Thr Gly Pro Ser Tyr Asp
            180                 185                 190

tat atg gag ttc cat aac tgg ctc gat ctc agc ctg ttc aag gag ctg      624
Tyr Met Glu Phe His Asn Trp Leu Asp Leu Ser Leu Phe Lys Glu Leu
        195                 200                 205

gag aaa gat aag gac ccc aag cga gct gct cga cga aag cga cac aag      672
Glu Lys Asp Lys Asp Pro Lys Arg Ala Ala Arg Arg Lys Arg His Lys
    210                 215                 220

atc ccc cga tct gga atc gct gct tcc aag aaa ctc gcc gct ggt atc      720
Ile Pro Arg Ser Gly Ile Ala Ala Ser Lys Lys Leu Ala Ala Gly Ile
225                 230                 235                 240

ttc tgg atc gtt ctg tgg acc cag gtg gac tct cga atc tcc acc gcc      768
Phe Trp Ile Val Leu Trp Thr Gln Val Asp Ser Arg Ile Ser Thr Ala
                245                 250                 255

tac gct tac tca gac gca ttc acc aag gag cac aac atc ttt gga cga      816
Tyr Ala Tyr Ser Asp Ala Phe Thr Lys Glu His Asn Ile Phe Gly Arg
            260                 265                 270

att gtg tac ctc tac atg ctc ggt ttc atg tac cga ctc aag tac tac      864
Ile Val Tyr Leu Tyr Met Leu Gly Phe Met Tyr Arg Leu Lys Tyr Tyr
        275                 280                 285

gga gcc tgg tcc att tcc gag gga gcc tgc atc ttg tct ggc ctc gga      912
Gly Ala Trp Ser Ile Ser Glu Gly Ala Cys Ile Leu Ser Gly Leu Gly
    290                 295                 300
```

```
ttc cat ggc gtg gac ccc aaa act ggc aag tac aag tgg gac cgt gtc        960
Phe His Gly Val Asp Pro Lys Thr Gly Lys Tyr Lys Trp Asp Arg Val
305                 310                 315                 320

cag aac gtg gac ccg tgg gga ttc gaa act ggt caa aac aca aag gct       1008
Gln Asn Val Asp Pro Trp Gly Phe Glu Thr Gly Gln Asn Thr Lys Ala
                325                 330                 335

ctg ctg gag gcc tgg aac cag aac act aac aag tgg cta cga aac tat       1056
Leu Leu Glu Ala Trp Asn Gln Asn Thr Asn Lys Trp Leu Arg Asn Tyr
            340                 345                 350

gtg tac ctc cga gtg gtg ccc aaa ggc caa aag cct gga ttc cga gcc       1104
Val Tyr Leu Arg Val Val Pro Lys Gly Gln Lys Pro Gly Phe Arg Ala
        355                 360                 365

act atc ttc aca ttt gtg gtt tcc gcc ttc tgg cat gga act cga cct       1152
Thr Ile Phe Thr Phe Val Val Ser Ala Phe Trp His Gly Thr Arg Pro
    370                 375                 380

ggc tac tat ctc acc ttt gtg acc gct gcc atg tac cag tct gtt ggt       1200
Gly Tyr Tyr Leu Thr Phe Val Thr Ala Ala Met Tyr Gln Ser Val Gly
385                 390                 395                 400

aag ttc ttc cga cga tac ctg cga ccc ttc ttc atg gag tct gat gga       1248
Lys Phe Phe Arg Arg Tyr Leu Arg Pro Phe Phe Met Glu Ser Asp Gly
                405                 410                 415

aag act gcc ggt ccc tat aag atc tac tac gac att gtg tgt tgg atc       1296
Lys Thr Ala Gly Pro Tyr Lys Ile Tyr Tyr Asp Ile Val Cys Trp Ile
            420                 425                 430

gtt gtc caa acc gca ttt gga tac gct acc cag tcc ttt atg att cta       1344
Val Val Gln Thr Ala Phe Gly Tyr Ala Thr Gln Ser Phe Met Ile Leu
        435                 440                 445

gac ttc tgg ctg tcg ctc aag tgt tgg aag aac tcc tgg ttc ctg tac       1392
Asp Phe Trp Leu Ser Leu Lys Cys Trp Lys Asn Ser Trp Phe Leu Tyr
    450                 455                 460

cac att gct ctg ggc gcc atc ttt gca att tct agc ccc tac aag gca       1440
His Ile Ala Leu Gly Ala Ile Phe Ala Ile Ser Ser Pro Tyr Lys Ala
465                 470                 475                 480

tgg gcg att ccc aag atc aag aaa aag cag gct gga gcc gtc act gac       1488
Trp Ala Ile Pro Lys Ile Lys Lys Lys Gln Ala Gly Ala Val Thr Asp
                485                 490                 495

aag aag gac gcc aag gag gag gtg aag aag gac acc atc aag acc aag       1536
Lys Lys Asp Ala Lys Glu Glu Val Lys Lys Asp Thr Ile Lys Thr Lys
            500                 505                 510

taa                                                                   1539
```

<210> 17
<211> 512
<212> PRT
<213> Yarrowia lipolytica

<400> 17

```
Met Ala Phe Pro Trp Ala Asp Lys Trp Ala Ala Asp Ala Ser Ala Ser
1               5                   10                  15
```

```
Thr Gly Leu Pro Pro Asp Leu Leu Lys Ile Ala Phe Thr Leu Val Met
            20                  25                  30

Ser Tyr Pro Leu Ser Ser Leu Met Lys Arg Leu Pro Asp Asp Ala Lys
        35                  40                  45

Asn Leu Lys Ile Ile Tyr Ile Ile Ser Val Ser Ile Phe Tyr Met Val
    50                  55                  60

Gly Val Phe Ser Leu Tyr Gly Gly Ala Ala Thr Leu Leu Phe Ser Ser
65                  70                  75                  80

Met Gly Thr Phe Phe Ile Thr Gln Trp Lys Ser Pro Tyr Met Pro Trp
                85                  90                  95

Val Asn Phe Gly Phe Val Met Thr His Leu Phe Val Asn His Leu Arg
            100                 105                 110

Ser Gln Phe Phe Pro Glu Thr Tyr Asp Pro Asn Val Ile Asp Ile Thr
        115                 120                 125

Gly Ala Gln Met Val Leu Cys Met Lys Leu Ser Ser Phe Gly Trp Asn
    130                 135                 140

Val Tyr Asp Gly Trp Gln Ile Glu Lys Gly Glu Gln Leu Ser Glu Phe
145                 150                 155                 160

Gln Thr Lys Arg Ala Val Leu Lys His Pro Ser Leu Met Asp Phe Leu
                165                 170                 175

Ala Phe Val Phe Tyr Phe Pro Ser Ile Leu Thr Gly Pro Ser Tyr Asp
            180                 185                 190

Tyr Met Glu Phe His Asn Trp Leu Asp Leu Ser Leu Phe Lys Glu Leu
        195                 200                 205

Glu Lys Asp Lys Asp Pro Lys Arg Ala Ala Arg Arg Lys Arg His Lys
    210                 215                 220

Ile Pro Arg Ser Gly Ile Ala Ala Ser Lys Lys Leu Ala Ala Gly Ile
225                 230                 235                 240

Phe Trp Ile Val Leu Trp Thr Gln Val Asp Ser Arg Ile Ser Thr Ala
                245                 250                 255

Tyr Ala Tyr Ser Asp Ala Phe Thr Lys Glu His Asn Ile Phe Gly Arg
            260                 265                 270
```

```
Ile Val Tyr Leu Tyr Met Leu Gly Phe Met Tyr Arg Leu Lys Tyr Tyr
        275                 280                 285

Gly Ala Trp Ser Ile Ser Glu Gly Ala Cys Ile Leu Ser Gly Leu Gly
    290                 295                 300

Phe His Gly Val Asp Pro Lys Thr Gly Lys Tyr Lys Trp Asp Arg Val
305                 310                 315                 320

Gln Asn Val Asp Pro Trp Gly Phe Glu Thr Gly Gln Asn Thr Lys Ala
                325                 330                 335

Leu Leu Glu Ala Trp Asn Gln Asn Thr Asn Lys Trp Leu Arg Asn Tyr
            340                 345                 350

Val Tyr Leu Arg Val Val Pro Lys Gly Gln Lys Pro Gly Phe Arg Ala
        355                 360                 365

Thr Ile Phe Thr Phe Val Val Ser Ala Phe Trp His Gly Thr Arg Pro
    370                 375                 380

Gly Tyr Tyr Leu Thr Phe Val Thr Ala Ala Met Tyr Gln Ser Val Gly
385                 390                 395                 400

Lys Phe Phe Arg Arg Tyr Leu Arg Pro Phe Phe Met Glu Ser Asp Gly
                405                 410                 415

Lys Thr Ala Gly Pro Tyr Lys Ile Tyr Tyr Asp Ile Val Cys Trp Ile
            420                 425                 430

Val Val Gln Thr Ala Phe Gly Tyr Ala Thr Gln Ser Phe Met Ile Leu
        435                 440                 445

Asp Phe Trp Leu Ser Leu Lys Cys Trp Lys Asn Ser Trp Phe Leu Tyr
    450                 455                 460

His Ile Ala Leu Gly Ala Ile Phe Ala Ile Ser Ser Pro Tyr Lys Ala
465                 470                 475                 480

Trp Ala Ile Pro Lys Ile Lys Lys Lys Gln Ala Gly Ala Val Thr Asp
                485                 490                 495

Lys Lys Asp Ala Lys Glu Glu Val Lys Lys Asp Thr Ile Lys Thr Lys
            500                 505                 510
```

<210> 18
<211> 7

<212> PRT
<213> Artificial Sequence

<220>
<223> membrane bound O-acyltransferase motif

<220>
<221> MISC_FEATURE
<222> (2)..(2)
<223> Xaa = Val [V] or Ile [I]

<220>
<221> misc_feature
<222> (4)..(5)
<223> Xaa can be any naturally occurring amino acid

<400> 18

```
Met Xaa Leu Xaa Xaa Lys Leu
1               5
```

<210> 19
<211> 8
<212> PRT
<213> Artificial Sequence

<220>
<223> membrane bound O-acyltransferase motif

<220>
<221> misc_feature
<222> (2)..(2)
<223> Xaa can be any naturally occurring amino acid

<220>
<221> misc_feature
<222> (6)..(7)
<223> Xaa can be any naturally occurring amino acid

<400> 19

```
Arg Xaa Lys Tyr Tyr Xaa Xaa Trp
1               5
```

<210> 20
<211> 6
<212> PRT
<213> Artificial Sequence

<220>
<223> membrane bound O-acyltransferase motif

<220>
<221> misc_feature
<222> (3)..(3)
<223> Xaa can be any naturally occurring amino acid

<400> 20

Ser Ala Xaa Trp His Gly
1 5

<210> 21
<211> 20
<212> PRT
<213> Artificial Sequence

<220>
<223> motif

<220>
<221> misc_feature
<222> (2)..(12)
<223> Xaa can be any naturally occurring amino acid

<220>
<221> misc_feature
<222> (15)..(16)
<223> Xaa can be any naturally occurring amino acid

<220>
<221> MISC_FEATURE
<222> (17)..(17)
<223> Xaa = Thr [T] or Val [V]

<220>
<221> misc_feature
<222> (18)..(19)
<223> Xaa can be any naturally occurring amino acid

<300>
<302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION

<310> U.S. Patent Publication No. 2008-0145867-A1
<311> 2007-06-15
<312> 2008-06-19
<313> (1)..(15)

<400> 21

Glu Xaa Xaa Xaa Xaa Xaa Xaa Xaa Xaa Xaa Xaa Xaa Trp Asn Xaa Xaa
1 5 10 15

Xaa Xaa Xaa Trp
20

<210> 22
<211> 1870
<212> DNA
<213> Saccharomyces cerevisiae

<220>
<221> CDS
<222> (3)..(1862)
<223> synthetic Alel (codon-optimized for Yarrowia lipolytica)

<400> 22

```
ac atg tac aac ccc gtg gac gca gtg ttg act aag att att aca aac          47
   Met Tyr Asn Pro Val Asp Ala Val Leu Thr Lys Ile Ile Thr Asn
   1               5                   10                  15
```

```
tac gga att gat tct ttt acc ctg cga tat gcc att tgt ctg ttg gga        95
Tyr Gly Ile Asp Ser Phe Thr Leu Arg Tyr Ala Ile Cys Leu Leu Gly
                20                  25                  30

tct ttt cct ctt aac gct att ctg aag cgg att cct gaa aag cga atc       143
Ser Phe Pro Leu Asn Ala Ile Leu Lys Arg Ile Pro Glu Lys Arg Ile
            35                  40                  45

ggc ctg aag tgt tgt ttt atc att tct atg tcc atg ttt tat ctc ttc       191
Gly Leu Lys Cys Cys Phe Ile Ile Ser Met Ser Met Phe Tyr Leu Phe
        50                  55                  60

ggc gtt ctg aat ctc gtg agc gga ttt cga acc ctc ttc att tcc aca       239
Gly Val Leu Asn Leu Val Ser Gly Phe Arg Thr Leu Phe Ile Ser Thr
    65                  70                  75

atg ttc aca tac ctt atc tct cgg ttc tac cga tcc aag ttt atg ccc       287
Met Phe Thr Tyr Leu Ile Ser Arg Phe Tyr Arg Ser Lys Phe Met Pro
80                  85                  90                  95

cat ctc aac ttc atg ttc gtc atg ggc cac ttg gct atc aac cac att       335
His Leu Asn Phe Met Phe Val Met Gly His Leu Ala Ile Asn His Ile
                100                 105                 110

cat gct cag ttc ctg aac gaa caa act caa acg acc gtc gat att aca       383
His Ala Gln Phe Leu Asn Glu Gln Thr Gln Thr Thr Val Asp Ile Thr
            115                 120                 125

tcc tcg cag atg gtc ctg gct atg aag ctg aca agc ttt gcc tgg tct       431
Ser Ser Gln Met Val Leu Ala Met Lys Leu Thr Ser Phe Ala Trp Ser
        130                 135                 140

tac tat gac ggt tcg tgt acg agc gag tcc gac ttc aag gac ctt acc       479
Tyr Tyr Asp Gly Ser Cys Thr Ser Glu Ser Asp Phe Lys Asp Leu Thr
    145                 150                 155

gaa cac cag aag tcc cga gcc gtc cga ggc cat cct ccc ctt ctg aaa       527
Glu His Gln Lys Ser Arg Ala Val Arg Gly His Pro Pro Leu Leu Lys
160                 165                 170                 175

ttt ttg gct tac gcc ttt ttc tac tct acc ctt ctc acc ggt ccc tcc       575
Phe Leu Ala Tyr Ala Phe Phe Tyr Ser Thr Leu Leu Thr Gly Pro Ser
                180                 185                 190

ttc gat tac gct gat ttc gac tct tgg ctg aac tgc gaa atg ttc cgg       623
Phe Asp Tyr Ala Asp Phe Asp Ser Trp Leu Asn Cys Glu Met Phe Arg
            195                 200                 205

gac ctt ccc gag tcc aag aaa ccc atg cga aga cat cat cct ggt gag       671
Asp Leu Pro Glu Ser Lys Lys Pro Met Arg Arg His His Pro Gly Glu
        210                 215                 220

cgg cgt cag att ccc aag aac ggc aag ctc gcc ctg tgg aag gtt gtc       719
Arg Arg Gln Ile Pro Lys Asn Gly Lys Leu Ala Leu Trp Lys Val Val
    225                 230                 235

cag ggc ctc gcc tgg atg att ctg agc acg ttg ggt atg aag cac ttc       767
Gln Gly Leu Ala Trp Met Ile Leu Ser Thr Leu Gly Met Lys His Phe
240                 245                 250                 255

ccc gtg aag tac gtg ctg gac aag gac gga ttt cct acc cgt tcc ttt       815
Pro Val Lys Tyr Val Leu Asp Lys Asp Gly Phe Pro Thr Arg Ser Phe
                260                 265                 270

atc ttc cgt att cat tat ctg ttt ctg ctg gga ttc atc cac cga ttt       863
```

```
Ile Phe Arg Ile His Tyr Leu Phe Leu Leu Gly Phe Ile His Arg Phe
            275                 280                 285

aag tat tac gct gcg tgg acg att agc gaa ggt tcg tgc att ctc tgt        911
Lys Tyr Tyr Ala Ala Trp Thr Ile Ser Glu Gly Ser Cys Ile Leu Cys
        290                 295                 300

ggt ctt ggt tat aat gga tac gat tct aag acc cag aag atc cgg tgg        959
Gly Leu Gly Tyr Asn Gly Tyr Asp Ser Lys Thr Gln Lys Ile Arg Trp
    305                 310                 315

gat cga gtg cgg aat att gat att tgg aca gtg gag act gca caa aac       1007
Asp Arg Val Arg Asn Ile Asp Ile Trp Thr Val Glu Thr Ala Gln Asn
320                 325                 330                 335

acc cga gag atg ctg gaa gcg tgg aac atg aat act aac aaa tgg ctg       1055
Thr Arg Glu Met Leu Glu Ala Trp Asn Met Asn Thr Asn Lys Trp Leu
                340                 345                 350

aag tat agc gtg tat ctt aga gtg act aag aag ggt aag aag cca ggt       1103
Lys Tyr Ser Val Tyr Leu Arg Val Thr Lys Lys Gly Lys Lys Pro Gly
            355                 360                 365

ttt cga tct acc ctg ttt acc ttc ctg acc tcc gcc ttt tgg cac ggt       1151
Phe Arg Ser Thr Leu Phe Thr Phe Leu Thr Ser Ala Phe Trp His Gly
        370                 375                 380

acc cgt cct gga tac tac ctt acc ttc gca act ggt gcc ctg tac caa       1199
Thr Arg Pro Gly Tyr Tyr Leu Thr Phe Ala Thr Gly Ala Leu Tyr Gln
    385                 390                 395

acc tgt gga aag atc tat aga cga aac ttt cgt ccc atc ttt ctg aga       1247
Thr Cys Gly Lys Ile Tyr Arg Arg Asn Phe Arg Pro Ile Phe Leu Arg
400                 405                 410                 415

gaa gat ggc gtg aca cct ctc ccg tcc aag aag att tac gac ctg gtc       1295
Glu Asp Gly Val Thr Pro Leu Pro Ser Lys Lys Ile Tyr Asp Leu Val
                420                 425                 430

ggc att tac gct att aag ctg gcc ttt ggt tac atg gtt caa ccc ttc       1343
Gly Ile Tyr Ala Ile Lys Leu Ala Phe Gly Tyr Met Val Gln Pro Phe
            435                 440                 445

att atc ctt gac ctg aag ccc tct ctt atg gtt tgg gga tcc gtg tat       1391
Ile Ile Leu Asp Leu Lys Pro Ser Leu Met Val Trp Gly Ser Val Tyr
        450                 455                 460

ttc tac gtg cat att att gtg gcc ttc tcg ttc ttt ctg ttc cga gga       1439
Phe Tyr Val His Ile Ile Val Ala Phe Ser Phe Phe Leu Phe Arg Gly
    465                 470                 475

cca tac gct aag cag gtt act gaa ttt ttc aaa agc aag caa ccg aag       1487
Pro Tyr Ala Lys Gln Val Thr Glu Phe Phe Lys Ser Lys Gln Pro Lys
480                 485                 490                 495

gag atc ttc atc cga aag cag aag aag ttg gaa aaa gac atc tct gcc       1535
Glu Ile Phe Ile Arg Lys Gln Lys Lys Leu Glu Lys Asp Ile Ser Ala
                500                 505                 510

tct tcc ccc aac ctc gga ggt att ctt aag gca aaa atc gaa cat gag       1583
Ser Ser Pro Asn Leu Gly Gly Ile Leu Lys Ala Lys Ile Glu His Glu
            515                 520                 525

aag gga aag acg gca gag gag gaa gag atg aac ttg ggc att cca ccc       1631
Lys Gly Lys Thr Ala Glu Glu Glu Glu Met Asn Leu Gly Ile Pro Pro
```

```
        530                 535                 540

atc gaa ctg gag aag tgg gac aac gcc aag gag gac tgg gag gat ttc        1679
Ile Glu Leu Glu Lys Trp Asp Asn Ala Lys Glu Asp Trp Glu Asp Phe
    545                 550                 555

tgc aag gac tac aag gag tgg cgg aac aag aac gga ctg gaa att gaa        1727
Cys Lys Asp Tyr Lys Glu Trp Arg Asn Lys Asn Gly Leu Glu Ile Glu
560                 565                 570                 575

gag gag aac ctg tcc aag gcc ttc gag cga ttt aag cag gaa ttt tcc        1775
Glu Glu Asn Leu Ser Lys Ala Phe Glu Arg Phe Lys Gln Glu Phe Ser
                580                 585                 590

aac gct gcg tcg ggc tct ggt gaa cgg gtt cgg aaa atg tcc ttc tcc        1823
Asn Ala Ala Ser Gly Ser Gly Glu Arg Val Arg Lys Met Ser Phe Ser
            595                 600                 605

gga tat tct cct aaa ccc atc tcg aag aaa gaa gaa tag gcggccgc           1870
Gly Tyr Ser Pro Lys Pro Ile Ser Lys Lys Glu Glu
        610                 615
```

<210> 23
<211> 619
<212> PRT
<213> Saccharomyces cerevisiae

<400> 23

```
Met Tyr Asn Pro Val Asp Ala Val Leu Thr Lys Ile Ile Thr Asn Tyr
1               5                   10                  15

Gly Ile Asp Ser Phe Thr Leu Arg Tyr Ala Ile Cys Leu Leu Gly Ser
            20                  25                  30

Phe Pro Leu Asn Ala Ile Leu Lys Arg Ile Pro Glu Lys Arg Ile Gly
        35                  40                  45

Leu Lys Cys Cys Phe Ile Ile Ser Met Ser Met Phe Tyr Leu Phe Gly
    50                  55                  60

Val Leu Asn Leu Val Ser Gly Phe Arg Thr Leu Phe Ile Ser Thr Met
65                  70                  75                  80

Phe Thr Tyr Leu Ile Ser Arg Phe Tyr Arg Ser Lys Phe Met Pro His
                85                  90                  95

Leu Asn Phe Met Phe Val Met Gly His Leu Ala Ile Asn His Ile His
            100                 105                 110

Ala Gln Phe Leu Asn Glu Gln Thr Gln Thr Thr Val Asp Ile Thr Ser
        115                 120                 125

Ser Gln Met Val Leu Ala Met Lys Leu Thr Ser Phe Ala Trp Ser Tyr
    130                 135                 140
```

Tyr Asp Gly Ser Cys Thr Ser Glu Ser Asp Phe Lys Asp Leu Thr Glu
145 150 155 160

His Gln Lys Ser Arg Ala Val Arg Gly His Pro Pro Leu Leu Lys Phe
165 170 175

Leu Ala Tyr Ala Phe Phe Tyr Ser Thr Leu Leu Thr Gly Pro Ser Phe
180 185 190

Asp Tyr Ala Asp Phe Asp Ser Trp Leu Asn Cys Glu Met Phe Arg Asp
195 200 205

Leu Pro Glu Ser Lys Lys Pro Met Arg Arg His His Pro Gly Glu Arg
210 215 220

Arg Gln Ile Pro Lys Asn Gly Lys Leu Ala Leu Trp Lys Val Val Gln
225 230 235 240

Gly Leu Ala Trp Met Ile Leu Ser Thr Leu Gly Met Lys His Phe Pro
245 250 255

Val Lys Tyr Val Leu Asp Lys Asp Gly Phe Pro Thr Arg Ser Phe Ile
260 265 270

Phe Arg Ile His Tyr Leu Phe Leu Leu Gly Phe Ile His Arg Phe Lys
275 280 285

Tyr Tyr Ala Ala Trp Thr Ile Ser Glu Gly Ser Cys Ile Leu Cys Gly
290 295 300

Leu Gly Tyr Asn Gly Tyr Asp Ser Lys Thr Gln Lys Ile Arg Trp Asp
305 310 315 320

Arg Val Arg Asn Ile Asp Ile Trp Thr Val Glu Thr Ala Gln Asn Thr
325 330 335

Arg Glu Met Leu Glu Ala Trp Asn Met Asn Thr Asn Lys Trp Leu Lys
340 345 350

Tyr Ser Val Tyr Leu Arg Val Thr Lys Lys Gly Lys Lys Pro Gly Phe
355 360 365

Arg Ser Thr Leu Phe Thr Phe Leu Thr Ser Ala Phe Trp His Gly Thr
370 375 380

Arg Pro Gly Tyr Tyr Leu Thr Phe Ala Thr Gly Ala Leu Tyr Gln Thr
385 390 395 400

Cys Gly Lys Ile Tyr Arg Arg Asn Phe Arg Pro Ile Phe Leu Arg Glu
405 410 415

Asp Gly Val Thr Pro Leu Pro Ser Lys Lys Ile Tyr Asp Leu Val Gly
420 425 430

Ile Tyr Ala Ile Lys Leu Ala Phe Gly Tyr Met Val Gln Pro Phe Ile
435 440 445

Ile Leu Asp Leu Lys Pro Ser Leu Met Val Trp Gly Ser Val Tyr Phe
450 455 460

Tyr Val His Ile Ile Val Ala Phe Ser Phe Phe Leu Phe Arg Gly Pro
465 470 475 480

Tyr Ala Lys Gln Val Thr Glu Phe Phe Lys Ser Lys Gln Pro Lys Glu
485 490 495

Ile Phe Ile Arg Lys Gln Lys Lys Leu Glu Lys Asp Ile Ser Ala Ser
500 505 510

Ser Pro Asn Leu Gly Gly Ile Leu Lys Ala Lys Ile Glu His Glu Lys
515 520 525

Gly Lys Thr Ala Glu Glu Glu Glu Met Asn Leu Gly Ile Pro Pro Ile
530 535 540

Glu Leu Glu Lys Trp Asp Asn Ala Lys Glu Asp Trp Glu Asp Phe Cys
545 550 555 560

Lys Asp Tyr Lys Glu Trp Arg Asn Lys Asn Gly Leu Glu Ile Glu Glu
565 570 575

Glu Asn Leu Ser Lys Ala Phe Glu Arg Phe Lys Gln Glu Phe Ser Asn
580 585 590

Ala Ala Ser Gly Ser Gly Glu Arg Val Arg Lys Met Ser Phe Ser Gly
595 600 605

Tyr Ser Pro Lys Pro Ile Ser Lys Lys Glu Glu
610 615

<210> 24
<211> 849
<212> DNA
<213> Caenorhabditis elegans

<220>

<221> CDS
<222> (1)..(849)
<223> GenBank Accession No. CAA98276; "clone T06E8.1"

<300>
<302> METHOD FOR THE PRODUCTION OF POLYUNSATURATED FATTY ACIDS

<310> US 2006-0168687-A1
<311> 2004-01-29
<312> 2006-07-27
<313> (1)..(849)

<300>
<302> METHOD FOR THE PRODUCTION OF POLYUNSATURATED FATTY ACIDS

<310> WO 2004/076617
<311> 2004-01-29
<312> 2004-09-10
<313> (1)..(849)

<400> 24

```
atg gag aac ttc tgg tcg atc gtc gtg ttt ttt cta ctc tca att ctc        48
Met Glu Asn Phe Trp Ser Ile Val Val Phe Phe Leu Leu Ser Ile Leu
1               5                   10                  15

ttc att tta tat aac ata tcg aca gta tgc cac tac tat atg cgg att        96
Phe Ile Leu Tyr Asn Ile Ser Thr Val Cys His Tyr Tyr Met Arg Ile
            20                  25                  30

tcg ttt tat tac ttc aca att tta ttg cat gga atg gaa gtt tgt gtt       144
Ser Phe Tyr Tyr Phe Thr Ile Leu Leu His Gly Met Glu Val Cys Val
        35                  40                  45

aca atg atc cct tct tgg cta aat ggg aag ggt gct gat tac gtg ttt       192
Thr Met Ile Pro Ser Trp Leu Asn Gly Lys Gly Ala Asp Tyr Val Phe
    50                  55                  60

cac tcg ttt ttc tat tgg tgt aaa tgg act ggt gtt cat aca aca gtc       240
His Ser Phe Phe Tyr Trp Cys Lys Trp Thr Gly Val His Thr Thr Val
65                  70                  75                  80

tat gga tat gaa aaa aca caa gtt gaa ggt ccg gct gta gtt att tgt       288
Tyr Gly Tyr Glu Lys Thr Gln Val Glu Gly Pro Ala Val Val Ile Cys
                85                  90                  95

aat cat cag agt tct ctc gac att cta tcg atg gca tca atc tgg ccg       336
Asn His Gln Ser Ser Leu Asp Ile Leu Ser Met Ala Ser Ile Trp Pro
            100                 105                 110

aag aat tgt gtt gta atg atg aaa cga att ctt gcc tat gtt cca ttc       384
Lys Asn Cys Val Val Met Met Lys Arg Ile Leu Ala Tyr Val Pro Phe
        115                 120                 125

ttc aat ctc gga gcc tac ttt tcc aac aca atc ttc atc gat cga tat       432
Phe Asn Leu Gly Ala Tyr Phe Ser Asn Thr Ile Phe Ile Asp Arg Tyr
    130                 135                 140

aac cgt gaa cgt gcg atg gct tca gtt gat tat tgt gca tct gaa atg       480
Asn Arg Glu Arg Ala Met Ala Ser Val Asp Tyr Cys Ala Ser Glu Met
145                 150                 155                 160

aag aac aga aat ctt aaa ctt tgg gta ttt ccg gaa gga aca aga aat       528
Lys Asn Arg Asn Leu Lys Leu Trp Val Phe Pro Glu Gly Thr Arg Asn
                165                 170                 175

cgt gaa gga ggg ttc att cca ttc aag aaa gga gca ttc aat att gca       576
Arg Glu Gly Gly Phe Ile Pro Phe Lys Lys Gly Ala Phe Asn Ile Ala
            180                 185                 190
```

```
gtt cgt gcg cag att ccc att att cca gtt gta ttc tca gac tat cgg      624
Val Arg Ala Gln Ile Pro Ile Ile Pro Val Val Phe Ser Asp Tyr Arg
        195                 200                 205

gat ttc tac tca aag cca ggc cga tat ttc aag aat gat gga gaa gtt      672
Asp Phe Tyr Ser Lys Pro Gly Arg Tyr Phe Lys Asn Asp Gly Glu Val
    210                 215                 220

gtt att cga gtt ctg gat gcg att cca aca aaa ggg ctc act ctt gat      720
Val Ile Arg Val Leu Asp Ala Ile Pro Thr Lys Gly Leu Thr Leu Asp
225                 230                 235                 240

gac gtc agc gag ttg tct gat atg tgt cgg gac gtt atg ttg gca gcc      768
Asp Val Ser Glu Leu Ser Asp Met Cys Arg Asp Val Met Leu Ala Ala
                245                 250                 255

tat aag gaa gtt act cta gaa gct cag caa cga aat gcg aca cgg cgt      816
Tyr Lys Glu Val Thr Leu Glu Ala Gln Gln Arg Asn Ala Thr Arg Arg
            260                 265                 270

gga gaa aca aaa gac ggg aag aaa tct gag taa                          849
Gly Glu Thr Lys Asp Gly Lys Lys Ser Glu
        275                 280
```

<210> 25
<211> 282
<212> PRT
<213> Caenorhabditis elegans

<400> 25

```
Met Glu Asn Phe Trp Ser Ile Val Val Phe Phe Leu Leu Ser Ile Leu
1               5                   10                  15

Phe Ile Leu Tyr Asn Ile Ser Thr Val Cys His Tyr Tyr Met Arg Ile
            20                  25                  30

Ser Phe Tyr Tyr Phe Thr Ile Leu Leu His Gly Met Glu Val Cys Val
        35                  40                  45

Thr Met Ile Pro Ser Trp Leu Asn Gly Lys Gly Ala Asp Tyr Val Phe
    50                  55                  60

His Ser Phe Phe Tyr Trp Cys Lys Trp Thr Gly Val His Thr Thr Val
65                  70                  75                  80

Tyr Gly Tyr Glu Lys Thr Gln Val Glu Gly Pro Ala Val Val Ile Cys
                85                  90                  95

Asn His Gln Ser Ser Leu Asp Ile Leu Ser Met Ala Ser Ile Trp Pro
            100                 105                 110

Lys Asn Cys Val Val Met Met Lys Arg Ile Leu Ala Tyr Val Pro Phe
        115                 120                 125

Phe Asn Leu Gly Ala Tyr Phe Ser Asn Thr Ile Phe Ile Asp Arg Tyr
```

```
        130                 135                 140

Asn Arg Glu Arg Ala Met Ala Ser Val Asp Tyr Cys Ala Ser Glu Met
145                 150                 155                 160

Lys Asn Arg Asn Leu Lys Leu Trp Val Phe Pro Glu Gly Thr Arg Asn
                165                 170                 175

Arg Glu Gly Gly Phe Ile Pro Phe Lys Lys Gly Ala Phe Asn Ile Ala
            180                 185                 190

Val Arg Ala Gln Ile Pro Ile Ile Pro Val Val Phe Ser Asp Tyr Arg
        195                 200                 205

Asp Phe Tyr Ser Lys Pro Gly Arg Tyr Phe Lys Asn Asp Gly Glu Val
    210                 215                 220

Val Ile Arg Val Leu Asp Ala Ile Pro Thr Lys Gly Leu Thr Leu Asp
225                 230                 235                 240

Asp Val Ser Glu Leu Ser Asp Met Cys Arg Asp Val Met Leu Ala Ala
                245                 250                 255

Tyr Lys Glu Val Thr Leu Glu Ala Gln Gln Arg Asn Ala Thr Arg Arg
            260                 265                 270

Gly Glu Thr Lys Asp Gly Lys Lys Ser Glu
        275                 280
```

<210> 26
<211> 859
<212> DNA
<213> Caenorhabditis elegans

<220>
<221> CDS
<222> (3)..(851)
<223> synthetic LPCAT (codon-optimized for Yarrowia lipolytica)

<400> 26

```
cc atg gag aac ttc tgg tcc atc gtc gtg ttc ttt ctg ctc tcc att          47
   Met Glu Asn Phe Trp Ser Ile Val Val Phe Phe Leu Leu Ser Ile
   1               5                   10                  15

ctg ttc atc ctc tac aac att tcg aca gtc tgc cac tac tac atg cga         95
Leu Phe Ile Leu Tyr Asn Ile Ser Thr Val Cys His Tyr Tyr Met Arg
                20                  25                  30

atc tcc ttc tac tac ttt acc atc ctg ctt cac ggc atg gag gtg tgc        143
Ile Ser Phe Tyr Tyr Phe Thr Ile Leu Leu His Gly Met Glu Val Cys
            35                  40                  45

gtt acc atg att ccc tct tgg ctc aac ggc aag ggt gcc gac tac gtg        191
Val Thr Met Ile Pro Ser Trp Leu Asn Gly Lys Gly Ala Asp Tyr Val
```

```
        50                  55                  60

ttt cac tcg ttc ttc tac tgg tgc aag tgg act gga gtc cac acc act        239
Phe His Ser Phe Phe Tyr Trp Cys Lys Trp Thr Gly Val His Thr Thr
    65                  70                  75

gtg tat ggc tac gag aag acc cag gtc gaa ggt cct gcc gtg gtc atc        287
Val Tyr Gly Tyr Glu Lys Thr Gln Val Glu Gly Pro Ala Val Val Ile
80                  85                  90                  95

tgc aac cat cag tcc tcg ctc gac att ctg tct atg gct tcc atc tgg        335
Cys Asn His Gln Ser Ser Leu Asp Ile Leu Ser Met Ala Ser Ile Trp
                100                 105                 110

ccc aag aac tgt gtt gtc atg atg aag cgg att ctt gcc tac gtt ccc        383
Pro Lys Asn Cys Val Val Met Met Lys Arg Ile Leu Ala Tyr Val Pro
            115                 120                 125

ttc ttc aac ctg gga gcc tac ttt tcc aac acc atc ttc atc gac cga        431
Phe Phe Asn Leu Gly Ala Tyr Phe Ser Asn Thr Ile Phe Ile Asp Arg
        130                 135                 140

tac aac cga gag cga gct atg gct tct gtc gac tac tgt gcc tcc gag        479
Tyr Asn Arg Glu Arg Ala Met Ala Ser Val Asp Tyr Cys Ala Ser Glu
    145                 150                 155

atg aag aac cga aac ctg aag ctc tgg gtg ttt ccc gaa ggc act cgg        527
Met Lys Asn Arg Asn Leu Lys Leu Trp Val Phe Pro Glu Gly Thr Arg
160                 165                 170                 175

aat cga gag ggt gga ttc att ccc ttc aag aaa ggt gcc ttc aac atc        575
Asn Arg Glu Gly Gly Phe Ile Pro Phe Lys Lys Gly Ala Phe Asn Ile
                180                 185                 190

gct gtt cga gcc cag att ccc atc att cct gtc gtg ttc tct gac tat        623
Ala Val Arg Ala Gln Ile Pro Ile Ile Pro Val Val Phe Ser Asp Tyr
            195                 200                 205

cga gac ttc tac tcc aag cct ggc cga tac ttc aag aac gat gga gag        671
Arg Asp Phe Tyr Ser Lys Pro Gly Arg Tyr Phe Lys Asn Asp Gly Glu
        210                 215                 220

gtc gtg atc cga gtc ctg gat gcc att ccc acc aag ggt ctg acc ctc        719
Val Val Ile Arg Val Leu Asp Ala Ile Pro Thr Lys Gly Leu Thr Leu
    225                 230                 235

gat gac gtc tct gag ctt tcg gac atg tgt cga gac gtc atg ctg gct        767
Asp Asp Val Ser Glu Leu Ser Asp Met Cys Arg Asp Val Met Leu Ala
240                 245                 250                 255

gcc tac aag gaa gtt acc ctc gag gct cag caa cga aac gcc act cga        815
Ala Tyr Lys Glu Val Thr Leu Glu Ala Gln Gln Arg Asn Ala Thr Arg
                260                 265                 270

aga gga gag acc aag gac ggc aag aaa tcc gag taa gcggccgc               859
Arg Gly Glu Thr Lys Asp Gly Lys Lys Ser Glu
            275                 280
```

<210> 27
<211> 282
<212> PRT

<213> Caenorhabditis elegans

<400> 27

Met Glu Asn Phe Trp Ser Ile Val Val Phe Phe Leu Leu Ser Ile Leu
1               5                   10                  15

Phe Ile Leu Tyr Asn Ile Ser Thr Val Cys His Tyr Tyr Met Arg Ile
            20                  25                  30

Ser Phe Tyr Tyr Phe Thr Ile Leu Leu His Gly Met Glu Val Cys Val
        35                  40                  45

Thr Met Ile Pro Ser Trp Leu Asn Gly Lys Gly Ala Asp Tyr Val Phe
    50                  55                  60

His Ser Phe Phe Tyr Trp Cys Lys Trp Thr Gly Val His Thr Thr Val
65                  70                  75                  80

Tyr Gly Tyr Glu Lys Thr Gln Val Glu Gly Pro Ala Val Val Ile Cys
                85                  90                  95

Asn His Gln Ser Ser Leu Asp Ile Leu Ser Met Ala Ser Ile Trp Pro
            100                 105                 110

Lys Asn Cys Val Val Met Met Lys Arg Ile Leu Ala Tyr Val Pro Phe
        115                 120                 125

Phe Asn Leu Gly Ala Tyr Phe Ser Asn Thr Ile Phe Ile Asp Arg Tyr
    130                 135                 140

Asn Arg Glu Arg Ala Met Ala Ser Val Asp Tyr Cys Ala Ser Glu Met
145                 150                 155                 160

Lys Asn Arg Asn Leu Lys Leu Trp Val Phe Pro Glu Gly Thr Arg Asn
                165                 170                 175

Arg Glu Gly Gly Phe Ile Pro Phe Lys Lys Gly Ala Phe Asn Ile Ala
            180                 185                 190

Val Arg Ala Gln Ile Pro Ile Ile Pro Val Val Phe Ser Asp Tyr Arg
        195                 200                 205

Asp Phe Tyr Ser Lys Pro Gly Arg Tyr Phe Lys Asn Asp Gly Glu Val
    210                 215                 220

Val Ile Arg Val Leu Asp Ala Ile Pro Thr Lys Gly Leu Thr Leu Asp
225                 230                 235                 240

Asp Val Ser Glu Leu Ser Asp Met Cys Arg Asp Val Met Leu Ala Ala
                245                 250                 255

Tyr Lys Glu Val Thr Leu Glu Ala Gln Gln Arg Asn Ala Thr Arg Arg
260 265 270

Gly Glu Thr Lys Asp Gly Lys Lys Ser Glu
275 280

<210> 28
<211> 945
<212> DNA
<213> Mortierella alpina

<220>
<221> CDS
<222> (1)..(945)
<223> LPAAT1

<300>
<302> High eicosapentaenoic acid producing strains of Yarrowia lipolytica

<310> US-2006-0115881-A1
<311> 2005-11-02
<312> 2006-06-01
<313> (1)..(945)

<300>
<302> High eicosapentaenoic acid producing strains of Yarrowia lipolytica

<310> WO 2006/052870
<311> 2005-11-03
<312> 2006-05-18
<313> (1)..(945)

<400> 28

```
atg tcc ata ggt tct tcc aat cct gtc ctg ctg gca gcg atc ccc ttc         48
Met Ser Ile Gly Ser Ser Asn Pro Val Leu Leu Ala Ala Ile Pro Phe
1               5                   10                  15

gtc tac ctc ttc gtc ctc cct cgt gtc ctc gcc ttc ctc cct caa aag         96
Val Tyr Leu Phe Val Leu Pro Arg Val Leu Ala Phe Leu Pro Gln Lys
            20                  25                  30

gcc cag ttc ctc gca aaa tgc atc gtg gtc ttg atc gcc acc ctt atc        144
Ala Gln Phe Leu Ala Lys Cys Ile Val Val Leu Ile Ala Thr Leu Ile
        35                  40                  45

atg tcc gtc gca ggc tgc ttc att tcc atc gtc tgt gcg ctc ctc gat        192
Met Ser Val Ala Gly Cys Phe Ile Ser Ile Val Cys Ala Leu Leu Asp
    50                  55                  60

aaa cgc tat gtg atc aac tac gtc gtc tca aga ctc ttc tca ttc ctc        240
Lys Arg Tyr Val Ile Asn Tyr Val Val Ser Arg Leu Phe Ser Phe Leu
65                  70                  75                  80

gct gca aga ccc tgc ggt gtc acc tac aag atc gtc ggc gag gaa cat        288
Ala Ala Arg Pro Cys Gly Val Thr Tyr Lys Ile Val Gly Glu Glu His
                85                  90                  95

ctg gac aag tac ccc gcc att gtc gtc tgc aac cac cag agc tcc atg        336
Leu Asp Lys Tyr Pro Ala Ile Val Val Cys Asn His Gln Ser Ser Met
            100                 105                 110

gac atg atg gtc ctg gga cgc gtc ttc cca aag cac tgt gtc gtc atg        384
```

```
Asp Met Met Val Leu Gly Arg Val Phe Pro Lys His Cys Val Val Met
        115                 120                 125

gca aag aag gaa ctt ctt tac ttt ccg ttc ctg ggc atg ttt atg aag      432
Ala Lys Lys Glu Leu Leu Tyr Phe Pro Phe Leu Gly Met Phe Met Lys
    130                 135                 140

ctg agt aac gcc atc ttc att gac cgc aag aac cac aag aag gcg atc      480
Leu Ser Asn Ala Ile Phe Ile Asp Arg Lys Asn His Lys Lys Ala Ile
145                 150                 155                 160

gag tcc acc acc caa gct gtc gcc gac atg aag aag cac aac tct gga      528
Glu Ser Thr Thr Gln Ala Val Ala Asp Met Lys Lys His Asn Ser Gly
                165                 170                 175

atc tgg att ttc ccc gaa gga aca cgt tcc cgc ttg gac aag gcc gat      576
Ile Trp Ile Phe Pro Glu Gly Thr Arg Ser Arg Leu Asp Lys Ala Asp
            180                 185                 190

ctc ttg ccc ttc aag aag gga gcc ttc cac ctc gcc att caa gcc caa      624
Leu Leu Pro Phe Lys Lys Gly Ala Phe His Leu Ala Ile Gln Ala Gln
        195                 200                 205

ctc ccg atc ctc ccc atc atc tcg caa gga tac tca cac atc tac gat      672
Leu Pro Ile Leu Pro Ile Ile Ser Gln Gly Tyr Ser His Ile Tyr Asp
    210                 215                 220

tcg tca aaa cgc tac ttc ccc ggt gga gag ctc gag atc aga gtc ctg      720
Ser Ser Lys Arg Tyr Phe Pro Gly Gly Glu Leu Glu Ile Arg Val Leu
225                 230                 235                 240

gaa cct atc ccc acc acg gga ttg acc aca gac gat gtg aac gac ctg      768
Glu Pro Ile Pro Thr Thr Gly Leu Thr Thr Asp Asp Val Asn Asp Leu
                245                 250                 255

atg gac aag act cgc aac ctg atg ctg aag cac ctc aag gag atg gat      816
Met Asp Lys Thr Arg Asn Leu Met Leu Lys His Leu Lys Glu Met Asp
            260                 265                 270

tct caa tac tcc tcc tcc acc gct gaa aac gga tcg acc cat att gac      864
Ser Gln Tyr Ser Ser Ser Thr Ala Glu Asn Gly Ser Thr His Ile Asp
        275                 280                 285

gcc gat atc gca aag tca act gcc aca tcg atc gga aac acg gac gat      912
Ala Asp Ile Ala Lys Ser Thr Ala Thr Ser Ile Gly Asn Thr Asp Asp
    290                 295                 300

gct atc aca aag agg agg aca cca aaa gag tag                          945
Ala Ile Thr Lys Arg Arg Thr Pro Lys Glu
305                 310
```

<210> 29
<211> 314
<212> PRT
<213> Mortierella alpina

<400> 29

```
Met Ser Ile Gly Ser Ser Asn Pro Val Leu Leu Ala Ala Ile Pro Phe
1               5                   10                  15

Val Tyr Leu Phe Val Leu Pro Arg Val Leu Ala Phe Leu Pro Gln Lys
            20                  25                  30
```

Ala Gln Phe Leu Ala Lys Cys Ile Val Val Leu Ile Ala Thr Leu Ile
35 40 45

Met Ser Val Ala Gly Cys Phe Ile Ser Ile Val Cys Ala Leu Leu Asp
50 55 60

Lys Arg Tyr Val Ile Asn Tyr Val Val Ser Arg Leu Phe Ser Phe Leu
65 70 75 80

Ala Ala Arg Pro Cys Gly Val Thr Tyr Lys Ile Val Gly Glu Glu His
85 90 95

Leu Asp Lys Tyr Pro Ala Ile Val Val Cys Asn His Gln Ser Ser Met
100 105 110

Asp Met Met Val Leu Gly Arg Val Phe Pro Lys His Cys Val Val Met
115 120 125

Ala Lys Lys Glu Leu Leu Tyr Phe Pro Phe Leu Gly Met Phe Met Lys
130 135 140

Leu Ser Asn Ala Ile Phe Ile Asp Arg Lys Asn His Lys Lys Ala Ile
145 150 155 160

Glu Ser Thr Thr Gln Ala Val Ala Asp Met Lys Lys His Asn Ser Gly
165 170 175

Ile Trp Ile Phe Pro Glu Gly Thr Arg Ser Arg Leu Asp Lys Ala Asp
180 185 190

Leu Leu Pro Phe Lys Lys Gly Ala Phe His Leu Ala Ile Gln Ala Gln
195 200 205

Leu Pro Ile Leu Pro Ile Ile Ser Gln Gly Tyr Ser His Ile Tyr Asp
210 215 220

Ser Ser Lys Arg Tyr Phe Pro Gly Gly Glu Leu Glu Ile Arg Val Leu
225 230 235 240

Glu Pro Ile Pro Thr Thr Gly Leu Thr Thr Asp Asp Val Asn Asp Leu
245 250 255

Met Asp Lys Thr Arg Asn Leu Met Leu Lys His Leu Lys Glu Met Asp
260 265 270

Ser Gln Tyr Ser Ser Ser Thr Ala Glu Asn Gly Ser Thr His Ile Asp
275 280 285

```
Ala Asp Ile Ala Lys Ser Thr Ala Thr Ser Ile Gly Asn Thr Asp Asp
    290                 295                 300

Ala Ile Thr Lys Arg Arg Thr Pro Lys Glu
305                 310
```

<210> 30
<211> 1549
<212> DNA
<213> Yarrowia lipolytica

<220>
<221> CDS
<222> (501)..(1349)
<223> LPAAT1

<300>
<302> High eicosapentaenoic acid producing strains of Yarrowia lipolytica

<310> US-2006-0115881-A1
<311> 2005-11-02
<312> 2006-06-01
<313> (1)..(1549)

<300>
<302> High eicosapentaenoic acid producing strains of Yarrowia lipolytica

<310> WO 2006/052870
<311> 2005-11-03
<312> 2006-05-18
<313> (1)..(1549)

<400> 30

```
cacagcataa taccacggca tgaccccgct gactccaacc ttcatttcgg cacatgtagg      60
tgcacaaggg acttcaagag gggccaattt catgcggaca catggcgcaa aaaacgcccg     120
actttgatta cacagacacg taataacgac gaagccgaga tgagcacacg tggccaagtc     180
tgccaatggc cccctggacc cccctgacaa agtttcccaa caagcccagc cgtgcatggt     240
gtgtttttgt gcggagacac acgccaatta ggctcatttg agggtatgca gcgaaaaaaa     300
attagtgtgg gtagtttgtt tgcaggaatc aagtgggtgg ttgaaaaaca agaaagagcg     360
acgacaagag agagagaaaa agagagagag actccataaa gcgtgcatca aaattaaggt     420
gtgtgactat ccgaaaacca aacatgaaca gttggatata tgtcgctgtg attgcagttg     480
ctgccgttct cattgcccga atg tcc gtt gca tcc aag ctc gtc ttc tac gtc     533
                      Met Ser Val Ala Ser Lys Leu Val Phe Tyr Val
                      1               5                   10

cgc gcc gcc atc gcc gtg gtc atc ttt gcc gcc tgt gcc acc tac ggc       581
Arg Ala Ala Ile Ala Val Val Ile Phe Ala Ala Cys Ala Thr Tyr Gly
            15                  20                  25

gtg ctg gcg tcc acc att ctc acc gcc atc ggc aag cag ggc ctg gcc       629
Val Leu Ala Ser Thr Ile Leu Thr Ala Ile Gly Lys Gln Gly Leu Ala
        30                  35                  40
```

```
caa tgg acc gtt gcc aga gcc ttc tac tac tcg gtg cgc atc ttc ctg        677
Gln Trp Thr Val Ala Arg Ala Phe Tyr Tyr Ser Val Arg Ile Phe Leu
    45                  50                  55

ggt atc agc atc aag ctg cgt agc cgg cag gtg acc gga acc gcc ggt        725
Gly Ile Ser Ile Lys Leu Arg Ser Arg Gln Val Thr Gly Thr Ala Gly
60                  65                  70                  75

ctg gat gcc tcc aag atc cag gtc gcc aac acc acc aag ccc att gac        773
Leu Asp Ala Ser Lys Ile Gln Val Ala Asn Thr Thr Lys Pro Ile Asp
                80                  85                  90

gac atc acc aaa cac ctg ccc cga cca tgc att ctg att tcc aac cac        821
Asp Ile Thr Lys His Leu Pro Arg Pro Cys Ile Leu Ile Ser Asn His
            95                  100                 105

cag aac gaa atg gac att ctg gtg ctc ggt cgc atc ttc ccc cag tac        869
Gln Asn Glu Met Asp Ile Leu Val Leu Gly Arg Ile Phe Pro Gln Tyr
        110                 115                 120

tgc tcc gtc acc gcc aaa aag gcc ctc aag tgg tac cct ctg ctg ggc        917
Cys Ser Val Thr Ala Lys Lys Ala Leu Lys Trp Tyr Pro Leu Leu Gly
    125                 130                 135

cag ttc atg gcg ctg tcc ggc acc atc ttc ctg gac cga aag gac cga        965
Gln Phe Met Ala Leu Ser Gly Thr Ile Phe Leu Asp Arg Lys Asp Arg
140                 145                 150                 155

acc aag tcc gtg cag acc ctc ggc ggc gcc gtc aag acc atc cag agc       1013
Thr Lys Ser Val Gln Thr Leu Gly Gly Ala Val Lys Thr Ile Gln Ser
                160                 165                 170

ggc aac gga ggc aag ggc cag agc gtc ttc atg ttc ccc gag gga acc       1061
Gly Asn Gly Gly Lys Gly Gln Ser Val Phe Met Phe Pro Glu Gly Thr
            175                 180                 185

cga tcc tac tcc aag gac gtc ggc atc atg ccc ttc aag aag ggc tgt       1109
Arg Ser Tyr Ser Lys Asp Val Gly Ile Met Pro Phe Lys Lys Gly Cys
        190                 195                 200

ttc cac ctg gcg gtc cag tcg ggc gct ccc att gtc ccc gtg gtg gtc       1157
Phe His Leu Ala Val Gln Ser Gly Ala Pro Ile Val Pro Val Val Val
    205                 210                 215

cag aac acc tcc cga atg ttt tct ttc ggc cga ggc aag ctg gac gcc       1205
Gln Asn Thr Ser Arg Met Phe Ser Phe Gly Arg Gly Lys Leu Asp Ala
220                 225                 230                 235

gga gag atc ctt gtc gac gtc ctg agc ccc att gag acc aag ggt ctg       1253
Gly Glu Ile Leu Val Asp Val Leu Ser Pro Ile Glu Thr Lys Gly Leu
                240                 245                 250

gac gcc agc aac gtc gac gct ctc atg gcc acc act tat aag gcc atg       1301
Asp Ala Ser Asn Val Asp Ala Leu Met Ala Thr Thr Tyr Lys Ala Met
            255                 260                 265

tgc gag act gcc gac cag att ggc tac gct ggc cag aag act cag tag       1349
Cys Glu Thr Ala Asp Gln Ile Gly Tyr Ala Gly Gln Lys Thr Gln
        270                 275                 280

agactgcagc acaagaagtg cttgtagcta ctttaggaga gagataggta atatgaaaca     1409

tttttcagat cgacacccac ggcgaaccat tggctgtgga gctatgggtg aatggattaa     1469

tatagcaacg aaatctacct cgattaccaa cgcaaaacga gcccactttc tctgtactgt     1529
```

gctatatcgt gtatacccca 1549

<210> 31
<211> 282
<212> PRT
<213> Yarrowia lipolytica

<400> 31

Met Ser Val Ala Ser Lys Leu Val Phe Tyr Val Arg Ala Ala Ile Ala
1               5                   10                  15

Val Val Ile Phe Ala Ala Cys Ala Thr Tyr Gly Val Leu Ala Ser Thr
            20                  25                  30

Ile Leu Thr Ala Ile Gly Lys Gln Gly Leu Ala Gln Trp Thr Val Ala
        35                  40                  45

Arg Ala Phe Tyr Tyr Ser Val Arg Ile Phe Leu Gly Ile Ser Ile Lys
    50                  55                  60

Leu Arg Ser Arg Gln Val Thr Gly Thr Ala Gly Leu Asp Ala Ser Lys
65                  70                  75                  80

Ile Gln Val Ala Asn Thr Thr Lys Pro Ile Asp Asp Ile Thr Lys His
                85                  90                  95

Leu Pro Arg Pro Cys Ile Leu Ile Ser Asn His Gln Asn Glu Met Asp
            100                 105                 110

Ile Leu Val Leu Gly Arg Ile Phe Pro Gln Tyr Cys Ser Val Thr Ala
        115                 120                 125

Lys Lys Ala Leu Lys Trp Tyr Pro Leu Leu Gly Gln Phe Met Ala Leu
    130                 135                 140

Ser Gly Thr Ile Phe Leu Asp Arg Lys Asp Arg Thr Lys Ser Val Gln
145                 150                 155                 160

Thr Leu Gly Gly Ala Val Lys Thr Ile Gln Ser Gly Asn Gly Gly Lys
                165                 170                 175

Gly Gln Ser Val Phe Met Phe Pro Glu Gly Thr Arg Ser Tyr Ser Lys
            180                 185                 190

Asp Val Gly Ile Met Pro Phe Lys Lys Gly Cys Phe His Leu Ala Val
        195                 200                 205

Gln Ser Gly Ala Pro Ile Val Pro Val Val Val Gln Asn Thr Ser Arg
    210                 215                 220

Met Phe Ser Phe Gly Arg Gly Lys Leu Asp Ala Gly Glu Ile Leu Val
225 230 235 240

Asp Val Leu Ser Pro Ile Glu Thr Lys Gly Leu Asp Ala Ser Asn Val
245 250 255

Asp Ala Leu Met Ala Thr Thr Tyr Lys Ala Met Cys Glu Thr Ala Asp
260 265 270

Gln Ile Gly Tyr Ala Gly Gln Lys Thr Gln
275 280

<210> 32
<211> 303
<212> PRT
<213> Saccharomyces cerevisiae

<220>
<221> MISC_FEATURE
<222> (1)..(303)
<223> Slc1p; GenBank Accession No. NP_010231

<400> 32

```
Met Ser Val Ile Gly Arg Phe Leu Tyr Tyr Leu Arg Ser Val Leu Val
1               5                   10                  15

Val Leu Ala Leu Ala Gly Cys Gly Phe Tyr Gly Val Ile Ala Ser Ile
            20                  25                  30

Leu Cys Thr Leu Ile Gly Lys Gln His Leu Ala Gln Trp Ile Thr Ala
        35                  40                  45

Arg Cys Phe Tyr His Val Met Lys Leu Met Leu Gly Leu Asp Val Lys
    50                  55                  60

Val Val Gly Glu Glu Asn Leu Ala Lys Lys Pro Tyr Ile Met Ile Ala
65                  70                  75                  80

Asn His Gln Ser Thr Leu Asp Ile Phe Met Leu Gly Arg Ile Phe Pro
                85                  90                  95

Pro Gly Cys Thr Val Thr Ala Lys Lys Ser Leu Lys Tyr Val Pro Phe
            100                 105                 110

Leu Gly Trp Phe Met Ala Leu Ser Gly Thr Tyr Phe Leu Asp Arg Ser
        115                 120                 125

Lys Arg Gln Glu Ala Ile Asp Thr Leu Asn Lys Gly Leu Glu Asn Val
    130                 135                 140
```

Lys Lys Asn Lys Arg Ala Leu Trp Val Phe Pro Glu Gly Thr Arg Ser
145 150 155 160

Tyr Thr Ser Glu Leu Thr Met Leu Pro Phe Lys Lys Gly Ala Phe His
165 170 175

Leu Ala Gln Gln Gly Lys Ile Pro Ile Val Pro Val Val Val Ser Asn
180 185 190

Thr Ser Thr Leu Val Ser Pro Lys Tyr Gly Val Phe Asn Arg Gly Cys
195 200 205

Met Ile Val Arg Ile Leu Lys Pro Ile Ser Thr Glu Asn Leu Thr Lys
210 215 220

Asp Lys Ile Gly Glu Phe Ala Glu Lys Val Arg Asp Gln Met Val Asp
225 230 235 240

Thr Leu Lys Glu Ile Gly Tyr Ser Pro Ala Ile Asn Asp Thr Thr Leu
245 250 255

Pro Pro Gln Ala Ile Glu Tyr Ala Ala Leu Gln His Asp Lys Lys Val
260 265 270

Asn Lys Lys Ile Lys Asn Glu Pro Val Pro Ser Val Ser Ile Ser Asn
275 280 285

Asp Val Asn Thr His Asn Glu Gly Ser Ser Val Lys Lys Met His
290 295 300

<210> 33
<211> 7
<212> PRT
<213> Artificial Sequence

<220>
<223> 1-acyl-sn-glycerol-3-phosphate acyltransferase motif

<220>
<221> misc_feature
<222> (3)..(6)
<223> Xaa can be any naturally occurring amino acid

<400> 33

Asn His Xaa Xaa Xaa Xaa Asp
1 5

<210> 34

<211> 4
<212> PRT
<213> Artificial Sequence

<220>
<223> 1-acyl-sn-glycerol-3-phosphate acyltransferase motif

<400> 34

Glu Gly Thr Arg
1

<210> 35
<211> 955
<212> DNA
<213> Mortierella alpina

<220>
<221> CDS
<222> (3)..(947)
<223> synthetic LPAAT1 (codon-optimized for Yarrowia lipolytica)

<400> 35

```
ac atg tct att ggt tcg tcc aac ccc gtg ctc ttg gct gcg att ccc        47
   Met Ser Ile Gly Ser Ser Asn Pro Val Leu Leu Ala Ala Ile Pro
   1               5                   10                  15

ttc gtc tac ctg ttt gtc ctc cca cga gtc ctg gct ttc ctg cct cag       95
Phe Val Tyr Leu Phe Val Leu Pro Arg Val Leu Ala Phe Leu Pro Gln
                20                  25                  30

aag gct cag ttc ctg gcc aaa tgt att gtg gtc ctg att gcc acg ctt      143
Lys Ala Gln Phe Leu Ala Lys Cys Ile Val Val Leu Ile Ala Thr Leu
            35                  40                  45

atc atg tcc gtt gca ggc tgc ttc atc tcg atc gtg tgc gct ctt ctg      191
Ile Met Ser Val Ala Gly Cys Phe Ile Ser Ile Val Cys Ala Leu Leu
        50                  55                  60

gac aag aga tac gtc atc aat tac gtt gtg tcg cga ttg ttc tcc ttc      239
Asp Lys Arg Tyr Val Ile Asn Tyr Val Val Ser Arg Leu Phe Ser Phe
    65                  70                  75

ctt gcc gct cga ccg tgt ggt gtg acc tat aag att gtt ggt gag gaa      287
Leu Ala Ala Arg Pro Cys Gly Val Thr Tyr Lys Ile Val Gly Glu Glu
80                  85                  90                  95

cac ctc gat aag tac cct gct atc gtg gtc tgt aac cat caa tcc tct      335
His Leu Asp Lys Tyr Pro Ala Ile Val Val Cys Asn His Gln Ser Ser
                100                 105                 110

atg gat atg atg gtt ttg gga cga gtt ttt cca aag cac tgc gtt gtc      383
Met Asp Met Met Val Leu Gly Arg Val Phe Pro Lys His Cys Val Val
            115                 120                 125

atg gcg aag aag gaa ctc ctg tac ttt ccc ttt ttg gga atg ttt atg      431
Met Ala Lys Lys Glu Leu Leu Tyr Phe Pro Phe Leu Gly Met Phe Met
        130                 135                 140

aaa ctg agc aac gct atc ttc atc gac cgg aag aac cac aag aaa gcc      479
Lys Leu Ser Asn Ala Ile Phe Ile Asp Arg Lys Asn His Lys Lys Ala
    145                 150                 155

atc gag tct acc acc caa gcc gtg gcg gac atg aag aag cac aac tct      527
Ile Glu Ser Thr Thr Gln Ala Val Ala Asp Met Lys Lys His Asn Ser
```

```
160                 165                 170                 175

gga atc tgg att ttc cca gag ggc acc cgg tct aga ctg gac aag gca        575
Gly Ile Trp Ile Phe Pro Glu Gly Thr Arg Ser Arg Leu Asp Lys Ala
                180                 185                 190

gac ctg ctg ccc ttc aag aaa ggt gcc ttt cat ctt gca att cag gcc        623
Asp Leu Leu Pro Phe Lys Lys Gly Ala Phe His Leu Ala Ile Gln Ala
            195                 200                 205

cag ctc cct att ctc ccc att atc tcg cag ggc tat tcc cat atc tac        671
Gln Leu Pro Ile Leu Pro Ile Ile Ser Gln Gly Tyr Ser His Ile Tyr
        210                 215                 220

gac tct tcg aag cgg tac ttc ccc ggt gga gag ctc gag atc aga gtc        719
Asp Ser Ser Lys Arg Tyr Phe Pro Gly Gly Glu Leu Glu Ile Arg Val
    225                 230                 235

ctg gag ccc att cct aca act ggc ctc act act gat gat gtg aac gac        767
Leu Glu Pro Ile Pro Thr Thr Gly Leu Thr Thr Asp Asp Val Asn Asp
240                 245                 250                 255

ctg atg gac aag aca cga aac ctt atg ctc aag cac ttg aag gag atg        815
Leu Met Asp Lys Thr Arg Asn Leu Met Leu Lys His Leu Lys Glu Met
                260                 265                 270

gat tcc cag tat tcg tcg agc act gct gaa aat gga tcc acg cac atc        863
Asp Ser Gln Tyr Ser Ser Ser Thr Ala Glu Asn Gly Ser Thr His Ile
            275                 280                 285

gac gcc gat att gcc aag tct aca gcc acc agc att ggc aac act gac        911
Asp Ala Asp Ile Ala Lys Ser Thr Ala Thr Ser Ile Gly Asn Thr Asp
        290                 295                 300

gac gca att aca aaa cgt cgt acc cct aag gaa taa gcggccgc               955
Asp Ala Ile Thr Lys Arg Arg Thr Pro Lys Glu
    305                 310
```

<210> 36
<211> 314
<212> PRT
<213> Mortierella alpina

<400> 36

```
Met Ser Ile Gly Ser Ser Asn Pro Val Leu Leu Ala Ala Ile Pro Phe
1               5                   10                  15

Val Tyr Leu Phe Val Leu Pro Arg Val Leu Ala Phe Leu Pro Gln Lys
            20                  25                  30

Ala Gln Phe Leu Ala Lys Cys Ile Val Val Leu Ile Ala Thr Leu Ile
        35                  40                  45

Met Ser Val Ala Gly Cys Phe Ile Ser Ile Val Cys Ala Leu Leu Asp
    50                  55                  60

Lys Arg Tyr Val Ile Asn Tyr Val Val Ser Arg Leu Phe Ser Phe Leu
65                  70                  75                  80
```

```
Ala Ala Arg Pro Cys Gly Val Thr Tyr Lys Ile Val Gly Glu Glu His
                85                  90                  95

Leu Asp Lys Tyr Pro Ala Ile Val Val Cys Asn His Gln Ser Ser Met
            100                 105                 110

Asp Met Met Val Leu Gly Arg Val Phe Pro Lys His Cys Val Val Met
        115                 120                 125

Ala Lys Lys Glu Leu Leu Tyr Phe Pro Phe Leu Gly Met Phe Met Lys
    130                 135                 140

Leu Ser Asn Ala Ile Phe Ile Asp Arg Lys Asn His Lys Lys Ala Ile
145                 150                 155                 160

Glu Ser Thr Thr Gln Ala Val Ala Asp Met Lys Lys His Asn Ser Gly
                165                 170                 175

Ile Trp Ile Phe Pro Glu Gly Thr Arg Ser Arg Leu Asp Lys Ala Asp
            180                 185                 190

Leu Leu Pro Phe Lys Lys Gly Ala Phe His Leu Ala Ile Gln Ala Gln
        195                 200                 205

Leu Pro Ile Leu Pro Ile Ile Ser Gln Gly Tyr Ser His Ile Tyr Asp
    210                 215                 220

Ser Ser Lys Arg Tyr Phe Pro Gly Gly Glu Leu Glu Ile Arg Val Leu
225                 230                 235                 240

Glu Pro Ile Pro Thr Thr Gly Leu Thr Thr Asp Asp Val Asn Asp Leu
                245                 250                 255

Met Asp Lys Thr Arg Asn Leu Met Leu Lys His Leu Lys Glu Met Asp
            260                 265                 270

Ser Gln Tyr Ser Ser Ser Thr Ala Glu Asn Gly Ser Thr His Ile Asp
        275                 280                 285

Ala Asp Ile Ala Lys Ser Thr Ala Thr Ser Ile Gly Asn Thr Asp Asp
    290                 295                 300

Ala Ile Thr Lys Arg Arg Thr Pro Lys Glu
305                 310
```

<210> 37
<211> 1185

<212> DNA
<213> Yarrowia lipolytica

<220>
<221> CDS
<222> (1)..(1185)
<223> diacylglycerol cholinephosphotransferase (YlCPT1)

<300>
<302> HIGH EICOSAPENTAENOIC ACID PRODUCING STRAINS OF

<310> WO 2006/052870
<311> 2005-11-03
<312> 2006-05-18
<313> (1)..(1185)

<300>
<302> HIGH EICOSAPENTAENOIC ACID PRODUCING STRAINS OF

<310> US 2006-0115881-A1
<311> 2005-11-02
<312> 2006-06-01
<313> (1)..(1185)

<400> 37

```
atg ggc gta ttc att aaa cag gag cag ctt ccg gct ctc aag aag tac          48
Met Gly Val Phe Ile Lys Gln Glu Gln Leu Pro Ala Leu Lys Lys Tyr
1               5                   10                  15

aag tac tcc gcc gag gat cac tcg ttc atc tcc aac aac att ctg cgc          96
Lys Tyr Ser Ala Glu Asp His Ser Phe Ile Ser Asn Asn Ile Leu Arg
            20                  25                  30

ccc ttc tgg cga cag ttt gtc aaa atc ttc cct ctg tgg atg gcc ccc          144
Pro Phe Trp Arg Gln Phe Val Lys Ile Phe Pro Leu Trp Met Ala Pro
        35                  40                  45

aac atg gtg act ctg ttg ggc ttc ttc ttt gtc att gtg aac ttc atc          192
Asn Met Val Thr Leu Leu Gly Phe Phe Phe Val Ile Val Asn Phe Ile
    50                  55                  60

acc atg ctc att gtt gat ccc acc cac gac cgc gag cct ccc aga tgg          240
Thr Met Leu Ile Val Asp Pro Thr His Asp Arg Glu Pro Pro Arg Trp
65                  70                  75                  80

gtc tac ctc acc tac gct ctg ggt ctg ttc ctt tac cag aca ttt gat          288
Val Tyr Leu Thr Tyr Ala Leu Gly Leu Phe Leu Tyr Gln Thr Phe Asp
                85                  90                  95

gcc tgt gac gga tcc cat gcc cga cga act ggc cag agt gga ccc ctt          336
Ala Cys Asp Gly Ser His Ala Arg Arg Thr Gly Gln Ser Gly Pro Leu
            100                 105                 110

gga gag ctg ttt gac cac tgt gtc gac gcc atg aat acc tct ctg att          384
Gly Glu Leu Phe Asp His Cys Val Asp Ala Met Asn Thr Ser Leu Ile
        115                 120                 125

ctc acg gtg gtg gtg tcc acc acc cat atg gga tat aac atg aag ctg          432
Leu Thr Val Val Val Ser Thr Thr His Met Gly Tyr Asn Met Lys Leu
    130                 135                 140

ctg att gtg cag att gcc gct ctc gga aac ttc tac ctg tcg acc tgg          480
Leu Ile Val Gln Ile Ala Ala Leu Gly Asn Phe Tyr Leu Ser Thr Trp
145                 150                 155                 160

gag acc tac cat acc gga act ctg tac ctt tct ggc ttc tct ggt cct          528
Glu Thr Tyr His Thr Gly Thr Leu Tyr Leu Ser Gly Phe Ser Gly Pro
                165                 170                 175
```

```
gtt gaa ggt atc ttg att ctg gtg gct ctt ttc gtc ctc acc ttc ttc        576
Val Glu Gly Ile Leu Ile Leu Val Ala Leu Phe Val Leu Thr Phe Phe
            180                 185                 190

act ggt ccc aac gtg tac gct ctg acc gtc tac gag gct ctt ccc gaa        624
Thr Gly Pro Asn Val Tyr Ala Leu Thr Val Tyr Glu Ala Leu Pro Glu
        195                 200                 205

tcc atc act tcg ctg ctg cct gcc agc ttc ctg gac gtc acc atc acc        672
Ser Ile Thr Ser Leu Leu Pro Ala Ser Phe Leu Asp Val Thr Ile Thr
    210                 215                 220

cag atc tac att gga ttc gga gtg ctg ggc atg gtg ttc aac atc tac        720
Gln Ile Tyr Ile Gly Phe Gly Val Leu Gly Met Val Phe Asn Ile Tyr
225                 230                 235                 240

ggc gcc tgc gga aac gtg atc aag tac tac aac aac aag ggc aag agc        768
Gly Ala Cys Gly Asn Val Ile Lys Tyr Tyr Asn Asn Lys Gly Lys Ser
                245                 250                 255

gct ctc ccc gcc att ctc gga atc gcc ccc ttt ggc atc ttc tac gtc        816
Ala Leu Pro Ala Ile Leu Gly Ile Ala Pro Phe Gly Ile Phe Tyr Val
            260                 265                 270

ggc gtc ttt gcc tgg gcc cat gtt gct cct ctg ctt ctc tcc aag tac        864
Gly Val Phe Ala Trp Ala His Val Ala Pro Leu Leu Leu Ser Lys Tyr
        275                 280                 285

gcc atc gtc tat ctg ttt gcc att ggg gct gcc ttt gcc atg caa gtc        912
Ala Ile Val Tyr Leu Phe Ala Ile Gly Ala Ala Phe Ala Met Gln Val
    290                 295                 300

ggc cag atg att ctt gcc cat ctc gtg ctt gct ccc ttc ccc cac tgg        960
Gly Gln Met Ile Leu Ala His Leu Val Leu Ala Pro Phe Pro His Trp
305                 310                 315                 320

aac gtg ctg ctc ttc ttc ccc ttt gtg gga ctg gca gtg cac tac att       1008
Asn Val Leu Leu Phe Phe Pro Phe Val Gly Leu Ala Val His Tyr Ile
                325                 330                 335

gca ccc gtg ttt ggc tgg gac gcc gat atc gtg tcg gtt aac act ctc       1056
Ala Pro Val Phe Gly Trp Asp Ala Asp Ile Val Ser Val Asn Thr Leu
            340                 345                 350

ttc acc tgt ttt ggc gcc acc ctc tcc att tac gcc ttc ttt gtg ctt       1104
Phe Thr Cys Phe Gly Ala Thr Leu Ser Ile Tyr Ala Phe Phe Val Leu
        355                 360                 365

gag atc atc gac gag atc acc aac tac ctc gat atc tgg tgt ctg cga       1152
Glu Ile Ile Asp Glu Ile Thr Asn Tyr Leu Asp Ile Trp Cys Leu Arg
    370                 375                 380

atc aag tac cct cag gag aag aag act gag taa                           1185
Ile Lys Tyr Pro Gln Glu Lys Lys Thr Glu
385                 390
```

<210> 38
<211> 394
<212> PRT
<213> Yarrowia lipolytica

<400> 38

Met Gly Val Phe Ile Lys Gln Glu Gln Leu Pro Ala Leu Lys Lys Tyr
1 5 10 15

Lys Tyr Ser Ala Glu Asp His Ser Phe Ile Ser Asn Asn Ile Leu Arg
20 25 30

Pro Phe Trp Arg Gln Phe Val Lys Ile Phe Pro Leu Trp Met Ala Pro
35 40 45

Asn Met Val Thr Leu Leu Gly Phe Phe Phe Val Ile Val Asn Phe Ile
50 55 60

Thr Met Leu Ile Val Asp Pro Thr His Asp Arg Glu Pro Pro Arg Trp
65 70 75 80

Val Tyr Leu Thr Tyr Ala Leu Gly Leu Phe Leu Tyr Gln Thr Phe Asp
85 90 95

Ala Cys Asp Gly Ser His Ala Arg Arg Thr Gly Gln Ser Gly Pro Leu
100 105 110

Gly Glu Leu Phe Asp His Cys Val Asp Ala Met Asn Thr Ser Leu Ile
115 120 125

Leu Thr Val Val Val Ser Thr Thr His Met Gly Tyr Asn Met Lys Leu
130 135 140

Leu Ile Val Gln Ile Ala Ala Leu Gly Asn Phe Tyr Leu Ser Thr Trp
145 150 155 160

Glu Thr Tyr His Thr Gly Thr Leu Tyr Leu Ser Gly Phe Ser Gly Pro
165 170 175

Val Glu Gly Ile Leu Ile Leu Val Ala Leu Phe Val Leu Thr Phe Phe
180 185 190

Thr Gly Pro Asn Val Tyr Ala Leu Thr Val Tyr Glu Ala Leu Pro Glu
195 200 205

Ser Ile Thr Ser Leu Leu Pro Ala Ser Phe Leu Asp Val Thr Ile Thr
210 215 220

Gln Ile Tyr Ile Gly Phe Gly Val Leu Gly Met Val Phe Asn Ile Tyr
225 230 235 240

Gly Ala Cys Gly Asn Val Ile Lys Tyr Tyr Asn Asn Lys Gly Lys Ser
245 250 255

Ala Leu Pro Ala Ile Leu Gly Ile Ala Pro Phe Gly Ile Phe Tyr Val

```
                260                 265                 270

Gly Val Phe Ala Trp Ala His Val Ala Pro Leu Leu Leu Ser Lys Tyr
        275                 280                 285

Ala Ile Val Tyr Leu Phe Ala Ile Gly Ala Ala Phe Ala Met Gln Val
    290                 295                 300

Gly Gln Met Ile Leu Ala His Leu Val Leu Ala Pro Phe Pro His Trp
305                 310                 315                 320

Asn Val Leu Leu Phe Phe Pro Phe Val Gly Leu Ala Val His Tyr Ile
                325                 330                 335

Ala Pro Val Phe Gly Trp Asp Ala Asp Ile Val Ser Val Asn Thr Leu
            340                 345                 350

Phe Thr Cys Phe Gly Ala Thr Leu Ser Ile Tyr Ala Phe Phe Val Leu
        355                 360                 365

Glu Ile Ile Asp Glu Ile Thr Asn Tyr Leu Asp Ile Trp Cys Leu Arg
    370                 375                 380

Ile Lys Tyr Pro Gln Glu Lys Lys Thr Glu
385                 390
```

<210> 39
<211> 1515
<212> DNA
<213> Rhizobium leguminosarum bv. viciae 3841

<220>
<221> CDS
<222> (1)..(1515)
<223> GenBank Accession No. YP_766603

<400> 39

```
gtg agc aac cat ctt ttc gac gcc atg cgg gct gcc gcg ccc ggt gac        48
Val Ser Asn His Leu Phe Asp Ala Met Arg Ala Ala Ala Pro Gly Asp
1               5                   10                  15

gca ccg ttc atc cgg atc gat aac gcg cgc aca tgg acc tat gat gac        96
Ala Pro Phe Ile Arg Ile Asp Asn Ala Arg Thr Trp Thr Tyr Asp Asp
            20                  25                  30

gcg atc gct ctt tcc ggc cgt att gcc ggc gcg atg gac gcg ctc ggc       144
Ala Ile Ala Leu Ser Gly Arg Ile Ala Gly Ala Met Asp Ala Leu Gly
        35                  40                  45

att cgc ccc ggc gac cgg gtg gcg gtg cag gtc gag aaa agt gcc gag       192
Ile Arg Pro Gly Asp Arg Val Ala Val Gln Val Glu Lys Ser Ala Glu
    50                  55                  60

gcg ttg atc ctc tat ctc gcc tgt ctt cga acc ggc gcc gtc tac ctg       240
Ala Leu Ile Leu Tyr Leu Ala Cys Leu Arg Thr Gly Ala Val Tyr Leu
```

```
65                  70                  75                  80

ccg ctc aac acc gcc tat acg ctg gct gag ctc gat tac ttt atc ggc        288
Pro Leu Asn Thr Ala Tyr Thr Leu Ala Glu Leu Asp Tyr Phe Ile Gly
                85                  90                  95

gat gcg gag ccg cgt ttg gtg gtg gtt gcg ccg gcg gct cga ggc ggc        336
Asp Ala Glu Pro Arg Leu Val Val Val Ala Pro Ala Ala Arg Gly Gly
            100                 105                 110

gtg gag aca atc gcc aag cgc cac ggc gcg atc gtc gaa acg ctc gat        384
Val Glu Thr Ile Ala Lys Arg His Gly Ala Ile Val Glu Thr Leu Asp
        115                 120                 125

gct gat ggc cgc ggc tca ttg ctg gat ctc gca cgc gat gag ccg gcc        432
Ala Asp Gly Arg Gly Ser Leu Leu Asp Leu Ala Arg Asp Glu Pro Ala
    130                 135                 140

gac ttt gtc gat gcc tcg cgt tcc gcg gat gat ctg gcc gcg atc ctc        480
Asp Phe Val Asp Ala Ser Arg Ser Ala Asp Asp Leu Ala Ala Ile Leu
145                 150                 155                 160

tac acg tcg gga acg acc gga cgc tcc aag ggg gcg atg ctc acg cat        528
Tyr Thr Ser Gly Thr Thr Gly Arg Ser Lys Gly Ala Met Leu Thr His
                165                 170                 175

ggg aac ctg ctc tcg aac gcc ctg acc ttg cga gac tat tgg cgc gtc        576
Gly Asn Leu Leu Ser Asn Ala Leu Thr Leu Arg Asp Tyr Trp Arg Val
            180                 185                 190

acc gct gac gat cgg ctg atc cat gcc ttg ccg atc ttc cac acg cat        624
Thr Ala Asp Asp Arg Leu Ile His Ala Leu Pro Ile Phe His Thr His
        195                 200                 205

ggg ctg ttc gtc gcc acg aac gtc aca ctg ctt gcc ggc gcc tcg atg        672
Gly Leu Phe Val Ala Thr Asn Val Thr Leu Leu Ala Gly Ala Ser Met
    210                 215                 220

ttc ctg ctg tcg aaa ttc gac gcc gac gag gtc gtt tca ctg atg cca        720
Phe Leu Leu Ser Lys Phe Asp Ala Asp Glu Val Val Ser Leu Met Pro
225                 230                 235                 240

cag gca act atg ctg atg ggc gtg ccg acc ttc tac gtg cgc ctc ctg        768
Gln Ala Thr Met Leu Met Gly Val Pro Thr Phe Tyr Val Arg Leu Leu
                245                 250                 255

caa agc ccg cgc ctc gaa aaa ggg gcg gtc gcc agc atc cgc ctc ttc        816
Gln Ser Pro Arg Leu Glu Lys Gly Ala Val Ala Ser Ile Arg Leu Phe
            260                 265                 270

att tcc ggt tcg gct ccc ctg ctg gcc gaa acc cat gcc gag ttc cat        864
Ile Ser Gly Ser Ala Pro Leu Leu Ala Glu Thr His Ala Glu Phe His
        275                 280                 285

gcg cgt acc ggt cac gcc att ctc gag cgc tac ggc atg acg gaa acc        912
Ala Arg Thr Gly His Ala Ile Leu Glu Arg Tyr Gly Met Thr Glu Thr
    290                 295                 300

aat atg aac acg tcc aac ccc tat gag ggc aaa cgg att gcc gga acg        960
Asn Met Asn Thr Ser Asn Pro Tyr Glu Gly Lys Arg Ile Ala Gly Thr
305                 310                 315                 320

gtt ggt ttc ccg ctg cct gat gtg acg gtg cgc gtc acc gat ccc gcc       1008
Val Gly Phe Pro Leu Pro Asp Val Thr Val Arg Val Thr Asp Pro Ala
                325                 330                 335
```

```
acc ggg ctc gtg ctg cca cct gaa gag acg ggc atg atc gag atc aag        1056
Thr Gly Leu Val Leu Pro Pro Glu Glu Thr Gly Met Ile Glu Ile Lys
            340                 345                 350

gga ccg aac gtc ttc aag ggc tat tgg cgc atg ccg gaa aag acc gcg        1104
Gly Pro Asn Val Phe Lys Gly Tyr Trp Arg Met Pro Glu Lys Thr Ala
        355                 360                 365

gcc gaa ttc acc gcc gac ggt ttc ttc atc agc ggc gat ctc ggc aag        1152
Ala Glu Phe Thr Ala Asp Gly Phe Phe Ile Ser Gly Asp Leu Gly Lys
    370                 375                 380

atc gac cgg gaa ggt tat gtc cac atc gtc ggt cgc ggc aag gat ctg        1200
Ile Asp Arg Glu Gly Tyr Val His Ile Val Gly Arg Gly Lys Asp Leu
385                 390                 395                 400

gtg atc tcg ggt gga tac aac atc tat ccg aaa gag gtc gaa ggc gag        1248
Val Ile Ser Gly Gly Tyr Asn Ile Tyr Pro Lys Glu Val Glu Gly Glu
                405                 410                 415

atc gac cag atc gag ggt gtg gtt gag agc gct gtg atc ggt gtg ccg        1296
Ile Asp Gln Ile Glu Gly Val Val Glu Ser Ala Val Ile Gly Val Pro
            420                 425                 430

cat ccc gat ttc gga gaa ggc gta acg gct gtc gtc gta tgt aag ccc        1344
His Pro Asp Phe Gly Glu Gly Val Thr Ala Val Val Val Cys Lys Pro
        435                 440                 445

ggc gcc gtc ctc gat gaa aag acc atc gtc agc gcc ctc cag gac cgt        1392
Gly Ala Val Leu Asp Glu Lys Thr Ile Val Ser Ala Leu Gln Asp Arg
    450                 455                 460

ctc gcc cgc tac aaa caa ccc aag cgc atc atc ttc gcc gac gac ctg        1440
Leu Ala Arg Tyr Lys Gln Pro Lys Arg Ile Ile Phe Ala Asp Asp Leu
465                 470                 475                 480

ccg cgc aac act atg ggt aag gtt cag aag aat atc ctg cgg cag caa        1488
Pro Arg Asn Thr Met Gly Lys Val Gln Lys Asn Ile Leu Arg Gln Gln
                485                 490                 495

tac gcc gat ctt tat acc agg agg taa                                    1515
Tyr Ala Asp Leu Tyr Thr Arg Arg
            500
```

<210> 40
<211> 504
<212> PRT
<213> Rhizobium leguminosarum bv. viciae 3841

<400> 40

```
Val Ser Asn His Leu Phe Asp Ala Met Arg Ala Ala Ala Pro Gly Asp
1               5                   10                  15

Ala Pro Phe Ile Arg Ile Asp Asn Ala Arg Thr Trp Thr Tyr Asp Asp
            20                  25                  30

Ala Ile Ala Leu Ser Gly Arg Ile Ala Gly Ala Met Asp Ala Leu Gly
        35                  40                  45
```

```
Ile Arg Pro Gly Asp Arg Val Ala Val Gln Val Glu Lys Ser Ala Glu
    50                  55                  60

Ala Leu Ile Leu Tyr Leu Ala Cys Leu Arg Thr Gly Ala Val Tyr Leu
65                  70                  75                  80

Pro Leu Asn Thr Ala Tyr Thr Leu Ala Glu Leu Asp Tyr Phe Ile Gly
                85                  90                  95

Asp Ala Glu Pro Arg Leu Val Val Val Ala Pro Ala Ala Arg Gly Gly
            100                 105                 110

Val Glu Thr Ile Ala Lys Arg His Gly Ala Ile Val Glu Thr Leu Asp
        115                 120                 125

Ala Asp Gly Arg Gly Ser Leu Leu Asp Leu Ala Arg Asp Glu Pro Ala
    130                 135                 140

Asp Phe Val Asp Ala Ser Arg Ser Ala Asp Asp Leu Ala Ala Ile Leu
145                 150                 155                 160

Tyr Thr Ser Gly Thr Thr Gly Arg Ser Lys Gly Ala Met Leu Thr His
                165                 170                 175

Gly Asn Leu Leu Ser Asn Ala Leu Thr Leu Arg Asp Tyr Trp Arg Val
            180                 185                 190

Thr Ala Asp Asp Arg Leu Ile His Ala Leu Pro Ile Phe His Thr His
        195                 200                 205

Gly Leu Phe Val Ala Thr Asn Val Thr Leu Leu Ala Gly Ala Ser Met
    210                 215                 220

Phe Leu Leu Ser Lys Phe Asp Ala Asp Glu Val Val Ser Leu Met Pro
225                 230                 235                 240

Gln Ala Thr Met Leu Met Gly Val Pro Thr Phe Tyr Val Arg Leu Leu
                245                 250                 255

Gln Ser Pro Arg Leu Glu Lys Gly Ala Val Ala Ser Ile Arg Leu Phe
            260                 265                 270

Ile Ser Gly Ser Ala Pro Leu Leu Ala Glu Thr His Ala Glu Phe His
        275                 280                 285

Ala Arg Thr Gly His Ala Ile Leu Glu Arg Tyr Gly Met Thr Glu Thr
    290                 295                 300

Asn Met Asn Thr Ser Asn Pro Tyr Glu Gly Lys Arg Ile Ala Gly Thr
```

305 310 315 320

Val Gly Phe Pro Leu Pro Asp Val Thr Val Arg Val Thr Asp Pro Ala
325 330 335

Thr Gly Leu Val Leu Pro Pro Glu Glu Thr Gly Met Ile Glu Ile Lys
340 345 350

Gly Pro Asn Val Phe Lys Gly Tyr Trp Arg Met Pro Glu Lys Thr Ala
355 360 365

Ala Glu Phe Thr Ala Asp Gly Phe Phe Ile Ser Gly Asp Leu Gly Lys
370 375 380

Ile Asp Arg Glu Gly Tyr Val His Ile Val Gly Arg Gly Lys Asp Leu
385 390 395 400

Val Ile Ser Gly Gly Tyr Asn Ile Tyr Pro Lys Glu Val Glu Gly Glu
405 410 415

Ile Asp Gln Ile Glu Gly Val Val Glu Ser Ala Val Ile Gly Val Pro
420 425 430

His Pro Asp Phe Gly Glu Gly Val Thr Ala Val Val Val Cys Lys Pro
435 440 445

Gly Ala Val Leu Asp Glu Lys Thr Ile Val Ser Ala Leu Gln Asp Arg
450 455 460

Leu Ala Arg Tyr Lys Gln Pro Lys Arg Ile Ile Phe Ala Asp Asp Leu
465 470 475 480

Pro Arg Asn Thr Met Gly Lys Val Gln Lys Asn Ile Leu Arg Gln Gln
485 490 495

Tyr Ala Asp Leu Tyr Thr Arg Arg
500

<210> 41
<211> 1518
<212> DNA
<213> Rhizobium leguminosarum bv. viciae 3841

<220>
<221> CDS
<222> (1)..(1518)
<223> synthetic malonyl-CoA synthetase (codon-optimized for Yarrowia lipolytica)

<400> 41

atg gtc tcc aac cac ctg ttc gac gcc atg cga gct gcc gct ccc gga 48

```
Met Val Ser Asn His Leu Phe Asp Ala Met Arg Ala Ala Ala Pro Gly
1               5                   10                  15

gac gca cct ttc att cga atc gac aac gct cgg acc tgg act tac gat        96
Asp Ala Pro Phe Ile Arg Ile Asp Asn Ala Arg Thr Trp Thr Tyr Asp
            20                  25                  30

gac gcc att gct ctt tcc ggt cga atc gct gga gct atg gac gca ctc       144
Asp Ala Ile Ala Leu Ser Gly Arg Ile Ala Gly Ala Met Asp Ala Leu
        35                  40                  45

ggc att cga ccc gga gac aga gtt gcc gtg cag gtc gag aag tct gcc       192
Gly Ile Arg Pro Gly Asp Arg Val Ala Val Gln Val Glu Lys Ser Ala
    50                  55                  60

gag gcg ttg att ctc tac ctg gcc tgt ctt cga acc gga gct gtc tac       240
Glu Ala Leu Ile Leu Tyr Leu Ala Cys Leu Arg Thr Gly Ala Val Tyr
65                  70                  75                  80

ctg cct ctc aac act gcc tac acc ctg gcc gag ctc gac tac ttc atc       288
Leu Pro Leu Asn Thr Ala Tyr Thr Leu Ala Glu Leu Asp Tyr Phe Ile
                85                  90                  95

ggc gat gcc gaa ccg cgt ctg gtg gtc gtt gct ccc gca gct cga ggt       336
Gly Asp Ala Glu Pro Arg Leu Val Val Val Ala Pro Ala Ala Arg Gly
            100                 105                 110

ggc gtg gag aca att gcc aag cga cac ggt gct atc gtc gaa acc ctc       384
Gly Val Glu Thr Ile Ala Lys Arg His Gly Ala Ile Val Glu Thr Leu
        115                 120                 125

gac gcc gat gga cga ggc tcc ttg ctg gac ctt gct aga gat gag cct       432
Asp Ala Asp Gly Arg Gly Ser Leu Leu Asp Leu Ala Arg Asp Glu Pro
    130                 135                 140

gcc gac ttt gtc gat gct tcg cga tct gcc gac gat ctg gct gct att       480
Ala Asp Phe Val Asp Ala Ser Arg Ser Ala Asp Asp Leu Ala Ala Ile
145                 150                 155                 160

ctc tac act tcc ggt aca acc gga cga tcg aag ggt gcc atg ctt act       528
Leu Tyr Thr Ser Gly Thr Thr Gly Arg Ser Lys Gly Ala Met Leu Thr
                165                 170                 175

cat ggc aat ctg ctc tcc aac gct ctc acc ttg cga gac tat tgg aga       576
His Gly Asn Leu Leu Ser Asn Ala Leu Thr Leu Arg Asp Tyr Trp Arg
            180                 185                 190

gtt acc gca gac gat cga ctc atc cat gcc ttg cca atc ttt cac act       624
Val Thr Ala Asp Asp Arg Leu Ile His Ala Leu Pro Ile Phe His Thr
        195                 200                 205

cat ggt ctg ttc gtt gct acg aac gtc aca ctg ctt gca gga gcc tcg       672
His Gly Leu Phe Val Ala Thr Asn Val Thr Leu Leu Ala Gly Ala Ser
    210                 215                 220

atg ttt ctg ctc tcc aag ttc gat gcc gac gag gtc gtt tct ctc atg       720
Met Phe Leu Leu Ser Lys Phe Asp Ala Asp Glu Val Val Ser Leu Met
225                 230                 235                 240

cca cag gcc acc atg ctt atg ggc gtg ccc aca ttc tac gtt cga ttg       768
Pro Gln Ala Thr Met Leu Met Gly Val Pro Thr Phe Tyr Val Arg Leu
                245                 250                 255

ctg cag agt cct cga ctc gag aag ggt gct gtg gcc agc atc aga ctg       816
Leu Gln Ser Pro Arg Leu Glu Lys Gly Ala Val Ala Ser Ile Arg Leu
```

```
            260                 265                 270

ttc att tct gga tca gct ccc ttg ctt gcc gaa acc cac gcc gag ttt         864
Phe Ile Ser Gly Ser Ala Pro Leu Leu Ala Glu Thr His Ala Glu Phe
        275                 280                 285

cat gct cgt act ggt cac gcc att ctc gag cga tac ggc atg acg gaa         912
His Ala Arg Thr Gly His Ala Ile Leu Glu Arg Tyr Gly Met Thr Glu
    290                 295                 300

acc aac atg aat act tcc aac ccc tac gag ggc aag cgt att gcc gga         960
Thr Asn Met Asn Thr Ser Asn Pro Tyr Glu Gly Lys Arg Ile Ala Gly
305                 310                 315                 320

acc gtt ggt ttt cct ctg ccc gac gtc act gtg cga gtc acc gat ccc        1008
Thr Val Gly Phe Pro Leu Pro Asp Val Thr Val Arg Val Thr Asp Pro
                325                 330                 335

gcc acc ggt ctc gtt ctt cca cct gaa gag act ggc atg atc gag atc        1056
Ala Thr Gly Leu Val Leu Pro Pro Glu Glu Thr Gly Met Ile Glu Ile
            340                 345                 350

aag gga ccc aac gtc ttc aag ggc tat tgg cga atg ccc gaa aag acc        1104
Lys Gly Pro Asn Val Phe Lys Gly Tyr Trp Arg Met Pro Glu Lys Thr
        355                 360                 365

gct gcc gag ttt acc gca gac ggt ttc ttt atc tct gga gat ctc ggc        1152
Ala Ala Glu Phe Thr Ala Asp Gly Phe Phe Ile Ser Gly Asp Leu Gly
    370                 375                 380

aag atc gac cga gaa ggt tac gtt cac att gtg gga cga ggc aag gac        1200
Lys Ile Asp Arg Glu Gly Tyr Val His Ile Val Gly Arg Gly Lys Asp
385                 390                 395                 400

ctg gtc att tcc ggt ggc tac aac atc tat ccc aaa gag gtc gaa ggc        1248
Leu Val Ile Ser Gly Gly Tyr Asn Ile Tyr Pro Lys Glu Val Glu Gly
                405                 410                 415

gag atc gac cag atc gag ggt gtg gtc gag tct gct gtc att ggt gtt        1296
Glu Ile Asp Gln Ile Glu Gly Val Val Glu Ser Ala Val Ile Gly Val
            420                 425                 430

cct cat ccc gat ttc gga gaa ggt gtc acc gct gtt gtc gtg tgc aaa        1344
Pro His Pro Asp Phe Gly Glu Gly Val Thr Ala Val Val Val Cys Lys
        435                 440                 445

cct ggt gcc gtt ctc gac gaa aag acc atc gtg tct gct ctg cag gac        1392
Pro Gly Ala Val Leu Asp Glu Lys Thr Ile Val Ser Ala Leu Gln Asp
    450                 455                 460

cgt ctt gcc cga tac aag caa ccc aag cgg att atc ttt gcc gac gat        1440
Arg Leu Ala Arg Tyr Lys Gln Pro Lys Arg Ile Ile Phe Ala Asp Asp
465                 470                 475                 480

ctg cct cga aac act atg gga aag gtt cag aag aac att ctt cga cag        1488
Leu Pro Arg Asn Thr Met Gly Lys Val Gln Lys Asn Ile Leu Arg Gln
                485                 490                 495

caa tac gcc gat ctc tac acc aga cga taa                                1518
Gln Tyr Ala Asp Leu Tyr Thr Arg Arg
            500                 505
```

<210> 42
<211> 505
<212> PRT
<213> Rhizobium leguminosarum bv. viciae 3841

<400> 42

```
Met Val Ser Asn His Leu Phe Asp Ala Met Arg Ala Ala Ala Pro Gly
1               5                   10                  15

Asp Ala Pro Phe Ile Arg Ile Asp Asn Ala Arg Thr Trp Thr Tyr Asp
            20                  25                  30

Asp Ala Ile Ala Leu Ser Gly Arg Ile Ala Gly Ala Met Asp Ala Leu
        35                  40                  45

Gly Ile Arg Pro Gly Asp Arg Val Ala Val Gln Val Glu Lys Ser Ala
    50                  55                  60

Glu Ala Leu Ile Leu Tyr Leu Ala Cys Leu Arg Thr Gly Ala Val Tyr
65                  70                  75                  80

Leu Pro Leu Asn Thr Ala Tyr Thr Leu Ala Glu Leu Asp Tyr Phe Ile
                85                  90                  95

Gly Asp Ala Glu Pro Arg Leu Val Val Val Ala Pro Ala Ala Arg Gly
            100                 105                 110

Gly Val Glu Thr Ile Ala Lys Arg His Gly Ala Ile Val Glu Thr Leu
        115                 120                 125

Asp Ala Asp Gly Arg Gly Ser Leu Leu Asp Leu Ala Arg Asp Glu Pro
    130                 135                 140

Ala Asp Phe Val Asp Ala Ser Arg Ser Ala Asp Asp Leu Ala Ala Ile
145                 150                 155                 160

Leu Tyr Thr Ser Gly Thr Thr Gly Arg Ser Lys Gly Ala Met Leu Thr
                165                 170                 175

His Gly Asn Leu Leu Ser Asn Ala Leu Thr Leu Arg Asp Tyr Trp Arg
            180                 185                 190

Val Thr Ala Asp Asp Arg Leu Ile His Ala Leu Pro Ile Phe His Thr
        195                 200                 205

His Gly Leu Phe Val Ala Thr Asn Val Thr Leu Leu Ala Gly Ala Ser
    210                 215                 220

Met Phe Leu Leu Ser Lys Phe Asp Ala Asp Glu Val Val Ser Leu Met
225                 230                 235                 240
```

Pro Gln Ala Thr Met Leu Met Gly Val Pro Thr Phe Tyr Val Arg Leu
245 250 255

Leu Gln Ser Pro Arg Leu Glu Lys Gly Ala Val Ala Ser Ile Arg Leu
260 265 270

Phe Ile Ser Gly Ser Ala Pro Leu Leu Ala Glu Thr His Ala Glu Phe
275 280 285

His Ala Arg Thr Gly His Ala Ile Leu Glu Arg Tyr Gly Met Thr Glu
290 295 300

Thr Asn Met Asn Thr Ser Asn Pro Tyr Glu Gly Lys Arg Ile Ala Gly
305 310 315 320

Thr Val Gly Phe Pro Leu Pro Asp Val Thr Val Arg Val Thr Asp Pro
325 330 335

Ala Thr Gly Leu Val Leu Pro Pro Glu Glu Thr Gly Met Ile Glu Ile
340 345 350

Lys Gly Pro Asn Val Phe Lys Gly Tyr Trp Arg Met Pro Glu Lys Thr
355 360 365

Ala Ala Glu Phe Thr Ala Asp Gly Phe Phe Ile Ser Gly Asp Leu Gly
370 375 380

Lys Ile Asp Arg Glu Gly Tyr Val His Ile Val Gly Arg Gly Lys Asp
385 390 395 400

Leu Val Ile Ser Gly Gly Tyr Asn Ile Tyr Pro Lys Glu Val Glu Gly
405 410 415

Glu Ile Asp Gln Ile Glu Gly Val Val Glu Ser Ala Val Ile Gly Val
420 425 430

Pro His Pro Asp Phe Gly Glu Gly Val Thr Ala Val Val Val Cys Lys
435 440 445

Pro Gly Ala Val Leu Asp Glu Lys Thr Ile Val Ser Ala Leu Gln Asp
450 455 460

Arg Leu Ala Arg Tyr Lys Gln Pro Lys Arg Ile Ile Phe Ala Asp Asp
465 470 475 480

Leu Pro Arg Asn Thr Met Gly Lys Val Gln Lys Asn Ile Leu Arg Gln
485 490 495

Gln Tyr Ala Asp Leu Tyr Thr Arg Arg

500 505

<210> 43
<211> 777
<212> DNA
<213> Euglena gracilis

<220>
<221> CDS
<222> (1)..(777)
<223> delta-9 elongase

<300>
<302> DELTA-9 ELONGASES AND THEIR USE IN MAKING POLYUNSATURATED FATTY ACIDS

<310> WO 2007/061742
<311> 2006-11-16
<312> 2007-05-31
<313> (1)..(777)

<300>
<302> DELTA-9 ELONGASES AND THEIR USE IN MAKING POLYUNSATURATED FATTY ACIDS

<310> US 2007-0117190-A1
<311> 2006-11-16
<312> 2007-05-24
<313> (1)..(777)

<400> 43

```
atg gag gtg gtg aat gaa ata gtc tca att ggg cag gaa gtt tta ccc      48
Met Glu Val Val Asn Glu Ile Val Ser Ile Gly Gln Glu Val Leu Pro
1               5                   10                  15

aaa gtt gat tat gcc caa ctc tgg agt gat gcc agt cac tgt gag gtg      96
Lys Val Asp Tyr Ala Gln Leu Trp Ser Asp Ala Ser His Cys Glu Val
            20                  25                  30

ctt tac ttg tcc atc gca ttt gtc atc ttg aag ttc act ctt ggc ccc     144
Leu Tyr Leu Ser Ile Ala Phe Val Ile Leu Lys Phe Thr Leu Gly Pro
        35                  40                  45

ctt ggt cca aaa ggt cag tct cgt atg aag ttt gtt ttc acc aat tac     192
Leu Gly Pro Lys Gly Gln Ser Arg Met Lys Phe Val Phe Thr Asn Tyr
    50                  55                  60

aac ctt ctc atg tcc att tat tcg ttg gga tca ttc ctc tca atg gca     240
Asn Leu Leu Met Ser Ile Tyr Ser Leu Gly Ser Phe Leu Ser Met Ala
65                  70                  75                  80

tat gcc atg tac acc atc ggt gtt atg tct gac aac tgc gag aag gct     288
Tyr Ala Met Tyr Thr Ile Gly Val Met Ser Asp Asn Cys Glu Lys Ala
                85                  90                  95

ttt gac aac aac gtc ttc agg atc acc acg cag ttg ttc tat ttg agc     336
Phe Asp Asn Asn Val Phe Arg Ile Thr Thr Gln Leu Phe Tyr Leu Ser
            100                 105                 110

aag ttc ctg gag tat att gac tcc ttc tat ttg cca ctg atg ggc aag     384
Lys Phe Leu Glu Tyr Ile Asp Ser Phe Tyr Leu Pro Leu Met Gly Lys
        115                 120                 125

cct ctg acc tgg ttg caa ttc ttc cat cat ttg ggg gca ccg atg gat     432
Pro Leu Thr Trp Leu Gln Phe Phe His His Leu Gly Ala Pro Met Asp
```

```
    130                 135                 140

atg tgg ctg ttc tat aat tac cga aat gaa gct gtt tgg att ttt gtg        480
Met Trp Leu Phe Tyr Asn Tyr Arg Asn Glu Ala Val Trp Ile Phe Val
145                 150                 155                 160

ctg ttg aat ggt ttc atc cac tgg atc atg tac ggt tat tat tgg acc        528
Leu Leu Asn Gly Phe Ile His Trp Ile Met Tyr Gly Tyr Tyr Trp Thr
                165                 170                 175

aga ttg atc aag ctg aag ttc ccc atg cca aaa tcc ctg att aca tca        576
Arg Leu Ile Lys Leu Lys Phe Pro Met Pro Lys Ser Leu Ile Thr Ser
            180                 185                 190

atg cag atc att caa ttc aat gtt ggt ttc tac att gtc tgg aag tac        624
Met Gln Ile Ile Gln Phe Asn Val Gly Phe Tyr Ile Val Trp Lys Tyr
        195                 200                 205

agg aac att ccc tgt tat cgc caa gat ggg atg agg atg ttt ggc tgg        672
Arg Asn Ile Pro Cys Tyr Arg Gln Asp Gly Met Arg Met Phe Gly Trp
    210                 215                 220

ttc ttc aat tac ttt tat gtt ggc aca gtc ttg tgt ttg ttc ttg aat        720
Phe Phe Asn Tyr Phe Tyr Val Gly Thr Val Leu Cys Leu Phe Leu Asn
225                 230                 235                 240

ttc tat gtg caa acg tat atc gtc agg aag cac aag gga gcc aaa aag        768
Phe Tyr Val Gln Thr Tyr Ile Val Arg Lys His Lys Gly Ala Lys Lys
                245                 250                 255

att cag tga                                                            777
Ile Gln
```

<210> 44
<211> 258
<212> PRT
<213> Euglena gracilis

<400> 44

```
Met Glu Val Val Asn Glu Ile Val Ser Ile Gly Gln Glu Val Leu Pro
1               5                   10                  15

Lys Val Asp Tyr Ala Gln Leu Trp Ser Asp Ala Ser His Cys Glu Val
            20                  25                  30

Leu Tyr Leu Ser Ile Ala Phe Val Ile Leu Lys Phe Thr Leu Gly Pro
        35                  40                  45

Leu Gly Pro Lys Gly Gln Ser Arg Met Lys Phe Val Phe Thr Asn Tyr
    50                  55                  60

Asn Leu Leu Met Ser Ile Tyr Ser Leu Gly Ser Phe Leu Ser Met Ala
65                  70                  75                  80

Tyr Ala Met Tyr Thr Ile Gly Val Met Ser Asp Asn Cys Glu Lys Ala
                85                  90                  95
```

```
Phe Asp Asn Asn Val Phe Arg Ile Thr Thr Gln Leu Phe Tyr Leu Ser
            100                 105                 110

Lys Phe Leu Glu Tyr Ile Asp Ser Phe Tyr Leu Pro Leu Met Gly Lys
        115                 120                 125

Pro Leu Thr Trp Leu Gln Phe Phe His His Leu Gly Ala Pro Met Asp
    130                 135                 140

Met Trp Leu Phe Tyr Asn Tyr Arg Asn Glu Ala Val Trp Ile Phe Val
145                 150                 155                 160

Leu Leu Asn Gly Phe Ile His Trp Ile Met Tyr Gly Tyr Tyr Trp Thr
                165                 170                 175

Arg Leu Ile Lys Leu Lys Phe Pro Met Pro Lys Ser Leu Ile Thr Ser
            180                 185                 190

Met Gln Ile Ile Gln Phe Asn Val Gly Phe Tyr Ile Val Trp Lys Tyr
        195                 200                 205

Arg Asn Ile Pro Cys Tyr Arg Gln Asp Gly Met Arg Met Phe Gly Trp
    210                 215                 220

Phe Phe Asn Tyr Phe Tyr Val Gly Thr Val Leu Cys Leu Phe Leu Asn
225                 230                 235                 240

Phe Tyr Val Gln Thr Tyr Ile Val Arg Lys His Lys Gly Ala Lys Lys
                245                 250                 255

Ile Gln
```

<210> 45
<211> 777
<212> DNA
<213> Euglena gracilis

<220>
<221> CDS
<222> (1)..(777)
<223> synthetic delta-9 elongase (codon-optimized for Yarrowia lipolytica)

<300>
<302> DELTA-9 ELONGASES AND THEIR USE IN MAKING POLYUNSATURATED FATTY ACIDS

<310> WO 2007/061742
<311> 2006-11-16
<312> 2007-05-31
<313> (1)..(777)

<300>
<302> DELTA-9 ELONGASES AND THEIR USE IN MAKING POLYUNSATURATED FATTY ACIDS

<310> US-2007-0117190-A1
<311> 2006-11-16
<312> 2007-05-24
<313> (1)..(777)

<400> 45

```
atg gag gtc gtg aac gaa atc gtc tcc att ggc cag gag gtt ctt ccc      48
Met Glu Val Val Asn Glu Ile Val Ser Ile Gly Gln Glu Val Leu Pro
1               5                   10                  15

aag gtc gac tat gct cag ctc tgg tct gat gcc tcg cac tgc gag gtg      96
Lys Val Asp Tyr Ala Gln Leu Trp Ser Asp Ala Ser His Cys Glu Val
            20                  25                  30

ctg tac ctc tcc atc gcc ttc gtc atc ctg aag ttc acc ctt ggt cct     144
Leu Tyr Leu Ser Ile Ala Phe Val Ile Leu Lys Phe Thr Leu Gly Pro
        35                  40                  45

ctc gga ccc aag ggt cag tct cga atg aag ttt gtg ttc acc aac tac     192
Leu Gly Pro Lys Gly Gln Ser Arg Met Lys Phe Val Phe Thr Asn Tyr
    50                  55                  60

aac ctg ctc atg tcc atc tac tcg ctg ggc tcc ttc ctc tct atg gcc     240
Asn Leu Leu Met Ser Ile Tyr Ser Leu Gly Ser Phe Leu Ser Met Ala
65                  70                  75                  80

tac gcc atg tac acc att ggt gtc atg tcc gac aac tgc gag aag gct     288
Tyr Ala Met Tyr Thr Ile Gly Val Met Ser Asp Asn Cys Glu Lys Ala
                85                  90                  95

ttc gac aac aat gtc ttc cga atc acc act cag ctg ttc tac ctc agc     336
Phe Asp Asn Asn Val Phe Arg Ile Thr Thr Gln Leu Phe Tyr Leu Ser
            100                 105                 110

aag ttc ctc gag tac att gac tcc ttc tat ctg ccc ctc atg ggc aag     384
Lys Phe Leu Glu Tyr Ile Asp Ser Phe Tyr Leu Pro Leu Met Gly Lys
        115                 120                 125

cct ctg acc tgg ttg cag ttc ttt cac cat ctc gga gct cct atg gac     432
Pro Leu Thr Trp Leu Gln Phe Phe His His Leu Gly Ala Pro Met Asp
    130                 135                 140

atg tgg ctg ttc tac aac tac cga aac gaa gcc gtt tgg atc ttt gtg     480
Met Trp Leu Phe Tyr Asn Tyr Arg Asn Glu Ala Val Trp Ile Phe Val
145                 150                 155                 160

ctg ctc aac ggc ttc att cac tgg atc atg tac ggc tac tat tgg acc     528
Leu Leu Asn Gly Phe Ile His Trp Ile Met Tyr Gly Tyr Tyr Trp Thr
                165                 170                 175

cga ctg atc aag ctc aag ttc cct atg ccc aag tcc ctg att act tct     576
Arg Leu Ile Lys Leu Lys Phe Pro Met Pro Lys Ser Leu Ile Thr Ser
            180                 185                 190

atg cag atc att cag ttc aac gtt ggc ttc tac atc gtc tgg aag tac     624
Met Gln Ile Ile Gln Phe Asn Val Gly Phe Tyr Ile Val Trp Lys Tyr
        195                 200                 205

cgg aac att ccc tgc tac cga caa gat gga atg aga atg ttt ggc tgg     672
Arg Asn Ile Pro Cys Tyr Arg Gln Asp Gly Met Arg Met Phe Gly Trp
    210                 215                 220
```

```
ttt ttc aac tac ttc tac gtt ggt act gtc ctg tgt ctg ttc ctc aac        720
Phe Phe Asn Tyr Phe Tyr Val Gly Thr Val Leu Cys Leu Phe Leu Asn
225                 230                 235                 240

ttc tac gtg cag acc tac atc gtc cga aag cac aag gga gcc aaa aag        768
Phe Tyr Val Gln Thr Tyr Ile Val Arg Lys His Lys Gly Ala Lys Lys
                245                 250                 255

att cag tga                                                            777
Ile Gln
```

<210> 46
<211> 258
<212> PRT
<213> Euglena gracilis

<400> 46

```
Met Glu Val Val Asn Glu Ile Val Ser Ile Gly Gln Glu Val Leu Pro
1               5                   10                  15

Lys Val Asp Tyr Ala Gln Leu Trp Ser Asp Ala Ser His Cys Glu Val
            20                  25                  30

Leu Tyr Leu Ser Ile Ala Phe Val Ile Leu Lys Phe Thr Leu Gly Pro
        35                  40                  45

Leu Gly Pro Lys Gly Gln Ser Arg Met Lys Phe Val Phe Thr Asn Tyr
    50                  55                  60

Asn Leu Leu Met Ser Ile Tyr Ser Leu Gly Ser Phe Leu Ser Met Ala
65                  70                  75                  80

Tyr Ala Met Tyr Thr Ile Gly Val Met Ser Asp Asn Cys Glu Lys Ala
                85                  90                  95

Phe Asp Asn Asn Val Phe Arg Ile Thr Thr Gln Leu Phe Tyr Leu Ser
            100                 105                 110

Lys Phe Leu Glu Tyr Ile Asp Ser Phe Tyr Leu Pro Leu Met Gly Lys
        115                 120                 125

Pro Leu Thr Trp Leu Gln Phe Phe His His Leu Gly Ala Pro Met Asp
    130                 135                 140

Met Trp Leu Phe Tyr Asn Tyr Arg Asn Glu Ala Val Trp Ile Phe Val
145                 150                 155                 160

Leu Leu Asn Gly Phe Ile His Trp Ile Met Tyr Gly Tyr Tyr Trp Thr
                165                 170                 175

Arg Leu Ile Lys Leu Lys Phe Pro Met Pro Lys Ser Leu Ile Thr Ser
```

180 185 190

Met Gln Ile Ile Gln Phe Asn Val Gly Phe Tyr Ile Val Trp Lys Tyr
195 200 205

Arg Asn Ile Pro Cys Tyr Arg Gln Asp Gly Met Arg Met Phe Gly Trp
210 215 220

Phe Phe Asn Tyr Phe Tyr Val Gly Thr Val Leu Cys Leu Phe Leu Asn
225 230 235 240

Phe Tyr Val Gln Thr Tyr Ile Val Arg Lys His Lys Gly Ala Lys Lys
245 250 255

Ile Gln

<210> 47
<211> 792
<212> DNA
<213> Eutreptiella sp. CCMP389

<220>
<221> CDS
<222> (1)..(792)
<223> delta-9 elongase

<300>
<302> DELTA-9 ELONGASES AND THEIR USE IN MAKING POLYUNSATURATED FATTY ACIDS

<310> WO 2007/061742
<311> 2006-11-16
<312> 2007-05-31
<313> (1)..(792)

<300>
<302> DELTA-9 ELONGASES AND THEIR USE IN MAKING POLYUNSATURATED FATTY ACIDS

<310> US 2007-0117190-A1
<311> 2006-11-16
<312> 2007-05-24
<313> (1)..(792)

<400> 47

```
atg gct gcg gtg ata gag gtc gcc aac gag ttt gta gcc atc acg gca        48
Met Ala Ala Val Ile Glu Val Ala Asn Glu Phe Val Ala Ile Thr Ala
1               5                   10                  15

gaa acg ctc ccc aaa gtt gac tat caa cga cta tgg cga gac att tac        96
Glu Thr Leu Pro Lys Val Asp Tyr Gln Arg Leu Trp Arg Asp Ile Tyr
            20                  25                  30

agt tgt gag cta ctg tat ttc tcc att gcc ttc gtg atc ttg aag ttt       144
Ser Cys Glu Leu Leu Tyr Phe Ser Ile Ala Phe Val Ile Leu Lys Phe
        35                  40                  45

acg ttg ggc gag ttg agc gac agc gga aaa aag att ttg aga gtg ttg       192
Thr Leu Gly Glu Leu Ser Asp Ser Gly Lys Lys Ile Leu Arg Val Leu
```

```
    50                  55                  60

ttc aag tgg tac aat ctc ttc atg tcc gtg ttc tcc ttg gtg tct ttc         240
Phe Lys Trp Tyr Asn Leu Phe Met Ser Val Phe Ser Leu Val Ser Phe
65                  70                  75                  80

ctt tgc atg ggc tat gcc att tat acc gtg ggc cta tac tct aac gaa         288
Leu Cys Met Gly Tyr Ala Ile Tyr Thr Val Gly Leu Tyr Ser Asn Glu
                85                  90                  95

tgc gac agg gct ttc gac aac tcg ttg ttc cgc ttt gca aca aag gtg         336
Cys Asp Arg Ala Phe Asp Asn Ser Leu Phe Arg Phe Ala Thr Lys Val
            100                 105                 110

ttc tac tac agt aag ttt ttg gag tac atc gac tct ttt tat ctt ccg         384
Phe Tyr Tyr Ser Lys Phe Leu Glu Tyr Ile Asp Ser Phe Tyr Leu Pro
        115                 120                 125

ctc atg gcc aag ccg ctg tct ttc ctg caa ttc ttc cat cac ttg gga         432
Leu Met Ala Lys Pro Leu Ser Phe Leu Gln Phe Phe His His Leu Gly
    130                 135                 140

gcc ccc atg gac atg tgg ctc ttt gtc caa tat tct ggg gaa tct att         480
Ala Pro Met Asp Met Trp Leu Phe Val Gln Tyr Ser Gly Glu Ser Ile
145                 150                 155                 160

tgg atc ttt gtg ttt ttg aat ggg ttc att cac ttt gtt atg tac ggg         528
Trp Ile Phe Val Phe Leu Asn Gly Phe Ile His Phe Val Met Tyr Gly
                165                 170                 175

tac tac tgg act cgg ctg atg aag ttc aat ttc cca atg ccc aag cag         576
Tyr Tyr Trp Thr Arg Leu Met Lys Phe Asn Phe Pro Met Pro Lys Gln
            180                 185                 190

ttg att acc gcg atg cag atc acg cag ttc aac gtt ggt ttc tac ctc         624
Leu Ile Thr Ala Met Gln Ile Thr Gln Phe Asn Val Gly Phe Tyr Leu
        195                 200                 205

gtg tgg tgg tac aaa gat att ccc tgc tac cga aag gat ccc atg cga         672
Val Trp Trp Tyr Lys Asp Ile Pro Cys Tyr Arg Lys Asp Pro Met Arg
    210                 215                 220

atg ttg gcc tgg atc ttc aat tac tgg tat gtt ggg act gtc ttg ctg         720
Met Leu Ala Trp Ile Phe Asn Tyr Trp Tyr Val Gly Thr Val Leu Leu
225                 230                 235                 240

ctg ttc att aat ttc ttc gtc aaa tcc tat gtg ttc cca aag ccg aag         768
Leu Phe Ile Asn Phe Phe Val Lys Ser Tyr Val Phe Pro Lys Pro Lys
                245                 250                 255

act gca gat aaa aag gtc caa tag                                         792
Thr Ala Asp Lys Lys Val Gln
            260
```

<210> 48
<211> 263
<212> PRT
<213> Eutreptiella sp. CCMP389

<400> 48

Met Ala Ala Val Ile Glu Val Ala Asn Glu Phe Val Ala Ile Thr Ala
1               5                   10                  15

```
Glu Thr Leu Pro Lys Val Asp Tyr Gln Arg Leu Trp Arg Asp Ile Tyr
            20                  25                  30

Ser Cys Glu Leu Leu Tyr Phe Ser Ile Ala Phe Val Ile Leu Lys Phe
        35                  40                  45

Thr Leu Gly Glu Leu Ser Asp Ser Gly Lys Lys Ile Leu Arg Val Leu
    50                  55                  60

Phe Lys Trp Tyr Asn Leu Phe Met Ser Val Phe Ser Leu Val Ser Phe
65                  70                  75                  80

Leu Cys Met Gly Tyr Ala Ile Tyr Thr Val Gly Leu Tyr Ser Asn Glu
                85                  90                  95

Cys Asp Arg Ala Phe Asp Asn Ser Leu Phe Arg Phe Ala Thr Lys Val
            100                 105                 110

Phe Tyr Tyr Ser Lys Phe Leu Glu Tyr Ile Asp Ser Phe Tyr Leu Pro
        115                 120                 125

Leu Met Ala Lys Pro Leu Ser Phe Leu Gln Phe Phe His His Leu Gly
    130                 135                 140

Ala Pro Met Asp Met Trp Leu Phe Val Gln Tyr Ser Gly Glu Ser Ile
145                 150                 155                 160

Trp Ile Phe Val Phe Leu Asn Gly Phe Ile His Phe Val Met Tyr Gly
                165                 170                 175

Tyr Tyr Trp Thr Arg Leu Met Lys Phe Asn Phe Pro Met Pro Lys Gln
            180                 185                 190

Leu Ile Thr Ala Met Gln Ile Thr Gln Phe Asn Val Gly Phe Tyr Leu
        195                 200                 205

Val Trp Trp Tyr Lys Asp Ile Pro Cys Tyr Arg Lys Asp Pro Met Arg
    210                 215                 220

Met Leu Ala Trp Ile Phe Asn Tyr Trp Tyr Val Gly Thr Val Leu Leu
225                 230                 235                 240

Leu Phe Ile Asn Phe Phe Val Lys Ser Tyr Val Phe Pro Lys Pro Lys
                245                 250                 255

Thr Ala Asp Lys Lys Val Gln
            260
```

<210> 49
<211> 792
<212> DNA
<213> Eutreptiella sp. CCMP389

<220>
<221> CDS
<222> (1)..(792)
<223> synthetic delta-9 elongase (codon-optimized for Yarrowia lipolytica)

<300>
<302> DELTA-9 ELONGASES AND THEIR USE IN MAKING POLYUNSATURATED FATTY ACIDS

<310> WO 2007/061742
<311> 2006-11-16
<312> 2007-05-31
<313> (1)..(792)

<300>
<302> DELTA-9 ELONGASES AND THEIR USE IN MAKING POLYUNSATURATED FATTY ACIDS

<310> US 2007-0117190-A1
<311> 2006-11-16
<312> 2007-05-24
<313> (1)..(792)

<400> 49

```
atg gct gcc gtc atc gag gtg gcc aac gag ttc gtc gct atc act gcc        48
Met Ala Ala Val Ile Glu Val Ala Asn Glu Phe Val Ala Ile Thr Ala
1               5                   10                  15

gag acc ctt ccc aag gtg gac tat cag cga ctc tgg cga gac atc tac        96
Glu Thr Leu Pro Lys Val Asp Tyr Gln Arg Leu Trp Arg Asp Ile Tyr
            20                  25                  30

tcc tgc gag ctc ctg tac ttc tcc att gct ttc gtc atc ctc aag ttt       144
Ser Cys Glu Leu Leu Tyr Phe Ser Ile Ala Phe Val Ile Leu Lys Phe
        35                  40                  45

acc ctt ggc gag ctc tcg gat tct ggc aaa aag att ctg cga gtg ctg       192
Thr Leu Gly Glu Leu Ser Asp Ser Gly Lys Lys Ile Leu Arg Val Leu
    50                  55                  60

ttc aag tgg tac aac ctc ttc atg tcc gtc ttt tcg ctg gtg tcc ttc       240
Phe Lys Trp Tyr Asn Leu Phe Met Ser Val Phe Ser Leu Val Ser Phe
65                  70                  75                  80

ctc tgt atg ggt tac gcc atc tac acc gtt gga ctg tac tcc aac gaa       288
Leu Cys Met Gly Tyr Ala Ile Tyr Thr Val Gly Leu Tyr Ser Asn Glu
                85                  90                  95

tgc gac aga gct ttc gac aac agc ttg ttc cga ttt gcc acc aag gtc       336
Cys Asp Arg Ala Phe Asp Asn Ser Leu Phe Arg Phe Ala Thr Lys Val
            100                 105                 110

ttc tac tat tcc aag ttt ctg gag tac atc gac tct ttc tac ctt ccc       384
Phe Tyr Tyr Ser Lys Phe Leu Glu Tyr Ile Asp Ser Phe Tyr Leu Pro
        115                 120                 125

ctc atg gcc aag cct ctg tcc ttt ctg cag ttc ttt cat cac ttg gga       432
Leu Met Ala Lys Pro Leu Ser Phe Leu Gln Phe Phe His His Leu Gly
    130                 135                 140
```

```
gct cct atg gac atg tgg ctc ttc gtg cag tac tct ggc gaa tcc att         480
Ala Pro Met Asp Met Trp Leu Phe Val Gln Tyr Ser Gly Glu Ser Ile
145                 150                 155                 160

tgg atc ttt gtg ttc ctg aac gga ttc att cac ttt gtc atg tac ggc         528
Trp Ile Phe Val Phe Leu Asn Gly Phe Ile His Phe Val Met Tyr Gly
                165                 170                 175

tac tat tgg aca cgg ctg atg aag ttc aac ttt ccc atg ccc aag cag         576
Tyr Tyr Trp Thr Arg Leu Met Lys Phe Asn Phe Pro Met Pro Lys Gln
            180                 185                 190

ctc att acc gca atg cag atc acc cag ttc aac gtt ggc ttc tac ctc         624
Leu Ile Thr Ala Met Gln Ile Thr Gln Phe Asn Val Gly Phe Tyr Leu
        195                 200                 205

gtg tgg tgg tac aag gac att ccc tgt tac cga aag gat ccc atg cga         672
Val Trp Trp Tyr Lys Asp Ile Pro Cys Tyr Arg Lys Asp Pro Met Arg
    210                 215                 220

atg ctg gcc tgg atc ttc aac tac tgg tac gtc ggt acc gtt ctt ctg         720
Met Leu Ala Trp Ile Phe Asn Tyr Trp Tyr Val Gly Thr Val Leu Leu
225                 230                 235                 240

ctc ttc atc aac ttc ttt gtc aag tcc tac gtg ttt ccc aag cct aag         768
Leu Phe Ile Asn Phe Phe Val Lys Ser Tyr Val Phe Pro Lys Pro Lys
                245                 250                 255

act gcc gac aaa aag gtc cag tag                                         792
Thr Ala Asp Lys Lys Val Gln
            260
```

<210> 50
<211> 263
<212> PRT
<213> Eutreptiella sp. CCMP389

<400> 50

Met Ala Ala Val Ile Glu Val Ala Asn Glu Phe Val Ala Ile Thr Ala
1               5                   10                  15

Glu Thr Leu Pro Lys Val Asp Tyr Gln Arg Leu Trp Arg Asp Ile Tyr
            20                  25                  30

Ser Cys Glu Leu Leu Tyr Phe Ser Ile Ala Phe Val Ile Leu Lys Phe
        35                  40                  45

Thr Leu Gly Glu Leu Ser Asp Ser Gly Lys Lys Ile Leu Arg Val Leu
    50                  55                  60

Phe Lys Trp Tyr Asn Leu Phe Met Ser Val Phe Ser Leu Val Ser Phe
65                  70                  75                  80

Leu Cys Met Gly Tyr Ala Ile Tyr Thr Val Gly Leu Tyr Ser Asn Glu
                85                  90                  95

Cys Asp Arg Ala Phe Asp Asn Ser Leu Phe Arg Phe Ala Thr Lys Val

```
                100                 105                 110

Phe Tyr Tyr Ser Lys Phe Leu Glu Tyr Ile Asp Ser Phe Tyr Leu Pro
            115                 120                 125

Leu Met Ala Lys Pro Leu Ser Phe Leu Gln Phe Phe His His Leu Gly
    130                 135                 140

Ala Pro Met Asp Met Trp Leu Phe Val Gln Tyr Ser Gly Glu Ser Ile
145                 150                 155                 160

Trp Ile Phe Val Phe Leu Asn Gly Phe Ile His Phe Val Met Tyr Gly
                165                 170                 175

Tyr Tyr Trp Thr Arg Leu Met Lys Phe Asn Phe Pro Met Pro Lys Gln
            180                 185                 190

Leu Ile Thr Ala Met Gln Ile Thr Gln Phe Asn Val Gly Phe Tyr Leu
        195                 200                 205

Val Trp Trp Tyr Lys Asp Ile Pro Cys Tyr Arg Lys Asp Pro Met Arg
    210                 215                 220

Met Leu Ala Trp Ile Phe Asn Tyr Trp Tyr Val Gly Thr Val Leu Leu
225                 230                 235                 240

Leu Phe Ile Asn Phe Phe Val Lys Ser Tyr Val Phe Pro Lys Pro Lys
                245                 250                 255

Thr Ala Asp Lys Lys Val Gln
            260
```

<210> 51
<211> 774
<212> DNA
<213> Euglena anabaena UTEX 373

<220>
<221> CDS
<222> (1)..(774)
<223> delta-9 elongase

<300>
<302> DELTA-9 ELONGASES AND THEIR USE IN MAKING POLYUNSATURATED FATTY ACIDS

<310> U.S. 2008-0254522-A1
<311> 2008-04-15
<312> 2008-10-16
<313> (1)..(774)

<300>
<302> DELTA-9 ELONGASES AND THEIR USE IN MAKING POLYUNSATURATED FATTY ACIDS

<310> WO 2008/128241
<311> 2008-04-16
<312> 2008-10-23
<313> (1)..(774)

<400> 51

```
atg gaa gca gcc aaa gaa ttg gtt tcc atc gtc caa gag gag ctc ccc       48
Met Glu Ala Ala Lys Glu Leu Val Ser Ile Val Gln Glu Glu Leu Pro
1               5                   10                  15

aag gtg gac tat gcc cag ctt tgg cag gat gcc agc agc tgt gag gtc       96
Lys Val Asp Tyr Ala Gln Leu Trp Gln Asp Ala Ser Ser Cys Glu Val
            20                  25                  30

ctt tac ctc tcg gtg gca ttc gtg gcg atc aag ttc atg ctg cgc cca      144
Leu Tyr Leu Ser Val Ala Phe Val Ala Ile Lys Phe Met Leu Arg Pro
        35                  40                  45

ctg gac ctg aag cgc cag gcc acc ttg aag aag ctg ttc aca gca tac      192
Leu Asp Leu Lys Arg Gln Ala Thr Leu Lys Lys Leu Phe Thr Ala Tyr
    50                  55                  60

aac ttc ctc atg tcg atc tat tcc ttt ggc tcc ttc ctg gcc atg gcc      240
Asn Phe Leu Met Ser Ile Tyr Ser Phe Gly Ser Phe Leu Ala Met Ala
65                  70                  75                  80

tat gcc cta tca gta act ggc atc ctc tcc ggc gac tgt gag acg gcg      288
Tyr Ala Leu Ser Val Thr Gly Ile Leu Ser Gly Asp Cys Glu Thr Ala
                85                  90                  95

ttc aac aac gat gtg ttc agg atc aca act cag ctg ttc tac ctc agc      336
Phe Asn Asn Asp Val Phe Arg Ile Thr Thr Gln Leu Phe Tyr Leu Ser
            100                 105                 110

aag ttc gta gag tac atc gac tcc ttc tac ctt ccc ctt atg gac aag      384
Lys Phe Val Glu Tyr Ile Asp Ser Phe Tyr Leu Pro Leu Met Asp Lys
        115                 120                 125

cca ctg tcg ttc ctt cag ttc ttc cat cat ttg ggg gcc ccc att gac      432
Pro Leu Ser Phe Leu Gln Phe Phe His His Leu Gly Ala Pro Ile Asp
    130                 135                 140

atg tgg cta ttc tac aaa tac cgc aac gaa gga gtc tgg atc ttt gtc      480
Met Trp Leu Phe Tyr Lys Tyr Arg Asn Glu Gly Val Trp Ile Phe Val
145                 150                 155                 160

ctg ttg aat ggg ttc att cac tgg atc atg tac ggt tac tat tgg acg      528
Leu Leu Asn Gly Phe Ile His Trp Ile Met Tyr Gly Tyr Tyr Trp Thr
                165                 170                 175

cgg ctc atc aag ctg aac ttc ccc atg ccc aag aac ctg atc acc tcc      576
Arg Leu Ile Lys Leu Asn Phe Pro Met Pro Lys Asn Leu Ile Thr Ser
            180                 185                 190

atg cag atc atc cag ttc aat gtc ggg ttc tac atc gtc tgg aag tac      624
Met Gln Ile Ile Gln Phe Asn Val Gly Phe Tyr Ile Val Trp Lys Tyr
        195                 200                 205

cgc aat gtg cca tgc tac cgc cag gat ggg atg cgc atg ttt gcc tgg      672
Arg Asn Val Pro Cys Tyr Arg Gln Asp Gly Met Arg Met Phe Ala Trp
    210                 215                 220

atc ttc aac tac tgg tat gtc ggg acg gtc ttg ctg ctg ttc ctc aac      720
Ile Phe Asn Tyr Trp Tyr Val Gly Thr Val Leu Leu Leu Phe Leu Asn
225                 230                 235                 240
```

```
ttt tac gtg cag acg tac atc cgg aag ccg agg aag aac cga ggg aag        768
Phe Tyr Val Gln Thr Tyr Ile Arg Lys Pro Arg Lys Asn Arg Gly Lys
                245                 250                 255

aag gag                                                                774
Lys Glu
```

<210> 52
<211> 258
<212> PRT
<213> Euglena anabaena UTEX 373

<400> 52

```
Met Glu Ala Ala Lys Glu Leu Val Ser Ile Val Gln Glu Glu Leu Pro
1               5                   10                  15

Lys Val Asp Tyr Ala Gln Leu Trp Gln Asp Ala Ser Ser Cys Glu Val
            20                  25                  30

Leu Tyr Leu Ser Val Ala Phe Val Ala Ile Lys Phe Met Leu Arg Pro
        35                  40                  45

Leu Asp Leu Lys Arg Gln Ala Thr Leu Lys Lys Leu Phe Thr Ala Tyr
    50                  55                  60

Asn Phe Leu Met Ser Ile Tyr Ser Phe Gly Ser Phe Leu Ala Met Ala
65                  70                  75                  80

Tyr Ala Leu Ser Val Thr Gly Ile Leu Ser Gly Asp Cys Glu Thr Ala
                85                  90                  95

Phe Asn Asn Asp Val Phe Arg Ile Thr Thr Gln Leu Phe Tyr Leu Ser
            100                 105                 110

Lys Phe Val Glu Tyr Ile Asp Ser Phe Tyr Leu Pro Leu Met Asp Lys
        115                 120                 125

Pro Leu Ser Phe Leu Gln Phe Phe His His Leu Gly Ala Pro Ile Asp
    130                 135                 140

Met Trp Leu Phe Tyr Lys Tyr Arg Asn Glu Gly Val Trp Ile Phe Val
145                 150                 155                 160

Leu Leu Asn Gly Phe Ile His Trp Ile Met Tyr Gly Tyr Tyr Trp Thr
                165                 170                 175

Arg Leu Ile Lys Leu Asn Phe Pro Met Pro Lys Asn Leu Ile Thr Ser
            180                 185                 190
```

```
Met Gln Ile Ile Gln Phe Asn Val Gly Phe Tyr Ile Val Trp Lys Tyr
        195                 200                 205

Arg Asn Val Pro Cys Tyr Arg Gln Asp Gly Met Arg Met Phe Ala Trp
    210                 215                 220

Ile Phe Asn Tyr Trp Tyr Val Gly Thr Val Leu Leu Leu Phe Leu Asn
225                 230                 235                 240

Phe Tyr Val Gln Thr Tyr Ile Arg Lys Pro Arg Lys Asn Arg Gly Lys
                245                 250                 255

Lys Glu
```

<210> 53
<211> 774
<212> DNA
<213> Euglena anabaena UTEX 373

<220>
<221> CDS
<222> (1)..(774)
<223> synthetic delta-9 elongase (codon-optimized for Yarrowia lipolytica)

<300>
<302> DELTA-9 ELONGASES AND THEIR USE IN MAKING POLYUNSATURATED FATTY ACIDS

<310> U.S. 2008-0254522-A1
<311> 2008-04-15
<312> 2008-10-16
<313> (1)..(774)

<300>
<302> DELTA-9 ELONGASES AND THEIR USE IN MAKING POLYUNSATURATED FATTY ACIDS

<310> WO 2008/128241
<311> 2008-04-16
<312> 2008-10-23
<313> (1)..(774)

<400> 53

```
atg gag gct gcc aag gag ctg gtc tcc atc gtc cag gag gaa ctt ccc         48
Met Glu Ala Ala Lys Glu Leu Val Ser Ile Val Gln Glu Glu Leu Pro
1               5                   10                  15

aag gtg gac tac gcc cag ctc tgg cag gac gcc tcc tct tgc gag gtt         96
Lys Val Asp Tyr Ala Gln Leu Trp Gln Asp Ala Ser Ser Cys Glu Val
            20                  25                  30

ctg tac ctc tcg gtc gct ttc gtg gcc atc aag ttc atg ctt cga cct        144
Leu Tyr Leu Ser Val Ala Phe Val Ala Ile Lys Phe Met Leu Arg Pro
        35                  40                  45

ctg gac ctc aag cga caa gcc acc ctc aaa aag ctg ttc acc gca tac        192
Leu Asp Leu Lys Arg Gln Ala Thr Leu Lys Lys Leu Phe Thr Ala Tyr
    50                  55                  60
```

```
aac ttt ctc atg tcc atc tac tcg ttc ggc tcc ttc ctg gcg atg gcc         240
Asn Phe Leu Met Ser Ile Tyr Ser Phe Gly Ser Phe Leu Ala Met Ala
65                  70                  75                  80

tac gct ctc tct gtc act ggt att ctt tcc ggc gat tgt gag act gcc         288
Tyr Ala Leu Ser Val Thr Gly Ile Leu Ser Gly Asp Cys Glu Thr Ala
                85                  90                  95

ttc aac aat gac gtg ttc cga atc acc act cag ctg ttc tac ctc agc         336
Phe Asn Asn Asp Val Phe Arg Ile Thr Thr Gln Leu Phe Tyr Leu Ser
            100                 105                 110

aag ttc gtc gag tac atc gac tcc ttc tac ctt ccc ctc atg gac aag         384
Lys Phe Val Glu Tyr Ile Asp Ser Phe Tyr Leu Pro Leu Met Asp Lys
        115                 120                 125

ccc ttg tcg ttt ctg cag ttc ttt cac cat ctc gga gct ccc atc gac         432
Pro Leu Ser Phe Leu Gln Phe Phe His His Leu Gly Ala Pro Ile Asp
    130                 135                 140

atg tgg ctg ttc tac aag tat cga aac gaa ggc gtc tgg atc ttt gtt         480
Met Trp Leu Phe Tyr Lys Tyr Arg Asn Glu Gly Val Trp Ile Phe Val
145                 150                 155                 160

ctg ctc aac ggc ttc att cac tgg atc atg tac ggt tac tat tgg acg         528
Leu Leu Asn Gly Phe Ile His Trp Ile Met Tyr Gly Tyr Tyr Trp Thr
                165                 170                 175

cga ctc atc aag ctg aac ttc cct atg ccc aag aac ctc att acc tcc         576
Arg Leu Ile Lys Leu Asn Phe Pro Met Pro Lys Asn Leu Ile Thr Ser
            180                 185                 190

atg caa att atc cag ttc aac gtc gga ttc tac atc gtc tgg aag tac         624
Met Gln Ile Ile Gln Phe Asn Val Gly Phe Tyr Ile Val Trp Lys Tyr
        195                 200                 205

cga aac gtg ccc tgc tac cgg cag gac ggt atg cga atg ttt gcc tgg         672
Arg Asn Val Pro Cys Tyr Arg Gln Asp Gly Met Arg Met Phe Ala Trp
    210                 215                 220

atc ttc aac tac tgg tat gtc ggc acg gtg ctg ctt ctg ttc ctc aac         720
Ile Phe Asn Tyr Trp Tyr Val Gly Thr Val Leu Leu Leu Phe Leu Asn
225                 230                 235                 240

ttc tac gtc cag acc tac att cgg aag cct cga aag aac cga ggc aaa         768
Phe Tyr Val Gln Thr Tyr Ile Arg Lys Pro Arg Lys Asn Arg Gly Lys
                245                 250                 255

aag gag                                                                 774
Lys Glu
```

<210> 54
<211> 258
<212> PRT
<213> Euglena anabaena UTEX 373

<400> 54

Met Glu Ala Ala Lys Glu Leu Val Ser Ile Val Gln Glu Glu Leu Pro
1               5                   10                  15

Lys Val Asp Tyr Ala Gln Leu Trp Gln Asp Ala Ser Ser Cys Glu Val

```
            20                  25                  30

Leu Tyr Leu Ser Val Ala Phe Val Ala Ile Lys Phe Met Leu Arg Pro
        35                  40                  45

Leu Asp Leu Lys Arg Gln Ala Thr Leu Lys Lys Leu Phe Thr Ala Tyr
    50                  55                  60

Asn Phe Leu Met Ser Ile Tyr Ser Phe Gly Ser Phe Leu Ala Met Ala
65                  70                  75                  80

Tyr Ala Leu Ser Val Thr Gly Ile Leu Ser Gly Asp Cys Glu Thr Ala
                85                  90                  95

Phe Asn Asn Asp Val Phe Arg Ile Thr Thr Gln Leu Phe Tyr Leu Ser
            100                 105                 110

Lys Phe Val Glu Tyr Ile Asp Ser Phe Tyr Leu Pro Leu Met Asp Lys
        115                 120                 125

Pro Leu Ser Phe Leu Gln Phe Phe His His Leu Gly Ala Pro Ile Asp
    130                 135                 140

Met Trp Leu Phe Tyr Lys Tyr Arg Asn Glu Gly Val Trp Ile Phe Val
145                 150                 155                 160

Leu Leu Asn Gly Phe Ile His Trp Ile Met Tyr Gly Tyr Tyr Trp Thr
                165                 170                 175

Arg Leu Ile Lys Leu Asn Phe Pro Met Pro Lys Asn Leu Ile Thr Ser
            180                 185                 190

Met Gln Ile Ile Gln Phe Asn Val Gly Phe Tyr Ile Val Trp Lys Tyr
        195                 200                 205

Arg Asn Val Pro Cys Tyr Arg Gln Asp Gly Met Arg Met Phe Ala Trp
    210                 215                 220

Ile Phe Asn Tyr Trp Tyr Val Gly Thr Val Leu Leu Leu Phe Leu Asn
225                 230                 235                 240

Phe Tyr Val Gln Thr Tyr Ile Arg Lys Pro Arg Lys Asn Arg Gly Lys
                245                 250                 255

Lys Glu
```

<210> 55
<211> 1271
<212> DNA
<213> Euglena gracilis

<220>
<221> CDS
<222> (4)..(1269)
<223> delta-8 desaturase ("Eg5" or "EgD8")

<300>
<302> DELTA-8 DESATURASE AND ITS USE IN MAKING POLYUNSATURATED FATTY ACIDS

<310> WO 2006/012325 and WO 2006/012326
<311> 2005-06-24
<312> 2006-02-02
<313> (1)..(1271)

<300>
<302> DELTA-8 DESATURASE AND ITS USE IN MAKING POLYUNSATURATED FATTY ACIDS

<310> U.S. 7,256,033
<311> 2005-06-24
<312> 2007-08-14
<313> (1)..(1271)

<400> 55

```
gaa atg aag tca aag cgc caa gcg ctt ccc ctt aca att gat gga aca       48
    Met Lys Ser Lys Arg Gln Ala Leu Pro Leu Thr Ile Asp Gly Thr
    1               5                   10                  15

aca tat gat gtg tct gcc tgg gtc aat ttc cac cct ggt ggt gcg gaa       96
Thr Tyr Asp Val Ser Ala Trp Val Asn Phe His Pro Gly Gly Ala Glu
                20                  25                  30

att ata gag aat tac caa gga agg gat gcc act gat gcc ttc atg gtt      144
Ile Ile Glu Asn Tyr Gln Gly Arg Asp Ala Thr Asp Ala Phe Met Val
            35                  40                  45

atg cac tct caa gaa gcc ttc gac aag ctc aag cgc atg ccc aaa atc      192
Met His Ser Gln Glu Ala Phe Asp Lys Leu Lys Arg Met Pro Lys Ile
        50                  55                  60

aat ccc agt tct gag ttg cca ccc cag gct gca gtg aat gaa gct caa      240
Asn Pro Ser Ser Glu Leu Pro Pro Gln Ala Ala Val Asn Glu Ala Gln
    65                  70                  75

gag gat ttc cgg aag ctc cga gaa gag ttg atc gca act ggc atg ttt      288
Glu Asp Phe Arg Lys Leu Arg Glu Glu Leu Ile Ala Thr Gly Met Phe
80                  85                  90                  95

gat gcc tcc ccc ctc tgg tac tca tac aaa atc agc acc aca ctg ggc      336
Asp Ala Ser Pro Leu Trp Tyr Ser Tyr Lys Ile Ser Thr Thr Leu Gly
                100                 105                 110

ctt gga gtg ctg ggt tat ttc ctg atg gtt cag tat cag atg tat ttc      384
Leu Gly Val Leu Gly Tyr Phe Leu Met Val Gln Tyr Gln Met Tyr Phe
            115                 120                 125

att ggg gca gtg ttg ctt ggg atg cac tat caa cag atg ggc tgg ctt      432
Ile Gly Ala Val Leu Leu Gly Met His Tyr Gln Gln Met Gly Trp Leu
        130                 135                 140

tct cat gac att tgc cac cac cag act ttc aag aac cgg aac tgg aac      480
Ser His Asp Ile Cys His His Gln Thr Phe Lys Asn Arg Asn Trp Asn
    145                 150                 155
```

```
aac ctc gtg gga ctg gta ttt ggc aat ggt ctg caa ggt ttt tcc gtg        528
Asn Leu Val Gly Leu Val Phe Gly Asn Gly Leu Gln Gly Phe Ser Val
160                 165                 170                 175

aca tgg tgg aag gac aga cac aat gca cat cat tcg gca acc aat gtt        576
Thr Trp Trp Lys Asp Arg His Asn Ala His His Ser Ala Thr Asn Val
                180                 185                 190

caa ggg cac gac cct gat att gac aac ctc ccc ctc tta gcc tgg tct        624
Gln Gly His Asp Pro Asp Ile Asp Asn Leu Pro Leu Leu Ala Trp Ser
            195                 200                 205

gag gat gac gtc aca cgg gcg tca ccg att tcc cgc aag ctc att cag        672
Glu Asp Asp Val Thr Arg Ala Ser Pro Ile Ser Arg Lys Leu Ile Gln
        210                 215                 220

ttc cag cag tac tat ttc ttg gtc atc tgt atc ttg ttg cgg ttc att        720
Phe Gln Gln Tyr Tyr Phe Leu Val Ile Cys Ile Leu Leu Arg Phe Ile
    225                 230                 235

tgg tgt ttc cag agc gtg ttg acc gtg cgc agt ttg aag gac aga gat        768
Trp Cys Phe Gln Ser Val Leu Thr Val Arg Ser Leu Lys Asp Arg Asp
240                 245                 250                 255

aac caa ttc tat cgc tct cag tat aag aag gag gcc att ggc ctc gcc        816
Asn Gln Phe Tyr Arg Ser Gln Tyr Lys Lys Glu Ala Ile Gly Leu Ala
                260                 265                 270

ctg cac tgg acc ttg aag acc ctg ttc cac tta ttc ttt atg ccc agc        864
Leu His Trp Thr Leu Lys Thr Leu Phe His Leu Phe Phe Met Pro Ser
            275                 280                 285

atc ctc aca tcg ctg ttg gtg ttt ttc gtt tcg gag ctg gtt ggc ggc        912
Ile Leu Thr Ser Leu Leu Val Phe Phe Val Ser Glu Leu Val Gly Gly
        290                 295                 300

ttc ggc att gcg atc gtg gtg ttc atg aac cac tac cca ctg gag aag        960
Phe Gly Ile Ala Ile Val Val Phe Met Asn His Tyr Pro Leu Glu Lys
    305                 310                 315

atc ggg gac tca gtc tgg gat ggc cat gga ttc tcg gtt ggc cag atc       1008
Ile Gly Asp Ser Val Trp Asp Gly His Gly Phe Ser Val Gly Gln Ile
320                 325                 330                 335

cat gag acc atg aac att cgg cga ggg att atc aca gat tgg ttt ttc       1056
His Glu Thr Met Asn Ile Arg Arg Gly Ile Ile Thr Asp Trp Phe Phe
                340                 345                 350

gga ggc ttg aat tac cag att gag cac cat ttg tgg ccg acc ctc cct       1104
Gly Gly Leu Asn Tyr Gln Ile Glu His His Leu Trp Pro Thr Leu Pro
            355                 360                 365

cgc cac aac ctg aca gcg gtt agc tac cag gtg gaa cag ctg tgc cag       1152
Arg His Asn Leu Thr Ala Val Ser Tyr Gln Val Glu Gln Leu Cys Gln
        370                 375                 380

aag cac aac ctg ccg tat cgg aac ccg ctg ccc cat gaa ggg ttg gtc       1200
Lys His Asn Leu Pro Tyr Arg Asn Pro Leu Pro His Glu Gly Leu Val
    385                 390                 395

atc ctg ctg cgc tat ctg gcg gtg ttc gcc cgg atg gcg gag aag caa       1248
Ile Leu Leu Arg Tyr Leu Ala Val Phe Ala Arg Met Ala Glu Lys Gln
400                 405                 410                 415
```

```
ccc gcg ggg aag gct cta taa gg                                       1271
Pro Ala Gly Lys Ala Leu
                    420
```

<210> 56
<211> 421
<212> PRT
<213> Euglena gracilis

<400> 56

```
Met Lys Ser Lys Arg Gln Ala Leu Pro Leu Thr Ile Asp Gly Thr Thr
1               5                   10                  15

Tyr Asp Val Ser Ala Trp Val Asn Phe His Pro Gly Gly Ala Glu Ile
            20                  25                  30

Ile Glu Asn Tyr Gln Gly Arg Asp Ala Thr Asp Ala Phe Met Val Met
        35                  40                  45

His Ser Gln Glu Ala Phe Asp Lys Leu Lys Arg Met Pro Lys Ile Asn
    50                  55                  60

Pro Ser Ser Glu Leu Pro Pro Gln Ala Ala Val Asn Glu Ala Gln Glu
65                  70                  75                  80

Asp Phe Arg Lys Leu Arg Glu Glu Leu Ile Ala Thr Gly Met Phe Asp
                85                  90                  95

Ala Ser Pro Leu Trp Tyr Ser Tyr Lys Ile Ser Thr Thr Leu Gly Leu
            100                 105                 110

Gly Val Leu Gly Tyr Phe Leu Met Val Gln Tyr Gln Met Tyr Phe Ile
        115                 120                 125

Gly Ala Val Leu Leu Gly Met His Tyr Gln Gln Met Gly Trp Leu Ser
    130                 135                 140

His Asp Ile Cys His His Gln Thr Phe Lys Asn Arg Asn Trp Asn Asn
145                 150                 155                 160

Leu Val Gly Leu Val Phe Gly Asn Gly Leu Gln Gly Phe Ser Val Thr
                165                 170                 175

Trp Trp Lys Asp Arg His Asn Ala His His Ser Ala Thr Asn Val Gln
            180                 185                 190

Gly His Asp Pro Asp Ile Asp Asn Leu Pro Leu Leu Ala Trp Ser Glu
        195                 200                 205

Asp Asp Val Thr Arg Ala Ser Pro Ile Ser Arg Lys Leu Ile Gln Phe
```

```
    210                 215                 220

Gln Gln Tyr Tyr Phe Leu Val Ile Cys Ile Leu Leu Arg Phe Ile Trp
225                 230                 235                 240

Cys Phe Gln Ser Val Leu Thr Val Arg Ser Leu Lys Asp Arg Asp Asn
                245                 250                 255

Gln Phe Tyr Arg Ser Gln Tyr Lys Lys Glu Ala Ile Gly Leu Ala Leu
            260                 265                 270

His Trp Thr Leu Lys Thr Leu Phe His Leu Phe Phe Met Pro Ser Ile
        275                 280                 285

Leu Thr Ser Leu Leu Val Phe Phe Val Ser Glu Leu Val Gly Gly Phe
    290                 295                 300

Gly Ile Ala Ile Val Val Phe Met Asn His Tyr Pro Leu Glu Lys Ile
305                 310                 315                 320

Gly Asp Ser Val Trp Asp Gly His Gly Phe Ser Val Gly Gln Ile His
                325                 330                 335

Glu Thr Met Asn Ile Arg Arg Gly Ile Ile Thr Asp Trp Phe Phe Gly
            340                 345                 350

Gly Leu Asn Tyr Gln Ile Glu His His Leu Trp Pro Thr Leu Pro Arg
        355                 360                 365

His Asn Leu Thr Ala Val Ser Tyr Gln Val Glu Gln Leu Cys Gln Lys
    370                 375                 380

His Asn Leu Pro Tyr Arg Asn Pro Leu Pro His Glu Gly Leu Val Ile
385                 390                 395                 400

Leu Leu Arg Tyr Leu Ala Val Phe Ala Arg Met Ala Glu Lys Gln Pro
                405                 410                 415

Ala Gly Lys Ala Leu
            420
```

<210> 57
<211> 1272
<212> DNA
<213> Euglena gracilis

<220>
<221> CDS

<222> (2)..(1270)
<223> synthetic delta-8 desaturase (codon-optimized for Yarrowia lipolytica) ("D8SF" or "EgD8S")

<300>
<302> DELTA-8 DESATURASE AND ITS USE IN MAKING POLYUNSATURATED FATTY ACIDS

<310> WO 2006/012325 and WO 2006/012326
<311> 2005-06-24
<312> 2006-02-02
<313> (1)..(1272)

<300>
<302> DELTA-8 DESATURASE AND ITS USE IN MAKING POLYUNSATURATED FATTY ACIDS

<310> U.S. 7,256,033
<311> 2005-06-24
<312> 2007-08-14
<313> (1)..(1272)

<400> 57

```
c atg gtg aag tcc aag cga cag gct ctg ccc ctc acc atc gac gga act        49
  Met Val Lys Ser Lys Arg Gln Ala Leu Pro Leu Thr Ile Asp Gly Thr
  1               5                   10                  15

acc tac gac gtc tcc gct tgg gtg aac ttc cac cct ggt gga gct gaa          97
Thr Tyr Asp Val Ser Ala Trp Val Asn Phe His Pro Gly Gly Ala Glu
            20                  25                  30

atc att gag aac tac cag gga cga gat gct act gac gcc ttc atg gtt         145
Ile Ile Glu Asn Tyr Gln Gly Arg Asp Ala Thr Asp Ala Phe Met Val
        35                  40                  45

atg cac tct cag gaa gcc ttc gac aag ctc aag cga atg ccc aag atc         193
Met His Ser Gln Glu Ala Phe Asp Lys Leu Lys Arg Met Pro Lys Ile
    50                  55                  60

aac ccc tcc tcc gag ctg cct ccc cag gct gcc gtc aac gaa gct cag         241
Asn Pro Ser Ser Glu Leu Pro Pro Gln Ala Ala Val Asn Glu Ala Gln
65                  70                  75                  80

gag gat ttc cga aag ctc cga gaa gag ctg atc gcc act ggc atg ttt         289
Glu Asp Phe Arg Lys Leu Arg Glu Glu Leu Ile Ala Thr Gly Met Phe
                85                  90                  95

gac gcc tct ccc ctc tgg tac tcg tac aag atc tcc acc acc ctg ggt         337
Asp Ala Ser Pro Leu Trp Tyr Ser Tyr Lys Ile Ser Thr Thr Leu Gly
            100                 105                 110

ctt ggc gtg ctt gga tac ttc ctg atg gtc cag tac cag atg tac ttc         385
Leu Gly Val Leu Gly Tyr Phe Leu Met Val Gln Tyr Gln Met Tyr Phe
        115                 120                 125

att ggt gct gtg ctg ctc ggt atg cac tac cag caa atg gga tgg ctg         433
Ile Gly Ala Val Leu Leu Gly Met His Tyr Gln Gln Met Gly Trp Leu
    130                 135                 140

tct cat gac atc tgc cac cac cag acc ttc aag aac cga aac tgg aat         481
Ser His Asp Ile Cys His His Gln Thr Phe Lys Asn Arg Asn Trp Asn
145                 150                 155                 160

aac ctc gtg ggt ctg gtc ttt ggc aac gga ctc cag ggc ttc tcc gtg         529
Asn Leu Val Gly Leu Val Phe Gly Asn Gly Leu Gln Gly Phe Ser Val
                165                 170                 175

acc tgg tgg aag gac aga cac aac gcc cat cat tct gct acc aac gtt         577
Thr Trp Trp Lys Asp Arg His Asn Ala His His Ser Ala Thr Asn Val
```

```
            180                 185                 190

cag ggt cac gat ccc gac att gat aac ctg cct ctg ctc gcc tgg tcc        625
Gln Gly His Asp Pro Asp Ile Asp Asn Leu Pro Leu Leu Ala Trp Ser
        195                 200                 205

gag gac gat gtc act cga gct tct ccc atc tcc cga aag ctc att cag        673
Glu Asp Asp Val Thr Arg Ala Ser Pro Ile Ser Arg Lys Leu Ile Gln
    210                 215                 220

ttc caa cag tac tat ttc ctg gtc atc tgt att ctc ctg cga ttc atc        721
Phe Gln Gln Tyr Tyr Phe Leu Val Ile Cys Ile Leu Leu Arg Phe Ile
225                 230                 235                 240

tgg tgt ttc cag tct gtg ctg acc gtt cga tcc ctc aag gac cga gac        769
Trp Cys Phe Gln Ser Val Leu Thr Val Arg Ser Leu Lys Asp Arg Asp
                245                 250                 255

aac cag ttc tac cga tct cag tac aag aaa gag gcc att gga ctc gct        817
Asn Gln Phe Tyr Arg Ser Gln Tyr Lys Lys Glu Ala Ile Gly Leu Ala
            260                 265                 270

ctg cac tgg act ctc aag acc ctg ttc cac ctc ttc ttt atg ccc tcc        865
Leu His Trp Thr Leu Lys Thr Leu Phe His Leu Phe Phe Met Pro Ser
        275                 280                 285

atc ctg acc tcg ctc ctg gtg ttc ttt gtt tcc gag ctc gtc ggt ggc        913
Ile Leu Thr Ser Leu Leu Val Phe Phe Val Ser Glu Leu Val Gly Gly
    290                 295                 300

ttc gga att gcc atc gtg gtc ttc atg aac cac tac cct ctg gag aag        961
Phe Gly Ile Ala Ile Val Val Phe Met Asn His Tyr Pro Leu Glu Lys
305                 310                 315                 320

atc ggt gat tcc gtc tgg gac gga cat ggc ttc tct gtg ggt cag atc       1009
Ile Gly Asp Ser Val Trp Asp Gly His Gly Phe Ser Val Gly Gln Ile
                325                 330                 335

cat gag acc atg aac att cga cga ggc atc att act gac tgg ttc ttt       1057
His Glu Thr Met Asn Ile Arg Arg Gly Ile Ile Thr Asp Trp Phe Phe
            340                 345                 350

gga ggc ctg aac tac cag atc gag cac cat ctc tgg ccc acc ctg cct       1105
Gly Gly Leu Asn Tyr Gln Ile Glu His His Leu Trp Pro Thr Leu Pro
        355                 360                 365

cga cac aac ctc act gcc gtt tcc tac cag gtg gaa cag ctg tgc cag       1153
Arg His Asn Leu Thr Ala Val Ser Tyr Gln Val Glu Gln Leu Cys Gln
    370                 375                 380

aag cac aac ctc ccc tac cga aac cct ctg ccc cat gaa ggt ctc gtc       1201
Lys His Asn Leu Pro Tyr Arg Asn Pro Leu Pro His Glu Gly Leu Val
385                 390                 395                 400

atc ctg ctc cga tac ctg gcc gtg ttc gct cga atg gcc gag aag cag       1249
Ile Leu Leu Arg Tyr Leu Ala Val Phe Ala Arg Met Ala Glu Lys Gln
                405                 410                 415

ccc gct ggc aag gct ctc taa gc                                        1272
Pro Ala Gly Lys Ala Leu
            420
```

<210> 58
<211> 422
<212> PRT
<213> Euglena gracilis

<400> 58

Met Val Lys Ser Lys Arg Gln Ala Leu Pro Leu Thr Ile Asp Gly Thr
1               5                   10                  15

Thr Tyr Asp Val Ser Ala Trp Val Asn Phe His Pro Gly Gly Ala Glu
            20                  25                  30

Ile Ile Glu Asn Tyr Gln Gly Arg Asp Ala Thr Asp Ala Phe Met Val
        35                  40                  45

Met His Ser Gln Glu Ala Phe Asp Lys Leu Lys Arg Met Pro Lys Ile
    50                  55                  60

Asn Pro Ser Ser Glu Leu Pro Pro Gln Ala Ala Val Asn Glu Ala Gln
65                  70                  75                  80

Glu Asp Phe Arg Lys Leu Arg Glu Glu Leu Ile Ala Thr Gly Met Phe
                85                  90                  95

Asp Ala Ser Pro Leu Trp Tyr Ser Tyr Lys Ile Ser Thr Thr Leu Gly
            100                 105                 110

Leu Gly Val Leu Gly Tyr Phe Leu Met Val Gln Tyr Gln Met Tyr Phe
        115                 120                 125

Ile Gly Ala Val Leu Leu Gly Met His Tyr Gln Gln Met Gly Trp Leu
    130                 135                 140

Ser His Asp Ile Cys His His Gln Thr Phe Lys Asn Arg Asn Trp Asn
145                 150                 155                 160

Asn Leu Val Gly Leu Val Phe Gly Asn Gly Leu Gln Gly Phe Ser Val
                165                 170                 175

Thr Trp Trp Lys Asp Arg His Asn Ala His His Ser Ala Thr Asn Val
            180                 185                 190

Gln Gly His Asp Pro Asp Ile Asp Asn Leu Pro Leu Leu Ala Trp Ser
        195                 200                 205

Glu Asp Asp Val Thr Arg Ala Ser Pro Ile Ser Arg Lys Leu Ile Gln
    210                 215                 220

Phe Gln Gln Tyr Tyr Phe Leu Val Ile Cys Ile Leu Leu Arg Phe Ile
225                 230                 235                 240

```
Trp Cys Phe Gln Ser Val Leu Thr Val Arg Ser Leu Lys Asp Arg Asp
                245                 250                 255

Asn Gln Phe Tyr Arg Ser Gln Tyr Lys Lys Glu Ala Ile Gly Leu Ala
            260                 265                 270

Leu His Trp Thr Leu Lys Thr Leu Phe His Leu Phe Phe Met Pro Ser
        275                 280                 285

Ile Leu Thr Ser Leu Leu Val Phe Phe Val Ser Glu Leu Val Gly Gly
    290                 295                 300

Phe Gly Ile Ala Ile Val Val Phe Met Asn His Tyr Pro Leu Glu Lys
305                 310                 315                 320

Ile Gly Asp Ser Val Trp Asp Gly His Gly Phe Ser Val Gly Gln Ile
                325                 330                 335

His Glu Thr Met Asn Ile Arg Arg Gly Ile Ile Thr Asp Trp Phe Phe
            340                 345                 350

Gly Gly Leu Asn Tyr Gln Ile Glu His His Leu Trp Pro Thr Leu Pro
        355                 360                 365

Arg His Asn Leu Thr Ala Val Ser Tyr Gln Val Glu Gln Leu Cys Gln
    370                 375                 380

Lys His Asn Leu Pro Tyr Arg Asn Pro Leu Pro His Glu Gly Leu Val
385                 390                 395                 400

Ile Leu Leu Arg Tyr Leu Ala Val Phe Ala Arg Met Ala Glu Lys Gln
                405                 410                 415

Pro Ala Gly Lys Ala Leu
            420
```

<210> 59
<211> 1272
<212> DNA
<213> Artificial Sequence

<220>
<223> mutant EgD8S-23 delta-8 desaturase (also "EgD8M")

<220>
<221> CDS
<222> (2)..(1270)

<300>
<302> MUTANT DELTA-8 DESATURASE GENES ENGINEERED BY TARGETED MUTAGENSIS AND THEIR USE IN MAKING POLYUNSATURATED FATTY ACIDS

<310> WO 2008/073271
<311> 2007-12-05
<312> 2008-06-19
<313> (1)..(1272)

<300>
<302> MUTANT DELTA-8 DESATURASE GENES ENGINEERED BY TARGETED MUTAGENSIS AND THEIR USE IN MAKING POLYUNSATURATED FATTY ACIDS

<310> US 2008-0138868-A1
<311> 2006-12-07
<312> 2008-06-12
<313> (1)..(1272)

<400> 59

```
c atg gtg aag gct tct cga cag gct ctg ccc ctc gtc atc gac gga aag        49
  Met Val Lys Ala Ser Arg Gln Ala Leu Pro Leu Val Ile Asp Gly Lys
  1               5                   10                  15

gtg tac gac gtc tcc gct tgg gtg aac ttc cac cct ggt gga gct gaa          97
Val Tyr Asp Val Ser Ala Trp Val Asn Phe His Pro Gly Gly Ala Glu
            20                  25                  30

atc att gag aac tac cag gga cga gat gct act gac gcc ttc atg gtt         145
Ile Ile Glu Asn Tyr Gln Gly Arg Asp Ala Thr Asp Ala Phe Met Val
        35                  40                  45

atg cac tct cag gaa gcc ttc gac aag ctc aag cga atg ccc aag atc         193
Met His Ser Gln Glu Ala Phe Asp Lys Leu Lys Arg Met Pro Lys Ile
    50                  55                  60

aac cag gct tcc gag ctg cct ccc cag gct gcc gtc aac gaa gct cag         241
Asn Gln Ala Ser Glu Leu Pro Pro Gln Ala Ala Val Asn Glu Ala Gln
65                  70                  75                  80

gag gat ttc cga aag ctc cga gaa gag ctg atc gcc act ggc atg ttt         289
Glu Asp Phe Arg Lys Leu Arg Glu Glu Leu Ile Ala Thr Gly Met Phe
                85                  90                  95

gac gcc tct ccc ctc tgg tac tcg tac aag atc ttg acc acc ctg ggt         337
Asp Ala Ser Pro Leu Trp Tyr Ser Tyr Lys Ile Leu Thr Thr Leu Gly
            100                 105                 110

ctt ggc gtg ctt gcc ttc ttc atg ctg gtc cag tac cac ctg tac ttc         385
Leu Gly Val Leu Ala Phe Phe Met Leu Val Gln Tyr His Leu Tyr Phe
        115                 120                 125

att ggt gct ctc gtg ctc ggt atg cac tac cag caa atg gga tgg ctg         433
Ile Gly Ala Leu Val Leu Gly Met His Tyr Gln Gln Met Gly Trp Leu
    130                 135                 140

tct cat gac atc tgc cac cac cag acc ttc aag aac cga aac tgg aat         481
Ser His Asp Ile Cys His His Gln Thr Phe Lys Asn Arg Asn Trp Asn
145                 150                 155                 160

aac gtc ctg ggt ctg gtc ttt ggc aac gga ctc cag ggc ttc tcc gtg         529
Asn Val Leu Gly Leu Val Phe Gly Asn Gly Leu Gln Gly Phe Ser Val
                165                 170                 175

acc tgg tgg aag gac aga cac aac gcc cat cat tct gct acc aac gtt         577
Thr Trp Trp Lys Asp Arg His Asn Ala His His Ser Ala Thr Asn Val
            180                 185                 190

cag ggt cac gat ccc gac att gat aac ctg cct ctg ctc gcc tgg tcc         625
Gln Gly His Asp Pro Asp Ile Asp Asn Leu Pro Leu Leu Ala Trp Ser
        195                 200                 205
```

```
gag gac gat gtc act cga gct tct ccc atc tcc cga aag ctc att cag      673
Glu Asp Asp Val Thr Arg Ala Ser Pro Ile Ser Arg Lys Leu Ile Gln
    210                 215                 220

ttc caa cag tac tat ttc ctg gtc atc tgt att ctc ctg cga ttc atc      721
Phe Gln Gln Tyr Tyr Phe Leu Val Ile Cys Ile Leu Leu Arg Phe Ile
225                 230                 235                 240

tgg tgt ttc cag tct gtg ctg acc gtt cga tcc ctc aag gac cga gac      769
Trp Cys Phe Gln Ser Val Leu Thr Val Arg Ser Leu Lys Asp Arg Asp
                245                 250                 255

aac cag ttc tac cga tct cag tac aag aaa gag gcc att gga ctc gct      817
Asn Gln Phe Tyr Arg Ser Gln Tyr Lys Lys Glu Ala Ile Gly Leu Ala
            260                 265                 270

ctg cac tgg act ctc aag acc ctg ttc cac ctc ttc ttt atg ccc tcc      865
Leu His Trp Thr Leu Lys Thr Leu Phe His Leu Phe Phe Met Pro Ser
        275                 280                 285

atc ctg acc tcg atg ctg gtg ttc ttt gtt tcc gag ctc gtc ggt ggc      913
Ile Leu Thr Ser Met Leu Val Phe Phe Val Ser Glu Leu Val Gly Gly
    290                 295                 300

ttc gga att gcc atc gtg gtc ttc atg aac cac tac cct ctg gag aag      961
Phe Gly Ile Ala Ile Val Val Phe Met Asn His Tyr Pro Leu Glu Lys
305                 310                 315                 320

atc ggt gat tcc gtc tgg gac gga cat ggc ttc tct gtg ggt cag atc      1009
Ile Gly Asp Ser Val Trp Asp Gly His Gly Phe Ser Val Gly Gln Ile
                325                 330                 335

cat gag acc atg aac att cga cga ggc atc att act gac tgg ttc ttt      1057
His Glu Thr Met Asn Ile Arg Arg Gly Ile Ile Thr Asp Trp Phe Phe
            340                 345                 350

gga ggc ctg aac tac cag atc gag cac cat ctc tgg ccc acc ctg cct      1105
Gly Gly Leu Asn Tyr Gln Ile Glu His His Leu Trp Pro Thr Leu Pro
        355                 360                 365

cga cac aac ctc act gcc gtt tcc tac cag gtg gaa cag ctg tgc cag      1153
Arg His Asn Leu Thr Ala Val Ser Tyr Gln Val Glu Gln Leu Cys Gln
    370                 375                 380

aag cac aac ctc ccc tac cga aac cct ctg ccc cat gaa ggt ctc gtc      1201
Lys His Asn Leu Pro Tyr Arg Asn Pro Leu Pro His Glu Gly Leu Val
385                 390                 395                 400

atc ctg ctc cga tac ctg tcc cag ttc gct cga atg gcc gag aag cag      1249
Ile Leu Leu Arg Tyr Leu Ser Gln Phe Ala Arg Met Ala Glu Lys Gln
                405                 410                 415

ccc ggt gcc aag gct cag taa gc                                       1272
Pro Gly Ala Lys Ala Gln
            420
```

<210> 60
<211> 422
<212> PRT
<213> Artificial Sequence

<220>
<223> Synthetic Construct

<400> 60

```
Met Val Lys Ala Ser Arg Gln Ala Leu Pro Leu Val Ile Asp Gly Lys
1               5                   10                  15

Val Tyr Asp Val Ser Ala Trp Val Asn Phe His Pro Gly Gly Ala Glu
            20                  25                  30

Ile Ile Glu Asn Tyr Gln Gly Arg Asp Ala Thr Asp Ala Phe Met Val
        35                  40                  45

Met His Ser Gln Glu Ala Phe Asp Lys Leu Lys Arg Met Pro Lys Ile
    50                  55                  60

Asn Gln Ala Ser Glu Leu Pro Pro Gln Ala Ala Val Asn Glu Ala Gln
65                  70                  75                  80

Glu Asp Phe Arg Lys Leu Arg Glu Glu Leu Ile Ala Thr Gly Met Phe
                85                  90                  95

Asp Ala Ser Pro Leu Trp Tyr Ser Tyr Lys Ile Leu Thr Thr Leu Gly
            100                 105                 110

Leu Gly Val Leu Ala Phe Phe Met Leu Val Gln Tyr His Leu Tyr Phe
        115                 120                 125

Ile Gly Ala Leu Val Leu Gly Met His Tyr Gln Gln Met Gly Trp Leu
    130                 135                 140

Ser His Asp Ile Cys His His Gln Thr Phe Lys Asn Arg Asn Trp Asn
145                 150                 155                 160

Asn Val Leu Gly Leu Val Phe Gly Asn Gly Leu Gln Gly Phe Ser Val
                165                 170                 175

Thr Trp Trp Lys Asp Arg His Asn Ala His His Ser Ala Thr Asn Val
            180                 185                 190

Gln Gly His Asp Pro Asp Ile Asp Asn Leu Pro Leu Leu Ala Trp Ser
        195                 200                 205

Glu Asp Asp Val Thr Arg Ala Ser Pro Ile Ser Arg Lys Leu Ile Gln
    210                 215                 220

Phe Gln Gln Tyr Tyr Phe Leu Val Ile Cys Ile Leu Leu Arg Phe Ile
225                 230                 235                 240

Trp Cys Phe Gln Ser Val Leu Thr Val Arg Ser Leu Lys Asp Arg Asp
                245                 250                 255
```

Asn Gln Phe Tyr Arg Ser Gln Tyr Lys Lys Glu Ala Ile Gly Leu Ala
260 265 270

Leu His Trp Thr Leu Lys Thr Leu Phe His Leu Phe Phe Met Pro Ser
275 280 285

Ile Leu Thr Ser Met Leu Val Phe Phe Val Ser Glu Leu Val Gly Gly
290 295 300

Phe Gly Ile Ala Ile Val Val Phe Met Asn His Tyr Pro Leu Glu Lys
305 310 315 320

Ile Gly Asp Ser Val Trp Asp Gly His Gly Phe Ser Val Gly Gln Ile
325 330 335

His Glu Thr Met Asn Ile Arg Arg Gly Ile Ile Thr Asp Trp Phe Phe
340 345 350

Gly Gly Leu Asn Tyr Gln Ile Glu His His Leu Trp Pro Thr Leu Pro
355 360 365

Arg His Asn Leu Thr Ala Val Ser Tyr Gln Val Glu Gln Leu Cys Gln
370 375 380

Lys His Asn Leu Pro Tyr Arg Asn Pro Leu Pro His Glu Gly Leu Val
385 390 395 400

Ile Leu Leu Arg Tyr Leu Ser Gln Phe Ala Arg Met Ala Glu Lys Gln
405 410 415

Pro Gly Ala Lys Ala Gln
420

<210> 61
<211> 1260
<212> DNA
<213> Euglena anabaena UTEX 373

<220>
<221> CDS
<222> (1)..(1260)
<223> delta-8 desaturase

<300>
<302> DELTA-8 DESATURASES AND THEIR USE IN MAKING POLYUNSATURATED FATTY ACIDS

<310> U.S. 2008-0254521-A1
<311> 2008-04-09
<312> 2008-10-16
<313> (1)..(1260)

<300>
<302> DELTA-8 DESATURASES AND THEIR USE IN MAKING POLYUNSATURATED FATTY ACIDS

<310> WO 2008/124194
<311> 2008-04-10
<312> 2008-10-16
<313> (1)..(1260)

<400> 61

```
atg gtg aaa agg cca gca ctt ccg ctg acc gtt gat ggt gtc acc tat        48
Met Val Lys Arg Pro Ala Leu Pro Leu Thr Val Asp Gly Val Thr Tyr
1               5                   10                  15

gat gtg tct gcc tgg ttg aac cat cat cca ggg ggt gct gac atc att        96
Asp Val Ser Ala Trp Leu Asn His His Pro Gly Gly Ala Asp Ile Ile
            20                  25                  30

gag aac tac cgc ggt cgt gat gcc act gat gtc ttt atg gtt atg cac       144
Glu Asn Tyr Arg Gly Arg Asp Ala Thr Asp Val Phe Met Val Met His
        35                  40                  45

tct gaa aat gct gtg agt aaa cta aga agg atg cct atc atg gaa cca       192
Ser Glu Asn Ala Val Ser Lys Leu Arg Arg Met Pro Ile Met Glu Pro
    50                  55                  60

tca tct cca ctg acg cct acg cca ccg aaa ccc aac tca gac gaa ccg       240
Ser Ser Pro Leu Thr Pro Thr Pro Pro Lys Pro Asn Ser Asp Glu Pro
65                  70                  75                  80

cag gag gat ttc cgc aag ctc cga gat gag ctc atc gca gca gga atg       288
Gln Glu Asp Phe Arg Lys Leu Arg Asp Glu Leu Ile Ala Ala Gly Met
                85                  90                  95

ttc gac gca tca ccg atg tgg tac gca tat aag acg ctc agt acg ctg       336
Phe Asp Ala Ser Pro Met Trp Tyr Ala Tyr Lys Thr Leu Ser Thr Leu
            100                 105                 110

ggc ctc ggg gtc ctc gcg gtg cta ttg atg acc cag tgg cac tgg tac       384
Gly Leu Gly Val Leu Ala Val Leu Leu Met Thr Gln Trp His Trp Tyr
        115                 120                 125

ctc gtc ggg gca atc gtg ttg ggc att cac ttc caa caa atg ggt tgg       432
Leu Val Gly Ala Ile Val Leu Gly Ile His Phe Gln Gln Met Gly Trp
    130                 135                 140

ttg tcg cac gat atc tgc cac cat cag ctg ttc aag gac cga tcg atc       480
Leu Ser His Asp Ile Cys His His Gln Leu Phe Lys Asp Arg Ser Ile
145                 150                 155                 160

aac aac gcc atc ggc ttg ctt ttc ggg aac gtc ttg caa ggg ttc tct       528
Asn Asn Ala Ile Gly Leu Leu Phe Gly Asn Val Leu Gln Gly Phe Ser
                165                 170                 175

gtg acc tgg tgg aag gac agg cac aat gca cac cac tcc gcc acc aac       576
Val Thr Trp Trp Lys Asp Arg His Asn Ala His His Ser Ala Thr Asn
            180                 185                 190

gtg caa ggc cac gac ccc gac att gac aac ctg ccg ctg ctg gca tgg       624
Val Gln Gly His Asp Pro Asp Ile Asp Asn Leu Pro Leu Leu Ala Trp
        195                 200                 205

tcc aag gag gac gtg gag agg gcc ggc ccg ttc tca cgg cgg atg atc       672
Ser Lys Glu Asp Val Glu Arg Ala Gly Pro Phe Ser Arg Arg Met Ile
    210                 215                 220

aag tac cag caa tac tac ttc ttc ttc atc tgt gcc ctc ctg agg ttc       720
```

```
Lys Tyr Gln Gln Tyr Tyr Phe Phe Phe Ile Cys Ala Leu Leu Arg Phe
225                 230                 235                 240

atc tgg tgc ttc cag agc atc cac aca gcc acg ggc ctg aag gat cgc      768
Ile Trp Cys Phe Gln Ser Ile His Thr Ala Thr Gly Leu Lys Asp Arg
                245                 250                 255

agc aac cag tac tac cgc agg cag tac gag aaa gag agc gtg ggc ctg      816
Ser Asn Gln Tyr Tyr Arg Arg Gln Tyr Glu Lys Glu Ser Val Gly Leu
            260                 265                 270

gcc ctc cac tgg ggc ctg aag gcg ttg ttc tac tac ttt tat atg cca      864
Ala Leu His Trp Gly Leu Lys Ala Leu Phe Tyr Tyr Phe Tyr Met Pro
        275                 280                 285

agc ttc ttg acc gga ctc atg gtg ttt ttc gtg tcc gag ttg ctt ggg      912
Ser Phe Leu Thr Gly Leu Met Val Phe Phe Val Ser Glu Leu Leu Gly
    290                 295                 300

ggc ttc ggc atc gcc atc gtg gtg ttc atg aac cac tac ccc ctg gag      960
Gly Phe Gly Ile Ala Ile Val Val Phe Met Asn His Tyr Pro Leu Glu
305                 310                 315                 320

aag atc cag gac tcg gtg tgg gac ggc cac ggc ttt tgc gcc ggc cag     1008
Lys Ile Gln Asp Ser Val Trp Asp Gly His Gly Phe Cys Ala Gly Gln
                325                 330                 335

att cac gaa acg atg aac gtc cag cgg gga ctc gtc acg gac tgg ttc     1056
Ile His Glu Thr Met Asn Val Gln Arg Gly Leu Val Thr Asp Trp Phe
            340                 345                 350

ttc ggt ggg ctg aat tac caa atc gag cac cac ctg tgg ccg acg ctg     1104
Phe Gly Gly Leu Asn Tyr Gln Ile Glu His His Leu Trp Pro Thr Leu
        355                 360                 365

ccc cgg cac aac ctg acg gcg gcc agc atc aaa gtg gag cag ttg tgc     1152
Pro Arg His Asn Leu Thr Ala Ala Ser Ile Lys Val Glu Gln Leu Cys
    370                 375                 380

aag aag cac aac ttg ccg tat cgc agc ccc cca atg ctg gag ggg gtg     1200
Lys Lys His Asn Leu Pro Tyr Arg Ser Pro Pro Met Leu Glu Gly Val
385                 390                 395                 400

ggc atc ctg atc agc tac ctg ggc acc ttt gcc cgc atg gtg gca aag     1248
Gly Ile Leu Ile Ser Tyr Leu Gly Thr Phe Ala Arg Met Val Ala Lys
                405                 410                 415

gcc gac aag gcg                                                     1260
Ala Asp Lys Ala
            420
```

<210> 62
<211> 420
<212> PRT
<213> Euglena anabaena UTEX 373

<400> 62

Met Val Lys Arg Pro Ala Leu Pro Leu Thr Val Asp Gly Val Thr Tyr
1 5 10 15

Asp Val Ser Ala Trp Leu Asn His His Pro Gly Gly Ala Asp Ile Ile
20 25 30

```
Glu Asn Tyr Arg Gly Arg Asp Ala Thr Asp Val Phe Met Val Met His
        35                  40                  45

Ser Glu Asn Ala Val Ser Lys Leu Arg Arg Met Pro Ile Met Glu Pro
    50                  55                  60

Ser Ser Pro Leu Thr Pro Thr Pro Pro Lys Pro Asn Ser Asp Glu Pro
65                  70                  75                  80

Gln Glu Asp Phe Arg Lys Leu Arg Asp Glu Leu Ile Ala Ala Gly Met
                85                  90                  95

Phe Asp Ala Ser Pro Met Trp Tyr Ala Tyr Lys Thr Leu Ser Thr Leu
            100                 105                 110

Gly Leu Gly Val Leu Ala Val Leu Leu Met Thr Gln Trp His Trp Tyr
        115                 120                 125

Leu Val Gly Ala Ile Val Leu Gly Ile His Phe Gln Gln Met Gly Trp
    130                 135                 140

Leu Ser His Asp Ile Cys His His Gln Leu Phe Lys Asp Arg Ser Ile
145                 150                 155                 160

Asn Asn Ala Ile Gly Leu Leu Phe Gly Asn Val Leu Gln Gly Phe Ser
                165                 170                 175

Val Thr Trp Trp Lys Asp Arg His Asn Ala His His Ser Ala Thr Asn
            180                 185                 190

Val Gln Gly His Asp Pro Asp Ile Asp Asn Leu Pro Leu Leu Ala Trp
        195                 200                 205

Ser Lys Glu Asp Val Glu Arg Ala Gly Pro Phe Ser Arg Arg Met Ile
    210                 215                 220

Lys Tyr Gln Gln Tyr Tyr Phe Phe Phe Ile Cys Ala Leu Leu Arg Phe
225                 230                 235                 240

Ile Trp Cys Phe Gln Ser Ile His Thr Ala Thr Gly Leu Lys Asp Arg
                245                 250                 255

Ser Asn Gln Tyr Tyr Arg Arg Gln Tyr Glu Lys Glu Ser Val Gly Leu
            260                 265                 270

Ala Leu His Trp Gly Leu Lys Ala Leu Phe Tyr Tyr Phe Tyr Met Pro
        275                 280                 285
```

```
Ser Phe Leu Thr Gly Leu Met Val Phe Phe Val Ser Glu Leu Leu Gly
    290                 295                 300

Gly Phe Gly Ile Ala Ile Val Val Phe Met Asn His Tyr Pro Leu Glu
305                 310                 315                 320

Lys Ile Gln Asp Ser Val Trp Asp Gly His Gly Phe Cys Ala Gly Gln
                325                 330                 335

Ile His Glu Thr Met Asn Val Gln Arg Gly Leu Val Thr Asp Trp Phe
            340                 345                 350

Phe Gly Gly Leu Asn Tyr Gln Ile Glu His His Leu Trp Pro Thr Leu
        355                 360                 365

Pro Arg His Asn Leu Thr Ala Ala Ser Ile Lys Val Glu Gln Leu Cys
    370                 375                 380

Lys Lys His Asn Leu Pro Tyr Arg Ser Pro Pro Met Leu Glu Gly Val
385                 390                 395                 400

Gly Ile Leu Ile Ser Tyr Leu Gly Thr Phe Ala Arg Met Val Ala Lys
                405                 410                 415

Ala Asp Lys Ala
            420
```

<210> 63
<211> 1260
<212> DNA
<213> Euglena anabaena UTEX 373

<220>
<221> CDS
<222> (1)..(1260)
<223> synthetic delta-8 desaturase (codon-optimized for Yarrowia lipolytica)

<300>
<302> DELTA-8 DESATURASES AND THEIR USE IN MAKING POLYUNSATURATED FATTY ACIDS

<310> U.S. 2008-0254521-A1
<311> 2008-04-09
<312> 2008-10-16
<313> (1)..(1260)

<300>
<302> DELTA-8 DESATURASES AND THEIR USE IN MAKING POLYUNSATURATED FATTY ACIDS

<310> WO 2008/124194
<311> 2008-04-10
<312> 2008-10-16
<313> (1)..(1260)

<400> 63

```
atg gtc aag cga ccc gct ctg cct ctc acc gtg gac ggt gtc acc tac      48
Met Val Lys Arg Pro Ala Leu Pro Leu Thr Val Asp Gly Val Thr Tyr
1               5                   10                  15

gac gtt tct gcc tgg ctc aac cac cat ccc gga ggt gcc gac att atc      96
Asp Val Ser Ala Trp Leu Asn His His Pro Gly Gly Ala Asp Ile Ile
            20                  25                  30

gag aac tac cga ggt cgg gat gct acc gac gtc ttc atg gtt atg cac      144
Glu Asn Tyr Arg Gly Arg Asp Ala Thr Asp Val Phe Met Val Met His
        35                  40                  45

tcc gag aac gcc gtg tcc aaa ctc aga cga atg ccc atc atg gaa cct      192
Ser Glu Asn Ala Val Ser Lys Leu Arg Arg Met Pro Ile Met Glu Pro
    50                  55                  60

tcc tct ccc ctg act cca aca cct ccc aag cca aac tcc gac gaa cct      240
Ser Ser Pro Leu Thr Pro Thr Pro Pro Lys Pro Asn Ser Asp Glu Pro
65                  70                  75                  80

cag gag gat ttc cga aag ctg cga gac gag ctc att gct gca ggc atg      288
Gln Glu Asp Phe Arg Lys Leu Arg Asp Glu Leu Ile Ala Ala Gly Met
                85                  90                  95

ttc gat gcc tct ccc atg tgg tac gct tac aag acc ctg tcg act ctc      336
Phe Asp Ala Ser Pro Met Trp Tyr Ala Tyr Lys Thr Leu Ser Thr Leu
            100                 105                 110

gga ctg ggt gtc ctt gcc gtg ctg ttg atg acc cag tgg cac tgg tac      384
Gly Leu Gly Val Leu Ala Val Leu Leu Met Thr Gln Trp His Trp Tyr
        115                 120                 125

ctg gtt ggt gct atc gtc ctc ggc att cac ttt caa cag atg gga tgg      432
Leu Val Gly Ala Ile Val Leu Gly Ile His Phe Gln Gln Met Gly Trp
    130                 135                 140

ctc tcg cac gac att tgc cat cac cag ctg ttc aag gac cga tcc atc      480
Leu Ser His Asp Ile Cys His His Gln Leu Phe Lys Asp Arg Ser Ile
145                 150                 155                 160

aac aat gcc att ggc ctg ctc ttc gga aac gtg ctt cag ggc ttt tct      528
Asn Asn Ala Ile Gly Leu Leu Phe Gly Asn Val Leu Gln Gly Phe Ser
                165                 170                 175

gtc act tgg tgg aag gac cga cac aac gct cat cac tcc gcc acc aac      576
Val Thr Trp Trp Lys Asp Arg His Asn Ala His His Ser Ala Thr Asn
            180                 185                 190

gtg cag ggt cac gat ccc gac atc gac aac ctg cct ctc ctg gcg tgg      624
Val Gln Gly His Asp Pro Asp Ile Asp Asn Leu Pro Leu Leu Ala Trp
        195                 200                 205

tcc aag gag gac gtc gag cga gct ggc ccg ttt tct cga cgg atg atc      672
Ser Lys Glu Asp Val Glu Arg Ala Gly Pro Phe Ser Arg Arg Met Ile
    210                 215                 220

aag tac caa cag tat tac ttc ttt ttc atc tgt gcc ctt ctg cga ttc      720
Lys Tyr Gln Gln Tyr Tyr Phe Phe Phe Ile Cys Ala Leu Leu Arg Phe
225                 230                 235                 240

atc tgg tgc ttt cag tcc att cat act gcc acg ggt ctc aag gat cga      768
Ile Trp Cys Phe Gln Ser Ile His Thr Ala Thr Gly Leu Lys Asp Arg
                245                 250                 255
```

```
agc aat cag tac tat cga aga cag tac gag aag gag tcc gtc ggt ctg      816
Ser Asn Gln Tyr Tyr Arg Arg Gln Tyr Glu Lys Glu Ser Val Gly Leu
                260                 265                 270

gca ctc cac tgg ggt ctc aag gcc ttg ttc tac tat ttc tac atg ccc      864
Ala Leu His Trp Gly Leu Lys Ala Leu Phe Tyr Tyr Phe Tyr Met Pro
            275                 280                 285

tcg ttt ctc acc gga ctc atg gtg ttc ttt gtc tcc gag ctg ctt ggt      912
Ser Phe Leu Thr Gly Leu Met Val Phe Phe Val Ser Glu Leu Leu Gly
    290                 295                 300

ggc ttc gga att gcc atc gtt gtc ttc atg aac cac tac cct ctg gag      960
Gly Phe Gly Ile Ala Ile Val Val Phe Met Asn His Tyr Pro Leu Glu
305                 310                 315                 320

aag att cag gac tcc gtg tgg gat ggt cat ggc ttc tgt gct gga cag     1008
Lys Ile Gln Asp Ser Val Trp Asp Gly His Gly Phe Cys Ala Gly Gln
                325                 330                 335

att cac gag acc atg aac gtt cag cga ggc ctc gtc aca gac tgg ttt     1056
Ile His Glu Thr Met Asn Val Gln Arg Gly Leu Val Thr Asp Trp Phe
            340                 345                 350

ttc ggt ggc ctc aac tac cag atc gaa cat cac ctg tgg cct act ctt     1104
Phe Gly Gly Leu Asn Tyr Gln Ile Glu His His Leu Trp Pro Thr Leu
        355                 360                 365

ccc aga cac aac ctc acc gct gcc tcc atc aaa gtg gag cag ctg tgc     1152
Pro Arg His Asn Leu Thr Ala Ala Ser Ile Lys Val Glu Gln Leu Cys
    370                 375                 380

aag aag cac aac ctg ccc tac cga tct cct ccc atg ctc gaa ggt gtc     1200
Lys Lys His Asn Leu Pro Tyr Arg Ser Pro Pro Met Leu Glu Gly Val
385                 390                 395                 400

ggc att ctt atc tcc tac ctg ggc acc ttc gct cga atg gtt gcc aag     1248
Gly Ile Leu Ile Ser Tyr Leu Gly Thr Phe Ala Arg Met Val Ala Lys
                405                 410                 415

gca gac aag gcc                                                     1260
Ala Asp Lys Ala
            420
```

<210> 64
<211> 420
<212> PRT
<213> Euglena anabaena UTEX 373

<400> 64

```
Met Val Lys Arg Pro Ala Leu Pro Leu Thr Val Asp Gly Val Thr Tyr
1               5                   10                  15

Asp Val Ser Ala Trp Leu Asn His His Pro Gly Gly Ala Asp Ile Ile
            20                  25                  30

Glu Asn Tyr Arg Gly Arg Asp Ala Thr Asp Val Phe Met Val Met His
        35                  40                  45

Ser Glu Asn Ala Val Ser Lys Leu Arg Arg Met Pro Ile Met Glu Pro
```

```
        50                  55                  60

Ser Ser Pro Leu Thr Pro Thr Pro Pro Lys Pro Asn Ser Asp Glu Pro
65                  70                  75                  80

Gln Glu Asp Phe Arg Lys Leu Arg Asp Glu Leu Ile Ala Ala Gly Met
                85                  90                  95

Phe Asp Ala Ser Pro Met Trp Tyr Ala Tyr Lys Thr Leu Ser Thr Leu
            100                 105                 110

Gly Leu Gly Val Leu Ala Val Leu Leu Met Thr Gln Trp His Trp Tyr
        115                 120                 125

Leu Val Gly Ala Ile Val Leu Gly Ile His Phe Gln Gln Met Gly Trp
    130                 135                 140

Leu Ser His Asp Ile Cys His His Gln Leu Phe Lys Asp Arg Ser Ile
145                 150                 155                 160

Asn Asn Ala Ile Gly Leu Leu Phe Gly Asn Val Leu Gln Gly Phe Ser
                165                 170                 175

Val Thr Trp Trp Lys Asp Arg His Asn Ala His His Ser Ala Thr Asn
            180                 185                 190

Val Gln Gly His Asp Pro Asp Ile Asp Asn Leu Pro Leu Leu Ala Trp
        195                 200                 205

Ser Lys Glu Asp Val Glu Arg Ala Gly Pro Phe Ser Arg Arg Met Ile
    210                 215                 220

Lys Tyr Gln Gln Tyr Tyr Phe Phe Phe Ile Cys Ala Leu Leu Arg Phe
225                 230                 235                 240

Ile Trp Cys Phe Gln Ser Ile His Thr Ala Thr Gly Leu Lys Asp Arg
                245                 250                 255

Ser Asn Gln Tyr Tyr Arg Arg Gln Tyr Glu Lys Glu Ser Val Gly Leu
            260                 265                 270

Ala Leu His Trp Gly Leu Lys Ala Leu Phe Tyr Tyr Phe Tyr Met Pro
        275                 280                 285

Ser Phe Leu Thr Gly Leu Met Val Phe Phe Val Ser Glu Leu Leu Gly
    290                 295                 300

Gly Phe Gly Ile Ala Ile Val Val Phe Met Asn His Tyr Pro Leu Glu
305                 310                 315                 320
```

```
Lys Ile Gln Asp Ser Val Trp Asp Gly His Gly Phe Cys Ala Gly Gln
                325                 330                 335

Ile His Glu Thr Met Asn Val Gln Arg Gly Leu Val Thr Asp Trp Phe
            340                 345                 350

Phe Gly Gly Leu Asn Tyr Gln Ile Glu His His Leu Trp Pro Thr Leu
        355                 360                 365

Pro Arg His Asn Leu Thr Ala Ala Ser Ile Lys Val Glu Gln Leu Cys
    370                 375                 380

Lys Lys His Asn Leu Pro Tyr Arg Ser Pro Pro Met Leu Glu Gly Val
385                 390                 395                 400

Gly Ile Leu Ile Ser Tyr Leu Gly Thr Phe Ala Arg Met Val Ala Lys
                405                 410                 415

Ala Asp Lys Ala
            420
```

<210> 65
<211> 1350
<212> DNA
<213> Euglena gracilis

<220>
<221> CDS
<222> (1)..(1350)
<223> delta-5 desaturase

<300>
<302> DELTA-5 DESATURASE AND ITS USE IN MAKING POLYUNSATURATED FATTY ACIDS

<310> WO 2007/136671
<311> 2007-05-16
<312> 2007-11-29
<313> (1)..(1350)

<300>
<302> DELTA-5 DESATURASE AND ITS USE IN MAKING POLYUNSATURATED FATTY ACIDS

<310> US 2007-0292924-A1
<311> 2007-05-15
<312> 2007-12-20
<313> (1)..(1350)

<400> 65

```
atg gct ctc agt ctt acc aca gaa cag ctg tta gaa cgc cct gat ttg           48
Met Ala Leu Ser Leu Thr Thr Glu Gln Leu Leu Glu Arg Pro Asp Leu
1               5                   10                  15

gtt gcg att gat ggc atc ctc tac gac ctt gaa ggg ctt gcc aaa gtt           96
Val Ala Ile Asp Gly Ile Leu Tyr Asp Leu Glu Gly Leu Ala Lys Val
            20                  25                  30
```

```
cat cca gga gga gat ttg att ctc gct tct ggt gcc tct gat gcc tcc        144
His Pro Gly Gly Asp Leu Ile Leu Ala Ser Gly Ala Ser Asp Ala Ser
        35                  40                  45

cct ctc ttt tat tca atg cat cca tac gtc aaa ccg gag aat tcc aaa        192
Pro Leu Phe Tyr Ser Met His Pro Tyr Val Lys Pro Glu Asn Ser Lys
    50                  55                  60

ttg ctt caa cag ttc gtc cga ggg aag cat gac cgc acc tcg aag gac        240
Leu Leu Gln Gln Phe Val Arg Gly Lys His Asp Arg Thr Ser Lys Asp
65                  70                  75                  80

att gtc tac acg tat gat tct ccc ttc gca caa gac gtt aag cgg aca        288
Ile Val Tyr Thr Tyr Asp Ser Pro Phe Ala Gln Asp Val Lys Arg Thr
                85                  90                  95

atg cgc gag gtg atg aaa ggg agg aac tgg tac gca acc cct ggc ttc        336
Met Arg Glu Val Met Lys Gly Arg Asn Trp Tyr Ala Thr Pro Gly Phe
            100                 105                 110

tgg ctg cgc acc gtt ggg atc atc gcc gtg acg gcc ttt tgc gag tgg        384
Trp Leu Arg Thr Val Gly Ile Ile Ala Val Thr Ala Phe Cys Glu Trp
        115                 120                 125

cac tgg gct acc acg ggg atg gtg ctg tgg ggc ctg ttg act gga ttc        432
His Trp Ala Thr Thr Gly Met Val Leu Trp Gly Leu Leu Thr Gly Phe
    130                 135                 140

atg cac atg cag atc ggc tta tcc atc cag cat gat gcg tcc cac ggg        480
Met His Met Gln Ile Gly Leu Ser Ile Gln His Asp Ala Ser His Gly
145                 150                 155                 160

gcc atc agc aag aag cct tgg gtc aac gcc ctc ttc gcc tac ggc att        528
Ala Ile Ser Lys Lys Pro Trp Val Asn Ala Leu Phe Ala Tyr Gly Ile
                165                 170                 175

gac gtc atc gga tcg tcc cgg tgg att tgg ctg cag tcg cac atc atg        576
Asp Val Ile Gly Ser Ser Arg Trp Ile Trp Leu Gln Ser His Ile Met
            180                 185                 190

cgg cac cac acc tac acc aac cag cac ggc ctc gac ctg gat gcg gag        624
Arg His His Thr Tyr Thr Asn Gln His Gly Leu Asp Leu Asp Ala Glu
        195                 200                 205

tcg gca gag ccg ttc ctg gtg ttc cac aac tac ccc gcc gca aac acc        672
Ser Ala Glu Pro Phe Leu Val Phe His Asn Tyr Pro Ala Ala Asn Thr
    210                 215                 220

gcc cga aag tgg ttc cac cgc ttc caa gct tgg tac atg tac ctt gtg        720
Ala Arg Lys Trp Phe His Arg Phe Gln Ala Trp Tyr Met Tyr Leu Val
225                 230                 235                 240

ctg ggg gca tac ggg gta tcg ctg gtg tac aac ccg ctc tac att ttc        768
Leu Gly Ala Tyr Gly Val Ser Leu Val Tyr Asn Pro Leu Tyr Ile Phe
                245                 250                 255

cgg atg cag cac aat gac acc atc cca gag tct gtc acg gcc atg cgg        816
Arg Met Gln His Asn Asp Thr Ile Pro Glu Ser Val Thr Ala Met Arg
            260                 265                 270

gag aat ggc ttt ctg cgg cgc tac cgc aca ctt gca ttc gtg atg cga        864
Glu Asn Gly Phe Leu Arg Arg Tyr Arg Thr Leu Ala Phe Val Met Arg
        275                 280                 285
```

```
gct ttc ttc atc ttc cgg acc gca ttc ttg ccc tgg tac ctc act ggg       912
Ala Phe Phe Ile Phe Arg Thr Ala Phe Leu Pro Trp Tyr Leu Thr Gly
    290                 295                 300

acc tca ttg ctg atc acc att cct ctg gtg ccc act gca act ggt gcc       960
Thr Ser Leu Leu Ile Thr Ile Pro Leu Val Pro Thr Ala Thr Gly Ala
305                 310                 315                 320

ttc ttg acg ttc ttc ttc att ttg tcc cac aat ttt gat ggc tcc gaa      1008
Phe Leu Thr Phe Phe Phe Ile Leu Ser His Asn Phe Asp Gly Ser Glu
                325                 330                 335

cgg atc ccc gac aag aac tgc aag gtt aag agc tct gag aag gac gtt      1056
Arg Ile Pro Asp Lys Asn Cys Lys Val Lys Ser Ser Glu Lys Asp Val
            340                 345                 350

gag gct gac caa att gac tgg tat cgg gcg cag gtg gag acg tcc tcc      1104
Glu Ala Asp Gln Ile Asp Trp Tyr Arg Ala Gln Val Glu Thr Ser Ser
        355                 360                 365

aca tac ggt ggc ccc atc gcc atg ttc ttc act ggc ggt ctc aat ttc      1152
Thr Tyr Gly Gly Pro Ile Ala Met Phe Phe Thr Gly Gly Leu Asn Phe
    370                 375                 380

cag atc gag cac cac ctc ttt ccc cgg atg tcg tct tgg cac tac ccc      1200
Gln Ile Glu His His Leu Phe Pro Arg Met Ser Ser Trp His Tyr Pro
385                 390                 395                 400

ttc gtc cag cag gcg gtc cgg gag tgt tgc gaa cgc cat gga gtg cga      1248
Phe Val Gln Gln Ala Val Arg Glu Cys Cys Glu Arg His Gly Val Arg
                405                 410                 415

tat gtt ttc tac cct acc atc gtc ggc aac atc atc tcc acc ctg aag      1296
Tyr Val Phe Tyr Pro Thr Ile Val Gly Asn Ile Ile Ser Thr Leu Lys
            420                 425                 430

tac atg cat aag gtg ggt gtc gtc cac tgc gtg aag gac gca cag gat      1344
Tyr Met His Lys Val Gly Val Val His Cys Val Lys Asp Ala Gln Asp
        435                 440                 445

tcc tga                                                              1350
Ser
```

<210> 66
<211> 449
<212> PRT
<213> Euglena gracilis

<400> 66

```
Met Ala Leu Ser Leu Thr Thr Glu Gln Leu Leu Glu Arg Pro Asp Leu
1               5                   10                  15

Val Ala Ile Asp Gly Ile Leu Tyr Asp Leu Glu Gly Leu Ala Lys Val
            20                  25                  30

His Pro Gly Gly Asp Leu Ile Leu Ala Ser Gly Ala Ser Asp Ala Ser
        35                  40                  45

Pro Leu Phe Tyr Ser Met His Pro Tyr Val Lys Pro Glu Asn Ser Lys
```

```
    50                  55                  60

Leu Leu Gln Gln Phe Val Arg Gly Lys His Asp Arg Thr Ser Lys Asp
65                  70                  75                  80

Ile Val Tyr Thr Tyr Asp Ser Pro Phe Ala Gln Asp Val Lys Arg Thr
                85                  90                  95

Met Arg Glu Val Met Lys Gly Arg Asn Trp Tyr Ala Thr Pro Gly Phe
            100                 105                 110

Trp Leu Arg Thr Val Gly Ile Ile Ala Val Thr Ala Phe Cys Glu Trp
        115                 120                 125

His Trp Ala Thr Thr Gly Met Val Leu Trp Gly Leu Leu Thr Gly Phe
    130                 135                 140

Met His Met Gln Ile Gly Leu Ser Ile Gln His Asp Ala Ser His Gly
145                 150                 155                 160

Ala Ile Ser Lys Lys Pro Trp Val Asn Ala Leu Phe Ala Tyr Gly Ile
                165                 170                 175

Asp Val Ile Gly Ser Ser Arg Trp Ile Trp Leu Gln Ser His Ile Met
            180                 185                 190

Arg His His Thr Tyr Thr Asn Gln His Gly Leu Asp Leu Asp Ala Glu
        195                 200                 205

Ser Ala Glu Pro Phe Leu Val Phe His Asn Tyr Pro Ala Ala Asn Thr
    210                 215                 220

Ala Arg Lys Trp Phe His Arg Phe Gln Ala Trp Tyr Met Tyr Leu Val
225                 230                 235                 240

Leu Gly Ala Tyr Gly Val Ser Leu Val Tyr Asn Pro Leu Tyr Ile Phe
                245                 250                 255

Arg Met Gln His Asn Asp Thr Ile Pro Glu Ser Val Thr Ala Met Arg
            260                 265                 270

Glu Asn Gly Phe Leu Arg Arg Tyr Arg Thr Leu Ala Phe Val Met Arg
        275                 280                 285

Ala Phe Phe Ile Phe Arg Thr Ala Phe Leu Pro Trp Tyr Leu Thr Gly
    290                 295                 300

Thr Ser Leu Leu Ile Thr Ile Pro Leu Val Pro Thr Ala Thr Gly Ala
305                 310                 315                 320
```

```
Phe Leu Thr Phe Phe Phe Ile Leu Ser His Asn Phe Asp Gly Ser Glu
                325                 330                 335

Arg Ile Pro Asp Lys Asn Cys Lys Val Lys Ser Ser Glu Lys Asp Val
            340                 345                 350

Glu Ala Asp Gln Ile Asp Trp Tyr Arg Ala Gln Val Glu Thr Ser Ser
        355                 360                 365

Thr Tyr Gly Gly Pro Ile Ala Met Phe Phe Thr Gly Gly Leu Asn Phe
    370                 375                 380

Gln Ile Glu His His Leu Phe Pro Arg Met Ser Ser Trp His Tyr Pro
385                 390                 395                 400

Phe Val Gln Gln Ala Val Arg Glu Cys Cys Glu Arg His Gly Val Arg
                405                 410                 415

Tyr Val Phe Tyr Pro Thr Ile Val Gly Asn Ile Ile Ser Thr Leu Lys
            420                 425                 430

Tyr Met His Lys Val Gly Val Val His Cys Val Lys Asp Ala Gln Asp
        435                 440                 445

Ser
```

<210> 67
<211> 1350
<212> DNA
<213> Euglena gracilis

<220>
<221> CDS
<222> (1)..(1350)
<223> synthetic delta-5 desaturase (codon-optimized for Yarrowia lipolytica)

<300>
<302> DELTA-5 DESATURASE AND ITS USE IN MAKING POLYUNSATURATED FATTY ACIDS

<310> WO 2007/136671
<311> 2007-05-16
<312> 2007-11-29
<313> (1)..(1350)

<300>
<302> DELTA-5 DESATURASE AND ITS USE IN MAKING POLYUNSATURATED FATTY ACIDS

<310> US 2007-0292924-A1
<311> 2007-05-15
<312> 2007-12-20
<313> (1)..(1350)

<400> 67

atg gct ctc tcc ctt act acc gag cag ctg ctc gag cga ccc gac ctg 48
Met Ala Leu Ser Leu Thr Thr Glu Gln Leu Leu Glu Arg Pro Asp Leu
1 5 10 15

gtt gcc atc gac ggc att ctc tac gat ctg gaa ggt ctt gcc aag gtc 96
Val Ala Ile Asp Gly Ile Leu Tyr Asp Leu Glu Gly Leu Ala Lys Val
20 25 30

cat ccc gga ggc gac ttg atc ctc gct tct ggt gcc tcc gat gct tct 144
His Pro Gly Gly Asp Leu Ile Leu Ala Ser Gly Ala Ser Asp Ala Ser
35 40 45

cct ctg ttc tac tcc atg cac cct tac gtc aag ccc gag aac tcg aag 192
Pro Leu Phe Tyr Ser Met His Pro Tyr Val Lys Pro Glu Asn Ser Lys
50 55 60

ctg ctt caa cag ttc gtg cga ggc aag cac gac cga acc tcc aag gac 240
Leu Leu Gln Gln Phe Val Arg Gly Lys His Asp Arg Thr Ser Lys Asp
65 70 75 80

att gtc tac acc tac gac tct ccc ttt gca cag gac gtc aag cga act 288
Ile Val Tyr Thr Tyr Asp Ser Pro Phe Ala Gln Asp Val Lys Arg Thr
85 90 95

atg cga gag gtc atg aaa ggt cgg aac tgg tat gcc aca cct gga ttc 336
Met Arg Glu Val Met Lys Gly Arg Asn Trp Tyr Ala Thr Pro Gly Phe
100 105 110

tgg ctg cga acc gtt ggc atc att gct gtc acc gcc ttt tgc gag tgg 384
Trp Leu Arg Thr Val Gly Ile Ile Ala Val Thr Ala Phe Cys Glu Trp
115 120 125

cac tgg gct act acc gga atg gtg ctg tgg ggt ctc ttg act gga ttc 432
His Trp Ala Thr Thr Gly Met Val Leu Trp Gly Leu Leu Thr Gly Phe
130 135 140

atg cac atg cag atc ggc ctg tcc att cag cac gat gcc tct cat ggt 480
Met His Met Gln Ile Gly Leu Ser Ile Gln His Asp Ala Ser His Gly
145 150 155 160

gcc atc agc aaa aag ccc tgg gtc aac gct ctc ttt gcc tac ggc atc 528
Ala Ile Ser Lys Lys Pro Trp Val Asn Ala Leu Phe Ala Tyr Gly Ile
165 170 175

gac gtc att gga tcg tcc aga tgg atc tgg ctg cag tct cac atc atg 576
Asp Val Ile Gly Ser Ser Arg Trp Ile Trp Leu Gln Ser His Ile Met
180 185 190

cga cat cac acc tac acc aat cag cat ggt ctc gac ctg gat gcc gag 624
Arg His His Thr Tyr Thr Asn Gln His Gly Leu Asp Leu Asp Ala Glu
195 200 205

tcc gca gaa cca ttc ctt gtg ttc cac aac tac cct gct gcc aac act 672
Ser Ala Glu Pro Phe Leu Val Phe His Asn Tyr Pro Ala Ala Asn Thr
210 215 220

gct cga aag tgg ttt cac cga ttc cag gcc tgg tac atg tac ctc gtg 720
Ala Arg Lys Trp Phe His Arg Phe Gln Ala Trp Tyr Met Tyr Leu Val
225 230 235 240

ctt gga gcc tac ggc gtt tcg ctg gtg tac aac cct ctc tac atc ttc 768
Leu Gly Ala Tyr Gly Val Ser Leu Val Tyr Asn Pro Leu Tyr Ile Phe
245 250 255

```
cga atg cag cac aac gac acc att ccc gag tct gtc aca gcc atg cga        816
Arg Met Gln His Asn Asp Thr Ile Pro Glu Ser Val Thr Ala Met Arg
            260                 265                 270

gag aac ggc ttt ctg cga cgg tac cga acc ctt gca ttc gtt atg cga        864
Glu Asn Gly Phe Leu Arg Arg Tyr Arg Thr Leu Ala Phe Val Met Arg
        275                 280                 285

gct ttc ttc atc ttt cga acc gcc ttc ttg ccc tgg tat ctc act gga        912
Ala Phe Phe Ile Phe Arg Thr Ala Phe Leu Pro Trp Tyr Leu Thr Gly
    290                 295                 300

acc tcc ctg ctc atc acc att cct ctg gtg ccc act gct acc ggt gcc        960
Thr Ser Leu Leu Ile Thr Ile Pro Leu Val Pro Thr Ala Thr Gly Ala
305                 310                 315                 320

ttc ctc acc ttc ttt ttc atc ttg tct cac aac ttc gat ggc tcg gag       1008
Phe Leu Thr Phe Phe Phe Ile Leu Ser His Asn Phe Asp Gly Ser Glu
                325                 330                 335

cga atc ccc gac aag aac tgc aag gtc aag agc tcc gag aag gac gtt       1056
Arg Ile Pro Asp Lys Asn Cys Lys Val Lys Ser Ser Glu Lys Asp Val
            340                 345                 350

gaa gcc gat cag atc gac tgg tac aga gct cag gtg gag acc tct tcc       1104
Glu Ala Asp Gln Ile Asp Trp Tyr Arg Ala Gln Val Glu Thr Ser Ser
        355                 360                 365

acc tac ggt gga ccc att gcc atg ttc ttt act ggc ggt ctc aac ttc       1152
Thr Tyr Gly Gly Pro Ile Ala Met Phe Phe Thr Gly Gly Leu Asn Phe
    370                 375                 380

cag atc gag cat cac ctc ttt cct cga atg tcg tct tgg cac tat ccc       1200
Gln Ile Glu His His Leu Phe Pro Arg Met Ser Ser Trp His Tyr Pro
385                 390                 395                 400

ttc gtg cag caa gct gtc cga gag tgt tgc gaa cga cac gga gtt cgg       1248
Phe Val Gln Gln Ala Val Arg Glu Cys Cys Glu Arg His Gly Val Arg
                405                 410                 415

tac gtc ttc tac cct acc att gtg ggc aac atc att tcc acc ctc aag       1296
Tyr Val Phe Tyr Pro Thr Ile Val Gly Asn Ile Ile Ser Thr Leu Lys
            420                 425                 430

tac atg cac aaa gtc ggt gtg gtt cac tgt gtc aag gac gct cag gat       1344
Tyr Met His Lys Val Gly Val Val His Cys Val Lys Asp Ala Gln Asp
        435                 440                 445

tcc taa                                                               1350
Ser
```

<210> 68
<211> 449
<212> PRT
<213> Euglena gracilis

<400> 68

```
Met Ala Leu Ser Leu Thr Thr Glu Gln Leu Leu Glu Arg Pro Asp Leu
1               5                   10                  15
```

```
Val Ala Ile Asp Gly Ile Leu Tyr Asp Leu Glu Gly Leu Ala Lys Val
            20                  25                  30

His Pro Gly Gly Asp Leu Ile Leu Ala Ser Gly Ala Ser Asp Ala Ser
        35                  40                  45

Pro Leu Phe Tyr Ser Met His Pro Tyr Val Lys Pro Glu Asn Ser Lys
    50                  55                  60

Leu Leu Gln Gln Phe Val Arg Gly Lys His Asp Arg Thr Ser Lys Asp
65                  70                  75                  80

Ile Val Tyr Thr Tyr Asp Ser Pro Phe Ala Gln Asp Val Lys Arg Thr
                85                  90                  95

Met Arg Glu Val Met Lys Gly Arg Asn Trp Tyr Ala Thr Pro Gly Phe
            100                 105                 110

Trp Leu Arg Thr Val Gly Ile Ile Ala Val Thr Ala Phe Cys Glu Trp
        115                 120                 125

His Trp Ala Thr Thr Gly Met Val Leu Trp Gly Leu Leu Thr Gly Phe
    130                 135                 140

Met His Met Gln Ile Gly Leu Ser Ile Gln His Asp Ala Ser His Gly
145                 150                 155                 160

Ala Ile Ser Lys Lys Pro Trp Val Asn Ala Leu Phe Ala Tyr Gly Ile
                165                 170                 175

Asp Val Ile Gly Ser Ser Arg Trp Ile Trp Leu Gln Ser His Ile Met
            180                 185                 190

Arg His His Thr Tyr Thr Asn Gln His Gly Leu Asp Leu Asp Ala Glu
        195                 200                 205

Ser Ala Glu Pro Phe Leu Val Phe His Asn Tyr Pro Ala Ala Asn Thr
    210                 215                 220

Ala Arg Lys Trp Phe His Arg Phe Gln Ala Trp Tyr Met Tyr Leu Val
225                 230                 235                 240

Leu Gly Ala Tyr Gly Val Ser Leu Val Tyr Asn Pro Leu Tyr Ile Phe
                245                 250                 255

Arg Met Gln His Asn Asp Thr Ile Pro Glu Ser Val Thr Ala Met Arg
            260                 265                 270

Glu Asn Gly Phe Leu Arg Arg Tyr Arg Thr Leu Ala Phe Val Met Arg
```

```
            275                 280                 285

Ala Phe Phe Ile Phe Arg Thr Ala Phe Leu Pro Trp Tyr Leu Thr Gly
        290                 295                 300

Thr Ser Leu Leu Ile Thr Ile Pro Leu Val Pro Thr Ala Thr Gly Ala
305                 310                 315                 320

Phe Leu Thr Phe Phe Phe Ile Leu Ser His Asn Phe Asp Gly Ser Glu
                325                 330                 335

Arg Ile Pro Asp Lys Asn Cys Lys Val Lys Ser Ser Glu Lys Asp Val
            340                 345                 350

Glu Ala Asp Gln Ile Asp Trp Tyr Arg Ala Gln Val Glu Thr Ser Ser
        355                 360                 365

Thr Tyr Gly Gly Pro Ile Ala Met Phe Phe Thr Gly Gly Leu Asn Phe
    370                 375                 380

Gln Ile Glu His His Leu Phe Pro Arg Met Ser Ser Trp His Tyr Pro
385                 390                 395                 400

Phe Val Gln Gln Ala Val Arg Glu Cys Cys Glu Arg His Gly Val Arg
                405                 410                 415

Tyr Val Phe Tyr Pro Thr Ile Val Gly Asn Ile Ile Ser Thr Leu Lys
            420                 425                 430

Tyr Met His Lys Val Gly Val Val His Cys Val Lys Asp Ala Gln Asp
        435                 440                 445

Ser
```

<210> 69
<211> 1350
<212> DNA
<213> Euglena gracilis

<220>
<221> CDS
<222> (1)..(1350)
<223> mutant delta-5 desaturase

<400> 69

```
atg gct ctc agt ctt acc aca gaa cag ctg tta gaa cgc cct gat ttg        48
Met Ala Leu Ser Leu Thr Thr Glu Gln Leu Leu Glu Arg Pro Asp Leu
1               5                   10                  15

gtt gcg att gat ggc atc ctc tac gac ctt gaa ggg ctt gcc aaa gtt        96
Val Ala Ile Asp Gly Ile Leu Tyr Asp Leu Glu Gly Leu Ala Lys Val
```

```
            20                  25                  30

cat cca gga tcc gat ttg att ctc gct tct ggt gcc tct gat gcc tcc        144
His Pro Gly Ser Asp Leu Ile Leu Ala Ser Gly Ala Ser Asp Ala Ser
        35                  40                  45

cct ctc ttt tat tca atg cat cca tac gtc aaa ccg gag aac tcc aaa        192
Pro Leu Phe Tyr Ser Met His Pro Tyr Val Lys Pro Glu Asn Ser Lys
    50                  55                  60

ttg ctt caa cag ttc gtc cga ggg aag cat gac cgc acc tcg aag gac        240
Leu Leu Gln Gln Phe Val Arg Gly Lys His Asp Arg Thr Ser Lys Asp
65                  70                  75                  80

att gtc tac acg tat gat tct ccc ttc gca caa gac gtt aag cgg aca        288
Ile Val Tyr Thr Tyr Asp Ser Pro Phe Ala Gln Asp Val Lys Arg Thr
                85                  90                  95

atg cgc gag gtg atg aaa ggg agg aac tgg tac gca acc cct ggc ttc        336
Met Arg Glu Val Met Lys Gly Arg Asn Trp Tyr Ala Thr Pro Gly Phe
            100                 105                 110

tgg ctg cgc acc gtt ggg atc atc gcc gtg acg gcc ttt tgc gag tgg        384
Trp Leu Arg Thr Val Gly Ile Ile Ala Val Thr Ala Phe Cys Glu Trp
        115                 120                 125

cac tgg gct acc acg ggg atg gtg ctg tgg ggc ctg ttg act gga ttc        432
His Trp Ala Thr Thr Gly Met Val Leu Trp Gly Leu Leu Thr Gly Phe
    130                 135                 140

atg cac atg cag atc ggc tta tcc atc cag cat gat gcg tcc cac ggg        480
Met His Met Gln Ile Gly Leu Ser Ile Gln His Asp Ala Ser His Gly
145                 150                 155                 160

gcc atc agc aag aag cct tgg gtc aac gcc ctc ttc gcc tac ggc att        528
Ala Ile Ser Lys Lys Pro Trp Val Asn Ala Leu Phe Ala Tyr Gly Ile
                165                 170                 175

gac gtc atc gga tcg tcc cgg tgg att tgg ctg cag tcg cac atc atg        576
Asp Val Ile Gly Ser Ser Arg Trp Ile Trp Leu Gln Ser His Ile Met
            180                 185                 190

cgg cac cac acc tac acc aac cag cac ggc ctc gac ctg gat gcg gag        624
Arg His His Thr Tyr Thr Asn Gln His Gly Leu Asp Leu Asp Ala Glu
        195                 200                 205

tcg gca gag ccg ttc ctg gtg ttc cac aac tac ccc gcc gca aac acc        672
Ser Ala Glu Pro Phe Leu Val Phe His Asn Tyr Pro Ala Ala Asn Thr
    210                 215                 220

gcc cga aag tgg ttc cac cgc ttc cag gct tgg tac atg tac ctt gtg        720
Ala Arg Lys Trp Phe His Arg Phe Gln Ala Trp Tyr Met Tyr Leu Val
225                 230                 235                 240

ctg ggg gca tac ggg gta tcg ctg gtg tac aac ccg ctc tac att ttc        768
Leu Gly Ala Tyr Gly Val Ser Leu Val Tyr Asn Pro Leu Tyr Ile Phe
                245                 250                 255

cgg atg cag cac aat gac acc atc cca gag tct gtc acg gcc atg cgg        816
Arg Met Gln His Asn Asp Thr Ile Pro Glu Ser Val Thr Ala Met Arg
            260                 265                 270

gaa aat ggc ttt ctg cgg cgc tac cgc aca ctt gca ttc gtg atg cga        864
Glu Asn Gly Phe Leu Arg Arg Tyr Arg Thr Leu Ala Phe Val Met Arg
        275                 280                 285
```

```
gct ttc ttc atc ttc cgg acc gca ttc ttg ccc tgg tac ctc act ggg        912
Ala Phe Phe Ile Phe Arg Thr Ala Phe Leu Pro Trp Tyr Leu Thr Gly
    290                 295                 300

acc tca ttg ctg atc acc att cct ctg gtg ccc acc gca act ggt gcc        960
Thr Ser Leu Leu Ile Thr Ile Pro Leu Val Pro Thr Ala Thr Gly Ala
305                 310                 315                 320

ttc ttg acg ttc ttc ttc att ttg tcc cac aat ttt gat ggc tcc gaa       1008
Phe Leu Thr Phe Phe Phe Ile Leu Ser His Asn Phe Asp Gly Ser Glu
                325                 330                 335

cgg atc ccc gac aag aac tgc aag gtt aag cga tct gag aag gac gtt       1056
Arg Ile Pro Asp Lys Asn Cys Lys Val Lys Arg Ser Glu Lys Asp Val
            340                 345                 350

gag gct gac caa att gac tgg tat cgg gcg cag gtg gag acg tcc tcc       1104
Glu Ala Asp Gln Ile Asp Trp Tyr Arg Ala Gln Val Glu Thr Ser Ser
        355                 360                 365

aca tac ggt ggc ccc atc gcc atg ttc ttc act ggc ggt ctc aat ttc       1152
Thr Tyr Gly Gly Pro Ile Ala Met Phe Phe Thr Gly Gly Leu Asn Phe
    370                 375                 380

cag atc gag cac cac ctc ttt ccc cgg atg tcg tct tgg cac tac ccc       1200
Gln Ile Glu His His Leu Phe Pro Arg Met Ser Ser Trp His Tyr Pro
385                 390                 395                 400

ttc gtc cag cag gcg gtc cgg gag tgt tgc gaa cga cat gga gtg cga       1248
Phe Val Gln Gln Ala Val Arg Glu Cys Cys Glu Arg His Gly Val Arg
                405                 410                 415

tat gtt ttc tac cct acc atc gtc ggc aac atc atc tcc acc ctg aag       1296
Tyr Val Phe Tyr Pro Thr Ile Val Gly Asn Ile Ile Ser Thr Leu Lys
            420                 425                 430

tac atg cat aag gtg ggt gtc gtc cac tgc gtg aag gac gca cag gat       1344
Tyr Met His Lys Val Gly Val Val His Cys Val Lys Asp Ala Gln Asp
        435                 440                 445

tcc taa                                                               1350
Ser
```

<210> 70
<211> 449
<212> PRT
<213> Euglena gracilis

<400> 70

```
Met Ala Leu Ser Leu Thr Thr Glu Gln Leu Leu Glu Arg Pro Asp Leu
1               5                   10                  15

Val Ala Ile Asp Gly Ile Leu Tyr Asp Leu Glu Gly Leu Ala Lys Val
            20                  25                  30

His Pro Gly Ser Asp Leu Ile Leu Ala Ser Gly Ala Ser Asp Ala Ser
        35                  40                  45
```

```
Pro Leu Phe Tyr Ser Met His Pro Tyr Val Lys Pro Glu Asn Ser Lys
    50                  55                  60

Leu Leu Gln Gln Phe Val Arg Gly Lys His Asp Arg Thr Ser Lys Asp
65                  70                  75                  80

Ile Val Tyr Thr Tyr Asp Ser Pro Phe Ala Gln Asp Val Lys Arg Thr
                85                  90                  95

Met Arg Glu Val Met Lys Gly Arg Asn Trp Tyr Ala Thr Pro Gly Phe
            100                 105                 110

Trp Leu Arg Thr Val Gly Ile Ile Ala Val Thr Ala Phe Cys Glu Trp
        115                 120                 125

His Trp Ala Thr Thr Gly Met Val Leu Trp Gly Leu Leu Thr Gly Phe
    130                 135                 140

Met His Met Gln Ile Gly Leu Ser Ile Gln His Asp Ala Ser His Gly
145                 150                 155                 160

Ala Ile Ser Lys Lys Pro Trp Val Asn Ala Leu Phe Ala Tyr Gly Ile
                165                 170                 175

Asp Val Ile Gly Ser Ser Arg Trp Ile Trp Leu Gln Ser His Ile Met
            180                 185                 190

Arg His His Thr Tyr Thr Asn Gln His Gly Leu Asp Leu Asp Ala Glu
        195                 200                 205

Ser Ala Glu Pro Phe Leu Val Phe His Asn Tyr Pro Ala Ala Asn Thr
    210                 215                 220

Ala Arg Lys Trp Phe His Arg Phe Gln Ala Trp Tyr Met Tyr Leu Val
225                 230                 235                 240

Leu Gly Ala Tyr Gly Val Ser Leu Val Tyr Asn Pro Leu Tyr Ile Phe
                245                 250                 255

Arg Met Gln His Asn Asp Thr Ile Pro Glu Ser Val Thr Ala Met Arg
            260                 265                 270

Glu Asn Gly Phe Leu Arg Arg Tyr Arg Thr Leu Ala Phe Val Met Arg
        275                 280                 285

Ala Phe Phe Ile Phe Arg Thr Ala Phe Leu Pro Trp Tyr Leu Thr Gly
    290                 295                 300

Thr Ser Leu Leu Ile Thr Ile Pro Leu Val Pro Thr Ala Thr Gly Ala
```

```
305                 310                 315                 320

Phe Leu Thr Phe Phe Phe Ile Leu Ser His Asn Phe Asp Gly Ser Glu
                325                 330                 335

Arg Ile Pro Asp Lys Asn Cys Lys Val Lys Arg Ser Glu Lys Asp Val
            340                 345                 350

Glu Ala Asp Gln Ile Asp Trp Tyr Arg Ala Gln Val Glu Thr Ser Ser
        355                 360                 365

Thr Tyr Gly Gly Pro Ile Ala Met Phe Phe Thr Gly Gly Leu Asn Phe
    370                 375                 380

Gln Ile Glu His His Leu Phe Pro Arg Met Ser Ser Trp His Tyr Pro
385                 390                 395                 400

Phe Val Gln Gln Ala Val Arg Glu Cys Cys Glu Arg His Gly Val Arg
                405                 410                 415

Tyr Val Phe Tyr Pro Thr Ile Val Gly Asn Ile Ile Ser Thr Leu Lys
            420                 425                 430

Tyr Met His Lys Val Gly Val Val His Cys Val Lys Asp Ala Gln Asp
        435                 440                 445

Ser
```

<210> 71
<211> 1350
<212> DNA
<213> Euglena gracilis

<220>
<221> CDS
<222> (1)..(1350)
<223> synthetic mutant delta-5 desaturase (codon-optimized for Yarrowia lipolytica)

<400> 71

```
atg gct ctc tcc ctt act acc gag cag ctg ctc gag cga ccc gac ctg        48
Met Ala Leu Ser Leu Thr Thr Glu Gln Leu Leu Glu Arg Pro Asp Leu
1               5                   10                  15

gtt gcc atc gac ggc att ctc tac gat ctg gaa ggt ctt gcc aag gtc        96
Val Ala Ile Asp Gly Ile Leu Tyr Asp Leu Glu Gly Leu Ala Lys Val
            20                  25                  30

cat ccc gga tcc gac ttg atc ctc gct tct ggt gcc tcc gat gct tct       144
His Pro Gly Ser Asp Leu Ile Leu Ala Ser Gly Ala Ser Asp Ala Ser
        35                  40                  45

cct ctg ttc tac tcc atg cac cct tac gtc aag ccc gag aac tcg aag       192
```

```
Pro Leu Phe Tyr Ser Met His Pro Tyr Val Lys Pro Glu Asn Ser Lys
    50                  55                  60

ctg ctt caa cag ttc gtg cga ggc aag cac gac cga acc tcc aag gac      240
Leu Leu Gln Gln Phe Val Arg Gly Lys His Asp Arg Thr Ser Lys Asp
65                  70                  75                  80

att gtc tac acc tac gac tct ccc ttt gca cag gac gtc aag cga act      288
Ile Val Tyr Thr Tyr Asp Ser Pro Phe Ala Gln Asp Val Lys Arg Thr
                85                  90                  95

atg cga gag gtc atg aaa ggt cgg aac tgg tat gcc aca cct gga ttc      336
Met Arg Glu Val Met Lys Gly Arg Asn Trp Tyr Ala Thr Pro Gly Phe
            100                 105                 110

tgg ctg cga acc gtt ggc atc att gct gtc acc gcc ttt tgc gag tgg      384
Trp Leu Arg Thr Val Gly Ile Ile Ala Val Thr Ala Phe Cys Glu Trp
        115                 120                 125

cac tgg gct act acc gga atg gtg ctg tgg ggt ctc ttg act gga ttc      432
His Trp Ala Thr Thr Gly Met Val Leu Trp Gly Leu Leu Thr Gly Phe
    130                 135                 140

atg cac atg cag atc ggc ctg tcc att cag cac gat gcc tct cat ggt      480
Met His Met Gln Ile Gly Leu Ser Ile Gln His Asp Ala Ser His Gly
145                 150                 155                 160

gcc atc agc aaa aag ccc tgg gtc aac gct ctc ttt gcc tac ggc atc      528
Ala Ile Ser Lys Lys Pro Trp Val Asn Ala Leu Phe Ala Tyr Gly Ile
                165                 170                 175

gac gtc att gga tcg tcc aga tgg atc tgg ctg cag tct cac atc atg      576
Asp Val Ile Gly Ser Ser Arg Trp Ile Trp Leu Gln Ser His Ile Met
            180                 185                 190

cga cat cac acc tac acc aat cag cat ggt ctc gac ctg gat gcc gag      624
Arg His His Thr Tyr Thr Asn Gln His Gly Leu Asp Leu Asp Ala Glu
        195                 200                 205

tcc gca gaa cca ttc ctt gtg ttc cac aac tac cct gct gcc aac act      672
Ser Ala Glu Pro Phe Leu Val Phe His Asn Tyr Pro Ala Ala Asn Thr
    210                 215                 220

gct cga aag tgg ttt cac cga ttc cag gcc tgg tac atg tac ctc gtg      720
Ala Arg Lys Trp Phe His Arg Phe Gln Ala Trp Tyr Met Tyr Leu Val
225                 230                 235                 240

ctt gga gcc tac ggc gtt tcg ctg gtg tac aac cct ctc tac atc ttc      768
Leu Gly Ala Tyr Gly Val Ser Leu Val Tyr Asn Pro Leu Tyr Ile Phe
                245                 250                 255

cga atg cag cac aac gac acc att ccc gag tct gtc aca gcc atg cga      816
Arg Met Gln His Asn Asp Thr Ile Pro Glu Ser Val Thr Ala Met Arg
            260                 265                 270

gag aac ggc ttt ctg cga cgg tac cga acc ctt gca ttc gtt atg cga      864
Glu Asn Gly Phe Leu Arg Arg Tyr Arg Thr Leu Ala Phe Val Met Arg
        275                 280                 285

gct ttc ttc atc ttt cga acc gcc ttc ttg ccc tgg tat ctc act gga      912
Ala Phe Phe Ile Phe Arg Thr Ala Phe Leu Pro Trp Tyr Leu Thr Gly
    290                 295                 300

acc tcc ctg ctc atc acc att cct ctg gtg ccc act gct acc ggt gcc      960
Thr Ser Leu Leu Ile Thr Ile Pro Leu Val Pro Thr Ala Thr Gly Ala
```

```
305                 310                 315                 320

ttc ctc acc ttc ttt ttc atc ttg tct cac aac ttc gat ggc tcg gag      1008
Phe Leu Thr Phe Phe Phe Ile Leu Ser His Asn Phe Asp Gly Ser Glu
                325                 330                 335

cga atc ccc gac aag aac tgc aag gtc aag agc tcc gag aag gac gtt      1056
Arg Ile Pro Asp Lys Asn Cys Lys Val Lys Ser Ser Glu Lys Asp Val
            340                 345                 350

gaa gcc gat cag atc gac tgg tac aga gct cag gtg gag acc tct tcc      1104
Glu Ala Asp Gln Ile Asp Trp Tyr Arg Ala Gln Val Glu Thr Ser Ser
        355                 360                 365

acc tac ggt gga ccc att gcc atg ttc ttt act ggc ggt ctc aac ttc      1152
Thr Tyr Gly Gly Pro Ile Ala Met Phe Phe Thr Gly Gly Leu Asn Phe
    370                 375                 380

cag atc gag cat cac ctc ttt cct cga atg tcg tct tgg cac tat ccc      1200
Gln Ile Glu His His Leu Phe Pro Arg Met Ser Ser Trp His Tyr Pro
385                 390                 395                 400

ttc gtg cag caa gct gtc cga gag tgt tgc gaa cga cac gga gtt cgg      1248
Phe Val Gln Gln Ala Val Arg Glu Cys Cys Glu Arg His Gly Val Arg
                405                 410                 415

tac gtc ttc tac cct acc att gtg ggc aac atc att tcc acc ctc aag      1296
Tyr Val Phe Tyr Pro Thr Ile Val Gly Asn Ile Ile Ser Thr Leu Lys
            420                 425                 430

tac atg cac aaa gtc ggt gtg gtt cac tgt gtc aag gac gct cag gat      1344
Tyr Met His Lys Val Gly Val Val His Cys Val Lys Asp Ala Gln Asp
        435                 440                 445

tcc taa                                                              1350
Ser
```

<210> 72
<211> 449
<212> PRT
<213> Euglena gracilis

<400> 72

```
Met Ala Leu Ser Leu Thr Thr Glu Gln Leu Leu Glu Arg Pro Asp Leu
1               5                   10                  15

Val Ala Ile Asp Gly Ile Leu Tyr Asp Leu Glu Gly Leu Ala Lys Val
            20                  25                  30

His Pro Gly Ser Asp Leu Ile Leu Ala Ser Gly Ala Ser Asp Ala Ser
        35                  40                  45

Pro Leu Phe Tyr Ser Met His Pro Tyr Val Lys Pro Glu Asn Ser Lys
    50                  55                  60

Leu Leu Gln Gln Phe Val Arg Gly Lys His Asp Arg Thr Ser Lys Asp
65                  70                  75                  80
```

```
Ile Val Tyr Thr Tyr Asp Ser Pro Phe Ala Gln Asp Val Lys Arg Thr
                85                  90                  95

Met Arg Glu Val Met Lys Gly Arg Asn Trp Tyr Ala Thr Pro Gly Phe
            100                 105                 110

Trp Leu Arg Thr Val Gly Ile Ile Ala Val Thr Ala Phe Cys Glu Trp
        115                 120                 125

His Trp Ala Thr Thr Gly Met Val Leu Trp Gly Leu Leu Thr Gly Phe
    130                 135                 140

Met His Met Gln Ile Gly Leu Ser Ile Gln His Asp Ala Ser His Gly
145                 150                 155                 160

Ala Ile Ser Lys Lys Pro Trp Val Asn Ala Leu Phe Ala Tyr Gly Ile
                165                 170                 175

Asp Val Ile Gly Ser Ser Arg Trp Ile Trp Leu Gln Ser His Ile Met
            180                 185                 190

Arg His His Thr Tyr Thr Asn Gln His Gly Leu Asp Leu Asp Ala Glu
        195                 200                 205

Ser Ala Glu Pro Phe Leu Val Phe His Asn Tyr Pro Ala Ala Asn Thr
    210                 215                 220

Ala Arg Lys Trp Phe His Arg Phe Gln Ala Trp Tyr Met Tyr Leu Val
225                 230                 235                 240

Leu Gly Ala Tyr Gly Val Ser Leu Val Tyr Asn Pro Leu Tyr Ile Phe
                245                 250                 255

Arg Met Gln His Asn Asp Thr Ile Pro Glu Ser Val Thr Ala Met Arg
            260                 265                 270

Glu Asn Gly Phe Leu Arg Arg Tyr Arg Thr Leu Ala Phe Val Met Arg
        275                 280                 285

Ala Phe Phe Ile Phe Arg Thr Ala Phe Leu Pro Trp Tyr Leu Thr Gly
    290                 295                 300

Thr Ser Leu Leu Ile Thr Ile Pro Leu Val Pro Thr Ala Thr Gly Ala
305                 310                 315                 320

Phe Leu Thr Phe Phe Phe Ile Leu Ser His Asn Phe Asp Gly Ser Glu
                325                 330                 335
```

Arg Ile Pro Asp Lys Asn Cys Lys Val Lys Ser Ser Glu Lys Asp Val
340 345 350

Glu Ala Asp Gln Ile Asp Trp Tyr Arg Ala Gln Val Glu Thr Ser Ser
355 360 365

Thr Tyr Gly Gly Pro Ile Ala Met Phe Phe Thr Gly Gly Leu Asn Phe
370 375 380

Gln Ile Glu His His Leu Phe Pro Arg Met Ser Ser Trp His Tyr Pro
385 390 395 400

Phe Val Gln Gln Ala Val Arg Glu Cys Cys Glu Arg His Gly Val Arg
405 410 415

Tyr Val Phe Tyr Pro Thr Ile Val Gly Asn Ile Ile Ser Thr Leu Lys
420 425 430

Tyr Met His Lys Val Gly Val Val His Cys Val Lys Asp Ala Gln Asp
435 440 445

Ser

<210> 73
<211> 1392
<212> DNA
<213> Peridinium sp. CCMP626

<220>
<221> CDS
<222> (1)..(1392)
<223> delta-5 desaturase

<300>
<302> DELTA-5 DESATURASE AND ITS USE IN MAKING POLYUNSATURATED FATTY ACIDS

<310> WO 2007/136646
<311> 2007-05-16
<312> 2007-11-29
<313> (1)..(1392)

<300>
<302> DELTA-5 DESATURASE AND ITS USE IN MAKING POLYUNSATURATED FATTY ACIDS

<310> US 2007-0271632-A1
<311> 2007-05-15
<312> 2007-11-22
<313> (1)..(1392)

<400> 73

```
atg gct cca gat gcg gac aag ttg aga cag cgc aag gcg caa tcg att      48
Met Ala Pro Asp Ala Asp Lys Leu Arg Gln Arg Lys Ala Gln Ser Ile
1               5                   10                  15

caa gac acg gct gat tcg caa gct acc gaa ctc aag att ggc acc ctg      96
```

```
Gln Asp Thr Ala Asp Ser Gln Ala Thr Glu Leu Lys Ile Gly Thr Leu
            20                  25                  30

aag ggc ttg cag ggg aca gaa atc gtc att gat gga gac att tac gat      144
Lys Gly Leu Gln Gly Thr Glu Ile Val Ile Asp Gly Asp Ile Tyr Asp
        35                  40                  45

ata aaa gac ttt gat cac ccc ggt ggt gaa tcc atc atg act ttt ggg      192
Ile Lys Asp Phe Asp His Pro Gly Gly Glu Ser Ile Met Thr Phe Gly
    50                  55                  60

gga aac gat gtc acc gcc acg tac aag atg atc cac ccc tac cac tct      240
Gly Asn Asp Val Thr Ala Thr Tyr Lys Met Ile His Pro Tyr His Ser
65                  70                  75                  80

aag cac cat ttg gag aag atg aag aaa gtg gga cga gtt ccg gac tac      288
Lys His His Leu Glu Lys Met Lys Lys Val Gly Arg Val Pro Asp Tyr
                85                  90                  95

acc tcg gaa tac aag ttt gat act ccc ttt gag cgt gaa atc aag caa      336
Thr Ser Glu Tyr Lys Phe Asp Thr Pro Phe Glu Arg Glu Ile Lys Gln
            100                 105                 110

gag gtc ttc aag att gtg cga cga ggc cgc gag ttt gga aca cct gga      384
Glu Val Phe Lys Ile Val Arg Arg Gly Arg Glu Phe Gly Thr Pro Gly
        115                 120                 125

tac ttc ttc cgg gct ttc tgc tac att gga ctt ttc ttt tac ttg cag      432
Tyr Phe Phe Arg Ala Phe Cys Tyr Ile Gly Leu Phe Phe Tyr Leu Gln
    130                 135                 140

tat ttg tgg gtc acg act ccc act acc ttt gcc ttg gcg atc ttc tat      480
Tyr Leu Trp Val Thr Thr Pro Thr Thr Phe Ala Leu Ala Ile Phe Tyr
145                 150                 155                 160

ggt gtt tcg caa gct ttc att ggt ttg aac gta caa cat gat gcc aac      528
Gly Val Ser Gln Ala Phe Ile Gly Leu Asn Val Gln His Asp Ala Asn
                165                 170                 175

cac gga gct gcc tcc aag aag cct tgg atc aat aac ttg cta gga ttg      576
His Gly Ala Ala Ser Lys Lys Pro Trp Ile Asn Asn Leu Leu Gly Leu
            180                 185                 190

ggg gct gac ttt atc gga ggt tcc aaa tgg ttg tgg atg aac cag cac      624
Gly Ala Asp Phe Ile Gly Gly Ser Lys Trp Leu Trp Met Asn Gln His
        195                 200                 205

tgg acg cac cac aca tac acc aac cac cat gag aag gat ccc gat gcc      672
Trp Thr His His Thr Tyr Thr Asn His His Glu Lys Asp Pro Asp Ala
    210                 215                 220

ttg ggc gct gaa cca atg ttg ttg ttc aat gat tat ccc ttg ggt cac      720
Leu Gly Ala Glu Pro Met Leu Leu Phe Asn Asp Tyr Pro Leu Gly His
225                 230                 235                 240

cca aag cgt act ttg att cac cac ttc cag gcc ttc tat tac ctt ttc      768
Pro Lys Arg Thr Leu Ile His His Phe Gln Ala Phe Tyr Tyr Leu Phe
                245                 250                 255

gtc ttg gcc gga tac tgg gtc tct tcg gtc ttc aac cct caa att ttg      816
Val Leu Ala Gly Tyr Trp Val Ser Ser Val Phe Asn Pro Gln Ile Leu
            260                 265                 270

gac ttg caa cac cgc ggt gct caa gcg gtt gga atg aaa atg gag aac      864
Asp Leu Gln His Arg Gly Ala Gln Ala Val Gly Met Lys Met Glu Asn
```

```
            275                 280                 285

gat tac att gcc aaa agc cga aag tat gcc atc ttc ttg cgt ctc ttg         912
Asp Tyr Ile Ala Lys Ser Arg Lys Tyr Ala Ile Phe Leu Arg Leu Leu
    290                 295                 300

tat att tac acc aac att gtc gct ccg atc caa aac caa ggc ttc tcg         960
Tyr Ile Tyr Thr Asn Ile Val Ala Pro Ile Gln Asn Gln Gly Phe Ser
305                 310                 315                 320

ttg acc gtg gtc gcc cac att ttg acc atg ggc gtc gct tcc agt ttg        1008
Leu Thr Val Val Ala His Ile Leu Thr Met Gly Val Ala Ser Ser Leu
                325                 330                 335

act ttg gcg act ctt ttt gcc ttg tcg cac aat ttt gaa aac gcg gat        1056
Thr Leu Ala Thr Leu Phe Ala Leu Ser His Asn Phe Glu Asn Ala Asp
            340                 345                 350

cgc gat ccc act tac gag gcc cgc aag gga gga gag cct gtt tgt tgg        1104
Arg Asp Pro Thr Tyr Glu Ala Arg Lys Gly Gly Glu Pro Val Cys Trp
        355                 360                 365

ttc aag tcg caa gtc gaa acc tcg tca act tac gga ggt ttc atc tcg        1152
Phe Lys Ser Gln Val Glu Thr Ser Ser Thr Tyr Gly Gly Phe Ile Ser
    370                 375                 380

ggt tgc ttg acg ggc gga ctc aac ttc caa gtg gaa cac cac ttg ttc        1200
Gly Cys Leu Thr Gly Gly Leu Asn Phe Gln Val Glu His His Leu Phe
385                 390                 395                 400

cct cgt atg agt tcg gcc tgg tac ccc tac att gcc cct act gtt cga        1248
Pro Arg Met Ser Ser Ala Trp Tyr Pro Tyr Ile Ala Pro Thr Val Arg
                405                 410                 415

gag gtt tgc aaa aag cac gga gtc aag tac gca tac tat ccc tgg gtc        1296
Glu Val Cys Lys Lys His Gly Val Lys Tyr Ala Tyr Tyr Pro Trp Val
            420                 425                 430

tgg caa aac ttg att tca act gtc aag tat ctg cat caa agc gga act        1344
Trp Gln Asn Leu Ile Ser Thr Val Lys Tyr Leu His Gln Ser Gly Thr
        435                 440                 445

gga tcc aac tgg aag aat ggc gcc aac ccc tac tcg gga aaa ttg taa        1392
Gly Ser Asn Trp Lys Asn Gly Ala Asn Pro Tyr Ser Gly Lys Leu
    450                 455                 460
```

<210> 74
<211> 463
<212> PRT
<213> Peridinium sp. CCMP626

<400> 74

Met Ala Pro Asp Ala Asp Lys Leu Arg Gln Arg Lys Ala Gln Ser Ile
1               5                   10                  15

Gln Asp Thr Ala Asp Ser Gln Ala Thr Glu Leu Lys Ile Gly Thr Leu
            20                  25                  30

Lys Gly Leu Gln Gly Thr Glu Ile Val Ile Asp Gly Asp Ile Tyr Asp
        35                  40                  45

```
Ile Lys Asp Phe Asp His Pro Gly Gly Glu Ser Ile Met Thr Phe Gly
    50                  55                  60

Gly Asn Asp Val Thr Ala Thr Tyr Lys Met Ile His Pro Tyr His Ser
65                  70                  75                  80

Lys His His Leu Glu Lys Met Lys Lys Val Gly Arg Val Pro Asp Tyr
                85                  90                  95

Thr Ser Glu Tyr Lys Phe Asp Thr Pro Phe Glu Arg Glu Ile Lys Gln
            100                 105                 110

Glu Val Phe Lys Ile Val Arg Arg Gly Arg Glu Phe Gly Thr Pro Gly
        115                 120                 125

Tyr Phe Phe Arg Ala Phe Cys Tyr Ile Gly Leu Phe Phe Tyr Leu Gln
    130                 135                 140

Tyr Leu Trp Val Thr Thr Pro Thr Thr Phe Ala Leu Ala Ile Phe Tyr
145                 150                 155                 160

Gly Val Ser Gln Ala Phe Ile Gly Leu Asn Val Gln His Asp Ala Asn
                165                 170                 175

His Gly Ala Ala Ser Lys Lys Pro Trp Ile Asn Asn Leu Leu Gly Leu
            180                 185                 190

Gly Ala Asp Phe Ile Gly Gly Ser Lys Trp Leu Trp Met Asn Gln His
        195                 200                 205

Trp Thr His His Thr Tyr Thr Asn His His Glu Lys Asp Pro Asp Ala
    210                 215                 220

Leu Gly Ala Glu Pro Met Leu Leu Phe Asn Asp Tyr Pro Leu Gly His
225                 230                 235                 240

Pro Lys Arg Thr Leu Ile His His Phe Gln Ala Phe Tyr Tyr Leu Phe
                245                 250                 255

Val Leu Ala Gly Tyr Trp Val Ser Ser Val Phe Asn Pro Gln Ile Leu
            260                 265                 270

Asp Leu Gln His Arg Gly Ala Gln Ala Val Gly Met Lys Met Glu Asn
        275                 280                 285

Asp Tyr Ile Ala Lys Ser Arg Lys Tyr Ala Ile Phe Leu Arg Leu Leu
    290                 295                 300
```

```
Tyr Ile Tyr Thr Asn Ile Val Ala Pro Ile Gln Asn Gln Gly Phe Ser
305                 310                 315                 320

Leu Thr Val Val Ala His Ile Leu Thr Met Gly Val Ala Ser Ser Leu
                325                 330                 335

Thr Leu Ala Thr Leu Phe Ala Leu Ser His Asn Phe Glu Asn Ala Asp
            340                 345                 350

Arg Asp Pro Thr Tyr Glu Ala Arg Lys Gly Gly Glu Pro Val Cys Trp
        355                 360                 365

Phe Lys Ser Gln Val Glu Thr Ser Ser Thr Tyr Gly Gly Phe Ile Ser
    370                 375                 380

Gly Cys Leu Thr Gly Gly Leu Asn Phe Gln Val Glu His His Leu Phe
385                 390                 395                 400

Pro Arg Met Ser Ser Ala Trp Tyr Pro Tyr Ile Ala Pro Thr Val Arg
                405                 410                 415

Glu Val Cys Lys Lys His Gly Val Lys Tyr Ala Tyr Tyr Pro Trp Val
            420                 425                 430

Trp Gln Asn Leu Ile Ser Thr Val Lys Tyr Leu His Gln Ser Gly Thr
        435                 440                 445

Gly Ser Asn Trp Lys Asn Gly Ala Asn Pro Tyr Ser Gly Lys Leu
    450                 455                 460
```

<210> 75
<211> 1392
<212> DNA
<213> Peridinium sp. CCMP626

<220>
<221> CDS
<222> (1)..(1392)
<223> synthetic delta-5 desaturase (codon-optimized for Yarrowia lipolytica)

<300>
<302> DELTA-5 DESATURASE AND ITS USE IN MAKING POLYUNSATURATED FATTY ACIDS

<310> WO 2007/136646
<311> 2007-05-16
<312> 2007-11-29
<313> (1)..(1392)

<300>
<302> DELTA-5 DESATURASE AND ITS USE IN MAKING POLYUNSATURATED FATTY ACIDS

<310> US 2007-0271632-A1
<311> 2007-05-15
<312> 2007-11-22
<313> (1)..(1392)

<400> 75

```
atg gct ccc gac gcc gac aag ctg cga cag cga aag gct cag tcc atc      48
Met Ala Pro Asp Ala Asp Lys Leu Arg Gln Arg Lys Ala Gln Ser Ile
1               5                   10                  15

cag gac act gcc gat tct cag gct acc gag ctc aag att ggc acc ctg      96
Gln Asp Thr Ala Asp Ser Gln Ala Thr Glu Leu Lys Ile Gly Thr Leu
            20                  25                  30

aag ggt ctc caa ggc acc gag atc gtc att gat ggc gac atc tac gac      144
Lys Gly Leu Gln Gly Thr Glu Ile Val Ile Asp Gly Asp Ile Tyr Asp
        35                  40                  45

atc aaa gac ttc gat cac cct gga ggc gaa tcc atc atg acc ttt ggt      192
Ile Lys Asp Phe Asp His Pro Gly Gly Glu Ser Ile Met Thr Phe Gly
    50                  55                  60

ggc aac gac gtt act gcc acc tac aag atg att cat ccc tac cac tcg      240
Gly Asn Asp Val Thr Ala Thr Tyr Lys Met Ile His Pro Tyr His Ser
65                  70                  75                  80

aag cat cac ctg gag aag atg aaa aag gtc ggt cga gtg ccc gac tac      288
Lys His His Leu Glu Lys Met Lys Lys Val Gly Arg Val Pro Asp Tyr
                85                  90                  95

acc tcc gag tac aag ttc gat act ccc ttc gaa cga gag atc aaa cag      336
Thr Ser Glu Tyr Lys Phe Asp Thr Pro Phe Glu Arg Glu Ile Lys Gln
            100                 105                 110

gag gtc ttc aag att gtg cga aga ggt cga gag ttt gga aca cct ggc      384
Glu Val Phe Lys Ile Val Arg Arg Gly Arg Glu Phe Gly Thr Pro Gly
        115                 120                 125

tac ttc ttt cga gcc ttc tgc tac atc ggt ctc ttc ttt tac ctg cag      432
Tyr Phe Phe Arg Ala Phe Cys Tyr Ile Gly Leu Phe Phe Tyr Leu Gln
    130                 135                 140

tat ctc tgg gtt acc act cct acc act ttc gcc ctt gct atc ttc tac      480
Tyr Leu Trp Val Thr Thr Pro Thr Thr Phe Ala Leu Ala Ile Phe Tyr
145                 150                 155                 160

ggt gtg tct cag gcc ttc att ggc ctg aac gtc cag cac gac gcc aac      528
Gly Val Ser Gln Ala Phe Ile Gly Leu Asn Val Gln His Asp Ala Asn
                165                 170                 175

cac gga gct gcc tcc aaa aag ccc tgg atc aac aat ttg ctc ggc ctg      576
His Gly Ala Ala Ser Lys Lys Pro Trp Ile Asn Asn Leu Leu Gly Leu
            180                 185                 190

ggt gcc gac ttt atc gga ggc tcc aag tgg ctc tgg atg aac cag cac      624
Gly Ala Asp Phe Ile Gly Gly Ser Lys Trp Leu Trp Met Asn Gln His
        195                 200                 205

tgg acc cat cac act tac acc aac cat cac gag aag gat ccc gac gcc      672
Trp Thr His His Thr Tyr Thr Asn His His Glu Lys Asp Pro Asp Ala
    210                 215                 220

ctg ggt gca gag cct atg ctg ctc ttc aac gac tat ccc ttg ggt cac      720
Leu Gly Ala Glu Pro Met Leu Leu Phe Asn Asp Tyr Pro Leu Gly His
225                 230                 235                 240

ccc aag cga acc ctc att cat cac ttc caa gcc ttc tac tat ctg ttt      768
```

```
Pro Lys Arg Thr Leu Ile His His Phe Gln Ala Phe Tyr Tyr Leu Phe
                245                 250                 255

gtc ctt gct ggc tac tgg gtg tct tcg gtg ttc aac cct cag atc ctg        816
Val Leu Ala Gly Tyr Trp Val Ser Ser Val Phe Asn Pro Gln Ile Leu
            260                 265                 270

gac ctc cag cac cga ggt gcc cag gct gtc ggc atg aag atg gag aac        864
Asp Leu Gln His Arg Gly Ala Gln Ala Val Gly Met Lys Met Glu Asn
        275                 280                 285

gac tac att gcc aag tct cga aag tac gct atc ttc ctg cga ctc ctg        912
Asp Tyr Ile Ala Lys Ser Arg Lys Tyr Ala Ile Phe Leu Arg Leu Leu
    290                 295                 300

tac atc tac acc aac att gtg gct ccc atc cag aac caa ggc ttt tcg        960
Tyr Ile Tyr Thr Asn Ile Val Ala Pro Ile Gln Asn Gln Gly Phe Ser
305                 310                 315                 320

ctc acc gtc gtt gct cac att ctt act atg ggt gtc gcc tcc agc ctg       1008
Leu Thr Val Val Ala His Ile Leu Thr Met Gly Val Ala Ser Ser Leu
                325                 330                 335

acc ctc gct act ctg ttc gcc ctc tcc cac aac ttc gag aac gca gat       1056
Thr Leu Ala Thr Leu Phe Ala Leu Ser His Asn Phe Glu Asn Ala Asp
            340                 345                 350

cgg gat ccc acc tac gag gct cga aag gga ggc gag cct gtc tgt tgg       1104
Arg Asp Pro Thr Tyr Glu Ala Arg Lys Gly Gly Glu Pro Val Cys Trp
        355                 360                 365

ttc aag tcg cag gtg gaa acc tcc tct act tac ggt ggc ttc att tcc       1152
Phe Lys Ser Gln Val Glu Thr Ser Ser Thr Tyr Gly Gly Phe Ile Ser
    370                 375                 380

ggt tgc ctt aca ggc gga ctc aac ttt cag gtc gag cat cac ctg ttt       1200
Gly Cys Leu Thr Gly Gly Leu Asn Phe Gln Val Glu His His Leu Phe
385                 390                 395                 400

cct cga atg tcc tct gcc tgg tac ccc tac atc gct cct acc gtt cga       1248
Pro Arg Met Ser Ser Ala Trp Tyr Pro Tyr Ile Ala Pro Thr Val Arg
                405                 410                 415

gag gtc tgc aaa aag cac ggc gtc aag tac gcc tac tat ccc tgg gtg       1296
Glu Val Cys Lys Lys His Gly Val Lys Tyr Ala Tyr Tyr Pro Trp Val
            420                 425                 430

tgg cag aac ctc atc tcg acc gtc aag tac ctg cat cag tcc gga act       1344
Trp Gln Asn Leu Ile Ser Thr Val Lys Tyr Leu His Gln Ser Gly Thr
        435                 440                 445

ggc tcg aac tgg aag aac ggt gcc aat ccc tac tct ggc aag ctg taa       1392
Gly Ser Asn Trp Lys Asn Gly Ala Asn Pro Tyr Ser Gly Lys Leu
    450                 455                 460
```

<210> 76
<211> 463
<212> PRT
<213> Peridinium sp. CCMP626

<400> 76

Met Ala Pro Asp Ala Asp Lys Leu Arg Gln Arg Lys Ala Gln Ser Ile
1               5                   10                  15

```
Gln Asp Thr Ala Asp Ser Gln Ala Thr Glu Leu Lys Ile Gly Thr Leu
            20                  25                  30

Lys Gly Leu Gln Gly Thr Glu Ile Val Ile Asp Gly Asp Ile Tyr Asp
        35                  40                  45

Ile Lys Asp Phe Asp His Pro Gly Gly Glu Ser Ile Met Thr Phe Gly
    50                  55                  60

Gly Asn Asp Val Thr Ala Thr Tyr Lys Met Ile His Pro Tyr His Ser
65                  70                  75                  80

Lys His His Leu Glu Lys Met Lys Lys Val Gly Arg Val Pro Asp Tyr
                85                  90                  95

Thr Ser Glu Tyr Lys Phe Asp Thr Pro Phe Glu Arg Glu Ile Lys Gln
            100                 105                 110

Glu Val Phe Lys Ile Val Arg Arg Gly Arg Glu Phe Gly Thr Pro Gly
        115                 120                 125

Tyr Phe Phe Arg Ala Phe Cys Tyr Ile Gly Leu Phe Phe Tyr Leu Gln
    130                 135                 140

Tyr Leu Trp Val Thr Thr Pro Thr Thr Phe Ala Leu Ala Ile Phe Tyr
145                 150                 155                 160

Gly Val Ser Gln Ala Phe Ile Gly Leu Asn Val Gln His Asp Ala Asn
                165                 170                 175

His Gly Ala Ala Ser Lys Lys Pro Trp Ile Asn Asn Leu Leu Gly Leu
            180                 185                 190

Gly Ala Asp Phe Ile Gly Gly Ser Lys Trp Leu Trp Met Asn Gln His
        195                 200                 205

Trp Thr His His Thr Tyr Thr Asn His His Glu Lys Asp Pro Asp Ala
    210                 215                 220

Leu Gly Ala Glu Pro Met Leu Leu Phe Asn Asp Tyr Pro Leu Gly His
225                 230                 235                 240

Pro Lys Arg Thr Leu Ile His His Phe Gln Ala Phe Tyr Tyr Leu Phe
                245                 250                 255

Val Leu Ala Gly Tyr Trp Val Ser Ser Val Phe Asn Pro Gln Ile Leu
            260                 265                 270
```

Asp Leu Gln His Arg Gly Ala Gln Ala Val Gly Met Lys Met Glu Asn
275 280 285

Asp Tyr Ile Ala Lys Ser Arg Lys Tyr Ala Ile Phe Leu Arg Leu Leu
290 295 300

Tyr Ile Tyr Thr Asn Ile Val Ala Pro Ile Gln Asn Gln Gly Phe Ser
305 310 315 320

Leu Thr Val Val Ala His Ile Leu Thr Met Gly Val Ala Ser Ser Leu
325 330 335

Thr Leu Ala Thr Leu Phe Ala Leu Ser His Asn Phe Glu Asn Ala Asp
340 345 350

Arg Asp Pro Thr Tyr Glu Ala Arg Lys Gly Gly Glu Pro Val Cys Trp
355 360 365

Phe Lys Ser Gln Val Glu Thr Ser Ser Thr Tyr Gly Gly Phe Ile Ser
370 375 380

Gly Cys Leu Thr Gly Gly Leu Asn Phe Gln Val Glu His His Leu Phe
385 390 395 400

Pro Arg Met Ser Ser Ala Trp Tyr Pro Tyr Ile Ala Pro Thr Val Arg
405 410 415

Glu Val Cys Lys Lys His Gly Val Lys Tyr Ala Tyr Tyr Pro Trp Val
420 425 430

Trp Gln Asn Leu Ile Ser Thr Val Lys Tyr Leu His Gln Ser Gly Thr
435 440 445

Gly Ser Asn Trp Lys Asn Gly Ala Asn Pro Tyr Ser Gly Lys Leu
450 455 460

<210> 77
<211> 1362
<212> DNA
<213> Euglena anabaena UTEX 373

<220>
<221> CDS
<222> (1)..(1362)
<223> delta-5 desaturase

<300>
<302> DELTA-5 DESATURASES AND THEIR USE IN MAKING POLYUNSATURATED FATTY ACIDS

<310> U.S. 2008-0274521-A1

<311> 2008-04-29
<312> 2008-11-06
<313> (1)..(1362)

<300>
<302> DELTA-5 DESATURASES AND THEIR USE IN MAKING POLYUNSATURATED FATTY ACIDS

<310> WO 2008/137532
<311> 2008-05-01
<312> 2008-11-13
<313> (1)..(1362)

<400> 77

```
atg gcc acc atc tct ttg act act gag caa ctt tta gaa cac cca gaa          48
Met Ala Thr Ile Ser Leu Thr Thr Glu Gln Leu Leu Glu His Pro Glu
1               5                   10                  15

ctg gtt gca att gat ggg gtg ttg tac gat ctc ttc gga ctg gcg aaa          96
Leu Val Ala Ile Asp Gly Val Leu Tyr Asp Leu Phe Gly Leu Ala Lys
            20                  25                  30

gtg cat cca ggt ggc aac ctc att gaa gcc gcc ggt gcc tcc gac gga         144
Val His Pro Gly Gly Asn Leu Ile Glu Ala Ala Gly Ala Ser Asp Gly
        35                  40                  45

acc gcc ctg ttc tac tcc atg cac cct gga gtg aag cca gag aat tcg         192
Thr Ala Leu Phe Tyr Ser Met His Pro Gly Val Lys Pro Glu Asn Ser
    50                  55                  60

aag ctg ctg cag caa ttt gcc cga ggc aaa cac gaa cga agc tcg aag         240
Lys Leu Leu Gln Gln Phe Ala Arg Gly Lys His Glu Arg Ser Ser Lys
65                  70                  75                  80

gac cca gtg tac acc ttt gac agt ccc ttc gcc cag gat gtc aag cag         288
Asp Pro Val Tyr Thr Phe Asp Ser Pro Phe Ala Gln Asp Val Lys Gln
                85                  90                  95

agc gtt cgg gag gtc atg aag ggg cgc aac tgg tac gcc acg ccc ggc         336
Ser Val Arg Glu Val Met Lys Gly Arg Asn Trp Tyr Ala Thr Pro Gly
            100                 105                 110

ttt tgg ctg cgg acc gcg ctg atc atc gcg tgc act gcc ata ggc gaa         384
Phe Trp Leu Arg Thr Ala Leu Ile Ile Ala Cys Thr Ala Ile Gly Glu
        115                 120                 125

tgg tat tgg atc act acc ggg gca gtg atg tgg ggc atc ttc acc ggg         432
Trp Tyr Trp Ile Thr Thr Gly Ala Val Met Trp Gly Ile Phe Thr Gly
    130                 135                 140

tac ttc cac agc cag att ggg ttg gcg att caa cac gat gcc tct cac         480
Tyr Phe His Ser Gln Ile Gly Leu Ala Ile Gln His Asp Ala Ser His
145                 150                 155                 160

gga gcc atc agc aaa aag ccc tgg gtg aac gcc ttt ttc gcc tac ggc         528
Gly Ala Ile Ser Lys Lys Pro Trp Val Asn Ala Phe Phe Ala Tyr Gly
                165                 170                 175

atc gac gcc att gga tcc tcc cgc tgg atc tgg ctg cag tcc cac att         576
Ile Asp Ala Ile Gly Ser Ser Arg Trp Ile Trp Leu Gln Ser His Ile
            180                 185                 190

atg cgc cac cac acc tac acc aac cag cat ggc ctg gac ctg gac gct         624
Met Arg His His Thr Tyr Thr Asn Gln His Gly Leu Asp Leu Asp Ala
        195                 200                 205

gcc tcg gcg gag ccg ttc att ttg ttc cac tcc tac ccg gca aca aat         672
```

```
Ala Ser Ala Glu Pro Phe Ile Leu Phe His Ser Tyr Pro Ala Thr Asn
    210                 215                 220

gcg tca cga aag tgg tac cat cgg ttc cag gcg tgg tac atg tac atc      720
Ala Ser Arg Lys Trp Tyr His Arg Phe Gln Ala Trp Tyr Met Tyr Ile
225                 230                 235                 240

gtt ttg ggg atg tat ggt gtg tcg atg gtg tac aat ccg atg tac ttg      768
Val Leu Gly Met Tyr Gly Val Ser Met Val Tyr Asn Pro Met Tyr Leu
                245                 250                 255

ttc acg atg cag cac aac gac aca atc cca gag gcc acc tct ctt aga      816
Phe Thr Met Gln His Asn Asp Thr Ile Pro Glu Ala Thr Ser Leu Arg
            260                 265                 270

cca ggc agc ttt ttc aac cgg cag cgc gcc ttc gcc gtt tcc ctc cgc      864
Pro Gly Ser Phe Phe Asn Arg Gln Arg Ala Phe Ala Val Ser Leu Arg
        275                 280                 285

cta ctg ttc atc ttc cgc aac gcc ttc ctc ccc tgg tac atc gcg ggc      912
Leu Leu Phe Ile Phe Arg Asn Ala Phe Leu Pro Trp Tyr Ile Ala Gly
    290                 295                 300

gcc tct ccg ctg ctc acc atc ctg ctg gtg cca acg gtc aca ggc atc      960
Ala Ser Pro Leu Leu Thr Ile Leu Leu Val Pro Thr Val Thr Gly Ile
305                 310                 315                 320

ttc ttg aca ttt gtt ttt gtg ctg tcc cat aac ttt gaa ggc gct gag     1008
Phe Leu Thr Phe Val Phe Val Leu Ser His Asn Phe Glu Gly Ala Glu
                325                 330                 335

cgg acc ccc gaa aag aac tgc aag gcc aaa agg gcc aag gag ggg aag     1056
Arg Thr Pro Glu Lys Asn Cys Lys Ala Lys Arg Ala Lys Glu Gly Lys
            340                 345                 350

gag gtc cgc gat gta gag gag gac cgg gtg gac tgg tac cgg gcg cag     1104
Glu Val Arg Asp Val Glu Glu Asp Arg Val Asp Trp Tyr Arg Ala Gln
        355                 360                 365

gcc gag acc gcg gcg acc tac ggg ggc agc gtc ggg atg atg ctg acc     1152
Ala Glu Thr Ala Ala Thr Tyr Gly Gly Ser Val Gly Met Met Leu Thr
    370                 375                 380

ggc ggt ttg aac ctg cag atc gag cac cac ttg ttc ccc cgc atg tcc     1200
Gly Gly Leu Asn Leu Gln Ile Glu His His Leu Phe Pro Arg Met Ser
385                 390                 395                 400

tct tgg cac tac ccc ttc atc caa gat acg gtg cgg gaa tgt tgc aag     1248
Ser Trp His Tyr Pro Phe Ile Gln Asp Thr Val Arg Glu Cys Cys Lys
                405                 410                 415

cgc cat ggc gtg cgc tac aca tac tac ccg acc atc ctg gag aat ata     1296
Arg His Gly Val Arg Tyr Thr Tyr Tyr Pro Thr Ile Leu Glu Asn Ile
            420                 425                 430

atg tcc acg ctc cgc tac atg cag aag gtg ggc gtg gcc cac aca att     1344
Met Ser Thr Leu Arg Tyr Met Gln Lys Val Gly Val Ala His Thr Ile
        435                 440                 445

cag gat gcc cag gaa ttc                                             1362
Gln Asp Ala Gln Glu Phe
    450
```

<210> 78
<211> 454
<212> PRT
<213> Euglena anabaena UTEX 373

<400> 78

```
Met Ala Thr Ile Ser Leu Thr Thr Glu Gln Leu Leu Glu His Pro Glu
1               5                   10                  15

Leu Val Ala Ile Asp Gly Val Leu Tyr Asp Leu Phe Gly Leu Ala Lys
            20                  25                  30

Val His Pro Gly Gly Asn Leu Ile Glu Ala Ala Gly Ala Ser Asp Gly
        35                  40                  45

Thr Ala Leu Phe Tyr Ser Met His Pro Gly Val Lys Pro Glu Asn Ser
    50                  55                  60

Lys Leu Leu Gln Gln Phe Ala Arg Gly Lys His Glu Arg Ser Ser Lys
65                  70                  75                  80

Asp Pro Val Tyr Thr Phe Asp Ser Pro Phe Ala Gln Asp Val Lys Gln
                85                  90                  95

Ser Val Arg Glu Val Met Lys Gly Arg Asn Trp Tyr Ala Thr Pro Gly
            100                 105                 110

Phe Trp Leu Arg Thr Ala Leu Ile Ile Ala Cys Thr Ala Ile Gly Glu
        115                 120                 125

Trp Tyr Trp Ile Thr Thr Gly Ala Val Met Trp Gly Ile Phe Thr Gly
    130                 135                 140

Tyr Phe His Ser Gln Ile Gly Leu Ala Ile Gln His Asp Ala Ser His
145                 150                 155                 160

Gly Ala Ile Ser Lys Lys Pro Trp Val Asn Ala Phe Phe Ala Tyr Gly
                165                 170                 175

Ile Asp Ala Ile Gly Ser Ser Arg Trp Ile Trp Leu Gln Ser His Ile
            180                 185                 190

Met Arg His His Thr Tyr Thr Asn Gln His Gly Leu Asp Leu Asp Ala
        195                 200                 205

Ala Ser Ala Glu Pro Phe Ile Leu Phe His Ser Tyr Pro Ala Thr Asn
    210                 215                 220

Ala Ser Arg Lys Trp Tyr His Arg Phe Gln Ala Trp Tyr Met Tyr Ile
225                 230                 235                 240
```

```
Val Leu Gly Met Tyr Gly Val Ser Met Val Tyr Asn Pro Met Tyr Leu
                245                 250                 255

Phe Thr Met Gln His Asn Asp Thr Ile Pro Glu Ala Thr Ser Leu Arg
            260                 265                 270

Pro Gly Ser Phe Phe Asn Arg Gln Arg Ala Phe Ala Val Ser Leu Arg
        275                 280                 285

Leu Leu Phe Ile Phe Arg Asn Ala Phe Leu Pro Trp Tyr Ile Ala Gly
    290                 295                 300

Ala Ser Pro Leu Leu Thr Ile Leu Leu Val Pro Thr Val Thr Gly Ile
305                 310                 315                 320

Phe Leu Thr Phe Val Phe Val Leu Ser His Asn Phe Glu Gly Ala Glu
                325                 330                 335

Arg Thr Pro Glu Lys Asn Cys Lys Ala Lys Arg Ala Lys Glu Gly Lys
            340                 345                 350

Glu Val Arg Asp Val Glu Glu Asp Arg Val Asp Trp Tyr Arg Ala Gln
        355                 360                 365

Ala Glu Thr Ala Ala Thr Tyr Gly Gly Ser Val Gly Met Met Leu Thr
    370                 375                 380

Gly Gly Leu Asn Leu Gln Ile Glu His His Leu Phe Pro Arg Met Ser
385                 390                 395                 400

Ser Trp His Tyr Pro Phe Ile Gln Asp Thr Val Arg Glu Cys Cys Lys
                405                 410                 415

Arg His Gly Val Arg Tyr Thr Tyr Tyr Pro Thr Ile Leu Glu Asn Ile
            420                 425                 430

Met Ser Thr Leu Arg Tyr Met Gln Lys Val Gly Val Ala His Thr Ile
        435                 440                 445

Gln Asp Ala Gln Glu Phe
    450
```

<210> 79
<211> 1362
<212> DNA
<213> Euglena anabaena UTEX 373

<220>

<221> CDS
<222> (1)..(1362)
<223> synthetic delta-5 desaturase (codon-optimized for Yarrowia lipolytica)

<300>
<302> DELTA-5 DESATURASES AND THEIR USE IN MAKING POLYUNSATURATED FATTY ACIDS

<310> U.S. 2008-0274521-A1
<311> 2008-04-29
<312> 2008-11-06
<313> (1)..(1362)

<300>
<302> DELTA-5 DESATURASES AND THEIR USE IN MAKING POLYUNSATURATED FATTY ACIDS

<310> WO 2008/137532
<311> 2008-05-01
<312> 2008-11-13
<313> (1)..(1362)

<400> 79

```
atg gcc acc atc tcc ctg act acc gag cag ctc ctg gaa cac ccc gag         48
Met Ala Thr Ile Ser Leu Thr Thr Glu Gln Leu Leu Glu His Pro Glu
1               5                   10                  15

ctc gtt gcc atc gac gga gtc ctg tac gat ctc ttc ggt ctg gcc aag         96
Leu Val Ala Ile Asp Gly Val Leu Tyr Asp Leu Phe Gly Leu Ala Lys
            20                  25                  30

gtg cat cca gga ggc aac ctc atc gaa gct gcc ggt gca tcc gac gga        144
Val His Pro Gly Gly Asn Leu Ile Glu Ala Ala Gly Ala Ser Asp Gly
        35                  40                  45

acc gct ctg ttc tac tcc atg cat cct gga gtc aag cca gag aac tcg        192
Thr Ala Leu Phe Tyr Ser Met His Pro Gly Val Lys Pro Glu Asn Ser
    50                  55                  60

aag ctt ctg cag caa ttt gcc cga ggc aag cac gaa cga agc tcc aag        240
Lys Leu Leu Gln Gln Phe Ala Arg Gly Lys His Glu Arg Ser Ser Lys
65                  70                  75                  80

gat ccc gtg tac acc ttc gac tct ccc ttt gct cag gac gtc aag cag        288
Asp Pro Val Tyr Thr Phe Asp Ser Pro Phe Ala Gln Asp Val Lys Gln
                85                  90                  95

tcc gtt cga gag gtc atg aag ggt cga aac tgg tac gcc act cct ggc        336
Ser Val Arg Glu Val Met Lys Gly Arg Asn Trp Tyr Ala Thr Pro Gly
            100                 105                 110

ttc tgg ctg aga acc gca ctc atc atc gct tgt act gcc att ggc gag        384
Phe Trp Leu Arg Thr Ala Leu Ile Ile Ala Cys Thr Ala Ile Gly Glu
        115                 120                 125

tgg tac tgg atc aca acc gga gca gtg atg tgg ggt atc ttt act gga        432
Trp Tyr Trp Ile Thr Thr Gly Ala Val Met Trp Gly Ile Phe Thr Gly
    130                 135                 140

tac ttc cac tcg cag att ggc ttg gcc att caa cac gat gct tct cac        480
Tyr Phe His Ser Gln Ile Gly Leu Ala Ile Gln His Asp Ala Ser His
145                 150                 155                 160

gga gcc atc agc aaa aag ccc tgg gtc aac gcc ttt ttc gct tat ggc        528
Gly Ala Ile Ser Lys Lys Pro Trp Val Asn Ala Phe Phe Ala Tyr Gly
                165                 170                 175
```

```
atc gac gcc att ggt tcc tct cgt tgg atc tgg ctg cag tcc cac att        576
Ile Asp Ala Ile Gly Ser Ser Arg Trp Ile Trp Leu Gln Ser His Ile
            180                 185                 190

atg cga cat cac act tac acc aac cag cat ggc ctc gac ctg gat gct        624
Met Arg His His Thr Tyr Thr Asn Gln His Gly Leu Asp Leu Asp Ala
        195                 200                 205

gcc tcg gca gag ccg ttc atc ttg ttc cac tcc tat cct gct acc aac        672
Ala Ser Ala Glu Pro Phe Ile Leu Phe His Ser Tyr Pro Ala Thr Asn
    210                 215                 220

gcc tct cga aag tgg tac cac cga ttt cag gcg tgg tac atg tac atc        720
Ala Ser Arg Lys Trp Tyr His Arg Phe Gln Ala Trp Tyr Met Tyr Ile
225                 230                 235                 240

gtt ctg gga atg tat ggt gtc tcg atg gtg tac aat ccc atg tac ctc        768
Val Leu Gly Met Tyr Gly Val Ser Met Val Tyr Asn Pro Met Tyr Leu
                245                 250                 255

ttc aca atg cag cac aac gac acc att ccc gag gcc act tct ctc aga        816
Phe Thr Met Gln His Asn Asp Thr Ile Pro Glu Ala Thr Ser Leu Arg
            260                 265                 270

cca ggc agc ttt ttc aat cgg cag cga gct ttc gcc gtt tcc ctt cga        864
Pro Gly Ser Phe Phe Asn Arg Gln Arg Ala Phe Ala Val Ser Leu Arg
        275                 280                 285

ctg ctc ttc atc ttc cga aac gcc ttt ctt ccc tgg tac att gct ggt        912
Leu Leu Phe Ile Phe Arg Asn Ala Phe Leu Pro Trp Tyr Ile Ala Gly
    290                 295                 300

gcc tct cct ctg ctc acc att ctt ctg gtg ccc acg gtc aca ggc atc        960
Ala Ser Pro Leu Leu Thr Ile Leu Leu Val Pro Thr Val Thr Gly Ile
305                 310                 315                 320

ttc ctc acc ttt gtg ttc gtt ctg tcc cat aac ttc gag gga gcc gaa       1008
Phe Leu Thr Phe Val Phe Val Leu Ser His Asn Phe Glu Gly Ala Glu
                325                 330                 335

cgg acc cca gag aag aac tgc aag gcc aaa cga gct aag gaa ggc aag       1056
Arg Thr Pro Glu Lys Asn Cys Lys Ala Lys Arg Ala Lys Glu Gly Lys
            340                 345                 350

gag gtc aga gac gtg gaa gag gat cga gtc gac tgg tac cga gca cag       1104
Glu Val Arg Asp Val Glu Glu Asp Arg Val Asp Trp Tyr Arg Ala Gln
        355                 360                 365

gcc gag act gct gcc acc tac ggt ggc agc gtg gga atg atg ctt aca       1152
Ala Glu Thr Ala Ala Thr Tyr Gly Gly Ser Val Gly Met Met Leu Thr
    370                 375                 380

ggc ggt ctc aac ctg cag atc gag cat cac ttg ttt ccc cga atg tcc       1200
Gly Gly Leu Asn Leu Gln Ile Glu His His Leu Phe Pro Arg Met Ser
385                 390                 395                 400

tct tgg cac tat ccc ttc att caa gac acc gtt cgg gag tgt tgc aag       1248
Ser Trp His Tyr Pro Phe Ile Gln Asp Thr Val Arg Glu Cys Cys Lys
                405                 410                 415

cga cat ggc gtc cgt tac aca tac tat cct acc att ctc gag aac atc       1296
Arg His Gly Val Arg Tyr Thr Tyr Tyr Pro Thr Ile Leu Glu Asn Ile
            420                 425                 430

atg tcc act ctt cga tac atg cag aag gtg ggt gtt gct cac acc att       1344
```

```
Met Ser Thr Leu Arg Tyr Met Gln Lys Val Gly Val Ala His Thr Ile
        435                 440                 445

cag gat gcc cag gag ttc                                                  1362
Gln Asp Ala Gln Glu Phe
    450
```

<210> 80
<211> 454
<212> PRT
<213> Euglena anabaena UTEX 373

<400> 80

```
Met Ala Thr Ile Ser Leu Thr Thr Glu Gln Leu Leu Glu His Pro Glu
1               5                   10                  15

Leu Val Ala Ile Asp Gly Val Leu Tyr Asp Leu Phe Gly Leu Ala Lys
            20                  25                  30

Val His Pro Gly Gly Asn Leu Ile Glu Ala Ala Gly Ala Ser Asp Gly
        35                  40                  45

Thr Ala Leu Phe Tyr Ser Met His Pro Gly Val Lys Pro Glu Asn Ser
    50                  55                  60

Lys Leu Leu Gln Gln Phe Ala Arg Gly Lys His Glu Arg Ser Ser Lys
65                  70                  75                  80

Asp Pro Val Tyr Thr Phe Asp Ser Pro Phe Ala Gln Asp Val Lys Gln
                85                  90                  95

Ser Val Arg Glu Val Met Lys Gly Arg Asn Trp Tyr Ala Thr Pro Gly
            100                 105                 110

Phe Trp Leu Arg Thr Ala Leu Ile Ile Ala Cys Thr Ala Ile Gly Glu
        115                 120                 125

Trp Tyr Trp Ile Thr Thr Gly Ala Val Met Trp Gly Ile Phe Thr Gly
    130                 135                 140

Tyr Phe His Ser Gln Ile Gly Leu Ala Ile Gln His Asp Ala Ser His
145                 150                 155                 160

Gly Ala Ile Ser Lys Lys Pro Trp Val Asn Ala Phe Phe Ala Tyr Gly
                165                 170                 175

Ile Asp Ala Ile Gly Ser Ser Arg Trp Ile Trp Leu Gln Ser His Ile
            180                 185                 190

Met Arg His His Thr Tyr Thr Asn Gln His Gly Leu Asp Leu Asp Ala
        195                 200                 205
```

```
Ala Ser Ala Glu Pro Phe Ile Leu Phe His Ser Tyr Pro Ala Thr Asn
    210                 215                 220

Ala Ser Arg Lys Trp Tyr His Arg Phe Gln Ala Trp Tyr Met Tyr Ile
225                 230                 235                 240

Val Leu Gly Met Tyr Gly Val Ser Met Val Tyr Asn Pro Met Tyr Leu
                245                 250                 255

Phe Thr Met Gln His Asn Asp Thr Ile Pro Glu Ala Thr Ser Leu Arg
            260                 265                 270

Pro Gly Ser Phe Phe Asn Arg Gln Arg Ala Phe Ala Val Ser Leu Arg
        275                 280                 285

Leu Leu Phe Ile Phe Arg Asn Ala Phe Leu Pro Trp Tyr Ile Ala Gly
    290                 295                 300

Ala Ser Pro Leu Leu Thr Ile Leu Leu Val Pro Thr Val Thr Gly Ile
305                 310                 315                 320

Phe Leu Thr Phe Val Phe Val Leu Ser His Asn Phe Glu Gly Ala Glu
                325                 330                 335

Arg Thr Pro Glu Lys Asn Cys Lys Ala Lys Arg Ala Lys Glu Gly Lys
            340                 345                 350

Glu Val Arg Asp Val Glu Glu Asp Arg Val Asp Trp Tyr Arg Ala Gln
        355                 360                 365

Ala Glu Thr Ala Ala Thr Tyr Gly Gly Ser Val Gly Met Met Leu Thr
    370                 375                 380

Gly Gly Leu Asn Leu Gln Ile Glu His His Leu Phe Pro Arg Met Ser
385                 390                 395                 400

Ser Trp His Tyr Pro Phe Ile Gln Asp Thr Val Arg Glu Cys Cys Lys
                405                 410                 415

Arg His Gly Val Arg Tyr Thr Tyr Tyr Pro Thr Ile Leu Glu Asn Ile
            420                 425                 430

Met Ser Thr Leu Arg Tyr Met Gln Lys Val Gly Val Ala His Thr Ile
        435                 440                 445

Gln Asp Ala Gln Glu Phe
    450
```

<210> 81
<211> 1365
<212> DNA
<213> Euglena anabaena UTEX 373

<220>
<221> CDS
<222> (1)..(1365)
<223> synthetic mutant delta-5 desaturase (codon-optimized for Yarrowia lipolytica)

<400> 81

```
atg gcc acc atc tcc ctg act acc gag cag ctc ctg gaa cac ccc gag      48
Met Ala Thr Ile Ser Leu Thr Thr Glu Gln Leu Leu Glu His Pro Glu
1               5                   10                  15

ctc gtt gcc atc gac gga gtc ctg tac gat ctc ttc ggt ctg gcc aag      96
Leu Val Ala Ile Asp Gly Val Leu Tyr Asp Leu Phe Gly Leu Ala Lys
            20                  25                  30

gtc cat gcc gga ggc aac ctc atc gaa gct gcc ggt gca tcc gac gga     144
Val His Ala Gly Gly Asn Leu Ile Glu Ala Ala Gly Ala Ser Asp Gly
        35                  40                  45

acc gct ctg ttc tac tcc atg cat cct gga gtc aag cca gag aac tcg     192
Thr Ala Leu Phe Tyr Ser Met His Pro Gly Val Lys Pro Glu Asn Ser
    50                  55                  60

aag ctt ctg cag caa ttt gcc cga ggc aag cac gaa cga agc tcc aag     240
Lys Leu Leu Gln Gln Phe Ala Arg Gly Lys His Glu Arg Ser Ser Lys
65                  70                  75                  80

gat ccc gtg tac acc ttc gac tct ccc ttt gct cag gac gtc aag cag     288
Asp Pro Val Tyr Thr Phe Asp Ser Pro Phe Ala Gln Asp Val Lys Gln
                85                  90                  95

tcc gtt cga gag gtc atg aag ggt cga aac tgg tac gcc act cct ggc     336
Ser Val Arg Glu Val Met Lys Gly Arg Asn Trp Tyr Ala Thr Pro Gly
            100                 105                 110

ttc tgg ctg aga acc gca ctc atc atc gct tgt act gcc att ggc gag     384
Phe Trp Leu Arg Thr Ala Leu Ile Ile Ala Cys Thr Ala Ile Gly Glu
        115                 120                 125

tgg tac tgg atc aca acc gga gca gtg atg tgg ggt atc ttt act gga     432
Trp Tyr Trp Ile Thr Thr Gly Ala Val Met Trp Gly Ile Phe Thr Gly
    130                 135                 140

tac ttc cac tcg cag att ggc ttg gcc att caa cac gat gct tct cac     480
Tyr Phe His Ser Gln Ile Gly Leu Ala Ile Gln His Asp Ala Ser His
145                 150                 155                 160

gga gcc atc agc aaa aag ccc tgg gtc aac gcc ttt ttc gct tat ggc     528
Gly Ala Ile Ser Lys Lys Pro Trp Val Asn Ala Phe Phe Ala Tyr Gly
                165                 170                 175

atc gac gcc att ggt tcc tct cgt tgg atc tgg ctg cag tcc cac att     576
Ile Asp Ala Ile Gly Ser Ser Arg Trp Ile Trp Leu Gln Ser His Ile
            180                 185                 190

atg cga cat cac act tac acc aac cag cat ggc ctc gac ctg gat gct     624
Met Arg His His Thr Tyr Thr Asn Gln His Gly Leu Asp Leu Asp Ala
        195                 200                 205
```

```
gcc tcg gca gag ccg ttc atc ttg ttc cac tcc tat cct gct acc aac        672
Ala Ser Ala Glu Pro Phe Ile Leu Phe His Ser Tyr Pro Ala Thr Asn
    210                 215                 220

gcc tct cga aag tgg tac cac cga ttt cag gcg tgg tac atg tac atc        720
Ala Ser Arg Lys Trp Tyr His Arg Phe Gln Ala Trp Tyr Met Tyr Ile
225                 230                 235                 240

gtt ctg gga atg tat ggt gtc tcg atg gtg tac aat ccc atg tac ctc        768
Val Leu Gly Met Tyr Gly Val Ser Met Val Tyr Asn Pro Met Tyr Leu
                245                 250                 255

ttc aca atg cag cac aac gac acc att ccc gag gcc act tct ctc aga        816
Phe Thr Met Gln His Asn Asp Thr Ile Pro Glu Ala Thr Ser Leu Arg
            260                 265                 270

cca ggc agc ttt ttc aat cgg cag cga gct ttc gcc gtt tcc ctt cga        864
Pro Gly Ser Phe Phe Asn Arg Gln Arg Ala Phe Ala Val Ser Leu Arg
        275                 280                 285

ctg ctc ttc atc ttc cga aac gcc ttt ctt ccc tgg tac att gct ggt        912
Leu Leu Phe Ile Phe Arg Asn Ala Phe Leu Pro Trp Tyr Ile Ala Gly
    290                 295                 300

gcc tct cct ctg ctc acc att ctt ctg gtg ccc acg gtc aca ggc atc        960
Ala Ser Pro Leu Leu Thr Ile Leu Leu Val Pro Thr Val Thr Gly Ile
305                 310                 315                 320

ttc ctc acc ttt gtg ttc gtt ctg tcc cat aac ttc gag gga gcc gaa       1008
Phe Leu Thr Phe Val Phe Val Leu Ser His Asn Phe Glu Gly Ala Glu
                325                 330                 335

cgg acc cca gag aag aac tgc aag gcc aaa cga gct aag gaa ggc aag       1056
Arg Thr Pro Glu Lys Asn Cys Lys Ala Lys Arg Ala Lys Glu Gly Lys
            340                 345                 350

gag gtc aga gac gtg gaa gag gat cga gtc gac tgg tac cga gca cag       1104
Glu Val Arg Asp Val Glu Glu Asp Arg Val Asp Trp Tyr Arg Ala Gln
        355                 360                 365

gcc gag act gct gcc acc tac ggt ggc agc gtg gga atg atg ctt aca       1152
Ala Glu Thr Ala Ala Thr Tyr Gly Gly Ser Val Gly Met Met Leu Thr
    370                 375                 380

ggc ggt ctc aac ctg cag atc gag cat cac ttg ttt ccc cga atg tcc       1200
Gly Gly Leu Asn Leu Gln Ile Glu His His Leu Phe Pro Arg Met Ser
385                 390                 395                 400

tct tgg cac tat ccc ttc att caa gac acc gtt cgg gag tgt tgc aag       1248
Ser Trp His Tyr Pro Phe Ile Gln Asp Thr Val Arg Glu Cys Cys Lys
                405                 410                 415

cga cat ggc gtc cgt tac aca tac tat cct acc att ctc gag aac atc       1296
Arg His Gly Val Arg Tyr Thr Tyr Tyr Pro Thr Ile Leu Glu Asn Ile
            420                 425                 430

atg tcc act ctt cga tac atg cag aag gtg ggt gtt gct cac acc att       1344
Met Ser Thr Leu Arg Tyr Met Gln Lys Val Gly Val Ala His Thr Ile
        435                 440                 445

cag gat gcc cag gag ttc taa                                           1365
Gln Asp Ala Gln Glu Phe
    450
```

<210> 82
<211> 454
<212> PRT
<213> Euglena anabaena UTEX 373

<400> 82

```
Met Ala Thr Ile Ser Leu Thr Thr Glu Gln Leu Leu Glu His Pro Glu
1               5                   10                  15

Leu Val Ala Ile Asp Gly Val Leu Tyr Asp Leu Phe Gly Leu Ala Lys
            20                  25                  30

Val His Ala Gly Gly Asn Leu Ile Glu Ala Ala Gly Ala Ser Asp Gly
        35                  40                  45

Thr Ala Leu Phe Tyr Ser Met His Pro Gly Val Lys Pro Glu Asn Ser
    50                  55                  60

Lys Leu Leu Gln Gln Phe Ala Arg Gly Lys His Glu Arg Ser Ser Lys
65                  70                  75                  80

Asp Pro Val Tyr Thr Phe Asp Ser Pro Phe Ala Gln Asp Val Lys Gln
                85                  90                  95

Ser Val Arg Glu Val Met Lys Gly Arg Asn Trp Tyr Ala Thr Pro Gly
            100                 105                 110

Phe Trp Leu Arg Thr Ala Leu Ile Ile Ala Cys Thr Ala Ile Gly Glu
        115                 120                 125

Trp Tyr Trp Ile Thr Thr Gly Ala Val Met Trp Gly Ile Phe Thr Gly
    130                 135                 140

Tyr Phe His Ser Gln Ile Gly Leu Ala Ile Gln His Asp Ala Ser His
145                 150                 155                 160

Gly Ala Ile Ser Lys Lys Pro Trp Val Asn Ala Phe Phe Ala Tyr Gly
                165                 170                 175

Ile Asp Ala Ile Gly Ser Ser Arg Trp Ile Trp Leu Gln Ser His Ile
            180                 185                 190

Met Arg His His Thr Tyr Thr Asn Gln His Gly Leu Asp Leu Asp Ala
        195                 200                 205

Ala Ser Ala Glu Pro Phe Ile Leu Phe His Ser Tyr Pro Ala Thr Asn
    210                 215                 220

Ala Ser Arg Lys Trp Tyr His Arg Phe Gln Ala Trp Tyr Met Tyr Ile
```

```
225                 230                 235                 240

Val Leu Gly Met Tyr Gly Val Ser Met Val Tyr Asn Pro Met Tyr Leu
                245                 250                 255

Phe Thr Met Gln His Asn Asp Thr Ile Pro Glu Ala Thr Ser Leu Arg
            260                 265                 270

Pro Gly Ser Phe Phe Asn Arg Gln Arg Ala Phe Ala Val Ser Leu Arg
        275                 280                 285

Leu Leu Phe Ile Phe Arg Asn Ala Phe Leu Pro Trp Tyr Ile Ala Gly
    290                 295                 300

Ala Ser Pro Leu Leu Thr Ile Leu Leu Val Pro Thr Val Thr Gly Ile
305                 310                 315                 320

Phe Leu Thr Phe Val Phe Val Leu Ser His Asn Phe Glu Gly Ala Glu
                325                 330                 335

Arg Thr Pro Glu Lys Asn Cys Lys Ala Lys Arg Ala Lys Glu Gly Lys
            340                 345                 350

Glu Val Arg Asp Val Glu Glu Asp Arg Val Asp Trp Tyr Arg Ala Gln
        355                 360                 365

Ala Glu Thr Ala Ala Thr Tyr Gly Gly Ser Val Gly Met Met Leu Thr
    370                 375                 380

Gly Gly Leu Asn Leu Gln Ile Glu His His Leu Phe Pro Arg Met Ser
385                 390                 395                 400

Ser Trp His Tyr Pro Phe Ile Gln Asp Thr Val Arg Glu Cys Cys Lys
                405                 410                 415

Arg His Gly Val Arg Tyr Thr Tyr Tyr Pro Thr Ile Leu Glu Asn Ile
            420                 425                 430

Met Ser Thr Leu Arg Tyr Met Gln Lys Val Gly Val Ala His Thr Ile
        435                 440                 445

Gln Asp Ala Gln Glu Phe
    450
```

<210> 83
<211> 1086
<212> DNA

<213> Phytophthora ramorum

<220>
<221> CDS
<222> (1)..(1086)
<223> delta-17 desaturase

<300>
<302> DELTA-17 DESATURASES AND THEIR USE IN MAKING POLYUNSATURATED FATTY ACIDS

<310> WO 2007/123999
<311> 2007-04-19
<312> 2007-11-01
<313> (1)..(1086)

<300>
<302> DELTA-17 DESATURASES AND THEIR USE IN MAKING POLYUNSATURATED FATTY ACIDS

<310> US 2007-0249026-A1
<311> 2007-04-18
<312> 2007-10-25
<313> (1)..(1086)

<400> 83

```
atg gcg act aag cag ccg tac cag ttc ccg acc ctg acg gag atc aag      48
Met Ala Thr Lys Gln Pro Tyr Gln Phe Pro Thr Leu Thr Glu Ile Lys
1               5                   10                  15

cgg tcg ctg ccc agc gag tgc ttt gag gcc tcg gtg ccg ctg tcg ctc      96
Arg Ser Leu Pro Ser Glu Cys Phe Glu Ala Ser Val Pro Leu Ser Leu
            20                  25                  30

tac tac acg gtg cgc atc gtg gcc atc gcc gtg gcg ctg gcg ttc ggc     144
Tyr Tyr Thr Val Arg Ile Val Ala Ile Ala Val Ala Leu Ala Phe Gly
        35                  40                  45

ctc aac tac gcg cgc gcg ctg ccc gtg gtc gag agc ttg tgg gcg ctg     192
Leu Asn Tyr Ala Arg Ala Leu Pro Val Val Glu Ser Leu Trp Ala Leu
    50                  55                  60

gac gct gcg ctc tgc tgc ggt tac gtg ctg ctg cag ggc atc gtg ttc     240
Asp Ala Ala Leu Cys Cys Gly Tyr Val Leu Leu Gln Gly Ile Val Phe
65                  70                  75                  80

tgg ggc ttc ttc acg gtg ggc cat gac gcc ggc cac ggc gcc ttc tcg     288
Trp Gly Phe Phe Thr Val Gly His Asp Ala Gly His Gly Ala Phe Ser
                85                  90                  95

cgt tac cac ctg ctc aac ttc gtg gtg ggc acc ttc atc cac tcg ctc     336
Arg Tyr His Leu Leu Asn Phe Val Val Gly Thr Phe Ile His Ser Leu
            100                 105                 110

atc ctc acg ccc ttc gag tcg tgg aag ctc acg cac cgc cac cac cac     384
Ile Leu Thr Pro Phe Glu Ser Trp Lys Leu Thr His Arg His His His
        115                 120                 125

aag aac acg ggc aac att gac cgc gac gag atc ttc tac ccg cag cgc     432
Lys Asn Thr Gly Asn Ile Asp Arg Asp Glu Ile Phe Tyr Pro Gln Arg
    130                 135                 140

aag gcc gac gac cac ccg ctg tcg cgc aac ctc gtg ctg gcg ctc ggc     480
Lys Ala Asp Asp His Pro Leu Ser Arg Asn Leu Val Leu Ala Leu Gly
145                 150                 155                 160

gcc gcg tgg ttc gcc tac ctg gtc gag ggc ttc ccg ccc cgc aag gtc     528
Ala Ala Trp Phe Ala Tyr Leu Val Glu Gly Phe Pro Pro Arg Lys Val
                165                 170                 175
```

```
aac cac ttc aac cca ttc gag ccg ctg ttt gtg cgc cag gtg gcc gcc       576
Asn His Phe Asn Pro Phe Glu Pro Leu Phe Val Arg Gln Val Ala Ala
            180                 185                 190

gtc gtc atc tcg ctc tcc gcg cac ttc gcc gtg ttg gcg ctg tcc gtg       624
Val Val Ile Ser Leu Ser Ala His Phe Ala Val Leu Ala Leu Ser Val
        195                 200                 205

tat ctg agc ttc cag ttc ggt ctc aag acc atg gcg ctc tac tac tac       672
Tyr Leu Ser Phe Gln Phe Gly Leu Lys Thr Met Ala Leu Tyr Tyr Tyr
    210                 215                 220

ggc ccc gtc ttc gtg ttc ggc agc atg ctt gtg atc acc acc ttc ctg       720
Gly Pro Val Phe Val Phe Gly Ser Met Leu Val Ile Thr Thr Phe Leu
225                 230                 235                 240

cat cac aat gac gag gag acc cca tgg tac gga gac tcc gac tgg acc       768
His His Asn Asp Glu Glu Thr Pro Trp Tyr Gly Asp Ser Asp Trp Thr
                245                 250                 255

tac gtc aag ggc aac ctg tcg tcc gtg gac cgg tcc tac ggc gcg ttc       816
Tyr Val Lys Gly Asn Leu Ser Ser Val Asp Arg Ser Tyr Gly Ala Phe
            260                 265                 270

atc gac aac ctg agc cac aac atc ggc acg cac cag atc cac cac ctc       864
Ile Asp Asn Leu Ser His Asn Ile Gly Thr His Gln Ile His His Leu
        275                 280                 285

ttc ccc atc atc ccg cac tac aag ctc aac cgc gct acg gcg gca ttc       912
Phe Pro Ile Ile Pro His Tyr Lys Leu Asn Arg Ala Thr Ala Ala Phe
    290                 295                 300

cac cag gcc ttc ccc gag ctc gtg cgc aag agc gac gag ccg atc ctc       960
His Gln Ala Phe Pro Glu Leu Val Arg Lys Ser Asp Glu Pro Ile Leu
305                 310                 315                 320

aag gcc ttc tgg cgc gtc ggc cga ctg tac gcc aac tac ggc gtc gtg      1008
Lys Ala Phe Trp Arg Val Gly Arg Leu Tyr Ala Asn Tyr Gly Val Val
                325                 330                 335

gac ccg gac gcc aag ctc ttc acg ctc aag gag gcc aag gcg gcg tcc      1056
Asp Pro Asp Ala Lys Leu Phe Thr Leu Lys Glu Ala Lys Ala Ala Ser
            340                 345                 350

gag gcg gcg acc aag acc aag gcc acc taa                              1086
Glu Ala Ala Thr Lys Thr Lys Ala Thr
        355                 360
```

<210> 84
<211> 361
<212> PRT
<213> Phytophthora ramorum

<400> 84

```
Met Ala Thr Lys Gln Pro Tyr Gln Phe Pro Thr Leu Thr Glu Ile Lys
1               5                   10                  15

Arg Ser Leu Pro Ser Glu Cys Phe Glu Ala Ser Val Pro Leu Ser Leu
            20                  25                  30
```

Tyr Tyr Thr Val Arg Ile Val Ala Ile Ala Val Ala Leu Ala Phe Gly
35 40 45

Leu Asn Tyr Ala Arg Ala Leu Pro Val Val Glu Ser Leu Trp Ala Leu
50 55 60

Asp Ala Ala Leu Cys Cys Gly Tyr Val Leu Leu Gln Gly Ile Val Phe
65 70 75 80

Trp Gly Phe Phe Thr Val Gly His Asp Ala Gly His Gly Ala Phe Ser
85 90 95

Arg Tyr His Leu Leu Asn Phe Val Val Gly Thr Phe Ile His Ser Leu
100 105 110

Ile Leu Thr Pro Phe Glu Ser Trp Lys Leu Thr His Arg His His His
115 120 125

Lys Asn Thr Gly Asn Ile Asp Arg Asp Glu Ile Phe Tyr Pro Gln Arg
130 135 140

Lys Ala Asp Asp His Pro Leu Ser Arg Asn Leu Val Leu Ala Leu Gly
145 150 155 160

Ala Ala Trp Phe Ala Tyr Leu Val Glu Gly Phe Pro Pro Arg Lys Val
165 170 175

Asn His Phe Asn Pro Phe Glu Pro Leu Phe Val Arg Gln Val Ala Ala
180 185 190

Val Val Ile Ser Leu Ser Ala His Phe Ala Val Leu Ala Leu Ser Val
195 200 205

Tyr Leu Ser Phe Gln Phe Gly Leu Lys Thr Met Ala Leu Tyr Tyr Tyr
210 215 220

Gly Pro Val Phe Val Phe Gly Ser Met Leu Val Ile Thr Thr Phe Leu
225 230 235 240

His His Asn Asp Glu Glu Thr Pro Trp Tyr Gly Asp Ser Asp Trp Thr
245 250 255

Tyr Val Lys Gly Asn Leu Ser Ser Val Asp Arg Ser Tyr Gly Ala Phe
260 265 270

Ile Asp Asn Leu Ser His Asn Ile Gly Thr His Gln Ile His His Leu
275 280 285

Phe Pro Ile Ile Pro His Tyr Lys Leu Asn Arg Ala Thr Ala Ala Phe

290 295 300

His Gln Ala Phe Pro Glu Leu Val Arg Lys Ser Asp Glu Pro Ile Leu
305 310 315 320

Lys Ala Phe Trp Arg Val Gly Arg Leu Tyr Ala Asn Tyr Gly Val Val
325 330 335

Asp Pro Asp Ala Lys Leu Phe Thr Leu Lys Glu Ala Lys Ala Ala Ser
340 345 350

Glu Ala Ala Thr Lys Thr Lys Ala Thr
355 360

<210> 85
<211> 1086
<212> DNA
<213> Phytophthora ramorum

<220>
<221> CDS
<222> (1)..(1086)
<223> synthetic delta-17 desaturase (codon-optimized for Yarrowia lipolytica)

<300>
<302> DELTA-17 DESATURASES AND THEIR USE IN MAKING POLYUNSATURATED FATTY ACIDS

<310> WO 2007/123999
<311> 2007-04-19
<312> 2007-11-01
<313> (1)..(1086)

<300>
<302> DELTA-17 DESATURASES AND THEIR USE IN MAKING POLYUNSATURATED FATTY ACIDS

<310> US 2007-0249026-A1
<311> 2007-04-18
<312> 2007-10-25
<313> (1)..(1086)

<400> 85

```
atg gct acc aag cag ccc tac cag ttc cct act ctg acc gag atc aag          48
Met Ala Thr Lys Gln Pro Tyr Gln Phe Pro Thr Leu Thr Glu Ile Lys
1               5                   10                  15

cga tct ctt ccc tcc gag tgc ttt gaa gcc tcg gtc cct ctg tcc ttg          96
Arg Ser Leu Pro Ser Glu Cys Phe Glu Ala Ser Val Pro Leu Ser Leu
            20                  25                  30

tac tac acc gtg cga atc gtc gct att gcc gtt gct ctg gcc ttc gga         144
Tyr Tyr Thr Val Arg Ile Val Ala Ile Ala Val Ala Leu Ala Phe Gly
        35                  40                  45

ctc aac tac gct cga gcc ctt ccc gtg gtc gag tct ctg tgg gca ctc         192
Leu Asn Tyr Ala Arg Ala Leu Pro Val Val Glu Ser Leu Trp Ala Leu
    50                  55                  60

gac gct gcc ctt tgt tgc ggt tac gtt ctg ctc caa ggc att gtc ttc         240
```

```
Asp Ala Ala Leu Cys Cys Gly Tyr Val Leu Leu Gln Gly Ile Val Phe
65                  70                  75                  80

tgg gga ttc ttt acc gtg ggt cac gat gct gga cat ggt gcc ttc tct        288
Trp Gly Phe Phe Thr Val Gly His Asp Ala Gly His Gly Ala Phe Ser
                85                  90                  95

cga tac cac ctg ctc aac ttt gtc gtt ggc acc ttt atc cac tcc ctc        336
Arg Tyr His Leu Leu Asn Phe Val Val Gly Thr Phe Ile His Ser Leu
            100                 105                 110

att ctt act ccc ttc gag tcg tgg aag ctc aca cat cga cac cat cac        384
Ile Leu Thr Pro Phe Glu Ser Trp Lys Leu Thr His Arg His His His
        115                 120                 125

aag aac acc gga aac atc gac cga gac gaa atc ttc tac cct cag cga        432
Lys Asn Thr Gly Asn Ile Asp Arg Asp Glu Ile Phe Tyr Pro Gln Arg
    130                 135                 140

aag gcc gac gat cat cct ctg tct cga aac ctc gtc ctg gct ctc ggt        480
Lys Ala Asp Asp His Pro Leu Ser Arg Asn Leu Val Leu Ala Leu Gly
145                 150                 155                 160

gcc gct tgg ttt gcc tac ctt gtc gag ggc ttt cct ccc cga aag gtc        528
Ala Ala Trp Phe Ala Tyr Leu Val Glu Gly Phe Pro Pro Arg Lys Val
                165                 170                 175

aac cac ttc aac ccc ttc gaa cct ctg ttt gtg cga cag gtg gct gcc        576
Asn His Phe Asn Pro Phe Glu Pro Leu Phe Val Arg Gln Val Ala Ala
            180                 185                 190

gtt gtc att tcc ctc tct gct cac ttc gcc gtc ctg gca ctg tcc gtg        624
Val Val Ile Ser Leu Ser Ala His Phe Ala Val Leu Ala Leu Ser Val
        195                 200                 205

tat ctg agc ttt cag ttc ggt ctc aag aca atg gct ctg tac tac tat        672
Tyr Leu Ser Phe Gln Phe Gly Leu Lys Thr Met Ala Leu Tyr Tyr Tyr
    210                 215                 220

gga ccc gtc ttc gtg ttc ggc tcc atg ctc gtc att act acc ttt ctg        720
Gly Pro Val Phe Val Phe Gly Ser Met Leu Val Ile Thr Thr Phe Leu
225                 230                 235                 240

cat cac aat gac gag gaa act cct tgg tac gga gat tcc gac tgg acc        768
His His Asn Asp Glu Glu Thr Pro Trp Tyr Gly Asp Ser Asp Trp Thr
                245                 250                 255

tac gtc aag ggc aac ttg tct tcc gtg gac cga tct tac ggt gcc ttc        816
Tyr Val Lys Gly Asn Leu Ser Ser Val Asp Arg Ser Tyr Gly Ala Phe
            260                 265                 270

atc gac aac ctc tcg cac aac att ggc aca cac cag atc cac cat ctg        864
Ile Asp Asn Leu Ser His Asn Ile Gly Thr His Gln Ile His His Leu
        275                 280                 285

ttt ccc atc att cct cac tac aag ctc aac cga gcc acc gct gcc ttc        912
Phe Pro Ile Ile Pro His Tyr Lys Leu Asn Arg Ala Thr Ala Ala Phe
    290                 295                 300

cac cag gcc ttt ccc gaa ctt gtc cga aag agc gac gag ccc att ctc        960
His Gln Ala Phe Pro Glu Leu Val Arg Lys Ser Asp Glu Pro Ile Leu
305                 310                 315                 320

aag gct ttc tgg aga gtt ggt cga ctt tac gcc aac tac gga gtc gtg       1008
Lys Ala Phe Trp Arg Val Gly Arg Leu Tyr Ala Asn Tyr Gly Val Val
```

```
                325                     330                     335

gat ccc gac gca aag ctg ttt act ctc aag gag gcc aaa gct gcc tcc       1056
Asp Pro Asp Ala Lys Leu Phe Thr Leu Lys Glu Ala Lys Ala Ala Ser
            340                     345                     350

gag gct gcc acc aag acc aag gct act taa                               1086
Glu Ala Ala Thr Lys Thr Lys Ala Thr
        355                     360
```

<210> 86
<211> 361
<212> PRT
<213> Phytophthora ramorum

<400> 86

```
Met Ala Thr Lys Gln Pro Tyr Gln Phe Pro Thr Leu Thr Glu Ile Lys
1               5                   10                  15

Arg Ser Leu Pro Ser Glu Cys Phe Glu Ala Ser Val Pro Leu Ser Leu
            20                  25                  30

Tyr Tyr Thr Val Arg Ile Val Ala Ile Ala Val Ala Leu Ala Phe Gly
        35                  40                  45

Leu Asn Tyr Ala Arg Ala Leu Pro Val Val Glu Ser Leu Trp Ala Leu
    50                  55                  60

Asp Ala Ala Leu Cys Cys Gly Tyr Val Leu Leu Gln Gly Ile Val Phe
65                  70                  75                  80

Trp Gly Phe Phe Thr Val Gly His Asp Ala Gly His Gly Ala Phe Ser
                85                  90                  95

Arg Tyr His Leu Leu Asn Phe Val Val Gly Thr Phe Ile His Ser Leu
            100                 105                 110

Ile Leu Thr Pro Phe Glu Ser Trp Lys Leu Thr His Arg His His His
        115                 120                 125

Lys Asn Thr Gly Asn Ile Asp Arg Asp Glu Ile Phe Tyr Pro Gln Arg
    130                 135                 140

Lys Ala Asp Asp His Pro Leu Ser Arg Asn Leu Val Leu Ala Leu Gly
145                 150                 155                 160

Ala Ala Trp Phe Ala Tyr Leu Val Glu Gly Phe Pro Pro Arg Lys Val
                165                 170                 175

Asn His Phe Asn Pro Phe Glu Pro Leu Phe Val Arg Gln Val Ala Ala
            180                 185                 190
```

```
Val Val Ile Ser Leu Ser Ala His Phe Ala Val Leu Ala Leu Ser Val
        195                 200                 205

Tyr Leu Ser Phe Gln Phe Gly Leu Lys Thr Met Ala Leu Tyr Tyr Tyr
    210                 215                 220

Gly Pro Val Phe Val Phe Gly Ser Met Leu Val Ile Thr Thr Phe Leu
225                 230                 235                 240

His His Asn Asp Glu Glu Thr Pro Trp Tyr Gly Asp Ser Asp Trp Thr
                245                 250                 255

Tyr Val Lys Gly Asn Leu Ser Ser Val Asp Arg Ser Tyr Gly Ala Phe
            260                 265                 270

Ile Asp Asn Leu Ser His Asn Ile Gly Thr His Gln Ile His His Leu
        275                 280                 285

Phe Pro Ile Ile Pro His Tyr Lys Leu Asn Arg Ala Thr Ala Ala Phe
    290                 295                 300

His Gln Ala Phe Pro Glu Leu Val Arg Lys Ser Asp Glu Pro Ile Leu
305                 310                 315                 320

Lys Ala Phe Trp Arg Val Gly Arg Leu Tyr Ala Asn Tyr Gly Val Val
                325                 330                 335

Asp Pro Asp Ala Lys Leu Phe Thr Leu Lys Glu Ala Lys Ala Ala Ser
            340                 345                 350

Glu Ala Ala Thr Lys Thr Lys Ala Thr
        355                 360
```

<210> 87
<211> 1080
<212> DNA
<213> Pythium aphanidermatum

<220>
<221> CDS
<222> (1)..(1080)
<223> delta-17 desaturase

<300>
<302> DELTA-17 DESATURASES AND THEIR USE IN MAKING POLYUNSATURATED FATTY ACIDS

<310> WO 2008/054565
<311> 2007-07-19
<312> 2008-05-08
<313> (1)..(1080)

<300>
<302> DELTA-17 DESATURASES AND THEIR USE IN MAKING POLYUNSATURATED FATTY ACIDS

<310> US 2008-0125326-A1
<311> 2007-07-19
<312> 2008-05-29
<313> (1)..(1080)

<400> 87

```
atg gct tct tcc act gtt gct gcg ccg tac gag ttc ccg acg ctg acg      48
Met Ala Ser Ser Thr Val Ala Ala Pro Tyr Glu Phe Pro Thr Leu Thr
1               5                   10                  15

gag atc aag cgc tcg ctg cca gcg cac tgc ttt gag gcc tcg gtc ccg      96
Glu Ile Lys Arg Ser Leu Pro Ala His Cys Phe Glu Ala Ser Val Pro
            20                  25                  30

tgg tcg ctc tac tac acc gtg cgc gcg ctg ggc atc gcc ggc tcg ctc      144
Trp Ser Leu Tyr Tyr Thr Val Arg Ala Leu Gly Ile Ala Gly Ser Leu
        35                  40                  45

gcg ctc ggc ctc tac tac gcg cgc gcg ctc gcg atc gtg cag gag ttt      192
Ala Leu Gly Leu Tyr Tyr Ala Arg Ala Leu Ala Ile Val Gln Glu Phe
    50                  55                  60

gcc ctg ctg gat gcg gtg ctc tgc acg ggg tac att ctg ctg cag ggc      240
Ala Leu Leu Asp Ala Val Leu Cys Thr Gly Tyr Ile Leu Leu Gln Gly
65                  70                  75                  80

atc gta ttc tgg ggg ttc ttc acc atc ggc cat gac tgc ggc cac ggc      288
Ile Val Phe Trp Gly Phe Phe Thr Ile Gly His Asp Cys Gly His Gly
                85                  90                  95

gcg ttc tcg cgt tcg cac ctg ctc aac ttc agc gtc ggc acg ctc att      336
Ala Phe Ser Arg Ser His Leu Leu Asn Phe Ser Val Gly Thr Leu Ile
            100                 105                 110

cac tcg atc atc ctc acg ccg tac gag tca tgg aag atc tcg cac cgc      384
His Ser Ile Ile Leu Thr Pro Tyr Glu Ser Trp Lys Ile Ser His Arg
        115                 120                 125

cac cac cac aag aac acg ggc aac atc gac aag gac gag att ttc tac      432
His His His Lys Asn Thr Gly Asn Ile Asp Lys Asp Glu Ile Phe Tyr
    130                 135                 140

ccg cag cgc gag gcc gac tcg cac cca ctg tcc cga cac atg gtg atc      480
Pro Gln Arg Glu Ala Asp Ser His Pro Leu Ser Arg His Met Val Ile
145                 150                 155                 160

tcg ctc ggc tcg gcc tgg ttc gcg tac ctc gtt gcg ggc ttc cct cct      528
Ser Leu Gly Ser Ala Trp Phe Ala Tyr Leu Val Ala Gly Phe Pro Pro
                165                 170                 175

cgc aag gtg aac cac ttc aac cct tgg gaa ccg ttg tac ctg cgc cgc      576
Arg Lys Val Asn His Phe Asn Pro Trp Glu Pro Leu Tyr Leu Arg Arg
            180                 185                 190

atg tct gcc gtc atc atc tca ctc ggc tcg ctc gtg gcg ttc gcg ggc      624
Met Ser Ala Val Ile Ile Ser Leu Gly Ser Leu Val Ala Phe Ala Gly
        195                 200                 205

ttg tat gcg tat ctc acc tac gtc tat ggc ctt aag acc atg gcg ctg      672
Leu Tyr Ala Tyr Leu Thr Tyr Val Tyr Gly Leu Lys Thr Met Ala Leu
    210                 215                 220

tac tac ttc gcc cct ctc ttt ggg ttc gcc acg atg ctc gtg gtc act      720
```

```
Tyr Tyr Phe Ala Pro Leu Phe Gly Phe Ala Thr Met Leu Val Val Thr
225                 230                 235                 240

acc ttt ttg cac cac aat gac gag gaa acg cca tgg tac gcc gac tcg        768
Thr Phe Leu His His Asn Asp Glu Glu Thr Pro Trp Tyr Ala Asp Ser
                245                 250                 255

gag tgg acg tac gtc aag ggc aac ctc tcg tcc gtg gac cgc tcg tac        816
Glu Trp Thr Tyr Val Lys Gly Asn Leu Ser Ser Val Asp Arg Ser Tyr
            260                 265                 270

ggc gcg ctc atc gac aac ctg agc cac aac atc ggc acg cac cag atc        864
Gly Ala Leu Ile Asp Asn Leu Ser His Asn Ile Gly Thr His Gln Ile
        275                 280                 285

cac cac ctg ttt ccg atc atc ccg cac tac aag ctg aac gag gcg acg        912
His His Leu Phe Pro Ile Ile Pro His Tyr Lys Leu Asn Glu Ala Thr
    290                 295                 300

gca gcg ttc gcg cag gcg ttc ccg gag ctc gtg cgc aag agc gcg tcg        960
Ala Ala Phe Ala Gln Ala Phe Pro Glu Leu Val Arg Lys Ser Ala Ser
305                 310                 315                 320

ccg atc atc ccg acg ttc atc cgc atc ggg ctc atg tac gcc aag tac        1008
Pro Ile Ile Pro Thr Phe Ile Arg Ile Gly Leu Met Tyr Ala Lys Tyr
                325                 330                 335

ggc gtc gtg gac aag gac gcc aag atg ttt acg ctc aag gag gcc aag        1056
Gly Val Val Asp Lys Asp Ala Lys Met Phe Thr Leu Lys Glu Ala Lys
            340                 345                 350

gcc gcc aag acc aag gcc aac tag                                        1080
Ala Ala Lys Thr Lys Ala Asn
        355
```

<210> 88
<211> 359
<212> PRT
<213> Pythium aphanidermatum

<400> 88

```
Met Ala Ser Ser Thr Val Ala Ala Pro Tyr Glu Phe Pro Thr Leu Thr
1               5                   10                  15

Glu Ile Lys Arg Ser Leu Pro Ala His Cys Phe Glu Ala Ser Val Pro
            20                  25                  30

Trp Ser Leu Tyr Tyr Thr Val Arg Ala Leu Gly Ile Ala Gly Ser Leu
        35                  40                  45

Ala Leu Gly Leu Tyr Tyr Ala Arg Ala Leu Ala Ile Val Gln Glu Phe
    50                  55                  60

Ala Leu Leu Asp Ala Val Leu Cys Thr Gly Tyr Ile Leu Leu Gln Gly
65                  70                  75                  80

Ile Val Phe Trp Gly Phe Phe Thr Ile Gly His Asp Cys Gly His Gly
                85                  90                  95
```

```
Ala Phe Ser Arg Ser His Leu Leu Asn Phe Ser Val Gly Thr Leu Ile
            100                 105                 110

His Ser Ile Ile Leu Thr Pro Tyr Glu Ser Trp Lys Ile Ser His Arg
        115                 120                 125

His His His Lys Asn Thr Gly Asn Ile Asp Lys Asp Glu Ile Phe Tyr
    130                 135                 140

Pro Gln Arg Glu Ala Asp Ser His Pro Leu Ser Arg His Met Val Ile
145                 150                 155                 160

Ser Leu Gly Ser Ala Trp Phe Ala Tyr Leu Val Ala Gly Phe Pro Pro
                165                 170                 175

Arg Lys Val Asn His Phe Asn Pro Trp Glu Pro Leu Tyr Leu Arg Arg
            180                 185                 190

Met Ser Ala Val Ile Ile Ser Leu Gly Ser Leu Val Ala Phe Ala Gly
        195                 200                 205

Leu Tyr Ala Tyr Leu Thr Tyr Val Tyr Gly Leu Lys Thr Met Ala Leu
    210                 215                 220

Tyr Tyr Phe Ala Pro Leu Phe Gly Phe Ala Thr Met Leu Val Val Thr
225                 230                 235                 240

Thr Phe Leu His His Asn Asp Glu Glu Thr Pro Trp Tyr Ala Asp Ser
                245                 250                 255

Glu Trp Thr Tyr Val Lys Gly Asn Leu Ser Ser Val Asp Arg Ser Tyr
            260                 265                 270

Gly Ala Leu Ile Asp Asn Leu Ser His Asn Ile Gly Thr His Gln Ile
        275                 280                 285

His His Leu Phe Pro Ile Ile Pro His Tyr Lys Leu Asn Glu Ala Thr
    290                 295                 300

Ala Ala Phe Ala Gln Ala Phe Pro Glu Leu Val Arg Lys Ser Ala Ser
305                 310                 315                 320

Pro Ile Ile Pro Thr Phe Ile Arg Ile Gly Leu Met Tyr Ala Lys Tyr
                325                 330                 335

Gly Val Val Asp Lys Asp Ala Lys Met Phe Thr Leu Lys Glu Ala Lys
            340                 345                 350
```

Ala Ala Lys Thr Lys Ala Asn
355

<210> 89
<211> 1080
<212> DNA
<213> Pythium aphanidermatum

<220>
<221> CDS
<222> (1)..(1080)
<223> synthetic delta-17 desaturase (codon-optimized for Yarrowia lipolytica)

<300>
<302> DELTA-17 DESATURASES AND THEIR USE IN MAKING POLYUNSATURATED FATTY ACIDS

<310> WO 2008/054565
<311> 2007-07-19
<312> 2008-05-08
<313> (1)..(1080)

<300>
<302> DELTA-17 DESATURASES AND THEIR USE IN MAKING POLYUNSATURATED FATTY ACIDS

<310> US 2008-0125326-A1
<311> 2007-07-19
<312> 2008-05-29
<313> (1)..(1080)

<400> 89

```
atg gct tcc tct acc gtt gcc gct ccc tac gag ttc cct act ctc acc        48
Met Ala Ser Ser Thr Val Ala Ala Pro Tyr Glu Phe Pro Thr Leu Thr
1               5                   10                  15

gag atc aag cga tcc ctg cct gcc cac tgc ttc gaa gcc tct gtt ccc        96
Glu Ile Lys Arg Ser Leu Pro Ala His Cys Phe Glu Ala Ser Val Pro
            20                  25                  30

tgg tcc ctc tac tat acc gtg cga gct ctg ggc att gcc ggt tcc ctt       144
Trp Ser Leu Tyr Tyr Thr Val Arg Ala Leu Gly Ile Ala Gly Ser Leu
        35                  40                  45

gct ctc gga ctg tac tat gct cga gcc ctt gct atc gtg cag gag ttt       192
Ala Leu Gly Leu Tyr Tyr Ala Arg Ala Leu Ala Ile Val Gln Glu Phe
    50                  55                  60

gca ctg ctc gat gcc gtc ctt tgc act ggc tac att ctg ctc cag ggt       240
Ala Leu Leu Asp Ala Val Leu Cys Thr Gly Tyr Ile Leu Leu Gln Gly
65                  70                  75                  80

atc gtg ttc tgg gga ttc ttt acc atc ggt cac gac tgt gga cat ggt       288
Ile Val Phe Trp Gly Phe Phe Thr Ile Gly His Asp Cys Gly His Gly
                85                  90                  95

gcc ttc tcg cga tcc cac ctg ctc aac ttc tct gtt ggc aca ctc att       336
Ala Phe Ser Arg Ser His Leu Leu Asn Phe Ser Val Gly Thr Leu Ile
            100                 105                 110

cac tcc atc att ctg act ccc tac gag tcg tgg aag atc agc cat cga       384
His Ser Ile Ile Leu Thr Pro Tyr Glu Ser Trp Lys Ile Ser His Arg
        115                 120                 125
```

```
cac cat cac aag aac acc ggc aac atc gac aag gat gag atc ttc tac        432
His His His Lys Asn Thr Gly Asn Ile Asp Lys Asp Glu Ile Phe Tyr
    130                 135                 140

cct cag cga gaa gcc gac tct cat ccc ctg tcc cga cac atg gtc atc        480
Pro Gln Arg Glu Ala Asp Ser His Pro Leu Ser Arg His Met Val Ile
145                 150                 155                 160

tcc ctt ggt tcg gct tgg ttt gcc tac ctc gtt gct gga ttt cct ccc        528
Ser Leu Gly Ser Ala Trp Phe Ala Tyr Leu Val Ala Gly Phe Pro Pro
                165                 170                 175

cga aag gtc aac cac ttc aat ccc tgg gag cct ctc tac ctg cga aga        576
Arg Lys Val Asn His Phe Asn Pro Trp Glu Pro Leu Tyr Leu Arg Arg
            180                 185                 190

atg tct gcc gtc atc att tcc ctc ggc tct ctc gtg gcc ttt gct ggt        624
Met Ser Ala Val Ile Ile Ser Leu Gly Ser Leu Val Ala Phe Ala Gly
        195                 200                 205

ctg tac gcc tac ctt acc tac gtc tac ggc ctc aag acc atg gct ctg        672
Leu Tyr Ala Tyr Leu Thr Tyr Val Tyr Gly Leu Lys Thr Met Ala Leu
    210                 215                 220

tat tac ttc gca cct ctc ttt gga ttc gcc acc atg ctg gtt gtc act        720
Tyr Tyr Phe Ala Pro Leu Phe Gly Phe Ala Thr Met Leu Val Val Thr
225                 230                 235                 240

acc ttc ctc cat cac aac gac gag gaa act ccc tgg tac gcc gat tcg        768
Thr Phe Leu His His Asn Asp Glu Glu Thr Pro Trp Tyr Ala Asp Ser
                245                 250                 255

gag tgg acc tat gtc aag ggc aac ttg tcc tct gtg gac cga agc tac        816
Glu Trp Thr Tyr Val Lys Gly Asn Leu Ser Ser Val Asp Arg Ser Tyr
            260                 265                 270

gga gcc ctc atc gac aac ctg tcc cac aac att ggt aca cat cag atc        864
Gly Ala Leu Ile Asp Asn Leu Ser His Asn Ile Gly Thr His Gln Ile
        275                 280                 285

cac cat ctg ttt ccc atc att cct cac tac aag ctc aac gag gcc act        912
His His Leu Phe Pro Ile Ile Pro His Tyr Lys Leu Asn Glu Ala Thr
    290                 295                 300

gct gcc ttc gct cag gcc ttt ccc gaa ctg gtg cga aag tcg gct tct        960
Ala Ala Phe Ala Gln Ala Phe Pro Glu Leu Val Arg Lys Ser Ala Ser
305                 310                 315                 320

ccc atc att ccc acc ttc atc cga att ggt ctt atg tac gcc aag tac       1008
Pro Ile Ile Pro Thr Phe Ile Arg Ile Gly Leu Met Tyr Ala Lys Tyr
                325                 330                 335

ggc gtg gtc gac aag gat gcc aag atg ttt acc ctc aag gag gcc aag       1056
Gly Val Val Asp Lys Asp Ala Lys Met Phe Thr Leu Lys Glu Ala Lys
            340                 345                 350

gct gcc aag acc aaa gcc aac taa                                       1080
Ala Ala Lys Thr Lys Ala Asn
        355
```

<210> 90

<211> 359
<212> PRT
<213> Pythium aphanidermatum

<400> 90

```
Met Ala Ser Ser Thr Val Ala Ala Pro Tyr Glu Phe Pro Thr Leu Thr
1               5                   10                  15

Glu Ile Lys Arg Ser Leu Pro Ala His Cys Phe Glu Ala Ser Val Pro
            20                  25                  30

Trp Ser Leu Tyr Tyr Thr Val Arg Ala Leu Gly Ile Ala Gly Ser Leu
        35                  40                  45

Ala Leu Gly Leu Tyr Tyr Ala Arg Ala Leu Ala Ile Val Gln Glu Phe
    50                  55                  60

Ala Leu Leu Asp Ala Val Leu Cys Thr Gly Tyr Ile Leu Leu Gln Gly
65                  70                  75                  80

Ile Val Phe Trp Gly Phe Phe Thr Ile Gly His Asp Cys Gly His Gly
                85                  90                  95

Ala Phe Ser Arg Ser His Leu Leu Asn Phe Ser Val Gly Thr Leu Ile
            100                 105                 110

His Ser Ile Ile Leu Thr Pro Tyr Glu Ser Trp Lys Ile Ser His Arg
        115                 120                 125

His His His Lys Asn Thr Gly Asn Ile Asp Lys Asp Glu Ile Phe Tyr
    130                 135                 140

Pro Gln Arg Glu Ala Asp Ser His Pro Leu Ser Arg His Met Val Ile
145                 150                 155                 160

Ser Leu Gly Ser Ala Trp Phe Ala Tyr Leu Val Ala Gly Phe Pro Pro
                165                 170                 175

Arg Lys Val Asn His Phe Asn Pro Trp Glu Pro Leu Tyr Leu Arg Arg
            180                 185                 190

Met Ser Ala Val Ile Ile Ser Leu Gly Ser Leu Val Ala Phe Ala Gly
        195                 200                 205

Leu Tyr Ala Tyr Leu Thr Tyr Val Tyr Gly Leu Lys Thr Met Ala Leu
    210                 215                 220

Tyr Tyr Phe Ala Pro Leu Phe Gly Phe Ala Thr Met Leu Val Val Thr
225                 230                 235                 240

Thr Phe Leu His His Asn Asp Glu Glu Thr Pro Trp Tyr Ala Asp Ser
```

```
                245                 250                 255

Glu Trp Thr Tyr Val Lys Gly Asn Leu Ser Ser Val Asp Arg Ser Tyr
            260                 265                 270

Gly Ala Leu Ile Asp Asn Leu Ser His Asn Ile Gly Thr His Gln Ile
        275                 280                 285

His His Leu Phe Pro Ile Ile Pro His Tyr Lys Leu Asn Glu Ala Thr
    290                 295                 300

Ala Ala Phe Ala Gln Ala Phe Pro Glu Leu Val Arg Lys Ser Ala Ser
305                 310                 315                 320

Pro Ile Ile Pro Thr Phe Ile Arg Ile Gly Leu Met Tyr Ala Lys Tyr
                325                 330                 335

Gly Val Val Asp Lys Asp Ala Lys Met Phe Thr Leu Lys Glu Ala Lys
            340                 345                 350

Ala Ala Lys Thr Lys Ala Asn
        355
```

<210> 91
<211> 1434
<212> DNA
<213> Fusarium monoliforme

<220>
<221> CDS
<222> (1)..(1434)
<223> delta-12 desaturase

<300>
<302> DELTA-12 DESATURASES SUITABLE FOR ALTERING LEVELS OF POLYUNSATURATED FATTY ACIDS IN OLEAGINOUS YEAST

<310> WO 2005/047485
<311> 2004-11-12
<312> 2005-05-26
<313> (1)..(1434)

<300>
<302> DELTA-12 DESATURASES SUITABLE FOR ALTERING LEVELS OF POLYUNSATURATED FATTY ACIDS IN OLEAGINOUS YEAST

<310> U.S. 7,504,259
<311> 2004-11-10
<312> 2009-03-17
<313> (1)..(1434)

<400> 91

```
atg gcg tcc act tcg gct ctg ccc aag cag aac cct gcg ctt aga cgc          48
Met Ala Ser Thr Ser Ala Leu Pro Lys Gln Asn Pro Ala Leu Arg Arg
1               5                   10                  15

acc gtc acc tca act act gtg acg gat tct gag tct gcc gcc gtc tct          96
Thr Val Thr Ser Thr Thr Val Thr Asp Ser Glu Ser Ala Ala Val Ser
```

```
            20                  25                  30

cct tca gac tct ccc cgc cac tcg gcc tct tcc aca tcg ctc tcg tcc        144
Pro Ser Asp Ser Pro Arg His Ser Ala Ser Ser Thr Ser Leu Ser Ser
        35                  40                  45

atg tcc gag gtt gat atc gcc aag ccc aag tcc gag tat ggt gtc atg        192
Met Ser Glu Val Asp Ile Ala Lys Pro Lys Ser Glu Tyr Gly Val Met
    50                  55                  60

ctc gac acc tac ggc aac cag ttc gag gtt ccc gac ttt acc atc aag        240
Leu Asp Thr Tyr Gly Asn Gln Phe Glu Val Pro Asp Phe Thr Ile Lys
65                  70                  75                  80

gac atc tac aat gcc atc cct aag cac tgc ttc aag cgc tcc gct ctc        288
Asp Ile Tyr Asn Ala Ile Pro Lys His Cys Phe Lys Arg Ser Ala Leu
                85                  90                  95

aag gga tac ggt tat atc ctc cgc gac att gtc ctc ctg act acc act        336
Lys Gly Tyr Gly Tyr Ile Leu Arg Asp Ile Val Leu Leu Thr Thr Thr
            100                 105                 110

ttc agc atc tgg tac aac ttt gtg acc ccc gaa tat atc ccc tcc acc        384
Phe Ser Ile Trp Tyr Asn Phe Val Thr Pro Glu Tyr Ile Pro Ser Thr
        115                 120                 125

ccc gcc cgc gct ggt ctg tgg gcc gtg tac acc gtt ctt cag ggt ctt        432
Pro Ala Arg Ala Gly Leu Trp Ala Val Tyr Thr Val Leu Gln Gly Leu
    130                 135                 140

ttc ggt act ggt ctc tgg gtt att gcc cat gag tgc ggt cac ggt gct        480
Phe Gly Thr Gly Leu Trp Val Ile Ala His Glu Cys Gly His Gly Ala
145                 150                 155                 160

ttc tcc gat tct cgc atc atc aac gac att act ggc tgg gtt ctt cac        528
Phe Ser Asp Ser Arg Ile Ile Asn Asp Ile Thr Gly Trp Val Leu His
                165                 170                 175

tct tcc ctc ctt gtc ccc tac ttc agc tgg caa atc tcc cac cga aag        576
Ser Ser Leu Leu Val Pro Tyr Phe Ser Trp Gln Ile Ser His Arg Lys
            180                 185                 190

cac cac aag gcc act ggc aac atg gag cgt gac atg gtc ttc gtt ccc        624
His His Lys Ala Thr Gly Asn Met Glu Arg Asp Met Val Phe Val Pro
        195                 200                 205

cga acc cgc gag cag cag gct act cgt ctc gga aag atg acc cac gag        672
Arg Thr Arg Glu Gln Gln Ala Thr Arg Leu Gly Lys Met Thr His Glu
    210                 215                 220

ctc gct cat ctt act gag gag acc ccc gct ttc act ctt ctc atg ctc        720
Leu Ala His Leu Thr Glu Glu Thr Pro Ala Phe Thr Leu Leu Met Leu
225                 230                 235                 240

gtc ctt cag cag ctc gtt ggc tgg ccc aac tac ctc atc acc aat gtt        768
Val Leu Gln Gln Leu Val Gly Trp Pro Asn Tyr Leu Ile Thr Asn Val
                245                 250                 255

acc ggc cac aac tac cac gag cgc cag cgt gag ggt cgc ggc aag ggc        816
Thr Gly His Asn Tyr His Glu Arg Gln Arg Glu Gly Arg Gly Lys Gly
            260                 265                 270

aag cat aac ggc ctc ggc ggt ggt gtt aac cac ttc gat ccc cgc agc        864
Lys His Asn Gly Leu Gly Gly Gly Val Asn His Phe Asp Pro Arg Ser
        275                 280                 285
```

```
cct ctg tac gag aac agt gac gct aag ctc atc gtc ctc agc gat att        912
Pro Leu Tyr Glu Asn Ser Asp Ala Lys Leu Ile Val Leu Ser Asp Ile
    290                 295                 300

ggt atc ggt ctg atg gcc act gct ctg tac ttc ctc gtt cag aag ttc        960
Gly Ile Gly Leu Met Ala Thr Ala Leu Tyr Phe Leu Val Gln Lys Phe
305                 310                 315                 320

ggt ttc tac aac atg gcc atc tgg tac ttt gtt ccc tac ctc tgg gtt       1008
Gly Phe Tyr Asn Met Ala Ile Trp Tyr Phe Val Pro Tyr Leu Trp Val
                325                 330                 335

aac cac tgg ctc gtt gcc atc acc ttc ctc cag cac acc gac cct acc       1056
Asn His Trp Leu Val Ala Ile Thr Phe Leu Gln His Thr Asp Pro Thr
            340                 345                 350

ctt ccc cac tac acc aac gac gag tgg aac ttc gtc cgt ggt gcc gct       1104
Leu Pro His Tyr Thr Asn Asp Glu Trp Asn Phe Val Arg Gly Ala Ala
        355                 360                 365

gct acc att gac cgt gag atg ggc ttc atc ggc cgc cac ctt ctc cac       1152
Ala Thr Ile Asp Arg Glu Met Gly Phe Ile Gly Arg His Leu Leu His
    370                 375                 380

ggc atc atc gag act cat gtc ctc cac cac tac gtc agc agc atc ccc       1200
Gly Ile Ile Glu Thr His Val Leu His His Tyr Val Ser Ser Ile Pro
385                 390                 395                 400

ttc tac aac gcg gac gag gcc acc gag gcc att aag ccc atc atg ggc       1248
Phe Tyr Asn Ala Asp Glu Ala Thr Glu Ala Ile Lys Pro Ile Met Gly
                405                 410                 415

aag cac tac cgg gct gat gtc cag gat ggt cct cgt ggc ttc atc cgc       1296
Lys His Tyr Arg Ala Asp Val Gln Asp Gly Pro Arg Gly Phe Ile Arg
            420                 425                 430

gcc atg tac cgc agt gcg cgt atg tgc cag tgg gtt gag ccc agc gct       1344
Ala Met Tyr Arg Ser Ala Arg Met Cys Gln Trp Val Glu Pro Ser Ala
        435                 440                 445

ggt gcc gag ggt gct ggt aag ggt gtt ctg ttc ttc cgc aac cgc aac       1392
Gly Ala Glu Gly Ala Gly Lys Gly Val Leu Phe Phe Arg Asn Arg Asn
    450                 455                 460

aac gtg ggc acc ccc ccc gct gtt atc aag ccc gtt gct taa               1434
Asn Val Gly Thr Pro Pro Ala Val Ile Lys Pro Val Ala
465                 470                 475
```

<210> 92
<211> 477
<212> PRT
<213> Fusarium monoliforme

<400> 92

```
Met Ala Ser Thr Ser Ala Leu Pro Lys Gln Asn Pro Ala Leu Arg Arg
1               5                   10                  15

Thr Val Thr Ser Thr Thr Val Thr Asp Ser Glu Ser Ala Ala Val Ser
            20                  25                  30
```

Pro Ser Asp Ser Pro Arg His Ser Ala Ser Ser Thr Ser Leu Ser Ser
35 40 45

Met Ser Glu Val Asp Ile Ala Lys Pro Lys Ser Glu Tyr Gly Val Met
50 55 60

Leu Asp Thr Tyr Gly Asn Gln Phe Glu Val Pro Asp Phe Thr Ile Lys
65 70 75 80

Asp Ile Tyr Asn Ala Ile Pro Lys His Cys Phe Lys Arg Ser Ala Leu
85 90 95

Lys Gly Tyr Gly Tyr Ile Leu Arg Asp Ile Val Leu Leu Thr Thr Thr
100 105 110

Phe Ser Ile Trp Tyr Asn Phe Val Thr Pro Glu Tyr Ile Pro Ser Thr
115 120 125

Pro Ala Arg Ala Gly Leu Trp Ala Val Tyr Thr Val Leu Gln Gly Leu
130 135 140

Phe Gly Thr Gly Leu Trp Val Ile Ala His Glu Cys Gly His Gly Ala
145 150 155 160

Phe Ser Asp Ser Arg Ile Ile Asn Asp Ile Thr Gly Trp Val Leu His
165 170 175

Ser Ser Leu Leu Val Pro Tyr Phe Ser Trp Gln Ile Ser His Arg Lys
180 185 190

His His Lys Ala Thr Gly Asn Met Glu Arg Asp Met Val Phe Val Pro
195 200 205

Arg Thr Arg Glu Gln Gln Ala Thr Arg Leu Gly Lys Met Thr His Glu
210 215 220

Leu Ala His Leu Thr Glu Glu Thr Pro Ala Phe Thr Leu Leu Met Leu
225 230 235 240

Val Leu Gln Gln Leu Val Gly Trp Pro Asn Tyr Leu Ile Thr Asn Val
245 250 255

Thr Gly His Asn Tyr His Glu Arg Gln Arg Glu Gly Arg Gly Lys Gly
260 265 270

Lys His Asn Gly Leu Gly Gly Gly Val Asn His Phe Asp Pro Arg Ser
275 280 285

Pro Leu Tyr Glu Asn Ser Asp Ala Lys Leu Ile Val Leu Ser Asp Ile

```
    290                 295                 300

Gly Ile Gly Leu Met Ala Thr Ala Leu Tyr Phe Leu Val Gln Lys Phe
305                 310                 315                 320

Gly Phe Tyr Asn Met Ala Ile Trp Tyr Phe Val Pro Tyr Leu Trp Val
                325                 330                 335

Asn His Trp Leu Val Ala Ile Thr Phe Leu Gln His Thr Asp Pro Thr
            340                 345                 350

Leu Pro His Tyr Thr Asn Asp Glu Trp Asn Phe Val Arg Gly Ala Ala
        355                 360                 365

Ala Thr Ile Asp Arg Glu Met Gly Phe Ile Gly Arg His Leu Leu His
    370                 375                 380

Gly Ile Ile Glu Thr His Val Leu His His Tyr Val Ser Ser Ile Pro
385                 390                 395                 400

Phe Tyr Asn Ala Asp Glu Ala Thr Glu Ala Ile Lys Pro Ile Met Gly
                405                 410                 415

Lys His Tyr Arg Ala Asp Val Gln Asp Gly Pro Arg Gly Phe Ile Arg
            420                 425                 430

Ala Met Tyr Arg Ser Ala Arg Met Cys Gln Trp Val Glu Pro Ser Ala
        435                 440                 445

Gly Ala Glu Gly Ala Gly Lys Gly Val Leu Phe Phe Arg Asn Arg Asn
    450                 455                 460

Asn Val Gly Thr Pro Pro Ala Val Ile Lys Pro Val Ala
465                 470                 475
```

<210> 93
<211> 1434
<212> DNA
<213> Fusarium moniliforme

<220>
<221> CDS
<222> (1)..(1434)
<223> synthetic delta-12 desaturase (codon-optimized for Yarrowia lipolytica)

<300>
<302> DELTA-12 DESATURASES SUITABLE FOR ALTERING LEVELS OF POLYUNSATURATED FATTY ACIDS IN OLEAGINOUS YEAST

<310> WO 2005/047485
<311> 2004-11-12
<312> 2005-05-26
<313> (1)..(1434)

<300>
<302> DELTA-12 DESATURASES SUITABLE FOR ALTERING LEVELS OF POLYUNSATURATED FATTY ACIDS IN OLEAGINOUS YEAST

<310> U.S. 7,504,259
<311> 2004-11-10
<312> 2009-03-17
<313> (1)..(1434)

<400> 93

```
atg gcc tcc acc tcg gct ctg ccc aag cag aac cct gcc ctc cga cga        48
Met Ala Ser Thr Ser Ala Leu Pro Lys Gln Asn Pro Ala Leu Arg Arg
1               5                   10                  15

acc gtc act tcc acc act gtg acc gac tcg gag tct gct gcc gtc tct        96
Thr Val Thr Ser Thr Thr Val Thr Asp Ser Glu Ser Ala Ala Val Ser
            20                  25                  30

ccc tcc gat tct ccc aga cac tcg gcc tcc tct aca tcg ctg tct tcc       144
Pro Ser Asp Ser Pro Arg His Ser Ala Ser Ser Thr Ser Leu Ser Ser
        35                  40                  45

atg tcc gag gtg gac att gcc aag ccc aag tcc gag tac ggt gtc atg       192
Met Ser Glu Val Asp Ile Ala Lys Pro Lys Ser Glu Tyr Gly Val Met
    50                  55                  60

ctg gat acc tac ggc aac cag ttc gaa gtt ccc gac ttc acc atc aag       240
Leu Asp Thr Tyr Gly Asn Gln Phe Glu Val Pro Asp Phe Thr Ile Lys
65                  70                  75                  80

gac atc tac aac gct att ccc aag cac tgc ttc aag cga tct gct ctc       288
Asp Ile Tyr Asn Ala Ile Pro Lys His Cys Phe Lys Arg Ser Ala Leu
                85                  90                  95

aag gga tac ggc tac att ctt cga gac att gtc ctc ctg act acc act       336
Lys Gly Tyr Gly Tyr Ile Leu Arg Asp Ile Val Leu Leu Thr Thr Thr
            100                 105                 110

ttc agc atc tgg tac aac ttt gtg aca ccc gag tac att ccc tcc act       384
Phe Ser Ile Trp Tyr Asn Phe Val Thr Pro Glu Tyr Ile Pro Ser Thr
        115                 120                 125

cct gct cga gcc ggt ctg tgg gct gtg tac acc gtt ctt cag gga ctc       432
Pro Ala Arg Ala Gly Leu Trp Ala Val Tyr Thr Val Leu Gln Gly Leu
    130                 135                 140

ttc ggt act gga ctg tgg gtc att gcc cac gag tgt gga cat ggt gct       480
Phe Gly Thr Gly Leu Trp Val Ile Ala His Glu Cys Gly His Gly Ala
145                 150                 155                 160

ttc tcc gat tcc cga atc atc aac gac att act ggc tgg gtg ctt cac       528
Phe Ser Asp Ser Arg Ile Ile Asn Asp Ile Thr Gly Trp Val Leu His
                165                 170                 175

tct tcc ctg ctt gtt ccc tac ttc agc tgg caa atc tcc cac cgg aag       576
Ser Ser Leu Leu Val Pro Tyr Phe Ser Trp Gln Ile Ser His Arg Lys
            180                 185                 190

cat cac aag gcc act gga aac atg gag cga gac atg gtc ttc gtt cct       624
His His Lys Ala Thr Gly Asn Met Glu Arg Asp Met Val Phe Val Pro
        195                 200                 205

cga acc cga gag cag caa gct act cga ctc ggc aag atg acc cac gaa       672
Arg Thr Arg Glu Gln Gln Ala Thr Arg Leu Gly Lys Met Thr His Glu
```

```
    210                 215                 220

ctc gcc cat ctt acc gag gaa act cct gct ttc acc ctg ctc atg ctt        720
Leu Ala His Leu Thr Glu Glu Thr Pro Ala Phe Thr Leu Leu Met Leu
225                 230                 235                 240

gtg ctt cag caa ctg gtc ggt tgg ccc aac tat ctc att acc aac gtt        768
Val Leu Gln Gln Leu Val Gly Trp Pro Asn Tyr Leu Ile Thr Asn Val
                245                 250                 255

act gga cac aac tac cat gag cgg cag cga gag ggt cga ggc aag gga        816
Thr Gly His Asn Tyr His Glu Arg Gln Arg Glu Gly Arg Gly Lys Gly
            260                 265                 270

aag cac aac ggt ctt ggc ggt gga gtt aac cat ttc gat ccc cga tct        864
Lys His Asn Gly Leu Gly Gly Gly Val Asn His Phe Asp Pro Arg Ser
        275                 280                 285

cct ctg tac gag aac agc gac gcc aag ctc atc gtg ctc tcc gac att        912
Pro Leu Tyr Glu Asn Ser Asp Ala Lys Leu Ile Val Leu Ser Asp Ile
    290                 295                 300

ggc att ggt ctt atg gcc acc gct ctg tac ttt ctc gtt cag aag ttc        960
Gly Ile Gly Leu Met Ala Thr Ala Leu Tyr Phe Leu Val Gln Lys Phe
305                 310                 315                 320

gga ttc tac aac atg gcc atc tgg tac ttc gtt ccc tac ttg tgg gtt       1008
Gly Phe Tyr Asn Met Ala Ile Trp Tyr Phe Val Pro Tyr Leu Trp Val
                325                 330                 335

aac cac tgg ctc gtc gcc att acc ttt ctg cag cac aca gat cct act       1056
Asn His Trp Leu Val Ala Ile Thr Phe Leu Gln His Thr Asp Pro Thr
            340                 345                 350

ctt ccc cac tac acc aac gac gag tgg aac ttt gtg cga ggt gcc gct       1104
Leu Pro His Tyr Thr Asn Asp Glu Trp Asn Phe Val Arg Gly Ala Ala
        355                 360                 365

gca acc atc gac cga gag atg ggc ttc att gga cgt cat ctg ctc cac       1152
Ala Thr Ile Asp Arg Glu Met Gly Phe Ile Gly Arg His Leu Leu His
    370                 375                 380

ggc att atc gag act cac gtc ctg cat cac tac gtc tct tcc att ccc       1200
Gly Ile Ile Glu Thr His Val Leu His His Tyr Val Ser Ser Ile Pro
385                 390                 395                 400

ttc tac aat gcg gac gaa gct acc gag gcc atc aaa cct atc atg ggc       1248
Phe Tyr Asn Ala Asp Glu Ala Thr Glu Ala Ile Lys Pro Ile Met Gly
                405                 410                 415

aag cac tat cga gct gat gtc cag gac ggt cct cga gga ttc att cga       1296
Lys His Tyr Arg Ala Asp Val Gln Asp Gly Pro Arg Gly Phe Ile Arg
            420                 425                 430

gcc atg tac cga tct gca cga atg tgc cag tgg gtt gaa ccc tcc gct       1344
Ala Met Tyr Arg Ser Ala Arg Met Cys Gln Trp Val Glu Pro Ser Ala
        435                 440                 445

ggt gcc gag gga gct ggc aag ggt gtc ctg ttc ttt cga aac cga aac       1392
Gly Ala Glu Gly Ala Gly Lys Gly Val Leu Phe Phe Arg Asn Arg Asn
    450                 455                 460

aat gtg ggc act cct ccc gct gtc atc aag ccc gtt gcc taa               1434
Asn Val Gly Thr Pro Pro Ala Val Ile Lys Pro Val Ala
465                 470                 475
```

<210> 94
<211> 477
<212> PRT
<213> Fusarium moniliforme

<400> 94

```
Met Ala Ser Thr Ser Ala Leu Pro Lys Gln Asn Pro Ala Leu Arg Arg
1               5                   10                  15

Thr Val Thr Ser Thr Thr Val Thr Asp Ser Glu Ser Ala Ala Val Ser
            20                  25                  30

Pro Ser Asp Ser Pro Arg His Ser Ala Ser Ser Thr Ser Leu Ser Ser
        35                  40                  45

Met Ser Glu Val Asp Ile Ala Lys Pro Lys Ser Glu Tyr Gly Val Met
    50                  55                  60

Leu Asp Thr Tyr Gly Asn Gln Phe Glu Val Pro Asp Phe Thr Ile Lys
65                  70                  75                  80

Asp Ile Tyr Asn Ala Ile Pro Lys His Cys Phe Lys Arg Ser Ala Leu
                85                  90                  95

Lys Gly Tyr Gly Tyr Ile Leu Arg Asp Ile Val Leu Leu Thr Thr Thr
            100                 105                 110

Phe Ser Ile Trp Tyr Asn Phe Val Thr Pro Glu Tyr Ile Pro Ser Thr
        115                 120                 125

Pro Ala Arg Ala Gly Leu Trp Ala Val Tyr Thr Val Leu Gln Gly Leu
    130                 135                 140

Phe Gly Thr Gly Leu Trp Val Ile Ala His Glu Cys Gly His Gly Ala
145                 150                 155                 160

Phe Ser Asp Ser Arg Ile Ile Asn Asp Ile Thr Gly Trp Val Leu His
                165                 170                 175

Ser Ser Leu Leu Val Pro Tyr Phe Ser Trp Gln Ile Ser His Arg Lys
            180                 185                 190

His His Lys Ala Thr Gly Asn Met Glu Arg Asp Met Val Phe Val Pro
        195                 200                 205

Arg Thr Arg Glu Gln Gln Ala Thr Arg Leu Gly Lys Met Thr His Glu
    210                 215                 220
```

```
Leu Ala His Leu Thr Glu Glu Thr Pro Ala Phe Thr Leu Leu Met Leu
225                 230                 235                 240

Val Leu Gln Gln Leu Val Gly Trp Pro Asn Tyr Leu Ile Thr Asn Val
                245                 250                 255

Thr Gly His Asn Tyr His Glu Arg Gln Arg Glu Gly Arg Gly Lys Gly
            260                 265                 270

Lys His Asn Gly Leu Gly Gly Gly Val Asn His Phe Asp Pro Arg Ser
        275                 280                 285

Pro Leu Tyr Glu Asn Ser Asp Ala Lys Leu Ile Val Leu Ser Asp Ile
    290                 295                 300

Gly Ile Gly Leu Met Ala Thr Ala Leu Tyr Phe Leu Val Gln Lys Phe
305                 310                 315                 320

Gly Phe Tyr Asn Met Ala Ile Trp Tyr Phe Val Pro Tyr Leu Trp Val
                325                 330                 335

Asn His Trp Leu Val Ala Ile Thr Phe Leu Gln His Thr Asp Pro Thr
            340                 345                 350

Leu Pro His Tyr Thr Asn Asp Glu Trp Asn Phe Val Arg Gly Ala Ala
        355                 360                 365

Ala Thr Ile Asp Arg Glu Met Gly Phe Ile Gly Arg His Leu Leu His
    370                 375                 380

Gly Ile Ile Glu Thr His Val Leu His His Tyr Val Ser Ser Ile Pro
385                 390                 395                 400

Phe Tyr Asn Ala Asp Glu Ala Thr Glu Ala Ile Lys Pro Ile Met Gly
                405                 410                 415

Lys His Tyr Arg Ala Asp Val Gln Asp Gly Pro Arg Gly Phe Ile Arg
            420                 425                 430

Ala Met Tyr Arg Ser Ala Arg Met Cys Gln Trp Val Glu Pro Ser Ala
        435                 440                 445

Gly Ala Glu Gly Ala Gly Lys Gly Val Leu Phe Phe Arg Asn Arg Asn
    450                 455                 460

Asn Val Gly Thr Pro Pro Ala Val Ile Lys Pro Val Ala
465                 470                 475
```

<210> 95
<211> 828
<212> DNA
<213> Mortierella alpina

<220>
<221> CDS
<222> (1)..(828)
<223> C16/18 elongase

<300>
<302> A MORTIERELLA ALPINA C16/18 FATTY ACID ELONGASE

<310> WO 2007/046817
<311> 2005-11-04
<312> 2007-04-26
<313> (1)..(828)

<300>
<302> A MORTIERELLA ALPINA C16/18 FATTY ACID ELONGASE

<310> US 2007-0087420-A1
<311> 2005-10-19
<312> 2007-04-19
<313> (1)..(828)

<400> 95

```
atg gag tct gga cct atg cct gcc ggg atc ccc ttc cct gaa tac tat      48
Met Glu Ser Gly Pro Met Pro Ala Gly Ile Pro Phe Pro Glu Tyr Tyr
1               5                   10                  15

gac ttt ttc atg gac tgg aag aca ccc ctg gca att gct gcc acc tac      96
Asp Phe Phe Met Asp Trp Lys Thr Pro Leu Ala Ile Ala Ala Thr Tyr
            20                  25                  30

acc gcc gct gtt ggg ctc ttc aac ccc aag gtt ggc aaa gtc tcg cgc     144
Thr Ala Ala Val Gly Leu Phe Asn Pro Lys Val Gly Lys Val Ser Arg
        35                  40                  45

gtg gta gcc aag tcg gct aac gcc aag ccg gca gag cgc acg cag tcc     192
Val Val Ala Lys Ser Ala Asn Ala Lys Pro Ala Glu Arg Thr Gln Ser
    50                  55                  60

ggc gcc gcc atg acc gcc ttt gtc ttt gtc cac aac ctt atc ctc tgc     240
Gly Ala Ala Met Thr Ala Phe Val Phe Val His Asn Leu Ile Leu Cys
65                  70                  75                  80

gtg tac tct gga atc acc ttc tac tac atg ttc cca gcc atg gtc aag     288
Val Tyr Ser Gly Ile Thr Phe Tyr Tyr Met Phe Pro Ala Met Val Lys
                85                  90                  95

aac ttt aga aca cat acc ctc cat gag gcc tac tgc gat acg gat cag     336
Asn Phe Arg Thr His Thr Leu His Glu Ala Tyr Cys Asp Thr Asp Gln
            100                 105                 110

agc ctg tgg aac aac gcc ctt ggc tac tgg ggc tac ctc ttc tac ctt     384
Ser Leu Trp Asn Asn Ala Leu Gly Tyr Trp Gly Tyr Leu Phe Tyr Leu
        115                 120                 125

tca aag ttt tac gag gtc att gac acc atc atc atc atc ttg aag ggg     432
Ser Lys Phe Tyr Glu Val Ile Asp Thr Ile Ile Ile Ile Leu Lys Gly
    130                 135                 140

cgc cgc tcg tcc ctg ctc cag acc tac cac cac gcc ggc gct atg atc     480
Arg Arg Ser Ser Leu Leu Gln Thr Tyr His His Ala Gly Ala Met Ile
145                 150                 155                 160
```

```
acc atg tgg tcc ggc atc aac tac cag gca acg ccc att tgg att ttt      528
Thr Met Trp Ser Gly Ile Asn Tyr Gln Ala Thr Pro Ile Trp Ile Phe
                165                 170                 175

gtc gtc ttc aac tcg ttc atc cac acc atc atg tac tgt tac tat gcc      576
Val Val Phe Asn Ser Phe Ile His Thr Ile Met Tyr Cys Tyr Tyr Ala
            180                 185                 190

ttc acc tca atc ggc ttc cac ccc cca ggc aag aag tac ctc acc tcc      624
Phe Thr Ser Ile Gly Phe His Pro Pro Gly Lys Lys Tyr Leu Thr Ser
        195                 200                 205

atg cag atc acc cag ttt ttg gtc ggc atc act atc gcc gtc tct tat      672
Met Gln Ile Thr Gln Phe Leu Val Gly Ile Thr Ile Ala Val Ser Tyr
    210                 215                 220

ctc ttc gtc cct gga tgt atc cgc aca ccc ggt gct cag atg gct gtc      720
Leu Phe Val Pro Gly Cys Ile Arg Thr Pro Gly Ala Gln Met Ala Val
225                 230                 235                 240

tgg atc aac gtc gga tac ctc ttt ccc ctc act tat ctc ttt gtg gat      768
Trp Ile Asn Val Gly Tyr Leu Phe Pro Leu Thr Tyr Leu Phe Val Asp
                245                 250                 255

ttt gcc aag cgt act tac tcc aag cgt agt gcc atc gcc gct cag aag      816
Phe Ala Lys Arg Thr Tyr Ser Lys Arg Ser Ala Ile Ala Ala Gln Lys
            260                 265                 270

aag gcc cag taa                                                      828
Lys Ala Gln
        275
```

<210> 96
<211> 275
<212> PRT
<213> Mortierella alpina

<400> 96

```
Met Glu Ser Gly Pro Met Pro Ala Gly Ile Pro Phe Pro Glu Tyr Tyr
1               5                   10                  15

Asp Phe Phe Met Asp Trp Lys Thr Pro Leu Ala Ile Ala Ala Thr Tyr
            20                  25                  30

Thr Ala Ala Val Gly Leu Phe Asn Pro Lys Val Gly Lys Val Ser Arg
        35                  40                  45

Val Val Ala Lys Ser Ala Asn Ala Lys Pro Ala Glu Arg Thr Gln Ser
    50                  55                  60

Gly Ala Ala Met Thr Ala Phe Val Phe Val His Asn Leu Ile Leu Cys
65                  70                  75                  80

Val Tyr Ser Gly Ile Thr Phe Tyr Tyr Met Phe Pro Ala Met Val Lys
                85                  90                  95

Asn Phe Arg Thr His Thr Leu His Glu Ala Tyr Cys Asp Thr Asp Gln
```

```
                100                 105                 110

Ser Leu Trp Asn Asn Ala Leu Gly Tyr Trp Gly Tyr Leu Phe Tyr Leu
        115                 120                 125

Ser Lys Phe Tyr Glu Val Ile Asp Thr Ile Ile Ile Ile Leu Lys Gly
    130                 135                 140

Arg Arg Ser Ser Leu Leu Gln Thr Tyr His His Ala Gly Ala Met Ile
145                 150                 155                 160

Thr Met Trp Ser Gly Ile Asn Tyr Gln Ala Thr Pro Ile Trp Ile Phe
                165                 170                 175

Val Val Phe Asn Ser Phe Ile His Thr Ile Met Tyr Cys Tyr Tyr Ala
            180                 185                 190

Phe Thr Ser Ile Gly Phe His Pro Pro Gly Lys Lys Tyr Leu Thr Ser
        195                 200                 205

Met Gln Ile Thr Gln Phe Leu Val Gly Ile Thr Ile Ala Val Ser Tyr
    210                 215                 220

Leu Phe Val Pro Gly Cys Ile Arg Thr Pro Gly Ala Gln Met Ala Val
225                 230                 235                 240

Trp Ile Asn Val Gly Tyr Leu Phe Pro Leu Thr Tyr Leu Phe Val Asp
                245                 250                 255

Phe Ala Lys Arg Thr Tyr Ser Lys Arg Ser Ala Ile Ala Ala Gln Lys
            260                 265                 270

Lys Ala Gln
        275
```

<210> 97
<211> 828
<212> DNA
<213> Mortierella alpina

<220>
<221> CDS
<222> (1)..(828)
<223> synthetic C16/18 elongase (codon-optimized for Yarrowia lipolytica)

<300>
<302> A MORTIERELLA ALPINA C16/18 FATTY ACID ELONGASE

<310> U.S. 7,470,532

<311> 2005-10-19
<312> 2008-12-30
<313> (1)..(828)

<300>
<302> A MORTIERELLA ALPINA C16/18 FATTY ACID ELONGASE

<310> WO 2007/046817
<311> 2005-11-04
<312> 2007-04-26
<313> (1)..(828)

<400> 97

```
atg gag tct gga ccc atg cct gct ggc att ccc ttc cct gag tac tat      48
Met Glu Ser Gly Pro Met Pro Ala Gly Ile Pro Phe Pro Glu Tyr Tyr
1               5                   10                  15

gac ttc ttt atg gac tgg aag act ccc ctg gcc atc gct gcc acc tac      96
Asp Phe Phe Met Asp Trp Lys Thr Pro Leu Ala Ile Ala Ala Thr Tyr
            20                  25                  30

act gct gcc gtc ggt ctc ttc aac ccc aag gtt ggc aag gtc tcc cga     144
Thr Ala Ala Val Gly Leu Phe Asn Pro Lys Val Gly Lys Val Ser Arg
        35                  40                  45

gtg gtt gcc aag tcg gct aac gca aag cct gcc gag cga acc cag tcc     192
Val Val Ala Lys Ser Ala Asn Ala Lys Pro Ala Glu Arg Thr Gln Ser
    50                  55                  60

gga gct gcc atg act gcc ttc gtc ttt gtg cac aac ctc att ctg tgt     240
Gly Ala Ala Met Thr Ala Phe Val Phe Val His Asn Leu Ile Leu Cys
65                  70                  75                  80

gtc tac tct ggc atc acc ttc tac tac atg ttt cct gct atg gtc aag     288
Val Tyr Ser Gly Ile Thr Phe Tyr Tyr Met Phe Pro Ala Met Val Lys
                85                  90                  95

aac ttc cga acc cac aca ctg cac gaa gcc tac tgc gac acg gat cag     336
Asn Phe Arg Thr His Thr Leu His Glu Ala Tyr Cys Asp Thr Asp Gln
            100                 105                 110

tcc ctc tgg aac aac gca ctt ggc tac tgg ggt tac ctc ttc tac ctg     384
Ser Leu Trp Asn Asn Ala Leu Gly Tyr Trp Gly Tyr Leu Phe Tyr Leu
        115                 120                 125

tcc aag ttc tac gag gtc att gac acc atc atc atc atc ctg aag gga     432
Ser Lys Phe Tyr Glu Val Ile Asp Thr Ile Ile Ile Ile Leu Lys Gly
    130                 135                 140

cga cgg tcc tcg ctg ctt cag acc tac cac cat gct gga gcc atg att     480
Arg Arg Ser Ser Leu Leu Gln Thr Tyr His His Ala Gly Ala Met Ile
145                 150                 155                 160

acc atg tgg tct ggc atc aac tac caa gcc act ccc att tgg atc ttt     528
Thr Met Trp Ser Gly Ile Asn Tyr Gln Ala Thr Pro Ile Trp Ile Phe
                165                 170                 175

gtg gtc ttc aac tcc ttc att cac acc atc atg tac tgt tac tat gcc     576
Val Val Phe Asn Ser Phe Ile His Thr Ile Met Tyr Cys Tyr Tyr Ala
            180                 185                 190

ttc acc tct atc gga ttc cat cct cct ggc aaa aag tac ctg act tcg     624
Phe Thr Ser Ile Gly Phe His Pro Pro Gly Lys Lys Tyr Leu Thr Ser
        195                 200                 205

atg cag att act cag ttt ctg gtc ggt atc acc att gcc gtg tcc tac     672
Met Gln Ile Thr Gln Phe Leu Val Gly Ile Thr Ile Ala Val Ser Tyr
    210                 215                 220
```

```
ctc ttc gtt cct ggc tgc atc cga aca ccc ggt gct cag atg gct gtc        720
Leu Phe Val Pro Gly Cys Ile Arg Thr Pro Gly Ala Gln Met Ala Val
225                 230                 235                 240

tgg atc aac gtc ggc tac ctg ttt ccc ttg acc tat ctg ttc gtg gac        768
Trp Ile Asn Val Gly Tyr Leu Phe Pro Leu Thr Tyr Leu Phe Val Asp
                245                 250                 255

ttt gcc aag cga acc tac tcc aag cga tct gcc att gcc gct cag aaa        816
Phe Ala Lys Arg Thr Tyr Ser Lys Arg Ser Ala Ile Ala Ala Gln Lys
            260                 265                 270

aag gct cag taa                                                        828
Lys Ala Gln
        275
```

<210> 98
<211> 275
<212> PRT
<213> Mortierella alpina

<400> 98

```
Met Glu Ser Gly Pro Met Pro Ala Gly Ile Pro Phe Pro Glu Tyr Tyr
1               5                   10                  15

Asp Phe Phe Met Asp Trp Lys Thr Pro Leu Ala Ile Ala Ala Thr Tyr
            20                  25                  30

Thr Ala Ala Val Gly Leu Phe Asn Pro Lys Val Gly Lys Val Ser Arg
        35                  40                  45

Val Val Ala Lys Ser Ala Asn Ala Lys Pro Ala Glu Arg Thr Gln Ser
    50                  55                  60

Gly Ala Ala Met Thr Ala Phe Val Phe Val His Asn Leu Ile Leu Cys
65                  70                  75                  80

Val Tyr Ser Gly Ile Thr Phe Tyr Tyr Met Phe Pro Ala Met Val Lys
                85                  90                  95

Asn Phe Arg Thr His Thr Leu His Glu Ala Tyr Cys Asp Thr Asp Gln
            100                 105                 110

Ser Leu Trp Asn Asn Ala Leu Gly Tyr Trp Gly Tyr Leu Phe Tyr Leu
        115                 120                 125

Ser Lys Phe Tyr Glu Val Ile Asp Thr Ile Ile Ile Ile Leu Lys Gly
    130                 135                 140

Arg Arg Ser Ser Leu Leu Gln Thr Tyr His His Ala Gly Ala Met Ile
145                 150                 155                 160

Thr Met Trp Ser Gly Ile Asn Tyr Gln Ala Thr Pro Ile Trp Ile Phe
```

```
                165                 170                 175

Val Val Phe Asn Ser Phe Ile His Thr Ile Met Tyr Cys Tyr Tyr Ala
            180                 185                 190

Phe Thr Ser Ile Gly Phe His Pro Pro Gly Lys Lys Tyr Leu Thr Ser
        195                 200                 205

Met Gln Ile Thr Gln Phe Leu Val Gly Ile Thr Ile Ala Val Ser Tyr
    210                 215                 220

Leu Phe Val Pro Gly Cys Ile Arg Thr Pro Gly Ala Gln Met Ala Val
225                 230                 235                 240

Trp Ile Asn Val Gly Tyr Leu Phe Pro Leu Thr Tyr Leu Phe Val Asp
                245                 250                 255

Phe Ala Lys Arg Thr Tyr Ser Lys Arg Ser Ala Ile Ala Ala Gln Lys
            260                 265                 270

Lys Ala Gln
        275
```

<210> 99
<211> 12
<212> PRT
<213> Artificial Sequence

<220>
<223> membrane bound O-acyltransferase motif

<220>
<221> misc_feature
<222> (4)..(6)
<223> Xaa can be any naturally occurring amino acid

<220>
<221> misc_feature
<222> (9)..(11)
<223> Xaa can be any naturally occurring amino acid

<300>
<301> Hideo Shindou, Miki Etoa, Ryo Morimotoa and Takao Shimizua
<302> Identification of membrane O-acyltransferase family motifs
<303> Biochemical and Biophysical Research Communications
<304> 383
<305> 3
<306> 320-325
<307> 2009-04-08
<313> (1)..(12)

<400> 99

Trp His Gly Xaa Xaa Xaa Gly Tyr Xaa Xaa Xaa Phe
1 5 10

<210> 100
<211> 6
<212> PRT
<213> Artificial Sequence

<220>
<223> membrane bound O-acyltransferase motif

<220>
<221> misc_feature
<222> (2)..(5)
<223> Xaa can be any naturally occurring amino acid

<300>
<301> Hideo Shindou, Miki Etoa, Ryo Morimotoa and Takao Shimizua
<302> Identification of membrane O-acyltransferase family motifs
<303> Biochemical and Biophysical Research Communications
<304> 383
<305> 3
<306> 320-325
<307> 2009-04-08
<313> (1)..(6)

<400> 100

Tyr Xaa Xaa Xaa Xaa Phe
1 5

<210> 101
<211> 9
<212> PRT
<213> Artificial Sequence

<220>
<223> membrane bound O-acyltransferase motif

<220>
<221> misc_feature
<222> (2)..(4)
<223> Xaa can be any naturally occurring amino acid

<220>
<221> misc_feature
<222> (7)..(8)
<223> Xaa can be any naturally occurring amino acid

<300>
<301> Hideo Shindou, Miki Etoa, Ryo Morimotoa and Takao Shimizua
<302> Identification of membrane O-acyltransferase family motifs
<303> Biochemical and Biophysical Research Communications
<304> 383
<305> 3
<306> 320-325
<307> 2009-04-08
<313> (1)..(9)

<400> 101

Tyr Xaa Xaa Xaa Tyr Phe Xaa Xaa His
1               5

<210> 102
<211> 15
<212> PRT
<213> Artificial Sequence

<220>
<223> motif

<220>
<221> MISC_FEATURE
<222> (2)..(2)
<223> Xaa = Val [V] or Ile [I]

<220>
<221> MISC_FEATURE
<222> (3)..(3)
<223> Xaa = Leu [L] or Ile [I]

<220>
<221> misc_feature
<222> (4)..(5)
<223> Xaa can be any naturally occurring amino acid

<220>
<221> MISC_FEATURE
<222> (7)..(7)
<223> Xaa = Leu [L] or Ile [I] or Val [V]

<220>
<221> misc_feature
<222> (8)..(13)
<223> Xaa can be any naturally occurring amino acid

<300>
<302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION

<310> U.S. Patent Publication No. 2008-0145867-A1
<311> 2007-06-15
<312> 2008-06-19
<313> (1)..(15)

<400> 102

Met Xaa Xaa Xaa Xaa Lys Xaa Xaa Xaa Xaa Xaa Xaa Xaa Asp Gly
1               5                   10                  15

<210> 103
<211> 24
<212> PRT
<213> Artificial Sequence

<220>

<223> motif

<220>
<221> misc_feature
<222> (2)..(2)
<223> Xaa can be any naturally occurring amino acid

<220>
<221> misc_feature
<222> (6)..(7)
<223> Xaa can be any naturally occurring amino acid

<220>
<221> misc_feature
<222> (9)..(11)
<223> Xaa can be any naturally occurring amino acid

<220>
<221> MISC_FEATURE
<222> (12)..(12)
<223> Xaa = Glu [E] or Asp [D]

<220>
<221> MISC_FEATURE
<222> (13)..(13)
<223> Xaa = Ala [A] or Gly [G]

<220>
<221> misc_feature
<222> (14)..(18)
<223> Xaa can be any naturally occurring amino acid

<220>
<221> misc_feature
<222> (20)..(20)
<223> Xaa can be any naturally occurring amino acid

<220>
<221> MISC_FEATURE
<222> (22)..(22)
<223> Xaa = Phe [F] or Tyr [Y]

<220>
<221> misc_feature
<222> (23)..(23)
<223> Xaa can be any naturally occurring amino acid

<300>
<302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION

<310> U.S. Patent Publication No. 2008-0145867-A1
<311> 2007-06-15
<312> 2008-06-19
<313> (1)..(15)

<400> 103

Arg Xaa Lys Tyr Tyr Xaa Xaa Trp Xaa Xaa Xaa Xaa Xaa Xaa Xaa Xaa
1               5                   10                  15

Xaa Xaa Gly Xaa Gly Xaa Xaa Gly
            20

<210> 104
<211> 15
<212> PRT
<213> Artificial Sequence

<220>
<223> motif

<220>
<221> misc_feature
<222> (3)..(3)
<223> Xaa can be any naturally occurring amino acid

<220>
<221> misc_feature
<222> (7)..(8)
<223> Xaa can be any naturally occurring amino acid

<220>
<221> misc_feature
<222> (12)..(13)
<223> Xaa can be any naturally occurring amino acid

<220>
<221> MISC_FEATURE
<222> (14)..(14)
<223> Xaa = Thr [T] or Phe [F]

<300>
<302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION

<310> U.S. Patent Publication No. 2008-0145867-A1
<311> 2007-06-15
<312> 2008-06-19
<313> (1)..(15)

<400> 104

Ser Ala Xaa Trp His Gly Xaa Xaa Pro Gly Tyr Xaa Xaa Xaa Phe
1               5                   10                  15

<210> 105
<211> 7
<212> PRT
<213> Artificial Sequence

<220>
<223> 1-acyl-sn-glycerol-3-phosphate acyltransferase motif

<220>

<221> misc_feature
<222> (2)..(3)
<223> Xaa can be any naturally occurring amino acid

<220>
<221> MISC_FEATURE
<222> (6)..(6)
<223> Xaa = Asp [D] or Arg [R]

<300>
<301> Tal M. Lewin, Ping Wang, and Rosalind A. Coleman
<302> Analysis of Amino Acid Motifs Diagnostic for the sn-Glycerol-3-phosphate Acyltransferase Reaction
<303> Biochemistry
<304> 38
<305> 18
<306> 57645771
<307> 1999-04-15
<313> (1)..(7)

<300>
<301> Atsushi Yamashita, Hiroki Nakanishia, Hiroshi Suzukia, Ryo Kamataa, Ken Tanakaa, Keizo Wakua and Takayuki Sugiura<302> Topology of acyltransferase motifs and substrate specificity and accessibility in 1-acyl-sn-glycero-3-phosphate acyltransferase 1<303> Biochimica et Biophysica Acta<304> 1771<305> 9<306> 1202-1215<307> 2007-07-17
<313> (1)..(7)

<400> 105

```
Gly Xaa Xaa Phe Ile Xaa Arg
1               5
```

<210> 106
<211> 6
<212> PRT
<213> Artificial Sequence

<220>
<223> 1-acyl-sn-glycerol-3-phosphate acyltransferase motif

<220>
<221> MISC_FEATURE
<222> (1)..(1)
<223> Xaa = Val [V] or Ile [I]

<220>
<221> MISC_FEATURE
<222> (2)..(2)
<223> Xaa = Pro [P] or Xaa

<220>
<221> MISC_FEATURE
<222> (3)..(3)
<223> Xaa = Ile [I] or Val [V] or Leu [L]

<220>
<221> MISC_FEATURE
<222> (4)..(4)
<223> Xaa = Ile [I] or Val [V]

<220>
<221> MISC_FEATURE
<222> (6)..(6)
<223> Xaa = Val [V] or Ile [I]

<300>
<301> Atsushi Yamashita, Hiroki Nakanishia, Hiroshi Suzukia, Ryo Kamataa, Ken Tanakaa, Keizo Wakua and Takayuki Sugiura
<302> Topology of acyltransferase motifs and substrate specificity and accessibility in 1-acyl-sn-glycero-3-phosphate acyltransferase 1
<303> Biochimica et Biophysica Acta
<304> 1771
<305> 9
<306> 1202-1215
<307> 2007-07-17
<313> (1)..(6)

<400> 106

```
Xaa Xaa Xaa Xaa Pro Xaa
1               5
```

<210> 107
<211> 6
<212> PRT
<213> Artificial Sequence

<220>
<223> 1-acyl-sn-glycerol-3-phosphate acyltransferase motif

<300>
<301> Atsushi Yamashita, Hiroki Nakanishia, Hiroshi Suzukia, Ryo Kamataa, Ken Tanakaa, Keizo Wakua and Takayuki Sugiura<302> Topology of acyltransferase motifs and substrate specificity and accessibility in 1-acyl-sn-glycero-3-phosphate acyltransferase 1<303> Biochimica et Biophysica Acta<304> 1771<305> 9<306> 1202-1215<307> 2007-07-17
<313> (1)..(6)

<400> 107

```
Ile Val Pro Ile Val Met
1               5
```

<210> 108
<211> 1024
<212> PRT
<213> Yarrowia lipolytica

<220>
<221> MISC_FEATURE
<222> (1)..(1024)
<223> YlPex1p; GenBank Accession No. CAG82178

<400> 108

Met Thr Ser Lys Ser Asp Tyr Ser Gly Lys Asp Lys Ile Glu Leu Asp
1               5                   10                  15

Pro Val Phe Ala Lys Ser Ile Asp Leu Leu Pro Asn Thr Gln Val Val
            20                  25                  30

Ile Asp Ile Gln Leu Asn Pro Lys Ile Ala His Thr Ile His Leu Glu
        35                  40                  45

Pro Val Thr Val Ala Asp Trp Glu Ile Val Glu Leu His Ala Ala Tyr
    50                  55                  60

Leu Glu Ser Arg Met Ile Asn Gln Val Arg Ala Val Ser Pro Asn Gln
65                  70                  75                  80

Pro Val Thr Val Tyr Pro Ser Ser Thr Thr Ser Ala Thr Leu Lys Val
85 90 95

Ile Arg Ile Glu Pro Asp Leu Gly Ala Ala Gly Phe Ala Lys Leu Ser
100 105 110

Pro Asp Ser Glu Val Val Val Ala Pro Lys Gln Arg Lys Lys Glu Glu
115 120 125

Lys Gln Val Lys Lys Arg Ser Gly Ser Ala Arg Ser Thr Gly Ser Gln
130 135 140

Lys Arg Lys Gly Gly Arg Gly Pro His Ala Leu Arg Arg Ala Ile Ser
145 150 155 160

Glu Asp Phe Asp Gly His Leu Arg Leu Glu Val Ser Leu Asp Val Ser
165 170 175

Gln Leu Pro Pro Glu Phe His Gln Leu Lys Asn Val Ser Ile Lys Val
180 185 190

Ile Thr Pro Pro Asn Leu Ala Ser Pro Gln Gln Ala Ala Ser Ile Ala
195 200 205

Val Glu Glu Lys Ser Glu Glu Ser Leu Ser Gln Asn Lys Pro Pro Ser
210 215 220

Ser Glu Pro Lys Val Glu Val Pro Pro Asp Ile Ile Asn Pro Ala Ser
225 230 235 240

Glu Ile Val Ala Thr Leu Val Asn Asp Thr Thr Ser Pro Thr Gly His
245 250 255

Ala Lys Leu Ser Tyr Ala Leu Ala Asp Ala Leu Gly Ile Pro Ser Ser
260 265 270

Val Gly His Val Ile Arg Phe Glu Ser Ala Ser Lys Pro Leu Ser Gln
275 280 285

Lys Pro Gly Ala Leu Val Ile His Arg Phe Ile Thr Lys Thr Val Gly
290 295 300

Ala Ala Glu Gln Lys Ser Leu Arg Leu Lys Gly Glu Lys Asn Ala Asp
305 310 315 320

Asp Gly Val Ser Ala Asp Asp Gln Phe Ser Leu Leu Glu Glu Leu Lys
325 330 335

Lys Leu Gln Met Leu Glu Gly Pro Ile Thr Asn Phe Gln Arg Leu Pro
340 345 350

Pro Ile Pro Glu Leu Leu Pro Leu Gly Gly Val Ile Gly Leu Gln Asn
355 360 365

Ser Glu Gly Trp Ile Gln Gly Gly Tyr Leu Gly Glu Glu Pro Ile Pro
370 375 380

Phe Val Ser Gly Ser Glu Ile Leu Arg Ser Glu Ser Ser Leu Ser Pro
385 390 395 400

Ser Asn Ile Glu Ser Glu Asp Lys Arg Val Val Gly Leu Asp Asn Met
405 410 415

Leu Asn Lys Ile Asn Glu Val Leu Ser Arg Asp Ser Ile Gly Cys Leu
420 425 430

Val Tyr Gly Ser Arg Gly Ser Gly Lys Ser Ala Val Leu Asn His Ile
435 440 445

Lys Lys Glu Cys Lys Val Ser His Thr His Thr Val Ser Ile Ala Cys
450 455 460

Gly Leu Ile Ala Gln Asp Arg Val Gln Ala Val Arg Glu Ile Leu Thr
465 470 475 480

Lys Ala Phe Leu Glu Ala Ser Trp Phe Ser Pro Ser Val Leu Phe Leu
485 490 495

Asp Asp Ile Asp Ala Leu Met Pro Ala Glu Val Glu His Ala Asp Ser
500 505 510

Ser Arg Thr Arg Gln Leu Thr Gln Leu Phe Leu Glu Leu Ala Leu Pro
515 520 525

Ile Met Lys Ser Arg His Val Ser Val Val Ala Ser Ala Gln Ala Lys
530 535 540

Glu Ser Leu His Met Asn Leu Val Thr Gly His Val Phe Glu Glu Leu
545 550 555 560

Phe His Leu Lys Ser Pro Asp Lys Glu Ala Arg Leu Ala Ile Leu Ser
565 570 575

Glu Ala Val Lys Leu Met Asp Gln Asn Val Ser Phe Ser Gln Asn Asp
580 585 590

Val Leu Glu Ile Ala Ser Gln Val Asp Gly Tyr Leu Pro Gly Asp Leu

595 600 605

Trp Thr Leu Ser Glu Arg Ala Gln His Glu Met Ala Leu Arg Gln Ile
610 615 620

Glu Ile Gly Leu Glu Asn Pro Ser Ile Gln Leu Ala Asp Phe Met Lys
625 630 635 640

Ala Leu Glu Asp Phe Val Pro Ser Ser Leu Arg Gly Val Lys Leu Gln
645 650 655

Lys Ser Asn Val Lys Trp Asn Asp Ile Gly Gly Leu Lys Glu Thr Lys
660 665 670

Ala Val Leu Leu Glu Thr Leu Glu Trp Pro Thr Lys Tyr Ala Pro Ile
675 680 685

Phe Ala Ser Cys Pro Leu Arg Leu Arg Ser Gly Leu Leu Leu Tyr Gly
690 695 700

Tyr Pro Gly Cys Gly Lys Thr Tyr Leu Ala Ser Ala Val Ala Ala Gln
705 710 715 720

Cys Gly Leu Asn Phe Ile Ser Ile Lys Gly Pro Glu Ile Leu Asn Lys
725 730 735

Tyr Ile Gly Ala Ser Glu Gln Ser Val Arg Glu Leu Phe Glu Arg Ala
740 745 750

Gln Ala Ala Lys Pro Cys Ile Leu Phe Phe Asp Glu Phe Asp Ser Ile
755 760 765

Ala Pro Lys Arg Gly His Asp Ser Thr Gly Val Thr Asp Arg Val Val
770 775 780

Asn Gln Met Leu Thr Gln Met Asp Gly Ala Glu Gly Leu Asp Gly Val
785 790 795 800

Tyr Val Leu Ala Ala Thr Ser Arg Pro Asp Leu Ile Asp Pro Ala Leu
805 810 815

Leu Arg Pro Gly Arg Leu Asp Lys Met Leu Ile Cys Asp Leu Pro Ser
820 825 830

Tyr Glu Asp Arg Leu Asp Ile Leu Arg Ala Ile Val Asp Gly Lys Met
835 840 845

His Leu Asp Gly Glu Val Glu Leu Glu Tyr Val Ala Ser Arg Thr Asp
850 855 860

Gly Phe Ser Gly Ala Asp Leu Gln Ala Val Met Phe Asn Ala Tyr Leu
865 870 875 880

Glu Ala Ile His Glu Val Val Asp Val Ala Asp Asp Thr Ala Ala Asp
885 890 895

Thr Pro Ala Leu Glu Asp Lys Arg Leu Glu Phe Phe Gln Thr Thr Leu
900 905 910

Gly Asp Ala Lys Lys Asp Pro Ala Ala Val Gln Asn Glu Val Met Asn
915 920 925

Ala Arg Ala Ala Val Ala Glu Lys Ala Arg Val Thr Ala Lys Leu Glu
930 935 940

Ala Leu Phe Lys Gly Met Ser Val Gly Val Asp Asn Asp Asp Asp Lys
945 950 955 960

Pro Arg Lys Lys Ala Val Val Val Ile Lys Pro Gln His Met Asn Lys
965 970 975

Ser Leu Asp Glu Thr Ser Pro Ser Ile Ser Lys Lys Glu Leu Leu Lys
980 985 990

Leu Lys Gly Ile Tyr Ser Gln Phe Val Ser Gly Arg Ser Gly Asp Met
995 1000 1005

Pro Pro Gly Thr Ala Ser Thr Asp Val Gly Gly Arg Ala Thr Leu
1010 1015 1020

Ala

<210> 109
<211> 381
<212> PRT
<213> Yarrowia lipolytica

<220>
<221> MISC_FEATURE
<222> (1)..(381)
<223> YlPex2p; GenBank Accession No. CAG77647

<400> 109

Met Ser Ser Val Leu Arg Leu Phe Lys Ile Gly Ala Pro Val Pro Asn
1 5 10 15

Val Arg Val His Gln Leu Asp Ala Ser Leu Leu Asp Ala Glu Leu Val
20 25 30

```
Asp Leu Leu Lys Asn Gln Leu Phe Lys Gly Phe Thr Asn Phe His Pro
        35                  40                  45

Glu Phe Arg Asp Lys Tyr Glu Ser Glu Leu Val Leu Ala Leu Lys Leu
    50                  55                  60

Ile Leu Phe Lys Leu Thr Val Trp Asp His Ala Ile Thr Tyr Gly Gly
65                  70                  75                  80

Lys Leu Gln Asn Leu Lys Phe Ile Asp Ser Arg His Ser Ser Lys Leu
                85                  90                  95

Gln Ile Gln Pro Ser Val Ile Gln Lys Leu Gly Tyr Gly Ile Leu Val
            100                 105                 110

Val Gly Gly Gly Tyr Leu Trp Ser Lys Ile Glu Gly Tyr Leu Leu Ala
        115                 120                 125

Arg Ser Glu Asp Asp Val Ala Thr Asp Gly Thr Ser Val Arg Gly Ala
    130                 135                 140

Ser Ala Ala Arg Gly Ala Leu Lys Val Ala Asn Phe Ala Ser Leu Leu
145                 150                 155                 160

Tyr Ser Ala Ala Thr Leu Gly Asn Phe Val Ala Phe Leu Tyr Thr Gly
                165                 170                 175

Arg Tyr Ala Thr Val Ile Met Arg Leu Leu Arg Ile Arg Leu Val Pro
            180                 185                 190

Ser Gln Arg Thr Ser Ser Arg Gln Val Ser Tyr Glu Phe Gln Asn Arg
        195                 200                 205

Gln Leu Val Trp Asn Ala Phe Thr Glu Phe Leu Ile Phe Ile Leu Pro
    210                 215                 220

Leu Leu Gln Leu Pro Lys Leu Lys Arg Arg Ile Glu Arg Lys Leu Gln
225                 230                 235                 240

Ser Leu Asn Val Thr Arg Val Gly Asn Val Glu Glu Ala Ser Glu Gly
                245                 250                 255

Glu Leu Ala His Leu Pro Gln Lys Thr Cys Ala Ile Cys Phe Arg Asp
            260                 265                 270

Glu Glu Glu Gln Glu Gly Gly Gly Gly Ala Ser His Tyr Ser Thr Asp
        275                 280                 285
```

```
Val Thr Asn Pro Tyr Gln Ala Asp Cys Gly His Val Tyr Cys Tyr Val
    290                 295                 300

Cys Leu Val Thr Lys Leu Ala Gln Gly Asp Gly Asp Gly Trp Asn Cys
305                 310                 315                 320

Tyr Arg Cys Ala Lys Gln Val Gln Lys Met Lys Pro Trp Val Asp Val
                325                 330                 335

Asp Glu Ala Ala Val Val Gly Ala Ala Glu Met His Glu Lys Val Asp
            340                 345                 350

Val Ile Glu His Ala Glu Asp Asn Glu Gln Glu Glu Glu Glu Phe Asp
        355                 360                 365

Asp Asp Asp Glu Asp Ser Asn Phe Gln Leu Met Lys Asp
    370                 375                 380
```

<210> 110
<211> 431
<212> PRT
<213> Yarrowia lipolytica

<220>
<221> MISC_FEATURE
<222> (1)..(431)
<223> YlPex3p; GenBank Accession No. CAG78565

<400> 110

```
Met Asp Phe Phe Arg Arg His Gln Lys Lys Val Leu Ala Leu Val Gly
1               5                   10                  15

Val Ala Leu Ser Ser Tyr Leu Phe Ile Asp Tyr Val Lys Lys Lys Phe
            20                  25                  30

Phe Glu Ile Gln Gly Arg Leu Ser Ser Glu Arg Thr Ala Lys Gln Asn
        35                  40                  45

Leu Arg Arg Arg Phe Glu Gln Asn Gln Gln Asp Ala Asp Phe Thr Ile
    50                  55                  60

Met Ala Leu Leu Ser Ser Leu Thr Thr Pro Val Met Glu Arg Tyr Pro
65                  70                  75                  80

Val Asp Gln Ile Lys Ala Glu Leu Gln Ser Lys Arg Arg Pro Thr Asp
                85                  90                  95

Arg Val Leu Ala Leu Glu Ser Ser Thr Ser Ser Ser Ala Thr Ala Gln
            100                 105                 110
```

```
Thr Val Pro Thr Met Thr Ser Gly Ala Thr Glu Glu Gly Glu Lys Ser
        115                 120                 125

Lys Thr Gln Leu Trp Gln Asp Leu Lys Arg Thr Thr Ile Ser Arg Ala
    130                 135                 140

Phe Ser Leu Val Tyr Ala Asp Ala Leu Leu Ile Phe Phe Thr Arg Leu
145                 150                 155                 160

Gln Leu Asn Ile Leu Gly Arg Arg Asn Tyr Val Asn Ser Val Val Ala
                165                 170                 175

Leu Ala Gln Gln Gly Arg Glu Gly Asn Ala Glu Gly Arg Val Ala Pro
            180                 185                 190

Ser Phe Gly Asp Leu Ala Asp Met Gly Tyr Phe Gly Asp Leu Ser Gly
        195                 200                 205

Ser Ser Ser Phe Gly Glu Thr Ile Val Asp Pro Asp Leu Asp Glu Gln
    210                 215                 220

Tyr Leu Thr Phe Ser Trp Trp Leu Leu Asn Glu Gly Trp Val Ser Leu
225                 230                 235                 240

Ser Glu Arg Val Glu Glu Ala Val Arg Arg Val Trp Asp Pro Val Ser
                245                 250                 255

Pro Lys Ala Glu Leu Gly Phe Asp Glu Leu Ser Glu Leu Ile Gly Arg
            260                 265                 270

Thr Gln Met Leu Ile Asp Arg Pro Leu Asn Pro Ser Ser Pro Leu Asn
        275                 280                 285

Phe Leu Ser Gln Leu Leu Pro Pro Arg Glu Gln Glu Glu Tyr Val Leu
    290                 295                 300

Ala Gln Asn Pro Ser Asp Thr Ala Ala Pro Ile Val Gly Pro Thr Leu
305                 310                 315                 320

Arg Arg Leu Leu Asp Glu Thr Ala Asp Phe Ile Glu Ser Pro Asn Ala
                325                 330                 335

Ala Glu Val Ile Glu Arg Leu Val His Ser Gly Leu Ser Val Phe Met
            340                 345                 350

Asp Lys Leu Ala Val Thr Phe Gly Ala Thr Pro Ala Asp Ser Gly Ser
        355                 360                 365
```

Pro Tyr Pro Val Val Leu Pro Thr Ala Lys Val Lys Leu Pro Ser Ile
    370                 375                 380

Leu Ala Asn Met Ala Arg Gln Ala Gly Gly Met Ala Gln Gly Ser Pro
385                 390                 395                 400

Gly Val Glu Asn Glu Tyr Ile Asp Val Met Asn Gln Val Gln Glu Leu
                405                 410                 415

Thr Ser Phe Ser Ala Val Val Tyr Ser Ser Phe Asp Trp Ala Leu
            420                 425                 430

<210> 111
<211> 395
<212> PRT
<213> Yarrowia lipolytica

<220>
<221> MISC_FEATURE
<222> (1)..(395)
<223> YlPex3Bp; GenBank Accession No. CAG83356

<400> 111

```
Met Leu Gln Ser Leu Asn Arg Asn Lys Lys Arg Leu Ala Val Ser Thr
1               5                   10                  15

Gly Leu Ile Ala Val Ala Tyr Val Val Ile Ser Tyr Thr Thr Lys Arg
            20                  25                  30

Leu Ile Glu Lys Gln Glu Gln Lys Leu Glu Glu Glu Arg Ala Lys Glu
        35                  40                  45

Arg Leu Lys Gln Leu Phe Ala Gln Thr Gln Asn Glu Ala Ala Phe His
    50                  55                  60

Thr Ala Ser Val Leu Pro Gln Leu Cys Glu Gln Ile Met Glu Phe Val
65                  70                  75                  80

Ala Val Glu Lys Ile Ala Glu Gln Leu Gln Asn Met Arg Ala Glu Lys
                85                  90                  95

Arg Lys Lys Gln Asn Met Asp Asp Asp Lys His Ser Val Leu Ser Leu
            100                 105                 110

Gly Thr Glu Thr Thr Ala Ser Met Ala Asp Gly Gln Lys Met Ser Lys
        115                 120                 125

Ile Gln Leu Trp Asp Glu Leu Lys Ile Glu Ser Leu Thr Arg Ile Val
    130                 135                 140
```

```
Thr Leu Ile Tyr Cys Val Ser Leu Leu Asn Tyr Leu Ile Arg Leu Gln
145                 150                 155                 160

Thr Asn Ile Val Gly Arg Lys Arg Tyr Gln Asn Glu Ala Gly Pro Ala
                165                 170                 175

Gly Ala Thr Tyr Asp Met Ser Leu Glu Gln Cys Tyr Thr Trp Leu Leu
            180                 185                 190

Thr Arg Gly Trp Lys Ser Val Val Asp Asn Val Arg Arg Ser Val Gln
        195                 200                 205

Gln Val Phe Thr Gly Val Asn Pro Arg Gln Asn Leu Ser Leu Asp Glu
    210                 215                 220

Phe Ala Thr Leu Leu Lys Arg Val Gln Thr Leu Val Asn Ser Pro Pro
225                 230                 235                 240

Tyr Ser Thr Thr Pro Asn Thr Phe Leu Thr Ser Leu Leu Pro Pro Arg
                245                 250                 255

Glu Leu Glu Gln Leu Arg Leu Glu Lys Glu Lys Gln Ser Leu Ser Pro
            260                 265                 270

Asn Tyr Thr Tyr Gly Ser Pro Leu Lys Asp Leu Val Phe Glu Ser Ala
        275                 280                 285

Gln His Ile Gln Ser Pro Gln Gly Met Ser Ser Phe Arg Ala Ile Ile
    290                 295                 300

Asp Gln Ser Phe Lys Val Phe Leu Glu Lys Val Asn Glu Ser Gln Tyr
305                 310                 315                 320

Val Asn Pro Pro Ser Thr Gly Gly Lys Arg Ile Ala Val Gly Ala Leu
                325                 330                 335

Gln Pro Pro Ile Ile Ser Gly Gly Pro Lys Lys Val Lys Leu Ala Ser
            340                 345                 350

Leu Leu Ser Val Ala Thr Arg Gln Ser Ser Val Ile Ser His Ala Gln
        355                 360                 365

Pro Asn Pro Tyr Val Asp Ala Ile Asn Ser Val Ala Glu Tyr Asn Gly
    370                 375                 380

Leu Cys Ala Val Ile Tyr Ser Ser Phe Glu Gln
385                 390                 395
```

<210> 112
<211> 153
<212> PRT
<213> Yarrowia lipolytica

<220>
<221> MISC_FEATURE
<222> (1)..(153)
<223> YlPex4p; GenBank Accession No. CAG79130

<400> 112

Met Ala Ser Gln Lys Arg Leu Ile Lys Glu Leu Ala Ala Tyr Lys Lys
1               5                   10                  15

Asp Pro Asn Pro Cys Leu Ala Ser Leu Thr Ala Asp Gly Asp Ser Leu
            20                  25                  30

Tyr Lys Trp Thr Ala Val Met Arg Gly Thr Glu Gly Thr Ala Tyr Glu
        35                  40                  45

Asn Gly Leu Trp Gln Val Glu Ile Asn Ile Pro Glu Asn Tyr Pro Leu
    50                  55                  60

Gln Pro Pro Thr Met Phe Phe Arg Thr Lys Ile Cys His Pro Asn Ile
65                  70                  75                  80

His Phe Glu Thr Gly Glu Val Cys Ile Asp Val Leu Lys Thr Gln Trp
                85                  90                  95

Ser Pro Ala Trp Thr Ile Ser Ser Ala Cys Thr Ala Val Ser Ala Met
            100                 105                 110

Leu Ser Leu Pro Glu Pro Asp Ser Pro Leu Asn Ile Asp Ala Ala Asn
        115                 120                 125

Leu Val Arg Cys Gly Asp Glu Ser Ala Met Glu Gly Leu Val Arg Tyr
    130                 135                 140

Tyr Val Asn Lys Tyr Ala Ser Gly Asn
145                 150

<210> 113
<211> 598
<212> PRT
<213> Yarrowia lipolytica

<220>
<221> MISC_FEATURE
<222> (1)..(598)
<223> YlPex5p; GenBank Accession No. CAG78803

<400> 113

```
Met Ser Phe Met Arg Gly Gly Ser Glu Cys Ser Thr Gly Arg Asn Pro
1               5                   10                  15

Leu Ser Gln Phe Thr Lys His Thr Ala Glu Asp Arg Ser Leu Gln His
            20                  25                  30

Asp Arg Val Ala Gly Pro Ser Gly Gly Arg Val Gly Gly Met Arg Ser
        35                  40                  45

Asn Thr Gly Glu Met Ser Gln Gln Asp Arg Glu Met Met Ala Arg Phe
    50                  55                  60

Gly Ala Ala Gly Pro Glu Gln Ser Ser Phe Asn Tyr Glu Gln Met Arg
65                  70                  75                  80

His Glu Leu His Asn Met Gly Ala Gln Gly Gly Gln Ile Pro Gln Val
                85                  90                  95

Pro Ser Gln Gln Gly Ala Ala Asn Gly Gly Gln Trp Ala Arg Asp Phe
            100                 105                 110

Gly Gly Gln Gln Thr Ala Pro Gly Ala Ala Pro Gln Asp Ala Lys Asn
        115                 120                 125

Trp Asn Ala Glu Phe Gln Arg Gly Gly Ser Pro Ala Glu Ala Met Gln
    130                 135                 140

Gln Gln Gly Pro Gly Pro Met Gln Gly Gly Met Gly Met Gly Gly Met
145                 150                 155                 160

Pro Met Tyr Gly Met Ala Arg Pro Met Tyr Ser Gly Met Ser Ala Asn
                165                 170                 175

Met Ala Pro Gln Phe Gln Pro Gln Gln Ala Asn Ala Arg Val Val Glu
            180                 185                 190

Leu Asp Glu Gln Asn Trp Glu Glu Gln Phe Lys Gln Met Asp Ser Ala
        195                 200                 205

Val Gly Lys Gly Lys Glu Val Glu Glu Gln Thr Ala Glu Thr Ala Thr
    210                 215                 220

Ala Thr Glu Thr Val Thr Glu Thr Glu Thr Thr Thr Glu Asp Lys Pro
225                 230                 235                 240

Met Asp Ile Lys Asn Met Asp Phe Glu Asn Ile Trp Lys Asn Leu Gln
                245                 250                 255

Val Asn Val Leu Asp Asn Met Asp Glu Trp Leu Glu Glu Thr Asn Ser
```

260 265 270

Pro Ala Trp Glu Arg Asp Phe His Glu Tyr Thr His Asn Arg Pro Glu
275 280 285

Phe Ala Asp Tyr Gln Phe Glu Glu Asn Asn Gln Phe Met Glu His Pro
290 295 300

Asp Pro Phe Lys Ile Gly Val Glu Leu Met Glu Thr Gly Gly Arg Leu
305 310 315 320

Ser Glu Ala Ala Leu Ala Phe Glu Ala Ala Val Gln Lys Asn Thr Glu
325 330 335

His Ala Glu Ala Trp Gly Arg Leu Gly Ala Cys Gln Ala Gln Asn Glu
340 345 350

Lys Glu Asp Pro Ala Ile Arg Ala Leu Glu Arg Cys Ile Lys Leu Glu
355 360 365

Pro Gly Asn Leu Ser Ala Leu Met Asn Leu Ser Val Ser Tyr Thr Asn
370 375 380

Glu Gly Tyr Glu Asn Ala Ala Tyr Ala Thr Leu Glu Arg Trp Leu Ala
385 390 395 400

Thr Lys Tyr Pro Glu Val Val Asp Gln Ala Arg Asn Gln Glu Pro Arg
405 410 415

Leu Gly Asn Glu Asp Lys Phe Gln Leu His Ser Arg Val Thr Glu Leu
420 425 430

Phe Ile Arg Ala Ala Gln Leu Ser Pro Asp Gly Ala Asn Ile Asp Ala
435 440 445

Asp Val Gln Val Gly Leu Gly Val Leu Phe Tyr Gly Asn Glu Glu Tyr
450 455 460

Asp Lys Ala Ile Asp Cys Phe Asn Ala Ala Ile Ala Val Arg Pro Asp
465 470 475 480

Asp Ala Leu Leu Trp Asn Arg Leu Gly Ala Thr Leu Ala Asn Ser His
485 490 495

Arg Ser Glu Glu Ala Ile Asp Ala Tyr Tyr Lys Ala Leu Glu Leu Arg
500 505 510

Pro Ser Phe Val Arg Ala Arg Tyr Asn Leu Gly Val Ser Cys Ile Asn
515 520 525

Ile Gly Cys Tyr Lys Glu Ala Ala Gln Tyr Leu Leu Gly Ala Leu Ser
530 535 540

Met His Lys Val Glu Gly Val Gln Asp Asp Val Leu Ala Asn Gln Ser
545 550 555 560

Thr Asn Leu Tyr Asp Thr Leu Lys Arg Val Phe Leu Gly Met Asp Arg
565 570 575

Arg Asp Leu Val Ala Lys Val Gly Asn Gly Met Asp Val Asn Gln Phe
580 585 590

Arg Asn Glu Phe Glu Phe
595

<210> 114
<211> 1024
<212> PRT
<213> Yarrowia lipolytica

<220>
<221> MISC_FEATURE
<222> (1)..(1024)
<223> YlPex6p; GenBank Accession No. CAG82306

<400> 114

```
Met Pro Ser Ile Ser His Lys Pro Ile Thr Ala Lys Leu Val Ala Ala
1               5                   10                  15

Pro Asp Ala Thr Lys Leu Glu Leu Ser Ser Tyr Leu Tyr Gln Gln Leu
            20                  25                  30

Phe Ser Asp Lys Pro Ala Glu Pro Tyr Val Ala Phe Glu Ala Pro Gly
        35                  40                  45

Ile Lys Trp Ala Leu Tyr Pro Ala Ser Glu Asp Arg Ser Leu Pro Gln
    50                  55                  60

Tyr Thr Cys Lys Ala Asp Ile Arg His Val Ala Gly Ser Leu Lys Lys
65                  70                  75                  80

Phe Met Pro Val Val Leu Lys Arg Val Asn Pro Val Thr Ile Glu His
                85                  90                  95

Ala Ile Val Thr Val Pro Ala Ser Gln Tyr Glu Thr Leu Asn Thr Pro
            100                 105                 110

Glu Gln Val Leu Lys Ala Leu Glu Pro Gln Leu Asp Lys Asp Arg Pro
        115                 120                 125
```

```
Val Ile Arg Gln Gly Asp Val Leu Leu Asn Gly Cys Arg Val Arg Leu
    130                 135                 140

Cys Glu Pro Val Asn Gln Gly Lys Val Val Lys Gly Thr Thr Lys Leu
145                 150                 155                 160

Thr Val Ala Lys Glu Gln Glu Thr Ile Gln Pro Ala Asp Glu Ala Ala
                165                 170                 175

Asp Val Ala Phe Asp Ile Ala Glu Phe Leu Asp Phe Asp Thr Ser Val
            180                 185                 190

Ala Lys Thr Arg Glu Ser Thr Asn Leu Gln Val Ala Pro Leu Glu Gly
        195                 200                 205

Ala Ile Pro Thr Pro Leu Ser Asp Arg Phe Asp Asp Cys Glu Ser Arg
    210                 215                 220

Gly Phe Val Lys Ser Glu Thr Met Ser Lys Leu Gly Val Phe Ser Gly
225                 230                 235                 240

Asp Ile Val Ser Ile Lys Thr Lys Asn Gly Ala Glu Arg Val Leu Arg
                245                 250                 255

Leu Phe Ala Tyr Pro Glu Pro Asn Thr Val Lys Tyr Asp Val Val Tyr
            260                 265                 270

Val Ser Pro Ile Leu Tyr His Asn Ile Gly Asp Lys Glu Ile Glu Val
        275                 280                 285

Thr Pro Asn Gly Glu Thr His Lys Ser Val Gly Glu Ala Leu Asp Ser
    290                 295                 300

Val Leu Glu Ala Ala Glu Glu Val Lys Leu Ala Arg Val Leu Gly Pro
305                 310                 315                 320

Thr Thr Thr Asp Arg Thr Phe Gln Thr Ala Tyr His Ala Gly Leu Gln
                325                 330                 335

Ala Tyr Phe Lys Pro Val Lys Arg Ala Val Arg Val Gly Asp Leu Ile
            340                 345                 350

Pro Ile Pro Phe Asp Ser Ile Leu Ala Arg Thr Ile Gly Glu Asp Pro
        355                 360                 365

Glu Met Ser His Ile Pro Leu Glu Ala Leu Ala Val Lys Pro Asp Ser
    370                 375                 380
```

```
Val Ala Trp Phe Gln Val Thr Ser Leu Asn Gly Ser Glu Asp Pro Ala
385                 390                 395                 400

Ser Lys Gln Tyr Leu Val Asp Ser Ser Gln Thr Lys Leu Ile Glu Gly
                405                 410                 415

Gly Thr Thr Ser Ser Ala Val Ile Pro Thr Ser Val Pro Trp Arg Glu
            420                 425                 430

Tyr Leu Gly Leu Asp Thr Leu Pro Lys Phe Gly Ser Glu Phe Ala Tyr
        435                 440                 445

Ala Asp Lys Ile Arg Asn Leu Val Gln Ile Ser Thr Ser Ala Leu Ser
    450                 455                 460

His Ala Lys Leu Asn Thr Ser Val Leu Leu His Ser Ala Lys Arg Gly
465                 470                 475                 480

Val Gly Lys Ser Thr Val Leu Arg Ser Val Ala Ala Gln Cys Gly Ile
                485                 490                 495

Ser Val Phe Glu Ile Ser Cys Phe Gly Leu Ile Gly Asp Asn Glu Ala
            500                 505                 510

Gln Thr Leu Gly Thr Leu Arg Ala Lys Leu Asp Arg Ala Tyr Gly Cys
        515                 520                 525

Ser Pro Cys Val Val Val Leu Gln His Leu Glu Ser Ile Ala Lys Lys
    530                 535                 540

Ser Asp Gln Asp Gly Lys Asp Glu Gly Ile Val Ser Lys Leu Val Asp
545                 550                 555                 560

Val Leu Ala Asp Tyr Ser Gly His Gly Val Leu Leu Ala Ala Thr Ser
                565                 570                 575

Asn Asp Pro Asp Lys Ile Ser Glu Ala Ile Arg Ser Arg Phe Gln Phe
            580                 585                 590

Glu Ile Glu Ile Gly Val Pro Ser Glu Pro Gln Arg Arg Gln Ile Phe
        595                 600                 605

Ser His Leu Thr Lys Ser Gly Pro Gly Gly Asp Ser Ile Arg Asn Ala
    610                 615                 620

Pro Ile Ser Leu Arg Ser Asp Val Ser Val Glu Asn Leu Ala Leu Gln
625                 630                 635                 640
```

```
Ser Ala Gly Leu Thr Pro Pro Asp Leu Thr Ala Ile Val Gln Thr Thr
                645                 650                 655

Arg Leu Arg Ala Ile Asp Arg Leu Asn Lys Leu Thr Lys Asp Ser Asp
            660                 665                 670

Thr Thr Leu Asp Asp Leu Leu Thr Leu Ser His Gly Thr Leu Gln Leu
        675                 680                 685

Thr Pro Ser Asp Phe Asp Asp Ala Ile Ala Asp Ala Arg Gln Lys Tyr
    690                 695                 700

Ser Asp Ser Ile Gly Ala Pro Arg Ile Pro Asn Val Gly Trp Asp Asp
705                 710                 715                 720

Val Gly Gly Met Glu Gly Val Lys Lys Asp Ile Leu Asp Thr Ile Glu
                725                 730                 735

Thr Pro Leu Lys Tyr Pro His Trp Phe Ser Asp Gly Val Lys Lys Arg
            740                 745                 750

Ser Gly Ile Leu Phe Tyr Gly Pro Pro Gly Thr Gly Lys Thr Leu Leu
        755                 760                 765

Ala Lys Ala Ile Ala Thr Thr Phe Ser Leu Asn Phe Phe Ser Val Lys
    770                 775                 780

Gly Pro Glu Leu Leu Asn Met Tyr Ile Gly Glu Ser Glu Ala Asn Val
785                 790                 795                 800

Arg Arg Val Phe Gln Lys Ala Arg Asp Ala Lys Pro Cys Val Val Phe
                805                 810                 815

Phe Asp Glu Leu Asp Ser Val Ala Pro Gln Arg Gly Asn Gln Gly Asp
            820                 825                 830

Ser Gly Gly Val Met Asp Arg Ile Val Ser Gln Leu Leu Ala Glu Leu
        835                 840                 845

Asp Gly Met Ser Thr Ala Gly Gly Glu Gly Val Phe Val Val Gly Ala
    850                 855                 860

Thr Asn Arg Pro Asp Leu Leu Asp Glu Ala Leu Leu Arg Pro Gly Arg
865                 870                 875                 880

Phe Asp Lys Met Leu Tyr Leu Gly Ile Ser Asp Thr His Glu Lys Gln
                885                 890                 895

Gln Thr Ile Met Glu Ala Leu Thr Arg Lys Phe Arg Leu Ala Ala Asp
```

```
            900                 905                 910

Val Ser Leu Glu Ala Ile Ser Lys Arg Cys Pro Phe Thr Phe Thr Gly
        915                 920                 925

Ala Asp Phe Tyr Ala Leu Cys Ser Asp Ala Met Leu Asn Ala Met Thr
    930                 935                 940

Arg Thr Ala Asn Glu Val Asp Ala Lys Ile Lys Leu Leu Asn Lys Asn
945                 950                 955                 960

Arg Glu Glu Ala Gly Glu Glu Pro Val Ser Ile Arg Trp Trp Phe Asp
                965                 970                 975

His Glu Ala Thr Lys Ser Asp Ile Glu Val Glu Val Ala Gln Gln Asp
            980                 985                 990

Phe Glu Lys Ala Lys Asp Glu Leu  Ser Pro Ser Val Ser  Ala Glu Glu
        995                 1000                1005

Leu Gln  His Tyr Leu Lys Leu  Arg Gln Gln Phe Glu  Gly Gly Lys
    1010                 1015                1020

Lys
```

<210> 115
<211> 356
<212> PRT
<213> Yarrowia lipolytica

<220>
<221> MISC_FEATURE
<222> (1)..(356)
<223> YlPex7p; GenBank Accession No. CAG78389

<400> 115

```
Met Leu Gly Phe Lys Thr Gln Gly Phe Asn Gly Tyr Ala Ala Asn Tyr
1               5                   10                  15

Ser Pro Phe Phe Asn Asp Lys Ile Ala Val Gly Thr Ala Ala Asn Tyr
            20                  25                  30

Gly Leu Val Gly Asn Gly Lys Leu Phe Ile Leu Gly Ile Ser Pro Glu
        35                  40                  45

Gly Arg Met Val Cys Glu Gly Gln Phe Asp Thr Gln Asp Gly Ile Phe
    50                  55                  60

Asp Val Ala Trp Ser Glu Gln His Glu Asn His Val Ala Thr Ala Cys
```

```
65                  70                  75                  80

Gly Asp Gly Ser Val Lys Leu Phe Asp Ile Lys Ala Gly Ala Phe Pro
                85                  90                  95

Leu Val Ser Phe Lys Glu His Thr Arg Glu Val Phe Ser Val Asn Trp
            100                 105                 110

Asn Met Ala Asn Lys Ala Leu Phe Cys Thr Ser Ser Trp Asp Ser Thr
        115                 120                 125

Ile Lys Ile Trp Thr Pro Glu Arg Thr Asn Ser Ile Met Thr Leu Gly
    130                 135                 140

Gln Pro Ala Pro Ala Gln Gly Thr Asn Ala Ser Ala His Ile Gly Arg
145                 150                 155                 160

Gln Thr Ala Pro Asn Gln Ala Ala Ala Gln Glu Cys Ile Tyr Ser Ala
                165                 170                 175

Lys Phe Ser Pro His Thr Asp Ser Ile Ile Ala Ser Ala His Ser Thr
            180                 185                 190

Gly Met Val Lys Val Trp Asp Thr Arg Ala Pro Gln Pro Leu Gln Gln
        195                 200                 205

Gln Phe Ser Thr Gln Gln Thr Glu Ser Gly Gly Pro Pro Glu Val Leu
    210                 215                 220

Ser Leu Asp Trp Asn Lys Tyr Arg Pro Thr Val Ile Ala Thr Gly Gly
225                 230                 235                 240

Val Asp Arg Ser Val Gln Val Tyr Asp Ile Arg Met Thr Gln Pro Ala
                245                 250                 255

Ala Asn Gln Pro Val Gln Pro Leu Ser Leu Ile Leu Gly His Arg Leu
            260                 265                 270

Pro Val Arg Gly Val Ser Trp Ser Pro His His Ala Asp Leu Leu Leu
        275                 280                 285

Ser Cys Ser Tyr Asp Met Thr Ala Arg Val Trp Arg Asp Ala Ser Thr
    290                 295                 300

Gly Gly Asn Tyr Leu Ala Arg Gln Arg Gly Gly Thr Glu Val Lys Cys
305                 310                 315                 320

Met Asp Arg His Thr Glu Phe Val Ile Gly Gly Asp Trp Ser Leu Trp
                325                 330                 335
```

Gly Asp Pro Gly Trp Ile Thr Thr Val Gly Trp Asp Gln Met Val Tyr
340 345 350

Val Trp His Ala
355

<210> 116
<211> 671
<212> PRT
<213> Yarrowia lipolytica

<220>
<221> MISC_FEATURE
<222> (1)..(671)
<223> YlPex8p; GenBank Accession No. CAG80447

<400> 116

```
Met Asn Lys Tyr Leu Val Pro Pro Pro Gln Ala Asn Arg Thr Val Thr
1               5                   10                  15

Asn Leu Asp Leu Leu Ile Asn Asn Leu Arg Gly Ser Ser Thr Pro Gly
            20                  25                  30

Ala Ala Glu Val Asp Thr Arg Asp Ile Leu Gln Arg Ile Val Phe Ile
        35                  40                  45

Leu Pro Thr Ile Lys Asn Pro Leu Asn Leu Asp Leu Val Ile Lys Glu
    50                  55                  60

Ile Ile Asn Ser Pro Arg Leu Leu Pro Pro Leu Ile Asp Leu His Asp
65                  70                  75                  80

Tyr Gln Gln Leu Thr Asp Ala Phe Arg Ala Thr Ile Lys Arg Lys Ala
                85                  90                  95

Leu Val Thr Asp Pro Thr Ile Ser Phe Glu Ala Trp Leu Glu Thr Cys
            100                 105                 110

Phe Gln Val Ile Thr Arg Phe Ala Gly Pro Gly Trp Lys Lys Leu Pro
        115                 120                 125

Leu Leu Ala Gly Leu Ile Leu Ala Asp Tyr Asp Ile Ser Ala Asp Gly
    130                 135                 140

Pro Thr Leu Glu Arg Lys Pro Gly Phe Pro Ser Lys Leu Lys His Leu
145                 150                 155                 160

Leu Lys Arg Glu Phe Val Thr Thr Phe Asp Gln Cys Leu Ser Ile Asp
                165                 170                 175
```

```
Thr Arg Asn Arg Ser Asp Ala Thr Lys Trp Val Pro Val Leu Ala Cys
            180                 185                 190

Ile Ser Ile Ala Gln Val Tyr Ser Leu Leu Gly Asp Val Ala Ile Asn
        195                 200                 205

Tyr Arg Arg Phe Leu Gln Val Gly Leu Asp Leu Ile Phe Ser Asn Tyr
    210                 215                 220

Gly Leu Glu Met Gly Thr Ala Leu Ala Arg Leu His Ala Glu Ser Gly
225                 230                 235                 240

Gly Asp Ala Thr Thr Ala Gly Gly Leu Ile Gly Lys Lys Leu Lys Glu
                245                 250                 255

Pro Val Val Ala Leu Leu Asn Thr Phe Ala His Ile Ala Ser Ser Cys
            260                 265                 270

Ile Val His Val Asp Ile Asp Tyr Ile Asp Arg Ile Gln Asn Lys Ile
        275                 280                 285

Ile Leu Val Cys Glu Asn Gln Ala Glu Thr Trp Arg Ile Leu Thr Ile
    290                 295                 300

Glu Ser Pro Thr Val Met His His Gln Glu Ser Val Gln Tyr Leu Lys
305                 310                 315                 320

Trp Glu Leu Phe Thr Leu Cys Ile Ile Met Gln Gly Ile Ala Asn Met
                325                 330                 335

Leu Leu Thr Gln Lys Met Asn Gln Phe Met Tyr Leu Gln Leu Ala Tyr
            340                 345                 350

Lys Gln Leu Gln Ala Leu His Ser Ile Tyr Phe Ile Val Asp Gln Met
        355                 360                 365

Gly Ser Gln Phe Ala Ala Tyr Asp Tyr Val Phe Phe Ser Ala Ile Asp
    370                 375                 380

Val Leu Leu Ser Glu Tyr Ala Pro Tyr Ile Lys Asn Arg Gly Thr Ile
385                 390                 395                 400

Pro Pro Asn Lys Glu Phe Val Ala Glu Arg Leu Ala Ala Asn Leu Ala
                405                 410                 415

Gly Thr Ser Asn Val Gly Ser His Leu Pro Ile Asp Arg Ser Arg Val
            420                 425                 430
```

```
Leu Phe Ala Leu Asn Tyr Tyr Glu Gln Leu Val Thr Val Cys His Asp
        435                 440                 445

Ser Cys Val Glu Thr Ile Ile Tyr Pro Met Ala Arg Ser Phe Leu Tyr
    450                 455                 460

Pro Thr Ser Asp Ile Gln Gln Leu Lys Pro Leu Val Glu Ala Ala His
465                 470                 475                 480

Ser Val Ile Leu Ala Gly Leu Ala Val Pro Thr Asn Ala Val Val Asn
                485                 490                 495

Ala Lys Leu Ile Pro Glu Tyr Met Gly Gly Val Leu Pro Leu Phe Pro
            500                 505                 510

Gly Val Phe Ser Trp Asn Gln Phe Val Leu Ala Ile Gln Ser Ile Val
        515                 520                 525

Asn Thr Val Ser Pro Pro Ser Glu Val Phe Lys Thr Asn Gln Lys Leu
    530                 535                 540

Phe Arg Leu Val Leu Asp Ser Leu Met Lys Lys Cys Arg Asp Thr Pro
545                 550                 555                 560

Val Gly Ile Pro Val Pro His Ser Val Thr Val Ser Gln Glu Gln Glu
                565                 570                 575

Asp Ile Pro Pro Thr Gln Arg Ala Val Val Met Leu Ala Leu Ile Asn
            580                 585                 590

Ser Leu Pro Tyr Val Asp Ile Arg Ser Phe Glu Leu Trp Leu Gln Glu
        595                 600                 605

Thr Trp Asn Met Ile Glu Ala Thr Pro Met Leu Ala Glu Asn Ala Pro
    610                 615                 620

Asn Lys Glu Leu Ala His Ala Glu His Glu Phe Leu Val Leu Glu Met
625                 630                 635                 640

Trp Lys Met Ile Ser Gly Asn Ile Asp Gln Arg Leu Asn Asp Val Ala
                645                 650                 655

Ile Arg Trp Trp Tyr Lys Lys Asn Ala Arg Val His Gly Thr Leu
            660                 665                 670
```

<210> 117
<211> 377

<212> PRT
<213> Yarrowia lipolytica

<220>
<221> MISC_FEATURE
<222> (1)..(377)
<223> YlPex10p; GenBank Accession No. CAG81606

<400> 117

```
Met Trp Gly Ser Ser His Ala Phe Ala Gly Glu Ser Asp Leu Thr Leu
1               5                   10                  15

Gln Leu His Thr Arg Ser Asn Met Ser Asp Asn Thr Thr Ile Lys Lys
            20                  25                  30

Pro Ile Arg Pro Lys Pro Ile Arg Thr Glu Arg Leu Pro Tyr Ala Gly
        35                  40                  45

Ala Ala Glu Ile Ile Arg Ala Asn Gln Lys Asp His Tyr Phe Glu Ser
    50                  55                  60

Val Leu Glu Gln His Leu Val Thr Phe Leu Gln Lys Trp Lys Gly Val
65                  70                  75                  80

Arg Phe Ile His Gln Tyr Lys Glu Glu Leu Glu Thr Ala Ser Lys Phe
                85                  90                  95

Ala Tyr Leu Gly Leu Cys Thr Leu Val Gly Ser Lys Thr Leu Gly Glu
            100                 105                 110

Glu Tyr Thr Asn Leu Met Tyr Thr Ile Arg Asp Arg Thr Ala Leu Pro
        115                 120                 125

Gly Val Val Arg Arg Phe Gly Tyr Val Leu Ser Asn Thr Leu Phe Pro
    130                 135                 140

Tyr Leu Phe Val Arg Tyr Met Gly Lys Leu Arg Ala Lys Leu Met Arg
145                 150                 155                 160

Glu Tyr Pro His Leu Val Glu Tyr Asp Glu Asp Glu Pro Val Pro Ser
                165                 170                 175

Pro Glu Thr Trp Lys Glu Arg Val Ile Lys Thr Phe Val Asn Lys Phe
            180                 185                 190

Asp Lys Phe Thr Ala Leu Glu Gly Phe Thr Ala Ile His Leu Ala Ile
        195                 200                 205

Phe Tyr Val Tyr Gly Ser Tyr Tyr Gln Leu Ser Lys Arg Ile Trp Gly
    210                 215                 220
```

```
Met Arg Tyr Val Phe Gly His Arg Leu Asp Lys Asn Glu Pro Arg Ile
225                 230                 235                 240

Gly Tyr Glu Met Leu Gly Leu Leu Ile Phe Ala Arg Phe Ala Thr Ser
                245                 250                 255

Phe Val Gln Thr Gly Arg Glu Tyr Leu Gly Ala Leu Leu Glu Lys Ser
            260                 265                 270

Val Glu Lys Glu Ala Gly Glu Lys Glu Asp Glu Lys Glu Ala Val Val
        275                 280                 285

Pro Lys Lys Lys Ser Ser Ile Pro Phe Ile Glu Asp Thr Glu Gly Glu
    290                 295                 300

Thr Glu Asp Lys Ile Asp Leu Glu Asp Pro Arg Gln Leu Lys Phe Ile
305                 310                 315                 320

Pro Glu Ala Ser Arg Ala Cys Thr Leu Cys Leu Ser Tyr Ile Ser Ala
                325                 330                 335

Pro Ala Cys Thr Pro Cys Gly His Phe Phe Cys Trp Asp Cys Ile Ser
            340                 345                 350

Glu Trp Val Arg Glu Lys Pro Glu Cys Pro Leu Cys Arg Gln Gly Val
        355                 360                 365

Arg Glu Gln Asn Leu Leu Pro Ile Arg
    370                 375
```

<210> 118
<211> 408
<212> PRT
<213> Yarrowia lipolytica

<220>
<221> MISC_FEATURE
<222> (1)..(408)
<223> YlPex12p; GenBank Accession No. CAG81532

<400> 118

```
Met Asp Tyr Phe Ser Ser Leu Asn Ala Ser Gln Leu Asp Pro Asp Val
1               5                   10                  15

Pro Thr Leu Phe Glu Leu Leu Ser Ala Lys Gln Leu Glu Gly Leu Ile
            20                  25                  30

Ala Pro Ser Val Arg Tyr Ile Leu Ala Phe Tyr Ala Gln Arg His Pro
        35                  40                  45
```

```
Arg Tyr Leu Leu Arg Ile Val Asn Arg Tyr Asp Glu Leu Tyr Ala Leu
    50                  55                  60

Phe Met Gly Leu Val Glu Tyr Tyr Asn Leu Lys Thr Trp Asn Ala Ser
65                  70                  75                  80

Phe Thr Glu Lys Phe Tyr Gly Leu Lys Arg Thr Gln Ile Leu Thr Asn
                85                  90                  95

Pro Ala Leu Arg Thr Arg Gln Ala Val Pro Asp Leu Val Glu Ala Glu
            100                 105                 110

Lys Arg Leu Ser Lys Lys Lys Ile Trp Gly Ser Leu Phe Phe Leu Ile
        115                 120                 125

Val Val Pro Tyr Val Lys Glu Lys Leu Asp Ala Arg Tyr Glu Arg Leu
    130                 135                 140

Lys Gly Arg Tyr Leu Ala Arg Asp Ile Asn Glu Glu Arg Ile Glu Ile
145                 150                 155                 160

Lys Arg Thr Gly Thr Ala Gln Gln Ile Ala Val Phe Glu Phe Asp Tyr
                165                 170                 175

Trp Leu Leu Lys Leu Tyr Pro Ile Val Thr Met Gly Cys Thr Thr Ala
            180                 185                 190

Thr Leu Ala Phe His Met Leu Phe Leu Phe Ser Val Thr Arg Ala Tyr
        195                 200                 205

Ser Ile Asp Asp Phe Leu Leu Asn Ile Gln Phe Ser Arg Met Thr Arg
    210                 215                 220

Tyr Asp Tyr Gln Met Glu Thr Gln Arg Asp Ser Arg Asn Ala Ala Asn
225                 230                 235                 240

Val Ala His Thr Met Lys Ser Ile Ser Glu Tyr Pro Val Ala Glu Arg
                245                 250                 255

Val Met Leu Leu Leu Thr Thr Lys Ala Gly Ala Asn Ala Met Arg Ser
            260                 265                 270

Ala Ala Leu Ser Gly Leu Ser Tyr Val Leu Pro Thr Ser Ile Phe Ala
        275                 280                 285

Leu Lys Phe Leu Glu Trp Trp Tyr Ala Ser Asp Phe Ala Arg Gln Leu
    290                 295                 300

Asn Gln Lys Arg Arg Gly Asp Leu Glu Asp Asn Leu Pro Val Pro Asp
```

305 310 315 320

Lys Val Lys Gly Ala Asp Lys Leu Ala Glu Ser Val Ala Lys Trp Lys
325 330 335

Glu Asp Thr Ser Lys Cys Pro Leu Cys Ser Lys Glu Leu Val Asn Pro
340 345 350

Thr Val Ile Glu Ser Gly Tyr Val Phe Cys Tyr Thr Cys Ile Tyr Arg
355 360 365

His Leu Glu Asp Gly Asp Glu Glu Thr Gly Gly Arg Cys Pro Val Thr
370 375 380

Gly Gln Lys Leu Leu Gly Cys Arg Trp Gln Asp Asp Val Trp Gln Val
385 390 395 400

Thr Gly Leu Arg Arg Leu Met Val
405

<210> 119
<211> 412
<212> PRT
<213> Yarrowia lipolytica

<220>
<221> MISC_FEATURE
<222> (1)..(412)
<223> YlPexl3p; GenBank Accession No. CAG81789

<400> 119

```
Met Ser Val Pro Arg Pro Lys Pro Trp Glu Gly Ala Ser Gly Ser Ser
1               5                   10                  15

Ala Ala Thr Ala Thr Pro Ala Ala Thr Ala Thr Pro Ala Ser Thr Asp
            20                  25                  30

Ala Val Ser Ser Ser Ala Gly Ser Ala Thr Gly Ala Pro Glu Leu Pro
        35                  40                  45

Ser Arg Pro Ser Ala Met Gly Ser Thr Ser Asn Ala Leu Ser Ser Pro
    50                  55                  60

Met Gly Ser Ser Met Asn Ser Gly Tyr Gly Gly Met Asn Ser Gly Tyr
65                  70                  75                  80

Gly Gly Met Gly Ser Ser Tyr Gly Ser Gly Tyr Gly Ser Ser Tyr Gly
                85                  90                  95

Met Gly Ser Ser Tyr Gly Ser Gly Tyr Gly Ser Gly Leu Gly Gly Tyr
```

100 105 110

Gly Ser Tyr Gly Gly Met Gly Gly Met Gly Gly Met Tyr Gly Ser Arg
115 120 125

Tyr Gly Gly Tyr Gly Ser Tyr Gly Gly Met Gly Gly Tyr Gly Gly Tyr
130 135 140

Gly Gly Met Gly Gly Gly Pro Met Gly Gln Asn Gly Leu Ala Gly Gly
145 150 155 160

Thr Gln Ala Thr Phe Gln Leu Ile Glu Ser Ile Val Gly Ala Val Gly
165 170 175

Gly Phe Ala Gln Met Leu Glu Ser Thr Tyr Met Ala Thr Gln Ser Ser
180 185 190

Phe Phe Ala Met Val Ser Val Ala Glu Gln Phe Gly Asn Leu Lys Asn
195 200 205

Thr Leu Gly Ser Leu Leu Gly Ile Tyr Ala Ile Met Arg Trp Ala Arg
210 215 220

Arg Leu Val Ala Lys Leu Ser Gly Gln Pro Val Thr Gly Ala Asn Gly
225 230 235 240

Ile Thr Pro Ala Gly Phe Ala Lys Phe Glu Ala Thr Gly Gly Ala Ala
245 250 255

Gly Pro Gly Arg Gly Pro Arg Pro Ser Tyr Lys Pro Leu Leu Phe Phe
260 265 270

Leu Thr Ala Val Phe Gly Leu Pro Tyr Leu Leu Gly Arg Leu Ile Lys
275 280 285

Ala Leu Ala Ala Lys Gln Glu Gly Met Tyr Asp Glu His Gly Asn Leu
290 295 300

Leu Pro Gly Ala Gln Met Gly Met Gly Gly Pro Gly Met Glu Gly Gly
305 310 315 320

Ala Glu Ile Asp Pro Ser Lys Leu Glu Phe Cys Arg Ala Asn Phe Asp
325 330 335

Phe Val Pro Glu Asn Pro Gln Leu Glu Leu Glu Leu Arg Lys Gly Asp
340 345 350

Leu Val Ala Val Leu Ala Lys Thr Asp Pro Met Gly Asn Pro Ser Gln
355 360 365

```
Trp Trp Arg Val Arg Thr Arg Asp Gly Arg Ser Gly Tyr Val Pro Ala
    370                 375                 380

Asn Tyr Leu Glu Val Ile Pro Arg Pro Ala Val Glu Ala Pro Lys Lys
385                 390                 395                 400

Val Glu Glu Ile Gly Ala Ser Ala Val Pro Val Asn
                405                 410
```

<210> 120
<211> 380
<212> PRT
<213> Yarrowia lipolytica

<220>
<221> MISC_FEATURE
<222> (1)..(380)
<223> YlPexl4p; GenBank Accession No. CAG79323

<400> 120

```
Met Ile Pro Ser Cys Leu Ser Thr Gln His Met Ala Pro Arg Glu Asp
1               5                   10                  15

Leu Val Gln Ser Ala Val Ala Phe Leu Asn Asp Pro Gln Ala Ala Thr
            20                  25                  30

Ala Pro Leu Ala Lys Arg Ile Glu Phe Leu Glu Ser Lys Asp Met Thr
        35                  40                  45

Pro Glu Glu Ile Glu Glu Ala Leu Lys Arg Ala Gly Ser Gly Ser Ala
    50                  55                  60

Gln Ser His Pro Gly Ser Val Val Ser His Gly Gly Ala Ala Pro Thr
65                  70                  75                  80

Val Pro Ala Ser Tyr Ala Phe Gln Ser Ala Pro Pro Leu Pro Glu Arg
                85                  90                  95

Asp Trp Lys Asp Val Phe Ile Met Ala Thr Val Thr Val Gly Val Gly
            100                 105                 110

Phe Gly Leu Tyr Thr Val Ala Lys Arg Tyr Leu Met Pro Leu Ile Leu
        115                 120                 125

Pro Pro Thr Pro Pro Ser Leu Glu Ala Asp Lys Glu Ala Leu Glu Ala
    130                 135                 140

Glu Phe Ala Arg Val Gln Gly Leu Leu Asp Gln Val Gln Gln Asp Thr
145                 150                 155                 160
```

```
Glu Glu Val Lys Asn Ser Gln Val Glu Val Ala Lys Arg Val Thr Asp
                165                 170                 175

Ala Leu Lys Gly Val Glu Glu Thr Ile Asp Gln Leu Lys Ser Gln Thr
            180                 185                 190

Lys Lys Arg Asp Asp Glu Met Lys Leu Val Thr Ala Glu Val Glu Arg
        195                 200                 205

Ile Arg Asp Arg Leu Pro Lys Asn Ile Asp Lys Leu Lys Asp Ser Gln
    210                 215                 220

Glu Gln Gly Leu Ala Asp Ile Gln Ser Glu Leu Lys Ser Leu Lys Gln
225                 230                 235                 240

Leu Leu Ser Thr Arg Thr Ala Ala Ser Ser Gly Pro Lys Leu Pro Pro
                245                 250                 255

Ile Pro Pro Pro Ser Ser Tyr Leu Thr Arg Lys Ala Ser Pro Ala Val
            260                 265                 270

Pro Ala Ala Ala Pro Ala Pro Val Thr Pro Gly Ser Pro Val His Asn
        275                 280                 285

Val Ser Ser Ser Ser Thr Val Pro Ala Asp Arg Asp Asp Phe Ile Pro
    290                 295                 300

Thr Pro Ala Gly Ala Val Pro Met Ile Pro Gln Pro Ala Ser Met Ser
305                 310                 315                 320

Ser Ser Ser Thr Ser Thr Val Pro Asn Ser Ala Ile Ser Ser Ala Pro
                325                 330                 335

Ser Pro Ile Gln Glu Pro Glu Pro Phe Val Pro Glu Pro Gly Asn Ser
            340                 345                 350

Ala Val Lys Lys Pro Ala Pro Lys Ala Ser Ile Pro Ala Trp Gln Leu
        355                 360                 365

Ala Ala Leu Glu Lys Glu Lys Glu Lys Glu Lys Glu
    370                 375                 380
```

<210> 121
<211> 391
<212> PRT
<213> Yarrowia lipolytica

<220>

<221> MISC_FEATURE
<222> (1)..(391)
<223> YlPexl6p; GenBank Accession No. CAG79622

<400> 121

```
Met Thr Asp Lys Leu Val Lys Val Met Gln Lys Lys Lys Ser Ala Pro
1               5                   10                  15

Gln Thr Trp Leu Asp Ser Tyr Asp Lys Phe Leu Val Arg Asn Ala Ala
            20                  25                  30

Ser Ile Gly Ser Ile Glu Ser Thr Leu Arg Thr Val Ser Tyr Val Leu
        35                  40                  45

Pro Gly Arg Phe Asn Asp Val Glu Ile Ala Thr Glu Thr Leu Tyr Ala
    50                  55                  60

Val Leu Asn Val Leu Gly Leu Tyr His Asp Thr Ile Ile Ala Arg Ala
65                  70                  75                  80

Val Ala Ala Ser Pro Asn Ala Ala Ala Val Tyr Arg Pro Ser Pro His
                85                  90                  95

Asn Arg Tyr Thr Asp Trp Phe Ile Lys Asn Arg Lys Gly Tyr Lys Tyr
            100                 105                 110

Ala Ser Arg Ala Val Thr Phe Val Lys Phe Gly Glu Leu Val Ala Glu
        115                 120                 125

Met Val Ala Lys Lys Asn Gly Gly Glu Met Ala Arg Trp Lys Cys Ile
    130                 135                 140

Ile Gly Ile Glu Gly Ile Lys Ala Gly Leu Arg Ile Tyr Met Leu Gly
145                 150                 155                 160

Ser Thr Leu Tyr Gln Pro Leu Cys Thr Thr Pro Tyr Pro Asp Arg Glu
                165                 170                 175

Val Thr Gly Glu Leu Leu Glu Thr Ile Cys Arg Asp Glu Gly Glu Leu
            180                 185                 190

Asp Ile Glu Lys Gly Leu Met Asp Pro Gln Trp Lys Met Pro Arg Thr
        195                 200                 205

Gly Arg Thr Ile Pro Glu Ile Ala Pro Thr Asn Val Glu Gly Tyr Leu
    210                 215                 220

Leu Thr Lys Val Leu Arg Ser Glu Asp Val Asp Arg Pro Tyr Asn Leu
225                 230                 235                 240
```

Leu Ser Arg Leu Asp Asn Trp Gly Val Val Ala Glu Leu Leu Ser Ile
245 250 255

Leu Arg Pro Leu Ile Tyr Ala Cys Leu Leu Phe Arg Gln His Val Asn
260 265 270

Lys Thr Val Pro Ala Ser Thr Lys Ser Lys Phe Pro Phe Leu Asn Ser
275 280 285

Pro Trp Ala Pro Trp Ile Ile Gly Leu Val Ile Glu Ala Leu Ser Arg
290 295 300

Lys Met Met Gly Ser Trp Leu Leu Arg Gln Arg Gln Ser Gly Lys Thr
305 310 315 320

Pro Thr Ala Leu Asp Gln Met Glu Val Lys Gly Arg Thr Asn Leu Leu
325 330 335

Gly Trp Trp Leu Phe Arg Gly Glu Phe Tyr Gln Ala Tyr Thr Arg Pro
340 345 350

Leu Leu Tyr Ser Ile Val Ala Arg Leu Glu Lys Ile Pro Gly Leu Gly
355 360 365

Leu Phe Gly Ala Leu Ile Ser Asp Tyr Leu Tyr Leu Phe Asp Arg Tyr
370 375 380

Tyr Phe Thr Ala Ser Thr Leu
385 390

<210> 122
<211> 225
<212> PRT
<213> Yarrowia lipolytica

<220>
<221> MISC_FEATURE
<222> (1)..(225)
<223> YlPexl7p; GenBank Accession No. CAG84025

<400> 122

```
Met Ser Ala Phe Pro Glu Pro Ser Ser Phe Glu Ile Glu Phe Ala Lys
1               5                   10                  15

Gln Met Asn Arg Pro Arg Thr Val Gln Phe Lys Gln Leu Val Ala Val
            20                  25                  30

Leu Tyr Ile Phe Gly Gly Thr Ser Ala Leu Ile Tyr Ile Ile Ser Lys
        35                  40                  45

Thr Ile Leu Asn Pro Leu Phe Glu Glu Leu Thr Phe Ala Arg Ser Glu
    50                  55                  60

Tyr Ala Ile His Ala Arg Arg Leu Met Glu Gln Leu Asn Ala Lys Leu
65                  70                  75                  80

Ser Ser Met Ala Ser Tyr Ile Pro Pro Val Arg Ala Leu Gln Gly Gln
                85                  90                  95

Arg Phe Val Asp Ala Gln Thr Gln Thr Glu Asp Glu Glu Gly Glu Asp
            100                 105                 110

Ile Pro Asn Pro Ser Leu Gly Lys Ser Ser His Val Ser Phe Gly Glu
        115                 120                 125

Ser Pro Met Gln Leu Lys Leu Ala Glu Lys Glu Lys Gln Gln Lys Leu
    130                 135                 140

Ile Asp Asp Ser Val Asp Asn Leu Glu Arg Leu Ala Asp Ser Leu Lys
145                 150                 155                 160

His Ala Gly Glu Val Ser Asp Leu Ser Ala Leu Ser Gly Phe Lys Tyr
                165                 170                 175

Gln Val Glu Glu Leu Thr Asn Tyr Ser Asp Gln Leu Ala Met Ser Gly
            180                 185                 190

Tyr Ser Met Met Lys Ser Gly Leu Pro Gly His Glu Thr Ala Met Ser
        195                 200                 205

Glu Thr Lys Lys Glu Ile Arg Ser Leu Lys Gly Ser Val Leu Ser Val
    210                 215                 220
```

Arg
225

<210> 123
<211> 324
<212> PRT

<213> Yarrowia lipolytica

<220>
<221> MISC_FEATURE
<222> (1)..(324)
<223> YlPexl9p; GenBank Accession No. AAK84827

<400> 123

```
Met Ser His Glu Glu Asp Leu Asp Asp Leu Asp Asp Phe Leu Asp Glu
1               5                   10                  15
```

Phe Asp Glu Gln Val Leu Ser Lys Pro Pro Gly Ala Gln Lys Asp Ala
20 25 30

Thr Pro Thr Thr Ser Thr Ala Pro Thr Thr Ala Glu Ala Lys Pro Asp
35 40 45

Ala Thr Lys Lys Ser Thr Glu Thr Ser Gly Thr Asp Ser Lys Thr Glu
50 55 60

Gly Ala Asp Thr Ala Asp Lys Asn Ala Ala Thr Asp Ser Ala Glu Ala
65 70 75 80

Gly Ala Glu Lys Val Ser Leu Pro Asn Leu Glu Asp Gln Leu Ala Gly
85 90 95

Leu Lys Met Asp Asp Phe Leu Lys Asp Ile Glu Ala Asp Pro Glu Ser
100 105 110

Lys Ala Gln Phe Glu Ser Leu Leu Lys Glu Ile Asn Asn Val Thr Ser
115 120 125

Ala Thr Ala Ser Glu Lys Ala Gln Gln Pro Lys Ser Phe Lys Glu Thr
130 135 140

Ile Ser Ala Thr Ala Asp Arg Leu Asn Gln Ser Asn Gln Glu Met Gly
145 150 155 160

Asp Met Pro Leu Gly Asp Asp Met Leu Ala Gly Leu Met Glu Gln Leu
165 170 175

Ser Gly Ala Gly Gly Phe Gly Glu Gly Gly Glu Gly Asp Phe Gly Asp
180 185 190

Met Leu Gly Gly Ile Met Arg Gln Leu Ala Ser Lys Glu Val Leu Tyr
195 200 205

Gln Pro Leu Lys Glu Met His Asp Asn Tyr Pro Lys Trp Trp Asp Glu
210 215 220

His Gly Ser Lys Val Thr Glu Glu Lys Glu Arg Asp Arg Leu Lys Leu
225 230 235 240

Gln Gln Asp Ile Val Gly Lys Ile Cys Ala Lys Phe Glu Asp Pro Ser
245 250 255

Tyr Ser Asp Asp Ser Glu Ala Asp Arg Ala Val Ile Thr Gln Leu Met
260 265 270

Asp Glu Met Gln Glu Thr Gly Ala Pro Pro Asp Glu Ile Met Ser Asn
275 280 285

Val Ala Asp Gly Ser Ile Pro Gly Gly Leu Asp Gly Leu Gly Leu Gly
290 295 300

Gly Leu Gly Gly Gly Lys Met Pro Glu Met Pro Glu Asn Met Pro Glu
305 310 315 320

Cys Asn Gln Gln

<210> 124
<211> 417
<212> PRT
<213> Yarrowia lipolytica

<220>
<221> MISC_FEATURE
<222> (1)..(417)
<223> YlPex20p; GenBank Accession No. CAG79226

<400> 124

Met Ala Ser Cys Gly Pro Ser Asn Ala Leu Gln Asn Leu Ser Lys His
1 5 10 15

Ala Ser Ala Asp Arg Ser Leu Gln His Asp Arg Met Ala Pro Gly Gly
20 25 30

Ala Pro Gly Ala Gln Arg Gln Gln Phe Arg Ser Gln Thr Gln Gly Gly
35 40 45

Gln Leu Asn Asn Glu Phe Gln Gln Phe Ala Gln Ala Gly Pro Ala His
50 55 60

Asn Ser Phe Glu Gln Ser Gln Met Gly Pro His Phe Gly Gln Gln His
65 70 75 80

Phe Gly Gln Pro His Gln Pro Gln Met Gly Gln His Ala Pro Met Ala
85 90 95

His Gly Gln Gln Ser Asp Trp Ala Gln Ser Phe Ser Gln Leu Asn Leu
100 105 110

Gly Pro Gln Thr Gly Pro Gln His Thr Gln Gln Ser Asn Trp Gly Gln
115 120 125

Asp Phe Met Arg Gln Ser Pro Gln Ser His Gln Val Gln Pro Gln Met
130 135 140

```
Ala Asn Gly Val Met Gly Ser Met Ser Gly Met Ser Ser Phe Gly Pro
145                 150                 155                 160

Met Tyr Ser Asn Ser Gln Leu Met Asn Ser Thr Tyr Gly Leu Gln Thr
                165                 170                 175

Glu His Gln Gln Thr His Lys Thr Glu Thr Lys Ser Ser Gln Asp Ala
            180                 185                 190

Ala Phe Glu Ala Ala Phe Gly Ala Val Glu Glu Ser Ile Thr Lys Thr
        195                 200                 205

Ser Asp Lys Gly Lys Glu Val Glu Lys Asp Pro Met Glu Gln Thr Tyr
    210                 215                 220

Arg Tyr Asp Gln Ala Asp Ala Leu Asn Arg Gln Ala Glu His Ile Ser
225                 230                 235                 240

Asp Asn Ile Ser Arg Glu Glu Val Asp Ile Lys Thr Asp Glu Asn Gly
                245                 250                 255

Glu Phe Ala Ser Ile Ala Arg Gln Ile Ala Ser Ser Leu Glu Glu Ala
            260                 265                 270

Asp Lys Ser Lys Phe Glu Lys Ser Thr Phe Met Asn Leu Met Arg Arg
        275                 280                 285

Ile Gly Asn His Glu Val Thr Leu Asp Gly Asp Lys Leu Val Asn Lys
    290                 295                 300

Glu Gly Glu Asp Ile Arg Glu Glu Val Arg Asp Glu Leu Leu Arg Glu
305                 310                 315                 320

Gly Ala Ser Gln Glu Asn Gly Phe Gln Ser Glu Ala Gln Gln Thr Ala
                325                 330                 335

Pro Leu Pro Val His His Glu Ala Pro Pro Pro Glu Gln Ile His Pro
            340                 345                 350

His Thr Glu Thr Gly Asp Lys Gln Leu Glu Asp Pro Met Val Tyr Ile
        355                 360                 365

Glu Gln Glu Ala Ala Arg Arg Ala Ala Glu Ser Gly Arg Thr Val Glu
    370                 375                 380

Glu Glu Lys Leu Asn Phe Tyr Ser Pro Phe Glu Tyr Ala Gln Lys Leu
385                 390                 395                 400

Gly Pro Gln Gly Val Ala Lys Gln Ser Asn Trp Glu Glu Asp Tyr Asp
```

405 410 415

Phe

<210> 125
<211> 195
<212> PRT
<213> Yarrowia lipolytica

<220>
<221> MISC_FEATURE
<222> (1)..(195)
<223> YlPex22p; GenBank Accession No. CAG77876

<400> 125

```
Val Pro Arg Cys Thr Ser His Pro Cys Asn Leu Thr Leu His Leu Pro
1               5                   10                  15

Val Thr Thr Met Ala Pro Arg Lys Thr Arg Leu Pro Ala Val Ile Gly
            20                  25                  30

Ala Ala Ala Ala Ala Ala Ala Val Ala Tyr Leu Val Tyr Ser Phe Val
        35                  40                  45

Ala Lys Ser Asn Ser Asp Gln Asp Thr Phe Asp Ser Ser Val Gln Ser
    50                  55                  60

Ser Ser Lys Ser Ser Thr Lys Ser Pro Lys Ser Thr Ala Thr Asn Ser
65                  70                  75                  80

Lys Ile Thr Val Val Val Ser Gln Glu Leu Val Gln Ser Gln Leu Val
                85                  90                  95

Asp Phe Lys His Leu Met Ser Val His Pro Asn Leu Val Val Ile Val
            100                 105                 110

Pro Pro Met Val Ala Asn Lys Phe His Arg Ala Leu Lys Ser Ser Val
        115                 120                 125

Gly His Asp His Gly Val Lys Val Ile Arg Cys Asp Thr Asp Val Gly
    130                 135                 140

Val Ile His Val Ile Lys His Ile Arg Pro Asp Leu Ala Leu Ile Ala
145                 150                 155                 160

Asp Gly Val Gly Asp Asn Ile Gln Gly Glu Ile Lys Arg Phe Val Gly
                165                 170                 175

Ser Ser Glu Ala Leu Ser Gly Asp Val Asn Leu Ala Ala Glu Arg Leu


                180                 185                 190

    Thr Gly Leu
            195
```

<210> 126
<211> 386
<212> PRT
<213> Yarrowia lipolytica

<220>
<221> MISC_FEATURE
<222> (1)..(386)

<223> YlPex26p; GenBank Accession No. NC_006072, antisense translation of nucleotides 117230-118387

<400> 126

```
Met Pro Pro Ala Met Pro Gln Met Thr Thr Ser Thr Leu Leu Thr Asp
1               5                   10                  15

Ser Val Thr Ser Ala Val Asn Gln Ala Ala Thr Pro Lys Val Asp Gln
            20                  25                  30

Met Tyr Gln Thr Phe Gly Glu Ser Ala Arg Glu Phe Val Asn Lys Asn
        35                  40                  45

Phe Tyr Asn Ser Tyr Glu Leu Ile Arg Pro Phe Phe Asp Glu Ile Thr
    50                  55                  60

Ala Lys Gly Ala Gln Gln Asn Gly Ser Thr Val Leu Asp Ala Glu Asn
65                  70                  75                  80

Pro His Asn Ile Pro Leu Ser Leu Trp Ile Lys Val Trp Ser Leu Tyr
                85                  90                  95

Leu Ala Ile Leu Asp Ala Ser Cys Lys Gln Ala Gly Glu Ala Leu Leu
            100                 105                 110

Asn Ser Thr Gly Asp Leu Ser Gly Ser Asp Ser Gly Glu Trp Asn Gln
        115                 120                 125

Thr Arg Lys Leu Leu Ala Arg Lys Leu Thr Ser Gly Ser Val Trp Asp
    130                 135                 140

Glu Leu Val Thr Ala Ser Gly Gly Thr Gly Asn Ile His Pro Thr Ile
145                 150                 155                 160

Leu Ala Leu Leu Ala Ser Leu Ser Ile Arg His Asp Thr Asp Ala Lys
                165                 170                 175
```

```
Leu Met Ala Asp Asn Leu Glu Lys Phe Ile Val Thr Tyr Asn Asp Asn
            180                 185                 190

Gly Ser Asp Asp Val Lys Thr Lys Thr Ala Phe Tyr Lys Val Leu Asp
        195                 200                 205

Leu Tyr Leu Leu Arg Val Leu Pro Asp Leu Gly Gln Trp Asp Val Ala
    210                 215                 220

His Ser Phe Val Asn Asn Thr Asn Leu Phe Ser His Glu Gln Lys Lys
225                 230                 235                 240

Glu Met Thr His Lys Leu Asp Gln Ser Gln Lys His Ala Glu Gln Glu
                245                 250                 255

His Lys Arg Leu Leu Glu Glu Ala Gln Glu Lys Glu Lys Ser Asp Ala
            260                 265                 270

Lys Glu Lys Glu Arg Glu Glu Arg Val Ser Arg Asp Thr Gln Ser Arg
        275                 280                 285

Glu Ile Lys Ser Pro Ile Val Asp Ser Ser Thr Ser Ser Arg Asp Val
    290                 295                 300

Thr Arg Asp Thr Thr Arg Glu Leu Ser Lys Ser Ser Arg Gln Pro Arg
305                 310                 315                 320

Thr Leu Ser Gln Ile Ile Ser Thr Ser Leu Lys Ser Gln Phe Asp Gly
                325                 330                 335

Asn Ala Ile Phe Arg Thr Leu Ala Leu Ile Val Ile Val Ser Leu Ser
            340                 345                 350

Ala Ala Asn Pro Leu Ile Arg Lys Arg Val Val Asp Thr Leu Lys Met
        355                 360                 365

Leu Trp Ile Lys Ile Leu Gln Thr Leu Ser Met Gly Phe Lys Val Ser
    370                 375                 380

Tyr Leu
385
```

<210> 127
<211> 8739
<212> DNA
<213> Artificial Sequence

<220>

<223> Plasmid pY116

<400> 127

ggccgccacc gcggcccgag attccggcct cttcggccgc caagcgaccc gggtggacgt 60
ctagaggtac ctagcaatta acagatagtt tgccggtgat aattctctta acctcccaca 120
ctcctttgac ataacgattt atgtaacgaa actgaaattt gaccagatat tgtgtccgcg 180
gtggagctcc agcttttgtt ccctttagtg agggtttaaa cgagcttggc gtaatcatgg 240
tcatagctgt ttcctgtgtg aaattgttat ccgctcacaa ttccacacaa cgtacgagcc 300
ggaagcataa agtgtaaagc ctggggtgcc taatgagtga gctaactcac attaattgcg 360
ttgcgctcac tgcccgcttt ccagtcggga aacctgtcgt gccagctgca ttaatgaatc 420
ggccaacgcg cggggagagg cggtttgcgt attgggcgct cttccgcttc ctcgctcact 480
gactcgctgc gctcggtcgt tcggctgcgg cgagcggtat cagctcactc aaaggcggta 540
atacggttat ccacagaatc aggggataac gcaggaaaga acatgtgagc aaaaggccag 600
caaaaggcca ggaaccgtaa aaaggccgcg ttgctggcgt ttttccatag gctccgcccc 660
cctgacgagc atcacaaaaa tcgacgctca agtcagaggt ggcgaaaccc gacaggacta 720
taaagatacc aggcgtttcc ccctggaagc tccctcgtgc gctctcctgt tccgaccctg 780
ccgcttaccg gatacctgtc cgcctttctc ccttcgggaa gcgtggcgct ttctcatagc 840
tcacgctgta ggtatctcag ttcggtgtag gtcgttcgct ccaagctggg ctgtgtgcac 900
gaaccccccg ttcagcccga ccgctgcgcc ttatccggta actatcgtct tgagtccaac 960
ccggtaagac acgacttatc gccactggca gcagccactg gtaacaggat tagcagagcg 1020
aggtatgtag gcggtgctac agagttcttg aagtggtggc ctaactacgg ctacactaga 1080
aggacagtat ttggtatctg cgctctgctg aagccagtta ccttcggaaa aagagttggt 1140
agctcttgat ccggcaaaca aaccaccgct ggtagcggtg gtttttttgt ttgcaagcag 1200
cagattacgc gcagaaaaaa aggatctcaa gaagatcctt tgatcttttc tacggggtct 1260
gacgctcagt ggaacgaaaa ctcacgttaa gggattttgg tcatgagatt atcaaaaagg 1320
atcttcacct agatcctttt aaattaaaaa tgaagtttta aatcaatcta aagtatatat 1380
gagtaaactt ggtctgacag ttaccaatgc ttaatcagtg aggcacctat ctcagcgatc 1440
tgtctatttc gttcatccat agttgcctga ctccccgtcg tgtagataac tacgatacgg 1500
gagggcttac catctggccc cagtgctgca atgataccgc gagacccacg ctcaccggct 1560
ccagatttat cagcaataaa ccagccagcc ggaagggccg agcgcagaag tggtcctgca 1620
actttatccg cctccatcca gtctattaat tgttgccggg aagctagagt aagtagttcg 1680
ccagttaata gtttgcgcaa cgttgttgcc attgctacag gcatcgtggt gtcacgctcg 1740
tcgtttggta tggcttcatt cagctccggt tcccaacgat caaggcgagt tacatgatcc 1800
cccatgttgt gcaaaaaagc ggttagctcc ttcggtcctc cgatcgttgt cagaagtaag 1860
ttggccgcag tgttatcact catggttatg gcagcactgc ataattctct tactgtcatg 1920
ccatccgtaa gatgcttttc tgtgactggt gagtactcaa ccaagtcatt ctgagaatag 1980

tgtatgcggc gaccgagttg ctcttgcccg gcgtcaatac gggataatac cgcgccacat 2040
agcagaactt taaaagtgct catcattgga aaacgttctt cggggcgaaa actctcaagg 2100
atcttaccgc tgttgagatc cagttcgatg taacccactc gtgcacccaa ctgatcttca 2160
gcatctttta ctttcaccag cgtttctggg tgagcaaaaa caggaaggca aaatgccgca 2220
aaaaagggaa taagggcgac acggaaatgt tgaatactca tactcttcct ttttcaatat 2280
tattgaagca tttatcaggg ttattgtctc atgagcggat acatatttga atgtatttag 2340
aaaaataaac aaataggggt tccgcgcaca tttccccgaa aagtgccacc tgacgcgccc 2400
tgtagcggcg cattaagcgc ggcgggtgtg gtggttacgc gcagcgtgac cgctacactt 2460
gccagcgccc tagcgcccgc tcctttcgct ttcttccctt cctttctcgc cacgttcgcc 2520
ggctttcccc gtcaagctct aaatcggggg ctccctttag ggttccgatt tagtgcttta 2580
cggcacctcg accccaaaaa acttgattag ggtgatggtt cacgtagtgg gccatcgccc 2640
tgatagacgg tttttcgccc tttgacgttg gagtccacgt tctttaatag tggactcttg 2700
ttccaaactg gaacaacact caaccctatc tcggtctatt cttttgattt ataagggatt 2760
ttgccgattt cggcctattg gttaaaaaat gagctgattt aacaaaaatt taacgcgaat 2820
tttaacaaaa tattaacgct tacaatttcc attcgccatt caggctgcgc aactgttggg 2880
aagggcgatc ggtgcgggcc tcttcgctat tacgccagct ggcgaaaggg ggatgtgctg 2940
caaggcgatt aagttgggta acgccagggt tttcccagtc acgacgttgt aaaacgacgg 3000
ccagtgaatt gtaatacgac tcactatagg gcgaattggg taccgggccc cccctcgagg 3060
tcgatggtgt cgataagctt gatatcgaat tcatgtcaca caaaccgatc ttcgcctcaa 3120
ggaaacctaa ttctacatcc gagagactgc cgagatccag tctacactga ttaattttcg 3180
ggccaataat ttaaaaaaat cgtgttatat aatattatat gtattatata tatacatcat 3240
gatgatactg acagtcatgt cccattgcta aatagacaga ctccatctgc cgcctccaac 3300
tgatgttctc aatatttaag gggtcatctc gcattgttta ataataaaca gactccatct 3360
accgcctcca aatgatgttc tcaaaatata ttgtatgaac ttatttttat tacttagtat 3420
tattagacaa cttacttgct ttatgaaaaa cacttcctat ttaggaaaca atttataatg 3480
gcagttcgtt catttaacaa tttatgtaga ataaatgtta taaatgcgta tgggaaatct 3540
taaatatgga tagcataaat gatatctgca ttgcctaatt cgaaatcaac agcaacgaaa 3600
aaaatccctt gtacaacata aatagtcatc gagaaatatc aactatcaaa gaacagctat 3660
tcacacgtta ctattgagat tattattgga cgagaatcac acactcaact gtctttctct 3720
cttctagaaa tacaggtaca agtatgtact attctcattg ttcatacttc tagtcatttc 3780
atcccacata ttccttggat ttctctccaa tgaatgacat tctatcttgc aaattcaaca 3840
attataataa gatataccaa agtagcggta tagtggcaat caaaaagctt ctctggtgtg 3900

cttctcgtat ttatttttat tctaatgatc cattaaaggt atatatttat ttcttgttat 3960
ataatccttt tgtttattac atgggctgga tacataaagg tattttgatt taattttttg 4020
cttaaattca atccccccctc gttcagtgtc aactgtaatg gtaggaaatt accatacttt 4080
tgaagaagca aaaaaaatga aagaaaaaaa aaatcgtatt tccaggttag acgttccgca 4140
gaatctagaa tgcggtatgc ggtacattgt tcttcgaacg taaaagttgc gctccctgag 4200
atattgtaca tttttgcttt tacaagtaca agtacatcgt acaactatgt actactgttg 4260
atgcatccac aacagtttgt tttgtttttt tttgtttttt ttttttctaa tgattcatta 4320
ccgctatgta tacctacttg tacttgtagt aagccgggtt attggcgttc aattaatcat 4380
agacttatga atctgcacgg tgtgcgctgc gagttacttt tagcttatgc atgctacttg 4440
ggtgtaatat tgggatctgt tcggaaatca acggatgctc aaccgatttc gacagtaatt 4500
aattaatttg aatcgaatcg gagcctaaaa tgaacccgag tatatctcat aaaattctcg 4560
gtgagaggtc tgtgactgtc agtacaaggt gccttcatta tgccctcaac cttaccatac 4620
ctcactgaat gtagtgtacc tctaaaaatg aaatacagtg ccaaaagcca aggcactgag 4680
ctcgtctaac ggacttgata tacaaccaat taaaacaaat gaaaagaaat acagttcttt 4740
gtatcatttg taacaattac cctgtacaaa ctaaggtatt gaaatcccac aatattccca 4800
aagtccaccc ctttccaaat tgtcatgcct acaactcata taccaagcac taacctacca 4860
aacaccacta aaaccccaca aaatatatct taccgaatat acagtaacaa gctaccacca 4920
cactcgttgg gtgcagtcgc cagcttaaag atatctatcc acatcagcca caactccctt 4980
cctttaataa accgactaca cccttggcta ttgaggttat gagtgaatat actgtagaca 5040
agacactttc aagaagactg tttccaaaac gtaccactgt cctccactac aaacacaccc 5100
aatctgcttc ttctagtcaa ggttgctaca ccggtaaatt ataaatcatc atttcattag 5160
cagggcaggg cccttttttat agagtcttat acactagcgg accctgccgg tagaccaacc 5220
cgcaggcgcg tcagtttgct ccttccatca atgcgtcgta gaaacgactt actccttctt 5280
gagcagctcc ttgaccttgt tggcaacaag tctccgacct cggaggtgga ggaagagcct 5340
ccgatatcgg cggtagtgat accagcctcg acggactcct tgacggcagc ctcaacagcg 5400
tcaccggcgg gcttcatgtt aagagagaac ttgagcatca tggcggcaga cagaatggtg 5460
gcaatggggt tgaccttctg cttgccgaga tcgggggcag atccgtgaca gggctcgtac 5520
agaccgaacg cctcgttggt gtcgggcaga gaagccagag aggcggaggg cagcagaccc 5580
agagaaccgg ggatgacgga ggcctcgtcg gagatgatat cgccaaacat gttggtggtg 5640
atgatgatac cattcatctt ggagggctgc ttgatgagga tcatggcggc cgagtcgatc 5700
agctggtggt tgagctcgag ctgggggaat tcgtccttga ggactcgagt gacagtcttt 5760
cgccaaagtc gagaggaggc cagcacgttg gccttgtcaa gagaccacac gggaagaggg 5820
gggttgtgct gaagggccag gaaggcggcc attcgggcaa ttcgctcaac ctcaggaacg 5880

gagtaggtct cggtgtcgga agcgacgcca gatccgtcat cctcctttcg ctctccaaag 5940
tagatacctc cgacgagctc tcggacaatg atgaagtcgg tgccctcaac gtttcggatg 6000
ggggagagat cggcgagctt gggcgacagc agctggcagg gtcgcaggtt ggcgtacagg 6060
ttcaggtcct ttcgcagctt gaggagaccc tgctcgggtc gcacgtcggt tcgtccgtcg 6120
ggagtggtcc atacggtgtt ggcagcgcct ccgacagcac cgagcataat agagtcagcc 6180
tttcggcaga tgtcgagagt agcgtcggtg atgggctcgc cctccttctc aatggcagct 6240
cctccaatga gtcggtcctc aaacacaaac tcggtgccgg aggcctcagc aacagacttg 6300
agcaccttga cggcctcggc aatcacctcg gggccacaga agtcgccgcc gagaagaaca 6360
atcttcttgg agtcagtctt ggtcttctta gtttcgggtt ccattgtgga tgtgtgtggt 6420
tgtatgtgtg atgtggtgtg tggagtgaaa atctgtggct ggcaaacgct cttgtatata 6480
tacgcacttt tgcccgtgct atgtggaaga ctaaacctcc gaagattgtg actcaggtag 6540
tgcggtatcg gctagggacc caaaccttgt cgatgccgat agcgctatcg aacgtacccc 6600
agccggccgg gagtatgtcg gaggggacat acgagatcgt caagggtttg tggccaactg 6660
gtatttaaat gtagctaacg gtagcaggcg aactactggt acatacctcc cccggaatat 6720
gtacaggcat aatgcgtatc tgtgggacat gtggtcgttg cgccattatg taagcagcgt 6780
gtactcctct gactgtccat atggtttgct ccatctcacc ctcatcgttt tcattgttca 6840
caggcggcca caaaaaaact gtcttctctc cttctctctt cgccttagtc tactcggacc 6900
agttttagtt tagcttggcg ccactggata aatgagacct caggccttgt gatgaggagg 6960
tcacttatga agcatgttag gaggtgcttg tatggataga gaagcaccca aaataataag 7020
aataataata aaacaggggg cgttgtcatt tcatatcgtg ttttcaccat caatacacct 7080
ccaaacaatg cccttcatgt ggccagcccc aatattgtcc tgtagttcaa ctctatgcag 7140
ctcgtatctt attgagcaag taaaactctg tcagccgata ttgcccgacc cgcgacaagg 7200
gtcaacaagg tggtgtaagg ccttcgcaga agtcaaaact gtgccaaaca aacatctaga 7260
gtctctttgg tgtttctcgc atatatttwa tcggctgtct tacgtatttg cgcctcggta 7320
ccggactaat ttcggatcat ccccaatacg ctttttcttc gcagctgtca acagtgtcca 7380
tgatctatcc acctaaatgg gtcatatgag gcgtataatt tcgtggtgct gataataatt 7440
cccatatatt tgacacaaaa cttccccccc tagacataca tctcacaatc tcacttcttg 7500
tgcttctgtc acacatctcc tccagctgac ttcaactcac acctctgccc cagttggtct 7560
acagcggtat aaggtttctc cgcatagagg tgcaccactc ctcccgatac ttgtttgtgt 7620
gacttgtggg tcacgacata tatatctaca cacattgcgc caccctttgg ttcttccagc 7680
acaacaaaaa cacgacacgc taaccatggc caatttactg accgtacacc aaaatttgcc 7740
tgcattaccg gtcgatgcaa cgagtgatga ggttcgcaag aacctgatgg acatgttcag 7800

ggatcgccag gcgttttctg agcatacctg gaaaatgctt ctgtccgttt gccggtcgtg 7860

ggcggcatgg tgcaagttga ataaccggaa atggtttccc gcagaacctg aagatgttcg 7920

cgattatctt ctatatcttc aggcgcgcgg tctggcagta aaaactatcc agcaacattt 7980

gggccagcta aacatgcttc atcgtcggtc cgggctgcca cgaccaagtg acagcaatgc 8040

tgtttcactg gttatgcggc ggatccgaaa agaaaacgtt gatgccggtg aacgtgcaaa 8100

acaggctcta gcgttcgaac gcactgattt cgaccaggtt cgttcactca tggaaaatag 8160

cgatcgctgc caggatatac gtaatctggc atttctgggg attgcttata acaccctgtt 8220

acgtatagcc gaaattgcca ggatcagggt taaagatatc tcacgtactg acggtgggag 8280

aatgttaatc catattggca gaacgaaaac gctggttagc accgcaggtg tagagaaggc 8340

acttagcctg ggggtaacta aactggtcga gcgatggatt tccgtctctg gtgtagctga 8400

tgatccgaat aactacctgt tttgccgggt cagaaaaaat ggtgttgccg cgccatctgc 8460

caccagccag ctatcaactc gcgccctgga agggattttt gaagcaactc atcgattgat 8520

ttacggcgct aaggatgact ctggtcagag atacctggcc tggtctggac acagtgcccg 8580

tgtcggagcc gcgcgagata tggcccgcgc tggagtttca ataccggaga tcatgcaagc 8640

tggtggctgg accaatgtaa atattgtcat gaactatatc cgtaacctgg atagtgaaac 8700

aggggcaatg gtgcgcctgc tggaagatgg cgattaagc 8739

<210> 128
<211> 13975
<212> DNA
<213> Artificial Sequence

<220>
<223> Plasmid pZKSL-5S5A5

<400> 128

cgatggttaa tgctgctgtg tgctgtgtgt gtgtgttgtt tggcgctcat tgttgcgtta 60
tgcagcgtac accacaatat tggaagctta ttagcctttc tattttttcg tttgcaaggc 120
ttaacaacat tgctgtggag agggatgggg atatggaggc cgctggaggg agtcggagag 180
gcgttttgga gcggcttggc ctggcgccca gctcgcgaaa cgcacctagg accctttggc 240
acgccgaaat gtgccacttt tcagtctagt aacgccttac ctacgtcatt ccatgcgtgc 300
atgtttgcgc ctttttccc ttgcccttga tcgccacaca gtacagtgca ctgtacagtg 360
gaggttttgg gggggtctta gatgggagct aaaagcggcc tagcggtaca ctagtgggat 420
tgtatggagt ggcatggagc ctaggtggag cctgacagga cgcacgaccg gctagcccgt 480
gacagacgat gggtggctcc tgttgtccac cgcgtacaaa tgtttgggcc aaagtcttgt 540
cagccttgct tgcgaaccta attcccaatt ttgtcacttc gcaccccat tgatcgagcc 600
ctaaccctg cccatcaggc aatccaatta agctcgcatt gtctgccttg tttagtttgg 660
ctcctgcccg tttcggcgtc cacttgcaca aacacaaaca agcattatat ataaggctcg 720

tctctccctc ccaaccacac tcactttttt gcccgtcttc ccttgctaac acaaaagtca 780
agaacacaaa caaccacccc aaccccctta cacacaagac atatctacag caatggccat 840
ggctctcagt cttaccacag aacagctgtt agaacgccct gatttggttg cgattgatgg 900
catcctctac gaccttgaag ggcttgccaa agttcatcca ggatccgatt tgattctcgc 960
ttctggtgcc tctgatgcct cccctctctt ttattcaatg catccatacg tcaaaccgga 1020
gaactccaaa ttgcttcaac agttcgtccg agggaagcat gaccgcacct cgaaggacat 1080
tgtctacacg tatgattctc ccttcgcaca agacgttaag cggacaatgc gcgaggtgat 1140
gaaagggagg aactggtacg caacccctgg cttctggctg cgcaccgttg ggatcatcgc 1200
cgtgacggcc ttttgcgagt ggcactgggc taccacgggg atggtgctgt ggggcctgtt 1260
gactggattc atgcacatgc agatcggctt atccatccag catgatgcgt cccacggggc 1320
catcagcaag aagccttggg tcaacgccct cttcgcctac ggcattgacg tcatcggatc 1380
gtcccggtgg atttggctgc agtcgcacat catgcggcac cacacctaca ccaaccagca 1440
cggcctcgac ctggatgcgg agtcggcaga gccgttcctg gtgttccaca actacccgc 1500
cgcaaacacc gcccgaaagt ggttccaccg cttccaggct tggtacatgt accttgtgct 1560
gggggcatac ggggtatcgc tggtgtacaa cccgctctac attttccgga tgcagcacaa 1620
tgacaccatc ccagagtctg tcacggccat gcgggaaaat ggctttctgc ggcgctaccg 1680
cacacttgca ttcgtgatgc gagctttctt catcttccgg accgcattct tgccctggta 1740
cctcactggg acctcattgc tgatcaccat tcctctggtg cccaccgcaa ctggtgcctt 1800
cttgacgttc ttcttcattt tgtcccacaa ttttgatggc tccgaacgga tccccgacaa 1860
gaactgcaag gttaagcgat ctgagaagga cgttgaggct gaccaaattg actggtatcg 1920
ggcgcaggtg gagacgtcct ccacatacgg tggccccatc gccatgttct tcactggcgg 1980
tctcaatttc cagatcgagc accacctctt tccccggatg tcgtcttggc actacccctt 2040
cgtccagcag gcggtccggg agtgttgcga acgacatgga gtgcgatatg ttttctaccc 2100
taccatcgtc ggcaacatca tctccaccct gaagtacatg cataaggtgg gtgtcgtcca 2160
ctgcgtgaag gacgcacagg attcctaagc ggccgcattg atgattggaa acacacacat 2220
gggttatatc taggtgagag ttagttggac agttatatat taaatcagct atgccaacgg 2280
taacttcatt catgtcaacg aggaaccagt gactgcaagt aatatagaat ttgaccacct 2340
tgccattctc ttgcactcct ttactatatc tcatttattt cttatataca aatcacttct 2400
tcttcccagc atcgagctcg gaaacctcat gagcaataac atcgtggatc tcgtcaatag 2460
agggcttttt ggactccttg ctgttggcca ccttgtcctt gctgtctggc tcattctgtt 2520
tcaacgcctt ttaattaact ctcctgcagc ttcctcagcg agacactgct cctgtctgga 2580
cccgagctaa ggctctgttc gacaaacacg ttctgcgaat tggcgagtaa tttcattaat 2640

gcaaatagac gtgtatttga aaaggaggag atgatggtac cagtaattta cggtgtttga 2700
atagacaaat tatatatata aaattaacct gatgaatgag tgtatgaatg gattgcttaa 2760
tataccgagg gagagccggc attatcaata tttattgtcc taagaagcta aaatatgctg 2820
ttccgttgat agcgatgtct gtttgagagt caatggcaga acgcggagga gtattgttag 2880
ttggtgatcg gtttactcga catcgagtag gggcgagac agaagatatc ccgaatccat 2940
tgcattgttt attaggatgt tcacaacaca actccctcta gactctgggg atgtgcgtta 3000
gaagaatgac ctggagcaag agtgttcaga ttgatccgtt caaattttca agattactgt 3060
tggggttgtt tttgaatcca cattagctgg acgactattg catctgagcg ctcggaacgt 3120
ctccctttcc cttcagcttt atacgagtcg attccataag cgcgacctct cgaattgcct 3180
tcggttgtga agccataggc aaaggtgtgg ctatggaatg catgcgacgt cgggcccaat 3240
tcgccctata gtgagtcgta ttacaattca ctggccgtcg ttttacaacg tcgtgactgg 3300
gaaaaccctg gcgttaccca acttaatcgc cttgcagcac atcccccttt cgccagctgg 3360
cgtaatagcg aagaggcccg caccgatcgc ccttcccaac agttgcgcag cctgaatggc 3420
gaatggacgc gccctgtagc ggcgcattaa gcgcggcggg tgtggtggtt acgcgcagcg 3480
tgaccgctac acttgccagc gccctagcgc ccgctccttt cgctttcttc ccttcctttc 3540
tcgccacgtt cgccggcttt ccccgtcaag ctctaaatcg ggggctccct ttagggttcc 3600
gatttagtgc tttacggcac ctcgacccca aaaaacttga ttagggtgat ggttcacgta 3660
gtgggccatc gccctgatag acggtttttc gccctttgac gttggagtcc acgttcttta 3720
atagtggact cttgttccaa actggaacaa cactcaaccc tatctcggtc tattcttttg 3780
atttataagg gattttgccg atttcggcct attggttaaa aaatgagctg atttaacaaa 3840
aatttaacgc gaattttaac aaaatattaa cgcttacaat ttcctgatgc ggtattttct 3900
ccttacgcat ctgtgcggta tttcacaccg catcaggtgg cacttttcgg ggaaatgtgc 3960
gcggaacccc tatttgttta tttttctaaa tacattcaaa tatgtatccg ctcatgagac 4020
aataaccctg ataaatgctt caataatatt gaaaaaggaa gagtatgagt attcaacatt 4080
tccgtgtcgc ccttattccc ttttttgcgg cattttgcct tcctgttttt gctcacccag 4140
aaacgctggt gaaagtaaaa gatgctgaag atcagttggg tgcacgagtg ggttacatcg 4200
aactggatct caacagcggt aagatccttg agagttttcg ccccgaagaa cgttttccaa 4260
tgatgagcac ttttaaagtt ctgctatgtg gcgcggtatt atcccgtatt gacgccgggc 4320
aagagcaact cggtcgccgc atacactatt ctcagaatga cttggttgag tactcaccag 4380
tcacagaaaa gcatcttacg gatggcatga cagtaagaga attatgcagt gctgccataa 4440
ccatgagtga taacactgcg gccaacttac ttctgacaac gatcggagga ccgaaggagc 4500
taaccgcttt tttgcacaac atgggggatc atgtaactcg ccttgatcgt tgggaaccgg 4560
agctgaatga agccatacca aacgacgagc gtgacaccac gatgcctgta gcaatggcaa 4620

caacgttgcg caaactatta actggcgaac tacttactct agcttcccgg caacaattaa 4680
tagactggat ggaggcggat aaagttgcag gaccacttct gcgctcggcc cttccggctg 4740
gctggtttat tgctgataaa tctggagccg gtgagcgtgg gtctcgcggt atcattgcag 4800
cactggggcc agatggtaag ccctcccgta tcgtagttat ctacacgacg gggagtcagg 4860
caactatgga tgaacgaaat agacagatcg ctgagatagg tgcctcactg attaagcatt 4920
ggtaactgtc agaccaagtt tactcatata tactttagat tgatttaaaa cttcattttt 4980
aatttaaaag gatctaggtg aagatccttt ttgataatct catgaccaaa atcccttaac 5040
gtgagttttc gttccactga gcgtcagacc ccgtagaaaa gatcaaagga tcttcttgag 5100
atcctttttt tctgcgcgta atctgctgct tgcaaacaaa aaaaccaccg ctaccagcgg 5160
tggtttgttt gccggatcaa gagctaccaa ctctttttcc gaaggtaact ggcttcagca 5220
gagcgcagat accaaatact gttcttctag tgtagccgta gttaggccac cacttcaaga 5280
actctgtagc accgcctaca tacctcgctc tgctaatcct gttaccagtg gctgctgcca 5340
gtggcgataa gtcgtgtctt accgggttgg actcaagacg atagttaccg gataaggcgc 5400
agcggtcggg ctgaacgggg ggttcgtgca cacagcccag cttggagcga acgacctaca 5460
ccgaactgag atacctacag cgtgagctat gagaaagcgc cacgcttccc gaagggagaa 5520
aggcggacag gtatccggta agcggcaggg tcggaacagg agagcgcacg agggagcttc 5580
caggggggaaa cgcctggtat ctttatagtc ctgtcgggtt tcgccacctc tgacttgagc 5640
gtcgattttt gtgatgctcg tcaggggggc ggagcctatg gaaaaacgcc agcaacgcgg 5700
cctttttacg gttcctggcc ttttgctggc cttttgctca catgttcttt cctgcgttat 5760
cccctgattc tgtggataac cgtattaccg cctttgagtg agctgatacc gctcgccgca 5820
gccgaacgac cgagcgcagc gagtcagtga gcgaggaagc ggaagagcgc ccaatacgca 5880
aaccgcctct ccccgcgcgt tggccgattc attaatgcag ctggcgcgcc gaggtttcca 5940
acgagataac atcgtggcag ctgccaccat gaccgatatg cctgaaataa gtgaattgac 6000
catcagaagt tctgcattca aatagaacta tatcatattc ggctcagttt tttcaataat 6060
agtccaatcc ctaagtctcc tatccaaaat ggttcctgac cagccacatc catgatcatg 6120
actgcgtgac aggaacagtc attccgtgga tgaacgactt tacgctcagg tactgtaaat 6180
atctgtaaag ggcagacaac caaccaattg agtaacctgt gagacttgaa acgtaagatg 6240
acttcacaca caaagtcact tgactcaacc gtggctctca attgcacaaa atcactctgc 6300
actaatctat tgcaggagtc aggctatgaa caactagacg acagctactt atgtgttata 6360
tagaggaata ttaaaaaatc taagaataat cataaagtta caaaataatt atcagatttc 6420
gagccacagg tcacccctaa catgtgttat tgcacaccca caatcctcag cttgatgtca 6480
tttaattctt ccagccacca tctctctctc caaccctaat ggcaaacttt attttggtgg 6540

agcgatgact cttactcaac tgcagcatac ttaagcacaa ttgttcccca gcctgatacg 6600
acacaccatc cattgtcaag cttcaccaca tacaacaaca cagcgtacgc aactaacatg 6660
aatgaatacg atatacatca aagactatga tacgcagtat tgcacactgt acgagtaaga 6720
gcactagcca ctgcactcaa gtgaaaccgt tgcccgggta cgagtatgag tatgtacagt 6780
atgtttagta ttgtacttgg acagtgcttg tatcgtacat tctcaagtgt caaacataaa 6840
tatccgttgc tatatcctcg caccaccacg tagctcgcta tatccctgtg ttgaatccat 6900
ccatcttgga ttgccaattg tgcacacaga accgggcact cacttcccca tccacacttg 6960
cggccgctta ggaatcctga gcgtccttga cacagtgaac cacaccgact ttgtgcatgt 7020
acttgagggt ggaaatgatg ttgcccacaa tggtagggta gaagacgtac cgaactccgt 7080
gtcgttcgca acactctcgg acagcttgct gcacgaaggg atagtgccaa gacgacattc 7140
gaggaaagag gtgatgctcg atctggaagt tgagaccgcc agtaaagaac atggcaatgg 7200
gtccaccgta ggtggaagag gtctccacct gagctctgta ccagtcgatc tgatcggctt 7260
caacgtcctt ctcggagctc ttgaccttgc agttcttgtc ggggattcgc tccgagccat 7320
cgaagttgtg agacaagatg aaaaagaagg tgaggaaggc accggtagca gtgggcacca 7380
gaggaatggt gatgagcagg gaggttccag tgagatacca gggcaagaag gcggttcgaa 7440
agatgaagaa agctcgcata acgaatgcaa gggttcggta ccgtcgcaga aagccgttct 7500
ctcgcatggc tgtgacagac tcgggaatgg tgtcgttgtg ctgcattcgg aagatgtaga 7560
gagggttgta caccagcgaa acgccgtagg ctccaagcac gaggtacatg taccaggcct 7620
ggaatcggtg aaaccacttt cgagcagtgt tggcagcagg gtagttgtgg aacacaagga 7680
atggttctgc ggactcggca tccaggtcga gaccatgctg attggtgtag gtgtgatgtc 7740
gcatgatgtg agactgcagc cagatccatc tggacgatcc aatgacgtcg atgccgtagg 7800
caaagagagc gttgacccag ggctttttgc tgatggcacc atgagaggca tcgtgctgaa 7860
tggacaggcc gatctgcatg tgcatgaatc cagtcaagag accccacagc accattccgg 7920
tagtagccca gtgccactcg caaaaggcgg tgacagcaat gatgccaacg gttcgcagcc 7980
agaatccagg tgtggcatac cagttccgac ctttcatgac ctctcgcata gttcgcttga 8040
cgtcctgtgc aaagggagag tcgtaggtgt agacaatgtc cttggaggtt cggtcgtgct 8100
tgcctcgcac gaactgttga agcagcttcg agttctcggg cttgacgtaa gggtgcatgg 8160
agtagaacag aggagaagca tcggaggcac cagaagcgag gatcaagtcg gatccgggat 8220
ggaccttggc aagaccttcc agatcgtaga gaatgccgtc gatggcaacc aggtcgggtc 8280
gctcgagcag ctgctcggta gtaagggaga gagccatgga gagctgggtt agtttgtgta 8340
gagagtgtgt gttgctagcg actttcggat tgtgtcatta cacaaaacgc gtcgtctcga 8400
cactgatctt gtcgtggata ctcacggctc ggacatcgtc gccgacgatg acaccggact 8460
ttcgcttaag gacgtcagta acaggcattg tgtgatgtgt agtttagatt tcgaatctgt 8520

ggggaaagaa aggaaaaaag agactggcaa ccgattggga gagccactgt ttatatatac 8580
cctagacaag cccccgcttt gtaagatgtt ggtcaatgta aaccagtatt aaggttggca 8640
agtgcaggag aagcaaggtg tgggtaccga gcaatggaaa tgtgcggaag gcaaaaaaat 8700
gaggccacgg cctattgtcg gggctatatc caggggggcga ttgaagtaca ctaacatgac 8760
atgtgtccac agaccctcaa tctggcctga tgagccaaat ccatacgcgc tttcgcagct 8820
ctaaaggcta taacaagtca caccaccctg ctcgacctca gcgccctcac tttttgttaa 8880
gacaaactgt acacgctgtt ccagcgtttt ctgcctgcac ctggtgggac atttggtgca 8940
acctaaagtg ctcggaacct ctgtggtgtc cagatcagcg cagcagttcc gaggtagttt 9000
tgaggccctt agatgatgca atggtgtcag tcgctggatc acgagtctta atggcagtat 9060
tcgttcttat ttgtgccatt gagccccgtt atcctcgtat cttctacccc ccatcccatc 9120
cctttgttgg tgcaacccta cccatttatt gttgggtgca gcccaaccga cgtggagagc 9180
ttggcttggc catataaaaa ggcccccccc tagtggcaat ggcagaaagt cagctgtgag 9240
ttgttgaatt tgtcatctag gcggcctggc cgtcttctcc ggggcaattg gggctgtttt 9300
ttgggacaca aatacgccgc caacccggtc tctcctgaat tctgcagatg ggctgcagga 9360
attccgtcgt cgcctgagtc gactccaact tttcacactg agcgtaaaat gtggagaaga 9420
aatcggcact aaaaagtcag gtagactgga aaatgcgcca tgaaatgaat atctcttgct 9480
acagtaatgc ccagcatcga ggggtattgt gtcaccaaca ctatagtggc agctgaagcg 9540
ctcgtgattg tagtatgagt ctttattggt gatgggaaga gttcactcaa tattctcgtt 9600
actgccaaaa caccacggta atcggccaga caccatggat gtagatcacc aagcctgtga 9660
atgttattcg agctaaaatg cacatggttg gtgaaaggag tagttgctgt cgaattccgt 9720
cgtcgcctga gtcatcattt atttaccagt tggccacaaa cccttgacga tctcgtatgt 9780
cccctccgac atactcccgg ccggctgggg tacgttcgat agcgctatcg gcatcgacaa 9840
ggtttgggtc cctagccgat accgcactac ctgagtcaca atcttcggag gtttagtctt 9900
ccacatagca cgggcaaaag tgcgtatata tacaagagcg tttgccagcc acagattttc 9960
actccacaca ccacatcaca catacaacca cacacatcca caatggaacc cgaaactaag 10020
aagaccaaga ctgactccaa gaagattgtt cttctcggcg gcgacttctg tggccccgag 10080
gtgattgccg aggccgtcaa ggtgctcaag tctgttgctg aggcctccgg caccgagttt 10140
gtgtttgagg accgactcat tggaggagct gccattgaga aggagggcga gcccatcacc 10200
gacgctactc tcgacatctg ccgaaaggct gactctatta tgctcggtgc tgtcggaggc 10260
gctgccaaca ccgtatggac cactcccgac ggacgaaccg acgtgcgacc cgagcagggt 10320
ctcctcaagc tgcgaaagga cctgaacctg tacgccaacc tgcgaccctg ccagctgctg 10380
tcgcccaagc tcgccgatct ctcccccatc cgaaacgttg agggcaccga cttcatcatt 10440

gtccgagagc tcgtcggagg tatctacttt ggagagcgaa aggaggatga cggatctggc 10500
gtcgcttccg acaccgagac ctactccgtt cctgaggttg agcgaattgc ccgaatggcc 10560
gccttcctgg cccttcagca caaccccccct cttcccgtgt ggtctcttga caaggccaac 10620
gtgctggcct cctctcgact ttggcgaaag actgtcactc gagtcctcaa ggacgaattc 10680
ccccagctcg agctcaacca ccagctgatc gactcggccg ccatgatcct catcaagcag 10740
ccctccaaga tgaatggtat catcatcacc accaacatgt ttggcgatat catctccgac 10800
gaggcctccg tcatccccgg ttctctgggt ctgctgccct ccgcctctct ggcttctctg 10860
cccgacacca acgaggcgtt cggtctgtac gagccctgtc acggatctgc cccccgatctc 10920
ggcaagcaga aggtcaaccc cattgccacc attctgtctg ccgccatgat gctcaagttc 10980
tctcttaaca tgaagcccgc cggtgacgct gttgaggctg ccgtcaagga gtccgtcgag 11040
gctggtatca ctaccgccga tatcggaggc tcttcctcca cctccgaggt cggagacttg 11100
ttgccaacaa ggtcaaggag ctgctcaaga aggagtaagt cgtttctacg acgcattgat 11160
ggaaggagca aactgacgcg cctgcgggtt ggtctaccgg cagggtccgc tagtgtataa 11220
gactctataa aaagggccct gccctgctaa tgaaatgatg atttataatt taccggtgta 11280
gcaaccttga ctagaagaag cagattgggt gtgtttgtag tggaggacag tggtacgttt 11340
tggaaacagt cttcttgaaa gtgtcttgtc tacagtatat tcactcataa cctcaatagc 11400
caagggtgta gtcggtttat taaaggaagg gagttgtggc tgatgtggat agatatcttt 11460
aagctggcga ctgcacccaa cgagtgtggt ggtagcttgt ttaaacagag tgtgaaagac 11520
tcactatggt ccgggcttat ctcgaccaat agccaaagtc tggagtttct gagagaaaaa 11580
ggcaagatac gtatgtaaca aagcgacgca tggtacaata ataccggagg catgtatcat 11640
agagagttag tggttcgatg atggcactgg tgcctggtat gactttatac ggctgactac 11700
atatttgtcc tcagacatac aattacagtc aagcacttac ccttggacat ctgtaggtac 11760
cccccggcca agacgatctc agcgtgtcgt atgtcggatt ggcgtagctc cctcgctcgt 11820
caattggctc ccatctactt tcttctgctt ggctacaccc agcatgtctg ctatggctcg 11880
ttttcgtgcc ttatctatcc tcccagtatt accaactcta aatgacatga tgtgattggg 11940
tctacacttt catatcagag ataaggagta gcacagttgc ataaaaagcc caactctaat 12000
cagcttcttc ctttcttgta attagtacaa aggtgattag cgaaatctgg aagcttagtt 12060
ggccctaaaa aaatcaaaaa aagcaaaaaa cgaaaaacga aaaaccacag ttttgagaac 12120
agggaggtaa cgaaggatcg tatatatata tatatatata tatacccacg gatcccgaga 12180
ccggcctttg attcttccct acaaccaacc attctcacca ccctaattca caaccatggc 12240
caccatctcc ctgactaccg agcagctcct ggaacacccc gagctcgttg ccatcgacgg 12300
agtcctgtac gatctcttcg gtctggccaa ggtccatgcc ggaggcaacc tcatcgaagc 12360
tgccggtgca tccgacggaa ccgctctgtt ctactccatg catcctggag tcaagccaga 12420

gaactcgaag cttctgcagc aatttgcccg aggcaagcac gaacgaagct ccaaggatcc 12480
cgtgtacacc ttcgactctc cctttgctca ggacgtcaag cagtccgttc gagaggtcat 12540
gaagggtcga aactggtacg ccactcctgg cttctggctg agaaccgcac tcatcatcgc 12600
ttgtactgcc attggcgagt ggtactggat cacaaccgga gcagtgatgt ggggtatctt 12660
tactggatac ttccactcgc agattggctt ggccattcaa cacgatgctt ctcacggagc 12720
catcagcaaa aagccctggg tcaacgcctt tttcgcttat ggcatcgacg ccattggttc 12780
ctctcgttgg atctggctgc agtcccacat tatgcgacat cacacttaca ccaaccagca 12840
tggcctcgac ctggatgctg cctcggcaga gccgttcatc ttgttccact cctatcctgc 12900
taccaacgcc tctcgaaagt ggtaccaccg atttcaggcg tggtacatgt acatcgttct 12960
gggaatgtat ggtgtctcga tggtgtacaa tcccatgtac ctcttcacaa tgcagcacaa 13020
cgacaccatt cccgaggcca cttctctcag accaggcagc tttttcaatc ggcagcgagc 13080
tttcgccgtt tcccttcgac tgctcttcat cttccgaaac gcctttcttc cctggtacat 13140
tgctggtgcc tctcctctgc tcaccattct tctggtgccc acggtcacag gcatcttcct 13200
cacctttgtg ttcgttctgt cccataactt cgagggagcc gaacggaccc cagagaagaa 13260
ctgcaaggcc aaacgagcta aggaaggcaa ggaggtcaga gacgtggaag aggatcgagt 13320
cgactggtac cgagcacagg ccgagactgc tgccacctac ggtggcagcg tgggaatgat 13380
gcttacaggc ggtctcaacc tgcagatcga gcatcacttg tttccccgaa tgtcctcttg 13440
gcactatccc ttcattcaag acaccgttcg ggagtgttgc aagcgacatg gcgtccgtta 13500
cacatactat cctaccattc tcgagaacat catgtccact cttcgataca tgcagaaggt 13560
gggtgttgct cacaccattc aggatgccca ggagttctaa gcggccgcat gtacatacaa 13620
gattatttat agaaatgaat cgcgatcgaa caaagagtac gagtgtacga gtaggggatg 13680
atgataaaag tggaagaagt tccgcatctt tggatttatc aacgtgtagg acgatacttc 13740
ctgtaaaaat gcaatgtctt taccataggt tctgctgtag atgttattaa ctaccattaa 13800
catgtctact tgtacagttg cagaccagtt ggagtataga atggtacact taccaaaaag 13860
tgttgatggt tgtaactacg atatataaaa ctgttgacgg gatccccgct gatatgccta 13920
aggaacaatc aaagaggaag atattaattc agaatgctag tatacagtta gggat 13975

<210> 129
<211> 13066
<212> DNA
<213> Artificial Sequence

<220>
<223> Plasmid pZP3-Pa777U

<400> 129

tctcggtcta ttcttttgat ttataaggga ttttgccgat ttcggcctat tggttaaaaa 60

```
atgagctgat ttaacaaaaa tttaacgcga attttaacaa aatattaacg cttacaattt      120
cctgatgcgg tattttctcc ttacgcatct gtgcggtatt tcacaccgca tcaggtggca      180
cttttcgggg aaatgtgcgc ggaaccccta tttgtttatt tttctaaata cattcaaata      240
tgtatccgct catgagacaa taaccctgat aaatgcttca ataatattga aaaaggaaga      300
gtatgagtat tcaacatttc cgtgtcgccc ttattccctt ttttgcggca ttttgccttc      360
ctgtttttgc tcacccagaa acgctggtga aagtaaaaga tgctgaagat cagttgggtg      420
cacgagtggg ttacatcgaa ctggatctca acagcggtaa gatccttgag agttttcgcc      480
ccgaagaacg ttttccaatg atgagcactt ttaaagttct gctatgtggc gcggtattat      540
cccgtattga cgccgggcaa gagcaactcg gtcgccgcat acactattct cagaatgact      600
tggttgagta ctcaccagtc acagaaaagc atcttacgga tggcatgaca gtaagagaat      660
tatgcagtgc tgccataacc atgagtgata acactgcggc caacttactt ctgacaacga      720
tcggaggacc gaaggagcta accgcttttt tgcacaacat gggggatcat gtaactcgcc      780
ttgatcgttg ggaaccggag ctgaatgaag ccataccaaa cgacgagcgt gacaccacga      840
tgcctgtagc aatggcaaca acgttgcgca aactattaac tggcgaacta cttactctag      900
cttcccggca acaattaata gactggatgg aggcggataa agttgcagga ccacttctgc      960
gctcggccct tccggctggc tggtttattg ctgataaatc tggagccggt gagcgtgggt     1020
ctcgcggtat cattgcagca ctggggccag atggtaagcc ctcccgtatc gtagttatct     1080
acacgacggg gagtcaggca actatggatg aacgaaatag acagatcgct gagataggtg     1140
cctcactgat taagcattgg taactgtcag accaagttta ctcatatata ctttagattg     1200
atttaaaact tcatttttaa tttaaaagga tctaggtgaa gatccttttt gataatctca     1260
tgaccaaaat cccttaacgt gagttttcgt tccactgagc gtcagacccc gtagaaaaga     1320
tcaaaggatc ttcttgagat cctttttttc tgcgcgtaat ctgctgcttg caaacaaaaa     1380
aaccaccgct accagcggtg gtttgtttgc cggatcaaga gctaccaact ctttttccga     1440
aggtaactgg cttcagcaga gcgcagatac caaatactgt tcttctagtg tagccgtagt     1500
taggccacca cttcaagaac tctgtagcac cgcctacata cctcgctctg ctaatcctgt     1560
taccagtggc tgctgccagt ggcgataagt cgtgtcttac cgggttggac tcaagacgat     1620
agttaccgga taaggcgcag cggtcgggct gaacgggggg ttcgtgcaca cagcccagct     1680
tggagcgaac gacctacacc gaactgagat acctacagcg tgagctatga gaaagcgcca     1740
cgcttcccga agggagaaag gcggacaggt atccggtaag cggcagggtc ggaacaggag     1800
agcgcacgag ggagcttcca gggggaaacg cctggtatct ttatagtcct gtcgggtttc     1860
gccacctctg acttgagcgt cgatttttgt gatgctcgtc aggggggcgg agcctatgga     1920
aaaacgccag caacgcggcc tttttacggt tcctggcctt ttgctggcct tttgctcaca     1980
tgttctttcc tgcgttatcc cctgattctg tggataaccg tattaccgcc tttgagtgag     2040
```

ctgataccgc tcgccgcagc cgaacgaccg agcgcagcga gtcagtgagc gaggaagcgg 2100
aagagcgccc aatacgcaaa ccgcctctcc ccgcgcgttg gccgattcat taatgcagct 2160
ggcgcgccac caatcacaat tctgaaaagc acatcttgat ctcctcattg cggggagtcc 2220
aacggtggtc ttattccccc gaatttcccg ctcaatctcg ttccagaccg acccggacac 2280
agtgcttaac gccgttccga aactctaccg cagatatgct ccaacggact gggctgcata 2340
gatgtgatcc tcggcttgga gaaatggata aaagccggcc aaaaaaaaag cggaaaaaag 2400
cggaaaaaaa gagaaaaaaa atcgcaaaat ttgaaaaata gggggaaaag acgcaaaaac 2460
gcaaggaggg gggagtatat gacactgata agcaagctca caacggttcc tcttattttt 2520
ttcctcatct tctgcctagg ttcccaaaat cccagatgct tctctccagt gccaaaagta 2580
agtaccccac aggttttcgg ccgaaaattc cacgtgcagc aacgtcgtgt ggggtgttaa 2640
aatgtggggg gggggaacca ggacaagagg ctcttgtggg agccgaatga gagcacaaag 2700
cgggcgggtg tgataagggc atttttgccc attttccctt ctcctgtctc tccgacggtg 2760
atggcgttgt gcgtcctcta tttcttttta tttcttttttg ttttatttct ctgactaccg 2820
atttggtttg atttcctcaa ccccacacaa ataagctcgg gccgaggaat atatatatac 2880
acggacacag tcgccctgtg gacaacacgt cactacctct acgatacaca ccgtacgttg 2940
tgtggaagct tgtgagcgga taacaatttc acacaggaaa cagctatgac catgattacg 3000
ccaagctcga aattaaccct cactaaaggg aacaaaagct ggagctccac cgcggacaca 3060
atatctggtc aaatttcagt ttcgttacat ttaaattcct tcacttcaag ttcattcttc 3120
atctgcttct gttttacttt gacaggcaaa tgaagacatg gtacgacttg atggaggcca 3180
agaacgccat ttcaccccga gacaccgaag tgcctgaaat cctggctgcc cccattgata 3240
acatcggaaa ctacggtatt ccggaaagtg tatatagaac ctttccccag cttgtgtctg 3300
tggatatgga tggtgtaatc ccctttgagt actcgtcttg gcttctctcc gagcagtatg 3360
aggctctcta atctagcgca tttaatatct caatgtattt atatatttat cttctcatgc 3420
ggccgcttag ttggctttgg tcttggcagc cttggcctcc ttgagggtaa acatcttggc 3480
atccttgtcg accacgccgt acttggcgta cataagacca attcggatga aggtgggaat 3540
gatgggagaa gccgactttc gcaccagttc gggaaaggcc tgagcgaagg cagcagtggc 3600
ctcgttgagc ttgtagtgag gaatgatggg aaacagatgg tggatctgat gtgtaccaat 3660
gttgtgggac aggttgtcga tgagggctcc gtagcttcgg tccacagagg acaagttgcc 3720
cttgacatag gtccactccg aatcggcgta ccagggagtt tcctcgtcgt tgtgatggag 3780
gaaggtagtg acaaccagca tggtggcgaa tccaaagaga ggtgcgaagt aatacagagc 3840
catggtcttg aggccgtaga cgtaggtaag gtaggcgtac agaccagcaa aggccacgag 3900
agagccgagg gaaatgatga cggcagacat tcttcgcagg tagagaggct cccagggatt 3960

gaagtggttg acctttcggg gaggaaatcc agcaacgagg taggcaaacc aagccgaacc 4020
aagggagatg accatgtgtc gggacagggg atgagagtcg gcttctcgct gagggtagaa 4080
gatctcatcc ttgtcgatgt tgccggtgtt cttgtgatgg tgtcgatggc tgatcttcca 4140
cgactcgtag ggagtcagaa tgatggagtg aatgagtgtg ccaacagaga agttgagcag 4200
gtgggatcgc gagaaggcac catgtccaca gtcgtgaccg atggtaaaga atccccagaa 4260
cacgataccc tggagcagaa tgtagccagt gcaaaggacg gcatcgagca gtgcaaactc 4320
ctgcacgata gcaagggctc gagcatagta cagtccgaga gcaagggaac cggcaatgcc 4380
cagagctcgc acggtatagt agagggacca gggaacagag gcttcgaagc agtgggcagg 4440
cagggatcgc ttgatctcgg tgagagtagg gaactcgtag ggagcggcaa cggtagagga 4500
agccatggtt gtgaattagg gtggtgagaa tggttggttg tagggaagaa tcaaaggccg 4560
gtctcgggat ccgtgggtat atatatatat atatatatat acgatccttc gttacctccc 4620
tgttctcaaa actgtggttt ttcgttttc gtttttgct tttttgatt tttttagggc 4680
caactaagct tccagatttc gctaatcacc tttgtactaa ttacaagaaa ggaagaagct 4740
gattagagtt gggcttttta tgcaactgtg ctactcctta tctctgatat gaaagtgtag 4800
acccaatcac atcatgtcat ttagagttgg taatactggg aggatagata aggcacgaaa 4860
acgagccata gcagacatgc tgggtgtagc caagcagaag aaagtagatg ggagccaatt 4920
gacgagcgag ggagctacgc caatccgaca tacgacacgc tgagatcgtc ttggccgggg 4980
ggtacctaca gatgtccaag ggtaagtgct tgactgtaat tgtatgtctg aggacaaata 5040
tgtagtcagc cgtataaagt cataccaggc accagtgcca tcatcgaacc actaactctc 5100
tatgatacat gcctccggta ttattgtacc atgcgtcgct ttgttacata cgtatcttgc 5160
ctttttctct cagaaactcc agactttggc tattggtcga gataagcccg gaccatagtg 5220
agtctttcac actctacatt tctcccttgc tccaactatc gattgttgtc tactaactat 5280
cgtacgataa cttcgtatag catacattat acgaagttat cgcgtcgacg agtatctgtc 5340
tgactcgtca ttgccgcctt tggagtacga ctccaactat gagtgtgctt ggatcacttt 5400
gacgatacat tcttcgttgg aggctgtggg tctgacagct gcgttttcgg cgcggttggc 5460
cgacaacaat atcagctgca acgtcattgc tggctttcat catgatcaca tttttgtcgg 5520
caaaggcgac gcccagagag ccattgacgt tctttctaat ttggaccgat agccgtatag 5580
tccagtctat ctataagttc aactaactcg taactattac cataacatat acttcactgc 5640
cccagataag gttccgataa aaagttctgc agactaaatt tatttcagtc tcctcttcac 5700
caccaaaatg ccctcctacg aagctcgagc taacgtccac aagtccgcct ttgccgctcg 5760
agtgctcaag ctcgtggcag ccaagaaaac caacctgtgt gcttctctgg atgttaccac 5820
caccaaggag ctcattgagc ttgccgataa ggtcggacct tatgtgtgca tgatcaaaac 5880
ccatatcgac atcattgacg acttcaccta cgccggcact gtgctccccc tcaaggaact 5940

tgctcttaag cacggtttct tcctgttcga ggacagaaag ttcgcagata ttggcaacac 6000
tgtcaagcac cagtaccggt gtcaccgaat cgccgagtgg tccgatatca ccaacgccca 6060
cggtgtaccc ggaaccggaa tcattgctgg cctgcgagct ggtgccgagg aaactgtctc 6120
tgaacagaag aaggaggacg tctctgacta cgagaactcc cagtacaagg agttcctagt 6180
cccctctccc aacgagaagc tggccagagg tctgctcatg ctggccgagc tgtcttgcaa 6240
gggctctctg gccactggcg agtactccaa gcagaccatt gagcttgccc gatccgaccc 6300
cgagtttgtg gttggcttca ttgcccagaa ccgacctaag ggcgactctg aggactggct 6360
tattctgacc cccggggtgg gtcttgacga caagggagac gctctcggac agcagtaccg 6420
aactgttgag gatgtcatgt ctaccggaac ggatatcata attgtcggcc gaggtctgta 6480
cggccagaac cgagatccta ttgaggaggc caagcgatac cagaaggctg gctgggaggc 6540
ttaccagaag attaactgtt agaggttaga ctatggatat gtaatttaac tgtgtatata 6600
gagagcgtgc aagtatggag cgcttgttca gcttgtatga tggtcagacg acctgtctga 6660
tcgagtatgt atgatactgc acaacctgtg tatccgcatg atctgtccaa tggggcatgt 6720
tgttgtgttt ctcgatacgg agatgctggg tacagtgcta atacgttgaa ctacttatac 6780
ttatatgagg ctcgaagaaa gctgacttgt gtatgactta ttctcaacta catccccagt 6840
cacaatacca ccactgcact accactacac caaaaccatg atcaaaccac ccatggactt 6900
cctggaggca gaagaacttg ttatggaaaa gctcaagaga gagatcataa cttcgtatag 6960
catacattat acgaagttat cctgcaggta aaggaattca tgctgttcat cgtggttaat 7020
gctgctgtgt gctgtgtgtg tgtgttgttt ggcgctcatt gttgcgttat gcagcgtaca 7080
ccacaatatt ggaagcttat tagcctttct atttttttcgt ttgcaaggct taacaacatt 7140
gctgtggaga gggatgggga tatggaggcc gctggaggga gtcggagagg cgttttggag 7200
cggcttggcc tggcgcccag ctcgcgaaac gcacctagga ccctttggca cgccgaaatg 7260
tgccactttt cagtctagta acgccttacc tacgtcattc catgcgtgca tgtttgcgcc 7320
tttttttccct tgcccttgat cgccacacag tacagtgcac tgtacagtgg aggttttggg 7380
ggggtcttag atgggagcta aaagcggcct agcggtacac tagtgggatt gtatggagtg 7440
gcatggagcc taggtggagc ctgacaggac gcacgaccgg ctagcccgtg acagacgatg 7500
ggtggctcct gttgtccacc gcgtacaaat gtttgggcca aagtcttgtc agccttgctt 7560
gcgaacctaa ttcccaattt tgtcacttcg cacccccatt gatcgagccc taacccctgc 7620
ccatcaggca atccaattaa gctcgcattg tctgccttgt ttagtttggc tcctgcccgt 7680
ttcggcgtcc acttgcacaa acacaaacaa gcattatata taaggctcgt ctctccctcc 7740
caaccacact cactttttttg cccgtcttcc cttgctaaca caaaagtcaa gaacacaaac 7800
aaccacccca accccctac acacaagaca tatctacagc aatggccatg gcttcttcca 7860

ctgttgctgc gccgtacgag ttcccgacgc tgacggagat caagcgctcg ctgccagcgc 7920
actgctttga ggcctcggtc ccgtggtcgc tctactacac cgtgcgcgcg ctgggcatcg 7980
ccggctcgct cgcgctcggc ctctactacg cgcgcgcgct cgcgatcgtg caggagtttg 8040
ccctgctgga tgcggtgctc tgcacggggt acattctgct gcagggcatc gtattctggg 8100
ggttcttcac catcggccat gactgcggcc acggcgcgtt ctcgcgttcg cacctgctca 8160
acttcagcgt cggcacgctc attcactcga tcatcctcac gccgtacgag tcatggaaga 8220
tctcgcaccg ccaccaccac aagaacacgg gcaacatcga caaggacgag attttctacc 8280
cgcagcgcga ggccgactcg cacccactgt cccgacacat ggtgatctcg ctcggctcgg 8340
cctggttcgc gtacctcgtt gcgggcttcc ctcctcgcaa ggtgaaccac ttcaaccctt 8400
gggaaccgtt gtacctgcgc cgcatgtctg ccgtcatcat ctcactcggc tcgctcgtgg 8460
cgttcgcggg cttgtatgcg tatctcacct acgtctatgg ccttaagacc atggcgctgt 8520
actacttcgc ccctctcttt gggttcgcca cgatgctcgt ggtcactacc tttttgcacc 8580
acaatgacga ggaaacgcca tggtacgccg actcggagtg gacgtacgtc aagggcaacc 8640
tctcgtccgt ggaccgctcg tacggcgcgc tcatcgacaa cctgagccac aacatcggca 8700
cgcaccagat ccaccacctg tttccgatca tcccgcacta caagctgaac gaggcgacgg 8760
cagcgttcgc gcaggcgttc ccggagctcg tgcgcaagag cgcgtcgccg atcatcccga 8820
cgttcatccg catcgggctc atgtacgcca agtacggcgt cgtggacaag gacgccaaga 8880
tgtttacgct caaggaggcc aaggccgcca agaccaaggc caactaggcg gccgcattga 8940
tgattggaaa cacacacatg ggttatatct aggtgagagt tagttggaca gttatatatt 9000
aaatcagcta tgccaacggt aacttcattc atgtcaacga ggaaccagtg actgcaagta 9060
atatagaatt tgaccacctt gccattctct tgcactcctt tactatatct catttatttc 9120
ttatatacaa atcacttctt cttcccagca tcgagctcgg aaacctcatg agcaataaca 9180
tcgtggatct cgtcaataga gggctttttg gactccttgc tgttggccac cttgtccttg 9240
ctgtttaaac agtgtacgca gatctactat agaggaacat ttaaattgcc ccggagaaga 9300
cggccaggcc gcctagatga caaattcaac aactcacagc tgactttctg ccattgccac 9360
tagggggggg cctttttata tggccaagcc aagctctcca cgtcggttgg gctgcaccca 9420
acaataaatg ggtagggttg caccaacaaa gggatgggat gggggtaga agatacgagg 9480
ataacggggc tcaatggcac aaataagaac gaatactgcc attaagactc gtgatccagc 9540
gactgacacc attgcatcat ctaagggcct caaaactacc tcggaactgc tgcgctgatc 9600
tggacaccac agaggttccg agcactttag gttgcaccaa atgtcccacc aggtgcaggc 9660
agaaaacgct ggaacagcgt gtacagtttg tcttaacaaa aagtgagggc gctgaggtcg 9720
agcagggtgg tgtgacttgt tatagccttt agagctgcga aagcgcgtat ggatttggct 9780
catcaggcca gattgagggt ctgtggacac atgtcatgtt agtgtacttc aatcgccccc 9840

tggatatagc cccgacaata ggccgtggcc tcattttttt gccttccgca catttccatt 9900
gctcggtacc cacaccttgc ttctcctgca cttgccaacc ttaatactgg tttacattga 9960
ccaacatctt acaagcgggg ggcttgtcta gggtatatat aaacagtggc tctcccaatc 10020
ggttgccagt ctcttttttc ctttctttcc ccacagattc gaaatctaaa ctacacatca 10080
cagaattccg agccgtgagt atccacgaca agatcagtgt cgagacgacg cgttttgtgt 10140
aatgacacaa tccgaaagtc gctagcaaca cacactctct acacaaacta acccagctct 10200
ggtaccatgg cttcttccac tgttgctgcg ccgtacgagt tcccgacgct gacggagatc 10260
aagcgctcgc tgccagcgca ctgctttgag gcctcggtcc cgtggtcgct ctactacacc 10320
gtgcgcgcgc tgggcatcgc cggctcgctc gcgctcggcc tctactacgc gcgcgcgctc 10380
gcgatcgtgc aggagtttgc cctgctggat gcggtgctct gcacggggta cattctgctg 10440
cagggcatcg tattctgggg gttcttcacc atcggccatg actgcggcca cggcgcgttc 10500
tcgcgttcgc acctgctcaa cttcagcgtc ggcacgctca ttcactcgat catcctcacg 10560
ccgtacgagt catggaagat ctcgcaccgc caccaccaca agaacacggg caacatcgac 10620
aaggacgaga ttttctaccc gcagcgcgag gccgactcgc acccactgtc ccgacacatg 10680
gtgatctcgc tcggctcggc ctggttcgcg tacctcgttg cgggcttccc tcctcgcaag 10740
gtgaaccact tcaacccttg ggaaccgttg tacctgcgcc gcatgtctgc cgtcatcatc 10800
tcactcggct cgctcgtggc gttcgcgggc ttgtatgcgt atctcaccta cgtctatggc 10860
cttaagacca tggcgctgta ctacttcgcc cctctctttg ggttcgccac gatgctcgtg 10920
gtcactacct ttttgcacca caatgacgag gaaacgccat ggtacgccga ctcggagtgg 10980
acgtacgtca agggcaacct ctcgtccgtg gaccgctcgt acggcgcgct catcgacaac 11040
ctgagccaca acatcggcac gcaccagatc caccacctgt ttccgatcat cccgcactac 11100
aagctgaacg aggcgacggc agcgttcgcg caggcgttcc cggagctcgt gcgcaagagc 11160
gcgtcgccga tcatcccgac gttcatccgc atcgggctca tgtacgccaa gtacggcgtc 11220
gtggacaagg acgccaagat gtttacgctc aaggaggcca aggccgccaa gaccaaggcc 11280
aactaggcgg ccgcatggag cgtgtgttct gagtcgatgt ttctatggag ttgtgagtg 11340
ttagtagaca tgatgggttt atatatgatg aatgaataga tgtgattttg atttgcacga 11400
tggaattgag aactttgtaa acgtacatgg gaatgtatga atgtgggggt tttgtgactg 11460
gataactgac ggtcagtgga cgccgttgtt caaatatcca agagatgcga gaaactttgg 11520
gtcaagtgaa catgtcctct ctgttcaagt aaaccatcaa ctatgggtag tatatttagt 11580
aaggacaaga gttgagattc tttggagtcc tagaaacgta ttttcgcgtt ccaagatcaa 11640
attagtagag taatacgggc acgggaatcc attcatagtc tcaatcctgc aggtgagtta 11700
attaagatga cgacatttgc gagctggacg aggaatagat ggagcgtgtg ttctgagtcg 11760

atgttttcta tggagttgtg agtgttagta gacatgatgg gtttatatat gatgaatgaa 11820

tagatgtgat tttgatttgc acgatggaat tgagaacttt gtaaacgtac atgggaatgt 11880

atgaatgtgg gggttttgtg actggataac tgacggtcag tggacgccgt tgttcaaata 11940

tccaagagat gcgagaaact ttgggtcaag tgaacatgtc ctctctgttc aagtaaacca 12000

tcaactatgg gtagtatatt tagtaaggac aagagttgag attctttgga gtcctagaaa 12060

cgtattttcg cgttccaaga tcaaattagt agagtaatac gggcacggga atccattcat 12120

agtctcaatt ttcccatagg tgtgctacaa ggtgttgaga tgtggtacag taccaccatg 12180

attcgaggta aagagcccag aagtcattga tgaggtcaag aaatacacag atctacagct 12240

caatacaatg aatatcttct ttcatattct tcaggtgaca ccaagggtgt ctattttccc 12300

cagaaatgcg tgaaaaggcg cgtgtgtagc gtggagtatg ggttcggttg gcgtatcctt 12360

catatatcga cgaaatagta gggcaagaga tgacaaaaag tatctatatg tagacagcgt 12420

agaatatgga tttgattggt ataaattcat ttattgcgtg tctcacaaat actctcgata 12480

agttggggtt aaactggaga tggaacaatg tcgatatctc gacgcatgcg acgtcgggcc 12540

caattcgccc tatagtgagt cgtattacaa ttcactggcc gtcgttttac aacgtcgtga 12600

ctgggaaaac cctggcgtta cccaacttaa tcgccttgca gcacatcccc ctttcgccag 12660

ctggcgtaat agcgaagagg cccgcaccga tcgcccttcc caacagttgc gcagcctgaa 12720

tggcgaatgg acgcgccctg tagcggcgca ttaagcgcgg cgggtgtggt ggttacgcgc 12780

agcgtgaccg ctacacttgc cagcgcccta gcgcccgctc ctttcgcttt cttcccttcc 12840

tttctcgcca cgttcgccgg ctttccccgt caagctctaa atcgggggct ccctttaggg 12900

ttccgattta gtgctttacg gcacctcgac cccaaaaaac ttgattaggg tgatggttca 12960

cgtagtgggc catcgccctg atagacggtt tttcgccctt tgacgttgga gtccacgttc 13020

tttaatagtg gactcttgtt ccaaactgga acaacactca acccta 13066

<210> 130
<211> 4313
<212> DNA
<213> Artificial Sequence

<220>
<223> Plasmid pZKUM

<400> 130

```
taatcgagct tggcgtaatc atggtcatag ctgtttcctg tgtgaaattg ttatccgctc        60
acaattccac acaacatacg agccggaagc ataaagtgta aagcctgggg tgcctaatga       120
gtgagctaac tcacattaat tgcgttgcgc tcactgcccg ctttccagtc gggaaacctg       180
tcgtgccagc tgcattaatg aatcggccaa cgcgcgggga gaggcggttt gcgtattggg       240
cgctcttccg cttcctcgct cactgactcg ctgcgctcgg tcgttcggct gcggcgagcg       300
gtatcagctc actcaaaggc ggtaatacgg ttatccacag aatcagggga taacgcagga       360
```

aagaacatgt gagcaaaagg ccagcaaaag gccaggaacc gtaaaaaggc cgcgttgctg 420
gcgtttttcc ataggctccg cccccctgac gagcatcaca aaaatcgacg ctcaagtcag 480
aggtggcgaa acccgacagg actataaaga taccaggcgt ttccccctgg aagctccctc 540
gtgcgctctc ctgttccgac cctgccgctt accggatacc tgtccgcctt tctcccttcg 600
ggaagcgtgg cgctttctca tagctcacgc tgtaggtatc tcagttcggt gtaggtcgtt 660
cgctccaagc tgggctgtgt gcacgaaccc cccgttcagc ccgaccgctg cgccttatcc 720
ggtaactatc gtcttgagtc caacccggta agacacgact tatcgccact ggcagcagcc 780
actggtaaca ggattagcag agcgaggtat gtaggcggtg ctacagagtt cttgaagtgg 840
tggcctaact acggctacac tagaaggaca gtatttggta tctgcgctct gctgaagcca 900
gttaccttcg gaaaaagagt tggtagctct tgatccggca aacaaaccac cgctggtagc 960
ggtggttttt ttgtttgcaa gcagcagatt acgcgcagaa aaaaaggatc tcaagaagat 1020
cctttgatct tttctacggg gtctgacgct cagtggaacg aaaactcacg ttaagggatt 1080
ttggtcatga gattatcaaa aaggatcttc acctagatcc ttttaaatta aaaatgaagt 1140
tttaaatcaa tctaaagtat atatgagtaa acttggtctg acagttacca atgcttaatc 1200
agtgaggcac ctatctcagc gatctgtcta tttcgttcat ccatagttgc ctgactcccc 1260
gtcgtgtaga taactacgat acgggagggc ttaccatctg gccccagtgc tgcaatgata 1320
ccgcgagacc cacgctcacc ggctccagat ttatcagcaa taaaccagcc agccggaagg 1380
gccgagcgca gaagtggtcc tgcaacttta tccgcctcca tccagtctat taattgttgc 1440
cgggaagcta gagtaagtag ttcgccagtt aatagtttgc gcaacgttgt tgccattgct 1500
acaggcatcg tggtgtcacg ctcgtcgttt ggtatggctt cattcagctc cggttcccaa 1560
cgatcaaggc gagttacatg atcccccatg ttgtgcaaaa aagcggttag ctccttcggt 1620
cctccgatcg ttgtcagaag taagttggcc gcagtgttat cactcatggt tatggcagca 1680
ctgcataatt ctcttactgt catgccatcc gtaagatgct tttctgtgac tggtgagtac 1740
tcaaccaagt cattctgaga atagtgtatg cggcgaccga gttgctcttg cccggcgtca 1800
atacgggata ataccgcgcc acatagcaga actttaaaag tgctcatcat tggaaaacgt 1860
tcttcggggc gaaaactctc aaggatctta ccgctgttga gatccagttc gatgtaaccc 1920
actcgtgcac ccaactgatc ttcagcatct tttactttca ccagcgtttc tgggtgagca 1980
aaaacaggaa ggcaaaatgc cgcaaaaaag ggaataaggg cgacacggaa atgttgaata 2040
ctcatactct tcctttttca atattattga agcatttatc agggttattg tctcatgagc 2100
ggatacatat ttgaatgtat ttagaaaaat aaacaaatag gggttccgcg cacatttccc 2160
cgaaaagtgc cacctgacgc gccctgtagc ggcgcattaa gcgcggcggg tgtggtggtt 2220
acgcgcagcg tgaccgctac acttgccagc gccctagcgc ccgctccttt cgctttcttc 2280

ccttcctttc tcgccacgtt cgccggcttt ccccgtcaag ctctaaatcg ggggctccct 2340
ttagggttcc gatttagtgc tttacggcac ctcgacccca aaaaacttga ttagggtgat 2400
ggttcacgta gtgggccatc gccctgatag acggtttttc gccctttgac gttggagtcc 2460
acgttcttta atagtggact cttgttccaa actggaacaa cactcaaccc tatctcggtc 2520
tattcttttg atttataagg gattttgccg atttcggcct attggttaaa aaatgagctg 2580
atttaacaaa aatttaacgc gaattttaac aaaatattaa cgcttacaat ttccattcgc 2640
cattcaggct gcgcaactgt tgggaagggc gatcggtgcg ggcctcttcg ctattacgcc 2700
agctggcgaa agggggatgt gctgcaaggc gattaagttg ggtaacgcca gggttttccc 2760
agtcacgacg ttgtaaaacg acggccagtg aattgtaata cgactcacta tagggcgaat 2820
tgggtaccgg gccccccctc gaggtcgacg agtatctgtc tgactcgtca ttgccgcctt 2880
tggagtacga ctccaactat gagtgtgctt ggatcacttt gacgatacat tcttcgttgg 2940
aggctgtggg tctgacagct gcgttttcgg cgcggttggc cgacaacaat atcagctgca 3000
acgtcattgc tggctttcat catgatcaca tttttgtcgg caaaggcgac gcccagagag 3060
ccattgacgt tctttctaat ttggaccgat agccgtatag tccagtctat ctataagttc 3120
aactaactcg taactattac cataacatat acttcactgc cccagataag gttccgataa 3180
aaagttctgc agactaaatt tatttcagtc tcctcttcac caccaaaatg ccctcctacg 3240
aagctcgagt gctcaagctc gtggcagcca agaaaaccaa cctgtgtgct tctctggatg 3300
ttaccaccac caaggagctc attgagcttg ccgataaggt cggaccttat gtgtgcatga 3360
tcaaaaccca tatcgacatc attgacgact tcacctacgc cggcactgtg ctcccccctca 3420
aggaacttgc tcttaagcac ggtttcttcc tgttcgagga cagaaagttc gcagatattg 3480
gcaacactgt caagcaccag taccggtgtc accgaatcgc cgagtggtcc gatatcacca 3540
acgcccacgg tgtacccgga accggaatcg attgctggcc tgcgagctgg tgcgtacgag 3600
gaaactgtct ctgaacagaa gaaggaggac gtctctgact acgagaactc ccagtacaag 3660
gagttcctag tcccctctcc caacgagaag ctggccagag gtctgctcat gctggccgag 3720
ctgtcttgca agggctctct ggccactggc gagtactcca agcagaccat tgagcttgcc 3780
cgatccgacc ccgagtttgt ggttggcttc attgcccaga accgacctaa gggcgactct 3840
gaggactggc ttattctgac ccccggggtg ggtcttgacg acaagggaga cgctctcgga 3900
cagcagtacc gaactgttga ggatgtcatg tctaccggaa cggatatcat aattgtcggc 3960
cgaggtctgt acggccagaa ccgagatcct attgaggagg ccaagcgata ccagaaggct 4020
ggctgggagg cttaccagaa gattaactgt tagaggttag actatggata tgtaatttaa 4080
ctgtgtatat agagagcgtg caagtatgga gcgcttgttc agcttgtatg atggtcagac 4140
gacctgtctg atcgagtatg tatgatactg cacaacctgt gtatccgcat gatctgtcca 4200
atggggcatg ttgttgtgtt tctcgatacg gagatgctgg gtacagtgct aatacgttga 4260

actacttata cttatatgag gctcgaagaa agctgacttg tgtatgactt aat 4313

<210> 131
<211> 15991
<212> DNA
<213> Artificial Sequence

<220>
<223> Plasmid pZKL2-5mB89C

<400> 131

```
gtacgttatc atttgaacag tgaaaggcta cagtaacaga agcagttgta aacttcattc      60
cgttgattct gtactacagt accccactac gccgcttccg ctgacactgt tcaacccaaa     120
aactacatct gcgtgcgctg tgtaaggcta tcatcagata catactgtag attctgtaga     180
tgcgaacctg cttgtatcat atacatcccc ctccccctga cctgcacaag caagcaatgt     240
gacattgata ttgctgctta tctagtgccg aggatgtgaa agccgagact caaacatttc     300
ttttactctc ttgttcctga ccagacctgg cggagattac gccagtatga ttcttgcagg     360
tctgagacaa gcctggaaca gccaacattt atttttcgaa gcgagaaaca tgccacaccc     420
cggcacgttc agagatgcat atgatttgtt tttcgagtaa cagtaccccc cccccccccc     480
ccaatgaaac cagtattact cacaccatcc tcattcaaag cgttacactg attacgcgcc     540
catcaacgac agcatgaggg gactgctgat ctgatctaat caaatgacta caaaaatcgc     600
aataatgaag agcaaacgac aaaaaagaaa caggttaacc aatcccgctt caatgtctca     660
ccacaatcca gcactgtttc tcattacctc ctccctctaa tttcagagtt gcatcagggt     720
ccttgatggc gcgccagctg cattaatgaa tcggccaacg cgcggggaga ggcggtttgc     780
gtattgggcg ctcttccgct tcctcgctca ctgactcgct gcgctcggtc gttcggctgc     840
ggcgagcggt atcagctcac tcaaaggcgg taatacggtt atccacagaa tcaggggata     900
acgcaggaaa gaacatgtga gcaaaaggcc agcaaaaggc caggaaccgt aaaaaggccg     960
cgttgctggc gtttttccat aggctccgcc cccctgacga gcatcacaaa aatcgacgct    1020
caagtcagag gtggcgaaac ccgacaggac tataaagata ccaggcgttt ccccctggaa    1080
gctccctcgt gcgctctcct gttccgaccc tgccgcttac cggatacctg tccgcctttc    1140
tcccttcggg aagcgtggcg ctttctcata gctcacgctg taggtatctc agttcggtgt    1200
aggtcgttcg ctccaagctg ggctgtgtgc acgaaccccc cgttcagccc gaccgctgcg    1260
ccttatccgg taactatcgt cttgagtcca acccggtaag acacgactta tcgccactgg    1320
cagcagccac tggtaacagg attagcagag cgaggtatgt aggcggtgct acagagttct    1380
tgaagtggtg gcctaactac ggctacacta gaagaacagt atttggtatc tgcgctctgc    1440
tgaagccagt taccttcgga aaaagagttg gtagctcttg atccggcaaa caaaccaccg    1500
ctggtagcgg tggttttttt gtttgcaagc agcagattac gcgcagaaaa aaaggatctc    1560
```

aagaagatcc tttgatcttt tctacggggt ctgacgctca gtggaacgaa aactcacgtt 1620
aagggatttt ggtcatgaga ttatcaaaaa ggatcttcac ctagatcctt ttaaattaaa 1680
aatgaagttt taaatcaatc taaagtatat atgagtaaac ttggtctgac agttaccaat 1740
gcttaatcag tgaggcacct atctcagcga tctgtctatt tcgttcatcc atagttgcct 1800
gactccccgt cgtgtagata actacgatac gggagggctt accatctggc cccagtgctg 1860
caatgatacc gcgagaccca cgctcaccgg ctccagattt atcagcaata aaccagccag 1920
ccggaagggc cgagcgcaga agtggtcctg caactttatc cgcctccatc cagtctatta 1980
attgttgccg ggaagctaga gtaagtagtt cgccagttaa tagtttgcgc aacgttgttg 2040
ccattgctac aggcatcgtg gtgtcacgct cgtcgtttgg tatggcttca ttcagctccg 2100
gttcccaacg atcaaggcga gttacatgat cccccatgtt gtgcaaaaaa gcggttagct 2160
ccttcggtcc tccgatcgtt gtcagaagta agttggccgc agtgttatca ctcatggtta 2220
tggcagcact gcataattct cttactgtca tgccatccgt aagatgcttt tctgtgactg 2280
gtgagtactc aaccaagtca ttctgagaat agtgtatgcg gcgaccgagt tgctcttgcc 2340
cggcgtcaat acgggataat accgcgccac atagcagaac tttaaaagtg ctcatcattg 2400
gaaaacgttc ttcggggcga aaactctcaa ggatcttacc gctgttgaga tccagttcga 2460
tgtaacccac tcgtgcaccc aactgatctt cagcatcttt tactttcacc agcgtttctg 2520
ggtgagcaaa aacaggaagg caaaatgccg caaaaaaggg aataagggcg acacggaaat 2580
gttgaatact catactcttc ctttttcaat attattgaag catttatcag ggttattgtc 2640
tcatgagcgg atacatattt gaatgtattt agaaaaataa acaaataggg gttccgcgca 2700
catttccccg aaaagtgcca cctgatgcgg tgtgaaatac cgcacagatg cgtaaggaga 2760
aaataccgca tcaggaaatt gtaagcgtta atattttgtt aaaattcgcg ttaaattttt 2820
gttaaatcag ctcatttttt aaccaatagg ccgaaatcgg caaaatccct tataaatcaa 2880
aagaatagac cgagataggg ttgagtgttg ttccagtttg gaacaagagt ccactattaa 2940
agaacgtgga ctccaacgtc aaagggcgaa aaaccgtcta tcagggcgat ggcccactac 3000
gtgaaccatc accctaatca agttttttgg ggtcgaggtg ccgtaaagca ctaaatcgga 3060
accctaaagg gagcccccga tttagagctt gacggggaaa gccggcgaac gtggcgagaa 3120
aggaagggaa gaaagcgaaa ggagcgggcg ctagggcgct ggcaagtgta gcggtcacgc 3180
tgcgcgtaac caccacaccc gccgcgctta atgcgccgct acagggcgcg tccattcgcc 3240
attcaggctg cgcaactgtt gggaagggcg atcggtgcgg gcctcttcgc tattacgcca 3300
gctggcgaaa gggggatgtg ctgcaaggcg attaagttgg gtaacgccag ggttttccca 3360
gtcacgacgt tgtaaaacga cggccagtga attgtaatac gactcactat agggcgaatt 3420
gggcccgacg tcgcatgctg gtttcgattt gtcttagagg aacgcatata cagtaatcat 3480
agagaataaa cgatattcat ttattaaagt agatagttga ggtagaagtt gtaaagagtg 3540

ataaatagct tagataccac agacaccctc ggtgacgaag tactgcagat ggtttccaat 3600
cacattgacc tgctggagca gagtgttacc ggcagagcac tgtttattgc tctggccctg 3660
gcacatgaca acgttggaga gaggagggtg gatcaggggc cagtcaataa agacctcacc 3720
agagcagtgc tggtaaccgt cccagaaggg cacttgaggg acgatatctc ctcggtgggt 3780
gattcggtag agctttcggt ctttggacac cttggagaca tcggggttct cctggccaaa 3840
gaagagttta tcgacccagt tagcaaagcc agcgttaccg acaatgggct gaccaagagt 3900
aacaacgagg ggatcgtggc cgttaacctt gaggttgatt ccgaacagaa gggctgcagc 3960
tcctccgaga gagtgaccgg tgacagcaat ctggtagtcg ggatactgct caatcacaga 4020
gtcgagcttg gggccgatct gattgtaggt gttgttgtag gactggatga agccattgtg 4080
gacaagacag tcatcacaag tagcagtaga agagatgtta gcagcaagat caaagttaat 4140
taactcacct gcaggattga gactatgaat ggattcccgt gcccgtatta ctctactaat 4200
ttgatcttgg aacgcgaaaa tacgtttcta ggactccaaa gaatctcaac tcttgtcctt 4260
actaaatata ctacccatag ttgatggttt acttgaacag agaggacatg ttcacttgac 4320
ccaaagtttc tcgcatctct tggatatttg aacaacggcg tccactgacc gtcagttatc 4380
cagtcacaaa acccccacat tcatacattc ccatgtacgt ttacaaagtt ctcaattcca 4440
tcgtgcaaat caaaatcaca tctattcatt catcatatat aaacccatca tgtctactaa 4500
cactcacaac tccatagaaa acatcgactc agaacacacg ctccatgcgg ccgcttagga 4560
atcctgagcg tccttgacac agtgaaccac accgactttg tgcatgtact tgagggtgga 4620
aatgatgttg cccacaatgg tagggtagaa gacgtaccga actccgtgtc gttcgcaaca 4680
ctctcggaca gcttgctgca cgaagggata gtgccaagac gacattcgag gaaagaggtg 4740
atgctcgatc tggaagttga gaccgccagt aaagaacatg gcaatgggtc caccgtaggt 4800
ggaagaggtc tccacctgag ctctgtacca gtcgatctga tcggcttcaa cgtccttctc 4860
ggagctcttg accttgcagt tcttgtcggg gattcgctcc gagccatcga agttgtgaga 4920
caagatgaaa aagaaggtga ggaaggcacc ggtagcagtg ggcaccagag gaatggtgat 4980
gagcagggag gttccagtga gataccaggg caagaaggcg gttcgaaaga tgaagaaagc 5040
tcgcataacg aatgcaaggg ttcggtaccg tcgcagaaag ccgttctctc gcatggctgt 5100
gacagactcg ggaatggtgt cgttgtgctg cattcggaag atgtagagag ggttgtacac 5160
cagcgaaacg ccgtaggctc caagcacgag gtacatgtac caggcctgga atcggtgaaa 5220
ccactttcga gcagtgttgg cagcagggta gttgtggaac acaaggaatg gttctgcgga 5280
ctcggcatcc aggtcgagac catgctgatt ggtgtaggtg tgatgtcgca tgatgtgaga 5340
ctgcagccag atccatctgg acgatccaat gacgtcgatg ccgtaggcaa agagagcgtt 5400
gacccagggc tttttgctga tggcaccatg agaggcatcg tgctgaatgg acaggccgat 5460

ctgcatgtgc atgaatccag tcaagagacc ccacagcacc attccggtag tagcccagtg 5520
ccactcgcaa aaggcggtga cagcaatgat gccaacggtt cgcagccaga atccaggtgt 5580
ggcataccag ttccgacctt tcatgacctc tcgcatagtt cgcttgacgt cctgtgcaaa 5640
gggagagtcg taggtgtaga caatgtcctt ggaggttcgg tcgtgcttgc ctcgcacgaa 5700
ctgttgaagc agcttcgagt tctcgggctt gacgtaaggg tgcatggagt agaacagagg 5760
agaagcatcg gaggcaccag aagcgaggat caagtcggat ccgggatgga ccttggcaag 5820
accttccaga tcgtagagaa tgccgtcgat ggcaaccagg tcgggtcgct cgagcagctg 5880
ctcggtagta agggagagag ccatgggcag gacctgtgtt agtacattgt cggggagtca 5940
tcaattggtt cgacaggttg tcgactgtta gtatgagctc aattgggctc tggtgggtcg 6000
atgacacttg tcatctgttt ctgttgggtc atgtttccat caccttctat ggtactcaca 6060
attcgtccga ttcgcccgaa tccgttaata ccgactttga tggccatgtt gatgtgtgtt 6120
taattcaaga atgaatatag agaagagaag aagaaaaaag attcaattga gccggcgatg 6180
cagaccctta tataaatgtt gccttggaca gacggagcaa gcccgcccaa acctacgttc 6240
ggtataatat gttaagcttt ttaacacaaa ggtttggctt ggggtaacct gatgtggtgc 6300
aaaagaccgg gcgttggcga gccattgcgc gggcgaatgg ggccgtgact cgtctcaaat 6360
tcgagggcgt gcctcaattc gtgcccccgt ggctttttcc cgccgtttcc gccccgtttg 6420
caccactgca gccgcttctt tggttcggac accttgctgc gagctaggtg ccttgtgcta 6480
cttaaaaagt ggcctcccaa caccaacatg acatgagtgc gtgggccaag acacgttggc 6540
ggggtcgcag tcggctcaat ggcccggaaa aaacgctgct ggagctggtt cggacgcagt 6600
ccgccgcggc gtatggatat ccgcaaggtt ccatagcgcc attgccctcc gtcggcgtct 6660
atcccgcaac ctctaaatag agcgggaata taacccaagc ttcttttttt tcctttaaca 6720
cgcacacccc caactatcat gttgctgctg ctgtttgact ctactctgtg gaggggtgct 6780
cccacccaac ccaacctaca ggtggatccg gcgctgtgat tggctgataa gtctcctatc 6840
cggactaatt ctgaccaatg ggacatgcgc gcaggaccca aatgccgcaa ttacgtaacc 6900
ccaacgaaat gcctacccct ctttggagcc cagcggcccc aaatcccccc aagcagcccg 6960
gttctaccgg cttccatctc caagcacaag cagcccggaa ttctctctct tgagcttttc 7020
cataacaagt tcttctgcct ccaggaagtc catgggtggt ttgatcatgg ttttggtgta 7080
gtggtagtgc agtggtggta ttgtgactgg ggatgtagtt gagaataagt catacacaag 7140
tcagctttct tcgagcctca tataagtata agtagttcaa cgtattagca ctgtacccag 7200
catctccgta tcgagaaaca caacaacatg ccccattgga cagatcatgc ggatacacag 7260
gttgtgcagt atcatacata ctcgatcaga caggtcgtct gaccatcata caagctgaac 7320
aagcgctcca tacttgcacg ctctctatat acacagttaa attacatatc catagtctaa 7380
cctctaacag ttaatcttct ggtaagcctc ccagccagcc ttctggtatc gcttggcctc 7440

ctcaatagga tctcggttct ggccgtacag acctcggccg acaattatga tatccgttcc 7500

ggtagacatg acatcctcaa cagttcggta ctgctgtccg agagcgtctc ccttgtcgtc 7560

aagacccacc ccgggggtca gaataagcca gtcctcagag tcgcccttag gtcggttctg 7620

ggcaatgaag ccaaccacaa actcggggtc ggatcgggca agctcaatgg tctgcttgga 7680

gtactcgcca gtggccagag agcccttgca agacagctcg gccagcatga gcagacctct 7740

ggccagcttc tcgttgggag aggggactag gaactccttg tactgggagt tctcgtagtc 7800

agagacgtcc tccttcttct gttcagagac agtttcctcg gcaccagctc gcaggccagc 7860

aatgattccg gttccgggta caccgtgggc gttggtgata tcggaccact cggcgattcg 7920

gtgacaccgg tactggtgct tgacagtgtt gccaatatct gcgaactttc tgtcctcgaa 7980

caggaagaaa ccgtgcttaa gagcaagttc cttgaggggg agcacagtgc cggcgtaggt 8040

gaagtcgtca atgatgtcga tatgggtttt gatcatgcac acataaggtc cgaccttatc 8100

ggcaagctca atgagctcct tggtggtggt aacatccaga gaagcacaca ggttggtttt 8160

cttggctgcc acgagcttga gcactcgagc ggcaaaggcg gacttgtgga cgttagctcg 8220

agcttcgtag gagggcattt tggtggtgaa gaggagactg aaataaattt agtctgcaga 8280

actttttatc ggaaccttat ctggggcagt gaagtatatg ttatggtaat agttacgagt 8340

tagttgaact tatagataga ctggactata cggctatcgg tccaaattag aaagaacgtc 8400

aatggctctc tgggcgtcgc ctttgccgac aaaaatgtga tcatgatgaa agccagcaat 8460

gacgttgcag ctgatattgt tgtcggccaa ccgcgccgaa aacgcagctg tcagacccac 8520

agcctccaac gaagaatgta tcgtcaaagt gatccaagca cactcatagt tggagtcgta 8580

ctccaaaggc ggcaatgacg agtcagacag atactcgtcg accttttcct tgggaaccac 8640

caccgtcagc ccttctgact cacgtattgt agccaccgac acaggcaaca gtccgtggat 8700

agcagaatat gtcttgtcgg tccatttctc accaacttta ggcgtcaagt gaatgttgca 8760

gaagaagtat gtgccttcat tgagaatcgg tgttgctgat ttcaataaag tcttgagatc 8820

agtttggcca gtcatgttgt ggggggtaat tggattgagt tatcgcctac agtctgtaca 8880

ggtatactcg ctgcccactt tatacttttt gattccgctg cacttgaagc aatgtcgttt 8940

accaaaagtg agaatgctcc acagaacaca ccccagggta tggttgagca aaaaataaac 9000

actccgatac ggggaatcga accccggtct ccacggttct caagaagtat tcttgatgag 9060

agcgtatcga tcgaggaaga ggacaagcgg ctgcttctta agtttgtgac atcagtatcc 9120

aaggcaccat tgcaaggatt caaggctttg aacccgtcat ttgccattcg taacgctggt 9180

agacaggttg atcggttccc tacggcctcc acctgtgtca atcttctcaa gctgcctgac 9240

tatcaggaca ttgatcaact tcggaagaaa cttttgtatg ccattcgatc acatgctggt 9300

ttcgatttgt cttagaggaa cgcatataca gtaatcatag agaataaacg atattcattt 9360

attaaagtag atagttgagg tagaagttgt aaagagtgat aaatagcggc cgctcactga 9420
atctttttgg ctcccttgtg ctttcggacg atgtaggtct gcacgtagaa gttgaggaac 9480
agacacagga cagtaccaac gtagaagtag ttgaaaaacc agccaaacat tctcattcca 9540
tcttgtcggt agcagggaat gttccggtac ttccagacga tgtagaagcc aacgttgaac 9600
tgaatgatct gcatagaagt aatcagggac ttgggcatag ggaacttgag cttgatcagt 9660
cgggtccaat agtagccgta catgatccag tgaatgaagc cgttgagcag cacaaagatc 9720
caaacggctt cgtttcggta gttgtagaac agccacatgt ccataggagc tccgagatgg 9780
tgaaagaact gcaaccaggt cagaggcttg cccatgaggg gcagatagaa ggagtcaatg 9840
tactcgagga acttgctgag gtagaacagc tgagtggtga ttcggaagac attgttgtcg 9900
aaagccttct cgcagttgtc ggacatgaca ccaatggtgt acatggcgta ggccatagag 9960
aggaaggagc ccagcgagta gatggacatg agcaggttgt agttggtgaa cacaaacttc 10020
attcgagact gacccttggg tccgagagga ccaagggtga acttcaggat gacgaaggcg 10080
atggagaggt acagcacctc gcagtgcgag gcatcagacc agagctgagc atagtcgacc 10140
ttgggaagaa cctcctggcc aatggagacg atttcgttca cgacctccat ggttgtgaat 10200
tagggtggtg agaatggttg gttgtaggga agaatcaaag gccggtctcg ggatccgtgg 10260
gtatatatat atatatatat atatacgatc cttcgttacc tccctgttct caaaactgtg 10320
gtttttcgtt tttcgttttt tgcttttttt gattttttta gggccaacta agcttccaga 10380
tttcgctaat cacctttgta ctaattacaa gaaaggaaga agctgattag agttgggctt 10440
tttatgcaac tgtgctactc cttatctctg atatgaaagt gtagacccaa tcacatcatg 10500
tcatttagag ttggtaatac tgggaggata gataaggcac gaaaacgagc catagcagac 10560
atgctgggtg tagccaagca gaagaaagta gatgggagcc aattgacgag cgagggagct 10620
acgccaatcc gacatacgac acgctgagat cgtcttggcc gggggggtacc tacagatgtc 10680
caagggtaag tgcttgactg taattgtatg tctgaggaca aatatgtagt cagccgtata 10740
aagtcatacc aggcaccagt gccatcatcg aaccactaac tctctatgat acatgcctcc 10800
ggtattattg taccatgcgt cgctttgtta catacgtatc ttgccttttt ctctcagaaa 10860
ctccagactt tggctattgg tcgagataag cccggaccat agtgagtctt tcacactctg 10920
tttaaacacc actaaaaccc cacaaaatat atcttaccga atatacagat ctactataga 10980
ggaacaattg ccccggagaa gacggccagg ccgcctagat gacaaattca acaactcaca 11040
gctgactttc tgccattgcc actagggggg ggccttttta tatggccaag ccaagctctc 11100
cacgtcggtt gggctgcacc caacaataaa tgggtagggt tgcaccaaca aagggatggg 11160
atgggggggta gaagatacga ggataacggg gctcaatggc acaaataaga acgaatactg 11220
ccattaagac tcgtgatcca gcgactgaca ccattgcatc atctaagggc ctcaaaacta 11280
cctcggaact gctgcgctga tctggacacc acagaggttc cgagcacttt aggttgcacc 11340

aaatgtccca ccaggtgcag gcagaaaacg ctggaacagc gtgtacagtt tgtcttaaca 11400

aaaagtgagg gcgctgaggt cgagcagggt ggtgtgactt gttatagcct ttagagctgc 11460

gaaagcgcgt atggatttgg ctcatcaggc cagattgagg gtctgtggac acatgtcatg 11520

ttagtgtact tcaatcgccc cctggatata gccccgacaa taggccgtgg cctcattttt 11580

ttgccttccg cacatttcca ttgctcggta cccacacctt gcttctcctg cacttgccaa 11640

ccttaatact ggtttacatt gaccaacatc ttacaagcgg ggggcttgtc tagggtatat 11700

ataaacagtg gctctcccaa tcggttgcca gtctcttttt tcctttcttt ccccacagat 11760

tcgaaatcta aactacacat cacacaatgc ctgttactga cgtccttaag cgaaagtccg 11820

gtgtcatcgt cggcgacgat gtccgagccg tgagtatcca cgacaagatc agtgtcgaga 11880

cgacgcgttt tgtgtaatga cacaatccga aagtcgctag caacacacac tctctacaca 11940

aactaaccca gctctccatg gtgaaggctt ctcgacaggc tctgcccctc gtcatcgacg 12000

gaaaggtgta cgacgtctcc gcttgggtga acttccaccc tggtggagct gaaatcattg 12060

agaactacca gggacgagat gctactgacg ccttcatggt tatgcactct caggaagcct 12120

tcgacaagct caagcgaatg cccaagatca accaggcttc cgagctgcct ccccaggctg 12180

ccgtcaacga agctcaggag gatttccgaa agctccgaga agagctgatc gccactggca 12240

tgtttgacgc ctctcccctc tggtactcgt acaagatctt gaccaccctg ggtcttggcg 12300

tgcttgcctt cttcatgctg gtccagtacc acctgtactt cattggtgct ctcgtgctcg 12360

gtatgcacta ccagcaaatg ggatggctgt ctcatgacat ctgccaccac cagaccttca 12420

agaaccgaaa ctggaataac gtcctgggtc tggtctttgg caacggactc cagggcttct 12480

ccgtgacctg gtggaaggac agacacaacg cccatcattc tgctaccaac gttcagggtc 12540

acgatcccga cattgataac ctgcctctgc tcgcctggtc cgaggacgat gtcactcgag 12600

cttctcccat ctcccgaaag ctcattcagt tccaacagta ctatttcctg gtcatctgta 12660

ttctcctgcg attcatctgg tgtttccagt ctgtgctgac cgttcgatcc ctcaaggacc 12720

gagacaacca gttctaccga tctcagtaca agaaagaggc cattggactc gctctgcact 12780

ggactctcaa gaccctgttc cacctcttct ttatgccctc catcctgacc tcgatgctgg 12840

tgttctttgt ttccgagctc gtcggtggct tcggaattgc catcgtggtc ttcatgaacc 12900

actaccctct ggagaagatc ggtgattccg tctgggacgg acatggcttc tctgtgggtc 12960

agatccatga gaccatgaac attcgacgag gcatcattac tgactggttc tttggaggcc 13020

tgaactacca gatcgagcac catctctggc ccaccctgcc tcgacacaac ctcactgccg 13080

tttcctacca ggtggaacag ctgtgccaga agcacaacct cccctaccga aaccctctgc 13140

cccatgaagg tctcgtcatc ctgctccgat acctgtccca gttcgctcga atggccgaga 13200

agcagcccgg tgccaaggct cagtaagcgg ccgcatgaga agataaatat ataaatacat 13260

tgagatatta aatgcgctag attagagagc ctcatactgc tcggagagaa gccaagacga 13320

gtactcaaag gggattacac catccatatc cacagacaca agctggggaa aggttctata 13380

tacactttcc ggaataccgt agtttccgat gttatcaatg gggcagcca ggatttcagg 13440

cacttcggtg tctcggggtg aaatggcgtt cttggcctcc atcaagtcgt accatgtctt 13500

catttgcctg tcaaagtaaa acagaagcag atgaagaatg aacttgaagt gaaggaattt 13560

aaatagttgg agcaagggag aaatgtagag tgtgaaagac tcactatggt ccgggcttat 13620

ctcgaccaat agccaaagtc tggagtttct gagagaaaaa ggcaagatac gtatgtaaca 13680

aagcgacgca tggtacaata ataccggagg catgtatcat agagagttag tggttcgatg 13740

atggcactgg tgcctggtat gactttatac ggctgactac atatttgtcc tcagacatac 13800

aattacagtc aagcacttac ccttggacat ctgtaggtac cccccggcca agacgatctc 13860

agcgtgtcgt atgtcggatt ggcgtagctc cctcgctcgt caattggctc ccatctactt 13920

tcttctgctt ggctacaccc agcatgtctg ctatggctcg ttttcgtgcc ttatctatcc 13980

tcccagtatt accaactcta aatgacatga tgtgattggg tctacacttt catatcagag 14040

ataaggagta gcacagttgc ataaaaagcc caactctaat cagcttcttc ctttcttgta 14100

attagtacaa aggtgattag cgaaatctgg aagcttagtt ggccctaaaa aaatcaaaaa 14160

aagcaaaaaa cgaaaaacga aaaaccacag ttttgagaac agggaggtaa cgaaggatcg 14220

tatatatata tatatatata tatacccacg gatcccgaga ccggcctttg attcttccct 14280

acaaccaacc attctcacca ccctaattca caaccatggg cgtattcatt aaacaggagc 14340

agcttccggc tctcaagaag tacaagtact ccgccgagga tcactcgttc atctccaaca 14400

acattctgcg ccccttctgg cgacagtttg tcaaaatctt ccctctgtgg atggccccca 14460

acatggtgac tctgctgggc ttcttctttg tcattgtgaa cttcatcacc atgctcattg 14520

ttgatcccac ccacgaccgc gagcctccca gatgggtcta cctcacctac gctctgggtc 14580

tgttccttta ccagacattt gatgcctgtg acggatccca tgcccgacga actggccaga 14640

gtggacccct tggagagctg tttgaccact gtgtcgacgc catgaatacc tctctgattc 14700

tcacggtggt ggtgtccacc acccatatgg gatataacat gaagctactg attgtgcaga 14760

ttgccgctct cggaaacttc tacctgtcga cctgggagac ctaccatacc ggaactctgt 14820

acctttctgg cttctctggt cctgttgaag gtatcttgat tctggtggct cttttcgtcc 14880

tcaccttctt cactggtccc aacgtgtacg ctctgaccgt ctacgaggct cttcccgagt 14940

ccatcacttc gctgctgcct gccagcttcc tggacgtcac catcacccag atctacattg 15000

gattcggagt gctgggcatg gtgttcaaca tctacggcgc ctgcggaaac gtgatcaagt 15060

actacaacaa caagggcaag agcgctctcc ccgccattct cggaatcgcc ccctttggca 15120

tcttctacgt cggcgtcttt gcctgggccc atgttgctcc tctgcttctc tccaagtacg 15180

ccatcgtcta tctgtttgcc attggggctg cctttgccat gcaagtcggc cagatgattc 15240

ttgcccatct cgtgcttgct ccctttcccc actggaacgt gctgctcttc ttcccctttg 15300

tgggactggc agtgcactac attgcacccg tgtttggctg ggacgccgat atcgtgtcgg 15360

ttaacactct cttcacctgt tttggcgcca ccctctccat ttacgccttc tttgtgcttg 15420

agatcatcga cgagatcacc aactacctcg atatctggtg tctgcgaatc aagtaccctc 15480

aggagaagaa gaccgaataa gcggccgcat ggagcgtgtg ttctgagtcg atgttttcta 15540

tggagttgtg agtgttagta gacatgatgg gtttatatat gatgaatgaa tagatgtgat 15600

tttgatttgc acgatggaat tgagaacttt gtaaacgtac atgggaatgt atgaatgtgg 15660

gggttttgtg actggataac tgacggtcag tggacgccgt tgttcaaata tccaagagat 15720

gcgagaaact ttgggtcaag tgaacatgtc ctctctgttc aagtaaacca tcaactatgg 15780

gtagtatatt tagtaaggac aagagttgag attctttgga gtcctagaaa cgtattttcg 15840

cgttccaaga tcaaattagt agagtaatac gggcacggga atccattcat agtctcaatc 15900

ctgcaggtga gttaattaat cgagcttggc gtaatcatgg tcatagctgt ttcctgtgtg 15960

aaattgttat ccgctcacaa ttccacacaa c 15991

<210> 132
<211> 14554
<212> DNA
<213> Artificial Sequence

<220>
<223> Plasmid pZKL1-2SR9G85

<400> 132

cgatagttgg agcaagggag aaatgtagag tgtgaaagac tcactatggt ccgggcttat 60
ctcgaccaat agccaaagtc tggagtttct gagagaaaaa ggcaagatac gtatgtaaca 120
aagcgacgca tggtacaata ataccggagg catgtatcat agagagttag tggttcgatg 180
atggcactgg tgcctggtat gactttatac ggctgactac atatttgtcc tcagacatac 240
aattacagtc aagcacttac ccttggacat ctgtaggtac cccccggcca agacgatctc 300
agcgtgtcgt atgtcggatt ggcgtagctc cctcgctcgt caattggctc ccatctactt 360
tcttctgctt ggctacaccc agcatgtctg ctatggctcg ttttcgtgcc ttatctatcc 420
tcccagtatt accaactcta aatgacatga tgtgattggg tctacacttt catatcagag 480
ataaggagta gcacagttgc ataaaaagcc caactctaat cagcttcttc ctttcttgta 540
attagtacaa aggtgattag cgaaatctgg aagcttagtt ggccctaaaa aaatcaaaaa 600
aagcaaaaaa cgaaaaacga aaaaccacag ttttgagaac agggaggtaa cgaaggatcg 660
tatatatata tatatatata tatacccacg gatcccgaga ccggcctttg attcttccct 720
acaaccaacc attctcacca ccctaattca caaccatggc tgccgtcatc gaggtggcca 780
acgagttcgt cgctatcact gccgagaccc ttcccaaggt ggactatcag cgactctggc 840

gagacatcta ctcctgcgag ctcctgtact tctccattgc tttcgtcatc ctcaagttta 900
cccttggcga gctctcggat tctggcaaaa agattctgcg agtgctgttc aagtggtaca 960
acctcttcat gtccgtcttt tcgctggtgt ccttcctctg tatgggttac gccatctaca 1020
ccgttggact gtactccaac gaatgcgaca gagctttcga caacagcttg ttccgatttg 1080
ccaccaaggt cttctactat tccaagtttc tggagtacat cgactctttc taccttcccc 1140
tcatggccaa gcctctgtcc tttctgcagt tctttcatca cttgggagct cctatggaca 1200
tgtggctctt cgtgcagtac tctggcgaat ccatttggat ctttgtgttc ctgaacggat 1260
tcattcactt tgtcatgtac ggctactatt ggacacggct gatgaagttc aactttccca 1320
tgcccaagca gctcattacc gcaatgcaga tcacccagtt caacgttggc ttctacctcg 1380
tgtggtggta caaggacatt ccctgttacc gaaaggatcc catgcgaatg ctggcctgga 1440
tcttcaacta ctggtacgtc ggtaccgttc ttctgctctt catcaacttc tttgtcaagt 1500
cctacgtgtt tcccaagcct aagactgccg acaaaaaggt ccagggcgcc ggtcccgctc 1560
gacctgccgg acttcctccc gctacctact acgactctct ggccgtcatg ggatccgtga 1620
aggcttctcg acaggctctg cccctcgtca tcgacggaaa ggtgtacgac gtctccgctt 1680
gggtgaactt ccaccctggt ggagctgaaa tcattgagaa ctaccaggga cgagatgcta 1740
ctgacgcctt catggttatg cactctcagg aagccttcga caagctcaag cgaatgccca 1800
agatcaacca ggcttccgag ctgcctcccc aggctgccgt caacgaagct caggaggatt 1860
tccgaaagct ccgagaagag ctgatcgcca ctggcatgtt tgacgcctct cccctctggt 1920
actcgtacaa gatcttgacc accctgggtc ttggcgtgct tgccttcttc atgctggtcc 1980
agtaccacct gtacttcatt ggtgctctcg tgctcggtat gcactaccag caaatgggat 2040
ggctgtctca tgacatctgc caccaccaga ccttcaagaa ccgaaactgg aataacgtcc 2100
tgggtctggt ctttggcaac ggactccagg gcttctccgt gacctggtgg aaggacagac 2160
acaacgccca tcattctgct accaacgttc agggtcacga tcccgacatt gataacctgc 2220
ctctgctcgc ctggtccgag gacgatgtca ctcgagcttc tcccatctcc cgaaagctca 2280
ttcagttcca acagtactat ttcctggtca tctgtattct cctgcgattc atctggtgtt 2340
tccagtctgt gctgaccgtt cgatccctca aggaccgaga caaccagttc taccgatctc 2400
agtacaagaa agaggccatt ggactcgctc tgcactggac tctcaagacc ctgttccacc 2460
tcttctttat gccctccatc ctgacctcga tgctggtgtt ctttgtttcc gagctcgtcg 2520
gtggcttcgg aattgccatc gtggtcttca tgaaccacta ccctctggag aagatcggtg 2580
attccgtctg ggacggacat ggcttctctg tgggtcagat ccatgagacc atgaacattc 2640
gacgaggcat cattactgac tggttctttg gaggcctgaa ctaccagatc gagcaccatc 2700
tctggcccac cctgcctcga cacaacctca ctgccgtttc ctaccaggtg gaacagctgt 2760
gccagaagca caacctcccc taccgaaacc ctctgcccca tgaaggtctc gtcatcctgc 2820

tccgatacct gtcccagttc gctcgaatgg ccgagaagca gcccggtgcc aaggctcagt 2880
aagcggccgc atgagaagat aaatatataa atacattgag atattaaatg cgctagatta 2940
gagagcctca tactgctcgg agagaagcca agacgagtac tcaaagggga ttacaccatc 3000
catatccaca gacacaagct ggggaaaggt tctatataca ctttccggaa taccgtagtt 3060
tccgatgtta tcaatggggg cagccaggat ttcaggcact tcggtgtctc ggggtgaaat 3120
ggcgttcttg gcctccatca agtcgtacca tgtcttcatt tgcctgtcaa agtaaaacag 3180
aagcagatga agaatgaact tgaagtgaag gaatttaaat gtaacgaaac tgaaatttga 3240
ccagatattg tgtccgcggt ggagctccag cttttgttcc ctttagtgag ggttaatttc 3300
gagcttggcg taatcatggt catagctgtt tcctgtgtga aattgttatc cgctcacaag 3360
cttccacaca acgtacgata gttagtagac aacaatcaga acatctccct ccttatataa 3420
tcacacaggc cagaacgcgc taaactaaag cgctttggac actatgttac attggcattg 3480
attgaactga aaccacagtc tccctcgcct gaatcgagca atggatgttg tcggaagtca 3540
acttcactag aagagcggtt ctatgccttg tcaagatcat atcataaact cactctgtat 3600
taccccatct atagaacact tgttatgaat gggcggaaac attccgctat atgcaccttt 3660
ccacactaat gcaaagatgt gcatcttcaa cgggtagtaa gactggttcc gacttccgtt 3720
gcatggagag caatgacctc gataatgcga acatccccca catatacact cttacacagg 3780
ccaatataat ctgtgcattt actaaatatt taagtctatg cacctgcttg atgaaaagcg 3840
gcacggatgg tatcatctag tttccgccaa tccaagaacc aactgtgttg gcagtggtgt 3900
agcccatggc acacagacca aagatgaaaa tacagacatc ggcggttcga gccgtggtgc 3960
ctcgagcaac acccttgtaa tgcaaaagag gagggtaaat gtacaccaga ggcacacatg 4020
caaacgatcc ggtgagagcg acgaaccgat cgagatcgtc ggcacctccc catgcaacaa 4080
aggcggtgac aaacacaagg aagaaccgga aaatgttctt ctgccacttg atggtagagt 4140
tgtacttgcc tgatcgggtg aagagaccat tctcgatgat tcggatggcg cgccagctgc 4200
attaatgaat cggccaacgc gcggggagag gcggtttgcg tattgggcgc tcttccgctt 4260
cctcgctcac tgactcgctg cgctcggtcg ttcggctgcg gcgagcggta tcagctcact 4320
caaaggcggt aatacggtta tccacagaat caggggataa cgcaggaaag aacatgtgag 4380
caaaaggcca gcaaaaggcc aggaaccgta aaaaggccgc gttgctggcg tttttccata 4440
ggctccgccc ccctgacgag catcacaaaa atcgacgctc aagtcagagg tggcgaaacc 4500
cgacaggact ataaagatac caggcgtttc cccctggaag ctccctcgtg cgctctcctg 4560
ttccgaccct gccgcttacc ggatacctgt ccgcctttct cccttcggga agcgtggcgc 4620
tttctcatag ctcacgctgt aggtatctca gttcggtgta ggtcgttcgc tccaagctgg 4680
gctgtgtgca cgaacccccc gttcagcccg accgctgcgc cttatccggt aactatcgtc 4740

ttgagtccaa cccggtaaga cacgacttat cgccactggc agcagccact ggtaacagga 4800
ttagcagagc gaggtatgta ggcggtgcta cagagttctt gaagtggtgg cctaactacg 4860
gctacactag aagaacagta tttggtatct gcgctctgct gaagccagtt accttcggaa 4920
aaagagttgg tagctcttga tccggcaaac aaaccaccgc tggtagcggt ggtttttttg 4980
tttgcaagca gcagattacg cgcagaaaaa aaggatctca agaagatcct ttgatctttt 5040
ctacggggtc tgacgctcag tggaacgaaa actcacgtta agggattttg gtcatgagat 5100
tatcaaaaag gatcttcacc tagatccttt taaattaaaa atgaagtttt aaatcaatct 5160
aaagtatata tgagtaaact tggtctgaca gttaccaatg cttaatcagt gaggcaccta 5220
tctcagcgat ctgtctattt cgttcatcca tagttgcctg actccccgtc gtgtagataa 5280
ctacgatacg ggagggctta ccatctggcc ccagtgctgc aatgataccg cgagacccac 5340
gctcaccggc tccagattta tcagcaataa accagccagc cggaagggcc gagcgcagaa 5400
gtggtcctgc aactttatcc gcctccatcc agtctattaa ttgttgccgg gaagctagag 5460
taagtagttc gccagttaat agtttgcgca acgttgttgc cattgctaca ggcatcgtgg 5520
tgtcacgctc gtcgtttggt atggcttcat tcagctccgg ttcccaacga tcaaggcgag 5580
ttacatgatc ccccatgttg tgcaaaaaag cggttagctc cttcggtcct ccgatcgttg 5640
tcagaagtaa gttggccgca gtgttatcac tcatggttat ggcagcactg cataattctc 5700
ttactgtcat gccatccgta agatgctttt ctgtgactgg tgagtactca accaagtcat 5760
tctgagaata gtgtatgcgg cgaccgagtt gctcttgccc ggcgtcaata cgggataata 5820
ccgcgccaca tagcagaact ttaaaagtgc tcatcattgg aaaacgttct tcggggcgaa 5880
aactctcaag gatcttaccg ctgttgagat ccagttcgat gtaacccact cgtgcaccca 5940
actgatcttc agcatctttt actttcacca gcgtttctgg gtgagcaaaa acaggaaggc 6000
aaaatgccgc aaaaaaggga ataagggcga cacggaaatg ttgaatactc atactcttcc 6060
tttttcaata ttattgaagc atttatcagg gttattgtct catgagcgga tacatatttg 6120
aatgtattta gaaaaataaa caaatagggg ttccgcgcac atttccccga aaagtgccac 6180
ctgatgcggt gtgaaatacc gcacagatgc gtaaggagaa aataccgcat caggaaattg 6240
taagcgttaa tattttgtta aaattcgcgt taaatttttg ttaaatcagc tcatttttta 6300
accaataggc cgaaatcggc aaaatccctt ataaatcaaa agaatagacc gagatagggt 6360
tgagtgttgt tccagtttgg aacaagagtc cactattaaa gaacgtggac tccaacgtca 6420
aagggcgaaa aaccgtctat cagggcgatg gcccactacg tgaaccatca ccctaatcaa 6480
gtttttgggg gtcgaggtgc cgtaaagcac taaatcggaa ccctaaaggg agcccccgat 6540
ttagagcttg acggggaaag ccggcgaacg tggcgagaaa ggaagggaag aaagcgaaag 6600
gagcgggcgc tagggcgctg gcaagtgtag cggtcacgct gcgcgtaacc accacacccg 6660
ccgcgcttaa tgcgccgcta cagggcgcgt ccattcgcca ttcaggctgc gcaactgttg 6720

ggaagggcga tcggtgcggg cctcttcgct attacgccag ctggcgaaag ggggatgtgc 6780
tgcaaggcga ttaagttggg taacgccagg gttttcccag tcacgacgtt gtaaaacgac 6840
ggccagtgaa ttgtaatacg actcactata gggcgaattg ggcccgacgt cgcatgctta 6900
gaagtgagga ttacaagaag cctctggata tcaatgatga acgtactcag cggctggtca 6960
agcatttcga ccgtcgaatc gacgaggtgt tcacctttga caagcgaggg ttcccaattg 7020
atcacgttct cgagttgttc aaatcttctc tcaacatctc tctgcatgaa ctatctctgt 7080
tgacgaacgt gtcacccact gttcctcgaa cgcccttctc cgagtttggt ctgaacatct 7140
tcgatctcaa actgaccccc gcagtgatca atagtgccat gccactgccg atgcggtgcg 7200
aacatccctg gagggattct cggagctcta cacaatgcag attctgtcgt cgagtactct 7260
ctaccttgct cgaatgactt attgtgctac tactgcactc atgcttcgat catgtgccct 7320
actgcacccc aaatttggtg atctgattga gacagagtac cctcttcagc tgattcagaa 7380
gatcatcagc aacatgaatg atgtggttga ccaggcaggc tgttgtagtc acgtccttca 7440
cttcaagttc attcttcatc tgcttctgtt ttactttgac aggcaaatga agacatggta 7500
cgacttgatg gaggccaaga acgccatttc accccgagac accgaagtgc ctgaaatcct 7560
ggctgccccc attgataaca tcggaaacta cggtattccg gaaagtgtat atagaacctt 7620
tccccagctt gtgtctgtgg atatggatgg tgtaatcccc ttaattaact cacctgcagg 7680
attgagacta tgaatggatt cccgtgcccg tattactcta ctaatttgat cttggaacgc 7740
gaaaatacgt ttctaggact ccaaagaatc tcaactcttg tccttactaa atatactacc 7800
catagttgat ggtttacttg aacagagagg acatgttcac ttgacccaaa gtttctcgca 7860
tctcttggat atttgaacaa cggcgtccac tgaccgtcag ttatccagtc acaaaacccc 7920
cacattcata cattcccatg tacgtttaca aagttctcaa ttccatcgtg caaatcaaaa 7980
tcacatctat tcattcatca tatataaacc catcatgtct actaacactc acaactccat 8040
agaaaacatc gactcagaac acacgctcca tgcggccgct taggcaacgg gcttgatgac 8100
agcgggagga gtgcccacat tgtttcggtt tcgaaagaac aggacaccct tgccagctcc 8160
ctcggcacca gcggagggtt caacccactg gcacattcgt gcagatcggt acatggctcg 8220
aatgaatcct cgaggaccgt cctggacatc agctcgatag tgcttgccca tgataggttt 8280
gatggcctcg gtagcttcgt ccgcattgta gaagggaatg gaagagacgt agtgatgcag 8340
gacgtgagtc tcgataatgc cgtggagcag atgacgtcca atgaagccca tctctcggtc 8400
gatggttgca gcggcacctc gcacaaagtt ccactcgtcg ttggtgtagt ggggaagagt 8460
aggatctgtg tgctgcagaa aggtaatggc gacgagccag tggttaaccc acaagtaggg 8520
aacgaagtac cagatggcca tgttgtagaa tccgaacttc tgaacgagaa agtacagagc 8580
ggtggccata agaccaatgc caatgtcgga gagcacgatg agcttggcgt cgctgttctc 8640

gtacagagga gatcggggat cgaaatggtt aactccaccg ccaagaccgt tgtgctttcc 8700
cttgcctcga ccctctcgct gccgctcatg gtagttgtgt ccagtaacgt tggtaatgag 8760
atagttgggc caaccgacca gttgctgaag cacaagcatg agcagggtga aagcaggagt 8820
ttcctcggta agatgggcga gttcgtgggt catcttgccg agtcgagtag cttgctgctc 8880
tcgggttcga ggaacgaaga ccatgtctcg ctccatgttt ccagtggcct tgtgatgctt 8940
ccggtgggag atttgccagc tgaagtaggg aacaagcagg gaagagtgaa gcacccagcc 9000
agtaatgtcg ttgatgattc gggaatcgga gaaagcacca tgtccacact cgtgggcaat 9060
gacccacagt ccagtaccga agagtccctg aagaacggtg tacacagccc acagaccggc 9120
tcgagcagga gtggagggaa tgtactcggg tgtcacaaag ttgtaccaga tgctgaaagt 9180
ggtagtcagg aggacaatgt ctcgaagaat gtagccgtat cccttgagag cagatcgctt 9240
gaagcagtgc ttgggaatag cgttgtagat gtccttgatg gtgaagtcgg gaacttcgaa 9300
ctggttgccg taggtatcca gcatgacacc gtactcggac ttgggcttgg caatgtccac 9360
ctcggacatg gaagacagcg atgtagagga ggccgagtgt ctgggagaat cggagggaga 9420
gacggcagca gactccgagt cggtcacagt ggtggaagtg acggttcgtc ggagggcagg 9480
gttctgcttg ggcagagccg aggtggaggc catggccatt gctgtagata tgtcttgtgt 9540
gtaagggggt tggggtggtt gtttgtgttc ttgacttttg tgttagcaag ggaagacggg 9600
caaaaaagtg agtgtggttg ggagggagag acgagcctta tatataatgc ttgtttgtgt 9660
ttgtgcaagt ggacgccgaa acgggcagga gccaaactaa acaaggcaga caatgcgagc 9720
ttaattggat tgcctgatgg gcaggggtta gggctcgatc aatgggggtg cgaagtgaca 9780
aaattgggaa ttaggttcgc aagcaaggct gacaagactt tggcccaaac atttgtacgc 9840
ggtggacaac aggagccacc catcgtctgt cacgggctag ccggtcgtgc gtcctgtcag 9900
gctccaccta ggctccatgc cactccatac aatcccacta gtgtaccgct aggccgcttt 9960
tagctcccat ctaagacccc cccaaaacct ccactgtaca gtgcactgta ctgtgtggcg 10020
atcaagggca agggaaaaaa ggcgcaaaca tgcacgcatg gaatgacgta ggtaaggcgt 10080
tactagactg aaaagtggca catttcggcg tgccaaaggg tcctaggtgc gtttcgcgag 10140
ctgggcgcca ggccaagccg ctccaaaacg cctctccgac tccctccagc ggcctccata 10200
tccccatccc tctccacagc aatgttgtta agccttgcaa acgaaaaaat agaaaggcta 10260
ataagcttcc aatattgtgg tgtacgctgc ataacgcaac aatgagcgcc aaacaacaca 10320
cacacacagc acacagcagc attaaccacg atgaacagca tgaattctct ctcttgagct 10380
tttccataac aagttcttct gcctccagga agtccatggg tggtttgatc atggttttgg 10440
tgtagtggta gtgcagtggt ggtattgtga ctggggatgt agttgagaat aagtcataca 10500
caagtcagct ttcttcgagc ctcatataag tataagtagt tcaacgtatt agcactgtac 10560
ccagcatctc cgtatcgaga aacacaacaa catgccccat tggacagatc atgcggatac 10620

acaggttgtg cagtatcata catactcgat cagacaggtc gtctgaccat catacaagct 10680
gaacaagcgc tccatacttg cacgctctct atatacacag ttaaattaca tatccatagt 10740
ctaacctcta acagttaatc ttctggtaag cctcccagcc agccttctgg tatcgcttgg 10800
cctcctcaat aggatctcgg ttctggccgt acagacctcg gccgacaatt atgatatccg 10860
ttccggtaga catgacatcc tcaacagttc ggtactgctg tccgagagcg tctcccttgt 10920
cgtcaagacc caccccgggg gtcagaataa gccagtcctc agagtcgccc ttaggtcggt 10980
tctgggcaat gaagccaacc acaaactcgg ggtcggatcg ggcaagctca atggtctgct 11040
tggagtactc gccagtggcc agagagccct tgcaagacag ctcggccagc atgagcagac 11100
ctctggccag cttctcgttg ggagagggga ctaggaactc cttgtactgg gagttctcgt 11160
agtcagagac gtcctccttc ttctgttcag agacagtttc ctcggcacca gctcgcaggc 11220
cagcaatgat tccggttccg ggtacaccgt gggcgttggt gatatcggac cactcggcga 11280
ttcggtgaca ccggtactgg tgcttgacag tgttgccaat atctgcgaac tttctgtcct 11340
cgaacaggaa gaaaccgtgc ttaagagcaa gttccttgag gggagcaca gtgccggcgt 11400
aggtgaagtc gtcaatgatg tcgatatggg ttttgatcat gcacacataa ggtccgacct 11460
tatcggcaag ctcaatgagc tccttggtgg tggtaacatc cagagaagca cacaggttgg 11520
ttttcttggc tgccacgagc ttgagcactc gagcggcaaa ggcggacttg tggacgttag 11580
ctcgagcttc gtaggagggc attttggtgg tgaagaggag actgaaataa atttagtctg 11640
cagaactttt tatcggaacc ttatctgggg cagtgaagta tatgttatgg taatagttac 11700
gagttagttg aacttataga tagactggac tatacggcta tcggtccaaa ttagaaagaa 11760
cgtcaatggc tctctgggcg tcgcctttgc cgacaaaaat gtgatcatga tgaaagccag 11820
caatgacgtt gcagctgata ttgttgtcgg ccaaccgcgc cgaaaacgca gctgtcagac 11880
ccacagcctc caacgaagaa tgtatcgtca aagtgatcca agcacactca tagttggagt 11940
cgtactccaa aggcggcaat gacgagtcag acagatactc gtcgaccatt aattctcacg 12000
tgacacagat tattaacgtc tcgtaccaac cacagattac gacccattcg cagtcacagt 12060
tcactagggt ttgggttgca tccgttgaga gcggtttgtt tttaaccttc tccatgtgct 12120
cactcaggtt ttgggttcag atcaaatcaa ggcgtgaacc actttgtttg aggacaaatg 12180
tgacacaacc aaccagtgtc aggggcaagt ccgtgacaaa ggggaagata caatgcaatt 12240
actgacagtt acagactgcc tcgatgccct aaccttgccc caaaataaga caactgtcct 12300
cgtttaagcg caaccctatt cagcgtcacg tcataatagc gtttggatag cactagtcta 12360
tgaggagcgt tttatgttgc ggtgagggcg attggtgctc atatgggttc aattgaggtg 12420
gcggaacgag cttagtcttc aattgaggtg cgagcgacac aattgggtgt cacgtggcct 12480
aattgacctc gggtcgtgga gtccccagtt atacagcaac cacgaggtgc atgggtagga 12540

gacgtcacca gacaataggg ttttttttgg actggagagg gttgggcaaa agcgctcaac 12600
gggctgtttg gggagctgtg ggggaggaat tggcgatatt tgtgaggtta acggctccga 12660
tttgcgtgtt ttgtcgctcc tgcatctccc catacccata tcttccctcc ccacctcttt 12720
ccacgataat tttacggatc agcaataagg ttccttctcc tagtttccac gtccatatat 12780
atctatgctg cgtcgtcctt ttcgtgacat caccaaaaca catacaacca tggctctctc 12840
ccttactacc gagcagctgc tcgagcgacc cgacctggtt gccatcgacg gcattctcta 12900
cgatctggaa ggtcttgcca aggtccatcc cggatccgac ttgatcctcg cttctggtgc 12960
ctccgatgct tctcctctgt tctactccat gcacccttac gtcaagcccg agaactcgaa 13020
gctgcttcaa cagttcgtgc gaggcaagca cgaccgaacc tccaaggaca ttgtctacac 13080
ctacgactct ccctttgcac aggacgtcaa gcgaactatg cgagaggtca tgaaaggtcg 13140
gaactggtat gccacacctg gattctggct gcgaaccgtt ggcatcattg ctgtcaccgc 13200
cttttgcgag tggcactggg ctactaccgg aatggtgctg tggggtctct tgactggatt 13260
catgcacatg cagatcggcc tgtccattca gcacgatgcc tctcatggtg ccatcagcaa 13320
aaagccctgg gtcaacgctc tctttgccta cggcatcgac gtcattggat cgtccagatg 13380
gatctggctg cagtctcaca tcatgcgaca tcacacctac accaatcagc atggtctcga 13440
cctggatgcc gagtccgcag aaccattcct tgtgttccac aactaccctg ctgccaacac 13500
tgctcgaaag tggtttcacc gattccaggc ctggtacatg tacctcgtgc ttggagccta 13560
cggcgtttcg ctggtgtaca accctctcta catcttccga atgcagcaca acgacaccat 13620
tcccgagtct gtcacagcca tgcgagagaa cggctttctg cgacggtacc gaacccttgc 13680
attcgttatg cgagctttct tcatctttcg aaccgccttc ttgccctggt atctcactgg 13740
aacctccctg ctcatcacca ttcctctggt gcccactgct accggtgcct tcctcacctt 13800
ctttttcatc ttgtctcaca acttcgatgg ctcggagcga atccccgaca agaactgcaa 13860
ggtcaagagc tccgagaagg acgttgaagc cgatcagatc gactggtaca gagctcaggt 13920
ggagacctct tccacctacg gtggacccat tgccatgttc tttactggcg gtctcaactt 13980
ccagatcgag catcacctct ttcctcgaat gtcgtcttgg cactatccct tcgtgcagca 14040
agctgtccga gagtgttgcg aacgacacgg agttcggtac gtcttctacc ctaccattgt 14100
gggcaacatc atttccaccc tcaagtacat gcacaaagtc ggtgtggttc actgtgtcaa 14160
ggacgctcag gattcctaag cggccgcatg tacatacaag attatttata gaaatgaatc 14220
gcgatcgaac aaagagtacg agtgtacgag taggggatga tgataaaagt ggaagaagtt 14280
ccgcatcttt ggatttatca acgtgtagga cgatacttcc tgtaaaaatg caatgtcttt 14340
accataggtt ctgctgtaga tgttattaac taccattaac atgtctactt gtacagttgc 14400
agaccagttg gagtatagaa tggtacactt accaaaaagt gttgatggtt gtaactacga 14460
tatataaaac tgttgacggg atccccgctg atatgcctaa ggaacaatca aagaggaaga 14520

tattaattca gaatgctagt atacagttag ggat 14554

<210> 133
<211> 15119
<212> DNA
<213> Artificial Sequence

<220>
<223> Plasmid pZSCP-Ma83

<400> 133

gtacgacccc tctcaggcca agcagaaggc tgagtccatc aagaaggcca acgctatcat 60
tgtcttcaac ctcaagaaca aggctggcaa gaccgagtct tggtaccttg acctcaagaa 120
cgacggtgac gtcggcaagg gcaacaagtc ccccaagggt gatgctgaca tccagctcac 180
tctctctgac gaccacttcc agcagctcgt tgagggtaag gctaacgccc agcgactctt 240
catgaccggc aagctcaagg ttaagggcaa cgtcatgaag gctgccgcca ttgagggtat 300
cctcaagaac gctcagaaca acctctaagc gcatcattta ttgattaatt gatgatttac 360
tatattgatt tcgcaactgt agtgtgattg tatgtgatct ggctcgtagg cttcagtaaa 420
tactagacgg gtatcctacg tagttgtatc atacatcgag cctgtggtta cttgtacaat 480
aattcgtaat gtagagatac cccttgatcc attgcctgtt tctaacatac aatgatctcc 540
acgcaataat cccactcttg actaaaagtt gctactcttg cacggttacc tcggcatagt 600
cacgcctctc ttgtctcgtc tcgaacgcac aaagtcaatt gacaacgcca ctcactcgag 660
tgtgccccaa cagggcacca tatcgactaa tttgaggcca actagggtga ttttggatgg 720
aatttgatcg gaaaaaatag ctgcagaaat tcctggagag aaaaattgac cgcatccaca 780
tggtttgacc aaaaaatcgt ctccatctct gtgctcaact ctcctgacga gatatgcgcg 840
cgcacccccca catgatgtga ttgatctcaa caaacttcac ccagaccctt atctttccgg 900
gaaacttact gtataagtgg tcgtgcgaac agaaagtgtg cgcactttag gtgtctagat 960
ccgattgttc tcgttctgat aatgagccag ccccgcgagg caatgttttt tacaattgaa 1020
aacttcgtta accactcaca ttaccgtttt tgccccatat ttaccctctg gtacactccc 1080
tcttgcatac acacacactg cagtgaaaat gcactccgtt agcaccgttg tgattggttc 1140
agggcacgag tttggtggtt taaggcgcaa ctacatcaat atgaaaacag gagacgctga 1200
aaaggggtaa tatcggactg ctgctatgtt gtatgtactg catgacgaat tggtgttatt 1260
caagaccgtg gcacaggttg ctgcggtacg agacctggta gcttctctaa acggcatgtc 1320
taggtggcgc gccagctgca ttaatgaatc ggccaacgcg cggggagagg cggtttgcgt 1380
attgggcgct cttccgcttc ctcgctcact gactcgctgc gctcggtcgt tcggctgcgg 1440
cgagcggtat cagctcactc aaaggcggta atacggttat ccacagaatc aggggataac 1500
gcaggaaaga acatgtgagc aaaaggccag caaaaggcca ggaaccgtaa aaaggccgcg 1560

ttgctggcgt ttttccatag gctccgcccc cctgacgagc atcacaaaaa tcgacgctca 1620
agtcagaggt ggcgaaaccc gacaggacta taaagatacc aggcgtttcc ccctggaagc 1680
tccctcgtgc gctctcctgt tccgaccctg ccgcttaccg gatacctgtc cgcctttctc 1740
ccttcgggaa gcgtggcgct ttctcatagc tcacgctgta ggtatctcag ttcggtgtag 1800
gtcgttcgct ccaagctggg ctgtgtgcac gaaccccccg ttcagcccga ccgctgcgcc 1860
ttatccggta actatcgtct tgagtccaac ccggtaagac acgacttatc gccactggca 1920
gcagccactg gtaacaggat tagcagagcg aggtatgtag gcggtgctac agagttcttg 1980
aagtggtggc ctaactacgg ctacactaga agaacagtat ttggtatctg cgctctgctg 2040
aagccagtta ccttcggaaa aagagttggt agctcttgat ccggcaaaca aaccaccgct 2100
ggtagcggtg gtttttttgt ttgcaagcag cagattacgc gcagaaaaaa aggatctcaa 2160
gaagatcctt tgatcttttc tacggggtct gacgctcagt ggaacgaaaa ctcacgttaa 2220
gggattttgg tcatgagatt atcaaaaagg atcttcacct agatcctttt aaattaaaaa 2280
tgaagtttta aatcaatcta aagtatatat gagtaaactt ggtctgacag ttaccaatgc 2340
ttaatcagtg aggcacctat ctcagcgatc tgtctatttc gttcatccat agttgcctga 2400
ctccccgtcg tgtagataac tacgatacgg gagggcttac catctggccc cagtgctgca 2460
atgataccgc gagacccacg ctcaccggct ccagatttat cagcaataaa ccagccagcc 2520
ggaagggccg agcgcagaag tggtcctgca actttatccg cctccatcca gtctattaat 2580
tgttgccggg aagctagagt aagtagttcg ccagttaata gtttgcgcaa cgttgttgcc 2640
attgctacag gcatcgtggt gtcacgctcg tcgtttggta tggcttcatt cagctccggt 2700
tcccaacgat caaggcgagt tacatgatcc cccatgttgt gcaaaaaagc ggttagctcc 2760
ttcggtcctc cgatcgttgt cagaagtaag ttggccgcag tgttatcact catggttatg 2820
gcagcactgc ataattctct tactgtcatg ccatccgtaa gatgcttttc tgtgactggt 2880
gagtactcaa ccaagtcatt ctgagaatag tgtatgcggc gaccgagttg ctcttgcccg 2940
gcgtcaatac gggataatac cgcgccacat agcagaactt taaaagtgct catcattgga 3000
aaacgttctt cggggcgaaa actctcaagg atcttaccgc tgttgagatc cagttcgatg 3060
taacccactc gtgcacccaa ctgatcttca gcatctttta ctttcaccag cgtttctggg 3120
tgagcaaaaa caggaaggca aaatgccgca aaaaagggaa taagggcgac acggaaatgt 3180
tgaatactca tactcttcct ttttcaatat tattgaagca tttatcaggg ttattgtctc 3240
atgagcggat acatatttga atgtatttag aaaaataaac aaataggggt tccgcgcaca 3300
tttccccgaa aagtgccacc tgatgcggtg tgaaataccg cacagatgcg taaggagaaa 3360
ataccgcatc aggaaattgt aagcgttaat attttgttaa aattcgcgtt aaatttttgt 3420
taaatcagct cattttttaa ccaataggcc gaaatcggca aaatccctta taaatcaaaa 3480
gaatagaccg agatagggtt gagtgttgtt ccagtttgga acaagagtcc actattaaag 3540

aacgtggact ccaacgtcaa agggcgaaaa accgtctatc agggcgatgg cccactacgt 3600
gaaccatcac cctaatcaag ttttttgggg tcgaggtgcc gtaaagcact aaatcggaac 3660
cctaaaggga gcccccgatt tagagcttga cggggaaagc cggcgaacgt ggcgagaaag 3720
gaagggaaga aagcgaaagg agcgggcgct agggcgctgg caagtgtagc ggtcacgctg 3780
cgcgtaacca ccacacccgc cgcgcttaat gcgccgctac agggcgcgtc cattcgccat 3840
tcaggctgcg caactgttgg gaagggcgat cggtgcgggc ctcttcgcta ttacgccagc 3900
tggcgaaagg gggatgtgct gcaaggcgat taagttgggt aacgccaggg ttttcccagt 3960
cacgacgttg taaaacgacg gccagtgaat tgtaatacga ctcactatag ggcgaattgg 4020
gcccgacgtc gcatgcgtca ctaatcaagg atacctacca tgccactatg atgtttgcag 4080
gaggtgtacc tcggcagtca tcaaaaaatg gaactactgg ctttagatct tgttgtatgg 4140
catcgcgcct aaaaaagaaa ccccccttcca gcgagctact acaagtagtt gtagttgcgg 4200
gcgttggata ccgaaagtca caagcacatg tcgaagctct catctgaaac accgacagtc 4260
gtctgcaccc cgcaagtctc ggttcgtacc agcaccaatg ttaggcagaa ctatacacaa 4320
gagggcggac gatcacttcg gcgttaggca actgaaggct attttcggct ggtactgtag 4380
gggacagagg aaacgcaagt gattagtaaa tcggataata ggcctgttag tttaccgaaa 4440
tggtggggga ggggttccgt ggatatcttg aagttatgga ggctgatcgt tatttgtggg 4500
gatggatatc attgtatgga catactgtag ctactgtata aacaacggat cttacacctg 4560
cctcttgtat gcccattgct tgatcatcta tcgtgttact gtacatatac aatagatata 4620
gggaagaaaa gccggaagta gagaccatag tctggcagaa gtaacggcct cgggtcgaga 4680
gaactataac aaagtccaac ggcgggtctt agaatagccc caaggatcac acagttccgc 4740
aatccagttt cacatgttcc gttgcatgga cttttgcatg tctactgttg ctacgattcc 4800
cccattgcaa ccacagtttg ggttaccccc gcattatatt agcatgatta cgaaagagat 4860
aagtatcata tggaacatgt gaagggtagt atgcaggtcc ggcggagaaa gagaatgacg 4920
ttttcattaa gcgattcgct tggcggcttg tgggggatgt gacgatactt acggtaaaga 4980
ccctgtgtga gagctggtac tcgctcgtta cttcgctgat ctgttgggcc gtcaatcgaa 5040
tctcgtggaa cttgcattct tcttaactgt gtctatacaa gacacctaat gaaacataca 5100
agctaccgaa atcattttac tcgtactgac cggtacggta cttgcacaag tagtgaaact 5160
tccgaaaata gccagcctca tgcatcatcg cttcacccct tctgttgacc tcaaaagcat 5220
tccaacggta aaaaattata acgccgccaa ctggatggtt gtgacggcgt tgaccaccaa 5280
tgtgtggggg ctggcggtag gaccgagctt attcgtccca ataagctctt tggatttgat 5340
tctttggggt gtgtggtaaa attcacatgg ggaagaacac ggtggcagtt tgaggcagag 5400
gcccagcgtg tagttcctag ggcatgaata taccgaactc atggcgcaga attgagctga 5460

atgcgcaaaa agctacagga tcaaccgcgt tagaaatgcc gcaaatgtcc actaattccc 5520
cggactgttc caaatgattc tgtggggata aatctcaaac tgggttaggc tttgtcacgt 5580
ttctttgtgt cgtgtcggtt cgtccggggc aatgtgccca cgcttggctg tctccctaca 5640
cctcggtaaa aactatcaca tgctgcccct ctcgagcaag cattaaatgc atatagtcaa 5700
tctaacgaca tatatatagg tagggtgcat cctccggttt agctccccag aatatctctt 5760
attcattaca caaaaacaac aatgtctctc aaggtcgacg gcttcacttc ttaattaagt 5820
tgcgacacat gtcttgatag tatcttgaat tctctctctt gagcttttcc ataacaagtt 5880
cttctgcctc caggaagtcc atgggtggtt tgatcatggt tttggtgtag tggtagtgca 5940
gtggtggtat tgtgactggg gatgtagttg agaataagtc atacacaagt cagctttctt 6000
cgagcctcat ataagtataa gtagttcaac gtattagcac tgtacccagc atctccgtat 6060
cgagaaacac aacaacatgc cccattggac agatcatgcg gatacacagg ttgtgcagta 6120
tcatacatac tcgatcagac aggtcgtctg accatcatac aagctgaaca agcgctccat 6180
acttgcacgc tctctatata cacagttaaa ttacatatcc atagtctaac ctctaacagt 6240
taatcttctg gtaagcctcc cagccagcct tctggtatcg cttggcctcc tcaataggat 6300
ctcggttctg gccgtacaga cctcggccga caattatgat atccgttccg gtagacatga 6360
catcctcaac agttcggtac tgctgtccga gagcgtctcc cttgtcgtca agacccaccc 6420
cgggggtcag aataagccag tcctcagagt cgcccttagg tcggttctgg gcaatgaagc 6480
caaccacaaa ctcggggtcg gatcgggcaa gctcaatggt ctgcttggag tactcgccag 6540
tggccagaga gcccttgcaa gacagctcgg ccagcatgag cagacctctg gccagcttct 6600
cgttgggaga ggggactagg aactccttgt actgggagtt ctcgtagtca gagacgtcct 6660
ccttcttctg ttcagagaca gtttcctcgg caccagctcg caggccagca atgattccgg 6720
ttccgggtac accgtgggcg ttggtgatat cggaccactc ggcgattcgg tgacaccggt 6780
actggtgctt gacagtgttg ccaatatctg cgaactttct gtcctcgaac aggaagaaac 6840
cgtgcttaag agcaagttcc ttgaggggga gcacagtgcc ggcgtaggtg aagtcgtcaa 6900
tgatgtcgat atgggttttg atcatgcaca cataaggtcc gaccttatcg gcaagctcaa 6960
tgagctcctt ggtggtggta acatccagag aagcacacag gttggttttc ttggctgcca 7020
cgagcttgag cactcgagcg gcaaaggcgg acttgtggac gttagctcga gcttcgtagg 7080
agggcatttt ggtggtgaag aggagactga aataaattta gtctgcagaa ctttttatcg 7140
gaaccttatc tggggcagtg aagtatatgt tatggtaata gttacgagtt agttgaactt 7200
atagatagac tggactatac ggctatcggt ccaaattaga aagaacgtca atggctctct 7260
gggcgtcgcc tttgccgaca aaaatgtgat catgatgaaa gccagcaatg acgttgcagc 7320
tgatattgtt gtcggccaac cgcgccgaaa acgcagctgt cagacccaca gcctccaacg 7380
aagaatgtat cgtcaaagtg atccaagcac actcatagtt ggagtcgtac tccaaaggcg 7440

gcaatgacga gtcagacaga tactcgtcga ccttttcctt gggaaccacc accgtcagcc 7500
cttctgactc acgtattgta gccaccgaca caggcaacag tccgtggata gcagaatatg 7560
tcttgtcggt ccatttctca ccaactttag gcgtcaagtg aatgttgcag aagaagtatg 7620
tgccttcatt gagaatcggt gttgctgatt tcaataaagt cttgagatca gtttggccag 7680
tcatgttgtg gggggtaatt ggattgagtt atcgcctaca gtctgtacag gtatactcgc 7740
tgcccacttt atacttttg attccgctgc acttgaagca atgtcgttta ccaaaagtga 7800
gaatgctcca cagaacacac cccagggtat ggttgagcaa aaaataaaca ctccgatacg 7860
gggaatcgaa ccccggtctc cacggttctc aagaagtatt cttgatgaga gcgtatcgat 7920
ggaagccggt agaaccgggc tgcttgtgct tggagatgga agccggtaga accgggctgc 7980
ttggggggat ttggggccgc tgggctccaa agaggggtag gcatttcgtt ggggttacgt 8040
aattgcggca tttgggtcct gcgcgcatgt cccattggtc agaattagtc cggataggag 8100
acttatcagc caatcacagc gccggatcca cctgtaggtt gggttgggtg ggagcacccc 8160
tccacagagt agagtcaaac agcagcagca acatgatagt tgggggtgtg cgtgttaaag 8220
gaaaaaaaag aagcttgggt tatattcccg ctctatttag aggttgcggg atagacgccg 8280
acggagggca atggcgctat ggaaccttgc ggatatccat acgccgcggc ggactgcgtc 8340
cgaaccagct ccagcagcgt tttttccggg ccattgagcc gactgcgacc ccgccaacgt 8400
gtcttggccc acgcactcat gtcatgttgg tgttgggagg ccacttttta agtagcacaa 8460
ggcacctagc tcgcagcaag gtgtccgaac caaagaagcg gctgcagtgg tgcaaacggg 8520
gcggaaacgg cgggaaaaag ccacgggggc acgaattgag gcacgccctc gaatttgaga 8580
cgagtcacgg ccccattcgc ccgcgcaatg gctcgccaac gcccggtctt ttgcaccaca 8640
tcaggttacc ccaagccaaa cctttgtgtt aaaaagctta acatattata ccgaacgtag 8700
gtttgggcgg gcttgctccg tctgtccaag gcaacattta tataagggtc tgcatcgccg 8760
gctcaattga atctttttc ttcttctctt ctctatattc attcttgaat taaacacaca 8820
tcaaccatgg tcaagcgacc cgctctgcct ctcaccgtgg acggtgtcac ctacgacgtt 8880
tctgcctggc tcaaccacca tcccggaggt gccgacatta tcgagaacta ccgaggtcgg 8940
gatgctaccg acgtcttcat ggttatgcac tccgagaacg ccgtgtccaa actcagacga 9000
atgcccatca tggaaccttc ctctcccctg actccaacac ctcccaagcc aaactccgac 9060
gaacctcagg aggatttccg aaagctgcga gacgagctca ttgctgcagg catgttcgat 9120
gcctctccca tgtggtacgc ttacaagacc ctgtcgactc tcggactggg tgtccttgcc 9180
gtgctgttga tgacccagtg gcactggtac ctggttggtg ctatcgtcct cggcattcac 9240
tttcaacaga tgggatggct ctcgcacgac atttgccatc accagctgtt caaggaccga 9300
tccatcaaca atgccattgg cctgctcttc ggaaacgtgc ttcagggctt ttctgtcact 9360

tggtggaagg accgacacaa cgctcatcac tccgccacca acgtgcaggg tcacgatccc 9420
gacatcgaca acctgcctct cctggcgtgg tccaaggagg acgtcgagcg agctggcccg 9480
ttttctcgac ggatgatcaa gtaccaacag tattacttct ttttcatctg tgcccttctg 9540
cgattcatct ggtgctttca gtccattcat actgccacgg gtctcaagga tcgaagcaat 9600
cagtactatc gaagacagta cgagaaggag tccgtcggtc tggcactcca ctggggtctc 9660
aaggccttgt tctactattt ctacatgccc tcgtttctca ccggactcat ggtgttcttt 9720
gtctccgagc tgcttggtgg cttcggaatt gccatcgttg tcttcatgaa ccactaccct 9780
ctggagaaga ttcaggactc cgtgtgggat ggtcatggct tctgtgctgg acagattcac 9840
gagaccatga acgttcagcg aggcctcgtc acagactggt ttttcggtgg cctcaactac 9900
cagatcgaac atcacctgtg gcctactctt cccagacaca acctcaccgc tgcctccatc 9960
aaagtggagc agctgtgcaa gaagcacaac ctgccctacc gatctcctcc catgctcgaa 10020
ggtgtcggca ttcttatctc ctacctgggc accttcgctc gaatggttgc caaggcagac 10080
aaggcctaag cggccgcatt gatgattgga aacacacaca tgggttatat ctaggtgaga 10140
gttagttgga cagttatata ttaaatcagc tatgccaacg gtaacttcat tcatgtcaac 10200
gaggaaccag tgactgcaag taatatagaa tttgaccacc ttgccattct cttgcactcc 10260
tttactatat ctcatttatt tcttatatac aaatcacttc ttcttcccag catcgagctc 10320
ggaaacctca tgagcaataa catcgtggat ctcgtcaata gagggctttt tggactcctt 10380
gctgttggcc accttgtcct tgctgtttaa acagagtgtg aaagactcac tatggtccgg 10440
gcttatctcg accaatagcc aaagtctgga gtttctgaga gaaaaaggca agatacgtat 10500
gtaacaaagc gacgcatggt acaataatac cggaggcatg tatcatagag agttagtggt 10560
tcgatgatgg cactggtgcc tggtatgact ttatacggct gactacatat ttgtcctcag 10620
acatacaatt acagtcaagc acttaccctt ggacatctgt aggtaccccc cggccaagac 10680
gatctcagcg tgtcgtatgt cggattggcg tagctccctc gctcgtcaat tggctcccat 10740
ctactttctt ctgcttggct acacccagca tgtctgctat ggctcgtttt cgtgccttat 10800
ctatcctccc agtattacca actctaaatg acatgatgtg attgggtcta cactttcata 10860
tcagagataa ggagtagcac agttgcataa aaagcccaac tctaatcagc ttcttccttt 10920
cttgtaatta gtacaaaggt gattagcgaa atctggaagc ttagttggcc ctaaaaaaat 10980
caaaaaaagc aaaaaacgaa aaacgaaaaa ccacagtttt gagaacaggg aggtaacgaa 11040
ggatcgtata tatatatata tatatatata cccacggatc ccgagaccgg cctttgattc 11100
ttccctacaa ccaaccattc tcaccaccct aattcacaac catggtctcc aaccacctgt 11160
tcgacgccat gcgagctgcc gctcccggag acgcaccttt cattcgaatc gacaacgctc 11220
ggacctggac ttacgatgac gccattgctc tttccggtcg aatcgctgga gctatggacg 11280
cactcggcat tcgacccgga gacagagttg ccgtgcaggt cgagaagtct gccgaggcgt 11340

tgattctcta cctggcctgt cttcgaaccg gagctgtcta cctgcctctc aacactgcct 11400

acaccctggc cgagctcgac tacttcatcg gcgatgccga accgcgtctg gtggtcgttg 11460

ctcccgcagc tcgaggtggc gtggagacaa ttgccaagcg acacggtgct atcgtcgaaa 11520

ccctcgacgc cgatggacga ggctccttgc tggaccttgc tagagatgag cctgccgact 11580

ttgtcgatgc ttcgcgatct gccgacgatc tggctgctat tctctacact tccggtacaa 11640

ccggacgatc gaagggtgcc atgcttactc atggcaatct gctctccaac gctctcacct 11700

tgcgagacta ttggagagtt accgcagacg atcgactcat ccatgccttg ccaatctttc 11760

acactcatgg tctgttcgtt gctacgaacg tcacactgct tgcaggagcc tcgatgtttc 11820

tgctctccaa gttcgatgcc gacgaggtcg tttctctcat gccacaggcc accatgctta 11880

tgggcgtgcc cacattctac gttcgattgc tgcagagtcc tcgactcgag aagggtgctg 11940

tggccagcat cagactgttc atttctggat cagctccctt gcttgccgaa acccacgccg 12000

agtttcatgc tcgtactggt cacgccattc tcgagcgata cggcatgacg gaaaccaaca 12060

tgaatacttc caacccctac gagggcaagc gtattgccgg aaccgttggt ttcctctgc 12120

ccgacgtcac tgtgcgagtc accgatcccg ccaccggtct cgttcttcca cctgaagaga 12180

ctggcatgat cgagatcaag ggacccaacg tcttcaaggg ctattggcga atgcccgaaa 12240

agaccgctgc cgagtttacc gcagacggtt tctttatctc tggagatctc ggcaagatcg 12300

accgagaagg ttacgttcac attgtgggac gaggcaagga cctggtcatt tccggtggct 12360

acaacatcta tcccaaagag gtcgaaggcg agatcgacca gatcgagggt gtggtcgagt 12420

ctgctgtcat tggtgttcct catcccgatt tcggagaagg tgtcaccgct gttgtcgtgt 12480

gcaaacctgg tgccgttctc gacgaaaaga ccatcgtgtc tgctctgcag gaccgtcttg 12540

cccgatacaa gcaacccaag cggattatct ttgccgacga tctgcctcga aacactatgg 12600

gaaaggttca gaagaacatt cttcgacagc aatacgccga tctctacacc agacgataag 12660

cggccgcatg agaagataaa tatataaata cattgagata ttaaatgcgc tagattagag 12720

agcctcatac tgctcggaga gaagccaaga cgagtactca aaggggatta caccatccat 12780

atccacagac acaagctggg gaaaggttct atatacactt tccggaatac cgtagtttcc 12840

gatgttatca atgggggcag ccaggatttc aggcacttcg gtgtctcggg gtgaaatggc 12900

gttcttggcc tccatcaagt cgtaccatgt cttcatttgc ctgtcaaagt aaaacagaag 12960

cagatgaaga atgaacttga agtgaaggaa tttaaatgat gtcgacgcag taggatgtcc 13020

tgcacgggtc tttttgtggg gtgtggagaa aggggtgctt ggagatggaa gccggtagaa 13080

ccgggctgct tgtgcttgga gatggaagcc ggtagaaccg ggctgcttgg ggggatttgg 13140

ggccgctggg ctccaaagag gggtaggcat ttcgttgggg ttacgtaatt gcggcatttg 13200

ggtcctgcgc gcatgtccca ttggtcagaa ttagtccgga taggagactt atcagccaat 13260

cacagcgccg gatccacctg taggttgggt tgggtgggag cacccctcca cagagtagag 13320
tcaaacagca gcagcaacat gatagttggg ggtgtgcgtg ttaaaggaaa aaaaagaagc 13380
ttgggttata ttcccgctct atttagaggt tgcgggatag acgccgacgg agggcaatgg 13440
cgctatggaa ccttgcggat atccatacgc cgcggcggac tgcgtccgaa ccagctccag 13500
cagcgttttt tccgggccat tgagccgact gcgaccccgc caacgtgtct tggcccacgc 13560
actcatgtca tgttggtgtt gggaggccac tttttaagta gcacaaggca cctagctcgc 13620
agcaaggtgt ccgaaccaaa gaagcggctg cagtggtgca aacggggcgg aaacggcggg 13680
aaaaagccac gggggcacga attgaggcac gccctcgaat ttgagacgag tcacggcccc 13740
attcgcccgc gcaatggctc gccaacgccc ggtcttttgc accacatcag gttaccccaa 13800
gccaaacctt tgtgttaaaa agcttaacat attataccga acgtaggttt gggcgggctt 13860
gctccgtctg tccaaggcaa catttatata agggtctgca tcgccggctc aattgaatct 13920
tttttcttct tctcttctct atattcattc ttgaattaaa cacacatcaa ccatggagtc 13980
tggacccatg cctgctggca ttcccttccc tgagtactat gacttcttta tggactggaa 14040
gactcccctg gccatcgctg ccacctacac tgctgccgtc ggtctcttca accccaaggt 14100
tggcaaggtc tcccgagtgg ttgccaagtc ggctaacgca aagcctgccg agcgaaccca 14160
gtccggagct gccatgactg ccttcgtctt tgtgcacaac ctcattctgt gtgtctactc 14220
tggcatcacc ttctactaca tgtttcctgc tatggtcaag aacttccgaa cccacacact 14280
gcacgaagcc tactgcgaca cggatcagtc cctctggaac aacgcacttg gctactgggg 14340
ttacctcttc tacctgtcca agttctacga ggtcattgac accatcatca tcatcctgaa 14400
gggacgacgg tcctcgctgc ttcagaccta ccaccatgct ggagccatga ttaccatgtg 14460
gtctggcatc aactaccaag ccactcccat ttggatcttt gtggtcttca actccttcat 14520
tcacaccatc atgtactgtt actatgcctt cacctctatc ggattccatc ctcctggcaa 14580
aaagtacctg acttcgatgc agattactca gtttctggtc ggtatcacca ttgccgtgtc 14640
ctacctcttc gttcctggct gcatccgaac acccggtgct cagatggctg tctggatcaa 14700
cgtcggctac ctgtttccct tgacctatct gttcgtggac tttgccaagc gaacctactc 14760
caagcgatct gccattgccg ctcagaaaaa ggctcagtaa gcggccgcaa gtgtggatgg 14820
ggaagtgagt gcccggttct gtgtgcacaa ttggcaatcc aagatggatg gattcaacac 14880
agggatatag cgagctacgt ggtggtgcga ggatatagca acggatattt atgtttgaca 14940
cttgagaatg tacgatacaa gcactgtcca agtacaatac taaacatact gtacatactc 15000
atactcgtac ccgggcaacg gtttcacttg agtgcagtgg ctagtgctct tactcgtaca 15060
gtgtgcaata ctgcgtatca tagtctttga tgtatatcgt attcattcat gttagttgc 15119

<210> 134
<211> 14623
<212> DNA
<213> Artificial Sequence

<220>
<223> Plasmid pZKL4-398F2

<220>
<221> misc_feature
<222> (1048)..(1048)
<223> n is a, c, g, or t

<220>
<221> misc_feature
<222> (1053)..(1056)
<223> n is a, c, g, or t

<400> 134

taaaaggcgt tgaaacagaa tgagccagac agcaaggaca aggtggccaa cagcaaggag 60
tccaaaaagc cctctattga cgagatccac gatgttattg ctcatgaggt ttccgagctc 120
gatgctggga agaagaagtg atttgtatat aagaaataaa tgagatatag taaaggagtg 180
caagagaatg gcaaggtggt caaattctat attacttgca gtcactggtt cctcgttgac 240
atgaatgaag ttaccgttgg catagctgat ttaatatata actgtccaac taactctcac 300
ctagatataa cccatgtgtg tgtttccaat catcaatgcg gccgcgccta cttaagcaac 360
gggcttgata acagcggggg gggtgcccac gttgttgcgg ttgcggaaga acagaacacc 420
cttaccagca ccctcggcac cagcgctggg ctcaacccac tggcacatac gcgcactgcg 480
gtacatggcg cggatgaagc cacgaggacc atcctggaca tcagcccggt agtgcttgcc 540
catgatgggc ttaatggcct cggtggcctc gtccgcgttg tagaagggga tgctgctgac 600
gtagtggtgg aggacatgag tctcgatgat gccgtggaga aggtggcggc cgatgaagcc 660
catctcacgg tcaatggtag cagcggcacc acggacgaag ttccactcgt cgttggtgta 720
gtggggaagg gtagggtcgg tgtgctggag gaaggtgatg gcaacgagcc agtggttaac 780
ccagaggtag ggaacaaagt accagatggc catgttgtag aaaccgaact tctgaacgag 840
gaagtacaga gcagtggcca tcagaccgat accaatatcg ctgaggacga tgagcttagc 900
gtcactgttc tcgtacagag ggctgcgggg atcgaagtgg ttaacaccac cgccgaggcc 960
gttatgcttg cccttgccgc gaccctcacg ctggcgctcg tggtagttgt ggccggtaac 1020
attggtgatg aggtagttgg gccagccnac gannnnctca gtaagatgag cgagctcgtg 1080
ggtcatcttt ccgagacgag tagcctgctg ctcgcgggtt cggggaacga agaccatgtc 1140
acgctccatg ttgccagtgg ccttgtggtg ctttcggtgg gagatttgcc agctgaagta 1200
ggggacaagg agggaagagt gaagaaccca gccagtaatg tcgttgatga tgcgagaatc 1260
ggagaaagca ccgtgaccgc actcatgggc aataacccag agaccagtac cgaaaagacc 1320
ctgaagaacg gtgtacacgg cccacagacc agcgcgggcg ggggtggagg ggatatattc 1380
gggggtcaca aagttgtacc agatgctgaa agtggtagtc aggaggacaa tgtcgcggag 1440

gatataaccg tatcccttga gagcggagcg cttgaagcag tgcttaggga tggcattgta 1500
gatgtccttg atggtaaagt cgggaacctc gaactggttg ccgtaggtgt cgagcatgac 1560
accatactcg gacttgggct tggcgatatc aacctcggac atggacgaga gcgatgtgga 1620
agaggccgag tggcggggag agtctgaagg agagacggcg gcagactcag aatccgtcac 1680
agtagttgag gtgacggtgc gtctaagcgc agggttctgc ttgggcagag ccgaagtgga 1740
cgccatgggc aggacctgtg ttagtacatt gtcggggagt catcaattgg ttcgacaggt 1800
tgtcgactgt tagtatgagc tcaattgggc tctggtgggt cgatgacact tgtcatctgt 1860
ttctgttggg tcatgtttcc atcaccttct atggtactca caattcgtcc gattcgcccg 1920
aatccgttaa taccgacttt gatggccatg ttgatgtgtg tttaattcaa gaatgaatat 1980
agagaagaga agaagaaaaa agattcaatt gagccggcga tgcagaccct tatataaatg 2040
ttgccttgga cagacggagc aagcccgccc aaacctacgt tcggtataat atgttaagct 2100
ttttaacaca aaggtttggc ttggggtaac ctgatgtggt gcaaaagacc gggcgttggc 2160
gagccattgc gcgggcgaat ggggccgtga ctcgtctcaa attcgagggc gtgcctcaat 2220
tcgtgccccc gtggcttttt cccgccgttt ccgccccgtt tgcaccactg cagccgcttc 2280
tttggttcgg acaccttgct gcgagctagg tgccttgtgc tacttaaaaa gtggcctccc 2340
aacaccaaca tgacatgagt gcgtgggcca agacacgttg gcggggtcgc agtcggctca 2400
atggcccgga aaaaacgctg ctggagctgg ttcggacgca gtccgccgcg gcgtatggat 2460
atccgcaagg ttccatagcg ccattgccct ccgtcggcgt ctatcccgca acctctaaat 2520
agagcgggaa tataacccaa gcttcttttt tttcctttaa cacgcacacc cccaactatc 2580
atgttgctgc tgctgtttga ctctactctg tggaggggtg ctcccaccca acccaaccta 2640
caggtggatc cggcgctgtg attggctgat aagtctccta tccggactaa ttctgaccaa 2700
tgggacatgc gcgcaggacc caaatgccgc aattacgtaa ccccaacgaa atgcctaccc 2760
ctctttggag cccagcggcc ccaaatcccc ccaagcagcc cggttctacc ggcttccatc 2820
tccaagcaca agcagcccgg aattctctct cttgagcttt tccataacaa gttcttctgc 2880
ctccaggaag tccatgggtg gtttgatcat ggttttggtg tagtggtagt gcagtggtgg 2940
tattgtgact ggggatgtag ttgagaataa gtcatacaca agtcagcttt cttcgagcct 3000
catataagta taagtagttc aacgtattag cactgtaccc agcatctccg tatcgagaaa 3060
cacaacaaca tgccccattg gacagatcat gcggatacac aggttgtgca gtatcataca 3120
tactcgatca gacaggtcgt ctgaccatca tacaagctga acaagcgctc catacttgca 3180
cgctctctat atacacagtt aaattacata tccatagtct aacctctaac agttaatctt 3240
ctggtaagcc tcccagccag ccttctggta tcgcttggcc tcctcaatag gatctcggtt 3300
ctggccgtac agacctcggc cgacaattat gatatccgtt ccggtagaca tgacatcctc 3360

aacagttcgg tactgctgtc cgagagcgtc tcccttgtcg tcaagaccca ccccgggggt 3420
cagaataagc cagtcctcag agtcgccctt aggtcggttc tgggcaatga agccaaccac 3480
aaactcgggg tcggatcggg caagctcaat ggtctgcttg gagtactcgc cagtggccag 3540
agagcccttg caagacagct cggccagcat gagcagacct ctggccagct tctcgttggg 3600
agaggggact aggaactcct tgtactggga gttctcgtag tcagagacgt cctccttctt 3660
ctgttcagag acagtttcct cggcaccagc tcgcaggcca gcaatgattc cggttccggg 3720
tacaccgtgg gcgttggtga tatcggacca ctcggcgatt cggtgacacc ggtactggtg 3780
cttgacagtg ttgccaatat ctgcgaactt tctgtcctcg aacaggaaga aaccgtgctt 3840
aagagcaagt tccttgaggg ggagcacagt gccggcgtag gtgaagtcgt caatgatgtc 3900
gatatgggtt ttgatcatgc acacataagg tccgacctta tcggcaagct caatgagctc 3960
cttggtggtg gtaacatcca gagaagcaca caggttggtt ttcttggctg ccacgagctt 4020
gagcactcga gcggcaaagg cggacttgtg gacgttagct cgagcttcgt aggagggcat 4080
tttggtggtg aagaggagac tgaaataaat ttagtctgca gaactttta tcggaacctt 4140
atctggggca gtgaagtata tgttatggta atagttacga gttagttgaa cttatagata 4200
gactggacta tacggctatc ggtccaaatt agaaagaacg tcaatggctc tctgggcgtc 4260
gcctttgccg acaaaaatgt gatcatgatg aaagccagca atgacgttgc agctgatatt 4320
gttgtcggcc aaccgcgccg aaaacgcagc tgtcagaccc acagcctcca acgaagaatg 4380
tatcgtcaaa gtgatccaag cacactcata gttggagtcg tactccaaag gcggcaatga 4440
cgagtcagac agatactcgt cgaccttttc cttgggaacc accaccgtca gcccttctga 4500
ctcacgtatt gtagccaccg acacaggcaa cagtccgtgg atagcagaat atgtcttgtc 4560
ggtccatttc tcaccaactt taggcgtcaa gtgaatgttg cagaagaagt atgtgccttc 4620
attgagaatc ggtgttgctg atttcaataa agtcttgaga tcagtttggc cagtcatgtt 4680
gtgggggggta attggattga gttatcgcct acagtctgta caggtatact cgctgcccac 4740
tttatacttt ttgattccgc tgcacttgaa gcaatgtcgt ttaccaaaag tgagaatgct 4800
ccacagaaca caccccaggg tatggttgag caaaaaataa acactccgat acggggaatc 4860
gaaccccggt ctccacggtt ctcaagaagt attcttgatg agagcgtatc gatcgaggaa 4920
gaggacaagc ggctgcttct taagtttgtg acatcagtat ccaaggcacc attgcaagga 4980
ttcaaggctt tgaacccgtc atttgccatt cgtaacgctg gtagacaggt tgatcggttc 5040
cctacggcct ccacctgtgt caatcttctc aagctgcctg actatcagga cattgatcaa 5100
cttcggaaga aacttttgta tgccattcga tcacatgctg gtttcgattt gtcttagagg 5160
aacgcatata cagtaatcat agagaataaa cgatattcat ttattaaagt agatagttga 5220
ggtagaagtt gtaaagagtg ataaatagcg gccgcttagg ccttgtctgc cttggcaacc 5280
attcgagcga aggtgcccag gtaggagata agaatgccga caccttcgag catgggagga 5340

gatcggtagg gcaggttgtg cttcttgcac agctgctcca ctttgatgga ggcagcggtg 5400
aggttgtgtc tgggaagagt aggccacagg tgatgttcga tctggtagtt gaggccaccg 5460
aaaaaccagt ctgtgacgag gcctcgctga acgttcatgg tctcgtgaat ctgtccagca 5520
cagaagccat gaccatccca cacggagtcc tgaatcttct ccagagggta gtggttcatg 5580
aagacaacga tggcaattcc gaagccacca agcagctcgg agacaaagaa caccatgagt 5640
ccggtgagaa acgagggcat gtagaaatag tagaacaagg ccttgagacc ccagtggagt 5700
gccagaccga cggactcctt ctcgtactgt cttcgatagt actgattgct tcgatccttg 5760
agacccgtgg cagtatgaat ggactgaaag caccagatga atcgcagaag ggcacagatg 5820
aaaaagaagt aatactgttg gtacttgatc atccgtcgag aaaacgggcc agctcgctcg 5880
acgtcctcct tggaccacgc caggagaggc aggttgtcga tgtcgggatc gtgaccctgc 5940
acgttggtgg cggagtgatg agcgttgtgt cggtccttcc accaagtgac agaaaagccc 6000
tgaagcacgt ttccgaagag caggccaatg gcattgttga tggatcggtc cttgaacagc 6060
tggtgatggc aaatgtcgtg cgagagccat cccatctgtt gaaagtgaat gccgaggacg 6120
atagcaccaa ccaggtacca gtgccactgg gtcatcaaca gcacggcaag gacacccagt 6180
ccgagagtcg acagggtctt gtaagcgtac cacatgggag aggcatcgaa catgcctgca 6240
gcaatgagct cgtctcgcag ctttcggaaa tcctcctgag gttcgtcgga gtttggcttg 6300
ggaggtgttg gagtcagggg agaggaaggt tccatgatgg gcattcgtct gagtttggac 6360
acggcgttct cggagtgcat aaccatgaag acgtcggtag catcccgacc tcggtagttc 6420
tcgataatgt cggcacctcc gggatggtgg ttgagccagg cagaaacgtc gtaggtgaca 6480
ccgtccacgg tgagaggcag agcgggtcgc ttgaccatgg atcccatgac ggccagagag 6540
tcgtagtagg tagcgggagg aagtccggca ggtcgagcgg gaccggcgcc ctccttttg 6600
cctcggttct ttcgaggctt ccgaatgtag gtctggacgt agaagttgag gaacagaagc 6660
agcaccgtgc cgacatacca gtagttgaag atccaggcaa acattcgcat accgtcctgc 6720
cggtagcagg gcacgtttcg gtacttccag acgatgtaga atccgacgtt gaactggata 6780
atttgcatgg aggtaatgag gttcttgggc atagggaagt tcagcttgat gagtcgcgtc 6840
caatagtaac cgtacatgat ccagtgaatg aagccgttga gcagaacaaa gatccagacg 6900
ccttcgtttc gatacttgta gaacagccac atgtcgatgg gagctccgag atggtgaaag 6960
aactgcagaa acgacaaggg cttgtccatg aggggaaggt agaaggagtc gatgtactcg 7020
acgaacttgc tgaggtagaa cagctgagtg gtgattcgga acacgtcatt gttgaaggca 7080
gtctcacaat cgccggaaag aataccagtg acagagagag cgtaggccat cgccaggaag 7140
gagccgaacg agtagatgga catgagaaag ttgtatgcgg tgaacagctt tttgagggtg 7200
gcttgtcgct tgaggtccag aggtcgaagc atgaacttga tggccacgaa agcgaccgag 7260

aggtacagaa cctcgcaaga ggaggcgtcc tgccagagct gggcgtagtc caccttggga 7320
agttcctcct ggacgatgga gaccagctcc ttggcagcct ccatggtacc agagctgggt 7380
tagtttgtgt agagagtgtg tgttgctagc gactttcgga ttgtgtcatt acacaaaacg 7440
cgtcgtctcg acactgatct tgtcgtggat actcacggct cggaattctg tgatgtgtag 7500
tttagatttc gaatctgtgg ggaaagaaag gaaaaaagag actggcaacc gattgggaga 7560
gccactgttt atatataccc tagacaagcc ccccgcttgt aagatgttgg tcaatgtaaa 7620
ccagtattaa ggttggcaag tgcaggagaa gcaaggtgtg ggtatcgagc aatggaaatg 7680
tgcggaaggc aaaaaaatga ggccacggcc tattgtcggg gctatatcca gggggcgatt 7740
gaagtacact aacatgacat gtgtccacag accctcaatc tggcctgatg agccaaatcc 7800
atacgcgctt tcgcagctct aaaggctata acaagtcaca ccaccctgct cgacctcagc 7860
gccctcactt tttgttaaga caaactgtac acgctgttcc agcgttttct gcctgcacct 7920
ggtgggacat ttggtgcaac ctaaagtgct cggaacctct gtggtgtcca gatcagcgca 7980
gcagttccga ggtagttttg aggcccttag atgatgcaat ggtgtcagtc gctggatcac 8040
gagtcttaat ggcagtattc gttcttattt gtgccattga gccccgttat cctcgtatct 8100
tctacccccc atcccatccc tttgttggtg caaccctacc catttattgt tgggtgcagc 8160
ccaaccgacg tggagagctt ggcttggcca tataaaaagg ccccccccta gtggcaatgg 8220
cagaaagtca gctgtgagtt gttgaatttg tcatctaggc ggcctggccg tcttctccgg 8280
ggcaatttaa atgttcctct atagtagatc tgcgtacact gtttaaacag agtgtgaaag 8340
actcactatg gtccgggctt atctcgacca atagccaaag tctggagttt ctgagagaaa 8400
aaggcaagat acgtatgtaa caaagcgacg catggtacaa taataccgga ggcatgtatc 8460
atagagagtt agtggttcga tgatggcact ggtgcctggt atgactttat acggctgact 8520
acatatttgt cctcagacat acaattacag tcaagcactt acccttggac atctgtaggt 8580
acccccccggc caagacgatc tcagcgtgtc gtatgtcgga ttggcgtagc tccctcgctc 8640
gtcaattggc tcccatctac tttcttctgc ttggctacac ccagcatgtc tgctatggct 8700
cgttttcgtg ccttatctat cctcccagta ttaccaactc taaatgacat gatgtgattg 8760
ggtctacact ttcatatcag agataaggag tagcacagtt gcataaaaag cccaactcta 8820
atcagcttct tcctttcttg taattagtac aaaggtgatt agcgaaatct ggaagcttag 8880
ttggccctaa aaaaatcaaa aaaagcaaaa aacgaaaaac gaaaaaccac agttttgaga 8940
acagggaggt aacgaaggat cgtatatata tatatatata tatataccca cggatcccga 9000
gaccggcctt tgattcttcc ctacaaccaa ccattctcac caccctaatt cacaaccatg 9060
gagtctggac ccatgcctgc tggcattccc ttccctgagt actatgactt ctttatggac 9120
tggaagactc ccctggccat cgctgccacc tacactgctg ccgtcggtct cttcaacccc 9180
aaggttggca aggtctcccg agtggttgcc aagtcggcta acgcaaagcc tgccgagcga 9240

acccagtccg gagctgccat gactgccttc gtctttgtgc acaacctcat tctgtgtgtc 9300
tactctggca tcaccttcta ctacatgttt cctgctatgg tcaagaactt ccgaacccac 9360
acactgcacg aagcctactg cgacacggat cagtccctct ggaacaacgc acttggctac 9420
tggggttacc tcttctacct gtccaagttc tacgaggtca ttgacaccat catcatcatc 9480
ctgaagggac gacggtcctc gctgcttcag acctaccacc atgctggagc catgattacc 9540
atgtggtctg gcatcaacta ccaagccact cccatttgga tctttgtggt cttcaactcc 9600
ttcattcaca ccatcatgta ctgttactat gccttcacct ctatcggatt ccatcctcct 9660
ggcaaaaagt acctgacttc gatgcagatt actcagtttc tggtcggtat caccattgcc 9720
gtgtcctacc tcttcgttcc tggctgcatc cgaacacccg gtgctcagat ggctgtctgg 9780
atcaacgtcg gctacctgtt tcccttgacc tatctgttcg tggactttgc caagcgaacc 9840
tactccaagc gatctgccat tgccgctcag aaaaaggctc agtaagcggc cgcatgagaa 9900
gataaatata taaatacatt gagatattaa atgcgctaga ttagagagcc tcatactgct 9960
cggagagaag ccaagacgag tactcaaagg ggattacacc atccatatcc acagacacaa 10020
gctggggaaa ggttctatat acactttccg gaataccgta gtttccgatg ttatcaatgg 10080
gggcagccag gatttcaggc acttcggtgt ctcggggtga aatggcgttc ttggcctcca 10140
tcaagtcgta ccatgtcttc atttgcctgt caaagtaaaa cagaagcaga tgaagaatga 10200
acttgaagtg aaggaattta aatgtaacga aactgaaatt tgaccagata ttgtgtccgc 10260
ggtggagctc cagcttttgt tccctttagt gagggttaat ttcgagcttg gcgtaatcat 10320
ggtcatagct gtttcctgtg tgaaattgtt atccgctcac aagcttccac acaacgtacg 10380
tagggatcag gtgcttagga agctcgacca accacggaga ctgttgaaac tggatgtcgg 10440
taacagcatc tggaatgctg aatgttcctc gaataacaac atatttctcc ttgttgaggt 10500
gatcataagc tatgtatccg gtgattgaag tggaatagaa gtctcctccg aagactgagt 10560
ccaacgtcat gttcgggaaa taccgacaac tctctccaca tgtaaaatca gttcgtagag 10620
gagtgactgg cgcattgaca cagtaggcga tgtttgcaat ccgagaaaac ttggccgtaa 10680
agttgtacag ctcctgggag gcttgaactc gagtttttga aagtgtcgct ggtggctcgc 10740
cgaagaggga ggcatagagg tacgcaacca cttgcccgag cgtgaggttc atgatgccaa 10800
tagtgaatgt catttatcac cgtactgcgc agtatttata tagggctcat cggtccatgt 10860
atagatctgt ccacttatga cacccccatg tctcattaat gtgtaaaggt ggagacgggt 10920
ggagtacagg tacagagttg gaggaaatca ggatagtggg gttaagacat gctccgagtc 10980
caaatttcaa ctctccattg tcacaagacc tctggtttca gagttattac agatctaggc 11040
ctgtttcaag gtgaggggac ctcatctgga tcggcacgac gatcgtcacc ttacagagga 11100
cgtctgtcgc agggaaaggt gatgtggcgc gccagctgca ttaatgaatc ggccaacgcg 11160

cggggagagg cggtttgcgt attgggcgct cttccgcttc ctcgctcact gactcgctgc 11220
gctcggtcgt tcggctgcgg cgagcggtat cagctcactc aaaggcggta atacggttat 11280
ccacagaatc aggggataac gcaggaaaga acatgtgagc aaaaggccag caaaaggcca 11340
ggaaccgtaa aaaggccgcg ttgctggcgt ttttccatag gctccgcccc cctgacgagc 11400
atcacaaaaa tcgacgctca agtcagaggt ggcgaaaccc gacaggacta taaagatacc 11460
aggcgtttcc ccctggaagc tccctcgtgc gctctcctgt tccgaccctg ccgcttaccg 11520
gatacctgtc cgcctttctc ccttcgggaa gcgtggcgct ttctcatagc tcacgctgta 11580
ggtatctcag ttcggtgtag gtcgttcgct ccaagctggg ctgtgtgcac gaaccccccg 11640
ttcagcccga ccgctgcgcc ttatccggta actatcgtct tgagtccaac ccggtaagac 11700
acgacttatc gccactggca gcagccactg gtaacaggat tagcagagcg aggtatgtag 11760
gcggtgctac agagttcttg aagtggtggc ctaactacgg ctacactaga agaacagtat 11820
ttggtatctg cgctctgctg aagccagtta ccttcggaaa aagagttggt agctcttgat 11880
ccggcaaaca aaccaccgct ggtagcggtg gtttttttgt ttgcaagcag cagattacgc 11940
gcagaaaaaa aggatctcaa gaagatcctt tgatcttttc tacggggtct gacgctcagt 12000
ggaacgaaaa ctcacgttaa gggattttgg tcatgagatt atcaaaaagg atcttcacct 12060
agatcctttt aaattaaaaa tgaagtttta aatcaatcta aagtatatat gagtaaactt 12120
ggtctgacag ttaccaatgc ttaatcagtg aggcacctat ctcagcgatc tgtctatttc 12180
gttcatccat agttgcctga ctccccgtcg tgtagataac tacgatacgg gagggcttac 12240
catctggccc cagtgctgca atgataccgc gagacccacg ctcaccggct ccagatttat 12300
cagcaataaa ccagccagcc ggaagggccg agcgcagaag tggtcctgca actttatccg 12360
cctccatcca gtctattaat tgttgccggg aagctagagt aagtagttcg ccagttaata 12420
gtttgcgcaa cgttgttgcc attgctacag gcatcgtggt gtcacgctcg tcgtttggta 12480
tggcttcatt cagctccggt tcccaacgat caaggcgagt tacatgatcc cccatgttgt 12540
gcaaaaaagc ggttagctcc ttcggtcctc cgatcgttgt cagaagtaag ttggccgcag 12600
tgttatcact catggttatg gcagcactgc ataattctct tactgtcatg ccatccgtaa 12660
gatgcttttc tgtgactggt gagtactcaa ccaagtcatt ctgagaatag tgtatgcggc 12720
gaccgagttg ctcttgcccg gcgtcaatac gggataatac cgcgccacat agcagaactt 12780
taaaagtgct catcattgga aaacgttctt cggggcgaaa actctcaagg atcttaccgc 12840
tgttgagatc cagttcgatg taacccactc gtgcacccaa ctgatcttca gcatctttta 12900
ctttcaccag cgtttctggg tgagcaaaaa caggaaggca aaatgccgca aaaaagggaa 12960
taagggcgac acggaaatgt tgaatactca tactcttcct ttttcaatat tattgaagca 13020
tttatcaggg ttattgtctc atgagcggat acatatttga atgtatttag aaaaataaac 13080
aaataggggt tccgcgcaca tttccccgaa aagtgccacc tgatgcggtg tgaaataccg 13140

cacagatgcg taaggagaaa ataccgcatc aggaaattgt aagcgttaat attttgttaa 13200
aattcgcgtt aaatttttgt taaatcagct cattttttaa ccaataggcc gaaatcggca 13260
aaatccctta taaatcaaaa gaatagaccg agatagggtt gagtgttgtt ccagtttgga 13320
acaagagtcc actattaaag aacgtggact ccaacgtcaa agggcgaaaa accgtctatc 13380
agggcgatgg cccactacgt gaaccatcac cctaatcaag ttttttgggg tcgaggtgcc 13440
gtaaagcact aaatcggaac cctaaaggga gcccccgatt tagagcttga cggggaaagc 13500
cggcgaacgt ggcgagaaag gaagggaaga aagcgaaagg agcgggcgct agggcgctgg 13560
caagtgtagc ggtcacgctg cgcgtaacca ccacacccgc cgcgcttaat gcgccgctac 13620
agggcgcgtc cattcgccat tcaggctgcg caactgttgg gaagggcgat cggtgcgggc 13680
ctcttcgcta ttacgccagc tggcgaaagg gggatgtgct gcaaggcgat taagttgggt 13740
aacgccaggg ttttcccagt cacgacgttg taaaacgacg gccagtgaat tgtaatacga 13800
ctcactatag ggcgaattgg gcccgacgtc gcatgccaag gcgtatatta gttggtggga 13860
accagtgtac gaccgggtcc tgtataacca ggttcagtgg catacttgta ggagttgttc 13920
ccgtggtatt tgggcatggc taagacattt cgccgaccaa tgttaagtgc acaatagccg 13980
atgtagtaga tgtaagccag atggttttgg agcaggtcga ttcgagacca cagattgaaa 14040
gtgcctcgat ggcaggcctc gttttctcct ccttcacaga cagacacttt gtcgagtgca 14100
gggtagacgc tttcttggtc aatgtacact tctccaatgg cgtgtgtgta attggaccaa 14160
tctggcagtc caacgaagat atcgttccag tgggtgagcc tgtagtttcg tcgcttgtcg 14220
tttacttcaa ggcctgtgtc attaaaccac agctggttga tgtagtttgc aaactctgag 14280
tttcctactc tcggctggcc aaagttgatc atggtaggat catgtccaag taatttgaag 14340
tgagttgcga aaagaagagc ttgagcagcg cccagcgagt gaccagtaac atacattttg 14400
tagtcagtgt ggttggtgag gaacttttca aactgaggag cagcattgac catagtttcg 14460
ttgaaggcct tggcgaaccc atcatggatc atgcagcctt tgcactcact agttttgatg 14520
ggaataagac gggggtcttc aaccaccaga gcccgctctt tgaggttgtc aagacctttg 14580
ttctccactt ccaagtctgg tcggactgcc catctctgtt aat 14623

<210> 135
<211> 14619
<212> DNA
<213> Artificial Sequence

<220>
<223> Plasmid pZP2-85m98F

<400> 135

cgatggaagc cggtagaacc gggctgcttg tgcttggaga tggaagccgg tagaaccggg 60

ctgcttgggg ggatttgggg ccgctgggct ccaaagaggg gtaggcattt cgttggggtt 120

acgtaattgc ggcatttggg tcctgcgcgc atgtcccatt ggtcagaatt agtccggata 180
ggagacttat cagccaatca cagcgccgga tccacctgta ggttgggttg ggtgggagca 240
cccctccaca gagtagagtc aaacagcagc agcaacatga tagttggggg tgtgcgtgtt 300
aaaggaaaaa aaagaagctt gggttatatt cccgctctat ttagaggttg cgggatagac 360
gccgacggag ggcaatggcg ctatggaacc ttgcggatat ccatacgccg cggcggactg 420
cgtccgaacc agctccagca gcgttttttc cgggccattg agccgactgc gaccccgcca 480
acgtgtcttg gcccacgcac tcatgtcatg ttggtgttgg gaggccactt tttaagtagc 540
acaaggcacc tagctcgcag caaggtgtcc gaaccaaaga agcggctgca gtggtgcaaa 600
cggggcggaa acggcgggaa aaagccacgg gggcacgaat tgaggcacgc cctcgaattt 660
gagacgagtc acggccccat tcgcccgcgc aatggctcgc caacgcccgg tcttttgcac 720
cacatcaggt taccccaagc caaacctttg tgttaaaaag cttaacatat tataccgaac 780
gtaggtttgg gcgggcttgc tccgtctgtc caaggcaaca tttatataag ggtctgcatc 840
gccggctcaa ttgaatcttt tttcttcttc tcttctctat attcattctt gaattaaaca 900
cacatcaacc atggtcaagc gacccgctct gcctctcacc gtggacggtg tcacctacga 960
cgtttctgcc tggctcaacc accatcccgg aggtgccgac attatcgaga actaccgagg 1020
tcgggatgct accgacgtct tcatggttat gcactccgag aacgccgtgt ccaaactcag 1080
acgaatgccc atcatggaac cttcctctcc cctgactcca acacctccca agccaaactc 1140
cgacgaacct caggaggatt tccgaaagct gcgagacgag ctcattgctg caggcatgtt 1200
cgatgcctct cccatgtggt acgcttacaa gaccctgtcg actctcggac tgggtgtcct 1260
tgccgtgctg ttgatgaccc agtggcactg gtacctggtt ggtgctatcg tcctcggcat 1320
tcactttcaa cagatgggat ggctctcgca cgacatttgc catcaccagc tgttcaagga 1380
ccgatccatc aacaatgcca ttggcctgct cttcggaaac gtgcttcagg gcttttctgt 1440
cacttggtgg aaggaccgac acaacgctca tcactccgcc accaacgtgc agggtcacga 1500
tcccgacatc gacaacctgc ctctcctggc gtggtccaag gaggacgtcg agcgagctgg 1560
cccgttttct cgacggatga tcaagtacca acagtattac ttcttttca tctgtgccct 1620
tctgcgattc atctggtgct ttcagtccat tcatactgcc acgggtctca aggatcgaag 1680
caatcagtac tatcgaagac agtacgagaa ggagtccgtc ggtctggcac tccactgggg 1740
tctcaaggcc ttgttctact atttctacat gccctcgttt ctcaccggac tcatggtgtt 1800
ctttgtctcc gagctgcttg gtggcttcgg aattgccatc gttgtcttca tgaaccacta 1860
ccctctggag aagattcagg actccgtgtg ggatggtcat ggcttctgtg ctggacagat 1920
tcacgagacc atgaacgttc agcgaggcct cgtcacagac tggtttttcg gtggcctcaa 1980
ctaccagatc gaacatcacc tgtggcctac tcttcccaga cacaacctca ccgctgcctc 2040
catcaaagtg gagcagctgt gcaagaagca caacctgccc taccgatctc ctcccatgct 2100

cgaaggtgtc ggcattctta tctcctacct gggcaccttc gctcgaatgg ttgccaaggc 2160
agacaaggcc taagcggccg cattgatgat tggaaacaca cacatgggtt atatctaggt 2220
gagagttagt tggacagtta tatattaaat cagctatgcc aacggtaact tcattcatgt 2280
caacgaggaa ccagtgactg caagtaatat agaatttgac caccttgcca ttctcttgca 2340
ctcctttact atatctcatt tatttcttat atacaaatca cttcttcttc ccagcatcga 2400
gctcggaaac ctcatgagca ataacatcgt ggatctcgtc aatagagggc tttttggact 2460
ccttgctgtt ggccaccttg tccttgctgt ttaaacatcg tggttaatgc tgctgtgtgc 2520
tgtgtgtgtg tgttgtttgg cgctcattgt tgcgttatgc agcgtacacc acaatattgg 2580
aagcttatta gcctttctat tttttcgttt gcaaggctta acaacattgc tgtggagagg 2640
gatggggata tggaggccgc tggagggagt cggagaggcg ttttggagcg gcttggcctg 2700
gcgcccagct cgcgaaacgc acctaggacc ctttggcacg ccgaaatgtg ccacttttca 2760
gtctagtaac gccttaccta cgtcattcca tgcgtgcatg tttgcgcctt ttttcccttg 2820
cccttgatcg ccacacagta cagtgcactg tacagtggag gttttggggg ggtcttagat 2880
gggagctaaa agcggcctag cggtacacta gtgggattgt atggagtggc atggagccta 2940
ggtggagcct gacaggacgc acgaccggct agcccgtgac agacgatggg tggctcctgt 3000
tgtccaccgc gtacaaatgt ttgggccaaa gtcttgtcag ccttgcttgc gaacctaatt 3060
cccaattttg tcacttcgca cccccattga tcgagcccta acccctgccc atcaggcaat 3120
ccaattaagc tcgcattgtc tgccttgttt agtttggctc ctgcccgttt cggcgtccac 3180
ttgcacaaac acaaacaagc attatatata aggctcgtct ctccctccca accacactca 3240
ctttttttgcc cgtcttccct tgctaacaca aaagtcaaga acacaaacaa ccacccccaac 3300
cccccttacac acaagacata tctacagcaa tggccatggc tctctccctt actaccgagc 3360
agctgctcga gcgacccgac ctggttgcca tcgacggcat tctctacgat ctggaaggtc 3420
ttgccaaggt ccatcccgga tccgacttga tcctcgcttc tggtgcctcc gatgcttctc 3480
ctctgttcta ctccatgcac ccttacgtca agcccgagaa ctcgaagctg cttcaacagt 3540
tcgtgcgagg caagcacgac cgaacctcca aggacattgt ctacacctac gactctccct 3600
ttgcacagga cgtcaagcga actatgcgag aggtcatgaa aggtcggaac tggtatgcca 3660
cacctggatt ctggctgcga accgttggca tcattgctgt caccgccttt tgcgagtggc 3720
actgggctac taccggaatg gtgctgtggg gtctcttgac tggattcatg cacatgcaga 3780
tcggcctgtc cattcagcac gatgcctctc atggtgccat cagcaaaaag ccctgggtca 3840
acgctctctt tgcctacggc atcgacgtca ttggatcgtc cagatggatc tggctgcagt 3900
ctcacatcat gcgacatcac acctacacca atcagcatgg tctcgacctg gatgccgagt 3960
ccgcagaacc attccttgtg ttccacaact accctgctgc caacactgct cgaaagtggt 4020

ttcaccgatt ccaggcctgg tacatgtacc tcgtgcttgg agcctacggc gtttcgctgg 4080
tgtacaaccc tctctacatc ttccgaatgc agcacaacga caccattccc gagtctgtca 4140
cagccatgcg agagaacggc tttctgcgac ggtaccgaac ccttgcattc gttatgcgag 4200
ctttcttcat ctttcgaacc gccttcttgc cctggtatct cactggaacc tccctgctca 4260
tcaccattcc tctggtgccc actgctaccg gtgccttcct caccttcttt ttcatcttgt 4320
ctcacaactt cgatggctcg gagcgaatcc ccgacaagaa ctgcaaggtc aagagctccg 4380
agaaggacgt tgaagccgat cagatcgact ggtacagagc tcaggtggag acctcttcca 4440
cctacggtgg acccattgcc atgttcttta ctggcggtct caacttccag atcgagcatc 4500
acctctttcc tcgaatgtcg tcttggcact atcccttcgt gcagcaagct gtccgagagt 4560
gttgcgaacg acacggagtt cggtacgtct tctaccctac cattgtgggc aacatcattt 4620
ccaccctcaa gtacatgcac aaagtcggtg tggttcactg tgtcaaggac gctcaggatt 4680
cctaagcggc cgcatgagaa gataaatata taaatacatt gagatattaa atgcgctaga 4740
ttagagagcc tcatactgct cggagagaag ccaagacgag tactcaaagg ggattacacc 4800
atccatatcc acagacacaa gctggggaaa ggttctatat acactttccg gaataccgta 4860
gtttccgatg ttatcaatgg gggcagccag gatttcaggc acttcggtgt ctcggggtga 4920
aatggcgttc ttggcctcca tcaagtcgta ccatgtcttc atttgcctgt caaagtaaaa 4980
cagaagcaga tgaagaatga acttgaagtg aaggaattta aatgtaacga aactgaaatt 5040
tgaccagata ttgtgtccgc ggtggagctc cagcttttgt tccctttagt gagggttaat 5100
ttcgagcttg gcgtaatcat ggtcatagct gtttcctgtg tgaaattgtt atccgctcac 5160
aagcttccac acaacgtacg ggcgtcgttg cttgtgtgat tttgaggac ccatcccttt 5220
ggtatataag tatactctgg ggttaaggtt gcccgtgtag tctaggttat agttttcatg 5280
tgaaataccg agagccgagg gagaataaac gggggtattt ggacttgttt ttttcgcgga 5340
aaagcgtcga atcaaccctg cgggccttgc accatgtcca cgacgtgttt ctcgccccaa 5400
ttcgcccctt gcacgtcaaa attaggcctc catctagacc cctccataac atgtgactgt 5460
ggggaaaagt ataagggaaa ccatgcaacc atagacgacg tgaaagacgg ggaggaacca 5520
atggaggcca aagaaatggg gtagcaacag tccaggagac agacaaggag acaaggagag 5580
ggcgcccgaa agatcggaaa aacaaacatg tccaattggg gcagtgacgg aaacgacacg 5640
gacacttcag tacaatggac cgaccatctc caagccaggg ttattccggt atcaccttgg 5700
ccgtaacctc ccgctggtac ctgatattgt acacgttcac attcaatata ctttcagcta 5760
caataagaga ggctgtttgt cgggcatgtg tgtccgtcgt atggggtgat gtccgagggc 5820
gaaattcgct acaagcttaa ctctggcgct tgtccagtat gaatagacaa gtcaagacca 5880
gtggtgccat gattgacagg gaggtacaag acttcgatac tcgagcatta ctcggacttg 5940
tggcgattga acagacgggc gatcgcttct cccccgtatt gccggcgcgc cagctgcatt 6000

aatgaatcgg ccaacgcgcg gggagaggcg gtttgcgtat tgggcgctct tccgcttcct 6060
cgctcactga ctcgctgcgc tcggtcgttc ggctgcggcg agcggtatca gctcactcaa 6120
aggcggtaat acggttatcc acagaatcag gggataacgc aggaaagaac atgtgagcaa 6180
aaggccagca aaaggccagg aaccgtaaaa aggccgcgtt gctggcgttt ttccataggc 6240
tccgcccccc tgacgagcat cacaaaaatc gacgctcaag tcagaggtgg cgaaacccga 6300
caggactata aagataccag gcgtttcccc ctggaagctc cctcgtgcgc tctcctgttc 6360
cgaccctgcc gcttaccgga tacctgtccg cctttctccc ttcgggaagc gtggcgcttt 6420
ctcatagctc acgctgtagg tatctcagtt cggtgtaggt cgttcgctcc aagctgggct 6480
gtgtgcacga accccccgtt cagcccgacc gctgcgcctt atccggtaac tatcgtcttg 6540
agtccaaccc ggtaagacac gacttatcgc cactggcagc agccactggt aacaggatta 6600
gcagagcgag gtatgtaggc ggtgctacag agttcttgaa gtggtggcct aactacggct 6660
acactagaag aacagtattt ggtatctgcg ctctgctgaa gccagttacc ttcggaaaaa 6720
gagttggtag ctcttgatcc ggcaaacaaa ccaccgctgg tagcggtggt ttttttgttt 6780
gcaagcagca gattacgcgc agaaaaaaag gatctcaaga agatcctttg atcttttcta 6840
cggggtctga cgctcagtgg aacgaaaact cacgttaagg gattttggtc atgagattat 6900
caaaaaggat cttcacctag atccttttaa attaaaaatg aagttttaaa tcaatctaaa 6960
gtatatatga gtaaacttgg tctgacagtt accaatgctt aatcagtgag gcacctatct 7020
cagcgatctg tctatttcgt tcatccatag ttgcctgact ccccgtcgtg tagataacta 7080
cgatacggga gggcttacca tctggcccca gtgctgcaat gataccgcga gacccacgct 7140
caccggctcc agatttatca gcaataaacc agccagccgg aagggccgag cgcagaagtg 7200
gtcctgcaac tttatccgcc tccatccagt ctattaattg ttgccgggaa gctagagtaa 7260
gtagttcgcc agttaatagt ttgcgcaacg ttgttgccat tgctacaggc atcgtggtgt 7320
cacgctcgtc gtttggtatg gcttcattca gctccggttc ccaacgatca aggcgagtta 7380
catgatcccc catgttgtgc aaaaaagcgg ttagctcctt cggtcctccg atcgttgtca 7440
gaagtaagtt ggccgcagtg ttatcactca tggttatggc agcactgcat aattctctta 7500
ctgtcatgcc atccgtaaga tgcttttctg tgactggtga gtactcaacc aagtcattct 7560
gagaatagtg tatgcggcga ccgagttgct cttgcccggc gtcaatacgg gataataccg 7620
cgccacatag cagaacttta aaagtgctca tcattggaaa acgttcttcg gggcgaaaac 7680
tctcaaggat cttaccgctg ttgagatcca gttcgatgta acccactcgt gcacccaact 7740
gatcttcagc atcttttact ttcaccagcg tttctgggtg agcaaaaaca ggaaggcaaa 7800
atgccgcaaa aaagggaata agggcgacac ggaaatgttg aatactcata ctcttccttt 7860
ttcaatatta ttgaagcatt tatcagggtt attgtctcat gagcggatac atatttgaat 7920

gtatttagaa aaataaacaa ataggggttc cgcgcacatt tccccgaaaa gtgccacctg 7980
atgcggtgtg aaataccgca cagatgcgta aggagaaaat accgcatcag gaaattgtaa 8040
gcgttaatat tttgttaaaa ttcgcgttaa atttttgtta aatcagctca ttttttaacc 8100
aataggccga aatcggcaaa atcccttata aatcaaaaga atagaccgag atagggttga 8160
gtgttgttcc agtttggaac aagagtccac tattaaagaa cgtggactcc aacgtcaaag 8220
ggcgaaaaac cgtctatcag ggcgatggcc cactacgtga accatcaccc taatcaagtt 8280
ttttggggtc gaggtgccgt aaagcactaa atcggaaccc taaagggagc ccccgattta 8340
gagcttgacg gggaaagccg gcgaacgtgg cgagaaagga agggaagaaa gcgaaaggag 8400
cgggcgctag ggcgctggca agtgtagcgg tcacgctgcg cgtaaccacc acacccgccg 8460
cgcttaatgc gccgctacag ggcgcgtcca ttcgccattc aggctgcgca actgttggga 8520
agggcgatcg gtgcgggcct cttcgctatt acgccagctg gcgaaagggg gatgtgctgc 8580
aaggcgatta agttgggtaa cgccagggtt ttcccagtca cgacgttgta aaacgacggc 8640
cagtgaattg taatacgact cactataggg cgaattgggc ccgacgtcgc atgcgctgat 8700
gacactttgg tctgaaagag atgcattttg aatcccaaac ttgcagtgcc caagtgacat 8760
acatctccgc gttttggaaa atgttcagaa acagttgatt gtgttggaat ggggaatggg 8820
gaatggaaaa atgactcaag tatcaattcc aaaaacttct ctggctggca gtacctactg 8880
tccatactac tgcattttct ccagtcaggc cactctatac tcgacgacac agtagtaaaa 8940
cccagataat ttcgacataa acaagaaaac agacccaata atatttatat atagtcagcc 9000
gtttgtccag ttcagactgt aatagccgaa aaaaaatcca aagtttctat tctaggaaaa 9060
tatattccaa tatttttaat tcttaatctc atttatttta ttctagcgaa atacatttca 9120
gctacttgag acatgtgata cccacaaatc ggattcggac tcggttgttc agaagagcat 9180
atggcattcg tgctcgcttg ttcacgtatt cttcctgttc catctcttgg ccgacaatca 9240
cacaaaaatg gggttttttt tttaattcta atgattcatt acagcaaaat tgagatatag 9300
cagaccacgt attccataat caccaaggaa gttcttgggc gtcttaatta actcacctgc 9360
aggattgaga ctatgaatgg attcccgtgc ccgtattact ctactaattt gatcttggaa 9420
cgcgaaaata cgtttctagg actccaaaga atctcaactc ttgtccttac taaatatact 9480
acccatagtt gatggtttac ttgaacagag aggacatgtt cacttgaccc aaagtttctc 9540
gcatctcttg gatatttgaa caacggcgtc cactgaccgt cagttatcca gtcacaaaac 9600
ccccacattc atacattccc atgtacgttt acaaagttct caattccatc gtgcaaatca 9660
aaatcacatc tattcattca tcatatataa acccatcatg tctactaaca ctcacaactc 9720
catagaaaac atcgactcag aacacacgct ccatgcggcc gcttactgag ccttggcacc 9780
gggctgcttc tcggccattc gagcgaactg ggacaggtat cggagcagga tgacgagacc 9840
ttcatggggc agagggtttc ggtaggggag gttgtgcttc tggcacagct gttccacctg 9900

gtaggaaacg gcagtgaggt tgtgtcgagg cagggtgggc cagagatggt gctcgatctg 9960
gtagttcagg cctccaaaga accagtcagt aatgatgcct cgtcgaatgt tcatggtctc 10020
atggatctga cccacagaga agccatgtcc gtcccagacg gaatcaccga tcttctccag 10080
agggtagtgg ttcatgaaga ccacgatggc aattccgaag ccaccgacga gctcggaaac 10140
aaagaacacc agcatcgagg tcaggatgga gggcataaag aagaggtgga acagggtctt 10200
gagagtccag tgcagagcga gtccaatggc ctctttcttg tactgagatc ggtagaactg 10260
gttgtctcgg tccttgaggg atcgaacggt cagcacagac tggaaacacc agatgaatcg 10320
caggagaata cagatgacca ggaaatagta ctgttggaac tgaatgagct ttcgggagat 10380
gggagaagct cgagtgacat cgtcctcgga ccaggcgagc agaggcaggt tatcaatgtc 10440
gggatcgtga ccctgaacgt tggtagcaga atgatgggcg ttgtgtctgt ccttccacca 10500
ggtcacggag aagccctgga gtccgttgcc aaagaccaga cccaggacgt tattccagtt 10560
tcggttcttg aaggtctggt ggtggcagat gtcatgagac agccatccca tttgctggta 10620
gtgcataccg agcacgagag caccaatgaa gtacaggtgg tactggacca gcatgaagaa 10680
ggcaagcacg ccaagaccca gggtggtcaa gatcttgtac gagtaccaga ggggagaggc 10740
gtcaaacatg ccagtggcga tcagctcttc tcggagcttt cggaaatcct cctgagcttc 10800
gttgacggca gcctggggag gcagctcgga agcctggttg atcttgggca ttcgcttgag 10860
cttgtcgaag gcttcctgag agtgcataac catgaaggcg tcagtagcat ctcgtccctg 10920
gtagttctca atgatttcag ctccaccagg gtggaagttc acccaagcgg agacgtcgta 10980
cacctttccg tcgatgacga ggggcagagc ctgtcgagaa gccttcacgg atcccatgac 11040
ggccagagag tcgtagtagg tagcgggagg aagtccggca ggtcgagcgg gaccggcgcc 11100
ctgaatcttt ttggctccct tgtgctttcg gacgatgtag gtctgcacgt agaagttgag 11160
gaacagacac aggacagtac caacgtagaa gtagttgaaa aaccagccaa acattctcat 11220
tccatcttgt cggtagcagg gaatgttccg gtacttccag acgatgtaga agccaacgtt 11280
gaactgaatg atctgcatag aagtaatcag ggacttgggc atagggaact tgagcttgat 11340
cagtcgggtc caatagtagc cgtacatgat ccagtgaatg aagccgttga gcagcacaaa 11400
gatccaaacg gcttcgtttc ggtagttgta gaacagccac atgtccatag gagctccgag 11460
atggtgaaag aactgcaacc aggtcagagg cttgcccatg aggggcagat agaaggagtc 11520
aatgtactcg aggaacttgc tgaggtagaa cagctgagtg gtgattcgga agacattgtt 11580
gtcgaaagcc ttctcgcagt tgtcggacat gacaccaatg gtgtacatgg cgtaggccat 11640
agagaggaag gagcccagcg agtagatgga catgagcagg ttgtagttgg tgaacacaaa 11700
cttcattcga gactgaccct tgggtccgag aggaccaagg gtgaacttca ggatgacgaa 11760
ggcgatggag aggtacagca cctcgcagtg cgaggcatca gaccagagct gagcatagtc 11820

gaccttggga agaacctcct ggccaatgga gacgatttcg ttcacgacct ccatggttgt 11880

gaattagggt ggtgagaatg gttggttgta gggaagaatc aaaggccggt ctcgggatcc 11940

gtgggtatat atatatatat atatatatac gatccttcgt tacctccctg ttctcaaaac 12000

tgtggttttt cgtttttcgt tttttgcttt ttttgatttt tttagggcca actaagcttc 12060

cagatttcgc taatcacctt tgtactaatt acaagaaagg aagaagctga ttagagttgg 12120

gctttttatg caactgtgct actccttatc tctgatatga aagtgtagac ccaatcacat 12180

catgtcattt agagttggta atactgggag gatagataag gcacgaaaac gagccatagc 12240

agacatgctg ggtgtagcca agcagaagaa agtagatggg agccaattga cgagcgaggg 12300

agctacgcca atccgacata cgacacgctg agatcgtctt ggccgggggg tacctacaga 12360

tgtccaaggg taagtgcttg actgtaattg tatgtctgag gacaaatatg tagtcagccg 12420

tataaagtca taccaggcac cagtgccatc atcgaaccac taactctcta tgatacatgc 12480

ctccggtatt attgtaccat gcgtcgcttt gttacatacg tatcttgcct tttctctca 12540

gaaactccag aattctctct cttgagcttt tccataacaa gttcttctgc ctccaggaag 12600

tccatgggtg gtttgatcat ggttttggtg tagtggtagt gcagtggtgg tattgtgact 12660

ggggatgtag ttgagaataa gtcatacaca agtcagcttt cttcgagcct catataagta 12720

taagtagttc aacgtattag cactgtaccc agcatctccg tatcgagaaa cacaacaaca 12780

tgccccattg gacagatcat gcggatacac aggttgtgca gtatcataca tactcgatca 12840

gacaggtcgt ctgaccatca tacaagctga acaagcgctc catacttgca cgctctctat 12900

atacacagtt aaattacata tccatagtct aacctctaac agttaatctt ctggtaagcc 12960

tcccagccag ccttctggta tcgcttggcc tcctcaatag gatctcggtt ctggccgtac 13020

agacctcggc cgacaattat gatatccgtt ccggtagaca tgacatcctc aacagttcgg 13080

tactgctgtc cgagagcgtc tcccttgtcg tcaagaccca ccccgggggt cagaataagc 13140

cagtcctcag agtcgccctt aggtcggttc tgggcaatga agccaaccac aaactcgggg 13200

tcggatcggg caagctcaat ggtctgcttg gagtactcgc cagtggccag agagcccttg 13260

caagacagct cggccagcat gagcagacct ctggccagct tctcgttggg agaggggact 13320

aggaactcct tgtactggga gttctcgtag tcagagacgt cctccttctt ctgttcagag 13380

acagtttcct cggcaccagc tcgcaggcca gcaatgattc cggttccggg tacaccgtgg 13440

gcgttggtga tatcggacca ctcggcgatt cggtgacacc ggtactggtg cttgacagtg 13500

ttgccaatat ctgcgaactt tctgtcctcg aacaggaaga aaccgtgctt aagagcaagt 13560

tccttgaggg ggagcacagt gccggcgtag gtgaagtcgt caatgatgtc gatatgggtt 13620

ttgatcatgc acacataagg tccgacctta tcggcaagct caatgagctc cttggtggtg 13680

gtaacatcca gagaagcaca caggttggtt ttcttggctg ccacgagctt gagcactcga 13740

gcggcaaagg cggacttgtg gacgttagct cgagcttcgt aggagggcat tttggtggtg 13800

aagaggagac tgaaataaat ttagtctgca gaacttttta tcggaacctt atctggggca 13860

gtgaagtata tgttatggta atagttacga gttagttgaa cttatagata gactggacta 13920

tacggctatc ggtccaaatt agaaagaacg tcaatggctc tctgggcgtc gcctttgccg 13980

acaaaaatgt gatcatgatg aaagccagca atgacgttgc agctgatatt gttgtcggcc 14040

aaccgcgccg aaaacgcagc tgtcagaccc acagcctcca acgaagaatg tatcgtcaaa 14100

gtgatccaag cacactcata gttggagtcg tactccaaag gcggcaatga cgagtcagac 14160

agatactcgt cgaccttttc cttgggaacc accaccgtca gcccttctga ctcacgtatt 14220

gtagccaccg acacaggcaa cagtccgtgg atagcagaat atgtcttgtc ggtccatttc 14280

tcaccaactt taggcgtcaa gtgaatgttg cagaagaagt atgtgccttc attgagaatc 14340

ggtgttgctg atttcaataa agtcttgaga tcagtttggc cagtcatgtt gtgggggta 14400

attggattga gttatcgcct acagtctgta caggtatact cgctgcccac tttatacttt 14460

ttgattccgc tgcacttgaa gcaatgtcgt ttaccaaaag tgagaatgct ccacagaaca 14520

caccccaggg tatggttgag caaaaaataa acactccgat acggggaatc gaaccccggt 14580

ctccacggtt ctcaagaagt attcttgatg agagcgtat 14619

<210> 136
<211> 15262
<212> DNA
<213> Artificial Sequence

<220>
<223> Plasmid pZK16-ML8N

<400> 136

```
gtacgcattc ttgacttgct ccccagttgc ccttttttggt cataactcag ccccgactct      60
ctccccaaag aggagatgga cttacaatag atacgagaca acagatattt tcggccacct     120
ggtgcaattg gtctgtttta ttacccccaa taatatccac agcgtccgaa tcagctcgac     180
aagctctttc tcagcatact gatcttaccc catacccccc agacagcggt gccatatcca     240
tacccgcact acctgagaca gtaaactcag ggcaaggagc ggtcttggtc catcgcgacg     300
ctgtaagctt tgcctttgcg ttttgtgatc caccacacgg cagtcaaggt ctcggttgaa     360
agatgggtct ggcaatgtgg attgctgaat cattgacata taaatacgag tgatcctgcc     420
atttcccaat ctttagaaat catcttttct tcacacatac aaaaaacaca catcacaatg     480
cagttctccg ccgccgccgt ccttgccctc gccgccttcg tctctgccca ggccgcctcc     540
gctgcttctg aagctggtgg tgccgttgtc tccggtgcct ccggtgccgc tgccgccgct     600
tcttctgctg tcattggctc cgagtctcag gccgcttctt ctgctgcctc ttctgctgct     660
gagtctgctt cttccgctgc ctctgagtcc gcctcctccg cttccgcctc cgagtccaag     720
gcctctgagt ccgcttccaa gtccgcctcc aaggcttctg agtctgcttc caaggccgag     780
```

tcttctgctg ccaaggcttc ctccgctgct ggttctgctt cttccgccgc tgcctctgcc 840
accaagtctg gtgatgcctc cggctcttcc aagccttccg gctctgcctc ctctaccggt 900
gacaagtccg gtgctggtat tgttgccccc gctgttggtg ccatcgttgt cggtgctctt 960
gctcagctca tctaagcggt ctttgtacaa ctacccactt aatatttata ttatgatata 1020
gactgcaata ctattgcatg tgtaatgctt cgtttgaaag tcgtagtccg gccattcatg 1080
gcagtgtatg gcagtattgc aaccaattgg cttaataaca tatacaaagt ggcaattctg 1140
tgttcgaacc atcacacaac ccaacatatt tgacattgct acaagtattc gacttgttct 1200
tcggccgcac gacatgcatt gctagcagca cacttgcaga agcttggcca atggttaggt 1260
tgacctttaa ccagtaaaac gcaatatagg ttgccaatta acaaacaaca cagcccagaa 1320
ctggtgtaaa gggcttgggt tttgtgcaat caactcattg actcacctcc cccaatgctc 1380
catgaaccca tgaatccata gtacctccat cccaattcag agaggctcag aaacacgcaa 1440
ttggcaaaag taaacaaagg tctcgaaacc ttaaggggtg cagtgttgca aagttaaaat 1500
tacactgttg gatggtgttc cactcaaact gtggatggat cagacggttg cactaaacca 1560
agattcaata tgctctacaa gtagcgtgcc atgaggtgta taatatcaca catgatcaga 1620
gttaaggcct ggaaaaaaag tgtccgcggc tatgtaacac tgctgaatac agtgtgtgcc 1680
gtttccacgg actggaatga atgcactcaa cacgttggta gctaacttcc ccaactattg 1740
acttcacttt actgtagcta ctctaagtac gataatgaca tcatgaatca ccaactttat 1800
cttccccacc gaaagtaaga gatgaaagag aggctaacag cttttgtaag aaaatcacaa 1860
aatctactta ccgtattaca aaagtacaag tatcagtaac caggcgcgcc agctgcatta 1920
atgaatcggc caacgcgcgg ggagaggcgg tttgcgtatt gggcgctctt ccgcttcctc 1980
gctcactgac tcgctgcgct cggtcgttcg gctgcggcga gcggtatcag ctcactcaaa 2040
ggcggtaata cggttatcca cagaatcagg ggataacgca ggaaagaaca tgtgagcaaa 2100
aggccagcaa aaggccagga accgtaaaaa ggccgcgttg ctggcgtttt tccataggct 2160
ccgcccccct gacgagcatc acaaaaatcg acgctcaagt cagaggtggc gaaacccgac 2220
aggactataa agataccagg cgtttccccc tggaagctcc ctcgtgcgct ctcctgttcc 2280
gaccctgccg cttaccggat acctgtccgc ctttctccct tcgggaagcg tggcgctttc 2340
tcatagctca cgctgtaggt atctcagttc ggtgtaggtc gttcgctcca agctgggctg 2400
tgtgcacgaa ccccccgttc agcccgaccg ctgcgcctta tccggtaact atcgtcttga 2460
gtccaacccg gtaagacacg acttatcgcc actggcagca gccactggta acaggattag 2520
cagagcgagg tatgtaggcg gtgctacaga gttcttgaag tggtggccta actacggcta 2580
cactagaaga acagtatttg gtatctgcgc tctgctgaag ccagttacct tcggaaaaag 2640
agttggtagc tcttgatccg gcaaacaaac caccgctggt agcggtggtt tttttgtttg 2700
caagcagcag attacgcgca gaaaaaaagg atctcaagaa gatcctttga tcttttctac 2760

gggggtctgac gctcagtgga acgaaaactc acgttaaggg attttggtca tgagattatc 2820

aaaaaggatc ttcacctaga tccttttaaa ttaaaaatga agttttaaat caatctaaag 2880

tatatatgag taaacttggt ctgacagtta ccaatgctta atcagtgagg cacctatctc 2940

agcgatctgt ctatttcgtt catccatagt tgcctgactc cccgtcgtgt agataactac 3000

gatacgggag ggcttaccat ctggccccag tgctgcaatg ataccgcgag acccacgctc 3060

accggctcca gatttatcag caataaacca gccagccgga agggccgagc gcagaagtgg 3120

tcctgcaact ttatccgcct ccatccagtc tattaattgt tgccgggaag ctagagtaag 3180

tagttcgcca gttaatagtt tgcgcaacgt tgttgccatt gctacaggca tcgtggtgtc 3240

acgctcgtcg tttggtatgg cttcattcag ctccggttcc caacgatcaa ggcgagttac 3300

atgatccccc atgttgtgca aaaaagcggt tagctccttc ggtcctccga tcgttgtcag 3360

aagtaagttg gccgcagtgt tatcactcat ggttatggca gcactgcata attctcttac 3420

tgtcatgcca tccgtaagat gcttttctgt gactggtgag tactcaacca agtcattctg 3480

agaatagtgt atgcggcgac cgagttgctc ttgcccggcg tcaatacggg ataataccgc 3540

gccacatagc agaactttaa aagtgctcat cattggaaaa cgttcttcgg ggcgaaaact 3600

ctcaaggatc ttaccgctgt tgagatccag ttcgatgtaa cccactcgtg cacccaactg 3660

atcttcagca tcttttactt tcaccagcgt ttctgggtga gcaaaaacag gaaggcaaaa 3720

tgccgcaaaa aagggaataa gggcgacacg gaaatgttga atactcatac tcttcctttt 3780

tcaatattat tgaagcattt atcagggtta ttgtctcatg agcggataca tatttgaatg 3840

tatttagaaa aataaacaaa taggggttcc gcgcacattt ccccgaaaag tgccacctga 3900

tgcggtgtga aataccgcac agatgcgtaa ggagaaaata ccgcatcagg aaattgtaag 3960

cgttaatatt ttgttaaaat tcgcgttaaa tttttgttaa atcagctcat tttttaacca 4020

ataggccgaa atcggcaaaa tcccttataa atcaaaagaa tagaccgaga tagggttgag 4080

tgttgttcca gtttggaaca agagtccact attaaagaac gtggactcca acgtcaaagg 4140

gcgaaaaacc gtctatcagg gcgatggccc actacgtgaa ccatcaccct aatcaagttt 4200

tttggggtcg aggtgccgta aagcactaaa tcggaaccct aaagggagcc cccgatttag 4260

agcttgacgg ggaaagccgg cgaacgtggc gagaaaggaa gggaagaaag cgaaaggagc 4320

gggcgctagg gcgctggcaa gtgtagcggt cacgctgcgc gtaaccacca cacccgccgc 4380

gcttaatgcg ccgctacagg gcgcgtccat tcgccattca ggctgcgcaa ctgttgggaa 4440

gggcgatcgg tgcgggcctc ttcgctatta cgccagctgg cgaaaggggg atgtgctgca 4500

aggcgattaa gttgggtaac gccagggttt tcccagtcac gacgttgtaa aacgacggcc 4560

agtgaattgt aatacgactc actatagggc gaattgggcc cgacgtcgca tgctatgtac 4620

actgtgtgag tgtagagggc aatggtggtg acaactgagc tactatacac acacagcttg 4680

tactggtata agttgtgagc tacagtacaa gtaatactac aaaattcagt atgtacaagt 4740
atgccggtac ggcagtagca tgtatcaaag agagacagaa agaaggctgg aaagcgaagg 4800
ccagctgagc gtgggaaccg cagtacgtgg aagtgccaaa cctgcgacag aatgacatgg 4860
tcttgcactt tttaccggta ccggtacagt acgataccgg tacgcacttg ctacagtaga 4920
tcatagacca ccagaataac cctgcattag cgccacgttg ccaagccctc aactttcttt 4980
gcagctacac cttttacacc ttttaggctg ccaggaagag cataaatgaa ttgtacttgg 5040
gggcacagaa tttgggttga agtgttcctg ggagagaatg tgcttggctg tatagttggg 5100
tggctctcct tcatagtaga tgtgaggtta cagcacttgt agattgtgta cgcgttctgt 5160
ttaaggtggt accagtatta tgggttgacg tgaaagaaag ggaggaggca ggggtacagc 5220
aagagtgtgg gcgcacactc acacatgaag caagacctgt ttcgtcattt ctatttgttt 5280
aacaaggacg acataatata cttaggacag catgggccga ccggcaggaa taatctgcaa 5340
acacaatcgt attgtggcgt cccctctcga tcacgatagt tgctaagttt agcagtctga 5400
gagtttggct gtgacgcaag accgagagag agacagagag ttgtcccagt ttgagtactc 5460
gtaccgtact acagagtaaa tcgaaatcga gcgctcactt tctgcacttt gtgaaggcgc 5520
agcattcaac atttgtgtcc agcccctttc cgtttgaagc gacttgacca cgaaatgttg 5580
gaataaagct gtgttacata cgagacctac aagtagagtg tcggatatgc caactgaaca 5640
ggttagatga aagttactca acaacaggag agcggacacc aacgtgcgaa aatgtacgga 5700
tgaacccatt gtctgtccgc atagcaatct gttttcgcca gcagtgcttg tgtcgttgga 5760
ttgctgccat tgtactattt gtactttgtt ccattaacac tcgcatcttg ctcaccgatt 5820
agcagcactc ctgttgcctc actgggcttg tctgtttcag ggtggaatgc atgagcaatg 5880
cgatcgtcaa ggggaggttg aaagcgcgta tggaataact gtggttctat agctgagggg 5940
aggatatcga ggacagccac ggttcagtgg gggatttcgg gagctactca gtaacatgta 6000
ctcttgtccg aacgccctgc tgatggcaaa tatgacaaaa cgcaacaatg tggagcaaca 6060
tttccatttc gagagatatt acatgggttg cattggggcc tgggcatccc gtgcaataac 6120
cgagaccatg tgaaggtgca cgatcgacac ttatccagcc agtcatacgt ggtgtcgcaa 6180
gctaagcaac aatgggattc acgaatctat gtgggggaac gtgcacaggg gaaatgaaac 6240
aaaacggagg agggggtctt tgaaaatgta gagaaatata acaaaaaatc acaaaaacgc 6300
accaaaaata tgccaaaatc gtcaacagtg gaaatcacgc gcccctggct cgacttccca 6360
ctgacatacc ccccctcaac caatatcata tccaccctag ccaatcgctt aattaagttg 6420
cgacacatgt cttgatagta tcttgaattc tctctcttga gcttttccat aacaagttct 6480
tctgcctcca ggaagtccat gggtggtttg atcatggttt tggtgtagtg gtagtgcagt 6540
ggtggtattg tgactgggga tgtagttgag aataagtcat acacaagtca gctttcttcg 6600
agcctcatat aagtataagt agttcaacgt attagcactg tacccagcat ctccgtatcg 6660

agaaacacaa caacatgccc cattggacag atcatgcgga tacacaggtt gtgcagtatc 6720
atacatactc gatcagacag gtcgtctgac catcatacaa gctgaacaag cgctccatac 6780
ttgcacgctc tctatataca cagttaaatt acatatccat agtctaacct ctaacagtta 6840
atcttctggt aagcctccca gccagccttc tggtatcgct tggcctcctc aataggatct 6900
cggttctggc cgtacagacc tcggccgaca attatgatat ccgttccggt agacatgaca 6960
tcctcaacag ttcggtactg ctgtccgaga gcgtctccct tgtcgtcaag acccaccccg 7020
ggggtcagaa taagccagtc ctcagagtcg cccttaggtc ggttctgggc aatgaagcca 7080
accacaaact cggggtcgga tcgggcaagc tcaatggtct gcttggagta ctcgccagtg 7140
gccagagagc ccttgcaaga cagctcggcc agcatgagca gacctctggc cagcttctcg 7200
ttgggagagg ggactaggaa ctccttgtac tgggagttct cgtagtcaga gacgtcctcc 7260
ttcttctgtt cagagacagt ttcctcggca ccagctcgca ggccagcaat gattccggtt 7320
ccgggtacac cgtgggcgtt ggtgatatcg gaccactcgg cgattcggtg acaccggtac 7380
tggtgcttga cagtgttgcc aatatctgcg aactttctgt cctcgaacag gaagaaaccg 7440
tgcttaagag caagttcctt gaggggggagc acagtgccgg cgtaggtgaa gtcgtcaatg 7500
atgtcgatat gggttttgat catgcacaca taaggtccga ccttatcggc aagctcaatg 7560
agctccttgg tggtggtaac atccagagaa gcacacaggt tggttttctt ggctgccacg 7620
agcttgagca ctcgagcggc aaaggcggac ttgtggacgt tagctcgagc ttcgtaggag 7680
ggcattttgg tggtgaagag gagactgaaa taaatttagt ctgcagaact ttttatcgga 7740
accttatctg ggcagtgaa gtatatgtta tggtaatagt tacgagttag ttgaacttat 7800
agatagactg gactatacgg ctatcggtcc aaattagaaa gaacgtcaat ggctctctgg 7860
gcgtcgcctt tgccgacaaa aatgtgatca tgatgaaagc cagcaatgac gttgcagctg 7920
atattgttgt cggccaaccg cgccgaaaac gcagctgtca gacccacagc ctccaacgaa 7980
gaatgtatcg tcaaagtgat ccaagcacac tcatagttgg agtcgtactc caaaggcggc 8040
aatgacgagt cagacagata ctcgtcgacc ttttccttgg gaaccaccac cgtcagccct 8100
tctgactcac gtattgtagc caccgacaca ggcaacagtc cgtggatagc agaatatgtc 8160
ttgtcggtcc atttctcacc aactttaggc gtcaagtgaa tgttgcagaa gaagtatgtg 8220
ccttcattga gaatcggtgt tgctgatttc aataaagtct tgagatcagt ttggccagtc 8280
atgttgtggg gggtaattgg attgagttat cgcctacagt ctgtacaggt atactcgctg 8340
cccactttat actttttgat tccgctgcac ttgaagcaat gtcgtttacc aaaagtgaga 8400
atgctccaca gaacacaccc cagggtatgg ttgagcaaaa aataaacact ccgatacggg 8460
gaatcgaacc ccggtctcca cggttctcaa gaagtattct tgatgagagc gtatcgatag 8520
ttggagcaag ggagaaatgt agagtgtgaa agactcacta tggtccgggc ttatctcgac 8580

caatagccaa agtctggagt ttctgagaga aaaaggcaag atacgtatgt aacaaagcga 8640
cgcatggtac aataataccg gaggcatgta tcatagagag ttagtggttc gatgatggca 8700
ctggtgcctg gtatgacttt atacggctga ctacatattt gtcctcagac atacaattac 8760
agtcaagcac ttacccttgg acatctgtag gtaccccccg gccaagacga tctcagcgtg 8820
tcgtatgtcg gattggcgta gctccctcgc tcgtcaattg gctcccatct actttcttct 8880
gcttggctac acccagcatg tctgctatgg ctcgttttcg tgccttatct atcctcccag 8940
tattaccaac tctaaatgac atgatgtgat tgggtctaca ctttcatatc agagataagg 9000
agtagcacag ttgcataaaa agcccaactc taatcagctt cttcctttct tgtaattagt 9060
acaaaggtga ttagcgaaat ctggaagctt agttggccct aaaaaaatca aaaaaagcaa 9120
aaaacgaaaa acgaaaaacc acagttttga gaacagggag gtaacgaagg atcgtatata 9180
tatatatata tatatatacc cacggatccc gagaccggcc tttgattctt ccctacaacc 9240
aaccattctc accaccctaa ttcacaacca tgtctattgg ttcgtccaac cccgtgctct 9300
tggctgcgat tcccttcgtc tacctgtttg tcctcccacg agtcctggct ttcctgcctc 9360
agaaggctca gttcctggcc aaatgtattg tggtcctgat tgccacgctt atcatgtccg 9420
ttgcaggctg cttcatctcg atcgtgtgcg ctcttctgga caagagatac gtcatcaatt 9480
acgttgtgtc gcgattgttc tccttccttg ccgctcgacc gtgtggtgtg acctataaga 9540
ttgttggtga ggaacacctc gataagtacc ctgctatcgt ggtctgtaac catcaatcct 9600
ctatggatat gatggttttg ggacgagttt ttccaaagca ctgcgttgtc atggcgaaga 9660
aggaactcct gtactttccc tttttgggaa tgtttatgaa actgagcaac gctatcttca 9720
tcgaccggaa gaaccacaag aaagccatcg agtctaccac ccaagccgtg gcggacatga 9780
agaagcacaa ctctggaatc tggattttcc cagagggcac ccggtctaga ctggacaagg 9840
cagacctgct gcccttcaag aaaggtgcct ttcatcttgc aattcaggcc cagctcccta 9900
ttctccccat tatctcgcag ggctattccc atatctacga ctcttcgaag cggtacttcc 9960
ccggtggaga gctcgagatc agagtcctgg agcccattcc tacaactggc ctcactactg 10020
atgatgtgaa cgacctgatg gacaagacac gaaaccttat gctcaagcac ttgaaggaga 10080
tggattccca gtattcgtcg agcactgctg aaaatggatc cacgcacatc gacgccgata 10140
ttgccaagtc tacagccacc agcattggca acactgacga cgcaattaca aaacgtcgta 10200
cccctaagga ataagcggcc gcattgatga ttggaaacac acacatgggt tatatctagg 10260
tgagagttag ttggacagtt atatattaaa tcagctatgc caacggtaac ttcattcatg 10320
tcaacgagga accagtgact gcaagtaata tagaatttga ccaccttgcc attctcttgc 10380
actcctttac tatatctcat ttatttctta tatacaaatc acttcttctt cccagcatcg 10440
agctcggaaa cctcatgagc aataacatcg tggatctcgt caatagaggg ctttttggac 10500
tccttgctgt tggccacctt gtccttgctg tttaaaccat catctaaggg cctcaaaact 10560

acctcggaac tgctgcgctg atctggacac cacagaggtt ccgagcactt taggttgcac 10620
caaatgtccc accaggtgca ggcagaaaac gctggaacag cgtgtacagt ttgtcttaac 10680
aaaaagtgag ggcgctgagg tcgagcaggg tggtgtgact tgttatagcc tttagagctg 10740
cgaaagcgcg tatggatttg gctcatcagg ccagattgag ggtctgtgga cacatgtcat 10800
gttagtgtac ttcaatcgcc ccctggatat agccccgaca ataggccgtg gcctcatttt 10860
tttgccttcc gcacatttcc attgctcggt acccacacct tgcttctcct gcacttgcca 10920
accttaatac tggtttacat tgaccaacat cttacaagcg gggggcttgt ctagggtata 10980
tataaacagt ggctctccca atcggttgcc agtctctttt ttcctttctt tccccacaga 11040
ttcgaaatct aaactacaca tcacaccatg gtctccaacc acctgttcga cgccatgcga 11100
gctgccgctc ccggagacgc acctttcatt cgaatcgaca acgctcggac ctggacttac 11160
gatgacgcca ttgctctttc cggtcgaatc gctggagcta tggacgcact cggcattcga 11220
cccggagaca gagttgccgt gcaggtcgag aagtctgccg aggcgttgat tctctacctg 11280
gcctgtcttc gaaccggagc tgtctacctg cctctcaaca ctgcctacac cctggccgag 11340
ctcgactact tcatcggcga tgccgaaccg cgtctggtgg tcgttgctcc cgcagctcga 11400
ggtggcgtgg agacaattgc caagcgacac ggtgctatcg tcgaaaccct cgacgccgat 11460
ggacgaggct ccttgctgga ccttgctaga gatgagcctg ccgactttgt cgatgcttcg 11520
cgatctgccg acgatctggc tgctattctc tacacttccg gtacaaccgg acgatcgaag 11580
ggtgccatgc ttactcatgg caatctgctc tccaacgctc tcaccttgcg agactattgg 11640
agagttaccg cagacgatcg actcatccat gccttgccaa tctttcacac tcatggtctg 11700
ttcgttgcta cgaacgtcac actgcttgca ggagcctcga tgtttctgct ctccaagttc 11760
gatgccgacg aggtcgtttc tctcatgcca caggccacca tgcttatggg cgtgcccaca 11820
ttctacgttc gattgctgca gagtcctcga ctcgagaagg gtgctgtggc cagcatcaga 11880
ctgttcattt ctggatcagc tcccttgctt gccgaaaccc acgccgagtt tcatgctcgt 11940
actggtcacg ccattctcga gcgatacggc atgacggaaa ccaacatgaa tacttccaac 12000
ccctacgagg gcaagcgtat tgccggaacc gttggttttc ctctgcccga cgtcactgtg 12060
cgagtcaccg atcccgccac cggtctcgtt cttccacctg aagagactgg catgatcgag 12120
atcaagggac ccaacgtctt caagggctat tggcgaatgc ccgaaaagac cgctgccgag 12180
tttaccgcag acggtttctt tatctctgga gatctcggca agatcgaccg agaaggttac 12240
gttcacattg tgggacgagg caaggacctg gtcatttccg gtggctacaa catctatccc 12300
aaagaggtcg aaggcgagat cgaccagatc gagggtgtgg tcgagtctgc tgtcattggt 12360
gttcctcatc ccgatttcgg agaaggtgtc accgctgttg tcgtgtgcaa acctggtgcc 12420
gttctcgacg aaaagaccat cgtgtctgct ctgcaggacc gtcttgcccg atacaagcaa 12480

cccaagcgga ttatctttgc cgacgatctg cctcgaaaca ctatgggaaa ggttcagaag 12540

aacattcttc gacagcaata cgccgatctc tacaccagac gataagcggc cgcatgagaa 12600

gataaatata taaatacatt gagatattaa atgcgctaga ttagagagcc tcatactgct 12660

cggagagaag ccaagacgag tactcaaagg ggattacacc atccatatcc acagacacaa 12720

gctggggaaa ggttctatat acactttccg gaataccgta gtttccgatg ttatcaatgg 12780

gggcagccag gatttcaggc acttcggtgt ctcggggtga aatggcgttc ttggcctcca 12840

tcaagtcgta ccatgtcttc atttgcctgt caaagtaaaa cagaagcaga tgaagaatga 12900

acttgaagtg aaggaattta aatagttgga gcaagggaga aatgtagagt gtgaaagact 12960

cactatggtc cgggcttatc tcgaccaata gccaaagtct ggagtttctg agagaaaaag 13020

gcaagatacg tatgtaacaa agcgacgcat ggtacaataa taccggaggc atgtatcata 13080

gagagttagt ggttcgatga tggcactggt gcctggtatg actttatacg gctgactaca 13140

tatttgtcct cagacataca attacagtca agcacttacc cttggacatc tgtaggtacc 13200

ccccggccaa gacgatctca gcgtgtcgta tgtcggattg gcgtagctcc ctcgctcgtc 13260

aattggctcc catctacttt cttctgcttg gctacaccca gcatgtctgc tatggctcgt 13320

tttcgtgcct tatctatcct cccagtatta ccaactctaa atgacatgat gtgattgggt 13380

ctacactttc atatcagaga taaggagtag cacagttgca taaaaagccc aactctaatc 13440

agcttcttcc tttcttgtaa ttagtacaaa ggtgattagc gaaatctgga agcttagttg 13500

gccctaaaaa aatcaaaaaa agcaaaaaac gaaaaacgaa aaaccacagt tttgagaaca 13560

gggaggtaac gaaggatcgt atatatatat atatatatat atacccacgg atcccgagac 13620

cggcctttga ttcttcccta caaccaacca ttctcaccac cctaattcac aaccatggtg 13680

aaggcttctc gacaggctct gcccctcgtc atcgacggaa aggtgtacga cgtctccgct 13740

tgggtgaact tccaccctgg tggagctgaa atcattgaga actaccaggg acgagatgct 13800

actgacgcct tcatggttat gcactctcag gaagccttcg acaagctcaa gcgaatgccc 13860

aagatcaacc aggcttccga gctgcctccc caggctgccg tcaacgaagc tcaggaggat 13920

ttccgaaagc tccgagaaga gctgatcgcc actggcatgt ttgacgcctc tcccctctgg 13980

tactcgtaca agatcttgac caccctgggt cttggcgtgc ttgccttctt catgctggtc 14040

cagtaccacc tgtacttcat tggtgctctc gtgctcggta tgcactacca gcaaatggga 14100

tggctgtctc atgacatctg ccaccaccag accttcaaga accgaaactg gaataacgtc 14160

ctgggtctgg tctttggcaa cggactccag ggcttctccg tgacctggtg gaaggacaga 14220

cacaacgccc atcattctgc taccaacgtt cagggtcacg atcccgacat tgataacctg 14280

cctctgctcg cctggtccga ggacgatgtc actcgagctt ctcccatctc ccgaaagctc 14340

attcagttcc aacagtacta tttcctggtc atctgtattc tcctgcgatt catctggtgt 14400

ttccagtctg tgctgaccgt tcgatccctc aaggaccgag acaaccagtt ctaccgatct 14460

```
cagtacaaga aagaggccat tggactcgct ctgcactgga ctctcaagac cctgttccac    14520
ctcttcttta tgccctccat cctgacctcg atgctggtgt tctttgtttc cgagctcgtc    14580
ggtggcttcg gaattgccat cgtggtcttc atgaaccact accctctgga gaagatcggt    14640
gattccgtct gggacggaca tggcttctct gtgggtcaga tccatgagac catgaacatt    14700
cgacgaggca tcattactga ctggttcttt ggaggcctga actaccagat cgagcaccat    14760
ctctggccca ccctgcctcg acacaacctc actgccgttt cctaccaggt ggaacagctg    14820
tgccagaagc acaacctccc ctaccgaaac cctctgcccc atgaaggtct cgtcatcctg    14880
ctccgatacc tgtcccagtt cgctcgaatg gccgagaagc agcccggtgc caaggctcag    14940
taagcggccg caagtgtgga tggggaagtg agtgcccggt tctgtgtgca caattggcaa    15000
tccaagatgg atggattcaa cacagggata tagcgagcta cgtggtggtg cgaggatata    15060
gcaacggata tttatgtttg acacttgaga atgtacgata caagcactgt ccaagtacaa    15120
tactaaacat actgtacata ctcatactcg tacccgggca acggtttcac ttgagtgcag    15180
tggctagtgc tcttactcgt acagtgtgca atactgcgta tcatagtctt tgatgtatat    15240
cgtattcatt catgttagtt gc                                               15262
```

<210> 137
<211> 13075
<212> DNA
<213> Artificial Sequence

<220>
<223> Plasmid pZK16-ML

<400> 137

```
gtacgcattc ttgacttgct ccccagttgc cctttttggt cataactcag ccccgactct      60
ctccccaaag aggagatgga cttacaatag atacgagaca acagatattt tcggccacct     120
ggtgcaattg gtctgtttta ttacccccaa taatatccac agcgtccgaa tcagctcgac     180
aagctctttc tcagcatact gatcttaccc catacccccc agacagcggt gccatatcca     240
tacccgcact acctgagaca gtaaactcag ggcaaggagc ggtcttggtc catcgcgacg     300
ctgtaagctt tgcctttgcg ttttgtgatc caccacacgg cagtcaaggt ctcggttgaa     360
agatgggtct ggcaatgtgg attgctgaat cattgacata taaatacgag tgatcctgcc     420
atttcccaat ctttagaaat catcttttct tcacacatac aaaaaacaca catcacaatg     480
cagttctccg ccgccgccgt ccttgccctc gccgccttcg tctctgccca ggccgcctcc     540
gctgcttctg aagctggtgg tgccgttgtc tccggtgcct ccggtgccgc tgccgccgct     600
tcttctgctg tcattggctc cgagtctcag gccgcttctt ctgctgcctc ttctgctgct     660
gagtctgctt cttccgctgc ctctgagtcc gcctcctccg cttccgcctc cgagtccaag     720
gcctctgagt ccgcttccaa gtccgcctcc aaggcttctg agtctgcttc caaggccgag     780
```

tcttctgctg ccaaggcttc ctccgctgct ggttctgctt cttccgccgc tgcctctgcc 840
accaagtctg gtgatgcctc cggctcttcc aagccttccg gctctgcctc ctctaccggt 900
gacaagtccg gtgctggtat tgttgccccc gctgttggtg ccatcgttgt cggtgctctt 960
gctcagctca tctaagcggt ctttgtacaa ctacccactt aatatttata ttatgatata 1020
gactgcaata ctattgcatg tgtaatgctt cgtttgaaag tcgtagtccg gccattcatg 1080
gcagtgtatg gcagtattgc aaccaattgg cttaataaca tatacaaagt ggcaattctg 1140
tgttcgaacc atcacacaac ccaacatatt tgacattgct acaagtattc gacttgttct 1200
tcggccgcac gacatgcatt gctagcagca cacttgcaga agcttggcca atggttaggt 1260
tgacctttaa ccagtaaaac gcaatatagg ttgccaatta acaaacaaca cagcccagaa 1320
ctggtgtaaa gggcttgggt tttgtgcaat caactcattg actcacctcc cccaatgctc 1380
catgaaccca tgaatccata gtacctccat cccaattcag agaggctcag aaacacgcaa 1440
ttggcaaaag taaacaaagg tctcgaaacc ttaaggggtg cagtgttgca aagttaaaat 1500
tacactgttg gatggtgttc cactcaaact gtggatggat cagacggttg cactaaacca 1560
agattcaata tgctctacaa gtagcgtgcc atgaggtgta taatatcaca catgatcaga 1620
gttaaggcct ggaaaaaaag tgtccgcggc tatgtaacac tgctgaatac agtgtgtgcc 1680
gtttccacgg actggaatga atgcactcaa cacgttggta gctaacttcc ccaactattg 1740
acttcacttt actgtagcta ctctaagtac gataatgaca tcatgaatca ccaactttat 1800
cttccccacc gaaagtaaga gatgaaagag aggctaacag cttttgtaag aaaatcacaa 1860
aatctactta ccgtattaca aaagtacaag tatcagtaac caggcgcgcc agctgcatta 1920
atgaatcggc caacgcgcgg ggagaggcgg tttgcgtatt gggcgctctt ccgcttcctc 1980
gctcactgac tcgctgcgct cggtcgttcg gctgcggcga gcggtatcag ctcactcaaa 2040
ggcggtaata cggttatcca cagaatcagg ggataacgca ggaaagaaca tgtgagcaaa 2100
aggccagcaa aaggccagga accgtaaaaa ggccgcgttg ctggcgtttt tccataggct 2160
ccgcccccct gacgagcatc acaaaaatcg acgctcaagt cagaggtggc gaaacccgac 2220
aggactataa agataccagg cgtttccccc tggaagctcc ctcgtgcgct ctcctgttcc 2280
gaccctgccg cttaccggat acctgtccgc ctttctccct tcgggaagcg tggcgctttc 2340
tcatagctca cgctgtaggt atctcagttc ggtgtaggtc gttcgctcca agctgggctg 2400
tgtgcacgaa ccccccgttc agcccgaccg ctgcgcctta tccggtaact atcgtcttga 2460
gtccaacccg gtaagacacg acttatcgcc actggcagca gccactggta acaggattag 2520
cagagcgagg tatgtaggcg gtgctacaga gttcttgaag tggtggccta actacggcta 2580
cactagaaga acagtatttg gtatctgcgc tctgctgaag ccagttacct tcggaaaaag 2640
agttggtagc tcttgatccg gcaaacaaac caccgctggt agcggtggtt tttttgtttg 2700
caagcagcag attacgcgca gaaaaaaagg atctcaagaa gatcctttga tcttttctac 2760

ggggtctgac gctcagtgga acgaaaactc acgttaaggg attttggtca tgagattatc 2820
aaaaaggatc ttcacctaga tccttttaaa ttaaaaatga agttttaaat caatctaaag 2880
tatatatgag taaacttggt ctgacagtta ccaatgctta atcagtgagg cacctatctc 2940
agcgatctgt ctatttcgtt catccatagt tgcctgactc cccgtcgtgt agataactac 3000
gatacgggag ggcttaccat ctggccccag tgctgcaatg ataccgcgag acccacgctc 3060
accggctcca gatttatcag caataaacca gccagccgga agggccgagc gcagaagtgg 3120
tcctgcaact ttatccgcct ccatccagtc tattaattgt tgccgggaag ctagagtaag 3180
tagttcgcca gttaatagtt tgcgcaacgt tgttgccatt gctacaggca tcgtggtgtc 3240
acgctcgtcg tttggtatgg cttcattcag ctccggttcc caacgatcaa ggcgagttac 3300
atgatccccc atgttgtgca aaaaagcggt tagctccttc ggtcctccga tcgttgtcag 3360
aagtaagttg gccgcagtgt tatcactcat ggttatggca gcactgcata attctcttac 3420
tgtcatgcca tccgtaagat gcttttctgt gactggtgag tactcaacca agtcattctg 3480
agaatagtgt atgcggcgac cgagttgctc ttgcccggcg tcaatacggg ataataccgc 3540
gccacatagc agaactttaa aagtgctcat cattggaaaa cgttcttcgg ggcgaaaact 3600
ctcaaggatc ttaccgctgt tgagatccag ttcgatgtaa cccactcgtg cacccaactg 3660
atcttcagca tcttttactt tcaccagcgt ttctgggtga gcaaaaacag gaaggcaaaa 3720
tgccgcaaaa aagggaataa gggcgacacg gaaatgttga atactcatac tcttcctttt 3780
tcaatattat tgaagcattt atcagggtta ttgtctcatg agcggataca tatttgaatg 3840
tatttagaaa aataaacaaa taggggttcc gcgcacattt ccccgaaaag tgccacctga 3900
tgcggtgtga aataccgcac agatgcgtaa ggagaaaata ccgcatcagg aaattgtaag 3960
cgttaatatt ttgttaaaat tcgcgttaaa tttttgttaa atcagctcat tttttaacca 4020
ataggccgaa atcggcaaaa tcccttataa atcaaaagaa tagaccgaga tagggttgag 4080
tgttgttcca gtttggaaca agagtccact attaaagaac gtggactcca acgtcaaagg 4140
gcgaaaaacc gtctatcagg gcgatggccc actacgtgaa ccatcaccct aatcaagttt 4200
tttggggtcg aggtgccgta aagcactaaa tcggaaccct aaagggagcc cccgatttag 4260
agcttgacgg ggaaagccgg cgaacgtggc gagaaaggaa gggaagaaag cgaaaggagc 4320
gggcgctagg gcgctggcaa gtgtagcggt cacgctgcgc gtaaccacca cacccgccgc 4380
gcttaatgcg ccgctacagg gcgcgtccat tcgccattca ggctgcgcaa ctgttgggaa 4440
gggcgatcgg tgcgggcctc ttcgctatta cgccagctgg cgaaaggggg atgtgctgca 4500
aggcgattaa gttgggtaac gccagggttt tcccagtcac gacgttgtaa aacgacggcc 4560
agtgaattgt aatacgactc actatagggc gaattgggcc cgacgtcgca tgctatgtac 4620
actgtgtgag tgtagagggc aatggtggtg acaactgagc tactatacac acacagcttg 4680

tactggtata agttgtgagc tacagtacaa gtaatactac aaaattcagt atgtacaagt 4740
atgccggtac ggcagtagca tgtatcaaag agagacagaa agaaggctgg aaagcgaagg 4800
ccagctgagc gtgggaaccg cagtacgtgg aagtgccaaa cctgcgacag aatgacatgg 4860
tcttgcactt tttaccggta ccggtacagt acgataccgg tacgcacttg ctacagtaga 4920
tcatagacca ccagaataac cctgcattag cgccacgttg ccaagccctc aactttcttt 4980
gcagctacac cttttacacc ttttaggctg ccaggaagag cataaatgaa ttgtacttgg 5040
gggcacagaa tttgggttga agtgttcctg ggagagaatg tgcttggctg tatagttggg 5100
tggctctcct tcatagtaga tgtgaggtta cagcacttgt agattgtgta cgcgttctgt 5160
ttaaggtggt accagtatta tgggttgacg tgaaagaaag ggaggaggca ggggtacagc 5220
aagagtgtgg gcgcacactc acacatgaag caagacctgt ttcgtcattt ctatttgttt 5280
aacaaggacg acataatata cttaggacag catgggccga ccggcaggaa taatctgcaa 5340
acacaatcgt attgtggcgt cccctctcga tcacgatagt tgctaagttt agcagtctga 5400
gagtttggct gtgacgcaag accgagagag agacagagag ttgtcccagt ttgagtactc 5460
gtaccgtact acagagtaaa tcgaaatcga gcgctcactt tctgcacttt gtgaaggcgc 5520
agcattcaac atttgtgtcc agcccctttc cgtttgaagc gacttgacca cgaaatgttg 5580
gaataaagct gtgttacata cgagacctac aagtagagtg tcggatatgc caactgaaca 5640
ggttagatga aagttactca acaacaggag agcggacacc aacgtgcgaa aatgtacgga 5700
tgaacccatt gtctgtccgc atagcaatct gttttcgcca gcagtgcttg tgtcgttgga 5760
ttgctgccat tgtactattt gtactttgtt ccattaacac tcgcatcttg ctcaccgatt 5820
agcagcactc ctgttgcctc actgggcttg tctgtttcag ggtggaatgc atgagcaatg 5880
cgatcgtcaa ggggaggttg aaagcgcgta tggaataact gtggttctat agctgagggg 5940
aggatatcga ggacagccac ggttcagtgg gggatttcgg gagctactca gtaacatgta 6000
ctcttgtccg aacgccctgc tgatggcaaa tatgacaaaa cgcaacaatg tggagcaaca 6060
tttccatttc gagagatatt acatgggttg cattggggcc tgggcatccc gtgcaataac 6120
cgagaccatg tgaaggtgca cgatcgacac ttatccagcc agtcatacgt ggtgtcgcaa 6180
gctaagcaac aatgggattc acgaatctat gtgggggaac gtgcacaggg gaaatgaaac 6240
aaaacggagg agggggtctt tgaaaatgta gagaaatata acaaaaaatc acaaaaacgc 6300
accaaaaata tgccaaaatc gtcaacagtg gaaatcacgc gcccctggct cgacttccca 6360
ctgacatacc ccccctcaac caatatcata tccaccctag ccaatcgctt aattaagttg 6420
cgacacatgt cttgatagta tcttgaattc tctctcttga gcttttccat aacaagttct 6480
tctgcctcca ggaagtccat gggtggtttg atcatggttt tggtgtagtg gtagtgcagt 6540
ggtggtattg tgactgggga tgtagttgag aataagtcat acacaagtca gctttcttcg 6600
agcctcatat aagtataagt agttcaacgt attagcactg tacccagcat ctccgtatcg 6660

agaaacacaa caacatgccc cattggacag atcatgcgga tacacaggtt gtgcagtatc 6720
atacatactc gatcagacag gtcgtctgac catcatacaa gctgaacaag cgctccatac 6780
ttgcacgctc tctatataca cagttaaatt acatatccat agtctaacct ctaacagtta 6840
atcttctggt aagcctccca gccagccttc tggtatcgct tggcctcctc aataggatct 6900
cggttctggc cgtacagacc tcggccgaca attatgatat ccgttccggt agacatgaca 6960
tcctcaacag ttcggtactg ctgtccgaga gcgtctccct tgtcgtcaag acccaccccg 7020
ggggtcagaa taagccagtc ctcagagtcg cccttaggtc ggttctgggc aatgaagcca 7080
accacaaact cggggtcgga tcgggcaagc tcaatggtct gcttggagta ctcgccagtg 7140
gccagagagc ccttgcaaga cagctcggcc agcatgagca gacctctggc cagcttctcg 7200
ttgggagagg ggactaggaa ctccttgtac tgggagttct cgtagtcaga gacgtcctcc 7260
ttcttctgtt cagagacagt ttcctcggca ccagctcgca ggccagcaat gattccggtt 7320
ccgggtacac cgtgggcgtt ggtgatatcg gaccactcgg cgattcggtg acaccggtac 7380
tggtgcttga cagtgttgcc aatatctgcg aactttctgt cctcgaacag gaagaaaccg 7440
tgcttaagag caagttcctt gagggggagc acagtgccgg cgtaggtgaa gtcgtcaatg 7500
atgtcgatat gggttttgat catgcacaca taaggtccga ccttatcggc aagctcaatg 7560
agctccttgg tggtggtaac atccagagaa gcacacaggt tggttttctt ggctgccacg 7620
agcttgagca ctcgagcggc aaaggcggac ttgtggacgt tagctcgagc ttcgtaggag 7680
ggcattttgg tggtgaagag gagactgaaa taaatttagt ctgcagaact ttttatcgga 7740
accttatctg gggcagtgaa gtatatgtta tggtaatagt tacgagttag ttgaacttat 7800
agatagactg gactatacgg ctatcggtcc aaattagaaa gaacgtcaat ggctctctgg 7860
gcgtcgcctt tgccgacaaa aatgtgatca tgatgaaagc cagcaatgac gttgcagctg 7920
atattgttgt cggccaaccg cgccgaaaac gcagctgtca gacccacagc ctccaacgaa 7980
gaatgtatcg tcaaagtgat ccaagcacac tcatagttgg agtcgtactc caaaggcggc 8040
aatgacgagt cagacagata ctcgtcgacc ttttccttgg gaaccaccac cgtcagccct 8100
tctgactcac gtattgtagc caccgacaca ggcaacagtc cgtggatagc agaatatgtc 8160
ttgtcggtcc atttctcacc aactttaggc gtcaagtgaa tgttgcagaa gaagtatgtg 8220
ccttcattga gaatcggtgt tgctgatttc aataaagtct tgagatcagt ttggccagtc 8280
atgttgtggg gggtaattgg attgagttat cgcctacagt ctgtacaggt atactcgctg 8340
cccactttat actttttgat tccgctgcac ttgaagcaat gtcgtttacc aaaagtgaga 8400
atgctccaca gaacacaccc cagggtatgg ttgagcaaaa aataaacact ccgatacggg 8460
gaatcgaacc ccggtctcca cggttctcaa gaagtattct tgatgagagc gtatcgatag 8520
ttggagcaag ggagaaatgt agagtgtgaa agactcacta tggtccgggc ttatctcgac 8580

caatagccaa agtctggagt ttctgagaga aaaaggcaag atacgtatgt aacaaagcga 8640
cgcatggtac aataataccg gaggcatgta tcatagagag ttagtggttc gatgatggca 8700
ctggtgcctg gtatgacttt atacggctga ctacatattt gtcctcagac atacaattac 8760
agtcaagcac ttacccttgg acatctgtag gtacccccg gccaagacga tctcagcgtg 8820
tcgtatgtcg gattggcgta gctccctcgc tcgtcaattg gctcccatct actttcttct 8880
gcttggctac acccagcatg tctgctatgg ctcgttttcg tgccttatct atcctcccag 8940
tattaccaac tctaaatgac atgatgtgat tgggtctaca ctttcatatc agagataagg 9000
agtagcacag ttgcataaaa agcccaactc taatcagctt cttcctttct tgtaattagt 9060
acaaaggtga ttagcgaaat ctggaagctt agttggccct aaaaaaatca aaaaaagcaa 9120
aaaacgaaaa acgaaaaacc acagttttga gaacagggag gtaacgaagg atcgtatata 9180
tatatatata tatatatacc cacggatccc gagaccggcc tttgattctt ccctacaacc 9240
aaccattctc accaccctaa ttcacaacca tgtctattgg ttcgtccaac cccgtgctct 9300
tggctgcgat tcccttcgtc tacctgtttg tcctcccacg agtcctggct ttcctgcctc 9360
agaaggctca gttcctggcc aaatgtattg tggtcctgat tgccacgctt atcatgtccg 9420
ttgcaggctg cttcatctcg atcgtgtgcg ctcttctgga caagagatac gtcatcaatt 9480
acgttgtgtc gcgattgttc tccttccttg ccgctcgacc gtgtggtgtg acctataaga 9540
ttgttggtga ggaacacctc gataagtacc ctgctatcgt ggtctgtaac catcaatcct 9600
ctatggatat gatggttttg ggacgagttt ttccaaagca ctgcgttgtc atggcgaaga 9660
aggaactcct gtactttccc tttttgggaa tgtttatgaa actgagcaac gctatcttca 9720
tcgaccggaa gaaccacaag aaagccatcg agtctaccac ccaagccgtg gcggacatga 9780
agaagcacaa ctctggaatc tggattttcc cagagggcac ccggtctaga ctggacaagg 9840
cagacctgct gcccttcaag aaaggtgcct ttcatcttgc aattcaggcc cagctcccta 9900
ttctccccat tatctcgcag ggctattccc atatctacga ctcttcgaag cggtacttcc 9960
ccggtggaga gctcgagatc agagtcctgg agcccattcc tacaactggc ctcactactg 10020
atgatgtgaa cgacctgatg gacaagacac gaaaccttat gctcaagcac ttgaaggaga 10080
tggattccca gtattcgtcg agcactgctg aaaatggatc cacgcacatc gacgccgata 10140
ttgccaagtc tacagccacc agcattggca acactgacga cgcaattaca aaacgtcgta 10200
cccctaagga ataagcggcc gcattgatga ttggaaacac acacatgggt tatatctagg 10260
tgagagttag ttggacagtt atatattaaa tcagctatgc caacggtaac ttcattcatg 10320
tcaacgagga accagtgact gcaagtaata tagaatttga ccaccttgcc attctcttgc 10380
actcctttac tatatctcat ttatttctta tatacaaatc acttcttctt cccagcatcg 10440
agctcggaaa cctcatgagc aataacatcg tggatctcgt caatagaggg ctttttggac 10500
tccttgctgt tggccacctt gtccttgctg tttaaaccat catctaaggg cctcaaaact 10560

acctcggaac tgctgcgctg atctggacac cacagaggtt ccgagcactt taggttgcac 10620
caaatgtccc accaggtgca ggcagaaaac gctggaacag cgtgtacagt ttgtcttaac 10680
aaaaagtgag ggcgctgagg tcgagcaggg tggtgtgact tgttatagcc tttagagctg 10740
cgaaagcgcg tatggatttg gctcatcagg ccagattgag ggtctgtgga cacatgtcat 10800
gttagtgtac ttcaatcgcc ccctggatat agccccgaca ataggccgtg gcctcatttt 10860
tttgccttcc gcacatttcc attgctcggt acccacacct tgcttctcct gcacttgcca 10920
accttaatac tggtttacat tgaccaacat cttacaagcg gggggcttgt ctagggtata 10980
tataaacagt ggctctccca atcggttgcc agtctctttt ttcctttctt tccccacaga 11040
ttcgaaatct aaactacaca tcacaccatg gtctccaacc acctgttcga cgccatgcga 11100
gctgccgctc ccggagacgc acctttcatt cgaatcgaca acgctcggac ctggacttac 11160
gatgacgcca ttgctctttc cggtcgaatc gctggagcta tggacgcact cggcattcga 11220
cccggagaca gagttgccgt gcaggtcgag aagtctgccg aggcgttgat tctctacctg 11280
gcctgtcttc gaaccggagc tgtctacctg cctctcaaca ctgcctacac cctggccgag 11340
ctcgactact tcatcggcga tgccgaaccg cgtctggtgg tcgttgctcc cgcagctcga 11400
ggtggcgtgg agacaattgc caagcgacac ggtgctatcg tcgaaaccct cgacgccgat 11460
ggacgaggct ccttgctgga ccttgctaga gatgagcctg ccgactttgt cgatgcttcg 11520
cgatctgccg acgatctggc tgctattctc tacacttccg gtacaaccgg acgatcgaag 11580
ggtgccatgc ttactcatgg caatctgctc tccaacgctc tcaccttgcg agactattgg 11640
agagttaccg cagacgatcg actcatccat gccttgccaa tctttcacac tcatggtctg 11700
ttcgttgcta cgaacgtcac actgcttgca ggagcctcga tgtttctgct ctccaagttc 11760
gatgccgacg aggtcgtttc tctcatgcca caggccacca tgcttatggg cgtgcccaca 11820
ttctacgttc gattgctgca gagtcctcga ctcgagaagg gtgctgtggc cagcatcaga 11880
ctgttcattt ctggatcagc tcccttgctt gccgaaaccc acgccgagtt tcatgctcgt 11940
actggtcacg ccattctcga gcgatacggc atgacggaaa ccaacatgaa tacttccaac 12000
ccctacgagg gcaagcgtat tgccggaacc gttggttttc ctctgcccga cgtcactgtg 12060
cgagtcaccg atcccgccac cggtctcgtt cttccacctg aagagactgg catgatcgag 12120
atcaagggac ccaacgtctt caagggctat tggcgaatgc ccgaaaagac cgctgccgag 12180
tttaccgcag acggtttctt tatctctgga gatctcggca agatcgaccg agaaggttac 12240
gttcacattg tgggacgagg caaggacctg gtcatttccg gtggctacaa catctatccc 12300
aaagaggtcg aaggcgagat cgaccagatc gagggtgtgg tcgagtctgc tgtcattggt 12360
gttcctcatc ccgatttcgg agaaggtgtc accgctgttg tcgtgtgcaa acctggtgcc 12420
gttctcgacg aaaagaccat cgtgtctgct ctgcaggacc gtcttgcccg atacaagcaa 12480

cccaagcgga ttatctttgc cgacgatctg cctcgaaaca ctatgggaaa ggttcagaag 12540

aacattcttc gacagcaata cgccgatctc tacaccagac gataagcggc cgcatgagaa 12600

gataaatata taaatacatt gagatattaa atgcgctaga ttagagagcc tcatactgct 12660

cggagagaag ccaagacgag tactcaaagg ggattacacc atccatatcc acagacacaa 12720

gctggggaaa ggttctatat acactttccg gaataccgta gtttccgatg ttatcaatgg 12780

gggcagccag gatttcaggc acttcggtgt ctcggggtga aatggcgttc ttggcctcca 12840

tcaagtcgta ccatgtcttc atttgcctgt caaagtaaaa cagaagcaga tgaagaatga 12900

acttgaagtg aaggaattta aatgtaacga aactgaaatt tgaccagata ttgtgtccgc 12960

ggtggagctc cagcttttgt tccctttagt gagggttaat ttcgagcttg gcgtaatcat 13020

ggtcatagct gtttcctgtg tgaaattgtt atccgctcac aagcttccac acaac 13075

<210> 138
<211> 15799
<212> DNA
<213> Artificial Sequence

<220>
<223> Plasmid pZKL2-5m89C

<400> 138

```
gtacgttatc atttgaacag tgaaaggcta cagtaacaga agcagttgta aacttcattc      60
cgttgattct gtactacagt accccactac gccgcttccg ctgacactgt tcaacccaaa     120
aactacatct gcgtgcgctg tgtaaggcta tcatcagata catactgtag attctgtaga     180
tgcgaacctg cttgtatcat atacatcccc ctccccctga cctgcacaag caagcaatgt     240
gacattgata ttgctgctta tctagtgccg aggatgtgaa agccgagact caaacatttc     300
ttttactctc ttgttcctga ccagacctgg cggagattac gccagtatga ttcttgcagg     360
tctgagacaa gcctggaaca gccaacattt atttttcgaa gcgagaaaca tgccacaccc     420
cggcacgttc agagatgcat atgatttgtt tttcgagtaa cagtaccccc cccccccccc     480
ccaatgaaac cagtattact cacaccatcc tcattcaaag cgttacactg attacgcgcc     540
catcaacgac agcatgaggg gactgctgat ctgatctaat caaatgacta caaaaatcgc     600
aataatgaag agcaaacgac aaaaaagaaa caggttaacc aatcccgctt caatgtctca     660
ccacaatcca gcactgtttc tcattacctc ctccctctaa tttcagagtt gcatcagggt     720
ccttgatggc gcgccagctg cattaatgaa tcggccaacg cgcggggaga ggcggtttgc     780
gtattgggcg ctcttccgct tcctcgctca ctgactcgct gcgctcggtc gttcggctgc     840
ggcgagcggt atcagctcac tcaaaggcgg taatacggtt atccacagaa tcaggggata     900
acgcaggaaa gaacatgtga gcaaaaggcc agcaaaaggc caggaaccgt aaaaaggccg     960
cgttgctggc gtttttccat aggctccgcc cccctgacga gcatcacaaa aatcgacgct    1020
caagtcagag gtggcgaaac ccgacaggac tataaagata ccaggcgttt ccccctggaa    1080
```

gctccctcgt gcgctctcct gttccgaccc tgccgcttac cggatacctg tccgcctttc 1140

tcccttcggg aagcgtggcg ctttctcata gctcacgctg taggtatctc agttcggtgt 1200

aggtcgttcg ctccaagctg ggctgtgtgc acgaaccccc cgttcagccc gaccgctgcg 1260

ccttatccgg taactatcgt cttgagtcca acccggtaag acacgactta tcgccactgg 1320

cagcagccac tggtaacagg attagcagag cgaggtatgt aggcggtgct acagagttct 1380

tgaagtggtg gcctaactac ggctacacta gaagaacagt atttggtatc tgcgctctgc 1440

tgaagccagt taccttcgga aaaagagttg gtagctcttg atccggcaaa caaaccaccg 1500

ctggtagcgg tggttttttt gtttgcaagc agcagattac gcgcagaaaa aaaggatctc 1560

aagaagatcc tttgatcttt tctacggggt ctgacgctca gtggaacgaa aactcacgtt 1620

aagggatttt ggtcatgaga ttatcaaaaa ggatcttcac ctagatcctt ttaaattaaa 1680

aatgaagttt taaatcaatc taaagtatat atgagtaaac ttggtctgac agttaccaat 1740

gcttaatcag tgaggcacct atctcagcga tctgtctatt tcgttcatcc atagttgcct 1800

gactccccgt cgtgtagata actacgatac gggagggctt accatctggc cccagtgctg 1860

caatgatacc gcgagaccca cgctcaccgg ctccagattt atcagcaata aaccagccag 1920

ccggaagggc cgagcgcaga agtggtcctg caactttatc cgcctccatc cagtctatta 1980

attgttgccg ggaagctaga gtaagtagtt cgccagttaa tagtttgcgc aacgttgttg 2040

ccattgctac aggcatcgtg gtgtcacgct cgtcgtttgg tatggcttca ttcagctccg 2100

gttcccaacg atcaaggcga gttacatgat cccccatgtt gtgcaaaaaa gcggttagct 2160

ccttcggtcc tccgatcgtt gtcagaagta agttggccgc agtgttatca ctcatggtta 2220

tggcagcact gcataattct cttactgtca tgccatccgt aagatgcttt tctgtgactg 2280

gtgagtactc aaccaagtca ttctgagaat agtgtatgcg gcgaccgagt tgctcttgcc 2340

cggcgtcaat acgggataat accgcgccac atagcagaac tttaaaagtg ctcatcattg 2400

gaaaacgttc ttcggggcga aaactctcaa ggatcttacc gctgttgaga tccagttcga 2460

tgtaacccac tcgtgcaccc aactgatctt cagcatcttt tactttcacc agcgtttctg 2520

ggtgagcaaa aacaggaagg caaaatgccg caaaaaaggg aataagggcg acacggaaat 2580

gttgaatact catactcttc ctttttcaat attattgaag catttatcag ggttattgtc 2640

tcatgagcgg atacatattt gaatgtattt agaaaaataa acaaataggg gttccgcgca 2700

catttccccg aaaagtgcca cctgatgcgg tgtgaaatac cgcacagatg cgtaaggaga 2760

aaataccgca tcaggaaatt gtaagcgtta atattttgtt aaaattcgcg ttaaattttt 2820

gttaaatcag ctcatttttt aaccaatagg ccgaaatcgg caaaatccct tataaatcaa 2880

aagaatagac cgagataggg ttgagtgttg ttccagtttg gaacaagagt ccactattaa 2940

agaacgtgga ctccaacgtc aaagggcgaa aaaccgtcta tcagggcgat ggcccactac 3000

gtgaaccatc accctaatca agttttttgg ggtcgaggtg ccgtaaagca ctaaatcgga 3060
accctaaagg gagcccccga tttagagctt gacggggaaa gccggcgaac gtggcgagaa 3120
aggaagggaa gaaagcgaaa ggagcgggcg ctagggcgct ggcaagtgta gcggtcacgc 3180
tgcgcgtaac caccacaccc gccgcgctta atgcgccgct acagggcgcg tccattcgcc 3240
attcaggctg cgcaactgtt gggaagggcg atcggtgcgg gcctcttcgc tattacgcca 3300
gctggcgaaa gggggatgtg ctgcaaggcg attaagttgg gtaacgccag ggttttccca 3360
gtcacgacgt tgtaaaacga cggccagtga attgtaatac gactcactat agggcgaatt 3420
gggcccgacg tcgcatgctg gtttcgattt gtcttagagg aacgcatata cagtaatcat 3480
agagaataaa cgatattcat ttattaaagt agatagttga ggtagaagtt gtaaagagtg 3540
ataaatagct tagataccac agacaccctc ggtgacgaag tactgcagat ggtttccaat 3600
cacattgacc tgctggagca gagtgttacc ggcagagcac tgtttattgc tctggccctg 3660
gcacatgaca acgttggaga gaggagggtg gatcaggggc cagtcaataa agacctcacc 3720
agagcagtgc tggtaaccgt cccagaaggg cacttgaggg acgatatctc ctcggtgggt 3780
gattcggtag agctttcggt ctttggacac cttggagaca tcggggttct cctggccaaa 3840
gaagagttta tcgacccagt tagcaaagcc agcgttaccg acaatgggct gaccaagagt 3900
aacaacgagg ggatcgtggc cgttaacctt gaggttgatt ccgaacagaa gggctgcagc 3960
tcctccgaga gagtgaccgg tgacagcaat ctggtagtcg ggatactgct caatcacaga 4020
gtcgagcttg ggccgatct gattgtaggt gttgttgtag gactggatga agccattgtg 4080
gacaagacag tcatcacaag tagcagtaga agagatgtta gcagcaagat caaagttaat 4140
taactcacct gcaggattga gactatgaat ggattcccgt gcccgtatta ctctactaat 4200
ttgatcttgg aacgcgaaaa tacgtttcta ggactccaaa gaatctcaac tcttgtcctt 4260
actaaatata ctacccatag ttgatggttt acttgaacag agaggacatg ttcacttgac 4320
ccaaagtttc tcgcatctct tggatatttg aacaacggcg tccactgacc gtcagttatc 4380
cagtcacaaa acccccacat tcatacattc ccatgtacgt ttacaaagtt ctcaattcca 4440
tcgtgcaaat caaaatcaca tctattcatt catcatatat aaacccatca tgtctactaa 4500
cactcacaac tccatagaaa acatcgactc agaacacacg ctccatgcgg ccgcttagga 4560
atcctgagcg tccttgacac agtgaaccac accgactttg tgcatgtact tgagggtgga 4620
aatgatgttg cccacaatgg tagggtagaa gacgtaccga actccgtgtc gttcgcaaca 4680
ctctcggaca gcttgctgca cgaagggata gtgccaagac gacattcgag gaaagaggtg 4740
atgctcgatc tggaagttga gaccgccagt aaagaacatg gcaatgggtc caccgtaggt 4800
ggaagaggtc tccacctgag ctctgtacca gtcgatctga tcggcttcaa cgtccttctc 4860
ggagctcttg accttgcagt tcttgtcggg gattcgctcc gagccatcga agttgtgaga 4920
caagatgaaa aagaaggtga ggaaggcacc ggtagcagtg ggcaccagag gaatggtgat 4980

gagcagggag gttccagtga gataccaggg caagaaggcg gttcgaaaga tgaagaaagc 5040
tcgcataacg aatgcaaggg ttcggtaccg tcgcagaaag ccgttctctc gcatggctgt 5100
gacagactcg ggaatggtgt cgttgtgctg cattcggaag atgtagagag ggttgtacac 5160
cagcgaaacg ccgtaggctc caagcacgag gtacatgtac caggcctgga atcggtgaaa 5220
ccactttcga gcagtgttgg cagcagggta gttgtggaac acaaggaatg gttctgcgga 5280
ctcggcatcc aggtcgagac catgctgatt ggtgtaggtg tgatgtcgca tgatgtgaga 5340
ctgcagccag atccatctgg acgatccaat gacgtcgatg ccgtaggcaa agagagcgtt 5400
gacccagggc tttttgctga tggcaccatg agaggcatcg tgctgaatgg acaggccgat 5460
ctgcatgtgc atgaatccag tcaagagacc ccacagcacc attccggtag tagcccagtg 5520
ccactcgcaa aaggcggtga cagcaatgat gccaacggtt cgcagccaga atccaggtgt 5580
ggcataccag ttccgacctt tcatgacctc tcgcatagtt cgcttgacgt cctgtgcaaa 5640
gggagagtcg taggtgtaga caatgtcctt ggaggttcgg tcgtgcttgc ctcgcacgaa 5700
ctgttgaagc agcttcgagt tctcgggctt gacgtaaggg tgcatggagt agaacagagg 5760
agaagcatcg gaggcaccag aagcgaggat caagtcggat ccgggatgga ccttggcaag 5820
accttccaga tcgtagagaa tgccgtcgat ggcaaccagg tcgggtcgct cgagcagctg 5880
ctcggtagta agggagagag ccatggttgt gaattagggt ggtgagaatg gttggttgta 5940
gggaagaatc aaaggccggt ctcgggatcc gtgggtatat atatatatat atatatatac 6000
gatccttcgt tacctccctg ttctcaaaac tgtggttttt cgtttttcgt tttttgcttt 6060
ttttgatttt tttagggcca actaagcttc cagatttcgc taatcacctt tgtactaatt 6120
acaagaaagg aagaagctga ttagagttgg gctttttatg caactgtgct actccttatc 6180
tctgatatga aagtgtagac ccaatcacat catgtcattt agagttggta atactgggag 6240
gatagataag gcacgaaaac gagccatagc agacatgctg ggtgtagcca agcagaagaa 6300
agtagatggg agccaattga cgagcgaggg agctacgcca atccgacata cgacacgctg 6360
agatcgtctt ggccgggggg tacctacaga tgtccaaggg taagtgcttg actgtaattg 6420
tatgtctgag gacaaatatg tagtcagccg tataaagtca taccaggcac cagtgccatc 6480
atcgaaccac taactctcta tgatacatgc ctccggtatt attgtaccat gcgtcgcttt 6540
gttacatacg tatcttgcct ttttctctca gaaactccag aattctctct cttgagcttt 6600
tccataacaa gttcttctgc ctccaggaag tccatgggtg gtttgatcat ggttttggtg 6660
tagtggtagt gcagtggtgg tattgtgact ggggatgtag ttgagaataa gtcatacaca 6720
agtcagcttt cttcgagcct catataagta taagtagttc aacgtattag cactgtaccc 6780
agcatctccg tatcgagaaa cacaacaaca tgccccattg gacagatcat gcggatacac 6840
aggttgtgca gtatcataca tactcgatca gacaggtcgt ctgaccatca tacaagctga 6900

acaagcgctc catacttgca cgctctctat atacacagtt aaattacata tccatagtct 6960
aacctctaac agttaatctt ctggtaagcc tcccagccag ccttctggta tcgcttggcc 7020
tcctcaatag gatctcggtt ctggccgtac agacctcggc cgacaattat gatatccgtt 7080
ccggtagaca tgacatcctc aacagttcgg tactgctgtc cgagagcgtc tcccttgtcg 7140
tcaagaccca ccccgggggt cagaataagc cagtcctcag agtcgccctt aggtcggttc 7200
tgggcaatga agccaaccac aaactcgggg tcggatcggg caagctcaat ggtctgcttg 7260
gagtactcgc cagtggccag agagcccttg caagacagct cggccagcat gagcagacct 7320
ctggccagct tctcgttggg agaggggact aggaactcct tgtactggga gttctcgtag 7380
tcagagacgt cctccttctt ctgttcagag acagtttcct cggcaccagc tcgcaggcca 7440
gcaatgattc cggttccggg tacaccgtgg gcgttggtga tatcggacca ctcggcgatt 7500
cggtgacacc ggtactggtg cttgacagtg ttgccaatat ctgcgaactt tctgtcctcg 7560
aacaggaaga aaccgtgctt aagagcaagt tccttgaggg ggagcacagt gccggcgtag 7620
gtgaagtcgt caatgatgtc gatatgggtt ttgatcatgc acacataagg tccgacctta 7680
tcggcaagct caatgagctc cttggtggtg gtaacatcca gagaagcaca caggttggtt 7740
ttcttggctg ccacgagctt gagcactcga gcggcaaagg cggacttgtg gacgttagct 7800
cgagcttcgt aggagggcat tttggtggtg aagaggagac tgaaataaat ttagtctgca 7860
gaacttttta tcggaacctt atctggggca gtgaagtata tgttatggta atagttacga 7920
gttagttgaa cttatagata gactggacta tacggctatc ggtccaaatt agaaagaacg 7980
tcaatggctc tctgggcgtc gcctttgccg acaaaaatgt gatcatgatg aaagccagca 8040
atgacgttgc agctgatatt gttgtcggcc aaccgcgccg aaaacgcagc tgtcagaccc 8100
acagcctcca acgaagaatg tatcgtcaaa gtgatccaag cacactcata gttggagtcg 8160
tactccaaag gcggcaatga cgagtcagac agatactcgt cgaccttttc cttgggaacc 8220
accaccgtca gcccttctga ctcacgtatt gtagccaccg acacaggcaa cagtccgtgg 8280
atagcagaat atgtcttgtc ggtccatttc tcaccaactt taggcgtcaa gtgaatgttg 8340
cagaagaagt atgtgccttc attgagaatc ggtgttgctg atttcaataa agtcttgaga 8400
tcagtttggc cagtcatgtt gtgggggtta attggattga gttatcgcct acagtctgta 8460
caggtatact cgctgcccac tttatacttt ttgattccgc tgcacttgaa gcaatgtcgt 8520
ttaccaaaag tgagaatgct ccacagaaca caccccaggg tatggttgag caaaaaataa 8580
acactccgat acggggaatc gaaccccggt ctccacggtt ctcaagaagt attcttgatg 8640
agagcgtatc gatcgaggaa gaggacaagc ggctgcttct taagtttgtg acatcagtat 8700
ccaaggcacc attgcaagga ttcaaggctt tgaacccgtc atttgccatt cgtaacgctg 8760
gtagacaggt tgatcggttc cctacggcct ccacctgtgt caatcttctc aagctgcctg 8820
actatcagga cattgatcaa cttcggaaga aacttttgta tgccattcga tcacatgctg 8880

gtttcgattt gtcttagagg aacgcatata cagtaatcat agagaataaa cgatattcat 8940
ttattaaagt agatagttga ggtagaagtt gtaaagagtg ataaatagcg gccgctcact 9000
gaatcttttt ggctcccttg tgctttcgga cgatgtaggt ctgcacgtag aagttgagga 9060
acagacacag gacagtacca acgtagaagt agttgaaaaa ccagccaaac attctcattc 9120
catcttgtcg gtagcaggga atgttccggt acttccagac gatgtagaag ccaacgttga 9180
actgaatgat ctgcatagaa gtaatcaggg acttgggcat agggaacttg agcttgatca 9240
gtcgggtcca atagtagccg tacatgatcc agtgaatgaa gccgttgagc agcacaaaga 9300
tccaaacggc ttcgtttcgg tagttgtaga acagccacat gtccatagga gctccgagat 9360
ggtgaaagaa ctgcaaccag gtcagaggct tgcccatgag ggcagatag aaggagtcaa 9420
tgtactcgag gaacttgctg aggtagaaca gctgagtggt gattcggaag acattgttgt 9480
cgaaagcctt ctcgcagttg tcggacatga caccaatggt gtacatggcg taggccatag 9540
agaggaagga gcccagcgag tagatggaca tgagcaggtt gtagttggtg aacacaaact 9600
tcattcgaga ctgacccttg ggtccgagag gaccaagggt gaacttcagg atgacgaagg 9660
cgatggagag gtacagcacc tcgcagtgcg aggcatcaga ccagagctga gcatagtcga 9720
ccttgggaag aacctcctgg ccaatggaga cgatttcgtt cacgacctcc atggttgtga 9780
attagggtgg tgagaatggt tggttgtagg gaagaatcaa aggccggtct cgggatccgt 9840
gggtatatat atatatatat atatatacga tccttcgtta cctccctgtt ctcaaaactg 9900
tggtttttcg tttttcgttt tttgcttttt ttgatttttt tagggccaac taagcttcca 9960
gatttcgcta atcacctttg tactaattac aagaaaggaa gaagctgatt agagttgggc 10020
tttttatgca actgtgctac tccttatctc tgatatgaaa gtgtagaccc aatcacatca 10080
tgtcatttag agttggtaat actgggagga tagataaggc acgaaaacga gccatagcag 10140
acatgctggg tgtagccaag cagaagaaag tagatgggag ccaattgacg agcgagggag 10200
ctacgccaat ccgacatacg acacgctgag atcgtcttgg ccgggggta cctacagatg 10260
tccaagggta agtgcttgac tgtaattgta tgtctgagga caaatatgta gtcagccgta 10320
taaagtcata ccaggcacca gtgccatcat cgaaccacta actctctatg atacatgcct 10380
ccggtattat tgtaccatgc gtcgctttgt tacatacgta tcttgccttt ttctctcaga 10440
aactccagac tttggctatt ggtcgagata agcccggacc atagtgagtc tttcacactc 10500
tgtttaaaca ccactaaaac cccacaaaat atatcttacc gaatatacag atctactata 10560
gaggaacaat tgccccggag aagacggcca ggccgcctag atgacaaatt caacaactca 10620
cagctgactt tctgccattg ccactagggg ggggcctttt tatatggcca agccaagctc 10680
tccacgtcgg ttgggctgca cccaacaata aatgggtagg gttgcaccaa caaagggatg 10740
ggatgggggg tagaagatac gaggataacg gggctcaatg gcacaaataa gaacgaatac 10800

tgccattaag actcgtgatc cagcgactga caccattgca tcatctaagg gcctcaaaac 10860
tacctcggaa ctgctgcgct gatctggaca ccacagaggt tccgagcact ttaggttgca 10920
ccaaatgtcc caccaggtgc aggcagaaaa cgctggaaca gcgtgtacag tttgtcttaa 10980
caaaaagtga gggcgctgag gtcgagcagg gtggtgtgac ttgttatagc ctttagagct 11040
gcgaaagcgc gtatggattt ggctcatcag gccagattga gggtctgtgg acacatgtca 11100
tgttagtgta cttcaatcgc cccctggata tagccccgac aataggccgt ggcctcattt 11160
ttttgccttc cgcacatttc cattgctcgg tacccacacc ttgcttctcc tgcacttgcc 11220
aaccttaata ctggtttaca ttgaccaaca tcttacaagc ggggggcttg tctagggtat 11280
atataaacag tggctctccc aatcggttgc cagtctcttt tttcctttct ttccccacag 11340
attcgaaatc taaactacac atcacacaat gcctgttact gacgtcctta agcgaaagtc 11400
cggtgtcatc gtcggcgacg atgtccgagc cgtgagtatc cacgacaaga tcagtgtcga 11460
gacgacgcgt tttgtgtaat gacacaatcc gaaagtcgct agcaacacac actctctaca 11520
caaactaacc cagctctcca tggtgaaggc ttctcgacag gctctgcccc tcgtcatcga 11580
cggaaaggtg tacgacgtct ccgcttgggt gaacttccac cctggtggag ctgaaatcat 11640
tgagaactac cagggacgag atgctactga cgccttcatg gttatgcact ctcaggaagc 11700
cttcgacaag ctcaagcgaa tgcccaagat caaccaggct tccgagctgc ctccccaggc 11760
tgccgtcaac gaagctcagg aggatttccg aaagctccga gaagagctga tcgccactgg 11820
catgtttgac gcctctcccc tctggtactc gtacaagatc ttgaccaccc tgggtcttgg 11880
cgtgcttgcc ttcttcatgc tggtccagta ccacctgtac ttcattggtg ctctcgtgct 11940
cggtatgcac taccagcaaa tgggatggct gtctcatgac atctgccacc accagacctt 12000
caagaaccga aactggaata acgtcctggg tctggtcttt ggcaacggac tccagggctt 12060
ctccgtgacc tggtggaagg acagacacaa cgcccatcat tctgctacca acgttcaggg 12120
tcacgatccc gacattgata acctgcctct gctcgcctgg tccgaggacg atgtcactcg 12180
agcttctccc atctcccgaa agctcattca gttccaacag tactatttcc tggtcatctg 12240
tattctcctg cgattcatct ggtgtttcca gtctgtgctg accgttcgat ccctcaagga 12300
ccgagacaac cagttctacc gatctcagta caagaaagag gccattggac tcgctctgca 12360
ctggactctc aagaccctgt tccacctctt ctttatgccc tccatcctga cctcgatgct 12420
ggtgttcttt gtttccgagc tcgtcggtgg cttcggaatt gccatcgtgg tcttcatgaa 12480
ccactaccct ctggagaaga tcggtgattc cgtctgggac ggacatggct tctctgtggg 12540
tcagatccat gagaccatga acattcgacg aggcatcatt actgactggt tctttggagg 12600
cctgaactac cagatcgagc accatctctg gcccaccctg cctcgacaca acctcactgc 12660
cgtttcctac caggtggaac agctgtgcca gaagcacaac ctcccctacc gaaaccctct 12720
gccccatgaa ggtctcgtca tcctgctccg atacctgtcc cagttcgctc gaatggccga 12780

gaagcagccc ggtgccaagg ctcagtaagc ggccgcatga gaagataaat atataaatac 12840
attgagatat taaatgcgct agattagaga gcctcatact gctcggagag aagccaagac 12900
gagtactcaa aggggattac accatccata tccacagaca caagctgggg aaaggttcta 12960
tatacacttt ccggaatacc gtagtttccg atgttatcaa tgggggcagc caggatttca 13020
ggcacttcgg tgtctcgggg tgaaatggcg ttcttggcct ccatcaagtc gtaccatgtc 13080
ttcatttgcc tgtcaaagta aaacagaagc agatgaagaa tgaacttgaa gtgaaggaat 13140
ttaaatgatg tcgacgcagt aggatgtcct gcacgggtct ttttgtgggg tgtggagaaa 13200
ggggtgcttg gatcgatgga agccggtaga accgggctgc ttgtgcttgg agatggaagc 13260
cggtagaacc gggctgcttg gggggatttg gggccgctgg gctccaaaga ggggtaggca 13320
tttcgttggg gttacgtaat tgcggcattt gggtcctgcg cgcatgtccc attggtcaga 13380
attagtccgg ataggagact tatcagccaa tcacagcgcc ggatccacct gtaggttggg 13440
ttgggtggga gcacccctcc acagagtaga gtcaaacagc agcagcaaca tgatagttgg 13500
gggtgtgcgt gttaaaggaa aaaaaagaag cttgggttat attcccgctc tatttagagg 13560
ttgcgggata gacgccgacg gagggcaatg gcgctatgga accttgcgga tatccatacg 13620
ccgcggcgga ctgcgtccga accagctcca gcagcgtttt ttccgggcca ttgagccgac 13680
tgcgaccccg ccaacgtgtc ttggcccacg cactcatgtc atgttggtgt tgggaggcca 13740
ctttttaagt agcacaaggc acctagctcg cagcaaggtg tccgaaccaa agaagcggct 13800
gcagtggtgc aaacggggcg gaaacggcgg gaaaaagcca cgggggcacg aattgaggca 13860
cgccctcgaa tttgagacga gtcacggccc cattcgcccg cgcaatggct cgccaacgcc 13920
cggtcttttg caccacatca ggttacccca agccaaacct ttgtgttaaa aagcttaaca 13980
tattataccg aacgtaggtt tgggcgggct tgctccgtct gtccaaggca acatttatat 14040
aagggtctgc atcgccggct caattgaatc ttttttcttc ttctcttctc tatattcatt 14100
cttgaattaa acacacatca accatgggcg tattcattaa acaggagcag cttccggctc 14160
tcaagaagta caagtactcc gccgaggatc actcgttcat ctccaacaac attctgcgcc 14220
ccttctggcg acagtttgtc aaaatcttcc ctctgtggat ggcccccaac atggtgactc 14280
tgctgggctt cttctttgtc attgtgaact tcatcaccat gctcattgtt gatcccaccc 14340
acgaccgcga gcctcccaga tgggtctacc tcacctacgc tctgggtctg ttcctttacc 14400
agacatttga tgcctgtgac ggatcccatg cccgacgaac tggccagagt ggaccccttg 14460
gagagctgtt tgaccactgt gtcgacgcca tgaatacctc tctgattctc acggtggtgg 14520
tgtccaccac ccatatggga tataacatga agctactgat tgtgcagatt gccgctctcg 14580
gaaacttcta cctgtcgacc tgggagacct accataccgg aactctgtac ctttctggct 14640
tctctggtcc tgttgaaggt atcttgattc tggtggctct tttcgtcctc accttcttca 14700

```
ctggtcccaa cgtgtacgct ctgaccgtct acgaggctct tcccgagtcc atcacttcgc    14760
tgctgcctgc cagcttcctg gacgtcacca tcacccagat ctacattgga ttcggagtgc    14820
tgggcatggt gttcaacatc tacggcgcct gcggaaacgt gatcaagtac tacaacaaca    14880
agggcaagag cgctctcccc gccattctcg gaatcgcccc ctttggcatc ttctacgtcg    14940
gcgtctttgc ctgggcccat gttgctcctc tgcttctctc caagtacgcc atcgtctatc    15000
tgtttgccat tggggctgcc tttgccatgc aagtcggcca gatgattctt gcccatctcg    15060
tgcttgctcc ctttccccac tggaacgtgc tgctcttctt cccctttgtg ggactggcag    15120
tgcactacat tgcacccgtg tttggctggg acgccgatat cgtgtcggtt aacactctct    15180
tcacctgttt tggcgccacc ctctccattt acgccttctt tgtgcttgag atcatcgacg    15240
agatcaccaa ctacctcgat atctggtgtc tgcgaatcaa gtaccctcag gagaagaaga    15300
ccgaataagc ggccgcatgg agcgtgtgtt ctgagtcgat gttttctatg gagttgtgag    15360
tgttagtaga catgatgggt ttatatatga tgaatgaata gatgtgattt tgatttgcac    15420
gatggaattg agaactttgt aaacgtacat gggaatgtat gaatgtgggg gttttgtgac    15480
tggataactg acggtcagtg gacgccgttg ttcaaatatc caagagatgc gagaaacttt    15540
gggtcaagtg aacatgtcct ctctgttcaa gtaaaccatc aactatgggt agtatattta    15600
gtaaggacaa gagttgagat tctttggagt cctagaaacg tattttcgcg ttccaagatc    15660
aaattagtag agtaatacgg gcacgggaat ccattcatag tctcaatcct gcaggtgagt    15720
taattaatcg agcttggcgt aatcatggtc atagctgttt cctgtgtgaa attgttatcc    15780
gctcacaatt ccacacaac                                                 15799
```

<210> 139
<211> 9641
<212> DNA
<213> Artificial Sequence

<220>
<223> Plasmid pY201

<400> 139

```
gtacgataac ttcgtatagc atacattata cgaagttatc gcgtcgacga gtatctgtct      60
gactcgtcat tgccgccttt ggagtacgac tccaactatg agtgtgcttg gatcactttg     120
acgatacatt cttcgttgga ggctgtgggt ctgacagctg cgttttcggc gcggttggcc     180
gacaacaata tcagctgcaa cgtcattgct ggctttcatc atgatcacat ttttgtcggc     240
aaaggcgacg cccagagagc cattgacgtt ctttctaatt tggaccgata gccgtatagt     300
ccagtctatc tataagttca actaactcgt aactattacc ataacatata cttcactgcc     360
ccagataagg ttccgataaa aagttctgca gactaaattt atttcagtct cctcttcacc     420
accaaaatgc cctcctacga agctcgagct aacgtccaca agtccgcctt tgccgctcga     480
gtgctcaagc tcgtggcagc caagaaaacc aacctgtgtg cttctctgga tgttaccacc     540
```

accaaggagc tcattgagct tgccgataag gtcggacctt atgtgtgcat gatcaaaacc 600
catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag cccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
atttttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400
gtgggggttt tgtgactgga taactgacgg tcagtggacg ccgttgttca aatatccaag 2460

agatgcgaga aactttgggt caagtgaaca tgtcctctct gttcaagtaa accatcaact 2520
atgggtagta tatttagtaa ggacaagagt tgagattctt tggagtccta gaaacgtatt 2580
ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180
taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260
atgagtgata acactgcggc caacttactt ctgacaacga tcggaggacc gaaggagcta 4320
accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440

acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180
ccgaaaattc cacgtgcagc aacgtcgtgt ggggtgttaa aatgtggggg gggggaacca 6240
ggacaagagg ctcttgtggg agccgaatga gagcacaaag cgggcgggtg tgataagggc 6300
atttttgccc attttccctt ctcctgtctc tccgacggtg atggcgttgt gcgtcctcta 6360

tttcttttta tttctttttg ttttatttct ctgactaccg atttggtttg atttcctcaa 6420
ccccacacaa ataagctcgg gccgaggaat atatatatac acggacacag tcgccctgtg 6480
gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260
ccaaccattc tcaccaccct aattcacaac catgtacaac cccgtggacg cagtgttgac 7320
taagattatt acaaactacg gaattgattc ttttaccctg cgatatgcca tttgtctgtt 7380
gggatctttt cctcttaacg ctattctgaa gcggattcct gaaaagcgaa tcggcctgaa 7440
gtgttgtttt atcatttcta tgtccatgtt ttatctcttc ggcgttctga atctcgtgag 7500
cggatttcga accctcttca tttccacaat gttcacatac cttatctctc ggttctaccg 7560
atccaagttt atgccccatc tcaacttcat gttcgtcatg ggccacttgg ctatcaacca 7620
cattcatgct cagttcctga acgaacaaac tcaaacgacc gtcgatatta catcctcgca 7680
gatggtcctg gctatgaagc tgacaagctt tgcctggtct tactatgacg gttcgtgtac 7740
gagcgagtcc gacttcaagg accttaccga acaccagaag tcccgagccg tccgaggcca 7800
tcctcccctt ctgaaatttt tggcttacgc ctttttctac tctacccttc tcaccggtcc 7860
ctccttcgat tacgctgatt tcgactcttg gctgaactgc gaaatgttcc gggaccttcc 7920
cgagtccaag aaacccatgc gaagacatca tcctggtgag cggcgtcaga ttcccaagaa 7980
cggcaagctc gccctgtgga aggttgtcca gggcctcgcc tggatgattc tgagcacgtt 8040
gggtatgaag cacttccccg tgaagtacgt gctggacaag gacggatttc ctacccgttc 8100
ctttatcttc cgtattcatt atctgtttct gctgggattc atccaccgat ttaagtatta 8160
cgctgcgtgg acgattagcg aaggttcgtg cattctctgt ggtcttggtt ataatggata 8220
cgattctaag acccagaaga tccggtggga tcgagtgcgg aatattgata tttggacagt 8280
ggagactgca caaaacaccc gagagatgct ggaagcgtgg aacatgaata ctaacaaatg 8340

gctgaagtat agcgtgtatc ttagagtgac taagaagggt aagaagccag gttttcgatc 8400

taccctgttt accttcctga cctccgcctt ttggcacggt acccgtcctg gatactacct 8460

taccttcgca actggtgccc tgtaccaaac ctgtggaaag atctatagac gaaactttcg 8520

tcccatcttt ctgagagaag atggcgtgac acctctcccg tccaagaaga tttacgacct 8580

ggtcggcatt tacgctatta agctggcctt tggttacatg gttcaaccct tcattatcct 8640

tgacctgaag ccctctctta tggtttgggg atccgtgtat ttctacgtgc atattattgt 8700

ggccttctcg ttctttctgt tccgaggacc atacgctaag caggttactg aatttttcaa 8760

aagcaagcaa ccgaaggaga tcttcatccg aaagcagaag aagttggaaa aagacatctc 8820

tgcctcttcc cccaacctcg gaggtattct taaggcaaaa atcgaacatg agaagggaaa 8880

gacggcagag gaggaagaga tgaacttggg cattccaccc atcgaactgg agaagtggga 8940

caacgccaag gaggactggg aggatttctg caaggactac aaggagtggc ggaacaagaa 9000

cggactggaa attgaagagg agaacctgtc caaggccttc gagcgattta agcaggaatt 9060

ttccaacgct gcgtcgggct ctggtgaacg ggttcggaaa atgtccttct ccggatattc 9120

tcctaaaccc atctcgaaga aagaagaata ggcggccgca tgagaagata aatatataaa 9180

tacattgaga tattaaatgc gctagattag agagcctcat actgctcgga gagaagccaa 9240

gacgagtact caaaggggat tacaccatcc atatccacag acacaagctg gggaaaggtt 9300

ctatatacac tttccggaat accgtagttt ccgatgttat caatgggggc agccaggatt 9360

tcaggcactt cggtgtctcg ggtgaaatgg gcgttcttgg cctccatcaa gtcgtaccat 9420

gtcttcattt gcctgtcaaa gtaaaacaga agcagatgaa gaatgaactt gaagtgaagg 9480

aatttaaatg taacgaaact gaaatttgac cagatattgt gtccgcggtg gagctccagc 9540

ttttgttccc tttagtgagg gttaatttcg agcttggcgt aatcatggtc atagctgttt 9600

cctgtgtgaa attgttatcc gctcacaagc ttccacacaa c 9641

<210> 140
<211> 34
<212> DNA
<213> Escherichia coli

<400> 140
ataacttcgt ataatgtatg ctatacgaag ttat 34

<210> 141
<211> 40
<212> DNA
<213> Artificial Sequence

<220>
<223> Primer 798

<400> 141
ctaattcaca accatggcct ttccatgggc agataagtgg 40

<210> 142
<211> 36
<212> DNA
<213> Artificial Sequence

<220>
<223> Primer 799

<400> 142
ctcatgcggc cgcttacttg gtcttgatgg tgtcct 36

<210> 143
<211> 40
<212> DNA
<213> Artificial Sequence

<220>
<223> Primer 800

<400> 143
cgatagttag tagacaacaa tcgatagttg gagcaaggga 40

<210> 144
<211> 36
<212> DNA
<213> Artificial Sequence

<220>
<223> Primer 801

<400> 144
gaaaggccat ggttgtgaat tagggtggtg agaatg 36

<210> 145
<211> 9320
<212> DNA
<213> Artificial Sequence

<220>
<223> Plasmid pY168

<400> 145

```
gtacgataac ttcgtatagc atacattata cgaagttatc gcgtcgacga gtatctgtct      60
gactcgtcat tgccgccttt ggagtacgac tccaactatg agtgtgcttg gatcactttg     120
acgatacatt cttcgttgga ggctgtgggt ctgacagctg cgttttcggc gcggttggcc     180
gacaacaata tcagctgcaa cgtcattgct ggctttcatc atgatcacat ttttgtcggc     240
aaaggcgacg cccagagagc cattgacgtt ctttctaatt tggaccgata gccgtatagt     300
ccagtctatc tataagttca actaactcgt aactattacc ataacatata cttcactgcc     360
ccagataagg ttccgataaa aagttctgca gactaaattt atttcagtct cctcttcacc     420
accaaaatgc cctcctacga agctcgagct aacgtccaca agtccgcctt tgccgctcga     480
gtgctcaagc tcgtggcagc caagaaaacc aacctgtgtg cttctctgga tgttaccacc     540
```

accaaggagc tcattgagct tgccgataag gtcggacctt atgtgtgcat gatcaaaacc 600
catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag ccccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
attttttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400
gtgggggttt tgtgactgga taactgacgg tcagtggacg ccgttgttca aatatccaag 2460
agatgcgaga aactttgggt caagtgaaca tgtcctctct gttcaagtaa accatcaact 2520

atgggtagta tatttagtaa ggacaagagt tgagattctt tggagtccta gaaacgtatt 2580

ttcgcgttcc aagatcaaat tagtagagta atacgggcac gggaatccat tcatagtctc 2640

aattttccca taggtgtgct acaaggtgtt gagatgtggt acagtaccac catgattcga 2700

ggtaaagagc ccagaagtca ttgatgaggt caagaaatac acagatctac agctcaatac 2760

aatgaatatc ttctttcata ttcttcaggt gacaccaagg gtgtctattt tccccagaaa 2820

tgcgtgaaaa ggcgcgtgtg tagcgtggag tatgggttcg gttggcgtat ccttcatata 2880

tcgacgaaat agtagggcaa gagatgacaa aaagtatcta tatgtagaca gcgtagaata 2940

tggatttgat tggtataaat tcatttattg cgtgtctcac aaatactctc gataagttgg 3000

ggttaaactg gagatggaac aatgtcgata tctcgacgca tgcgacgtcg ggcccaattc 3060

gccctatagt gagtcgtatt acaattcact ggccgtcgtt ttacaacgtc gtgactggga 3120

aaaccctggc gttacccaac ttaatcgcct tgcagcacat ccccctttcg ccagctggcg 3180

taatagcgaa gaggcccgca ccgatcgccc ttcccaacag ttgcgcagcc tgaatggcga 3240

atggacgcgc cctgtagcgg cgcattaagc gcggcgggtg tggtggttac gcgcagcgtg 3300

accgctacac ttgccagcgc cctagcgccc gctcctttcg ctttcttccc ttcctttctc 3360

gccacgttcg ccggctttcc ccgtcaagct ctaaatcggg ggctcccttt agggttccga 3420

tttagtgctt tacggcacct cgaccccaaa aaacttgatt agggtgatgg ttcacgtagt 3480

gggccatcgc cctgatagac ggtttttcgc cctttgacgt tggagtccac gttctttaat 3540

agtggactct tgttccaaac tggaacaaca ctcaacccta tctcggtcta ttcttttgat 3600

ttataaggga ttttgccgat ttcggcctat tggttaaaaa atgagctgat ttaacaaaaa 3660

tttaacgcga attttaacaa aatattaacg cttacaattt cctgatgcgg tattttctcc 3720

ttacgcatct gtgcggtatt tcacaccgca tcaggtggca cttttcgggg aaatgtgcgc 3780

ggaaccccta tttgtttatt tttctaaata cattcaaata tgtatccgct catgagacaa 3840

taaccctgat aaatgcttca ataatattga aaaaggaaga gtatgagtat tcaacatttc 3900

cgtgtcgccc ttattccctt ttttgcggca ttttgccttc ctgtttttgc tcacccagaa 3960

acgctggtga aagtaaaaga tgctgaagat cagttgggtg cacgagtggg ttacatcgaa 4020

ctggatctca acagcggtaa gatccttgag agttttcgcc ccgaagaacg ttttccaatg 4080

atgagcactt ttaaagttct gctatgtggc gcggtattat cccgtattga cgccgggcaa 4140

gagcaactcg gtcgccgcat acactattct cagaatgact tggttgagta ctcaccagtc 4200

acagaaaagc atcttacgga tggcatgaca gtaagagaat tatgcagtgc tgccataacc 4260

atgagtgata acactgcggc caacttactt ctgacaacga tcggaggacc gaaggagcta 4320

accgcttttt tgcacaacat gggggatcat gtaactcgcc ttgatcgttg ggaaccggag 4380

ctgaatgaag ccataccaaa cgacgagcgt gacaccacga tgcctgtagc aatggcaaca 4440

acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180
ccgaaaattc cacgtgcagc aacgtcgtgt ggggtgttaa aatgtggggg gggggaacca 6240
ggacaagagg ctcttgtggg agccgaatga gagcacaaag cgggcgggtg tgataagggc 6300
atttttgccc attttccctt ctcctgtctc tccgacggtg atggcgttgt gcgtcctcta 6360
tttcttttta tttctttttg ttttatttct ctgactaccg atttggtttg atttcctcaa 6420

ccccacacaa ataagctcgg gccgaggaat atatatatac acggacacag tcgccctgtg 6480
gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260
ccaaccattc tcaccaccct aattcacaac catggccttt ccctgggcag ataagtgggc 7320
agccgatgcg tctgcatcta cagggctgcc tccggacctc ctcaagattg cattcactct 7380
ggtcatgtct tatccgctga gttctctcat gaaacggctg ccagatgacg ccaaaaacct 7440
caagatcatc tatatcatct ccgtgtccat cttctacatg gtgggtgtct tctccctcta 7500
tggcggagct gccactctgc tcttctcctc aatgggtacc ttcttcatca cccaatggaa 7560
gagcccttac atgccctggg tcaattttgg ttttgtcatg acccatctct tcgtcaatca 7620
cctgcgttcg cagtttttcc ccgaaacata cgaccccaat gtcattgaca tcaccggagc 7680
acagatggtt ctgtgtatga agctatcgtc ttttggatgg aacgtctacg atggatggca 7740
gattgagaag ggtgagcagc tcagcgagtt ccagactaaa agggctgttc tcaagcaccc 7800
cagtcttatg gacttcctag cttttgtgtt ctacttccct tccattctga caggtccttc 7860
ttacgactat atggagttcc ataactggct cgatctcagc ctgttcaagg agctggagaa 7920
agataaggac cccaagcgag ctgctcgacg aaagcgacac aagatccccc gatctggaat 7980
cgctgcttcc aagaaactcg ccgctggtat cttctggatc gttctgtgga cccaggtgga 8040
ctctcgaatc tccaccgcct acgcttactc agacgcattc accaaggagc acaacatctt 8100
tggacgaatt gtgtacctct acatgctcgg tttcatgtac cgactcaagt actacggagc 8160
ctggtccatt tccgagggag cctgcatctt gtctggcctc ggattccatg gcgtggaccc 8220
caaaactggc aagtacaagt gggaccgtgt ccagaacgtg gacccgtggg gattcgaaac 8280
tggtcaaaac acaaaggctc tgctggaggc ctggaaccag aacactaaca agtggctacg 8340

aaactatgtg tacctccgag tggtgcccaa aggccaaaag cctggattcc gagccactat 8400

cttcacattt gtggtttccg ccttctggca tggaactcga cctggctact atctcacctt 8460

tgtgaccgct gccatgtacc agtctgttgg taagttcttc cgacgatacc tgcgaccctt 8520

cttcatggag tctgatggaa agactgccgg tccctataag atctactacg acattgtgtg 8580

ttggatcgtt gtccaaaccg catttggata cgctacccag tcctttatga ttctagactt 8640

ctggctgtcg ctcaagtgtt ggaagaactc ctggttcctg taccacattg ctctgggcgc 8700

catctttgca atttctagcc cctacaaggc atgggcgatt cccaagatca agaaaaagca 8760

ggctggagcc gtcactgaca agaaggacgc caaggaggag gtgaagaagg acaccatcaa 8820

gaccaagtaa gcggccgcat gagaagataa atatataaat acattgagat attaaatgcg 8880

ctagattaga gagcctcata ctgctcggag agaagccaag acgagtactc aaaggggatt 8940

acaccatcca tatccacaga cacaagctgg ggaaaggttc tatatacact ttccggaata 9000

ccgtagtttc cgatgttatc aatgggggca gccaggattt caggcacttc ggtgtctcgg 9060

ggtgaaatgg cgttcttggc ctccatcaag tcgtaccatg tcttcatttg cctgtcaaag 9120

taaaacagaa gcagatgaag aatgaacttg aagtgaagga atttaaatgt aacgaaactg 9180

aaatttgacc agatattgtg tccgcggtgg agctccagct tttgttccct ttagtgaggg 9240

ttaatttcga gcttggcgta atcatggtca tagctgtttc ctgtgtgaaa ttgttatccg 9300

ctcacaagct tccacacaac 9320

<210> 146
<211> 8726
<212> DNA
<213> Artificial Sequence

<220>
<223> Plasmid pY208

<400> 146

```
gtacgataac ttcgtatagc atacattata cgaagttatc gcgtcgacga gtatctgtct      60
gactcgtcat tgccgccttt ggagtacgac tccaactatg agtgtgcttg gatcactttg     120
acgatacatt cttcgttgga ggctgtgggt ctgacagctg cgttttcggc gcggttggcc     180
gacaacaata tcagctgcaa cgtcattgct ggctttcatc atgatcacat ttttgtcggc     240
aaaggcgacg cccagagagc cattgacgtt ctttctaatt tggaccgata gccgtatagt     300
ccagtctatc tataagttca actaactcgt aactattacc ataacatata cttcactgcc     360
ccagataagg ttccgataaa aagttctgca gactaaattt atttcagtct cctcttcacc     420
accaaaatgc cctcctacga agctcgagct aacgtccaca agtccgcctt tgccgctcga     480
gtgctcaagc tcgtggcagc caagaaaacc aacctgtgtg cttctctgga tgttaccacc     540
accaaggagc tcattgagct tgccgataag gtcggacctt atgtgtgcat gatcaaaacc     600
catatcgaca tcattgacga cttcacctac gccggcactg tgctccccct caaggaactt     660
```

gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag cccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
atttttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400
gtgggggttt tgtgactgga taactgacgg tcagtggacg ccgttgttca aatatccaag 2460
agatgcgaga aactttgggt caagtgaaca tgtcctctct gttcaagtaa accatcaact 2520
atgggtagta tatttagtaa ggacaagagt tgagattctt tggagtccta gaaacgtatt 2580

ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180
taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260
atgagtgata acactgcggc caacttactt ctgacaacga tcggaggacc gaaggagcta 4320
accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440
acgttgcgca aactattaac tggcgaacta cttactctag cttcccggca acaattaata 4500
gactggatgg aggcggataa agttgcagga ccacttctgc gctcggccct tccggctggc 4560

tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180
ccgaaaattc cacgtgcagc aacgtcgtgt ggggtgttaa aatgtggggg gggggaacca 6240
ggacaagagg ctcttgtggg agccgaatga gagcacaaag cgggcgggtg tgataagggc 6300
atttttgccc attttccctt ctcctgtctc tccgacggtg atggcgttgt gcgtcctcta 6360
tttcttttta tttcttttttg ttttatttct ctgactaccg atttggtttg atttcctcaa 6420
ccccacacaa ataagctcgg gccgaggaat atatatatac acggacacag tcgccctgtg 6480

gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260
ccaaccattc tcaccaccct aattcacaac catgtctatt ggttcgtcca accccgtgct 7320
cttggctgcg attcccttcg tctacctgtt tgtcctccca cgagtcctgg ctttcctgcc 7380
tcagaaggct cagttcctgg ccaaatgtat tgtggtcctg attgccacgc ttatcatgtc 7440
cgttgcaggc tgcttcatct cgatcgtgtg cgctcttctg gacaagagat acgtcatcaa 7500
ttacgttgtg tcgcgattgt tctccttcct tgccgctcga ccgtgtggtg tgacctataa 7560
gattgttggt gaggaacacc tcgataagta ccctgctatc gtggtctgta accatcaatc 7620
ctctatggat atgatggttt tgggacgagt tttccaaaag cactgcgttg tcatggcgaa 7680
gaaggaactc ctgtactttc ccttttttggg aatgtttatg aaactgagca acgctatctt 7740
catcgaccgg aagaaccaca agaaagccat cgagtctacc acccaagccg tggcggacat 7800
gaagaagcac aactctggaa tctggatttt cccagagggc acccggtcta gactggacaa 7860
ggcagacctg ctgcccttca agaaaggtgc ctttcatctt gcaattcagg cccagctccc 7920
tattctcccc attatctcgc agggctattc ccatatctac gactcttcga agcggtactt 7980
ccccggtgga gagctcgaga tcagagtcct ggagcccatt cctacaactg gcctcactac 8040
tgatgatgtg aacgacctga tggacaagac acgaaacctt atgctcaagc acttgaagga 8100
gatggattcc cagtattcgt cgagcactgc tgaaaatgga tccacgcaca tcgacgccga 8160
tattgccaag tctacagcca ccagcattgg caacactgac gacgcaatta caaaacgtcg 8220
tacccctaag gaataagcgg ccgcatgaga agataaatat ataaatacat tgagatatta 8280
aatgcgctag attagagagc ctcatactgc tcggagagaa gccaagacga gtactcaaag 8340
gggattacac catccatatc cacagacaca agctggggaa aggttctata tacactttcc 8400
ggaataccgt agtttccgat gttatcaatg ggggcagcca ggatttcagg cacttcggtg 8460

tctcggggtg aaatggcgtt cttggcctcc atcaagtcgt accatgtctt catttgcctg 8520

tcaaagtaaa acagaagcag atgaagaatg aacttgaagt gaaggaattt aaatgtaacg 8580

aaactgaaat ttgaccagat attgtgtccg cggtggagct ccagcttttg ttccctttag 8640

tgagggttaa tttcgagctt ggcgtaatca tggtcatagc tgtttcctgt gtgaaattgt 8700

tatccgctca caagcttcca cacaac 8726

<210> 147
<211> 31
<212> DNA
<213> Artificial Sequence

<220>
<223> Primer 856

<400> 147
tcacaacaca tgtccgttgc atccaagctc g 31

<210> 148
<211> 28
<212> DNA
<213> Artificial Sequence

<220>
<223> Primer 857

<400> 148
tttagcggcc gcctactgag tcttctgg 28

<210> 149
<211> 8630
<212> DNA
<213> Artificial Sequence

<220>
<223> Plasmid pY207

<400> 149

```
gtacgataac ttcgtatagc atacattata cgaagttatc gcgtcgacga gtatctgtct      60
gactcgtcat tgccgccttt ggagtacgac tccaactatg agtgtgcttg gatcactttg     120
acgatacatt cttcgttgga ggctgtgggt ctgacagctg cgttttcggc gcggttggcc     180
gacaacaata tcagctgcaa cgtcattgct ggctttcatc atgatcacat ttttgtcggc     240
aaaggcgacg cccagagagc cattgacgtt ctttctaatt tggaccgata gccgtatagt     300
ccagtctatc tataagttca actaactcgt aactattacc ataacatata cttcactgcc     360
ccagataagg ttccgataaa aagttctgca gactaaattt atttcagtct cctcttcacc     420
accaaaatgc cctcctacga agctcgagct aacgtccaca agtccgcctt tgccgctcga     480
gtgctcaagc tcgtggcagc caagaaaacc aacctgtgtg cttctctgga tgttaccacc     540
accaaggagc tcattgagct tgccgataag gtcggacctt atgtgtgcat gatcaaaacc     600
```

catatcgaca tcattgacga cttcacctac gccggcactg tgctccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740
tatttgtgtc ccaaaaaaca gccccaattg ccccaattga ccccaaattg acccagtagc 1800
gggcccaacc ccggcgagag ccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
attttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400
gtgggggttt tgtgactgga taactgacgg tcagtggacg ccgttgttca aatatccaag 2460
agatgcgaga aactttgggt caagtgaaca tgtcctctct gttcaagtaa accatcaact 2520
atgggtagta tatttagtaa ggacaagagt tgagattctt tggagtccta gaaacgtatt 2580

ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180
taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260
atgagtgata acactgcggc caacttactt ctgacaacga tcggaggacc gaaggagcta 4320
accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440
acgttgcgca aactattaac tggcgaacta cttactctag cttcccggca acaattaata 4500

gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180
ccgaaaattc cacgtgcagc aacgtcgtgt ggggtgttaa aatgtggggg gggggaacca 6240
ggacaagagg ctcttgtggg agccgaatga gagcacaaag cgggcgggtg tgataagggc 6300
atttttgccc attttccctt ctcctgtctc tccgacggtg atggcgttgt gcgtcctcta 6360
tttcttttta tttcttttttg ttttatttct ctgactaccg atttggtttg atttcctcaa 6420
ccccacacaa ataagctcgg gccgaggaat atatatatac acggacacag tcgccctgtg 6480

gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260
ccaaccattc tcaccaccct aattcacaac catgtccgtt gcatccaagc tcgtcttcta 7320
cgtccgcgcc gccatcgccg tggtcatctt tgccgcctgt gccacctacg gcgtgctggc 7380
gtccaccatt ctcaccgcca tcggcaagca gggcctggcc caatggaccg ttgccagagc 7440
cttctactac tcggtgcgca tcttcctggg tatcagcatc aagctgcgta gccggcaggt 7500
gaccggaacc gccggtctgg atgcctccaa gatccaggtc gccaacacca ccaagcccat 7560
tgacgacatc accaaacacc tgccccgacc atgcattctg atttccaacc accagaacga 7620
aatggacatt ctggtgctcg gtcgcatctt cccccagtac tgctccgtca ccgccaaaaa 7680
ggccctcaag tggtaccctc tgctgggcca gttcatggcg ctgtccggca ccatcttcct 7740
ggaccgaaag gaccgaacca agtccgtgca gaccctcggc ggcgccgtca agaccatcca 7800
gagcggcaac ggaggcaagg gccagagcgt cttcatgttc cccgagggaa cccgatccta 7860
ctccaaggac gtcggcatca tgcccttcaa gaagggctgt ttccacctgg cggtccagtc 7920
gggcgctccc attgtccccg tggtggtcca gaacacctcc cgaatgtttt ctttcggccg 7980
aggcaagctg gacgccggag agatccttgt cgacgtcctg agccccattg agaccaaggg 8040
tctggacgcc agcaacgtcg acgctctcat ggccaccact tataaggcca tgtgcgagac 8100
tgccgaccag attggctacg ctggccagaa gactcagtag gcggccgcat gagaagataa 8160
atatataaat acattgagat attaaatgcg ctagattaga gagcctcata ctgctcggag 8220
agaagccaag acgagtactc aaaggggatt acaccatcca tatccacaga cacaagctgg 8280
ggaaaggttc tatatacact ttccggaata ccgtagtttc cgatgttatc aatgggggca 8340
gccaggattt caggcacttc ggtgtctcgg ggtgaaatgg cgttcttggc ctccatcaag 8400

tcgtaccatg tcttcatttg cctgtcaaag taaaacagaa gcagatgaag aatgaacttg 8460

aagtgaagga atttaaatgt aacgaaactg aaatttgacc agatattgtg tccgcggtgg 8520

agctccagct tttgttccct ttagtgaggg ttaatttcga gcttggcgta atcatggtca 8580

tagctgtttc ctgtgtgaaa ttgttatccg ctcacaagct tccacacaac 8630

<210> 150
<211> 8630
<212> DNA
<213> Artificial Sequence

<220>
<223> Plasmid pY175

<400> 150

```
gtacgataac ttcgtatagc atacattata cgaagttatc gcgtcgacga gtatctgtct      60
gactcgtcat tgccgccttt ggagtacgac tccaactatg agtgtgcttg gatcactttg     120
acgatacatt cttcgttgga ggctgtgggt ctgacagctg cgttttcggc gcggttggcc     180
gacaacaata tcagctgcaa cgtcattgct ggctttcatc atgatcacat ttttgtcggc     240
aaaggcgacg cccagagagc cattgacgtt ctttctaatt tggaccgata gccgtatagt     300
ccagtctatc tataagttca actaactcgt aactattacc ataacatata cttcactgcc     360
ccagataagg ttccgataaa aagttctgca gactaaattt atttcagtct cctcttcacc     420
accaaaatgc cctcctacga agctcgagct aacgtccaca agtccgcctt tgccgctcga     480
gtgctcaagc tcgtggcagc caagaaaacc aacctgtgtg cttctctgga tgttaccacc     540
accaaggagc tcattgagct tgccgataag gtcggacctt atgtgtgcat gatcaaaacc     600
catatcgaca tcattgacga cttcacctac gccggcactg tgctcccccct caaggaactt     660
gctcttaagc acggtttctt cctgttcgag gacagaaagt tcgcagatat tggcaacact     720
gtcaagcacc agtaccggtg tcaccgaatc gccgagtggt ccgatatcac caacgcccac     780
ggtgtacccg gaaccggaat cattgctggc ctgcgagctg gtgccgagga aactgtctct     840
gaacagaaga aggaggacgt ctctgactac gagaactccc agtacaagga gttcctagtc     900
ccctctccca acgagaagct ggccagaggt ctgctcatgc tggccgagct gtcttgcaag     960
ggctctctgg ccactggcga gtactccaag cagaccattg agcttgcccg atccgacccc    1020
gagtttgtgg ttggcttcat tgcccagaac cgacctaagg gcgactctga ggactggctt    1080
attctgaccc ccggggtggg tcttgacgac aagggagacg ctctcggaca gcagtaccga    1140
actgttgagg atgtcatgtc taccggaacg gatatcataa ttgtcggccg aggtctgtac    1200
ggccagaacc gagatcctat tgaggaggcc aagcgatacc agaaggctgg ctgggaggct    1260
taccagaaga ttaactgtta gaggttagac tatggatatg taatttaact gtgtatatag    1320
agagcgtgca agtatggagc gcttgttcag cttgtatgat ggtcagacga cctgtctgat    1380
cgagtatgta tgatactgca caacctgtgt atccgcatga tctgtccaat ggggcatgtt    1440
```

gttgtgtttc tcgatacgga gatgctgggt acagtgctaa tacgttgaac tacttatact 1500

tatatgaggc tcgaagaaag ctgacttgtg tatgacttat tctcaactac atccccagtc 1560

acaataccac cactgcacta ccactacacc aaaaccatga tcaaaccacc catggacttc 1620

ctggaggcag aagaacttgt tatggaaaag ctcaagagag agatcataac ttcgtatagc 1680

atacattata cgaagttatc ctgcaggtaa aggaattcag gagagaccgg gttggcggcg 1740

tatttgtgtc ccaaaaaaca gccccaattg ccccaattga ccccaaattg acccagtagc 1800

gggcccaacc ccggcgagag cccccttcac cccacatatc aaacctcccc cggttcccac 1860

acttgccgtt aagggcgtag ggtactgcag tctggaatct acgcttgttc agactttgta 1920

ctagtttctt tgtctggcca tccgggtaac ccatgccgga cgcaaaatag actactgaaa 1980

atttttttgc tttgtggttg ggactttagc caagggtata aaagaccacc gtccccgaat 2040

tacctttcct cttcttttct ctctctcctt gtcaactcac acccgaaatc gttaagcatt 2100

tccttctgag tataagaatc attcaccatg gacttcctgg aggcagaaga acttgttatg 2160

gaaaagctca agagagagaa gccaagatac tatcaagaca tgtgtcgcaa cttaattaag 2220

atgacgacat ttgcgagctg gacgaggaat agatggagcg tgtgttctga gtcgatgttt 2280

tctatggagt tgtgagtgtt agtagacatg atgggtttat atatgatgaa tgaatagatg 2340

tgattttgat ttgcacgatg gaattgagaa ctttgtaaac gtacatggga atgtatgaat 2400

gtgggggttt tgtgactgga taactgacgg tcagtggacg ccgttgttca aatatccaag 2460

agatgcgaga aactttgggt caagtgaaca tgtcctctct gttcaagtaa accatcaact 2520

atgggtagta tatttagtaa ggacaagagt tgagattctt tggagtccta gaaacgtatt 2580

ttcgcgttcc aagatcaaat tagtagagta atacgggcac gggaatccat tcatagtctc 2640

aattttccca taggtgtgct acaaggtgtt gagatgtggt acagtaccac catgattcga 2700

ggtaaagagc ccagaagtca ttgatgaggt caagaaatac acagatctac agctcaatac 2760

aatgaatatc ttctttcata ttcttcaggt gacaccaagg gtgtctattt tccccagaaa 2820

tgcgtgaaaa ggcgcgtgtg tagcgtggag tatgggttcg gttggcgtat ccttcatata 2880

tcgacgaaat agtagggcaa gagatgacaa aaagtatcta tatgtagaca gcgtagaata 2940

tggatttgat tggtataaat tcatttattg cgtgtctcac aaatactctc gataagttgg 3000

ggttaaactg gagatggaac aatgtcgata tctcgacgca tgcgacgtcg ggcccaattc 3060

gccctatagt gagtcgtatt acaattcact ggccgtcgtt ttacaacgtc gtgactggga 3120

aaaccctggc gttacccaac ttaatcgcct tgcagcacat ccccctttcg ccagctggcg 3180

taatagcgaa gaggcccgca ccgatcgccc ttcccaacag ttgcgcagcc tgaatggcga 3240

atggacgcgc cctgtagcgg cgcattaagc gcggcgggtg tggtggttac gcgcagcgtg 3300

accgctacac ttgccagcgc cctagcgccc gctcctttcg ctttcttccc ttcctttctc 3360

gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720
ttacgcatct gtgcggtatt tcacaccgca tcaggtggca cttttcgggg aaatgtgcgc 3780
ggaaccccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260
atgagtgata acactgcggc caacttactt ctgacaacga tcggaggacc gaaggagcta 4320
accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440
acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340

gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640
cgaacgaccg agcgcagcga gtcagtgagc gaggaagcgg aagagcgccc aatacgcaaa 5700
ccgcctctcc ccgcgcgttg gccgattcat taatgcagct ggcgcgccac caatcacaat 5760
tctgaaaagc acatcttgat ctcctcattg cggggagtcc aacggtggtc ttattccccc 5820
gaatttcccg ctcaatctcg ttccagaccg acccggacac agtgcttaac gccgttccga 5880
aactctaccg cagatatgct ccaacggact gggctgcata gatgtgatcc tcggcttgga 5940
gaaatggata aaagccggcc aaaaaaaaag cggaaaaaag cggaaaaaaa gagaaaaaaa 6000
atcgcaaaat ttgaaaaata gggggaaaag acgcaaaaac gcaaggaggg gggagtatat 6060
gacactgata agcaagctca caacggttcc tcttattttt ttcctcatct tctgcctagg 6120
ttcccaaaat cccagatgct tctctccagt gccaaaagta agtaccccac aggttttcgg 6180
ccgaaaattc cacgtgcagc aacgtcgtgt ggggtgttaa aatgtggggg gggggaacca 6240
ggacaagagg ctcttgtggg agccgaatga gagcacaaag cgggcgggtg tgataagggc 6300
atttttgccc attttccctt ctcctgtctc tccgacggtg atggcgttgt gcgtcctcta 6360
tttcttttta tttcttttttg ttttatttct ctgactaccg atttggtttg atttcctcaa 6420
ccccacacaa ataagctcgg gccgaggaat atatatatac acggacacag tcgccctgtg 6480
gacaacacgt cactacctct acgatacaca ccgtacgata gttagtagac aacaatcgat 6540
agttggagca agggagaaat gtagagtgtg aaagactcac tatggtccgg gcttatctcg 6600
accaatagcc aaagtctgga gtttctgaga gaaaaaggca agatacgtat gtaacaaagc 6660
gacgcatggt acaataatac cggaggcatg tatcatagag agttagtggt tcgatgatgg 6720
cactggtgcc tggtatgact ttatacggct gactacatat ttgtcctcag acatacaatt 6780
acagtcaagc acttaccctt ggacatctgt aggtaccccc cggccaagac gatctcagcg 6840
tgtcgtatgt cggattggcg tagctccctc gctcgtcaat tggctcccat ctactttctt 6900
ctgcttggct acacccagca tgtctgctat ggctcgtttt cgtgccttat ctatcctccc 6960
agtattacca actctaaatg acatgatgtg attgggtcta cactttcata tcagagataa 7020
ggagtagcac agttgcataa aaagcccaac tctaatcagc ttcttccttt cttgtaatta 7080
gtacaaaggt gattagcgaa atctggaagc ttagttggcc ctaaaaaaat caaaaaaagc 7140
aaaaaacgaa aaacgaaaaa ccacagtttt gagaacaggg aggtaacgaa ggatcgtata 7200
tatatatata tatatatata cccacggatc ccgagaccgg cctttgattc ttccctacaa 7260

ccaaccattc tcaccaccct aattcacaac catggagaac ttctggtcca tcgtcgtgtt 7320

ctttctgctc tccattctgt tcatcctcta caacatttcg acagtctgcc actactacat 7380

gcgaatctcc ttctactact ttaccatcct gcttcacggc atggaggtgt gcgttaccat 7440

gattccctct tggctcaacg gcaagggtgc cgactacgtg tttcactcgt tcttctactg 7500

gtgcaagtgg actggagtcc acaccactgt gtatggctac gagaagaccc aggtcgaagg 7560

tcctgccgtg gtcatctgca accatcagtc ctcgctcgac attctgtcta tggcttccat 7620

ctggcccaag aactgtgttg tcatgatgaa gcggattctt gcctacgttc ccttcttcaa 7680

cctgggagcc tacttttcca acaccatctt catcgaccga tacaaccgag agcgagctat 7740

ggcttctgtc gactactgtg cctccgagat gaagaaccga aacctgaagc tctgggtgtt 7800

tcccgaaggc actcggaatc gagagggtgg attcattccc ttcaagaaag gtgccttcaa 7860

catcgctgtt cgagcccaga ttcccatcat tcctgtcgtg ttctctgact atcgagactt 7920

ctactccaag cctggccgat acttcaagaa cgatggagag gtcgtgatcc gagtcctgga 7980

tgccattccc accaagggtc tgaccctcga tgacgtctct gagctttcgg acatgtgtcg 8040

agacgtcatg ctggctgcct acaaggaagt taccctcgag gctcagcaac gaaacgccac 8100

tcgaagagga gagaccaagg acggcaagaa atccgagtaa gcggccgcat gagaagataa 8160

atatataaat acattgagat attaaatgcg ctagattaga gagcctcata ctgctcggag 8220

agaagccaag acgagtactc aaaggggatt acaccatcca tatccacaga cacaagctgg 8280

ggaaaggttc tatatacact ttccggaata ccgtagtttc cgatgttatc aatgggggca 8340

gccaggattt caggcacttc ggtgtctcgg ggtgaaatgg cgttcttggc ctccatcaag 8400

tcgtaccatg tcttcatttg cctgtcaaag taaaacagaa gcagatgaag aatgaacttg 8460

aagtgaagga atttaaatgt aacgaaactg aaatttgacc agatattgtg tccgcggtgg 8520

agctccagct tttgttccct ttagtgaggg ttaatttcga gcttggcgta atcatggtca 8580

tagctgtttc ctgtgtgaaa ttgttatccg ctcacaagct tccacacaac 8630

<210> 151
<211> 926
<212> DNA
<213> Saccharomyces cerevisiae

<220>
<221> CDS
<222> (4)..(915)

<400> 151

```
aac atg tct gtt att gga cga ttt ctt tac tac ctg aga tcg gtg ctc         48
    Met Ser Val Ile Gly Arg Phe Leu Tyr Tyr Leu Arg Ser Val Leu
    1               5                   10                  15

gtc gtt ttg gcc ctc gct gga tgt ggc ttc tat ggc gtg att gcc tct         96
Val Val Leu Ala Leu Ala Gly Cys Gly Phe Tyr Gly Val Ile Ala Ser
                20                  25                  30
```

```
atc ctg tgt act ctc atc ggc aag cag cat ctc gcg caa tgg att acc      144
Ile Leu Cys Thr Leu Ile Gly Lys Gln His Leu Ala Gln Trp Ile Thr
            35                  40                  45

gcc cga tgc ttt tac cac gtg atg aaa ctg atg ctg gga ttg gac gtc      192
Ala Arg Cys Phe Tyr His Val Met Lys Leu Met Leu Gly Leu Asp Val
        50                  55                  60

aaa gtc gtg ggt gaa gag aac ctg gct aag aag ccc tat atc atg atc      240
Lys Val Val Gly Glu Glu Asn Leu Ala Lys Lys Pro Tyr Ile Met Ile
    65                  70                  75

gct aac cac cag tcc acc ctc gat atc ttt atg ctc ggc aga atc ttc      288
Ala Asn His Gln Ser Thr Leu Asp Ile Phe Met Leu Gly Arg Ile Phe
80                  85                  90                  95

cct ccc gga tgc acc gtt acc gca aag aag agc ctt aag tac gtc ccc      336
Pro Pro Gly Cys Thr Val Thr Ala Lys Lys Ser Leu Lys Tyr Val Pro
                100                 105                 110

ttc ctg ggc tgg ttt atg gcg ctt tcc ggt aca tac ttc ctc gac cgt      384
Phe Leu Gly Trp Phe Met Ala Leu Ser Gly Thr Tyr Phe Leu Asp Arg
            115                 120                 125

tcc aag cgg caa gag gct att gat acc ctg aac aaa gga ctg gag aac      432
Ser Lys Arg Gln Glu Ala Ile Asp Thr Leu Asn Lys Gly Leu Glu Asn
        130                 135                 140

gtc aaa aag aat aag cga gcc ctc tgg gtt ttt ccc gaa ggt acc cgg      480
Val Lys Lys Asn Lys Arg Ala Leu Trp Val Phe Pro Glu Gly Thr Arg
    145                 150                 155

tct tac acg tcg gag ctt acc atg ctg ccg ttc aag aag ggc gcc ttt      528
Ser Tyr Thr Ser Glu Leu Thr Met Leu Pro Phe Lys Lys Gly Ala Phe
160                 165                 170                 175

cat ttg gcc cag cag gga aag atc cct atc gtc cca gtg gtt gtg tct      576
His Leu Ala Gln Gln Gly Lys Ile Pro Ile Val Pro Val Val Val Ser
                180                 185                 190

aac act agc acg ctc gtc agc cct aag tac ggt gtg ttc aac cga ggc      624
Asn Thr Ser Thr Leu Val Ser Pro Lys Tyr Gly Val Phe Asn Arg Gly
            195                 200                 205

tgt atg att gtc cga att ctg aag ccc atc tcg act gag aac ctg aca      672
Cys Met Ile Val Arg Ile Leu Lys Pro Ile Ser Thr Glu Asn Leu Thr
        210                 215                 220

aaa gat aag att ggc gag ttc gct gag aaa gtg cga gat cag atg gtt      720
Lys Asp Lys Ile Gly Glu Phe Ala Glu Lys Val Arg Asp Gln Met Val
    225                 230                 235

gac aca ctg aag gaa atc ggt tat tcc ccc gca atc aac gac acc acc      768
Asp Thr Leu Lys Glu Ile Gly Tyr Ser Pro Ala Ile Asn Asp Thr Thr
240                 245                 250                 255

ctt cct ccg cag gca att gag tac gcc gca ttg cag cat gac aag aag      816
Leu Pro Pro Gln Ala Ile Glu Tyr Ala Ala Leu Gln His Asp Lys Lys
                260                 265                 270

gtg aac aaa aag att aag aac gaa cct gtt cca tcg gtg tcc att tct      864
Val Asn Lys Lys Ile Lys Asn Glu Pro Val Pro Ser Val Ser Ile Ser
            275                 280                 285
```

```
aat gat gtg aat act cac aac gaa gga tcg tct gtc aag aag atg cac        912
Asn Asp Val Asn Thr His Asn Glu Gly Ser Ser Val Lys Lys Met His
        290                 295                 300

tag gcggccgcat g                                                       926
```

<210> 152
<211> 303
<212> PRT
<213> Saccharomyces cerevisiae

<400> 152

```
Met Ser Val Ile Gly Arg Phe Leu Tyr Tyr Leu Arg Ser Val Leu Val
1               5                   10                  15

Val Leu Ala Leu Ala Gly Cys Gly Phe Tyr Gly Val Ile Ala Ser Ile
            20                  25                  30

Leu Cys Thr Leu Ile Gly Lys Gln His Leu Ala Gln Trp Ile Thr Ala
        35                  40                  45

Arg Cys Phe Tyr His Val Met Lys Leu Met Leu Gly Leu Asp Val Lys
    50                  55                  60

Val Val Gly Glu Glu Asn Leu Ala Lys Lys Pro Tyr Ile Met Ile Ala
65                  70                  75                  80

Asn His Gln Ser Thr Leu Asp Ile Phe Met Leu Gly Arg Ile Phe Pro
                85                  90                  95

Pro Gly Cys Thr Val Thr Ala Lys Lys Ser Leu Lys Tyr Val Pro Phe
            100                 105                 110

Leu Gly Trp Phe Met Ala Leu Ser Gly Thr Tyr Phe Leu Asp Arg Ser
        115                 120                 125

Lys Arg Gln Glu Ala Ile Asp Thr Leu Asn Lys Gly Leu Glu Asn Val
    130                 135                 140

Lys Lys Asn Lys Arg Ala Leu Trp Val Phe Pro Glu Gly Thr Arg Ser
145                 150                 155                 160

Tyr Thr Ser Glu Leu Thr Met Leu Pro Phe Lys Lys Gly Ala Phe His
                165                 170                 175

Leu Ala Gln Gln Gly Lys Ile Pro Ile Val Pro Val Val Val Ser Asn
            180                 185                 190

Thr Ser Thr Leu Val Ser Pro Lys Tyr Gly Val Phe Asn Arg Gly Cys
        195                 200                 205
```

```
Met Ile Val Arg Ile Leu Lys Pro Ile Ser Thr Glu Asn Leu Thr Lys
    210                 215                 220

Asp Lys Ile Gly Glu Phe Ala Glu Lys Val Arg Asp Gln Met Val Asp
225                 230                 235                 240

Thr Leu Lys Glu Ile Gly Tyr Ser Pro Ala Ile Asn Asp Thr Thr Leu
                245                 250                 255

Pro Pro Gln Ala Ile Glu Tyr Ala Ala Leu Gln His Asp Lys Lys Val
            260                 265                 270

Asn Lys Lys Ile Lys Asn Glu Pro Val Pro Ser Val Ser Ile Ser Asn
        275                 280                 285

Asp Val Asn Thr His Asn Glu Gly Ser Ser Val Lys Lys Met His
    290                 295                 300
```

<210> 153
<211> 7891
<212> DNA
<213> Artificial Sequence

<220>
<223> Plasmid pY222

<400> 153

```
tcgaccgtac gatagttagt agacaacaat cgatagttgg agcaagggag aaatgtagag      60
tgtgaaagac tcactatggt ccgggcttat ctcgaccaat agccaaagtc tggagtttct     120
gagagaaaaa ggcaagatac gtatgtaaca aagcgacgca tggtacaata ataccggagg     180
catgtatcat agagagttag tggttcgatg atggcactgg tgcctggtat gactttatac     240
ggctgactac atatttgtcc tcagacatac aattacagtc aagcacttac ccttggacat     300
ctgtaggtac cccccggcca agacgatctc agcgtgtcgt atgtcggatt ggcgtagctc     360
cctcgctcgt caattggctc ccatctactt tcttctgctt ggctacaccc agcatgtctg     420
ctatggctcg ttttcgtgcc ttatctatcc tcccagtatt accaactcta aatgacatga     480
tgtgattggg tctacacttt catatcagag ataaggagta gcacagttgc ataaaaagcc     540
caactctaat cagcttcttc ctttcttgta attagtacaa aggtgattag cgaaatctgg     600
aagcttagtt ggccctaaaa aaatcaaaaa aagcaaaaaa cgaaaaacga aaaaccacag     660
ttttgagaac agggaggtaa cgaaggatcg tatatatata tatatatata tatacccacg     720
gatcccgaga ccggcctttg attcttccct acaaccaacc attctcacca ccctaattca     780
caaccatgtc tgttattgga cgatttcttt actacctgag atcggtgctc gtcgttttgg     840
ccctcgctgg atgtggcttc tatggcgtga ttgcctctat cctgtgtact ctcatcggca     900
agcagcatct cgcgcaatgg attaccgccc gatgctttta ccacgtgatg aaactgatgc     960
```

tgggattgga cgtcaaagtc gtgggtgaag agaacctggc taagaagccc tatatcatga 1020

tcgctaacca ccagtccacc ctcgatatct ttatgctcgg cagaatcttc cctcccggat 1080

gcaccgttac cgcaaagaag agccttaagt acgtcccctt cctgggctgg tttatggcgc 1140

tttccggtac atacttcctc gaccgttcca agcggcaaga ggctattgat accctgaaca 1200

aaggactgga gaacgtcaaa aagaataagc gagccctctg ggtttttccc gaaggtaccc 1260

ggtcttacac gtcggagctt accatgctgc cgttcaagaa gggcgccttt catttggccc 1320

agcagggaaa gatccctatc gtcccagtgg ttgtgtctaa cactagcacg ctcgtcagcc 1380

ctaagtacgg tgtgttcaac cgaggctgta tgattgtccg aattctgaag cccatctcga 1440

ctgagaacct gacaaaagat aagattggcg agttcgctga gaaagtgcga gatcagatgg 1500

ttgacacact gaaggaaatc ggttattccc ccgcaatcaa cgacaccacc cttcctccgc 1560

aggcaattga gtacgccgca ttgcagcatg acaagaaggt gaacaaaaag attaagaacg 1620

aacctgttcc atcggtgtcc atttctaatg atgtgaatac tcacaacgaa ggatcgtctg 1680

tcaagaagat gcactaggcg gccgcatgag aagataaata tataaataca ttgagatatt 1740

aaatgcgcta gattagagag cctcatactg ctcggagaga agccaagacg agtactcaaa 1800

ggggattaca ccatccatat ccacagacac aagctgggga aaggttctat atacactttc 1860

cggaataccg tagtttccga tgttatcaat gggggcagcc aggatttcag gcacttcggt 1920

gtctcggggt gaaatggcgt tcttggcctc catcaagtcg taccatgtct tcatttgcct 1980

gtcaaagtaa aacagaagca gatgaagaat gaacttgaag tgaaggaatt taaatgtaac 2040

gaaactgaaa tttgaccaga tattgtgtcc gcggtggagc tccagctttt gttcccttta 2100

gtgagggtta atttcgagct tggcgtaatc atggtcatag ctgtttcctg tgtgaaattg 2160

ttatccgctc acaagcttcc acacaacgta cgagccggaa gcataaagtg taaagcctgg 2220

ggtgcctaat gagtgagcta actcacatta attgcgttgc gctcactgcc cgctttccag 2280

tcgggaaacc tgtcgtgcca gctgcattaa tgaatcggcc aacgcgcggg gagaggcggt 2340

ttgcgtattg ggcgctcttc cgcttcctcg ctcactgact cgctgcgctc ggtcgttcgg 2400

ctgcggcgag cggtatcagc tcactcaaag gcggtaatac ggttatccac agaatcaggg 2460

gataacgcag gaaagaacat gtgagcaaaa ggccagcaaa aggccaggaa ccgtaaaaag 2520

gccgcgttgc tggcgttttt ccataggctc cgcccccctg acgagcatca caaaaatcga 2580

cgctcaagtc agaggtggcg aaacccgaca ggactataaa gataccaggc gtttccccct 2640

ggaagctccc tcgtgcgctc tcctgttccg accctgccgc ttaccggata cctgtccgcc 2700

tttctccctt cgggaagcgt ggcgctttct catagctcac gctgtaggta tctcagttcg 2760

gtgtaggtcg ttcgctccaa gctgggctgt gtgcacgaac cccccgttca gcccgaccgc 2820

tgcgccttat ccggtaacta tcgtcttgag tccaacccgg taagacacga cttatcgcca 2880

ctggcagcag ccactggtaa caggattagc agagcgaggt atgtaggcgg tgctacagag 2940
ttcttgaagt ggtggcctaa ctacggctac actagaagga cagtatttgg tatctgcgct 3000
ctgctgaagc cagttacctt cggaaaaaga gttggtagct cttgatccgg caaacaaacc 3060
accgctggta gcggtggttt ttttgtttgc aagcagcaga ttacgcgcag aaaaaaagga 3120
tctcaagaag atcctttgat cttttctacg gggtctgacg ctcagtggaa cgaaaactca 3180
cgttaaggga ttttggtcat gagattatca aaaaggatct tcacctagat ccttttaaat 3240
taaaaatgaa gttttaaatc aatctaaagt atatatgagt aaacttggtc tgacagttac 3300
caatgcttaa tcagtgaggc acctatctca gcgatctgtc tatttcgttc atccatagtt 3360
gcctgactcc ccgtcgtgta gataactacg atacgggagg gcttaccatc tggccccagt 3420
gctgcaatga taccgcgaga cccacgctca ccggctccag atttatcagc aataaaccag 3480
ccagccggaa gggccgagcg cagaagtggt cctgcaactt tatccgcctc catccagtct 3540
attaattgtt gccgggaagc tagagtaagt agttcgccag ttaatagttt gcgcaacgtt 3600
gttgccattg ctacaggcat cgtggtgtca cgctcgtcgt ttggtatggc ttcattcagc 3660
tccggttccc aacgatcaag gcgagttaca tgatccccca tgttgtgcaa aaaagcggtt 3720
agctccttcg gtcctccgat cgttgtcaga agtaagttgg ccgcagtgtt atcactcatg 3780
gttatggcag cactgcataa ttctcttact gtcatgccat ccgtaagatg cttttctgtg 3840
actggtgagt actcaaccaa gtcattctga gaatagtgta tgcggcgacc gagttgctct 3900
tgcccggcgt caatacggga taataccgcg ccacatagca gaactttaaa agtgctcatc 3960
attggaaaac gttcttcggg gcgaaaactc tcaaggatct taccgctgtt gagatccagt 4020
tcgatgtaac ccactcgtgc acccaactga tcttcagcat cttttacttt caccagcgtt 4080
tctgggtgag caaaaacagg aaggcaaaat gccgcaaaaa agggaataag ggcgacacgg 4140
aaatgttgaa tactcatact cttccttttt caatattatt gaagcattta tcagggttat 4200
tgtctcatga gcggatacat atttgaatgt atttagaaaa ataaacaaat aggggttccg 4260
cgcacatttc cccgaaaagt gccacctgac gcgccctgta gcggcgcatt aagcgcggcg 4320
ggtgtggtgg ttacgcgcag cgtgaccgct acacttgcca gcgccctagc gcccgctcct 4380
ttcgctttct tcccttcctt tctcgccacg ttcgccggct ttccccgtca agctctaaat 4440
cgggggctcc ctttagggtt ccgatttagt gctttacggc acctcgaccc caaaaaactt 4500
gattagggtg atggttcacg tagtgggcca tcgccctgat agacggtttt tcgccctttg 4560
acgttggagt ccacgttctt taatagtgga ctcttgttcc aaactggaac aacactcaac 4620
cctatctcgg tctattcttt tgatttataa gggattttgc cgatttcggc ctattggtta 4680
aaaaatgagc tgatttaaca aaaatttaac gcgaatttta acaaaatatt aacgcttaca 4740
atttccattc gccattcagg ctgcgcaact gttgggaagg gcgatcggtg cgggcctctt 4800
cgctattacg ccagctggcg aaagggggat gtgctgcaag gcgattaagt tgggtaacgc 4860

cagggttttc ccagtcacga cgttgtaaaa cgacggccag tgaattgtaa tacgactcac 4920
tatagggcga attgggtacc gggccccccc tcgaggtcga tggtgtcgat aagcttgata 4980
tcgaattcat gtcacacaaa ccgatcttcg cctcaaggaa acctaattct acatccgaga 5040
gactgccgag atccagtcta cactgattaa ttttcgggcc aataatttaa aaaaatcgtg 5100
ttatataata ttatatgtat tatatatata catcatgatg atactgacag tcatgtccca 5160
ttgctaaata gacagactcc atctgccgcc tccaactgat gttctcaata tttaaggggt 5220
catctcgcat tgtttaataa taaacagact ccatctaccg cctccaaatg atgttctcaa 5280
aatatattgt atgaacttat ttttattact tagtattatt agacaactta cttgctttat 5340
gaaaaacact tcctatttag gaaacaattt ataatggcag ttcgttcatt taacaattta 5400
tgtagaataa atgttataaa tgcgtatggg aaatcttaaa tatggatagc ataaatgata 5460
tctgcattgc ctaattcgaa atcaacagca acgaaaaaaa tcccttgtac aacataaata 5520
gtcatcgaga aatatcaact atcaaagaac agctattcac acgttactat tgagattatt 5580
attggacgag aatcacacac tcaactgtct ttctctcttc tagaaataca ggtacaagta 5640
tgtactattc tcattgttca tacttctagt catttcatcc cacatattcc ttggatttct 5700
ctccaatgaa tgacattcta tcttgcaaat tcaacaatta taataagata taccaaagta 5760
gcggtatagt ggcaatcaaa aagcttctct ggtgtgcttc tcgtatttat ttttattcta 5820
atgatccatt aaaggtatat atttatttct tgttatataa tccttttgtt tattacatgg 5880
gctggataca taaaggtatt ttgatttaat tttttgctta aattcaatcc cccctcgttc 5940
agtgtcaact gtaatggtag gaaattacca tacttttgaa gaagcaaaaa aaatgaaaga 6000
aaaaaaaaat cgtatttcca ggttagacgt tccgcagaat ctagaatgcg gtatgcggta 6060
cattgttctt cgaacgtaaa agttgcgctc cctgagatat tgtacatttt tgcttttaca 6120
agtacaagta catcgtacaa ctatgtacta ctgttgatgc atccacaaca gtttgttttg 6180
ttttttttg ttttttttt ttctaatgat tcattaccgc tatgtatacc tacttgtact 6240
tgtagtaagc cgggttattg gcgttcaatt aatcatagac ttatgaatct gcacggtgtg 6300
cgctgcgagt tacttttagc ttatgcatgc tacttgggtg taatattggg atctgttcgg 6360
aaatcaacgg atgctcaatc gatttcgaca gtaattaatt aagtcataca caagtcagct 6420
ttcttcgagc ctcatataag tataagtagt tcaacgtatt agcactgtac ccagcatctc 6480
cgtatcgaga aacacaacaa catgccccat tggacagatc atgcggatac acaggttgtg 6540
cagtatcata catactcgat cagacaggtc gtctgaccat catacaagct gaacaagcgc 6600
tccatacttg cacgctctct atatacacag ttaaattaca tatccatagt ctaacctcta 6660
acagttaatc ttctggtaag cctcccagcc agccttctgg tatcgcttgg cctcctcaat 6720
aggatctcgg ttctggccgt acagacctcg gccgacaatt atgatatccg ttccggtaga 6780

```
catgacatcc tcaacagttc ggtactgctg tccgagagcg tctcccttgt cgtcaagacc     6840
caccccgggg gtcagaataa gccagtcctc agagtcgccc ttaggtcggt tctgggcaat     6900
gaagccaacc acaaactcgg ggtcggatcg ggcaagctca atggtctgct tggagtactc     6960
gccagtggcc agagagccct tgcaagacag ctcggccagc atgagcagac ctctggccag     7020
cttctcgttg ggagagggga ctaggaactc cttgtactgg gagttctcgt agtcagagac     7080
gtcctccttc ttctgttcag agacagtttc ctcggcacca gctcgcaggc cagcaatgat     7140
tccggttccg ggtacaccgt gggcgttggt gatatcggac cactcggcga ttcggtgaca     7200
ccggtactgg tgcttgacag tgttgccaat atctgcgaac tttctgtcct cgaacaggaa     7260
gaaaccgtgc ttaagagcaa gttccttgag ggggagcaca gtgccggcgt aggtgaagtc     7320
gtcaatgatg tcgatatggg ttttgatcat gcacacataa ggtccgacct tatcggcaag     7380
ctcaatgagc tccttggtgg tggtaacatc cagagaagca cacaggttgg ttttcttggc     7440
tgccacgagc ttgagcactc gagcggcaaa ggcggacttg tggacgttag ctcgagcttc     7500
gtaggagggc atttggtgg tgaagaggag actgaaataa atttagtctg cagaactttt     7560
tatcggaacc ttatctgggg cagtgaagta tatgttatgg taatagttac gagttagttg     7620
aacttataga tagactggac tatacggcta tcggtccaaa ttagaaagaa cgtcaatggc     7680
tctctgggcg tcgcctttgc cgacaaaaat gtgatcatga tgaaagccag caatgacgtt     7740
gcagctgata ttgttgtcgg ccaaccgcgc cgaaaacgca gctgtcagac ccacagcctc     7800
caacgaagaa tgtatcgtca aagtgatcca agcacactca tagttggagt cgtactccaa     7860
aggcggcaat gacgagtcag acagatactc g                                    7891
```

<210> 154
<211> 32
<212> DNA
<213> Artificial Sequence

<220>
<223> Primer 869

<400> 154
actttggcgcg ccactgccga gatccagtct ac 32

<210> 155
<211> 28
<212> DNA
<213> Artificial Sequence

<220>
<223> Primer 870

<400> 155
tcagcctagg agcatccgtt gatttccg 28

<210> 156
<211> 9598
<212> DNA
<213> Artificial Sequence

<220>
<223> Plasmid pY177

<400> 156

gtacgataac ttcgtatagc atacattata cgaagttatc gcgtcgacga gtatctgtct 60
gactcgtcat tgccgccttt ggagtacgac tccaactatg agtgtgcttg gatcactttg 120
acgatacatt cttcgttgga ggctgtgggt ctgacagctg cgttttcggc gcggttggcc 180
gacaacaata tcagctgcaa cgtcattgct ggctttcatc atgatcacat ttttgtcggc 240
aaaggcgacg cccagagagc cattgacgtt ctttctaatt tggaccgata gccgtatagt 300
ccagtctatc tataagttca actaactcgt aactattacc ataacatata cttcactgcc 360
ccagataagg ttccgataaa aagttctgca gactaaattt atttcagtct cctcttcacc 420
accaaaatgc cctcctacga agctcgagct aacgtccaca agtccgcctt tgccgctcga 480
gtgctcaagc tcgtggcagc caagaaaacc aacctgtgtg cttctctgga tgttaccacc 540
accaaggagc tcattgagct tgccgataag gtcggacctt atgtgtgcat gatcaaaacc 600
catatcgaca tcattgacga cttcacctac gccggcactg tgctcccccct caaggaactt 660
gctcttaagc acggtttctt cctgttcgag gacagaaagt tcgcagatat tggcaacact 720
gtcaagcacc agtaccggtg tcaccgaatc gccgagtggt ccgatatcac caacgcccac 780
ggtgtacccg gaaccggaat cattgctggc ctgcgagctg gtgccgagga aactgtctct 840
gaacagaaga aggaggacgt ctctgactac gagaactccc agtacaagga gttcctagtc 900
ccctctccca acgagaagct ggccagaggt ctgctcatgc tggccgagct gtcttgcaag 960
ggctctctgg ccactggcga gtactccaag cagaccattg agcttgcccg atccgacccc 1020
gagtttgtgg ttggcttcat tgcccagaac cgacctaagg gcgactctga ggactggctt 1080
attctgaccc ccggggtggg tcttgacgac aagggagacg ctctcggaca gcagtaccga 1140
actgttgagg atgtcatgtc taccggaacg gatatcataa ttgtcggccg aggtctgtac 1200
ggccagaacc gagatcctat tgaggaggcc aagcgatacc agaaggctgg ctgggaggct 1260
taccagaaga ttaactgtta gaggttagac tatggatatg taatttaact gtgtatatag 1320
agagcgtgca agtatggagc gcttgttcag cttgtatgat ggtcagacga cctgtctgat 1380
cgagtatgta tgatactgca caacctgtgt atccgcatga tctgtccaat ggggcatgtt 1440
gttgtgtttc tcgatacgga gatgctgggt acagtgctaa tacgttgaac tacttatact 1500
tatatgaggc tcgaagaaag ctgacttgtg tatgacttat tctcaactac atccccagtc 1560
acaataccac cactgcacta ccactacacc aaaaccatga tcaaaccacc catggacttc 1620
ctggaggcag aagaacttgt tatggaaaag ctcaagagag agatcataac ttcgtatagc 1680
atacattata cgaagttatc ctgcaggtaa aggaattcag gagagaccgg gttggcggcg 1740

tatttgtgtc ccaaaaaaca gccccaattg ccccaattga ccccaaattg acccagtagc 1800
gggcccaacc ccggcgagag ccccccttcac cccacatatc aaacctcccc cggttcccac 1860
acttgccgtt aagggcgtag ggtactgcag tctggaatct acgcttgttc agactttgta 1920
ctagtttctt tgtctggcca tccgggtaac ccatgccgga cgcaaaatag actactgaaa 1980
attttttgc tttgtggttg ggactttagc caagggtata aaagaccacc gtccccgaat 2040
tacctttcct cttcttttct ctctctcctt gtcaactcac acccgaaatc gttaagcatt 2100
tccttctgag tataagaatc attcaccatg gacttcctgg aggcagaaga acttgttatg 2160
gaaaagctca agagagagaa gccaagatac tatcaagaca tgtgtcgcaa cttaattaag 2220
atgacgacat ttgcgagctg gacgaggaat agatggagcg tgtgttctga gtcgatgttt 2280
tctatggagt tgtgagtgtt agtagacatg atgggtttat atatgatgaa tgaatagatg 2340
tgattttgat ttgcacgatg gaattgagaa ctttgtaaac gtacatggga atgtatgaat 2400
gtgggggttt tgtgactgga taactgacgg tcagtggacg ccgttgttca aatatccaag 2460
agatgcgaga aactttgggt caagtgaaca tgtcctctct gttcaagtaa accatcaact 2520
atgggtagta tatttagtaa ggacaagagt tgagattctt tggagtccta gaaacgtatt 2580
ttcgcgttcc aagatcaaat tagtagagta atacgggcac gggaatccat tcatagtctc 2640
aattttccca taggtgtgct acaaggtgtt gagatgtggt acagtaccac catgattcga 2700
ggtaaagagc ccagaagtca ttgatgaggt caagaaatac acagatctac agctcaatac 2760
aatgaatatc ttctttcata ttcttcaggt gacaccaagg gtgtctattt tccccagaaa 2820
tgcgtgaaaa ggcgcgtgtg tagcgtggag tatgggttcg gttggcgtat ccttcatata 2880
tcgacgaaat agtagggcaa gagatgacaa aaagtatcta tatgtagaca gcgtagaata 2940
tggatttgat tggtataaat tcatttattg cgtgtctcac aaatactctc gataagttgg 3000
ggttaaactg gagatggaac aatgtcgata tctcgacgca tgcgacgtcg ggcccaattc 3060
gccctatagt gagtcgtatt acaattcact ggccgtcgtt ttacaacgtc gtgactggga 3120
aaaccctggc gttacccaac ttaatcgcct tgcagcacat ccccctttcg ccagctggcg 3180
taatagcgaa gaggcccgca ccgatcgccc ttcccaacag ttgcgcagcc tgaatggcga 3240
atggacgcgc cctgtagcgg cgcattaagc gcggcgggtg tggtggttac gcgcagcgtg 3300
accgctacac ttgccagcgc cctagcgccc gctcctttcg ctttcttccc ttcctttctc 3360
gccacgttcg ccggctttcc ccgtcaagct ctaaatcggg ggctcccttt agggttccga 3420
tttagtgctt tacggcacct cgaccccaaa aaacttgatt agggtgatgg ttcacgtagt 3480
gggccatcgc cctgatagac ggtttttcgc cctttgacgt tggagtccac gttctttaat 3540
agtggactct tgttccaaac tggaacaaca ctcaacccta tctcggtcta ttcttttgat 3600
ttataaggga ttttgccgat ttcggcctat tggttaaaaa atgagctgat ttaacaaaaa 3660
tttaacgcga attttaacaa aatattaacg cttacaattt cctgatgcgg tattttctcc 3720

ttacgcatct gtgcggtatt tcacaccgca tcaggtggca cttttcgggg aaatgtgcgc 3780
ggaacccta tttgtttatt tttctaaata cattcaaata tgtatccgct catgagacaa 3840
taaccctgat aaatgcttca ataatattga aaaaggaaga gtatgagtat tcaacatttc 3900
cgtgtcgccc ttattccctt ttttgcggca ttttgccttc ctgtttttgc tcacccagaa 3960
acgctggtga aagtaaaaga tgctgaagat cagttgggtg cacgagtggg ttacatcgaa 4020
ctggatctca acagcggtaa gatccttgag agttttcgcc ccgaagaacg ttttccaatg 4080
atgagcactt ttaaagttct gctatgtggc gcggtattat cccgtattga cgccgggcaa 4140
gagcaactcg gtcgccgcat acactattct cagaatgact tggttgagta ctcaccagtc 4200
acagaaaagc atcttacgga tggcatgaca gtaagagaat tatgcagtgc tgccataacc 4260
atgagtgata acactgcggc caacttactt ctgacaacga tcggaggacc gaaggagcta 4320
accgcttttt tgcacaacat gggggatcat gtaactcgcc ttgatcgttg ggaaccggag 4380
ctgaatgaag ccataccaaa cgacgagcgt gacaccacga tgcctgtagc aatggcaaca 4440
acgttgcgca aactattaac tggcgaacta cttactctag cttcccggca acaattaata 4500
gactggatgg aggcggataa agttgcagga ccacttctgc gctcggccct tccggctggc 4560
tggtttattg ctgataaatc tggagccggt gagcgtgggt ctcgcggtat cattgcagca 4620
ctggggccag atggtaagcc ctcccgtatc gtagttatct acacgacggg gagtcaggca 4680
actatggatg aacgaaatag acagatcgct gagataggtg cctcactgat taagcattgg 4740
taactgtcag accaagttta ctcatatata ctttagattg atttaaaact tcatttttaa 4800
tttaaaagga tctaggtgaa gatccttttt gataatctca tgaccaaaat cccttaacgt 4860
gagttttcgt tccactgagc gtcagacccc gtagaaaaga tcaaaggatc ttcttgagat 4920
cctttttttc tgcgcgtaat ctgctgcttg caaacaaaaa aaccaccgct accagcggtg 4980
gtttgtttgc cggatcaaga gctaccaact ctttttccga aggtaactgg cttcagcaga 5040
gcgcagatac caaatactgt tcttctagtg tagccgtagt taggccacca cttcaagaac 5100
tctgtagcac cgcctacata cctcgctctg ctaatcctgt taccagtggc tgctgccagt 5160
ggcgataagt cgtgtcttac cgggttggac tcaagacgat agttaccgga taaggcgcag 5220
cggtcgggct gaacgggggg ttcgtgcaca cagcccagct tggagcgaac gacctacacc 5280
gaactgagat acctacagcg tgagctatga gaaagcgcca cgcttcccga agggagaaag 5340
gcggacaggt atccggtaag cggcagggtc ggaacaggag agcgcacgag ggagcttcca 5400
gggggaaacg cctggtatct ttatagtcct gtcgggtttc gccacctctg acttgagcgt 5460
cgatttttgt gatgctcgtc aggggggcgg agcctatgga aaaacgccag caacgcggcc 5520
tttttacggt tcctggcctt ttgctggcct tttgctcaca tgttctttcc tgcgttatcc 5580
cctgattctg tggataaccg tattaccgcc tttgagtgag ctgataccgc tcgccgcagc 5640

cgaacgaccg agcgcagcga gtcagtgagc gaggaagcgg aagagcgccc aatacgcaaa 5700
ccgcctctcc ccgcgcgttg gccgattcat taatgcagct ggcgcgccac tgccgagatc 5760
cagtctacac tgattaattt tcgggccaat aatttaaaaa aatcgtgtta tataatatta 5820
tatgtattat atatatacat catgatgata ctgacagtca tgtcccattg ctaaatagac 5880
agactccatc tgccgcctcc aactgatgtt ctcaatattt aaggggtcat ctcgcattgt 5940
ttaataataa acagactcca tctaccgcct ccaaatgatg ttctcaaaat atattgtatg 6000
aacttatttt tattacttag tattattaga caacttactt gctttatgaa aaacacttcc 6060
tatttaggaa acaatttata atggcagttc gttcatttaa caatttatgt agaataaatg 6120
ttataaatgc gtatgggaaa tcttaaatat ggatagcata aatgatatct gcattgccta 6180
attcgaaatc aacagcaacg aaaaaaatcc cttgtacaac ataaatagtc atcgagaaat 6240
atcaactatc aaagaacagc tattcacacg ttactattga gattattatt ggacgagaat 6300
cacacactca actgtctttc tctcttctag aaatacaggt acaagtatgt actattctca 6360
ttgttcatac ttctagtcat ttcatcccac atattccttg gatttctctc caatgaatga 6420
cattctatct tgcaaattca acaattataa taagatatac caaagtagcg gtatagtggc 6480
aatcaaaaag cttctctggt gtgcttctcg tatttatttt tattctaatg atccattaaa 6540
ggtatatatt tatttcttgt tatataatcc ttttgtttat tacatgggct ggatacataa 6600
aggtattttg atttaatttt ttgcttaaat tcaatccccc ctcgttcagt gtcaactgta 6660
atggtaggaa attaccatac ttttgaagaa gcaaaaaaaa tgaaagaaaa aaaaaatcgt 6720
atttccaggt tagacgttcc gcagaatcta gaatgcggta tgcggtacat tgttcttcga 6780
acgtaaaagt tgcgctccct gagatattgt acatttttgc ttttacaagt acaagtacat 6840
cgtacaacta tgtactactg ttgatgcatc cacaacagtt tgttttgttt tttttttgttt 6900
tttttttttc taatgattca ttaccgctat gtatacctac ttgtacttgt agtaagccgg 6960
gttattggcg ttcaattaat catagactta tgaatctgca cggtgtgcgc tgcgagttac 7020
ttttagctta tgcatgctac ttgggtgtaa tattgggatc tgttcggaaa tcaacggatg 7080
ctcctaggtt cccaaaatcc cagatgcttc tctccagtgc caaaagtaag taccccacag 7140
gttttcggcc gaaaattcca cgtgcagcaa cgtcgtgtgg ggtgttaaaa tgtggggggg 7200
gggaaccagg acaagaggct cttgtgggag ccgaatgaga gcacaaagcg ggcgggtgtg 7260
ataagggcat ttttgcccat tttcccttct cctgtctctc cgacggtgat ggcgttgtgc 7320
gtcctctatt tctttttatt tctttttgtt ttatttctct gactaccgat ttggtttgat 7380
ttcctcaacc ccacacaaat aagctcgggc cgaggaatat atatatacac ggacacagtc 7440
gccctgtgga caacacgtca ctacctctac gatacacacc gtacgatagt tagtagacaa 7500
caatcgatag ttggagcaag ggagaaatgt agagtgtgaa agactcacta tggtccgggc 7560
ttatctcgac caatagccaa agtctggagt ttctgagaga aaaaggcaag atacgtatgt 7620

aacaaagcga cgcatggtac aataataccg gaggcatgta tcatagagag ttagtggttc 7680
gatgatggca ctggtgcctg gtatgacttt atacggctga ctacatattt gtcctcagac 7740
atacaattac agtcaagcac ttacccttgg acatctgtag gtaccccccg gccaagacga 7800
tctcagcgtg tcgtatgtcg gattggcgta gctccctcgc tcgtcaattg gctcccatct 7860
actttcttct gcttggctac acccagcatg tctgctatgg ctcgttttcg tgccttatct 7920
atcctcccag tattaccaac tctaaatgac atgatgtgat tgggtctaca ctttcatatc 7980
agagataagg agtagcacag ttgcataaaa agcccaactc taatcagctt cttcctttct 8040
tgtaattagt acaaaggtga ttagcgaaat ctggaagctt agttggccct aaaaaaatca 8100
aaaaaagcaa aaaacgaaaa acgaaaaacc acagttttga gaacagggag gtaacgaagg 8160
atcgtatata tatatatata tatatatacc cacggatccc gagaccggcc tttgattctt 8220
ccctacaacc aaccattctc accaccctaa ttcacaacca tgtccgttgc atccaagctc 8280
gtcttctacg tccgcgccgc catcgccgtg gtcatctttg ccgcctgtgc cacctacggc 8340
gtgctggcgt ccaccattct caccgccatc ggcaagcagg gcctggccca atggaccgtt 8400
gccagagcct tctactactc ggtgcgcatc ttcctgggta tcagcatcaa gctgcgtagc 8460
cggcaggtga ccggaaccgc cggtctggat gcctccaaga tccaggtcgc caacaccacc 8520
aagcccattg acgacatcac caaacacctg ccccgaccat gcattctgat ttccaaccac 8580
cagaacgaaa tggacattct ggtgctcggt cgcatcttcc cccagtactg ctccgtcacc 8640
gccaaaaagg ccctcaagtg gtaccctctg ctgggccagt tcatggcgct gtccggcacc 8700
atcttcctgg accgaaagga ccgaaccaag tccgtgcaga ccctcggcgg cgccgtcaag 8760
accatccaga gcggcaacgg aggcaagggc cagagcgtct tcatgttccc cgagggaacc 8820
cgatcctact ccaaggacgt cggcatcatg cccttcaaga agggctgttt ccacctggcg 8880
gtccagtcgg gcgctcccat tgtccccgtg gtggtccaga acacctcccg aatgttttct 8940
ttcggccgag gcaagctgga cgccggagag atccttgtcg acgtcctgag ccccattgag 9000
accaagggtc tggacgccag caacgtcgac gctctcatgg ccaccactta taaggccatg 9060
tgcgagactg ccgaccagat tggctacgct ggccagaaga ctcagtaggc ggccgcatga 9120
gaagataaat atataaatac attgagatat taaatgcgct agattagaga gcctcatact 9180
gctcggagag aagccaagac gagtactcaa aggggattac accatccata tccacagaca 9240
caagctgggg aaaggttcta tatacacttt ccggaatacc gtagtttccg atgttatcaa 9300
tgggggcagc caggatttca ggcacttcgg tgtctcgggg tgaaatggcg ttcttggcct 9360
ccatcaagtc gtaccatgtc ttcatttgcc tgtcaaagta aaacagaagc agatgaagaa 9420
tgaacttgaa gtgaaggaat ttaaatgtaa cgaaactgaa atttgaccag atattgtgtc 9480
cgcggtggag ctccagcttt tgttcccttt agtgagggtt aatttcgagc ttggcgtaat 9540

```
catggtcata gctgtttcct gtgtgaaatt gttatccgct cacaagcttc cacacaac        9598
```

## Description

### FIELD OF THE INVENTION

This invention is in the field of biotechnology. More specifically, this invention pertains to an engineered strain of the oleaginous yeast *Yarrowia lipolytica* that is capable of efficiently producing eicosapentaenoic acid, an ω-3 polyunsaturated fatty acid ["PUFA"], in high concentrations.

### BACKGROUND OF THE INVENTION

The clinical and pharmaceutical value of eicosapentaenoic acid ["EPA"; *cis-5,* 8, 11, 14, 17-eicosapentaenoic acid; ω-3] are well known (U.S. Pat. Appl. Pub. No. 2009-0093543-A1). Similarly, the advantages of producing EPA in microbes using recombinant means, as opposed to producing EPA from natural microbial sources or via isolation from fish oil and marine plankton, are also well recognized. Ibanez Gonzalez et al. Journal of the American Oil Chemists' Society, 78(3) 277-285 (2001) discuss purification of EPA from fish oil and microalgae. Robles Medina et al. Journal of Biotechnology, 70, 379-391 (1999) discuss triglyceride production using PUFA concentrates from fish and microalgae oils.

Although the literature reports a number of recent examples whereby various portions of the ω-3/ω-6 polyunsaturated fatty acid ["PUFA"] biosynthetic pathway, responsible for EPA production, have been introduced into plants and non-oleaginous yeast, significant efforts by the Applicants' Assignee has focused on the use of the oleaginous yeast, *Yarrowia lipolytica* (U.S. Patent 7,238,482; U.S. Pat. Appl. Pub. No. 2006-0115881-A1; U.S. Pat. Appl. Pub. No. 2009-0093543-A1). Oleaginous yeast are defined as those yeast that are naturally capable of oil synthesis and accumulation, wherein oil accumulation is at least 25% of the cellular dry weight.

More specifically, U.S. Pat. Appl. Pub. No. 2006-0115881-A1 demonstrated production of 9% EPA of total fatty acids in a recombinant *Yarrowia lipolytica* strain without co-synthesis of γ-linolenic acid ["GLA"; ω-6], by expression of the following genes: Δ9 elongase, Δ8 desaturase, Δ5 desaturase, Δ17 desaturase, Δ12 desaturase and $C_{16/18}$ elongase.

U.S. Pat. Appl. Pub. No. 2009-0093543-A1 describes optimized recombinant *Yarrowia lipolytica* strains for EPA production and demonstrated production of up to 55.6% EPA of total fatty acids in a recombinant *Y. lipolytica* strain by expression of the following genes: Δ9 elongase, Δ8 desaturase, Δ5 desaturase, Δ17 desaturase, Δ12 desaturase, $C_{16/18}$ elongase and diacylglycerol cholinephosphotransferase, within a host cell comprising a disruption in the native peroxisome biogenesis factor 10 protein (PEX10).

Despite the disclosures cited above, strain improvements are necessary for commercial production of EPA that will permit production of high EPA as a weight percent of the total fatty acids <u>in addition to</u> high total lipid content, while minimizing production of intermediate fatty acids, such as linoleic acid ["LA"; ω-6], and byproduct fatty acids in the final oil product. Applicants have solved the stated problem by engineering improved optimized strains of *Yarrowia lipolytica,* wherein the improvement enables at least one of the following: production of 61.8% EPA in the total oil fraction, production of 39.6% total fatty acids as a percent of the dry cell weight, or production of lipids having an EPA to LA ratio of 6.1.

### SUMMARY OF THE INVENTION

In a first embodiment, the invention concerns an extracted unconcentrated oil:

(a) comprising at least 50 weight percent of eicosapentaenoic acid measured as a weight percent of total fatty acids; and,
(b) having a ratio of at least 3.1 of eicosapentaenoic acid, measured as a weight percent of total fatty acids, to linoleic acid, measured as a weight percent of total fatty acids.

Preferably, the oil is microbial.

In a second embodiment, the invention concerns an extracted oil of the invention wherein said oil is extracted from fermented recombinant *Yarrowia* sp. cells engineered for production of eicosapentaenoic acid, wherein said cells comprise:

a) at least at least one multizyme which comprises a polypeptide having at least one Δ9 elongase linked to at least one Δ8 desaturase;
(b) at least one peroxisome biogenesis factor protein whose expression has been down-regulated; and,
(c) at least one recombinant construct comprising a nucleotide sequence encoding an enzyme selected from the group consisting of malonyl CoA synthetase and acyl-CoA lysophospholipid acyltransferase.

Preferably, the malonyl CoA synthetase consists essentially of a sequence selected from the group consisting of SEQ ID NO:40 and SEQ ID NO:42.

Preferably, the acyl-CoA lysophospholipid acyltransferase consists essentially of a sequence selected from the group consisting of SEQ ID NO:15, SEQ ID NO:17, SEQ ID NO:25, SEQ ID NO:29, SEQ ID NO:31 and SEQ ID NO:32.

Preferably, the multizyme linker is selected from the group consisting of: SEQ ID NO:1, SEQ ID NO:2, SEQ ID NO:3, SEQ ID NO:4, SEQ ID NO:5, SEQ ID NO:6 and SEQ ID NO:7.

Preferably, the multizyme consists essentially of a sequence selected from the group consisting of: SEQ ID NO:9, SEQ ID NO:11 and SEQ ID NO:13.

In a third embodiment, the invention concerns oil of the invention wherein said oil is extracted from a recombinant *Yarrowia* sp. host cell using a process selected from the group consisting of: extraction with organic solvents, sonication and supercritical fluid extraction.

The oil of the invention can be used to blend with other oils, optionally with additional fatty acids, and in food or feeds, pharmaceutical products and dietary supplements..

BIOLOGICAL DEPOSITS

The following biological materials have been deposited with the American Type Culture Collection (ATCC), 10801 University Boulevard, Manassas, VA 20110-2209, and bear the following designations, accession numbers and dates of deposit.

| Biological Material | Accession No. | Date of Deposit |
|---|---|---|
| *Yarrowia lipolytica* Y8406 | ATCC PTA-10025 | May 14, 2009 |
| *Yarrowia lipolytica* Y8412 | ATCC PTA-10026 | May 14, 2009 |
| *Yarrowia lipolytica* Y8259 | ATCC PTA-10027 | May 14, 2009 |

The biological materials listed above were deposited under the terms of the Budapest Treaty on the International Recognition of the Deposit of Microorganisms for the Purposes of Patent Procedure. The listed deposit will be maintained in the indicated international depository for at least 30 years and will be made available to the public upon the grant of a patent disclosing it. The availability of a deposit does not constitute a license to practice the subject invention in derogation of patent rights granted by government action.

BRIEF DESCRIPTION OF THE DRAWINGS AND

SEQUENCE DESCRIPTIONS

FIG. 1A and FIG. 1B illustrate the ω-3/ω-6 fatty acid biosynthetic pathway, and should be viewed together when considering the description of this pathway below.

FIG. 2 diagrams the development of *Yarrowia lipolytica* strains Y9481, Y9497 and Y9502, producing greater than 60.9% EPA in the total lipid fraction.

FIG. 3 provides a plasmid map for pY116.

FIG. 4 provides plasmid maps for the following: (A) pZKSL-5S5A5; and, (B) pZP3-Pa777U.

FIG. 5 provides plasmid maps for the following: (A) pZKUM; and, (B) pZKL2-5mB89C.

FIG. 6 provides plasmid maps for the following: (A) pZKL1-2SR9G85; and, (B) pZSCP-Ma83.

FIG. 7 provides plasmid maps for the following: (A) pZKL4-398F2; and, (B) pZP2-85m98F.

FIG. 8 provides plasmid maps for the following: (A) pZK16-MLBN; and, (B) pZK16-ML.

FIG. 9 diagrams the development of *Yarrowia lipolytica* strain Y8672, producing greater than 61.8% EPA in the total lipid fraction.

FIG. 10 provides plasmid maps for the following: (A) pZKL2-5m89C; and, (B) pY201, comprising a chimeric YAT1::ScAle1S::Lip1 gene.

FIG. 11 provides plasmid maps for the following: (A) pY168, comprising a chimeric YAT1::YlAle1::Lip1 gene; and, (B) pY208, comprising a chimeric YAT1::MaLPAAT1S::Lip1 gene.

FIG. 12 provides plasmid maps for the following: (A) pY207, comprising a chimeric YAT1::YlLPAAT1::Lip1 gene; and, (B) pY175, comprising a chimeric YAT1::CeLPCATS::Lip1 gene.

FIG. 13 provides a comparison of EPA % TFAs, LA % TFAs and the ratio of EPA % TFAs to LA % TFAs in each of the strains described in the Examples.

FIG. 14 provides plasmid maps for the following: (A) pY222, comprising a chimeric YAT1::ScLPAATS::Lip1 gene; and, (B) pY177, comprising a chimeric YAT1::YlLPAAT1::Lip1 gene.

The invention can be more fully understood from the following detailed description and the accompanying sequence descriptions, which form a part of this application.

The following sequences comply with 37 C.F.R. §1.821-1.825 ("requirements for Patent Applications Containing Nucleotide Sequences and/or Amino Acid Sequence Disclosures - the Sequence Rules") and are consistent with World Intellectual Property Organization (WIPO) Standard ST.25 (1998) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. § 1.822.

SEQ ID NOs:1-156 are ORFs encoding promoters, genes or proteins (or fragments thereof) or plasmids, as identified in Table 1.

Table 1: Summary of Gene and Protein SEQ ID Numbers

| Description | Nucleic acid SEQ ID NO. | Protein SEQ ID NO. |
|---|---|---|
| Multizyme linker GAGPARPAGLPPATYYDSLAVMGS | -- | 1 |
| Multizyme linker GPARPAGLPPATYYDSLAV | -- | 2 |
| Multizyme linker PARPAGLPPATYYDSLAV | -- | 3 |
| Multizyme linker PTRPAGPPPATYYDSLAV | -- | 4 |
| Multizyme linker PGGPGKPSEIASLPPPIRPVGNPPAAYYDALAT | -- | 5 |
| Multizyme linker PARPAGLPPATYYDSLAVSGRT | -- | 6 |
| Multizyme linker PGGPGKPSEIASLPPPIRPVGNPPAAYYDALATGR T | -- | 7 |
| DGLA synthase, comprising EgD9eS/EgD8M gene fusion | 8 (2112 bp) | 9 (703 AA) |
| DGLA synthase, comprising EaD9eS/EaD8S gene fusion | 10 (2109 bp) | 11 (702 AA) |
| DGLA synthase, comprising E389D9eS/EgD8M gene fusion | 12 (2127 bp) | 13 (708 AA) |
| *Saccharomyces cerevisiae* Ale1 ("ScAle1"; also ORF "YOR175C") | 14 (1860 bp) | 15 (619 AA) |
| *Yarrowia lipolytica* Ale 1 ("YlAle1") | 16 (1539 bp) | 17 (512 AA) |
| membrane bound O-acyltransferase motif M(V/I)LxxKL | -- | 18 |
| membrane bound O-acyltransferase motif RxKYYxxW | -- | 19 |
| membrane bound O-acyltransferase motif SAxWHG | -- | 20 |
| U.S. Pat. Appl. Pub. No. 2008-0145867-A1 motif $EX_{11}WNX_2$-[T/V]-$X_2W$ | -- | 21 |
| Synthetic Ale1 derived from *Saccharomyces cerevisiae,* codon-optimized for expression in *Yarrowia lipolytica* ("ScAle1S") | 22 (1870 bp) | 23 (619 AA) |
| *Caenorhabditis elegans* LPCAT ("CeLPCAT") | 24 (849 bp) | 25 (282 AA) |
| Synthetic LPCAT derived from *Caenorhabditis elegans,* codon-optimized for expression in *Yarrowia lipolytica* ("CeLPCATS") | 26 (859 bp) | 27 (282 AA) |
| *Mortierella alpina* LPAAT1 ("MaLPAAT1") | 28 (945 bp) | 29 (314 AA) |

(continued)

| Description | Nucleic acid SEQ ID NO. | Protein SEQ ID NO. |
|---|---|---|
| *Yarrowia lipolytica* LPAAT1 ("YlLPAAT1") | 30 (1549 bp) | 31 (282 AA) |
| *Saccharomyces cerevisiae* LPAAT ("ScLPAAT"; also ORF "YDL052C") | - | 32 (303 AA) |
| 1-acyl-sn-glycerol-3-phosphate acyltransferase motif NHxxxxD | -- | 33 |
| 1-acyl-sn-glycerol-3-phosphate acyltransferase motif EGTR | -- | 34 |
| Synthetic LPAAT1 derived from *Mortierella alpina,* codon-optimized for expression in *Yarrowia lipolytica* ("MaLPAAT1S") | 35 (955 bp) | 36 (314 AA) |
| *Yarrowia lipolytica* diacylglycerol cholinephosphotransferase gene ("YlCPT1") | 37 (1185 bp) | 38 (394 AA) |
| *Rhizobium leguminosarum* bv. viciae 3841 malonyl-CoA synthetase (GenBank Accession No. YP 766603) ("rMCS") | 39 (1515 bp) | 40 (504 AA) |
| Synthetic malonyl-CoA synthetase derived from *Rhizobium leguminosarum* bv. viciae 3841 (GenBank Accession No. YP_766603), codon-optimized for expression in *Yarrowia lipolytica* ("MCS") | 41 (1518 bp) | 42 (505 AA) |
| *Euglena gracilis* Δ9 elongase ("EgD9e") | 43 (777 bp) | 44 (258 AA) |
| Synthetic Δ9 elongase derived from *Euglena gracilis,* codon-optimized for expression in *Yarrowia lipolytica* ("EgD9eS") | 45 (777 bp) | 46 (258 AA) |
| *Eutreptiella sp.* CCMP389 Δ9 elongase ("E389D9e") | 47 (792 bp) | 48 (263 AA) |
| Synthetic Δ9 elongase derived from *Eutreptiella* sp. CCMP389 codon-optimized for expression in *Yarrowia lipolytica* ("E389D9eS") | 49 (792 bp) | 50 (263 AA) |
| *Euglena anabaena* UTEX 373 Δ9 elongase ("EaD9Elo1") | 51 (774 bp) | 52 (258 AA) |
| Synthetic Δ9 elongase derived from *Euglena anabaena* UTEX 373, codon-optimized for expression in *Yarrowia lipolytica* ("EaD9eS") | 53 (774 bp) | 54 (258 AA) |
| *Euglena gracilis* Δ8 desaturase ("Eg5" or "EgD8") | 55 (1271 bp) | 56 (421 AA) |
| Synthetic Δ8 desaturase derived from *Euglena gracilis,* codon-optimized for expression in *Yarrowia lipolytica* ("D8SF" or "EgD8S") | 57 (1272 bp) | 58 (422 AA) |
| Synthetic mutant Δ8 desaturase ("EgD8M"), derived from *Euglena gracilis* ("EgD8S") | 59 (1272 bp) | 60 (422 AA) |
| *Euglena anabaena* UTEX 373 Δ8 desaturase ("EaD8es3") | 61 (1260 bp) | 62 (420 AA) |
| Synthetic Δ8 desaturase derived from *Euglena anabaena* UTEX 373, codon-optimized for expression in *Yarrowia lipolytica* ("EaD8S") | 63 (1260 bp) | 64 (420 AA) |
| *Euglena gracilis* Δ5 desaturase ("EgD5") | 65 (1350 bp) | 66 (449 AA) |
| Synthetic Δ5 desaturase derived from *Euglena gracilis,* codon-optimized for expression in *Yarrowia lipolytica* ("FgD5S") | 67 (1350 bp) | 68 (449 AA) |
| Mutant Δ5 desaturase ("EgD5M"), derived from Euglena gracilis ("EgD5") (U.S. Pat. Pub. No. 2010-0075386-A1) | 69 (1350 bp) | 70 (449 AA) |

(continued)

| Description | Nucleic acid SEQ ID NO. | Protein SEQ ID NO. |
|---|---|---|
| Synthetic mutant Δ5 desaturase ("EgD5SM"), derived from *Euglena gracilis* ("EgD5S") (U.S. Pat. Pub. No. 2010-0075386-A1) | 71 (1350 bp) | 72 (449 AA) |
| *Peridinium sp.* CCMP626 Δ5 desaturase ("RD5") | 73 (1392 bp) | 74 (463 AA) |
| Synthetic Δ5 desaturase derived from *Peridinium sp.* CCMP626, codon-optimized for expression in *Yarrowia lipolytica* ("RD5S") | 75 (1392 bp) | 76 (463 AA) |
| *Euglena anabaena* UTEX 373 Δ5 desaturase ("EaD5") | 77 (1362 bp) | 78 (454 AA) |
| Synthetic Δ5 desaturase derived from *Euglena anabaena* UTEX 373, codon-optimized for expression in *Yarrowia lipolytica* ("EaD5S") | 79 (1362 bp) | 80 (454 AA) |
| Synthetic mutant Δ5 desaturase ("EaD5SM"), derived from *Euglena anabaena* ("EaD5S") (U.S. Pat. Pub. No. 2010-0075386-A1) | 81 (1365 bp) | 82 (454 AA) |
| *Phytophthora ramorum* Δ17 desaturase ("PrD17") | 83 (1086 bp) | 84 (361 AA) |
| Synthetic Δ17 desaturase derived from *Phytophthora ramorum,* codon-optimized for expression in *Yarrowia lipolytica* ("PrD17S") | 85 (1086 bp) | 86 (361 AA) |
| *Pythium aphanidermatum* Δ17 desaturase ("PaD17") | 87 (1080 bp) | 88 (359 AA) |
| Synthetic Δ17 desaturase derived from *Pythium aphanidermatum,* codon-optimized for expression in *Yarrowia lipolytica* ("PaD17S") | 89 (1080 bp) | 90 (359 AA) |
| *Fusarium moniliforme* Δ12 desaturase ("FmD12") | 91 (1434 bp) | 92 (477 AA) |
| Synthetic Δ12 desaturase derived from *Fusarium moniliforme,* codon-optimized for expression in *Yarrowia lipolytica* ("FmD12S") | 93 (1434 bp) | 94 (477 AA) |
| *Mortierella alpina* $C_{16/18}$ elongase | 95 (828 bp) | 96 (275 AA) |
| Synthetic $C_{16/18}$ elongase derived from *Mortierella alpina* ELO3, codon-optimized for expression in *Yarrowia lipolytica* ("ME3S") | 97 (828 bp) | 98 (275 AA) |
| Shindou et al. membrane bound O-acyltransferase motif WHGxxxGYxxxF | -- | 99 |
| Shindou et al. membrane bound O-acyltransferase motif YxxxxF | -- | 100 |
| Shindou et al. membrane bound O-acyltransferase motif YxxxYFxxH | -- | 101 |
| U.S. Pat. Appl. Pub. No. 2008-0145867-A1 motif M-[V/I]-[L/I]-xxK-[L/V/I]-xxxxxxDG | -- | 102 |
| U.S. Pat. Appl. Pub. No. 2008-0145867-A1 motif RxKYYxxWxxx-[E/D]-[A/G]xxxxGxG-[F/Y]-xG | -- | 103 |
| U.S. Pat. Appl. Pub. No. 2008-0145867-A1 motif SAxWHGxxPGYxx-[T/F]-F | -- | 104 |
| Lewin, T.W. et al. & Yamashita et al. 1-acyl-*sn*-glycerol-3-phosphate acyltransferase motif GxxFI-[D/R]-R | -- | 105 |
| Lewin, T.W. et al. 1-acyl-*sn*-glycerol-3-phosphate acyltransferase motif [V/I]-[P/X]-[I/V/L]-[I/V]-P-[V/I] | -- | 106 |
| Yamashita et al. 1-acyl-sn-glycerol-3-phosphate acyltransferase motif IVPIVM | -- | 107 |

(continued)

| Description | Nucleic acid SEQ ID NO. | Protein SEQ ID NO. |
|---|---|---|
| *Yarrowia lipolytica* Pex1 p (GenBank Accession No. CAG82178) | -- | 108 (1024 AA) |
| *Yarrowia lipolytica* Pex2p (GenBank Accession No. CAG77647) | -- | 109 (381 AA) |
| *Yarrowia lipolytica* Pex3p (GenBank Accession No. CAG78565) | -- | 110 (431 AA) |
| *Yarrowia lipolytica* Pex3Bp (GenBank Accession No. CAG83356) | -- | 111 (395 AA) |
| *Yarrowia lipolytica* Pex4p (GenBank Accession No. CAG79130) | -- | 112 (153 AA) |
| *Yarrowia lipolytica* Pex5p (GenBank Accession No. CAG78803) | -- | 113 (598 AA) |
| *Yarrowia lipolytica* Pex6p (GenBank Accession No. CAG82306) | -- | 114 (1024 AA) |
| *Yarrowia lipolytica* Pex7p (GenBank Accession No. CAG78389) | -- | 115 (356 AA) |
| *Yarrowia lipolytica* Pex8p (GenBank Accession No. CAG80447) | -- | 116 (671 AA) |
| *Yarrowia lipolytica* Pex10p (GenBank Accession No. CAG81606) | -- | 117 (377 AA) |
| *Yarrowia lipolytica* Pex12p (GenBank Accession No. CAG81532) | -- | 118 (408 AA) |
| *Yarrowia lipolytica* Pex13p (GenBank Accession No. CAG81789) | -- | 119 (412 AA) |
| *Yarrowia lipolytica* Pex14p (GenBank Accession No. CAG79323) | -- | 120 (380 AA) |
| *Yarrowia lipolytica* Pex16p (GenBank Accession No. CAG79622) | -- | 121 (391 AA) |
| *Yarrowia lipolytica* Pex17p (GenBank Accession No. CAG84025) | -- | 122 (225 AA) |
| *Yarrowia lipolytica* Pex19p (GenBank Accession No. AAK84827) | -- | 123 (324 AA) |
| *Yarrowia lipolytica* Pex20p (GenBank Accession No. CAG79226) | -- | 124 (417 AA) |
| *Yarrowia lipolytica* Pex22p (GenBank Accession No. CAG77876) | -- | 125 (195 AA) |
| *Yarrowia lipolytica* Pex26p (GenBank Accession No. NC_006072, antisense translation of nucleotides 117230-118387) | -- | 126 (386 AA) |
| Plasmid pY116 | 127 (8739 bp) | -- |

(continued)

| Description | Nucleic acid SEQ ID NO. | Protein SEQ ID NO. |
|---|---|---|
| Plasmid pZKSL-5S5A5 | 128 (13975 bp) | -- |
| Plasmid pZP3-Pa777U | 129 (13066 bp) | -- |
| Plasmid pZKUM | 130 (4313 bp) | -- |
| Plasmid pZKL2-5mB89C | 131 (15991 bp) | -- |
| Plasmid pZKL1-2SR9G85 | 132 (14554 bp) | -- |
| Plasmid pZSCP-Ma83 | 133 (15119 bp) | -- |
| Plasmid pZKL4-398F2 | 134 (14623 bp) | -- |
| Plasmid pZP2-85m98F | 135 (14619 bp) | -- |
| Plasmid pZK16-ML8N | 136 (15262 bp) | -- |
| Plasmid pZK16-ML | 137 (13075bp) | -- |
| Plasmid pZKL2-5m89C | 138 (15799 bp) | -- |
| Plasmid pY201 | 139 (9641 bp) | -- |
| *Escherichia coli* LoxP recombination site, recognized by a Cre recombinase enzyme | 140 (34 bp) | -- |
| Primer 798 | 141 | -- |
| Primer 799 | 142 | -- |
| Primer 800 | 143 | -- |
| Primer 801 | 144 | -- |
| Plasmid pY168 | 145 (9320 bp) | -- |
| Plasmid pY208 | 146 (8726 bp) | -- |
| Primer 856 | 147 | -- |
| Primer 857 | 148 | -- |
| Plasmid pY207 | 149 (8630 bp) | -- |
| Plasmid pY175 | 150 (8630 bp) | -- |
| Synthetic LPAAT derived from Saccharomyces cerevisiae, codon-optimized for expression in Yarrowia lipolytica ("ScLPAATS") | 151 (926 bp) | 152 (303 AA) |

(continued)

| Description | Nucleic acid SEQ ID NO. | Protein SEQ ID NO. |
|---|---|---|
| Plasmid pY222 | 153 (7891 bp) | -- |
| Primer 869 | 154 | -- |
| Primer 870 | 155 | -- |
| Plasmid pY177 | 156 (9598 bp) | -- |

DETAILED DESCRIPTION OF THE INVENTION

Described herein are production host strains of *Yarrowia lipolytica* that are capable of producing greater than 50% eicosapentaenoic acid ["EPA"; 20:5 ω-3]. Accumulation of this particular polyunsaturated fatty acid ["PUFA"] is accomplished by introduction of a functional ω-3/ω-6 fatty acid biosynthetic pathway comprising proteins with Δ9 elongase, Δ8 desaturase, Δ5 desaturase, Δ17 desaturase, Δ12 desaturase and $C_{16/18}$ elongase activities, which thereby enables production of an EPA oil with minimal γ-linolenic acid ["GLA"]. Thus, this disclosure demonstrates that *Y. lipolytica* can be engineered to enable commercial production of EPA and derivatives thereof. Extracted unconcentrated oils therefrom are claimed.

PUFAs, such as EPA (or derivatives thereof), are used as dietary substitutes, or supplements, particularly infant formulas, for patients undergoing intravenous feeding or for preventing or treating malnutrition. Alternatively, the purified PUFAs (or derivatives thereof) may be incorporated into cooking oils, fats or margarines formulated so that in normal use the recipient would receive the desired amount for dietary supplementation. The PUFAs may also be incorporated into infant formulas, nutritional supplements or other food products and may find use as anti-inflammatory or cholesterol lowering agents. Optionally, the compositions may be used for pharmaceutical use, either human or veterinary.

Supplementation of humans or animals with PUFAs produced by recombinant means can result in increased levels of the added PUFAs, as well as their metabolic progeny. For example, treatment with EPA can result not only in increased levels of EPA, but also downstream products of EPA such as eicosanoids (i.e., prostaglandins, leukotrienes, thromboxanes), docosapentaenoic acid ["DPA"; *cis*-7, 10, 13, 16, 19-docosapentaenoic; 22:5 ω-3] and docosahexaenoic acid ["DHA"; *cis*-4, 7, 10, 13, 16, 19-docosahexaenoic acid; 22:6 ω-3]. Complex regulatory mechanisms can make it desirable to combine various PUFAs, or add different conjugates of PUFAs, in order to prevent, control or overcome such mechanisms to achieve the desired levels of specific PUFAs in an individual.

Alternately, PUFAs, or derivatives thereof, made by the methodology disclosed herein can be utilized in the synthesis of animal and aquaculture feeds, such as dry feeds, semi-moist and wet feeds, since these formulations generally require at least 1-2% of the nutrient composition to be ω-3 and/or ω-6 PUFAs.

In this disclosure, a number of terms and abbreviations are used. The following definitions are provided.

"Open reading frame" is abbreviated as "ORF".

"Polymerase chain reaction" is abbreviated as "PCR".

"American Type Culture Collection" is abbreviated as "ATCC".

"Polyunsaturated fatty acid(s)" is abbreviated as "PUFA(s)".

"Triacylglycerols" are abbreviated as "TAGs".

"Co-enzyme A" is abbreviated as "CoA".

"Total fatty acids" are abbreviated as "TFAs".

"Fatty acid methyl esters" are abbreviated as "FAMEs".

"Dry cell weight" is abbreviated as "DCW".

As used herein the term "invention" or "present invention" is intended to refer to all aspects and embodiments of the invention as described in the claims herein and should not be read so as to be limited to any particular embodiment or aspect.

The term "food product" refers to any food generally suitable for human consumption. Typical food products include, but are not limited to: meat products, cereal products, baked foods, snack foods, dairy products, beverages and the like. The terms "food analog", "functional food", "medical food" and "medical nutritional" are defined as in U.S. Pat. Appl. Pub. No. 2006-0115881-A1.

The term "pharmaceutical" as used herein means a compound or substance which if sold in the United States would be controlled by Section 503 or 505 of the Federal Food, Drug and Cosmetic Act.

The term "infant formula" means a food which is designed exclusively for consumption by the human infant by reason of its simulation of human breast milk. Typical commercial examples of infant formula include, but are not limited to: Similac® and Isomil®.

The term "dietary supplement" refers to a product that: (i) is intended to supplement the diet and thus is not represented for use as a conventional food or as a sole item of a meal or the diet; (ii) contains one or more dietary ingredients (including, e.g., vitamins, minerals, herbs or other botanicals, amino acids, enzymes and glandulars) or their constituents; (iii) is intended to be taken by mouth as a pill, capsule, tablet, or liquid; and, (iv) is labeled as being a dietary supplement.

The term "animal feed" refers to feeds intended exclusively for consumption by animals, including domestic animals such as pets, farm animals, etc. or for animals raised for the production of food, such as for e.g., fish farming. The terms "aquaculture feed", "aquafeed" and "feed nutrient" are as defined in U.S. Pat. Appl. Pub. No. 2006-0115881-A1.

As used herein the term "biomass" refers specifically to spent or used yeast cellular material from the fermentation of a recombinant production host producing EPA in commercially significant amounts, wherein the preferred production host is a recombinant strain of the oleaginous yeast, *Yarrowia lipolytica.* The biomass may be in the form of whole cells, whole cell lysates, homogenized cells, partially hydrolyzed cellular material, and/or partially purified cellular material (e.g., microbially produced oil).

The term "'lipids" refer to any fat-soluble (i.e., lipophilic), naturally-occurring molecule. A general overview of lipids is provided in U.S. Pat. Appl. Pub. No. 2009-0093543-A1 (see Table 2 therein).

The term "glycerophospholipids" refers to a broad class of molecules, having a glycerol core with fatty acids at the sn-1 position and sn-2 position, and a polar head group (e.g., phosphate, choline, ethanolamine, glycerol, inositol, serine, cardiolipin) joined at the sn-3 position via a phosphodiester bond. Glycerophospholipids thus include phosphatidic acid ["PA"], phosphatidylcholines ["PC"], phosphatidylethanolamines ["PE"], phosphatidylglycerols ["PG"], phosphatidylinositols ["PI"], phosphatidylserines ["PS"] and cardiolipins ["CL"]. Glycerophospholipids possess tremendous diversity, not only resulting from variable phosphoyl head groups, but also as a result of differing chain lengths and degrees of saturation of their fatty acids. Generally, saturated and monounsaturated fatty acids are esterified at the *sn*-1 position, while polyunsaturated fatty acids are esterified at the *sn*-2 position.

"Lysophospholipids" are derived from glycerophospholipids, by deacylation of the *sn*-2 position fatty acid. Lysophospholipids include, e.g., lysophosphatidic acid ["LPA"], lysophosphatidylcholine ["LPC"], lysophosphatidyletanolamine ["LPE"], lysophosphatidylserine ["LPS"], lysophosphatidylglycerol ["LPG"] and lysophosphatidylinositol ["LPI"].

The term "oil" refers to a lipid substance that is liquid at 25 °C and usually polyunsaturated. In oleaginous organisms, oil constitutes a major part of the total lipid. "Oil" is composed primarily of triacylglycerols ["TAGs"] but may also contain other neutral lipids, phospholipids and free fatty acids. The fatty acid composition in the oil and the fatty acid composition of the total lipid are generally similar; thus, an increase or decrease in the concentration of PUFAs in the total lipid will correspond with an increase or decrease in the concentration of PUFAs in the oil, and vice versa.

"Neutral lipids" refer to those lipids commonly found in cells in lipid bodies as storage fats and are so called because at cellular pH, the lipids bear no charged groups. Generally, they are completely non-polar with no affinity for water. Neutral lipids generally refer to mono-, di-, and/or triesters of glycerol with fatty acids, also called monoacylglycerol, diacylglycerol or triacylglycerol, respectively, or collectively, acylglycerols. A hydrolysis reaction must occur to release free fatty acids from acylglycerols.

The term "triacylglycerols" ["TAGs"] refers to neutral lipids composed of three fatty acyl residues esterified to a glycerol molecule. TAGs can contain long chain PUFAs and saturated fatty acids, as well as shorter chain saturated and unsaturated fatty acids.

The term "total fatty acids" ["TFAs"] herein refer to the sum of all cellular fatty acids that can be derivitized to fatty acid methyl esters ["FAMEs"] by the base transesterification method (as known in the art) in a given sample, which may be the biomass or oil, for example. Thus, total fatty acids include fatty acids from neutral lipid fractions (including diacylglycerols, monoacylglycerols and TAGs) and from polar lipid fractions (including, e.g.,the PC and the PE fractions) but not free fatty acids.

The term "total lipid content" of cells is a measure of TFAs as a percent of the dry cell weight ["DCW"], athough total lipid content can be approximated as a measure of FAMEs as a percent of the DCW ["FAMEs % DCW"]. Thus, total lipid content ["TFAs % DCW"] is equivalent to, e.g., milligrams of total fatty acids per 100 milligrams of DCW.

The concentration of a fatty acid in the total lipid is expressed herein as a weight percent of TFAs ["% TFAs"], e.g., milligrams of the given fatty acid per 100 milligrams of TFAs. Unless otherwise specifically stated in the disclosure herein, reference to the percent of a given fatty acid with respect to total lipids is equivalent to concentration of the fatty acid as % TFAs (e.g., % EPA of total lipids is equivalent to EPA % TFAs).

In some cases, it is useful to express the content of a given fatty acid(s) in a cell as its weight percent of the dry cell weight ["% DCW"]. Thus, for example, EPA % DCW would be determined according to the following formula: (EPA % TFAs) * (TFAs % DCW)]/100. The content of a given fatty acid(s) in a cell as its weight percent of the dry cell weight ["% DCW"] can be approximated, however, as: (EPA % TFAs) * (FAMEs % DCW)]/100.

The terms "lipid profile" and "lipid composition" are interchangeable and refer to the amount of individual fatty acids contained in a particular lipid fraction, such as in the total lipid or the oil, wherein the amount is expressed as a weight percent of TFAs. The sum of each individual fatty acid present in the mixture should be 100.

The term "extracted oil" refers to an oil that has been separated from other cellular materials, such as the microorganism in which the oil was synthesized. Extracted oils are obtained through a wide variety of methods, the simplest of which involves physical means alone. For example, mechanical crushing using various press configurations (e.g., screw, expeller, piston, bead beaters, etc.) can separate oil from cellular materials. Alternately, oil extraction can occur via treatment with various organic solvents (e.g., hexane), via enzymatic extraction, via osmotic shock, via ultrasonic extraction, via supercritical fluid extraction (e.g., $CO_2$ extraction), via saponification and via combinations of these methods. An extracted oil does not require that it is not necessarily purified or further concentrated. The extracted oils described herein will comprise at least 50 EPA % TFAs.

The term "blended oil" refers to an oil that is obtained by admixing, or blending, the extracted oil described herein with any combination of, or individual, oil to obtain a desired composition. Thus, for example, types of oils from different microbes can be mixed together to obtain a desired PUFA composition. Alternatively, or additionally, the PUFA-containing oils disclosed herein can be blended with fish oil, vegetable oil or a mixture of both to obtain a desired composition.

The term "fatty acids" refers to long chain aliphatic acids (alkanoic acids) of varying chain lengths, from about $C_{12}$ to $C_{22}$, although both longer and shorter chain-length acids are known. The predominant chain lengths are between $C_{16}$ and $C_{22}$. The structure of a fatty acid is represented by a simple notation system of "X:Y", where X is the total number of carbon ["C"] atoms in the particular fatty acid and Y is the number of double bonds. Additional details concerning the differentiation between "saturated fatty acids" versus "unsaturated fatty acids", "monounsaturated fatty acids" versus "polyunsaturated fatty acids" ["PUFAs"], and "omega-6 fatty acids" ["ω-6" or "*n*-6"] versus "omega-3 fatty acids" ["ω-3" or "*n*-3"] are provided in U.S. Patent 7,238,482.

Nomenclature used to describe PUFAs herein is given in Table 2. In the column titled "Shorthand Notation", the omega-reference system is used to indicate the number of carbons, the number of double bonds and the position of the double bond closest to the omega carbon, counting from the omega carbon, which is numbered 1 for this purpose. The remainder of the Table summarizes the common names of ω-3 and ω-6 fatty acids and their precursors, the abbreviations that will be used throughout the specification and the chemical name of each compound.

Table 2: Nomenclature of Polyunsaturated Fatty Acids And Precursors

| Common Name | Abbreviation | Chemical Name | Shorthand Notation |
|---|---|---|---|
| Myristic | -- | tetradecanoic | 14:0 |
| Palmitic | Palmitate | hexadecanoic | 16:0 |
| Palmitoleic | -- | 9-hexadecenoic | 16:1 |
| Stearic | -- | octadecanoic | 18:0 |
| Oleic | -- | *cis*-9-octadecenoic | 18:1 |
| Linoleic | LA | *cis*-9, 12-octadecadienoic | 18:2 ω-6 |
| γ-Linolenic | GLA | *cis-6,* 9, 12-octadecatrienoic | 18:3 ω-6 |
| Eicosadienoic | EDA | *cis*-11, 14-eicosadienoic | 20:2 ω-6 |
| Dihomo-γ-Linolenic | DGLA | *cis*-8, 11, 14-eicosatrienoic | 20:3 ω-6 |
| Arachidonic | ARA | *cis*-5, 8, 11, 14-eicosatetraenoic | 20:4 ω-6 |
| α-Linolenic | ALA | *cis*-9, 12, 15-octadecatrienoic | 18:3 ω-3 |
| Stearidonic | STA | *cis-6,* 9, 12, 15-octadecatetraenoic | 18:4 ω-3 |
| Eicosatrienoic | ETrA | *cis*-11, 14, 17-eicosatrienoic | 20:3 ω-3 |
| Sciadonic | SCI | *cis-5,* 11, 14-eicosatrienoic | 20:3b ω-6 |
| Juniperonic | JUP | *cis-5,* 11, 14, 17-eicosatetraenoic | 20:4b ω-3 |
| Eicosatetraenoic | ETA | *cis-8,* 11, 14, 17-eicosatetraenoic | 20:4 ω-3 |
| Eicosapentaenoic | EPA | *cis-5,* 8, 11, 14, 17-eicosapentaenoic | 20:5 ω-3 |

(continued)

| Common Name | Abbreviation | Chemical Name | Shorthand Notation |
|---|---|---|---|
| Docosatetraenoic | DTA | *cis-7,* 10, 13, 16-docosatetraenoic | 22:4 ω-6 |
| Docosapentaenoic | DPAn-6 | *cis-4,* 7, 10, 13, 16-docosapentaenoic | 22:5 ω-6 |
| Docosapentaenoic | DPA | *cis-7,* 10, 13, 16, 19-docosapentaenoic | 22:5 ω-3 |
| Docosahexaenoic | DHA | *cis*-4, 7, 10, 13, 16, 19-docosahexaenoic | 22:6 ω-3 |

The term "PUFA biosynthetic pathway" refers to a metabolic process that converts oleic acid to ω-6 fatty acids such as LA, EDA, GLA, DGLA, ARA, DTA and DPAn-6 and ω-3 fatty acids such as ALA, STA, ETrA, ETA, EPA, DPA and DHA. This process is well described in the literature (e.g., see U.S. Pat. Appl. Pub. No. 2006-0115881-A1). Briefly, this process involves elongation of the carbon chain through the addition of carbon atoms and desaturation of the molecule through the addition of double bonds, via a series of special elongation and desaturation enzymes termed "PUFA biosynthetic pathway enzymes" that are present in the endoplasmic reticulum membrane. More specifically, "PUFA biosynthetic pathway enzymes" refer to any of the following enzymes (and genes which encode said enzymes) associated with the biosynthesis of a PUFA, including: Δ4 desaturase, Δ5 desaturase, Δ6 desaturase, Δ12 desaturase, Δ15 desaturase, Δ17 desaturase, Δ9 desaturase, Δ8 desaturase, Δ9 elongase, $C_{14/16}$ elongase, $C_{16/18}$ elongase, $C_{18/20}$ elongase and/or $C_{20/22}$ elongase.

The term "Δ9 elongase/Δ8 desaturase pathway" will refer to a PUFA biosynthetic pathway that minimally includes at least one Δ9 elongase and at least one Δ8 desaturase, thereby enabling biosynthesis of DGLA and/or ETA from LA and ALA, respectively, with EDA and/or ETrA as intermediate fatty acids. With expression of other desaturases and elongases, ARA, DTA, DPAn-6, EPA, DPA and DHA may also be synthesized.

The term "desaturase" refers to a polypeptide that can desaturate, i.e., introduce a double bond, in one or more fatty acids to produce a fatty acid or precursor of interest. Despite use of the omega-reference system throughout the specification to refer to specific fatty acids, it is more convenient to indicate the activity of a desaturase by counting from the carboxyl end of the substrate using the delta-system. Of particular interest herein are: Δ8 desaturases, Δ5 desaturases, Δ17 desaturases and Δ12 desaturases. Other useful desaturases can include Δ4 desaturases, Δ6 desaturases, Δ15 desaturases and Δ9 desaturases.

The term "elongase" refers to a polypeptide that can elongate a fatty acid carbon chain to produce an acid 2 carbons longer than the fatty acid substrate that the elongase acts upon. This process of elongation occurs in a multi-step mechanism in association with fatty acid synthase, as described in Intl. App. Pub. No. WO 2005/047480. Examples of reactions catalyzed by elongase systems are the conversion of GLA to DGLA, STA to ETA, ARA to DTA and EPA to DPA. In general, the substrate selectivity of elongases is somewhat broad but segregated by both chain length and the degree and type of unsaturation. For example, a $C_{14/16}$ elongase will utilize a $C_{14}$ substrate (e.g., myristic acid), a $C_{16/18}$ elongase will utilize a $C_{16}$ substrate (e.g., palmitate), a $C_{18/20}$ elongase will utilize a $C_{18}$ substrate (e.g., GLA, STA) and a $C_{20/22}$ elongase [also referred to as a Δ5 elongase or C20 elongase] will utilize a $C_{20}$ substrate (e.g., ARA, EPA). For the purposes herein, two distinct types of $C_{18/20}$ elongases can be defined: a Δ6 elongase will catalyze conversion of GLA and STA to DGLA and ETA, respectively, while a Δ9 elongase is able to catalyze the conversion of LA and ALA to EDA and ETrA, respectively.

The term "multizyme" or "fusion protein" refers to a single polypeptide having at least two independent and separable enzymatic activities, wherein the first enzymatic activity is preferably linked to the second enzymatic activity (U.S. Pat. Appl. Pub. No. 2008-0254191-A1). The "link" or "bond" between the at least two independent and separable enzymatic activities is minimally comprised of a single polypeptide bond, although the link may also be comprised of one amino acid residue, such as proline, or a polypeptide comprising at least one proline amino acid residue. Preferred linkers are selected from the group consisting of: SEQ ID NO:1, SEQ ID NO:2, SEQ ID NO:3, SEQ ID NO:4, SEQ ID NO:5, SEQ ID NO:6 and SEQ ID NO:7.

The term "DGLA synthase" refers to a multizyme, wherein a Δ9 elongase is linked to a Δ8 desaturase. The term "EgD9eS/EgD8M" refers to a DGLA synthase (SEQ ID NOs:8 and 9) created by linking the Δ9 elongase "EgD9eS" (U.S. Patent 7,645,604) to the Δ8 desaturase "EgD8M" (U.S. Patent 7,709,239) with a linker sequence (i.e., SEQ ID NO:1 [GAGPARPAGLPPATYYDSLAVMGS]; U.S. Pat. Appl. Pub. No. 2008-0254191-A1). Similarly, the term "EaD9eS/EaD8S" refers to a DGLA synthase (SEQ ID NOs:10 and 11) created by linking the Δ9 elongase "EaD9eS" (U.S. Pat. Appl. Pub. No. 2008-0254522-A1) to the Δ8 desaturase "EaD8S" (U.S. Pat. Appl. Pub. No. 2008-0254521-A1) with the linker sequence set forth as SEQ ID NO:1. And, the term "E389D9eS/EgD8M" refers to a DGLA synthase (SEQ ID NOs:12 and 13) created by linking the Δ9 elongase "E389D9eS" (U.S. Patent 7,645,604) to the Δ8 desaturase "EgD8M" *(supra)* with the linker sequence set forth as SEQ ID NO:1.

The terms "conversion efficiency" and "percent substrate conversion" refer to the efficiency by which a particular enzyme, such as a desaturase, elongase or multizyme, can convert substrate to product. The conversion efficiency is measured according to the following formula: ([product]/[substrate+product])*100, where 'product' includes the immediate product and all products in the pathway derived from it.

The term "$C_{18}$ to $C_{20}$ elongation conversion efficiency" refers to the efficiency by which $C_{18//20}$ elongases can convert $C_{18}$ substrates (i.e., LA, ALA, GLA, STA) to $C_{20}$ products (i.e., EDA, ETrA, DGLA, ETA). These $C_{18//20}$ elongases can be either Δ9 elongases or Δ6 elongases.

The term "Δ9 elongation conversion efficiency" refers to the efficiency by which Δ9 elongase can convert $C_{18}$ substrates (i.e., LA, ALA) to $C_{20}$ products (i.e., EDA, ETrA).

The term "acyltransferase" refers to an enzyme responsible for transferring an acyl group from a donor lipid to an acceptor lipid molecule.

The term "acyl-CoA:lysophospholipid acyltransferase" ["LPLAT"] refers to a broad class of acyltransferases, having the ability to acylate a variety of lysophospholipid substrates at the sn-2 position. More specifically, LPLATs include LPA acyltransferases ["LPAATs"] having the ability to catalyze conversion of LPA to PA, LPC acyltransferases ["LPCATs"] having the ability to catalyze conversion of LPC to PC, LPE acyltransferases ["LPEATs"] having the ability to catalyze conversion of LPE to PE, LPS acyltransferases ["LPSATs"] having the ability to catalyze conversion of LPS to PS, LPG acyltransferases ["LPGATs"] having the ability to catalyze conversion of LPG to PG, and LPI acyltransferases ["LPIATs"] having the ability to catalyze conversion of LPI to PI. Standardization of LPLAT nomenclature has not been formalized, so various other designations are used in the art (for example, LPAATs have also been referred to as acyl-CoA:1-acyl-sn-glycerol-3-phosphate 2-O-acyltransferases, 1-acyl-sn-glycerol-3-phosphate acyltransferases and/or 1-acylglycerolphosphate acyltransferases ["AGPATs"] and LPCATs are often referred to as acyl-CoA:1-acyl lysophosphatidyl-choline acyltransferases). Additionally, it is important to note that some LPLATs, such as the *Saccharomyces cerevisiae* Ale1 (ORF YOR175C; SEQ ID NO:15), have broad specificity and thus a single enzyme may be capable of catalyzing several LPLAT reactions, including LPAAT, LPCAT and LPEAT reactions (Tamaki, H. et al., J. Biol. Chem., 282:34288-34298 (2007); Ståhl, U. et al., FEBS Letters, 582:305-309 (2008); Chen, Q. et al., FEBS Letters, 581:5511-5516 (2007); Benghezal, M. et al., J. Biol. Chem., 282:30845-30855 (2007); Riekhof, et al., J. Biol. Chem., 282:28344-28352 (2007)).

More specifically, the term "polypeptide having at least lysophosphtidylcholine acyltransferase ["LPCAT"] activity" will refer to those enzymes capable of catalyzing the reaction: acyl-CoA + 1-acyl-*sn*-glycero-3-phosphocholine → CoA + 1,2-diacyl-*sn*-glycero-3-phosphocholine (EC 2.3.1.23). LPCAT activity has been described in two structurally distinct protein families, i.e., the LPAAT protein family (Hishikawa, et al., Proc. Natl. Acad. Sci. U.S.A., 105:2830-2835 (2008); Intl. App. Pub. No. WO 2004/076617) and the ALE1 protein family (Tamaki, H. et al., *supra;* St6hl, U. et al., *supra*; Chen, Q. et al., *supra;* Benghezal, M. et al., *supra*; Riekhof, et al., *supra).*

The term "LPCAT" refers to a protein of the ALE1 protein family that: 1) has LPCAT activity (EC 2.3.1.23) and shares at least about 45% amino acid identity, based on the Clustal W method of alignment, when compared to an amino acid sequence selected from the group consisting of SEQ ID NO:15 (ScAle1) and SEQ ID NO:17 (YlAle1); and/or 2) has LPCAT activity (EC 2.3.1.23) and has at least one membrane bound *O*-acyltransferase ["MBOAT"] protein family motif selected from the group consisting of: M(V/I)LxxKL (SEQ ID NO:18), RxKYYxxW (SEQ ID NO:19), SAxWHG (SEQ ID NO:20) and $EX_{11}WNX_2$-[T/V]-$X_2W$ (SEQ ID NO:21). Examples of ALE1 polypeptides include ScAle1 and YlAle1.

The term "ScAle1" refers to a LPCAT (SEQ ID NO:15) isolated from *Saccharomyces cerevisiae* (ORF "YOR175C"), encoded by the nucleotide sequence set forth as SEQ ID NO:14. In contrast, the term "ScAle1S" refers to a synthetic LPCAT derived from *S. cerevisiae* that is codon-optimized for expression in *Yarrowia lipolytica* (i.e., SEQ ID NOs:22 and 23).

The term "YlAle1" refers to a LPCAT (SEQ ID NO:17) isolated from *Yarrowia lipolytica,* encoded by the nucleotide sequence set forth as SEQ ID NO:16.

The term "LPCAT" also refers to a protein that has LPCAT activity (EC 2.3.1.23) and shares at least about 90% amino acid identity, based on the Clustal W method of alignment, when compared to an amino acid sequence as set forth in SEQ ID NO:25 (CeLPCAT).

The term "CeLPCAT" refers to a LPCAT enzyme (SEQ ID NO:25) isolated from *Caenorhabditis elegans,* encoded by the nucleotide sequence set forth as SEQ ID NO:24. In contrast, the term "CeLPCATS" refers to a synthetic LPCAT derived from *C. elegans* that is codon-optimized for expression in *Yarrowia lipolytica* (i.e., SEQ ID NOs:26 and 27).

The term "polypeptide having at least lysophosphatidic acid acyltransferase ["LPAAT"] activity" will refer to those enzymes capable of catalyzing the reaction: acyl-CoA + 1-acyl-sn-glycerol 3-phosphate → CoA + 1,2-diacyl-*sn*-glycerol 3-phosphate (EC 2.3.1.51).

The term "LPAAT" refers to a protein that: 1) has LPAAT activity and shares at least about 43.9% amino acid identity, based on the Clustal W method of alignment, when compared to an amino acid sequence selected from the group consisting of SEQ ID NO:29 (MaLPAAT1), SEQ ID NO:31 (YlLPAAT1) and SEQ ID NO:32 (ScLPAAT1); and/or, 2) has LPAAT activity and has aat least one 1-acyl-*sn*-glycerol-3-phosphate acyltransferase family motif selected from the group consisting of: NHxxxxD (SEQ ID NO:33) and EGTR (SEQ ID NO:34). Examples of LPAAT polypeptides include ScLPAAT, MaLPAAT1 and YlLPAAT1.

The term "ScLPAAT" refers to a LPAAT (SEQ ID NO:32) isolated from *Saccharomyces cerevisiae* (ORF "YDL052C").

The term "MaLPAAT1" refers to a LPAAT (SEQ ID NO:29) isolated from *Mortierella alpina,* encoded by the nucleotide sequence set forth as SEQ ID NO:28. In contrast, the term "MaLPAAT1S" refers to a synthetic LPAAT derived from *M. alpina* that is codon-optimized for expression in *Yarrowia lipolytica* (i.e., SEQ ID NOs:35 and 36).

The term "YlLPAAT1" refers to a LPAAT (SEQ ID NO:31) isolated from *Yarrowia lipolytica,* encoded by the nucleotide sequence set forth as SEQ ID NO:30.

The term "ortholog" refers to a homologous protein from a different species that evolved from a common ancestor protein as evidenced by being in one clade of phylogenetic tree analysis and that catalyzes the same enzymatic reaction.

The term "diacylglycerol cholinephosphotransferase" refers to an enzyme (EC 2.7.8.2) that catalyses the synthesis of phosphatidylcholines from CDP-choline and 1,2-diacylglycerols. This enzyme is part of the CDP-choline pathway, responsible for phosphatidylcholine ["PC"] biosynthesis.

The term "YlCPT1" refers to a diacylglycerol cholinephosphotransferase enzyme (SEQ ID NO:38) isolated from *Yarrowia lipolytica,* encoded by SEQ ID NO:37. YlCPT1 is described in Intl. App. Pub. No. WO 2006/052870 (see also GenBank Accession No. XM_501703 (YALI0C10989g)).

The term "malonic acid", also referred to as propanedioic acid according to International Union of Pure and Applied Chemistry ["IUPAC"] systematic nomenclature, refers to a dicarboxylic acid having the chemical structure set forth as $CH_2(COOH)_2$. The malonate or propanedioate ion is derived from malonic acid by loss of two hydrogen ions (i.e., $CH_2(COO)_2^{2-}$). Salts and esters of malonic acid include, but are not limited to, diethyl malonate $[(C_2H_5)_2(C_3H_2O_4)]$, dimethyl malonate $[(CH_3)_2(C_3H_2O_4)]$ and disodium malonate $[Na_2(C_3H_2O_4)]$.

"Malonates" refer to the ionised form of malonic acid, as well as its esters and salts. All of these are referred to herein collectively as "malonates".

"Malonyl-CoA" [CAS Registry No. 524-14-1] refers to an acyl thioester that can be formed by the carboxylation of acetyl-CoA to malonyl-CoA. Alternatively, malonyl-CoA is produced enzymatically from the substrate malonate, via a malonyl-CoA synthetase.

"Malonyl-CoA synthetase" [EC 6.2.1.-] catalyzes the following enzymatic reaction: malonate + ATP + CoA $\rightarrow$ malonyl-CoA + AMP + pyrophosphate (PPi). The enzyme was first purified from malonate-grown *Pseudomonas fluorescens* (Kim, Y.S. and S.K. Bang, J. Biol. Chem., 260:5098-5104 (1985)), although various *Rhizobia* homologs have since been isolated from bacteroids within legume nodules (see, for example, Kim, Y.S. and H.Z. Chae, Biochem. J., 273:511-516 (1991) and Kim, Y.S. and S.W. Kang, Biochem. J., 297:327-333 (1994)).

As used herein, the term "rMCS" refers to a gene (SEQ ID NO:39) encoding malonyl-CoA synthetase (SEQ ID NO:40) isolated from *Rhizobium leguminosarum* bv. viciae 3841 (GenBank Accession No. YP_766603). Similarly, the term "MCS" refers to a synthetic gene encoding malonyl-CoA synthetase derived from *Rhizobium leguminosarum* bv. viciae 3841 that is codon-optimized for expression in *Yarrowia lipolytica* (i.e., SEQ ID NOs:41 and 42).

The term "peroxisomes" refers to ubiquitous organelles found in all eukaryotic cells. They have a single lipid bilayer membrane that separates their contents from the cytosol and that contains various membrane proteins essential to the functions described below. Peroxisomes selectively import proteins via an "extended shuttle mechanism". More specifically, there are at least 32 known peroxisomal proteins, called peroxins, which participate in the process of importing proteins by means of ATP hydrolysis through the peroxisomal membrane. Once cellular proteins are imported into the peroxisome, they are typically subjected to some means of degradation. For example, peroxisomes contain oxidative enzymes, such as e.g., catalase, D-amino acid oxidase and uric acid oxidase, that enable degradation of substances that are toxic to the cell. Alternatively, peroxisomes breakdown fatty acid molecules to produce free molecules of acetyl-CoA which are exported back to the cytosol, in a process called $\beta$-oxidation.

The terms "peroxisome biogenesis factor protein", "peroxin" and "Pex protein" are interchangeable and refer to proteins involved in peroxisome biogenesis and/or that participate in the process of importing cellular proteins by means of ATP hydrolysis through the peroxisomal membrane. The acronym of a gene that encodes any of these proteins is "Pex gene". A system for nomenclature is described by Distel et al., J. Cell Biol., 135:1-3 (1996). At least 32 different Pex genes have been identified so far in various eukaryotic organisms. Many Pex genes have been isolated from the analysis of mutants that demonstrated abnormal peroxisomal functions or structures. Based on a review by Kiel, J. A. K. W., et al. (Traffic, 7:1291-1303 (2006)), wherein *in silico* analysis of the genomic sequences of 17 different fungal species was performed, the following Pex proteins were identified: Pex1p, Pex2p, Pex3p, Pex3Bp, Pex4p, Pex5p, Pex5Bp, Pex5Cp, Pex5/20p, Pex6p, Pex7p, Pex8p, Pex10p, Pex12p, Pex13p, Pex14p, Pex15p, Pex16p, Pex17p, Pex14/17p, Pex18p, Pex19p, Pex20p, Pex21 p, Pex21 Bp, Pex22p, Pex22p-like and Pex26p. Collectively, these proteins will be referred to herein as "Pex proteins", encoded by "Pex genes".

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential in the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

The term "down-regulated" in or in connection with at least one peroxisome biogenesis factor protein refers to reduction in, or abolishment of, the activity of a native peroxisome biogenesis factor protein, as compared to the activity of the wildtype protein. Down-regulation typically occurs when a native Pex gene has a "disruption", referring to an insertion, deletion, or targeted mutation within a portion of that gene, that results in either a complete gene knockout such that the gene is deleted from the genome and no protein is translated or a translated Pex protein having an insertion, deletion, amino acid substitution or other targeted mutation. The location of the disruption in the protein may be, for example, within the N-terminal portion of the protein or within the C-terminal portion of the protein. The disrupted Pex protein will have impaired activity with respect to the Pex protein that was not disrupted, and can be non-functional. Down-regulation that results in low or lack of expression of the Pex protein, could also result via manipulating the regulatory sequences, transcription and translation factors and/or signal transduction pathways or by use of sense, antisense or RNAi technology, etc.

The term "oleaginous" refers to those organisms that tend to store their energy source in the form of oil (Weete, In: Fungal Lipid Biochemistry, 2nd Ed., Plenum, 1980). The term "oleaginous yeast" refers to those microorganisms classified as yeasts that can make oil. Generally, the cellular oil content of oleaginous microorganisms follows a sigmoid curve, wherein the concentration of lipid increases until it reaches a maximum at the late logarithmic or early stationary growth phase and then gradually decreases during the late stationary and death phases (Yongmanitchai and Ward, Appl. Environ. Microbiol., 57:419-25 (1991)). It is not uncommon for oleaginous microorganisms to accumulate in excess of about 25% of their dry cell weight as oil. Examples of oleaginous yeast include, but are no means limited to, the following genera: *Yarrowia, Candida, Rhodotorula, Rhodosporidium, Cryptococcus, Trichosporon* and *Lipomyces.*

The term "fermentable carbon source" means a carbon source that a microorganism will metabolize to derive energy. Typical carbon sources include, but are not limited to: monosaccharides, oligosaccharides, polysaccharides, alkanes, fatty acids, esters of fatty acids, monoglycerides, diglycerides, triglycerides, carbon dioxide, methanol, formaldehyde, formate and carbon-containing amines.

The terms "polynucleotide", "polynucleotide sequence", "nucleic acid sequence", "nucleic acid fragment" and "isolated nucleic acid fragment" are used interchangeably herein. These terms encompass nucleotide sequences and the like. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof. Nucleotides (usually found in their 5'-monophosphate form) are referred to by a single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

A "substantial portion" of an amino acid or nucleotide sequence is that portion comprising enough of the amino acid sequence of a polypeptide or the nucleotide sequence of a gene to putatively identify that polypeptide or gene, either by manual evaluation of the sequence by one skilled in the art, or by computer-automated sequence comparison and identification using algorithms such as BLAST (Basic Local Alignment Search Tool; Altschul, S. F., et al., J. Mol. Biol., 215:403-410 (1993)). In general, a sequence of ten or more contiguous amino acids or thirty or more nucleotides is necessary in order to identify putatively a polypeptide or nucleic acid sequence as homologous to a known protein or gene. Moreover, with respect to nucleotide sequences, gene-specific oligonucleotide probes comprising 20-30 contiguous nucleotides may be used in sequence-dependent methods of gene identification (e.g., Southern hybridization) and isolation, such as *in situ* hybridization of bacterial colonies or bacteriophage plaques. In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to specifically identify and/or isolate a nucleic acid fragment comprising the sequence.

The term "complementary" is used to describe the relationship between nucleotide bases that are capable of hybridizing to one another. For example, with respect to DNA, adenosine is complementary to thymine and cytosine is complementary to guanine.

"Codon degeneracy" refers to the nature in the genetic code permitting variation of the nucleotide sequence without effecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

"Synthetic genes" can be assembled from oligonucleotide building blocks that are chemically synthesized using procedures known to those skilled in the art. These oligonucleotide building blocks are annealed and then ligated to form gene segments that are then enzymatically assembled to construct the entire gene. Accordingly, the genes can be tailored for optimal gene expression based on optimization of nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell, where sequence information is available. For example, the codon usage profile for *Yarrowia lipolytica* is provided in U.S. Patent 7,125,672.

"Gene" refers to a nucleic acid fragment that expresses a specific protein, and which may refer to the coding region alone or may include regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene that is introduced into the host organism by gene transfer. Foreign genes can comprise native genes inserted into a non-native organism, native genes introduced into a new location within the native host, or chimeric genes. A "transgene" is a gene that has been introduced into the genome by a transformation procedure. A "codon-optimized gene" is a gene having its frequency of codon usage designed to mimic the frequency of preferred codon usage of the host cell.

"Coding sequence" refers to a DNA sequence that codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, enhancers, silencers, 5' untranslated leader sequence (e.g., between the transcription start site and the translation initiation codon), introns, polyadenylation recognition sequences, RNA processing sites, effector binding sites and stem-loop structures.

"Promoter" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental or physiological conditions. Promoters that cause a gene to be expressed in most cell types at most times are commonly referred to as "constitutive promoters". It is further recognized that since in most cases the exact boundaries of regulatory sequences have not been completely defined, DNA fragments of different lengths may have identical promoter activity.

The terms "3' non-coding sequence" and "transcription terminator" refer to DNA sequences located downstream of a coding sequence. This includes polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The 3' region can influence the transcription, RNA processing or stability, or translation of the associated coding sequence.

The term "operably linked" refers to the association of nucleic acid sequences on a single nucleic acid fragment so that the function of one is affected by the other. For example, a promoter is operably linked with a coding sequence when it is capable of affecting the expression of that coding sequence. That is, the coding sequence is under the transcriptional control of the promoter. Coding sequences can be operably linked to regulatory sequences in sense or antisense orientation.

The term "expression", as used herein, refers to the transcription and stable accumulation of sense (mRNA) or antisense RNA. Expression may also refer to translation of mRNA into a polypeptide.

"Transformation" refers to the transfer of a nucleic acid molecule into a host organism. The nucleic acid molecule may be a plasmid that replicates autonomously, for example, or, it may integrate into the genome of the host organism. Host organisms containing the transformed nucleic acid fragments are referred to as "transgenic" or "recombinant" or "transformed" organisms or "transformant".

"Stable transformation" refers to the transfer of a nucleic acid fragment into the genome of a host organism, including both nuclear and organellar genomes, resulting in genetically stable inheritance (i.e., the nucleic acid fragment is "stably integrated"). In contrast, "transient transformation" refers to the transfer of a nucleic acid fragment into the nucleus, or DNA-containing organelle, of a host organism resulting in gene expression without integration or stable inheritance.

The terms "plasmid" and "vector" refer to an extra chromosomal element often carrying genes that are not part of the central metabolism of the cell, and usually in the form of circular double-stranded DNA fragments. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction that is capable of introducing an expression cassette(s) into a cell.

The term "expression cassette" refers to a fragment of DNA comprising the coding sequence of a selected gene and regulatory sequences preceding (5' non-coding sequences) and following (3' non-coding sequences) the coding sequence that are required for expression of the selected gene product. Thus, an expression cassette is typically composed of: 1) a promoter sequence; 2) a coding sequence (i.e., ORF) and, 3) a 3' untranslated region (i.e., a terminator) that, in eukaryotes, usually contains a polyadenylation site. The expression cassette(s) is usually included within a vector, to facilitate cloning and transformation. Different expression cassettes can be transformed into different organisms including bacteria, yeast, plants and mammalian cells, as long as the correct regulatory sequences are used for each host.

The term "sequence analysis software" refers to any computer algorithm or software program that is useful for the analysis of nucleotide or amino acid sequences. "Sequence analysis software" may be commercially available or independently developed. Typical sequence analysis software will include, but is not limited to: 1) the GCG suite of programs (Wisconsin Package Version 9.0, Genetics Computer Group (GCG), Madison, WI); 2) BLASTP, BLASTN, BLASTX (Altschul et al., J. Mol. Biol., 215:403-410 (1990)); 3) DNASTAR (DNASTAR, Inc. Madison, WI); 4) Sequencher (Gene Codes Corporation, Ann Arbor, MI); and, 5) the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Plenum: New York, NY). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

Standard recombinant DNA and molecular cloning techniques used herein are well known in the art and are described by Sambrook, J., Fritsch, E. F. and Maniatis, T., Molecular Cloning: A Laboratory Manual, 2nd ed., Cold Spring Harbor Laboratory: Cold Spring Harbor, NY (1989) (hereinafter "Maniatis"); by Silhavy, T. J., Bennan, M. L. and Enquist, L. W., Experiments with Gene Fusions, Cold Spring Harbor Laboratory: Cold Spring Harbor, NY (1984); and by Ausubel, F. M. et al., Current Protocols in Molecular Biology, published by Greene Publishing Assoc. and Wiley-Interscience, Hoboken, NJ (1987).

In general, lipid accumulation in oleaginous microorganisms is triggered in response to the overall carbon to nitrogen ratio present in the growth medium. This process, leading to the *de novo* synthesis of free palmitate (16:0) in oleaginous microorganisms, is described in detail in U.S. Patent 7,238,482. Palmitate is the precursor of longer-chain saturated and unsaturated fatty acid derivates, which are formed through the action of elongases and desaturases (FIG. 1).

A wide spectrum of fatty acids (including saturated and unsaturated fatty acids and short-chain and long-chain fatty acids) can be incorporated into TAGs, the primary storage unit for fatty acids. In the methods and host cells described herein, incorporation of EPA into TAGs is most desirable, although the structural form of the EPA is not limiting (thus, for example, the EPA may exist in the total lipids as free fatty acids or in esterified forms such as acylglycerols, phospholipids, sulfolipids or glycolipids).

Although most PUFAs are incorporated into TAGs as neutral lipids and are stored in lipid bodies, it is important to note that a measurement of the total PUFAs within an oleaginous organism should minimally include those PUFAs that are located in the PC, PE and TAG fractions.

The metabolic process wherein oleic acid is converted to EPA involves elongation of the carbon chain through the addition of carbon atoms and desaturation of the molecule through the addition of double bonds. This requires a series of special desaturation and elongation enzymes present in the endoplasmic reticulum membrane. However, as seen in FIG. 1 and as described below, multiple alternate pathways exist for EPA production.

Specifically, FIG. 1 depicts the pathways described below. All pathways require the initial conversion of oleic acid to linoleic acid ["LA"], the first of the ω-6 fatty acids, by a Δ12 desaturase. Then, using the "Δ9 elongase/Δ8 desaturase pathway" and LA as substrate, long-chain ω-6 fatty acids are formed as follows: 1) LA is converted to eicosadienoic acid ["EDA"] by a Δ9 elongase; 2) EDA is converted to dihomo-γ-linolenic acid ["DGLA"] by a Δ8 desaturase; 3) DGLA is converted to arachidonic acid ["ARA"] by a Δ5 desaturase; 4) ARA is converted to docosatetraenoic acid ["DTA"] by a $C_{20/22}$ elongase; and, 5) DTA is converted to docosapentaenoic acid ["DPAn-6"] by a Δ4 desaturase.

The "Δ9 elongase/ Δ8 desaturase pathway" can also use α-linolenic acid ["ALA"] as substrate to produce long-chain ω-3 fatty acids as follows: 1) LA is converted to ALA, the first of the ω-3 fatty acids, by a Δ15 desaturase; 2) ALA is converted to eicosatrienoic acid ["ETrA"] by a Δ9 elongase; 3) ETrA is converted to eicosatetraenoic acid ["ETA"] by a Δ8 desaturase; 4) ETA is converted to eicosapentaenoic acid ["EPA"] by a Δ5 desaturase; 5) EPA is converted to docosapentaenoic acid ["DPA"] by a $C_{20/22}$ elongase; and, 6) DPA is converted to docosahexaenoic acid ["DHA"] by a Δ4 desaturase. Optionally, ω-6 fatty acids may be converted to ω-3 fatty acids. For example, ETA and EPA are produced from DGLA and ARA, respectively, by Δ17 desaturase activity. Advantageously for the purposes herein, the Δ9 elongase/Δ8 desaturase pathway enables production of an EPA oil that lacks significant amounts of γ-linolenic acid ["GLA"].

Alternate pathways for the biosynthesis of ω-3/ω-6 fatty acids utilize a Δ6 desaturase and $C_{18/20}$ elongase, that is, the "Δ6 desaturase/ Δ6 elongase pathway". More specifically, LA and ALA may be converted to to GLA and stearidonic acid ["STA"], respectively, by a Δ6 desaturase; then, a $C_{18/20}$ elongase converts GLA to DGLA and/or STA to ETA.

Economical commercial production of EPA in a recombinant *Yarrowia* sp. host cell requires consideration of a variety of variables, including the EPA concentration ["EPA % TFAs"] and total lipid content ["TFAs % DCW"]. Furthermore, it is desirable to reduce the production of intermediate fatty acids and byproduct fatty acids in the final oil product, in order to maximize production of the desired fatty acid, i.e., EPA.

Intermediate fatty acids are those fatty acids (e.g., oleic acid, LA, ALA, EDA, DGLA, ETA) that can be further converted to EPA by the action of other metabolic pathway enzymes. In contrast, by-product fatty acids (e.g., sciadonic acid, juniperonic acid) refer to any fatty acid produced that is neither EPA nor an intermediate fatty acid that can be further converted to EPA.

U.S. Pat. Appl. Pub. No. 2009-0093543-A1 describes optimized strains of recombinant *Yarrowia lipolytica* having the ability to produce microbial oils comprising at least about 43.3 EPA % TFAs, with less than about 23.6 LA % TFAs (an EPA:LA ratio of 1.83). The preferred strain was Y4305, whose maximum production was 55.6 EPA % TFAs, with an EPA:LA ratio of 3.03. Generally, the EPA strains of U.S. Pat. Appl. Pub. No. 2009-0093543-A1 comprised the following genes of the ω-3/ω-6 fatty acid biosynthetic pathway:

a) at least one gene encoding Δ9 elongase; and,
b) at least one gene encoding Δ8 desaturase; and,
c) at least one gene encoding Δ5 desaturase; and,
d) at least one gene encoding Δ17 desaturase; and,
e) at least one gene encoding Δ12 desaturase; and,
f) at least one gene encoding $C_{16/18}$ elongase; and,
g) optionally, at least one gene encoding diacylglycerol cholinephosphotransferase (CPT1).

Examples of preferred genes having the enzymatic functionalities described above are set forth in Table 3 (although these genes are not intended to be limiting).

Table 3: Preferred Desaturases And Elonaases For EPA Biosynthesis In *Yarrowia lipolytica*

| ORF | Organism | Co-pending Patent Application References | Wildtype Abbreviation and SEQ ID NO | Codon-Optimized Abbreviation and SEQ ID NO | Mutant Abbreviation and SEQ ID NO |
|---|---|---|---|---|---|
| Δ9 elongase | *Euglena gracillis* | U.S. Patent 7,645,604 | "EgD9e" (SEQ ID NOs:43 and 44) | "EgD9eS" (SEQ ID NOs:45 and 46) | -- |
| | *Eutreptiella sp.* CCMP389 | U.S. Patent 7,645,604 | "E389D9e" (SEQ ID NOs:47 and 48) | "E389D9eS" (SEQ ID NOs:49 and 50) | -- |
| | *Euglena anabaena* UTEX 373 | U.S. Pat. Appl. Pub. No. 2008-0254522-A1; Intl. App. Pub. No. WO 2008/128241 | "EaD9e" * (SEQ ID NOs:51 and 52) | "EaD9eS" (SEQ ID NOs:53 and 54) | -- |
| Δ8 desaturase | *Euglena gracilis* | U.S. Patent 7,256,033; U.S. Patent 7,709,239 | "EgD8" * (SEQ ID NOs:55 and 56) | "EgD8S" * (SEQ ID NOs:57 and 58) | "EgD8M" * (SEQ ID NOs:59 and 60) |
| | *Euglena anabaena* UTEX 373 | U.S. Pat. Appl. Pub. No. 2008-0254521-A1; Intl. App. Pub. No. WO 2008/124194 | "EaD8" * (SEQ ID NOs:61 and 62) | "EaD8S" (SEQ ID NOs:63 and 64) | -- |
| Δ5 desaturase | *Euglena gracilis* | U.S. Patent 7,678,560; U.S. Pat. Pub. No. 2010-0075386-A1 | "EgD5" (SEQ ID NOs:65 and 66) | "EgD5S" (SEQ ID NOs:67 and 68) | "EgD5M" (SEQ ID NOs:69 and 70); "EgD5SM" (SEQ ID NOs:71 and 72) |
| | *Peridinium sp.* CCMP626 | U.S. Patent 7,695,950; U.S. Pat. Pub. No. 2010-0075386-A1 | "RD5" (SEQ ID NOs:73 and 74) | "RD5S" (SEQ ID NOs:75 and 76) | -- |
| | *Euglena anabaena* UTEX 373 | U.S. Pat. Appl. Pub. No. 2008-0274521-A1; U.S. Pat. Pub. No. 2010-0075386-A1 | "EaD5" * (SEQ ID NOs:77 and 78) | "EaD5S" * (SEQ ID NOs:79 and 80) | "EaD5SM" (SEQ ID NOs:81 and 82) |
| Δ17 desaturase | *Phytophthora ramorum* | U.S. Patent 7,465,793 | "PrD17" (SEQ ID NOs:83 and 84) | "PrD17S" (SEQ ID NOs:85 and 86) | -- |
| | *Pythium aphanidernatum* | U.S. Patent 7,556,949 | "PaD17" (SEQ ID NOs:87 and 88) | "PaD17S" (SEQ ID NOs:89 and 90) | -- |
| Δ12 desaturase | *Fusarium moniliforme* | U.S. Patent 7,504,259 | "FmD12" * (SEQ ID NOs:91 and 92) | "FmD12S" (SEQ ID NOs:93 and 94) | -- |
| $C_{16/18}$ elongase | *Mortierella alpina* | U.S. Patent 7,470,532 | "ELO3" (SEQ ID NOs:95 . and 96) | "ME3S" (SEQ ID NOs:97 and 98) | -- |

(continued)

| ORF | Organism | Co-pending Patent Application References | Wildtype Abbreviation and SEQ ID NO | Codon-Optimized Abbreviation and SEQ ID NO | Mutant Abbreviation and SEQ ID NO |
|---|---|---|---|---|---|
| Diacylglycerol choline-phosphotransferase | *Yarrowia lipolytica* | Intl. App. Pub. No. WO 2006/052870 | "YICPT" (SEQ ID NOs:37 and 38) | -- | -- |

*Notes: EaD9e was identified as "EaD9Elo1" in U.S. Pat. Appl. Pub. No. 2008-0254522-A1; EgD8 was identified as "Eg5" in U.S. Patent 7,256,033; EgD8S was identified as "D8SF" in U.S. Patent 7,256,033; EgD8M was identified as "EgD8S-23" in U.S. Patent 7,709,239; EaD8 was identified as "EaD8Des3" in U.S. Pat. Appl. Pub. No. 2008-0254521-A1; EaD5 was identified as "EaD5Des1" in U.S. Pat. Appl. Pub. No. 2008-0274521-A1; and, FmD12 was identified as "Fm2" in U.S. Patent 7,504,259.

Disclosed herein are optimized strains of recombinant *Yarrowia lipolytica* having the ability to produce improved microbial oils relative to those strains described in U.S. Pat. Appl. Pub. No. 2009-0093543-A1, based on the EPA % TFAs and the ratio of EPA:LA. In addition to expressing genes of the ω-3/ω-6 fatty acid biosynthetic pathway as defined above and as detailed in U.S. Pat. Appl. Pub. No. 2009-0093543-A1, these improved strains are distinguished by:

1) comprising at least one multizyme, wherein said multizyme comprises a polypeptide having at least one fatty acid Δ9 elongase linked to at least one fatty acid Δ8 desaturase [a "DGLA synthase"];
2) optionally comprising at least one polynucleotide encoding an enzyme selected from the group consisting of a malonyl CoA synthetase or an acyl-CoA lysophospholipid acyltransferase ["LPLAT"];
3) comprising at least one peroxisome biogenesis factor protein whose expression has been down-regulated;
4) producing at least about 50 EPA % TFAs; and,
5) having a ratio of EPA:LA of at least about 3.1.

U.S. Pat. Appl. Pub. No. 2008-0254191-A1, and especially Examples 55 and 56 describes DGLA synthases that possess improved enzymatic activity with respect to their individual Δ9 elongase and/or Δ8 desaturase counterparts, when heterologously expressed in *Yarrowia lipolytica.* Particularly relevant to the disclosure herein, a linker sequence (i.e., SEQ ID NO:1 [GAGPARPAGLPPATYYDSLAVMGS]) was used to fuse a Δ9 elongase (i.e., EgD9eS, EaD9eS or E389D9eS) to a Δ8 desaturase (i.e., EgD8M or EaD8S), thereby creating EgD9eS/EgD8M (SEQ ID NOs:8 and 9), EaD9eS/EaD8S (SEQ ID NOs:10 and 11) and E389D9eS/EgD8M (SEQ ID NOs:12 and 13). Surprisingly, fusing the two independent enzymes together as one fusion protein separated by a linker region increased flux from LA to DGLA, suggesting that the product of Δ9 elongase may be directly channeled as substrate of Δ8 desaturase in the fusion protein.

Table 4 below provides a summary of the improvements noted in conversion efficiency in U.S. Pat. Appl. Pub. No. 2008-0254191-A1, as a result of the gene fusion. Specifically, the number shown in bold text is the percent improvement in elongase or desaturase activity, while the details shown in parentheses provide the elongase or desaturase conversion efficiency in the gene fusion versus when the elongase or desaturase conversion efficiency when the gene was expressed alone.

Table 4: Improvement In Δ9 Elongase And Δ8 Desaturase Conversion As A Result Of Gene Fusion

| Gene fusion | Δ9 Improvement | Δ8 Improvement |
|---|---|---|
| EgD9eS/EgD8M (SEQ ID NOs:8 and 9) | **5%**<br>(21% versus 20% conversion) | **97%**<br>(73% versus 37% conversion) |
| EaD9eS/EaD8S (SEQ ID NOs:10 and 11) | **38%**<br>(18% versus 13% conversion) | **32%**<br>(58% versus 41% conversion) |
| E389D6eS/EgD8M (SEQ ID NOs:12 and 13) | **50%**<br>(18% versus 12% conversion) | **89%**<br>(70% versus 37% conversion) |

Based on the results described above, expression of at least one DGLA synthase, such as the EgD9eS/EgD8M, EaD9eS/EaD8S and E389D9eS/EgD8M gene fusions described above, is preferred in improved optimized strains of recombinant *Yarrowia lipolytica* having the ability to produce improved EPA % TFAs. This gene fusion can be created using any combination of preferred Δ9 elongases and Δ8 desaturases suitable for expression in *Y. lipolytica;* and, the linker can be selected from the group consisting of: SEQ ID NO:1, SEQ ID NO:2, SEQ ID NO:3, SEQ ID NO:4, SEQ ID NO:5, SEQ ID NO:6 and SEQ ID NO:7.

Previous studies have determined that many of the genetic mutations relating to engineering production of PUFAs in *Yarrowia lipolytica* result in increased byproduction of malonates during the fermentation (malonates accounted for ~45% of the total organic acids accumulated). Expression of a heterologous malonyl-CoA synthetase reversed this effect and resulted in substantially reduced byproduction of malonates.

More specifically, U.S. Pat. Appl. Pub. No. 2010-0159558-A1 describes generalized methods to avoid accumulation of organic acid (and in particular, malonate) "byproducts" that cannot be further utilized during a fermentation, during production of a product. This avoids carbon and energy waste within the organism, reduces the amount of base required to maintain an optimal pH range during the fermentation process, and reduces the amount of byproduct organic acids that require neutralization within the fermentation waste steam.

Malonyl-CoA synthetase [EC 6.2.1.-] catalyzes the following enzymatic reaction: malonate + ATP + CoA → malonyl-CoA + AMP + pyrophosphate (PPi). By converting the byproduct (i.e., malonate) into malonyl-CoA, this substrate becomes available for use during the synthesis of fatty acids within the organism. Specifically, fatty acid synthesis can be summarized by the following equation (ignoring $H^+$ and water): acetyl-CoA + 7 malonyl-CoA + 14 NADPH → palmitate

+ 7 $CO_2$ + 14 $NADP^+$ + 8 CoA.

A codon-optimized malonyl-CoA synthetase was created and expressed in *Yarrowia lipolytica* in U.S. Pat. Appl. Pub. No. 2010-0159558-A1. Specifically, the codon-optimized malonyl-CoA synthetase gene ("MCS", SEQ ID NO:41) was designed based on the coding sequence of the malonyl-CoA synthetase gene from *Rhizobium leguminosarum* bv. viciae 3841 (rMCS; SEQ ID NOs:39 and 40, corresponding to GenBank Accession No. YP_766603). In addition to modification of the translation initiation site, 233 bp of the 1515 bp coding region (including the stop codon) were modified (15.4%), 219 codons were optimized (43.4%), the GC content was reduced from 61.4% within the wild type gene (i.e., rMCS) to 55.6% within the synthetic gene (i.e., MCS) and the translation initiation codon "ATG" was added in front of the rMCS gene (SEQ ID NO:39) since *Yarrowia* cannot use the "GTG" codon for translation initiation. The codon-optimized MCS gene (SEQ ID NO:41) is 1518 bp encoding a peptide of 505 amino acids and a stop codon (SEQ ID NO:42).

Expression of MCS (SEQ ID NO:42) in *Yarrowia lipolytica* strain Y4305U, producing 49 EPA % TFAs, lowered the total amount of malonates (g/g DCW) ~94% without impacting either the fatty acid profile or the total lipid yield (TFAs % DCW).

Based on the results described above, expression of at least one malonyl-CoA synthetase in improved optimized strains of recombinant *Yarrowia lipolytica* is desirable, as a means to reduce generation of unwanted byproducts and thereby decrease the cost of manufacture. Preferred malonyl-CoA synthetases are set forth as SEQ ID NOs:40 and 42, but these are should not be limiting to the disclosure herein. One skilled in the art could readily identify alternate heterologous malonyl-CoA synthetases suitable for expression in *Y. lipolytica.*

Glycerophospholipids, the main component of biological membranes, contain a glycerol core with fatty acids attached as R groups at the *sn*-1 position and *sn*-2 position, and a polar head group joined at the *sn*-3 position via a phosphodiester bond. Table 5 below summarizes the steps in the *de novo* biosynthetic pathway, originally described by Kennedy and Weiss (J. Biol. Chem., 222:193-214 (1956)):

Table 5: General Reactions Of *de Novo* Glycerophospholipid Biosynthesis

| | |
|---|---|
| *sn*-Glycerol-3-Phosphate → Lysophosphatidic Acid (1-acyl-*sn*-glycerol 3-phosphate or "LPA") | Glycerol-3-phosphate acyltransferase (GPAT) [E.C. 2.3.1.15] esterifies 1st acyl-CoA to *sn*-1 position of *sn*-glycerol 3-phosphate |
| LPA → Phosphatidic Acid (1,2-diacylglycerol phosphate or "PA") | Lysophosphatidic acid acyltransferase (LPAAT) [E.C. 2.3.1.51] esterifies 2$^{nd}$ acyl-CoA to *sn-2* position of LPA |
| PA → 1,2-Diacylglycerol ("DAG")<br>**Or**<br>PA → Cytidine Diphosphate Diacylglycerol ("CDP-DG") | Phosphatidic acid phosphatase [E.C. 3.1.3.4] removes a phosphate from PA; DAG can subsequently be converted to phosphatidylcholines ["PC"], phosphatidylethanolamines ["PE"] or TAG (TAG synthesis requires either a diacylglycerol acyltransferase (DGAT) [E.C. 2.3.1.20] or a phospholipid:diacylglycerol acyltransferase (PDAT) [E.C.2.3.1.158])<br>CDP-diacylglycerol synthase [EC 2.7.7.41] causes condensation of PA and cytidine triphosphate, with elimination of pyrophosphate; CDP-DG can subsequently be converted to phosphatidylglycerols ["PG"], phosphatidylinositols ["PI"], phosphatidylserines ["PS"] or cardiolipins ["CL"] |

Following their *de novo* synthesis, glycerophospholipids can undergo rapid turnover of their fatty acyl composition at the sn-2 position. This "remodeling", or "acyl editing", has been attributed to deacylation of the glycerophospholipid and subsequent reacylation of the resulting lysophospholipid.

In the Lands' cycle (Lands, W.E., J. Biol. Chem., 231:883-888 (1958)), remodeling occurs through the concerted action of: 1) a phospholipase, such as phospholipase $A_2$, that releases fatty acids from the sn-2 position of phosphatidylcholine; and, 2) acyl-CoA:lysophospholipid acyltransferases ["LPLATs"], such as lysophosphatidylcholine acyltransferase ["LPCAT"] that reacylates the lysophosphatidylcholine ["LPC"] at the *sn*-2 position. Other glycerophospholipids can also be involved in the remodeling with their respective lysophospholipid acyltransferase activity, including LPLAT enzymes having lysophosphatidylethanolamine acyltransferase ["LPEAT"] activity, lysophosphatidylserine acyltransferase ["LPSAT"] activity, lysophosphatidylglycerol acyltransferase ["LPGAT"] activity and lysophosphatidylinositol acyltransferase ["LPIAT"] activity. In all cases, LPLATs are responsible for removing acyl-CoA fatty acids from the cellular acyl-CoA pool and acylating various lysophospholipid substrates at the *sn*-2 position in the phospholipid pool. Finally, LPLATs also include LPAAT enzymes that are involved in the *de novo* biosynthesis of PA from LPA.

In other cases, this *sn*-2 position remodeling has been attributed to the forward and reverse reactions of enzymes having LPCAT activity (Stymne S. and A.K. Stobart, Biochem. J., 223(2):305-314 (1984)).

Several recent reviews by Shindou et al. provide an overview of glycerophospholipid biosynthesis and the role of LPLATs (J. Biol. Chem., 284(1):1-5 (2009); J. Lipid Res., 50:S46-S51 (2009)). And, numerous LPLATs have been reported in public and patent literature, based on the presence of conserved motifs.

More specifically, a variety of LPLAT motifs have been proposed, with slight variation based on the specific species that are included in analyzed alignments. For example, Shindou et al. (Biochem. Biophys. Res. Comm., 383:320-325 (2009)) proposed the following membrane bound *O*-acyltransferase ["MBOAT"] family motifs to be important for LPLAT activity, based on alignment of sequences from *Homo sapiens*, *Gallus gallus, Danio rerio* and *Caenorhabditis elegans:* WD, WHGxxxGYxxxF (SEQ ID NO:99), YxxxxF (SEQ ID NO:100) and YxxxYFxxH (SEQ ID NO:101). Of these, the WD, WHGxxxGYxxxF and YxxxxF motifs are present in ScAle (SEQ ID NO:15) and YlAle1 (SEQ ID NO:17), but the YxxxYFxxH motif is not. Alternate non-plant motifs for Ale1 homologs are also described in U.S. Pat. Appl. Pub. No. 2008-0145867-A1; specifically, these include: M-[V/I]-[L/I]-xxK-[L/V/I]-xxxxxxDG (SEQ ID NO:102), RxKYYxx-Wxxx-[E/D]-[A/G]xxxxGxG-[F/Y]-xG (SEQ ID NO:103), $EX_{11}WNX_2$-[T/V]-$X_2$W (SEQ ID NO:21) and SAxWHGxxPGYxx-[T/F]-F (SEQ ID NO:104).

Similarly, Lewin, T.W. et al. (Biochemistry, 38:5764-5771 (1999) and Yamashita et al. (Biochim. Biophys. Acta, 1771:1202-1215 (2007)) proposed the following 1-acyl-sn-glycerol-3-phosphate acyltransferase ["LPAAT"] family motifs to be important for LPLAT activity, based on alignment of sequences from bacteria, yeast, nematodes and mammals: NHxxxxD (SEQ ID NO:33), GxxFI-[D/R]-R (SEQ ID NO:105), EGTR (SEQ ID NO:34) and either [V/I]-[P/X]-[I/V/L]-[I/V]-P-[V/I] (SEQ ID NO:106) or IVPIVM (SEQ ID NO:107). The NHxxxxD and EGTR motifs are present in MaLPAAT1 (SEQ ID NO:29), YlLPAAT1 (SEQ ID NO:31) and CeLPCAT (SEQ ID NO:25), but the other LPAAT family motifs are not.

Based on publicly available Ale1, LPCAT and LPAAT protein sequences, including those described herein, LPLATs for inclusion in the improved optimized strains of recombinant *Yarrowia lipolytica* hereinpossess either MBOAT family motifs selected from the group consisting of: M(V/I)LxxKL (SEQ ID NO:18), RxKYYxxW (SEQ ID NO:19), SAxWHG (SEQ ID NO:20) and $EX_{11}WNX_2$-[T/V]-$X_2$W (SEQ ID NO:21) or 1-acyl-*sn*-glycerol-3-phosphate acyltransferase family motifs selected from the group consisting of: NHxxxxD (SEQ ID NO:33) and EGTR (SEQ ID NO:34).

The effect of LPLATs on PUFA production has been contemplated, since fatty acid biosynthesis requires rapid exchange of acyl groups between the acyl-CoA pool and the phospholipid pool. Specifically, desaturations occur mainly at the sn-2 position of phospholipids, while elongation occurs in the acyl-CoA pool.

More specifically, it has been previously hypothesized that LPCATs could be important in the accumulation of EPA in the TAG fraction of *Yarrowia lipolytica* (U.S. Pat. Appl. Pub. No. 2006-0115881-A1). As described therein, this hypothesis was based on the following studies: 1) Stymne S. and A.K. Stobart (Biochem. J., 223(2):305-314(1984)), who hypothesized that the exchange between the acyl-CoA pool and PC pool may be attributed to the forward and backward reaction of LPCAT; 2) Domergue, F. et al. (J. Bio. Chem., 278:35115 (2003)), who suggested that accumulation of GLA at the sn-2 position of PC and the inability to efficiently synthesize ARA in yeast was a result of the elongation step involved in PUFA biosynthesis occurring within the acyl-CoA pool, while the Δ5 and Δ6 desaturation steps occurred predominantly at the *sn-2* position of PC; 3) Abbadi, A. et al. (The Plant Cell, 16:2734-2748 (2004)), who suggested that LPCAT plays a criticial role in the successful reconstitution of a Δ6 desaturase/Δ6 elongase pathway, based on analysis on the constraints of PUFA accumulation in transgenic oilseed plants; and, 4) the work of Renz, A. et al. in Intl. App. Publications No. WO 2004/076617 A2 and No. WO 2004/087902 A2.

More specifically, Intl. App. Pub. No. WO 2004/076617 A2 describes the isolation of a LPCAT from *Caenorhabditis elegans* (clone T06E8.1) ["CeLPCAT"] and reports increase in the efficiency of Δ6 desaturation and Δ6 elongation, as well as an increase in biosynthesis of the long-chain PUFAs eicosadienoic acid ["EDA"; 20:2] and eicosatetraenoic acid ["ETA"; 20:4], respectively, when the LPCAT was expressed in an engineered strain of *Saccharomyces cerevisiae* that was fed exogenous 18:2 or α-linolenic ["ALA"; 18:3] fatty acids, respectively.

Intl. App. Pub. No. WO 2004/087902 A2 (Example 16) describes the isolation of *Mortierella alpina* LPAAT-like proteins (encoded by proteins having 417 amino acids in length or 389 amino acids in length, respectively, that are identical except for an N-terminal extension of 28 amino acid residues) and reports expression of one of these proteins using similar methods to those of Intl. App. Pub. No. WO 2004/076617 A2, which results in similar improvements in EDA and ETA biosynthesis.

Both Intl. App. Publications No. WO 2004/076617 and No. WO 2004/087902 teach that the improvement in EDA and ETA biosynthesis is due to reversible LPCAT activity in CeLPLAT and some LPAAT-like proteins, although not all LPAAT-like proteins have LPCAT activity. Furthermore, Renz, A. et al. concluded that LPCAT allowed efficient and continuous exchange of the newly synthesized fatty acids between phospholipids and the acyl-CoA pool, since desaturases catalyze the introduction of double bonds in PC-coupled fatty acids while elongases exclusively catalyze the elongation of CoA esterified fatty acids (acyl-CoAs).

Numerous other references generally describe benefits of co-expressing LPLATs with PUFA biosynthetic genes, to increase the amount of a desired fatty acid in the oil of a transgenic organism, increase total oil content or selectively increase the content of desired fatty acids (e.g., Intl. App. Publications No. WO 2006/069936, No. WO 2006/052870, No. WO 2009/001315, No. WO 2009/014140).

Herein (and in WO 2010/147900), it is demonstrated that LPAAT and LPCAT are indeed important in the accumulation of EPA in the TAG fraction of *Yarrowia lipolytica.* Specifically, it was found that overexpression of LPLATs can result in an improvement in the Δ9 elongase conversion efficiency. As previously defined, conversion efficiency is a term that refers to the efficiency by which a particular enzyme, such as a Δ9 elongase, can convert substrate (e.g., LA) to product (e.g., EDA). Thus, in a strain engineered to produce EPA, improvement in Δ9 elongase conversion efficiency was demonstrated to result in increased EPA % TFAs and/or EPA % DCW.

These results, and additional supporting work, are the cornerstone of the following claimed method for improving $C_{18}$ to $C_{20}$ elongation conversion efficiency in a LC-PUFA-producing recombinant oleaginous microbial host cell, wherein said method comprises:

a) introducing into said LC-PUFA-producing recombinant host cell at least one isolated polynucleotide encoding a polypeptide having at least acyl-CoA:lysophospholipid acyltransferase activity wherein the polypeptide is selected from the group consisting of:

(i) a polypeptide having at least 45% amino acid identity, based on the Clustal W method of alignment, when compared to an amino acid sequence selected from the group consisting of SEQ ID NO:15 (ScAle1) and SEQ ID NO:17 (YlAle1);

(ii) a polypeptide having at least one membrane bound *O*-acyltransferase protein family motif selected from the group consisting of: M(V/I)LxxKL (SEQ ID NO:18), RxKYYxxW (SEQ ID NO:19), SAxWHG (SEQ ID NO:20) and $EX_{11}WNX_2$-[T/V]-$X_2W$ (SEQ ID NO:21);

(iii) a polypeptide having at least 90% amino acid identity, based on the Clustal W method of alignment, when compared to an amino acid sequence as set forth in SEQ ID NO:25 (CeLPCAT);

(iv) a polypeptide having at least 43.9% amino acid identity, based on the Clustal W method of alignment, when compared to an amino acid sequence selected from the group consisting of SEQ ID NO:29 (MaLPAAT1), SEQ ID NO:31 (YlLPAAT1) and SEQ ID NO:32 (ScLPAAT1); and,

(v) a polypeptide having at least one 1-acyl-sn-glycerol-3-phosphate acyltransferase protein family motif selected from the group consisting of: NHxxxxD (SEQ ID NO:33) and EGTR (SEQ ID NO:34);

wherein the at least one isolated polynucleotide encoding a polypeptide having at least acylCoA:lysophospholipid acyltransferase activity is operably linked to at least one regulatory sequence, said regulatory sequence being the same or different; and,

b) growing the oleaginous microbial host cell;

wherein the $C_{18}$ to $C_{20}$ elongation conversion efficiency of the oleaginous microbial host cell is increased relative to the control host cell.

Preferably, the polynucleotide encoding a polypeptide having at least acyl-CoA:lysophospholipid acyltransferase activity is stably integrated and the an increase in $C_{18}$ to $C_{20}$ elongation conversion is at least about 4%,

More preferred, the increase in $C_{18}$ to $C_{20}$ elongation conversion efficiency is at least about 4-10%, more preferred at least about 10-20%, more preferred at least about 20-40%, and most preferred at least about 40-60% or greater in at least one LC-PUFA-producing oleaginous microbial host cell when compared to the control host cell.

Based on the improvement in $C_{18}$ to $C_{20}$ elongation conversion efficiency described above, optimized strains of recombinant *Yarrowia lipolytica* having the ability to produce improved EPA % TFAs, relative to those strains described in U.S. Pat. Appl. Pub. No. 2009-0093543-A1, will optionally comprise at least one acyl-CoA lysophospholipid acyltransferase ["LPLAT"] as defined in the methods described above. In preferred embodiments, the amino acid sequence of the LPLAT is selected from the group consisting of: SEQ ID NO:15 (ScAle1), SEQ ID NO:16 (YlAle1), SEQ ID NO:25 (CeLPCAT), SEQ ID NO:29 (MaLPAAT1), SEQ ID NO:31 (YlLPAAT1) and SEQ ID NO:32 (ScLPAAT1).

U.S. Pat. Appl. Pub. No. 2009-0093543-A1 describes a variety of knockouts useful in recombinant *Yarrowia* sp., including those useful for selection of transformants, those that diminish fatty acid degradation and TAG degradation and those that appear to result in a phenotypically "neutral" mutation (wherein the *Yarrowia* host cell seems unaffected). Most preferred, however, are those gene knockouts (e.g., diacylglycerol acyltransferase gene knockouts, peroxisome biogenesis factor protein ["PEX"] gene knockouts) that result in increases in the concentration of EPA relative to the total fatty acids ["EPA % TFAs"].

More specifically, U.S. Pat. Appl. Pub. No. 2009-0093543-A1 contemplates that in some preferred recombinant *Yarrowia* production hosts, the host is devoid of a native gene encoding a peroxisome biogenesis factor protein selected from the group consisting of: Pex1p (SEQ ID NO:108), Pex2p (SEQ ID NO:109), Pex3p (SEQ ID NO:110), Pex3Bp (SEQ ID NO:111), Pex4p (SEQ ID NO:112), Pex5p (SEQ ID NO:113), Pex6p (SEQ ID NO:114), Pex7p (SEQ ID NO:115), Pex8p (SEQ ID NO:116), Pex10p (SEQ ID NO:117), Pex12p (SEQ ID NO:118), Pex13p (SEQ ID NO:119), Pex14p (SEQ ID NO:120), Pex16p (SEQ ID NO:121), Pex17p (SEQ ID NO:122), Pex19p (SEQ ID NO:123), Pex20p (SEQ ID NO:124), Pex22p (SEQ ID NO:125) and Pex26p (SEQ ID NO:126). More preferred disrupted peroxisome biogenesis factor proteins are Pex2p, Pex3p, Pex10p, Pex12p and Pex16p, although data is provided only concerning Pex10p.

Intl. App. Pub. No. WO 2009/046248 confirms and expands the hypotheses and studies presented in U.S. Pat. Appl. Pub. No. 2009-0093543-A1, by comparing ΔPex16, ΔPex3 and ΔPex10 strains of *Yarrowia lipolytica.* Results therein demonstrated that Pex10 disruption was responsible for a 3.3 fold increase in EPA % TFAs and a 1.7 fold increase in the amount of $C_{20}$ PUFAs relative to the non-disrupted strain engineered for EPA production. Similarly, a 1.65 fold increase in DGLA % TFAs and a 1.3 fold increase in $C_{20}$ PUFAs % TFAs was observed in a ΔPex16 strain engineered for DGLA production. A 2.0 fold increase in DGLA % TFAs and a 1.7 fold increase in $C_{20}$ PUFAs % TFAs was observed in a ΔPex3 strain engineered for DGLA production.

These results, and additional supporting work, are the cornerstone of the following claimed method for increasing the weight percent of one PUFA or a combination of PUFAs relative to the weight percent of total fatty acids in an oleaginous eukaryotic organism having a total lipid content, a total lipid fraction and an oil fraction, said method comprising:

a) providing an oleaginous eukaryotic organism comprising a disruption in a native gene encoding a peroxisome biogenesis factor protein, which creates a PEX-disruption organism; and genes encoding a functional PUFA biosynthetic pathway; and,

b) growing the PEX-disrupted organism under conditions wherein the weight percent of at least one PUFA is increased in the total lipid fraction and in the oil fraction relative to the weight percent of the total fatty acids, when compared with those weight percents in an oleaginous eukaryotic organism whose native peroxisome biogenesis factor protein has not been disrupted.

The amount of PUFAs that increases as a percent of total fatty acids can be: 1) the PUFA that is the desired end product of a functional PUFA biosynthetic pathway, as opposed to PUFA intermediates or by-products; 2) $C_{20}$ to $C_{22}$ PUFAs; and/or, 3) total PUFAs.

In addition to the increase in the weight percent of one or a combination of PUFAs relative to the weight percent of the total fatty acids, in some cases, the total lipid content (TFA % DCW) of the cell may be increased or decreased. What this means is that regardless of whether the disruption in the PEX gene causes the amount of total lipids in the PEX-disrupted cell to increase or decrease, the disruption always causes the weight percent of a PUFA or of a combination of PUFAs to increase.

Based on the above, optimized strains of recombinant *Yarrowia lipolytica* having the ability to produce improved EPA % TFAs, relative to those strains described in U.S. Pat. Appl. Pub. No. 2009-0093543-A1, will comprise at least one peroxisome biogenesis factor protein whose expression has been down-regulated (i.e., thereby producing a PEX-disrupted organism). In preferred strains, the down-regulated peroxisome biogenesis factor protein is Pex3p (SEQ ID NO:110 Pex10p (SEQ ID NO:117), or Pex16p (SEQ ID NO:121).

Although numerous techniques are available to one of skill in the art to achieve disruption of a native *Yarrowia* gene, generally the endogenous activity of a particular gene can be reduced or eliminated by the following techniques, for example: 1) disrupting the gene through insertion, substitution and/or deletion of all or part of the target gene; or, 2) manipulating the regulatory sequences controlling the expression of the protein. Both of these techniques are discussed in U.S. Pat. Appl. Pub. No. 2009-0093543-A1, as well as Intl. App. Pub. No. WO 2009/046248. One skilled in the art would appreciate that these and other methods are well described in the existing literature and are not limiting to the methods, host cells, and products described herein. One skilled in the art will also appreciate the most appropriate technique for use with any particular oleaginous yeast.

The optimized strains will produce at least about 40-50 EPA % TFAs, preferably at least about 50-55 EPA % TFAs, more preferably at least about 55-60 EPA % TFAs, more preferably at least 60-70 EPA % TFAs, and most preferably at least about 70-80 EPA % TFAs.

As will be clear to one of skill in the art, a multitude of different optimized *Yarrrowia* strains producing at least about 50 EPA % TFAs could be engineered using the methodologies described herein. Selection of a preferred strain for commercial purposes will therefore also consider the total lipid content of the engineered strain, since both the concentration of EPA as a percent of the total fatty acids ["EPA % TFAs"] <u>and</u> total lipid content ["TFAs % DCW"] affect the cellular content of EPA as a percent of the dry cell weight ["EPA % DCW"]. That is, EPA % DCW is calculated as: (EPA % TFAs) * (TFA % DCW)]/100. For example, a strain producing 50 EPA % TFAs and having 24 TFAs % DCW, a strain producing 55 EPA % TFAs and having 21.82 TFAs % DCW, a strain producing 60 EPA % TFAs and having 20 TFAs % DCW, a strain producing 65 EPA % TFAs and having 18.46 TFAs % DCW and a strain producing 70 EPA % TFAs and having 17.14 TFAs % DCW all produce 12 EPA % DCW. In preferred embodiments, the improved optimized strain of *Yarrrowia lipolytica* will produce at least about 10-12 EPA % DCW, preferably at least about 12-14 EPA % DCW, more preferably at least about 14-16 EPA % DCW, more preferably at least about 16-18 EPA % DCW, more preferably at least about 18-20 EPA % DCW, more preferably at least about 20-22 EPA % DCW, more preferably at least about 22-24 EPA % DCW, and most preferably at least about 24-26 EPA % DCW.

In addition to possessing at least about 50 EPA % TFAs, the lipid profile within the improved optimized strain of *Yarrrowia lipolytica,* or within extracted or unconcentrated oil therefrom, will have a ratio of EPA % TFAs to LA % TFAs of at least about 3.1. As demonstrated in U.S. Pat. Appl. Pub. No. 2009-0093543-A1 (Table 23), EPA, LA and oleic acid constituted approximately 76-80% of the fatty acids present in the lipid profile of a strain of *Y. lipolytica* producing greater than 40 EPA % TFAs. Of this, LA % TFAs was ca. three-fold greater than oleic acid % TFAs. Based on these observations, one of skill in the art will appreciate that minimizing the concentration of the intermediate fatty acid, LA (resulting in increased ratios of EPA:LA), will result in greater "pushing" of the carbon through the PUFA biosynthetic pathway and permit increased synthesis of EPA. In preferred embodiments, the ratio of EPA:LA will be at least about 3.1-3.5, more preferably at least about 3.5-4.5, more preferably at least about 4.5-5.5, and most preferably at least about 5.5-6.5.

Lipids produced by the improved optimized recombinant Y. *lipolytica* strains described herein will also be distinguished as having less than about 0.5% GLA or DHA (when measured by GC analysis using equipment having a detectable level down to about 0.1 %) and having a saturated fatty acid content of less than about 8%. This low percent of saturated fatty acids (i.e., 16:0 and 18:0) results in substantial health benefits to humans and animals.

Microbial expression systems and expression vectors containing regulatory sequences that direct high-level expression of foreign proteins are well known to those skilled in the art. Any of these could be used to construct chimeric genes encoding the preferred desaturase, elongase, CPT1, DGLA synthase, malonyl CoA synthetase and acyl-CoA lysophospholipid acyltransferase proteins. These chimeric genes could then be introduced into *Yarrowia lipolytica* using standard methods of transformation to provide high-level expression of the encoded enzymes.

Vectors (e.g., constructs, plasmids) and DNA expression cassettes useful for the transformation of *Yarrowia* host cells are well known in the art. The specific choice of sequences present in the construct is dependent upon the desired expression products, the nature of the host cell, and the proposed means of separating transformed cells versus non-transformed cells. Typically, however, the vector contains at least one expression cassette, a selectable marker and sequences allowing autonomous replication or chromosomal integration. Suitable expression cassettes typically comprise a region 5' of the gene that controls transcriptional initiation (e.g., a promoter), the gene coding sequence, and a region 3' of the DNA fragment that controls transcriptional termination (i.e., a terminator). It is most preferred when both control regions are derived from genes from the transformed host cell, although they need not be derived from genes native to the production host (e.g., *Yarrowia lipolytica*).

Where two or more genes are expressed from separate replicating vectors, it is desirable that each vector has a different means of selection and should lack homology to the other constructs to maintain stable expression and prevent reassortment of elements among constructs. Judicious choice of regulatory regions, selection means and method of propagation of the introduced construct can be experimentally determined so that all introduced genes are expressed at the necessary levels to provide for synthesis of the desired products.

Constructs or vectors comprising the gene(s) of interest may be introduced into a host cell such as *Yarrowia* by any standard technique. These techniques include transformation (e.g., lithium acetate transformation [Methods in Enzymology, 194:186-187 (1991)]), bolistic impact, electroporation, microinjection, or any other method that introduces the gene(s) of interest into the host cell. More preferred herein for *Yarrowia lipolytica* are integration techniques based on linearized fragments of DNA, as described in U.S. Patent 4,880,741 and U.S. Patent 5,071,764 and Chen, D. C. et al. (Appl. Microbiol. Biotechnol., 48(2):232-235 (1997)).

For convenience, a host cell that has been manipulated by any method to take up a DNA sequence (e.g., an expression cassette) is referred to herein as "transformed", "transformant" or "recombinant". The transformed host will have at least one copy of the expression cassette and may have two or more, depending upon whether the expression cassette is integrated into the genome or is present on an extrachromosomal element having multiple copy numbers. The transformed host cell can be identified by various selection techniques, as described in U.S. Patent 7,238,482 and U.S. Patent 7,259,255.

Preferred selection methods for use herein are resistance to kanamycin, hygromycin and the amino glycoside G418, as well as ability to grow on media lacking uracil, leucine, lysine, tryptophan or histidine. In alternate embodiments, 5-fluoroorotic acid (5-fluorouracil-6-carboxylic acid monohydrate; "5-FOA") is used for selection of yeast Ura- mutants (U.S. Pat. Appl. Pub. No. 2009-0093543-A1), or a native acetohydroxyacid synthase (or acetolactate synthase; E.C. 4.1.3.18) that confers sulfonyl urea herbicide resistance (Intl. App. Pub. No. WO 2006/052870) is utilized for selection of transformants. A unique method of "recycling" a pair of preferred selection markers for their use in multiple sequential transformations, by use of site-specific recombinase systems, is also taught in U.S. Pat. Appl. Pub. No. 2009-0093543-A1.

As is well known to one of skill in the art, merely inserting a gene (e.g., a desaturase, elongase, CPT1, DGLA synthase, malonyl CoA synthetase, acyl-CoA lysophospholipid acyltransferase) into a cloning vector does not ensure its expression at the desired rate, concentration, amount, etc. It may be desirable to manipulate a number of different genetic elements that control aspects of transcription, RNA stability, translation, protein stability and protein location, oxygen limitation and secretion from the host cell. More specifically, gene expression may be controlled by altering the following: the nature of the relevant transcriptional promoter and terminator sequences; the number of copies of the cloned gene; whether the gene is plasmid-borne or integrated into the genome of the host cell; the final cellular location of the synthesized foreign protein; the efficiency of translation in the host organism; the intrinsic stability of the cloned gene protein within the host cell; and, the codon usage within the cloned gene, such that its frequency approaches the frequency of preferred codon usage of the host cell. Several of these methods of overexpression will be discussed below, and are useful in recombinant *Yarrowia* host cells as a means to overexpress e.g., desaturases, elongases, CPT1 proteins, DGLA synthases, malonyl CoA synthetases and acyl-CoA lysophospholipid acyltransferases.

Expression of the desired gene(s) can be increased at the transcriptional level through the use of a stronger promoter (either regulated or constitutive) to cause increased expression, by removing/deleting destabilizing sequences from either the mRNA or the encoded protein, or by adding stabilizing sequences to the mRNA (U.S. Patent 4,910,141).

Transcription initiation control regions or promoters which are useful to drive expression of heterologous genes or portions thereof in *Yarrowia* host cells are numerous and known to those skilled in the art. Expression can be accomplished in an induced or constitutive fashion. Induced expression can be accomplished by inducing the activity of a regulatable promoter operably linked to the gene of interest, while constitutive expression can be achieved by the use of a constitutive promoter operably linked to the gene of interest. Virtually any promoter (i.e., native, synthetic, or chimeric) capable of directing expression of desaturase, elongase, CPT1, DGLA synthase, malonyl CoA synthetase and acyl-CoA lysophospholipid acyltransferase genes in *Yarrowia* will be suitable, although transcriptional and translational regions from the host species are particularly useful.

In general, the termination region can be derived from the 3' region of the gene from which the initiation region was obtained or from a different gene. A large number of termination regions are known and function satisfactorily in a variety of hosts when utilized both in the same and different genera and species from which they were derived. The termination region usually is selected more as a matter of convenience rather than because of any particular property. Preferably, the termination region is derived from a yeast gene. The 3'-region can also be synthetic, as one of skill in the art can utilize available information to design and synthesize a 3'-region sequence that functions as a transcription terminator. A termination site may be unnecessary, but it is highly preferred.

Although not intended to be limiting, preferred promoter regions and termination regions useful in the disclosure herein are those taught in U.S. Pat. Pub. No. 2009-0093543-A1.

Additional copies (i.e., more than one copy) of the PUFA biosynthetic pathway desaturase, elongase and DGLA synthase genes and/or CPT1, malonyl CoA synthetase and acyl-CoA lysophospholipid acyltransferase genes may be introduced into *Yarrowia lipolytica* to thereby increase EPA production and accumulation. Specifically, additional copies of genes may be cloned within a single expression construct; and/or, additional copies of the cloned gene(s) may be introduced into the host cell by increasing the plasmid copy number or by multiple integration of the cloned gene into the genome (*infra*).

It is important to note that the when preparing optimized strains of *Y. lipolytica* according to the methodology herein, copies of various desaturases, elongases, CPT1s, DGLA synthases, malonyl CoA synthetases and acyl-CoA lysophospholipid acyltransferases are often referred to. If, for example, 2 copies of a Δ9 elongase are required, this can refer to: 1) two copies of an identical coding sequence for a particular Δ9 elongase isolated from a single species; or, 2) one coding sequence for a Δ9 elongase isolated from a species "A" and one coding sequence for a Δ9 elongase isolated from a species "B", thus collectively resulting in two Δ9 elongases.

In general, once a DNA cassette (e.g., comprising a chimeric gene comprising a promoter, ORF and terminator) suitable for expression in an oleaginous yeast has been obtained, it is either placed in a plasmid vector capable of autonomous replication in a host cell or directly integrated into the genome of the host cell. Integration of expression cassettes can occur randomly within the host genome or can be targeted through the use of constructs containing regions of homology with the host genome sufficient to target recombination with the host locus. Although not relied on herein, all or some of the transcriptional and translational regulatory regions can be provided by the endogenous locus where constructs are targeted to an endogenous locus.

The preferred method of expressing genes in *Yarrowia lipolytica* is by integration of a linear DNA fragment into the genome of the host. Integration into multiple locations within the genome can be particularly useful when high level expression of genes are desired. Preferred loci include those taught in U.S. Pat. Pub.No. 2009-0093543-A1.

Juretzek et al. (Yeast, 18:97-113 (2001)) note that the stability of an integrated DNA fragment in *Yarrowia lipolytica* is dependent on the individual transformants, the recipient strain and the targeting platform used. Thus, the skilled artisan will recognize that multiple transformants must be screened in order to obtain a strain displaying the desired expression level and pattern. Such screening may be accomplished by Southern analysis of DNA blots (Southern, J. Mol. Biol., 98:503 (1975)), Northern analysis of mRNA expression (Kroczek, J. Chromatogr. Biomed. Appl., 618 (1-2):133-145 (1993)), Western analysis of protein expression, phenotypic analysis or GC analysis of the PUFA products.

The transformed microbial host cell is grown under conditions that optimize expression of chimeric genes (e.g., encoding desaturases, elongases, CPT1, DGLA synthases, malonyl CoA synthetases, acyl-CoA lysophospholipid acyltransferases) and produce the greatest and the most economical yield of EPA. In general, media conditions may be optimized by modifying the type and amount of carbon source, the type and amount of nitrogen source, the carbon-to-nitrogen ratio, the amount of different mineral ions, the oxygen level, growth temperature, pH, length of the biomass production phase, length of the oil accumulation phase and the time and method of cell harvest. *Yarrowia lipolytica* are generally grown in a complex media such as yeast extract-peptone-dextrose broth ["YPD"] or a defined minimal media that lacks a component necessary for growth and thereby forces selection of the desired expression cassettes (e.g., Yeast Nitrogen Base (DIFCO Laboratories, Detroit, MI)).

Fermentation media for the methods and host cells described herein must contain a suitable carbon source, such as are taught in U.S. Patent 7,238,482 and U.S. Pat. Appl. No. 12/641,929 (filed December 19, 2009). Although it is contemplated that the source of carbon utilized in the present invention may encompass a wide variety of carbon-containing sources, preferred carbon sources are sugars, glycerol and/or fatty acids. Most preferred is glucose, sucrose, invert sucrose, fructose and/or fatty acids containing between 10-22 carbons.

Nitrogen may be supplied from an inorganic (e.g., $(NH_4)_2SO_4$) or organic (e.g., urea or glutamate) source. In addition to appropriate carbon and nitrogen sources, the fermentation media must also contain suitable minerals, salts, cofactors, buffers, vitamins and other components known to those skilled in the art suitable for the growth of the high EPA-producing oleaginous yeast and the promotion of the enzymatic pathways for EPA production. Particular attention is given to several metal ions, such as $Fe^{+2}$, $Cu^{+2}$, $Mn^{+2}$, $Co^{+2}$, $Zn^{+2}$ and $Mg^{+2}$, that promote synthesis of lipids and PUFAs (Nakahara, T. et al., Ind. Appl. Single Cell Oils, D. J. Kyle and R. Colin, eds. pp 61-97 (1992)).

Preferred growth media for the methods and host cells described herein are common commercially prepared media, such as Yeast Nitrogen Base (DIFCO Laboratories, Detroit, MI). Other defined or synthetic growth media may also be used and the appropriate medium for growth of *Yarrowia lipolytica* will be known by one skilled in the art of microbiology or fermentation science. A suitable pH range for the fermentation is typically between about pH 4.0 to pH 8.0, wherein pH 5.5 to pH 7.5 is preferred as the range for the initial growth conditions. The fermentation may be conducted under aerobic or anaerobic conditions, wherein microaerobic conditions are preferred.

Typically, accumulation of high levels of PUFAs in oleaginous yeast cells requires a two-stage process, since the metabolic state must be "balanced" between growth and synthesis/storage of fats. Thus, most preferably, a two-stage fermentation process is necessary for the production of EPA in *Yarrowia lipolytica.* This approach is described in U.S. Patent 7,238,482, as are various suitable fermentation process designs (i.e., batch, fed-batch and continuous) and considerations during growth.

In some aspects herein, the primary product is oleaginous yeast biomass. As such, isolation and purification of the EPA-containing oils from the biomass may not be necessary (i.e., wherein the whole cell biomass is the product).

However, certain end uses and/or product forms may require partial and/or complete isolation/purification of the EPA-containing oil from the biomass, to result in partially purified biomass, purified oil, and/or purified EPA. PUFAs, including EPA, may be found in the host microorganism (e.g., *Yarrowia*) as free fatty acids or in esterified forms such as acylglycerols, phospholipids, sulfolipids or glycolipids. These fatty acids may be extracted from the host cell through a variety of means well-known in the art. One review of extraction techniques, quality analysis and acceptability standards for yeast lipids is that of Z. Jacobs (Critical Reviews in Biotechnology, 12(5/6):463-491 (1992)). A brief review of downstream processing is also available by A. Singh and O. Ward (Adv. Appl. Microbiol., 45:271-312 (1997)).

In general, means for the purification of EPA and other PUFAs from *Yarrowia* biomass may include extraction (e.g., U.S. Patent 6,797,303 and U.S. Patent 5,648,564) with organic solvents, sonication, supercritical fluid extraction (e.g., using carbon dioxide), saponification and physical means such as presses, bead beaters, or combinations thereof. One is referred to the teachings of U.S. Patent 7,238,482 for additional details.

Oils containing EPA that have been refined and/or purified can be hydrogenated, to thereby result in fats with various melting properties and textures. Many processed fats, including spreads, confectionary fats, hard butters, margarines, baking shortenings, etc., require varying degrees of solidity at room temperature and can only be produced through alteration of the source oil's physical properties. This is most commonly achieved through catalytic hydrogenation (see Intl. App. Pub. No. WO 2006/052870 for additional details and references).

The oils of the invention may be used in food products, infant formulas, functional foods, medical foods, medical nutritionals, dietary supplements, pharmaceutical compositions, animal feeds, and personal care products.

One of skill in the art of processing and formulation will understand how the amount and composition of the biomass, partially purified biomass, purified oil, and/or purified EPA may be added to a particular product according to target species and/or end use. More specifically, an "effective" amount should be incorporated into a product formulation, although this amount will depend on the food or feed product, the diet that the product is intended to supplement or the medical condition that the medical food or medical nutritional is intended to correct or treat. Most desirably, the effective amount of EPA will be sufficient to provide the desirable health characteristics associated with ω-3/ω-6 PUFA consumption. Typically, the amount of EPA incorporated into the product takes into account losses associated with processing conditions, typical handling and storage conditions, the stability of EPA in the product, and the bioavailability/ bioabsorption efficiency with the target species, to name a few.

Personal Care Products: Within the context of personal care products, ω-3 fatty acids have particular application in skin formulations where they may be used to enhance the skin conditioning effect. The skilled person will understand how to provide an effective amount of the relevant ω-3 fatty acid(s) or oil comprising the same to a skin care composition. In addition to the PUFA oil or ω-3 fatty acid, the skin care composition may further comprise a cosmetically acceptable medium for skin care compositions, examples of which are described by Philippe et al. in U.S. Patent 6,280,747. For example, the cosmetically acceptable medium may be an anhydrous composition containing a fatty substance in a proportion generally from about 10% to about 90% by weight relative to the total weight of the composition, where the fatty phase contains at least one liquid, solid or semi-solid fatty substance. The fatty substance includes, but is not limited to, oils, waxes, gums, and so-called pasty fatty substances. Alternatively, the compositions may be in the form of a stable dispersion such as a water-in-oil or oil-in-water emulsion. Additionally, the compositions may contain one or more conventional cosmetic or dermatological additives or adjuvants including, but not limited to, antioxidants, preserving agents, fillers, surfactants, UVA and/or UVB sunscreens, fragrances, thickeners, wetting agents, anionic or nonionic or amphoteric polymers, and dyes.

Foodstuffs: The market place currently supports a large variety of food and feed products, incorporating ω-3 and/or ω-6 fatty acids (particularly LA, GLA, ARA, EPA, DPA and DHA). It is contemplated that the yeast biomass, partially purified biomass, purified oil, and/or purified EPA described herein will function in food products to impart the health benefits of current formulations.

Food products will include, but not be limited to: food analogs, drinks, meat products, cereal products, baked foods, snack foods and dairy products.

Food analogs can be made using processes well known to those skilled in the art. There can be mentioned meat analogs, cheese analogs, milk analogs and the like. Meat analogs made from soybeans contain soy protein or tofu and other ingredients mixed together to simulate various kinds of meats. These meat alternatives are sold as frozen, canned or dried foods. Usually, they can be used the same way as the foods they replace. Examples of meat analogs include, but are not limited to: ham analogs, sausage analogs, bacon analogs, and the like.

Food analogs can be classified as imitation or substitutes depending on their functional and compositional characteristics. For example, an imitation cheese need only resemble the cheese it is designed to replace. However, a product can generally be called a substitute cheese only if it is nutritionally equivalent to the cheese it is replacing and meets the minimum compositional requirements for that cheese. Thus, substitute cheese will often have higher protein levels than imitation cheeses and be fortified with vitamins and minerals.

Milk analogs or nondairy food products include, but are not limited to: imitation milks and nondairy frozen desserts (e.g., those made from soybeans and/or soy protein products).

Meat products encompass a broad variety of products. In the United States "meat" includes "red meats" produced from cattle, hogs and sheep. In addition to the red meats there are poultry items which include chickens, turkeys, geese, guineas, ducks and the fish and shellfish. There is a wide assortment of seasoned and processed meat products: fresh, cured and fried, and cured and cooked. Sausages and hot dogs are examples of processed meat products. Thus, the term "meat products" as used herein includes, but is not limited to, processed meat products.

A cereal food product is a food product derived from the processing of a cereal grain. A cereal grain includes any plant from the grass family that yields an edible grain (seed). The most popular grains are barley, corn, millet, oats, quinoa, rice, rye, sorghum, triticale, wheat and wild rice. Examples of cereal food products include, but are not limited to: whole grain, crushed grain, grits, flour, bran, germ, breakfast cereals, extruded foods, pastas, and the like.

A baked goods product comprises any of the cereal food products mentioned above and which has been baked or processed in a manner comparable to baking, i.e., to dry or harden by subjecting to heat. Examples of a baked good product include, but are not limited to: bread, cakes, doughnuts, bars, pastas, bread crumbs, baked snacks, mini-biscuits, mini-crackers, mini-cookies, and mini-pretzels. As was mentioned above, oils from the recombinant EPA production host cells can be used as an ingredient.

A snack food product comprises any of the above or below described food products.

A fried food product comprises any of the above or below described food products that has been fried.

The beverage can be in a liquid or in a dry powdered form. For example, there can be mentioned: non-carbonated drinks; fruit juices, fresh, frozen, canned or concentrate; flavored or plain milk drinks, etc. Adult and infant nutritional formulas are well known in the art and commercially available (e.g., Similac®, Ensure®, Jevity®, and Alimentum® from Ross Products Division, Abbott Laboratories).

A dairy product is a product derived from milk. A milk analog or nondairy product is derived from a source other than milk, for example, soymilk as was discussed above. These products include, but are not limited to: whole milk, skim milk, fermented milk products such as yogurt or sour milk, cream, butter, condensed milk, dehydrated milk, coffee whitener, coffee creamer, ice cream, cheese, etc.

Additional food products into which the *Yarrowia* biomass, partially purified biomass, purified oil, and/or purified EPA could be included are, for example: chewing gums, confections and frostings, gelatins and puddings, hard and soft candies, jams and jellies, white granulated sugar, sugar substitutes, sweet sauces, toppings and syrups, and dry-blended powder mixes.

Infant Formulas: Infant formulas are liquids or reconstituted powders fed to infants and young children. "Infant formula" is defined herein as an enteral nutritional product which can be substituted for human breast milk in feeding infants and typically is composed of a desired percentage of fat mixed with desired percentages of carbohydrates and proteins in an aquous solution (e.g., see U.S. Patent 4,670,285). Based on worldwide composition studies, as well as levels specified by expert groups, average human breast milk typically contains about 0.20% to 0.40% of total fatty acids (assuming about 50% of calories from fat); and, generally the ratio of DHA to ARA would range from about 1:1 to 1:2 (see, e.g., formulations of Enfamil LIPIL™ [Mead Johnson & Company] and Similac Advance™ [Ross Products Division, Abbott Laboratories]). Infant formulas have a special role to play in the diets of infants because they are often the only source of nutrients for infants. Although breast-feeding is still the best nourishment for infants, infant formula is a close enough second that babies not only survive but thrive.

Health Food Products And Pharmaceuticals: The present biomass, partially purified biomass, purified oil, and/or purified EPA may be used in formulations to impart health benefit in health food products, including functional foods, medical foods, medical nutritionals and dietary supplements. Additionally, *Yarrowia* biomass, partially purified biomass, purified oil, and/or purified EPA may be used in standard pharmaceutical compositions. The present engineered strains of *Yarrowia lipolytica* or the microbial oils produced therefrom comprising EPA could readily be incorporated into the any of the above mentioned food products, to thereby produce e.g., a functional or medical food. For example, more concentrated formulations comprising EPA include capsules, powders, tablets, softgels, gelcaps, liquid concentrates and emulsions which can be used as a dietary supplement in humans or animals other than humans.

Animal Feed Products: Animal feeds are generically defined herein as products intended for use as feed or for mixing in feed for animals other than humans. The *Yarrowia* biomass, partially purified biomass, purified oil, and/or purified EPA described herein can be used as an ingredient in various animal feeds.

More specifically, although not to be construed as limiting, it is expected that the EPA products from the recombinant *Yarrowia* host cells can be used within pet food products, ruminant and poultry food products and aquacultural food products. Pet food products are those products intended to be fed to a pet, such as a dog, cat, bird, reptile, rodent. These products can include the cereal and health food products above, as well as meat and meat byproducts, soy protein products, grass and hay products (such as alfalfa, timothy, oat or brome grass) and vegetables. Ruminant and poultry food products are those wherein the product is intended to be fed to e.g., turkeys, chickens, cattle and swine. As with the pet foods above, these products can include cereal and health food products, soy protein products, meat and meat byproducts, and grass and hay products as listed above. Aquacultural food products (or "aquafeeds") are those products intended to be used in aquafarming, which concerns the propagation, cultivation or farming of aquatic organisms and/or animals in fresh or marine waters.

There is increasing awareness that EPA is an important ω-3 fatty acid in and of itself. As a result, it is expected herein that the EPA-enriched oils of the recombinant *Yarrowia* production hosts described herein will have very broad utility in a variety of therapeutic applications, e.g., inflammation, cardiovascular diseases, nutrient regulation of gene expression and dyslipidemia, and specifically in the treatment of clinical conditions including: coronary heart disease, high blood pressure, inflammatory disorders, Type II diabetes, ulcerative colitis, Crohn's disease, anorexia nervosa, burns, osteoarthritis, osteoporosis, depression, and attention deficit/hyperactivity disorder.

U.S. Pat. Appl. Pub. No. 2009-0093543-A1 describes additional clinical human studies, relating to EPA and inflammation, EPA and cardiovascular diseases, ω-3 PUFAs and nutrient regulation of gene expression, and ω-3 PUFAs and dyslipidemia and should be referred to therein. More recently, a randomized, double-blind placebo-controlled study was performed in 110 normal healthy subjects , wherein subjects were provided one of the following for 6 weeks, as a means to evluate the effectsof the oils on cardiovascular disease risk factors, adverse events and safety parameters: 600 mg/day EPA, 1800 mg/day EPA, 600 mg/day DHA or olive oil (placebo) (Gillies, P., "The New Science of Omega-3 Fatty Acids: Differential Nutritional Pharmacology" Texas Human Nutrition Conference, Texas A&M University, February 2010; U.S. Provisional Patent Applications No. 61/292915 [filed January 7, 2010] and No. 61/295347 [filed January 15, 2010], having E. I duPont de Nemours and Company Attorney Docket Numbers CL4938USPRV and CL4938USPRV1, respectively). The 600 mg EPA per day supplement was found to maintain healthy cholesterol levels already in the normal range. Notably, the EPA oils of the study were derived from engineered strains of *Yarrowa lipolytica.*

## EXAMPLES

The present invention is further defined in the following Examples. It should be understood that these Examples, while indicating preferred embodiments of the invention, are given by way of illustration only. From the above discussion and these Examples, one skilled in the art can ascertain the essential characteristics of this invention.

GENERAL METHODS

Standard recombinant DNA and molecular cloning techniques used in the Examples are well known in the art and are described by: 1) Sambrook, J., Fritsch, E. F. and Maniatis, T. Molecular Cloning: A Laboratory Manual; Cold Spring Harbor Laboratory: Cold Spring Harbor, NY (1989) (Maniatis); 2) T. J. Silhavy, M. L. Bennan, and L. W. Enquist, Experiments with Gene Fusions; Cold Spring Harbor Laboratory: Cold Spring Harbor, NY (1984); and, 3) Ausubel, F. M. et al., Current Protocols in Molecular Biology, published by Greene Publishing Assoc. and Wiley-Interscience, Hoboken, NJ (1987).

Materials and methods suitable for the maintenance and growth of microbial cultures are well known in the art. Techniques suitable for use in the following examples may be found as set out in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, Eds), American Society for Microbiology: Washington, D.C. (1994)); or by Thomas D. Brock in Biotechnology: A Textbook of Industrial Microbiology, 2nd ed., Sinauer Associates: Sunderland, MA (1989). All reagents, restriction enzymes and materials used for the growth and maintenance of microbial cells were obtained from Aldrich Chemicals (Milwaukee, WI), DIFCO Laboratories (Detroit, MI), New England Biolabs, Inc. (Beverly, MA), GIBCO/BRL (Gaithersburg, MD), or Sigma Chemical Company (St. Louis, MO), unless otherwise specified. *E. coli* strains were typically grown at 37 °C on Luria Bertani ["LB"] plates.

General molecular cloning was performed according to standard methods (Sambrook et al., *supra*). When PCR or site-directed mutagenesis was involved in subcloning, the constructs were sequenced to confirm that no errors had been introduced to the sequence. PCR products were cloned into Promega's pGEM-T-easy vector (Madison, WI).

The meaning of abbreviations is as follows: "sec" means second(s), "min" means minute(s), "h" means hour(s), "d" means day(s), "μL" means microliter(s), "mL" means milliliter(s), "L" means liter(s), "μM" means micromolar, "mM" means millimolar, "M" means molar, "mmol" means millimole(s), "μmole" mean micromole(s), "g" means gram(s), "μg" means microgram(s), "ng" means nanogram(s), "U" means unit(s), "bp" means base pair(s), "kB" means kilobase(s), "DCW" means dry cell weight, and "TFAs" means total fatty acids.

**Nomenclature For Expression Cassettes**

The structure of an expression cassette will be represented by a simple notation system of "X::Y::Z", wherein X describes the promoter fragment, Y describes the gene fragment, and Z describes the terminator fragment, which are all operably linked to one another.

**Transformation And Cultivation Of *Yarrowia lipolytica***

*Yarrowia lipolytica* strain ATCC #20362 was purchased from the American Type Culture Collection (Rockville, MD). *Yarrowia lipolytica* strains were routinely grown at 28-30 °C in several media, according to the recipes shown below. Agar plates were prepared as required by addition of 20 g/L agar to each liquid media, according to standard methodology.

YPD agar medium (per liter): 10 g of yeast extract [Difco], 20 g of Bacto peptone [Difco], and 20 g of glucose.

Basic Minimal Media ["MM"] (per liter): 20 g glucose, 1.7 g yeast nitrogen base without amino acids, 1.0 g proline, and pH 6.1 (do not need to adjust).

Minimal Media + Uracil ["MM+uracil or MMU"] (per liter): Prepare MM media as above and add 0.1 g uracil and 0.1 g uridine.

Minimal Media + Uracil + Sulfonylurea ["MMU+SU"] (per liter): Prepare MMU media as above and add 280 mg sulfonylurea.

Minimal Media + Uracil + Lysine ["MMUraLys"] (per liter): Prepare MM media as above and add 0.1 g uracil, 0.1 g uridine. and 0.1 g lysine.

Minimal Media + 5-Fluoroorotic Acid ["MM + 5-FOA"] (per litter): 20 g glucose, 6.7 g Yeast Nitrogen base, 75 mg uracil, 75 mg uridine and appropriate amount of FOA (Zymo Research Corp., Orange, CA), based on FOA activity testing against a range of concentrations from 100 mg/L to 1000 mg/L (since variation occurs within each batch received from the supplier).

High Glucose Media ["HGM"] (per liter): 80 glucose, 2.58 g $KH_2PO_4$ and 5.36 g $K_2HPO_4$, pH 7.5 (do not need to adjust).

Fermentation medium without Yeast Extract ["FM without YE"] (per liter): 6.70 g/L Yeast nitrogen base, 6.00 g $KH_2PO_4$, 2.00 g $K_2HPO_4$, 1.50 g $MgSO_4*7H_2O$, and 20 g glucose.

Fermentation medium ["FM"] (per liter): 6.70 g/L Yeast nitrogen base, 6.00 g $KH_2PO_4$, 2.00 g $K_2HPO_4$, 1.50 g $MgSO_4*7H_2O$, 20 g glucose and 5.00 g Yeast extract (BBL).

Transformation of *Y. lipolytica* was performed as described in U.S. Pat. Appl. Pub. No. 2009-0093543-A1.

Fatty Acid Analysis Of *Yarrowia lipolytica*

For fatty acid ["FA"] analysis, cells were collected by centrifugation and lipids were extracted as described in Bligh, E. G. & Dyer, W. J. (Can. J. Biochem. Physiol., 37:911-917 (1959)). Fatty acid methyl esters ["FAMEs"] were prepared by transesterification of the lipid extract with sodium methoxide (Roughan, G., and Nishida I., Arch Biochem Biophys., 276(1):38-46 (1990)) and subsequently analyzed with a Hewlett-Packard 6890 GC fitted with a 30-m X 0.25 mm (i.d.) HP-INNOWAX (Hewlett-Packard) column. The oven temperature was from 170 °C (25 min hold) to 185 °C at 3.5 °C/min.

For direct base transesterification, *Yarrowia* cells (0.5 mL culture) were harvested, washed once in distilled water, and dried under vacuum in a Speed-Vac for 5-10 min. Sodium methoxide (100 μl of 1 %) and a known amount of C15:0 triacylglycerol (C15:0 TAG; Cat. No. T-145, Nu-Check Prep, Elysian, MN) was added to the sample, and then the sample was vortexed and rocked for 30 min at 50 °C. After adding 3 drops of 1 M NaCl and 400 μl hexane, the sample was vortexed and spun. The upper layer was removed and analyzed by GC.

FAME peaks recorded via GC analysis were identified by their retention times, when compared to that of known fatty acids, and quantitated by comparing the FAME peak areas with that of the internal standard (C15:0 TAG) of known amount. Thus, the approximate amount (μg) of any fatty acid FAME ["μg FAME"] is calculated according to the formula: (area of the FAME peak for the specified fatty acid/ area of the standard FAME peak) * (μg of the standard C15:0 TAG), while the amount (μg) of any fatty acid ["μg FA"] is calculated according to the formula: (area of the FAME peak for the specified fatty acid/area of the standard FAME peak) * (μg of the standard C15:0 TAG) * 0.9503, since 1 μg of C15:0 TAG is equal to 0.9503 μg fatty acids. Note that the 0.9503 conversion factor is an approximation of the value determined for most fatty acids, which range between 0.95 and 0.96.

The lipid profile, summarizing the amount of each individual fatty acid as a weight percent of TFAs, was determined by dividing the individual FAME peak area by the sum of all FAME peak areas and multiplying by 100.

Analysis Of Total Lipid Content And Composition In *Yarrowia lipolytica* By Flask Assay

For a detailed analysis of the total lipid content and composition in a particular strain of *Y. lipolytica,* flask assays were conducted as followed. Specifically, one loop of freshly streaked cells was inoculated into 3 mL FM medium and grown overnight at 250 rpm and 30°C. The $OD_{600nm}$ was measured and an aliquot of the cells were added to a final $OD_{600nm}$ of 0.3 in 25 mL FM medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30°C, a 1 mL aliquot was used for fatty acid analysis (*supra*) and 10 mL dried for dry cell weight ["DCW"] determination.

For DCW determination, 10 mL culture was harvested by centrifugation for 5 min at 4000 rpm in a Beckman GH-3.8 rotor in a Beckman GS-6R centrifuge. The pellet was resuspended in 25 mL of water and re-harvested as above. The washed pellet was re-suspended in 20 mL of water and transferred to a pre-weighed aluminum pan. The cell suspension was dried overnight in a vacuum oven at 80 °C. The weight of the cells was determined.

Total lipid content of cells ["TFAs % DCW"] is calculated and considered in conjunction with data tabulating the concentration of each fatty acid as a weight percent of TFAs ["% TFAs"] and the EPA content as a percent of the dry cell weight ["EPA % DCW"]. Data from flask assays will be presented as a table that summarizes the total dry cell weight of the cells ["DCW"], the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids will be identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

EXAMPLE 1

Generation Of *Yarrowia lipolytica* Strain L135 (*Ura3*+, *Leu*-, Δpex3) To Produce About 46% DGLA Of Total Fatty Acids

The present Example describes the construction of strain L135, derived from *Yarrowia lipolytica* ATCC #20362, capable of producing about 46% DGLA relative to the total lipids via expression of a Δ9 elongase/Δ8 desaturase pathway.

Briefly, as diagrammed in FIG. 2, strain L135 was derived from *Yarrowia lipolytica* ATCC #20362 via construction of strain Y2224 (a FOA resistant mutant from an autonomous mutation of the *Ura3* gene of wildtype *Yarrowia* strain ATCC #20362), strain Y4001 (producing 17% EDA with a *Leu*- phenotype), strain Y4001U1 (*Leu*- and *Ura*-), strain Y4036 (producing 18% DGLA with a *Leu*- phenotype) and strain Y4036U (*Leu*- and *Ura*-). Further details regarding the construction of strains Y2224, Y4001, Y4001 U, Y4036 and Y4036U are described in the General Methods of U.S. Pat. App. Pub. No. 2008-0254191.

The final genotype of strain Y4036U with respect to wild type *Yarrowia lipolytica* ATCC #20362 was *Ura3-,* YAT1::ME3S::Pex16, EXP1::EgD9eS::Lip1, FBAINm::EgD9eS::Lip2, GPAT::EgD9e::Lip2, FBAINm::EgD8M::Pex20, EXP1::EgDBM::Pex16, GPD::FmD12::Pex20, YAT1::FmD12::OCT (wherein FmD12 is a *Fusarium moniliforme* Δ12 desaturase gene [U.S. Patent 7,504,259]; ME3S is a codon-optimized $C_{16/18}$ elongase gene, derived from *Mortierella alpina* [U.S. Patent 7,470,532]; EgD9e is a *Euglena gracilis* Δ9 elongase gene [U.S. Patent 7,645,604]; EgD9eS is a codon-optimized Δ9 elongase gene, derived from *Euglena gracilis* [U.S. Patent 7,645,604]; EgD8M is a synthetic mutant Δ8 desaturase [U.S. Patent 7,709,239], derived from *Euglena gracilis* [U.S. Patent 7,256,033]).

Generation Of L135 Strain With Chromosomal Deletion Of Pex3

Construction of strain L135 is described in Example 12 of Intl. App. Pub. No. WO 2009/046248. Briefly, construct pY157 was used to knock out the chromosomal gene encoding the peroxisome biogenesis factor 3 protein [peroxisomal assembly protein Peroxin 3 or "Pex3p"] in strain Y4036U, thereby producing strain L135 (also referred to as strain Y4036 (Δpex3)). Knockout of the chromosomal Pex3 gene in strain L135, as compared to in strain Y4036 (whose native Pex3p had not been knocked out) resulted in the following: higher lipid content (TFAs % DCW) (ca. 6.0% versus 4.7%), higher DGLA % TFAs (46% versus 19%), higher DGLA % DCW (ca. 2.8% versus 0.9%) and reduced LA % TFAs (12% versus 30%). Additionally, the Δ9 elongase percent conversion efficiency was increased from ca. 48% in strain Y4036 to 83% in strain L135.

The final genotype of strain L135 with respect to wildtype *Yarrowia lipolytica* ATCC #20362 was *Ura3*+, *Leu*-, *Pex3*-, *unknown1*-, YAT1::ME3S::Pex16, EXP1::EgD9eS::Lip1, FBAINm::EgD9eS::Lip2, GPAT::EgD9e::Lip2, FBAINm::EgD8M::Pex20, EXP1::EgDBM::Pex16, GPD::FmD12::Pex20, YAT1::FmD12::OCT.

EXAMPLE 2

Generation Of *Yarrowia lipolytica* Strains Producing From About 18% To About 41 % ARA Of Total Fatty Acids ["TFAs"]

The present Example describes the construction of strain Y8006, derived from *Yarrowia lipolytica* ATCC #20362, capable of producing about 41 % ARA relative to the total lipids via expression of a Δ9 elongase/Δ8 desaturase pathway.

The development of strain Y8006 (FIG. 2) required the construction of strains Y2224, Y4001, Y4001 U, Y4036, Y4036U and L135 (described in Example 1), as well as construction of strains L135U9 and Y8002.

Generation of L135U9 (*Leu-, Ursa3-*) Strain

Strain L135U was created *via* temporary expression of the *Cre* recombinase enzyme in plasmid pY116 (FIG. 3; SEQ ID NO:127; described in Example 7 of Intl. App. Pub. No. WO 2008/073367), within strain L135 to produce a *Leu*- and *Ura*- phenotype. Plasmid pY116 was used for transformation of freshly grown L135 cells according to the General Methods. The transformant cells were plated onto MMLeuUra and maintained at 30 °C for 3 to 4 days. Three colonies were picked, inoculated into 3 mL liquid YPD media at 30 °C and shaken at 250 rpm/min for 1 day. The cultures were diluted to 1:50,000 with liquid MMLeuUra media, and 100 μL was plated onto new YPD plates and maintained at 30 °C for 2 days. Eight colonies were picked from each of three plates (24 colonies total) and streaked onto MMLeu and MMLeuUra selection plates. The colonies that could grow on MMLeuUra plates but not on MMLeu plates were selected and analyzed by GC to confirm the presence of C20:2 (EDA). One strain, having a *Leu*- and *Ura*- phenotype, was designated as L135U9.

Generation Of Y8002 Strain To Produce About 32% ARA Of TFAs

Construct pZKSL-5S5A5 (FIG. 4A; SEQ ID NO:128) was generated to integrate three Δ5 desaturase genes into the *Lys* loci of strain L135U9, to thereby enable production of ARA. The pZKSL-5S5A5 plasmid contained the following components:

Table 6: Description of Plasmid pZKSL-5S5A5 (SEQ ID NO:128)

| RE Sites And Nucleotides Within SEQ ID NO:128 | Description Of Fragment And Chimeric Gene Components |
|---|---|
| *Asc*I/*BsiW*I (5925-6645) | 720 bp 5' portion of *Yarrowia Lys5* gene (GenBank Accession No. M34929; labeled as "lys5 5' region" in Figure) |
| *Pac*I/*Sph*I (2536-3225) | 689 bp 3' portion of *Yarrowia Lys5* gene (GenBank Accession No. M34929; labeled as "Lys5-3"' in Figure) |
| *EcoR*I/*BsiW*I (9338-6645) | FBAIN::EgD5SM::Pex20, comprising:<br>• FBAIN: *Yarrowia lipolytica* FBAIN promoter (U.S. Patent 7,202,356);<br>• EgD5SM: Synthetic mutant Δ5 desaturase (SEQ ID NO:71; U.S. Pat. Pub. No. 2010-0075386-A1), derived from *Euglena gracilis* (U.S. Patent 7,678,560) (labeled as "ED5S" in Figure);<br>• Pex20: Pex20 terminator sequence from *Yarrowia Pex20* gene (GenBank Accession No. AF054613) |
| *Pme*I/*Cla*I (11503-1) | YAT1::EaD5SM::OCT, comprising:<br>• YAT1: *Yarrowia lipolytica* YAT1 promoter (labeled as "YAT" in Figure; U.S. Pat. Appl. Pub. No. 2006-0094102-A1);<br>• EaD5SM: Synthetic mutant Δ5 desaturase (SEQ ID NO:81; U.S. Pat. Pub. No. 2010-0075386-A1), derived from *Euglena anabaena* (U.S. Pat. Appl. Pub. No. 2008-0274521-A1) (labeled as "EaD5S" in Figure);<br>• OCT: OCT terminator sequence of *Yarrowia OCT* gene (GenBank Accession No. X69988) |
| *Cla*I/*Pac*I (1-2536) | EXP1::EgD5M::Pex16, comprising:<br>• EXP1: *Yarrowia lipolytica* export protein (EXP1) promoter (labeled as "EXP" in Figure; Intl. App. Pub. No. WO 2006/052870);<br>• EgD5M: Mutant Δ5 desaturase (SEQ ID NO:69; U.S. Pat. Pub. No. 2010-0075386-A1) with elimination of internal *EcoR*I, *Bgl*II, *Hind*III and *Nco*I restriction enzyme sites, derived from *Euglena gracilis* (U.S. Patent 7,678,560) (labeled as "Euglena D5WT" in Figure);<br>• Pex16: Pex16 terminator sequence from *Yarrowia Pex16* gene (GenBank Accession No. U75433) |
| *Eco*RI/*Pme*I (9360-11503) | *Yarrowia Leu2* gene (GenBank Accession No. M37309) |

The pZKSL-5S5A5 plasmid was digested with *Asc*I/*Sph*I, and then used for transformation of strain L135U9 according to the General Methods. The transformant cells were plated onto MMUraLys plates and maintained at 30 °C for 2 to 3 days. Single colonies were then re-streaked onto MMUraLys plates, and then inoculated into liquid MMUraLys at 30 °C and shaken at 250 rpm/min for 2 days. The cells were subjected to fatty acid analysis, according to the General Methods.

GC analyses showed the presence of ARA in the transformants containing the 3 chimeric genes of pZKSL-5S5A5, but not in the parent L135U9 strain. Five strains (i.e., #28, #62, #73, #84 and #95) that produced about 32.2%, 32.9%, 34.4%, 32.1% and 38.6% ARA of TFAs were designated as strains Y8000, Y8001, Y8002, Y8003 and Y8004, respectively. Further analyses showed that the three chimeric genes of pZKSL-5S5A5 were not integrated into the *Lys5* site in the Y8000, Y8001, Y8002, Y8003 and Y8004 strains. All strains possessed a *Lys*+ phenotype.

The final genotype of strains Y8000, Y8001, Y8002, Y8003 and Y8004 with respect to wildtype *Yarrowia*

*lipolytica* ATCC #20362 was *Ura-, Pex3-, unknown 1-, unknown 2-*, *Leu+*, *Lys+*, YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgD8M::Pex16, FBAIN::EgD5SM::Pex20, EXP1::EgDSM::Pex16, YAT1::EaDSSM::Oct.

Generation Of Y8006 Strain To Produce About 41% ARA Of TFAs

Construct pZP3-Pa777U (FIG. 4B; SEQ ID NO:129; described in Table 9 of U.S. Pat. Appl. Pub. No. 2009-0093543-A1), was generated to integrate three Δ17 desaturase genes into the *Pox3* loci (GenBank Accession No. AJ001301) of strain Y8002.

The pZP3-Pa777U plasmid was digested with *Asc*I/*Sph*I, and then used for transformation of strain Y8002 according to the General Methods. The transformant cells were plated onto MM plates and maintained at 30 °C for 2 to 3 days. Single colonies were then re-streaked onto MM plates, and then inoculated into liquid MM at 30 °C and shaken at 250 rpm/min for 2 days. The cells were subjected to fatty acid analysis, according to the General Methods.

GC analyses showed the presence of 26% to 31% EPA of TFAs in most of the selected 96 transformants containing the 3 chimeric genes of pZP3-Pa777U, but not in the parent Y8002 strain. Strain #69 produced about 38% EPA of TFAs and was designated as Y8007. There was one strain (i.e., strain #9) that did not produce EPA, but produced about 41% ARA of TFAs. This strain was designated as Y8006. Based on the lack of EPA production in strain Y8006, its genotype with respect to wildtype *Yarrowia lipolytica* ATCC #20362 is assumed to be *Pex3-*, *unknown 1-*, *unknown 2-, unknown 3-*, *Leu+*, *Lys+*, Ura+, YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgDBM::Pex16, FBAIN::EgD5SM::Pex20, EXP1::EgD5M::Pex16, YAT1::EaD5SM::Oct.

In contrast, the final genotype of strain Y8007 with respect to wildtype *Yarrowia lipolytica* ATCC #20362 was *Pex3-*, *unknown 1-*, *unknown 2-*, *unknown 3-*, *Leu+*, *Lys+*, *Ura+*, YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgDBM::Pex16, FBAIN::EgD5SM::Pex20, EXP1::EgD5M::Pex16, YAT1::EaD5SM::Oct, YAT1::PaD17S::Lip1, EXP1::PaD17::Pex16, FBAINm::PaD17::Aco (wherein PaD17 is a *Pythium aphanidermatum* Δ17 desaturase [U.S. Patent 7,556,949] and PaD17S is a codon-optimized Δ17 desaturase, derived from *Pythium aphanidermatum* [U.S. Patent 7,556,949].

Integration of the 3 chimeric genes of pZP3-Pa777U into the *Pox3* loci (GenBank Accession No. AJ001301) in strains Y8006 and Y8007 was not confirmed.

EXAMPLE 3

Generation Of *Yarrowia lipolytica* Strains Producing From About 24% To About 56% EPA Of Total Fatty Acids ["TFAs"]

The present Example describes the construction of strain Y8412, derived from *Yarrowia lipolytica* ATCC #20362, capable of producing about 56% EPA relative to the total lipids via expression of a Δ9 elongase/Δ8 desaturase pathway.

The development of strain Y8412 (FIG. 2) required the construction of strains Y2224, Y4001, Y4001 U, Y4036, Y4036U and L135 (described in Example 1), strains L135U9 and Y8002 (described in Example 2), and strains Y8006U6, Y8069, Y8069U, Y8154, Y8154U, Y8269 and Y8269U.

Generation Of Strain Y8006U6 (*Ura3-*)

In order to disrupt the *Ura3* gene, construct pZKUM (FIG. 5A; SEQ ID NO:130; described in Table 15 of U.S. Pat. Appl. Pub. No. 2009-0093543-A1), was used to integrate a Ura3 mutant gene into the Ura3 gene of strain Y8006.

Plasmid pZKUM was digested with *Sal*I/*Pac*I, and then used to transform strain Y8006 according to the General Methods. Following transformation, cells were plated onto MM + 5-FOA selection plates and maintained at 30 °C for 2 to 3 days.

A total of 8 transformants grown on MM + 5-FOA plates were picked and re-streaked onto MM plates and MM + 5-FOA plates, separately. All 8 strains had a *Ura-* phenotype (i.e., cells could grow on MM + 5-FOA plates, but not on MM plates). The cells were scraped from the MM + 5-FOA plates and subjected to fatty acid analysis, according to the General Methods.

GC analyses showed the presence of 22.9%, 25.5%, 23.6% 21.6%, 21.6% and 25% ARA in the pZKUM-transformant strains #1, #2, #4, #5, #6 and #7, respectively, grown on MM + 5-FOA plates. These six strains were designated as strains Y8006U1, Y8006U2, Y8006U3, Y8006U4, Y8006U5 and Y8006U6, respectively (collectively, Y8006U).

Generation Of Y8069 Strain To Produce About 37.5% EPA Of TFAs

Construct pZP3-Pa777U (FIG. 4B; SEQ ID NO:129; described in Table 9 of U.S. Pat. Appl. Pub. No. 2009-0093543-A1) was used to integrate three Δ17 desaturase genes into the *Pox3* loci (GenBank Accession No. AJ001301) of strain Y8006U6.

The pZP3-Pa777U plasmid was digested with *Asc*I/*Sph*I, and then used for transformation of strain Y8006U6 according to the General Methods. The transformant cells were plated onto MM plates and maintained at 30 °C for 2 to 3 days. Single colonies were then re-streaked onto MM plates, and then inoculated into liquid MM at 30 °C and shaken at 250 rpm/min for 2 days. The cells were subjected to fatty acid analysis, according to the General Methods.

GC analyses showed the presence of EPA in the transformants containing the 3 chimeric genes of pZP3-Pa777U, but not in the parent Y8006U6 strain. Most of the selected 24 strains produced 24-37% EPA of TFAs. Four strains (i.e., #1, #6, #11 and #14) that produced 37.5%, 43.7%, 37.9% and 37.5% EPA of TFAs were designated as Y8066, Y8067, Y8068 and Y8069, respectively. Integration of the 3 chimeric genes of pZP3-Pa777U into the *Pox3* loci (GenBank Accession No. AJ001301) of strains Y8066, Y8067, Y8068 and Y8069 was not confirmed.

The final genotype of strains Y8066, Y8067, Y8068 and Y8069 with respect to wildtype *Yarrowia lipolytica* ATCC #20362 was Ura+, *Pex3*-, *unknown 1-, unknown 2-, unknown 3-, unknown 4-, Leu+, Lys*+, YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgD8M::Pex16, FBAIN::EgD5SM::Pex20, EXP1::EgD5M::Pex16, YAT1::EaDSSM::Oct, YAT1::PaD17S::Lip1, EXP1::PaD17::Pex16, FBAINm::PaD17::Aco.

Generation Of Strain Y8069U (*Ura3*-)

In order to disrupt the *Ura3* gene, construct pZKUM (FIG. 5A; SEQ ID NO:130 described in Table 15 of U.S. Pat. Appl. Pub. No. 2009-0093543-A1) was used to integrate a *Ura3* mutant gene into the *Ura3* gene of strain Y8069, in a manner similar to that described for pZKUM transformation of strain Y8006 *(supra*). A total of 3 transformants were grown and identified to possess a *Ura-* phenotype.

GC analyses showed the presence of 22.4%, 21.9% and 21.5% EPA in the pZKUM-transformant strains #1, #2 and #3, respectively, grown on MM + 5-FOA plates. These three strains were designated as strains Y8069U1, Y8069U2, and Y8069U3, respectively (collectively, Y8069U).

Generation Of Strain Y8154 To Produce about 44.8% EPA Of TFAs

Construct pZKL2-5mB89C (FIG. 5B; SEQ ID NO:131) was generated to integrate one Δ5 desaturase gene, one Δ9 elongase gene, one Δ8 desaturase gene, and one *Yarrowia lipolytica* diacylglycerol cholinephosphotransferase gene (CPT1) into the *Lip2* loci (GenBank Accession No. AJ012632) of strain Y8069U3 to thereby enable higher level production of EPA. The pZKL2-5mB89C plasmid contained the following components:

Table 7: Description of Plasmid pZKL2-5mB89C (SEQ ID NO:131)

| RE Sites And Nucleotides Within SEQ ID NO:131 | Description Of Fragment And Chimeric Gene Components |
|---|---|
| *Asc*I/*Bsi*WI (730-1) | 722 bp 5' portion of *Yarrowia Lip2* gene (labeled as "Lip2.5N" in Figure; GenBank Accession No. AJ012632) |
| *Pac*I/*Sph*I (4141-3438) | 697 bp 3' portion of *Yarrowia Lip2* gene (labeled as "Lip2.3N" in Figure; GenBank Accession No. AJ012632) |
| *Swa*I/*Bsi*WI (13561-1) | YAT1::YICPT1::Aco, comprising:<br>• YAT1: *Yarrowia lipolytica* YAT1 promoter (labeled as "YAT" in Figure; U.S. Pat. Appl. Pub. No. 2006-0094102-A1);<br>• YICPT1: *Yarrowia lipolytica* diacylglycerol cholinephosphotransferase gene (SEQ ID NO:37) (labeled as "Y. lipolytica CPT1 cDNA" in Figure; Intl. App. Pub. No. WO 2006/052870);<br>• Aco: Aco terminator sequence from *Yarrowia Aco* gene (GenBank Accession No. AJ001300) |

(continued)

| RE Sites And Nucleotides Within SEQ ID NO:131 | Description Of Fragment And Chimeric Gene Components |
|---|---|
| *Pme*I/*Swa*I (10924-13561) | FBAIN::EgD8M::Lip1 comprising:<br>• FBAIN: *Yarrowia lipolytica* FBAIN promoter (U.S. Patent 7,202,356);<br>• EgD8M: Synthetic mutant Δ8 desaturase (SEQ ID NO:59; U.S. Patent 7,709,239), derived from *Euglena gracilis* ("EgD8S"; U.S. Patent 7,256,033) (labeled as "D8S-23" in Figure);<br>• Lip1: Lip1 terminator sequence from *Yarrowia Lip1* gene (GenBank Accession No. Z50020) |
| *Pme*I/*Cla*I (10924-9068) | YAT1::EgD9eS::Lip2, comprising:<br>• YAT1: *Yarrowia lipolytica* YAT1 promoter (labeled as "YAT" in Figure; U.S. Pat. Appl. Pub. No. 2006-0094102-A1);<br>• EgD9eS: codon-optimized Δ9 elongase (SEQ ID NO:45), derived from *Euglena gracilis* (U.S. Patent 7,645,604);<br>• Lip2: Lip2 terminator sequence from *Yarrowia Lip2* gene (GenBank Accession No. AJ012632) |
| *Cla*I/*Eco*RI (9068-6999) | *Yarrowia* Ura3 gene (GenBank Accession No. AJ306421) |
| *Eco*RI/*Pac*I (6999-4141) | GPDIN::EgD5SM::ACO, comprising:<br>• GPDIN: *Yarrowia lipolytica* GPDIN promoter (U.S. Patent 7,459,546);<br>• EgD5SM: Synthetic mutant Δ5 desaturase (SEQ ID NO:71; U.S. Pat. Pub. No. 2010-0075386-A1), derived from *Euglena gracilis* (U.S. Patent 7,678,560) (labeled as "EgD5S-HPGS" in Figure);<br>• Aco: Aco terminator sequence from *Yarrowia Aco* gene (GenBank Accession No. AJ001300) |

The pZKL2-5mB89C plasmid was digested with *Asc*I/*Sph*I, and then used for transformation of strain Y8069U3 according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced approximately 38-44% EPA of TFAs. Seven strains (i.e., #1, #39, #49, #62, #70, #85 and #92) that produced about 44.7%, 45.2%, 45.4%, 44.8%, 46.1%, 48.6% and 45.9% EPA of TFAs were designated as strains Y8151, Y8152, Y8153, Y8154, Y8155, Y8156 and Y8157, respectively. Knockout of the Lip2 gene was not confirmed in these EPA strains.

The final genotype of strains Y8151, Y8152, Y8153, Y8154, Y8155, Y8156 and Y8157 with respect to wildtype *Yarrowia lipolytica* ATCC #20362 was *Ura*+, *Pex3*-, *unknown 1-, unknown 2-, unknown 3*-, *unknown 4*-, *unknown 5-, Leu*+, *Lys*+, YAT1::ME3S::Pex16, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::FmD12::Pex20, YAT1::FmD12::Oct, EXP1::EgD5M::Pex16, YAT1::EaD5SM::Oct, FBAIN::EgD5SM::Pex20, GPDIN::EgD5SM::Aco, FBAINm::PaD17::Aco, EXP1::PaD17::Pex16, YAT1::PaD17S::Lip1, YAT1::YlCPT::Aco.

<u>Generation Of Strain Y8154U1 (*Ura3*-)</u>

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8154, in a manner similar to that described for pZKUM transformation of strain Y8006 (*supra*). A total of 8 transformants were grown and identified to possess a Ura- phenotype.

GC analyses showed that there was 23.1 % EPA of TFAs in the pZKUM-transformant strain #7. This strain was designated as strain Y8154U1.

Generation Of Strain Y8269 To Produce About 45.3% EPA Of TFAs

Construct pZKL1-2SR9G85 (FIG. 6A; SEQ ID NO:132) was generated to integrate one DGLA synthase, one Δ12 desaturase gene and one Δ5 desaturase gene into the *Lip1* loci (GenBank Accession No. Z50020) of strain Y8154U1 to thereby enable higher level production of EPA. A DGLA synthase is a multizyme comprising a Δ9 elongase linked to a Δ8 desaturase.

The pZKL1-2SR9G85 plasmid contained the following components:

Table 8: Description of Plasmid pZKL1-2SR9G85 (SEQ ID NO:132)

| RE Sites And Nucleotides Within SEQ ID NO:132 | Description Of Fragment And Chimeric Gene Components |
|---|---|
| *Asc*I/*Bs*WI (4189-3373) | 809 bp 5' portion of *Yarrowia Lip1* gene (labeled as "Lip1-5'N" in Figure; GenBank Accession No. Z50020) |
| *Pac*I/*Sph*I (7666-6879) | 763 bp 3' portion of *Yarrowia Lip1* gene (labeled as "Lip1.3N" in Figure; GenBank Accession No. Z50020) |
| *Cla*I/*Swa*I (1-3217) | YAT1::E389D9eS/EgD8M::Lip1, comprising:<br>• YAT1: *Yarrowia lipolytica* YAT1 promoter (labeled as "YAT" in Figure; U.S. Pat. Appl. Pub. No. 2006-0094102-A1);<br>• E389D9eS/EgD8M: gene fusion comprising a codon-optimized Δ9 elongase derived from *Eutreptiella* sp. CCMP389 ("E389D9eS"), a linker, and the synthetic mutant Δ8 desaturase derived from *Euglena gracilis* ("EgD8M") (SEQ ID NO:12) (labeled as individually as "E389S", "Linker" and "EgD8M" in Figure; U.S. Pat. Appl. Pub. No. 2008-0254191-A1);<br>• Lip1: Lip1 terminator sequence from *Yarrowia Lip1* gene (GenBank Accession No. Z50020) |
| *Sal*I/*Cla*I (11982-1) | GPM::EgD5SM::Oct comprising:<br>• GPM: *Yarrowia lipolytica* GPM promoter (labeled as "GPML" in Figure; U.S. Patent 7,202,356);<br>• EgD5SM: Synthetic mutant Δ5 desaturase (SEQ ID NO:71; U.S. Pat. Pub. No. 2010-0075386-A1), derived from *Euglena gracilis* (U.S. Patent 7,678,560) (labeled as "ED5S" in Figure);<br>• OCT: OCT terminator sequence of *Yarrowia OCT* gene (GenBank Accession No. X69988) |
| *Sal*I/*Eco*RI (11982-10363) | *Yarrowia* Ura3 gene (GenBank Accession No. AJ306421) |
| *Eco*RI/*Pac*I (10363-7666) | EXP1::FmD12S::ACO, comprising:<br>• EXP1: *Yarrowia lipolytica* export protein (EXP1) promoter (labeled as "Exp" in Figure; Intl. App. Pub. No. WO 2006/052870);<br>• FmD12S: codon-optimized Δ12 elongase (SEQ ID NO:93), derived from *Fusarium moniliforme* (labeled as "FD12S" in Figure; U.S. Patent 7,504,259);<br>• Aco: Aco terminator sequence from *Yarrowia Aco* gene (GenBank Accession No. AJ001300) |

The pZKL1-2SR9G85 plasmid was digested with *Asc*I/*Sph*I, and then used for transformation of strain Y8154U1 according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced 40-44.5% EPA of total lipids. Five strains (i.e., #44, #46, #47, #66 and #87) that produced about 44.8%, 45.3%, 47%, 44.6% and 44.7% EPA of TFAs were designated as Y8268, Y8269, Y8270, Y8271 and Y8272, respectively. Knockout of the *Lip1* loci (GenBank Accession No. Z50020) was not confirmed in these EPA strains.

The final genotype of strains Y8268, Y8269, Y8270, Y8271 and Y8272 with respect to wildtype *Yarrowia lipolytica* ATCC #20362 was Ura+, *Pex3*-, *unknown 1*-, *unknown 2*-, *unknown 3*-, *unknown 4*-, *unknown 5*-, *unknown6*-, YAT1::ME3S::Pex16, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, YAT1::E389D9eS/EgD8M::Lip1, GPD::FmD12::Pex20, YAT1::FmD12::Oct, EXP1::FmD12S::Aco, EXP1::EgD5M::Pex16, YAT1::EaD5SM::Oct, FBAIN::EgD5SM::Pex20, GPDIN::EgD5SM::Aco, GPM::EgD5SM::Oct, FBAINm::PaD17::Aco, EXP1::PaD17::Pex16, YAT1::PaD17S::Lip1, YAT1::YlCPT::Aco.

Generation Of Strain Y8269U (*Ura3*-)

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8269, in a manner similar to that described for pZKUM transformation of strain Y8006 (*supra*). A total of 8 transformants were grown and identified to possess a *Ura*- phenotype.

GC analyses showed that there were 23.0%, 23.1% and 24.2% EPA of TFAs in pZKUM-transformant strains #2, #3 and #5, respectively. These strains were designated as strains Y8269U1, Y8269U2 and Y8269U3, respectively (collectively, Y8269U).

Generation Of Strain Y8406 And Strain Y8412 To Produce About 51.2% EPA And 55.8% EPA Of TFAs

Construct pZSCP-Ma83 (FIG. 6B; SEQ ID NO:133) was generated to integrate one Δ8 desaturase gene, one $C_{16/18}$ elongase gene and one malonyl-CoA synthetase gene into the *SCP2* loci (GenBank Accession No. XM_503410) of strain Y8269U1 to thereby enable higher level production of EPA. The pZSCP-Ma83 plasmid contained the following components:

Table 9: Description of Plasmid pZSCP-Ma83 (SEQ ID NO:133)

| RE Sites And Nucleotides Within SEQ ID NO:133 | Description Of Fragment And Chimeric Gene Components |
|---|---|
| *Bsi*WI/*Asc*I (1-1328) | 1327 bp 3' portion of *Yarrowia SCP2* gene (labeled as "SCP2-3"' in Figure; GenBank Accession No. XM_503410) |
| *Sph*I/*Pac*I (4036-5816) | 1780 bp 5' portion of *Yarrowia SCP2* gene (labeled as "SCP2-5"' in Figure; GenBank Accession No. XM_503410) |
| *Swa*I/*Bsi*WI (12994-1) | GPD::ME3S::Pex20, comprising:<br>• GPD: *Yarrowia lipolytica* GPD promoter (U.S. Patent 7,259,255);<br>• ME3S: codon-optimized $C_{16/18}$ elongase gene (SEQ ID NO:97), derived from M. *alpina* (U.S. Patent 7,470,532);<br>• Pex20: Pex20 terminator sequence from *Yarrowia Pex20* gene (GenBank Accession No. AF054613) |
| *Pme*I/*Swa*I (10409-12994) | YAT1::MCS::Lip1 comprising:<br>• YAT1: *Yarrowia lipolytica* YAT1 promoter (labeled as "YAT" in Figure; U.S. Pat. Appl. Pub. No. 2006/0094102-A1);<br>• MCS: codon-optimized malonyl-CoA synthetase gene (SEQ ID NO:41), derived from *Rhizobium leguminosarum* bv. viciae 3841 (U.S. Pat. Appl. Pub. No. 2010-0159558-A1);<br>• Lip1: Lip1 terminator sequence from *Yarrowia Lip1* gene (GenBank Accession No. Z50020) |

(continued)

| RE Sites And Nucleotides Within SEQ ID NO:133 | Description Of Fragment And Chimeric Gene Components |
|---|---|
| *Cla*I/*Pme*I (7917-10409) | GPD::EaD8S::Pex16 comprising:<br>• GPD: *Yarrowia lipolytica* GPD promoter (U.S. Patent 7,259,255);<br>• EaD8S: codon-optimized Δ8 desaturase gene (SEQ ID NO:63), derived from *Euglena anabaena* (U.S. Pat. Appl. Pub. No. 2008-0254521-A1);<br>• Pex16: Pex16 terminator sequence from *Yarrowia Pex16* gene (GenBank Accession No. U75433) |
| *Sal*I/*Eco*RI (7467-5848) | *Yarrowia* Ura3 gene (GenBank Accession No. AJ306421) |

The pZSCP-Ma83 plasmid was digested with *Asc*I/*Sph*I, and then used for transformation of strains Y8269U1, Y8269U2 and Y8269U3, separately, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

A total of 96 strains resulting from each pZSCP-Ma83 transformation (i.e., into Y8269U1, Y8269U2 and Y8269U3) were analyzed by GC. Most of the selected 288 strains produced 43-47% EPA of TFAs. Seven strains of Y8269U1 transformed with pZSCP-Ma83 (i.e., #59, #61, #65, #67, #70, #81 and #94) that produced about 51.3%, 47.9%, 50.8%, 48%, 47.8%, 47.8% and 47.8% EPA of TFAs were designated as strains Y8404, Y8405, Y8406, Y8407, Y8408, Y8409 and Y8410, respectively. Three strains of Y8269U2 transformed with pZSCP-Ma83 (i.e., #4, #13 and #17) that produced about 48.8%, 50.8%, and 49.3% EPA of TFAs were designated as Y8411, Y8412 and Y8413, respectively. And, two strains of Y8269U3 transformed with pZSCP-Ma83 (i.e., #2 and #16) that produced about 49.3% and 53.5% EPA of TFAs were designated as Y8414 and Y8415, respectively.

Knockout of the *SCP2* loci (GenBank Accession No. XM_503410) in strains Y8404, Y8405, Y8406, Y8407, Y8408, Y8409, Y8410, Y8411, Y8412, Y8413, Y8414 and Y8415 was not confirmed in any of these EPA strains, produced by transformation with pZSCP-Ma83.

The final genotype of strains Y8404, Y8405, Y8406, Y8407, Y8408, Y8409, Y8410, Y8411, Y8412, Y8413, Y8414 and Y8415 with respect to wildtype *Yarrowia lipolytica* ATCC #20362 was *Ura+, Pex3-, unknown 1-, unknown 2-*, *unknown 3-*, *unknown 4-*, *unknown 5-*, *unknown6-*, *unknown 7-*, YAT1::ME3S::Pex16, GPD::ME3S::Pex20, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16, YAT1::E389D9eS/EgD8M::Lip1, GPD::FmD12::Pex20, YAT1::FmD12::Oct, EXP1::FmD12S::Aco, EXP1::EgD5M::Pex16, YAT1::EaD5SM::Oct, FBAIN::EgD5SM::Pex20, GPDIN::EgD5SM::Aco, GPM::EgD5SM::Oct, FBAINm::PaD17::Aco, EXP1::PaD17::Pex16, YAT1::PaD17S::Lip1, YAT1::YlCPT::Aco, YAT1::MCS::Lip1.

*Yarrowia lipolytica* strain Y8406 was deposited with the American Type Culture Collection on May 14, 2009 and bears the designation ATCC PTA-10025. *Yarrowia lipolytica* strain Y8412 was deposited with the American Type Culture Collection on May 14, 2009 and bears the designation ATCC PTA-10026.

Analysis Of Total Lipid Content And Pomposition By Flask Assay

Cells from YPD plates of strains Y8404, Y8405, Y8406, Y8407, Y8408, Y8409, Y8410, Y8411, Y8412, Y8413, Y8414 and Y8415 were grown and analyzed for total lipid content and composition, according to the General Methods. Table 10 summarizes the total dry cell weight of the cells ["DCW'], the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

Table 10: Total Lipid Content And Composition In *Yarrowia* Strains Y8404, Y8405, Y8406, Y8407, Y8408, Y8409, Y8410, Y8411, Y8412, Y8413, Y8414 And Y8415 By Flask Assay

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8404 | 4.1 | 27.3 | 2.8 | 0.8 | 1.8 | 5.1 | 20.4 | 2.1 | 2.9 | 2.5 | 0.6 | 0.8 | 2.4 | 51.1 | 6.3 | 14.0 |
| Y8405 | 3.9 | 29.6 | 2.7 | 0.5 | 2.9 | 5.7 | 20.5 | 2.8 | 2.7 | 2.1 | 0.5 | 0.7 | 2.0 | 51.4 | 5.1 | 15.2 |
| **Y8406** | **4.0** | **30.7** | **2.6** | **0.5** | **2.9** | **5.7** | **20.3** | **2.8** | **2.8** | **2.1** | **0.5** | **0.8** | **2.1** | **51.2** | **5.4** | **15.7** |
| Y8407 | 4.0 | 29.4 | 2.6 | 0.5 | 3.0 | 5.6 | 20.5 | 2.8 | 2.7 | 2.1 | 0.4 | 0.7 | 2.1 | 51.5 | 5.1 | 15.2 |
| Y8408 | 4.1 | 29.8 | 2.9 | 0.6 | 2.7 | 5.7 | 20.2 | 2.8 | 2.6 | 2.1 | 0.5 | 0.9 | 2.1 | 51.2 | 5.5 | 15.3 |
| Y8409 | 3.9 | 30.8 | 2.8 | 0.5 | 2.9 | 5.7 | 20.6 | 2.7 | 2.7 | 2.1 | 0.5 | 0.8 | 2.1 | 51.0 | 5.2 | 15.7 |
| Y8410 | 4.0 | 31.8 | 2.7 | 0.5 | 3.0 | 5.7 | 20.5 | 2.9 | 2.7 | 2.1 | 0.5 | 0.7 | 2.1 | 50.9 | 5.3 | 16.2 |
| Y8411 | 3.6 | 30.5 | 2.7 | 0.3 | 3.3 | 5.1 | 19.9 | 2.6 | 2.4 | 2.0 | 0.5 | 0.6 | 1.8 | 52.9 | 5.7 | 16.1 |
| **Y8412** | **3.2** | **27.0** | **2.5** | **0.4** | **2.6** | **4.3** | **19.0** | **2.4** | **2.2** | **2.0** | **0.5** | **0.6** | **1.9** | **55.8** | **5.6** | **15.1** |
| Y8413 | 2.9 | 27.2 | 3.1 | 0.4 | 2.6 | 5.4 | 19.9 | 2.2 | 2.8 | 2.0 | 0.5 | 0.7 | 1.8 | 52.4 | 5.9 | 14.2 |
| Y8414 | 3.7 | 27.1 | 2.5 | 0.7 | 2.3 | 6.0 | 19.9 | 1.6 | 3.4 | 3.4 | 0.6 | 0.6 | 3.1 | 49.4 | 6.1 | 13.4 |
| Y8415 | 3.6 | 25.9 | 1.4 | 0.3 | 1.9 | 4.5 | 16.0 | 1.3 | 2.7 | 2.9 | 0.5 | 0.6 | 2.5 | 59.0 | 6.1 | 15.3 |

EXAMPLE 4

Generation Of *Yarrowia lipolytica* Strain Y8647 To Produce About 53.6% EPA Of Total Fatty Acids ["TFAs"] With 37.6% Total Lipid Content

The present Example describes the construction of strain Y8647, derived from *Yarrowia lipolytica* ATCC #20362, capable of producing about 53.6% EPA relative to the total lipids with 37.6% total lipid content ["TFAs % DCW"] via expression of a Δ9 elongase/Δ8 desaturase pathway. The development of strain Y8647 (FIG. 2) required the construction of strains Y2224, Y4001, Y4001 U, Y4036, Y4036U and L135 (described in Example 1), strains L135U9 and Y8002 (described in Example 2), strains Y8006U6, Y8069, Y8069U, Y8154, Y8154U, Y8269 and Y8269U (described in Example 3) and strain Y8412U6.

Generation Of Strain Y8412U6 (*Ura3*-)

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8412 (Example 3) in a manner similar to that described for pZKUM transformation of strain Y8006 (Example 3). A total of 8 transformants were grown and identified to possess a *Ura*- phenotype.

GC analyses showed that there were 25.9% and 26.9% EPA of TFAs in pZKUM-transformant strains #4 and #6, respectively. These two strains were designated as strains Y8412U6 and Y8412U8, respectively (collectively, Y8412U).

Generation Of Strain Y8647

Construct pZKL4-398F2 (FIG. 7A; SEQ ID NO:134) was generated to integrate one $C_{16/18}$ elongase gene, one DGLA synthase, and one Δ12 desaturase gene into the *Yarrowia* lipase-like locus (designated as *Lip4,* GenBank Accession No. XM_503825) of strain Y8412U6 to thereby enable higher level production of EPA. The pZKL4-398F2 plasmid contained the following components:

Table 11: Description of Plasmid pZKL4-398F2 (SEQ ID NO:134)

| RE Sites And Nucleotides Within SEQ ID NO:134 | Description Of Fragment And Chimeric Gene Components |
|---|---|
| *Asc*I / Bs*i*WI (11164-10412) | 745 bp 5' portion of *Yarrowia Lipase 4* locus (labeled as "Lip4" in Figure; GenBank Accession No. XM 503825) |
| *Pac*I / *Sph*I (1-13872) | 782 bp 3' portion of *Yarrowia Lipase 4* locus (labeled as "Lip4-3"' in Figure; GenBank Accession No. XM_503825) |
| *EcoR*I/*Pac*I (2877-1) | GPDIN::FmD12::Pex16, comprising:<br>• GPDIN: *Yarrowia lipolytica* GPDIN promoter (U.S. Patent 7,459,546);<br>• FmD12: *Fusarium moniliforme* Δ12 desaturase (SEQ ID NO:91) (labeled as "F.D12" in Figure; U.S. Patent 7,504,259);<br>• Pex16: Pex16 terminator sequence from *Yarrowia Pex16* gene (GenBank Accession No. U75433) |
| *Pme*I/*Swa*I (8361-10256) | YAT1::ME3S::Lip1 comprising:<br>• YAT1: *Yarrowia lipolytica* YAT1 promoter (labeled as "YAT" in Figure; U.S. Pat. Appl. Pub. No. 2006-0094102-A1);<br>• ME3S: codon-optimized $C_{16/18}$ elongase gene (SEQ ID NO:97), derived from *M. alpina* (U.S. Patent 7,470,532);<br>• Lip1: Lip1 terminator sequence from *Yarrowia Lip1* gene (GenBank Accession No. Z50020) |

(continued)

| RE Sites And Nucleotides Within SEQ ID NO:134 | Description Of Fragment And Chimeric Gene Components |
|---|---|
| SwaI /*Cla*I (8325-4946) | FBAINm::EaD9eS/EaD8S::Lip2 comprising:<br>• FBAINm: *Yarrovvia lipolytica* FBAINm promoter (U.S. Patent 7,202,356);<br>• EaD9eS/EaD8S: gene fusion comprising a codon-optimized Δ9 elongase derived from *Euglena anabaena* ("EaD9eS"), a linker, and a codon-optimized Δ8 desaturase derived from *Euglena anabaena* ("EaD8S") (SEQ ID NO:63) (labeled as individually as "EaD9E9S", "Linker" and "EaD8S" in Figure; U.S. Pat. Appl. Pub. No. 2008-0254191-A1);<br>• Lip2: Lip2 terminator sequence from *Yarrowia Lip2* gene (GenBank Accession No. AJ012632) |
| *Cla*I/*Eco*RI (49146-2877) | *Yarrowia* Ura3 gene (GenBank Accession No. AJ306421) |

The pZKL4-398F2 plasmid was digested with *Asc*I/*Sph*I, and then used for transformation of strain Y8412U6, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 transformant strains produced 50-52.7% EPA of TFAs. Seven strains (i.e., #31, #35, #38, #41, #60, #61 and #95) that produced about 52.8%, 53.1%, 52.8%, 53.2%, 53.1%, 52.8%, and 52.9% EPA of TFAs were designated as Y8646, Y8647, Y8648, Y8649, Y8650, Y8651 and Y8652, respectively.

Knockout of the *Lip4* locus (GenBank Accession No. XM_503825) in these EPA strains was not confirmed.

The final genotype of strains Y8646, Y8647, Y8648, Y8649, Y8650, Y8651 and Y8652 with respect to wildtype *Yarrowia lipolytica* ATCC #20362 was *Ura+, Pex3-, unknown 1-, unknown 2-, unknown 3-, unknown 4-, unknown 5-, unknown6-, unknown 7-, unknown 8-,* YAT1::ME3S::Pex16, GPD::ME3S::Pex20, YAT1::ME3S::Lip1, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16, YAT1::E389D9eS/EgD8M::Lip1, FBAINm::EaD9eS/EaD8S::Lip2, GPD::FmD12::Pex20, YAT1::FmD12::Oct, EXP1::FmD12S::Aco, GPDIN::FmD12::Pex16, EXP1::EgDSM::Pex16, FBAIN::EgD5SM:Pex20, GPDIN::EgD5SM::Aco, GPM::EgD5SM::Oct, YAT1::EaDSSM::Oct, FBAINm::PaD17::Aco, EXP1::PaD17::Pex16, YAT1::PaD17S::Lip1, YAT1::YICPT::Aco, YAT1::MCS::Lip1.

<u>Analysis Of Total Lipid Content And Composition By Flask Assay</u>

Cells from YPD plates of strains Y8647, Y8648, Y8649 and Y8650 were grown and analyzed for total lipid content and composition, according to the General Methods. Table 12 summarizes the total dry cell weight of the cells ["DCW'], the total lipid content of cells ["TFAs % DCW"], the concentration of each fatty acid as a weight percent of TFAs ["% TFAs"] and the EPA content as a percent of the dry cell weight ["EPA % DCW"]. Fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

Table 12: Total Lipid Content And Composition In *Yarrowia* Strains Y8647, Y8648 Y8649 And Y8650 By Flask Assay

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| **Y8647** | **3.8** | **37.6** | **1.3** | **0.2** | **2.1** | **4.7** | **20.3** | **1.7** | **3.3** | **3.6** | **0.7** | **0.6** | **3.0** | **53.6** | **4.5** | **20.1** |
| Y8648 | 3.5 | 27.8 | 2.3 | 0.3 | 2.7 | 4.3 | 18.6 | 2.3 | 2.1 | 2.2 | 0.6 | 0.6 | 1.9 | 56.7 | 4.9 | 15.7 |
| Y8649 | 3.6 | 27.9 | 2.4 | 0.3 | 2.9 | 3.7 | 18.8 | 2.2 | 2.1 | 2.4 | 0.6 | 0.8 | 2.1 | 55.8 | 5.5 | 15.6 |
| Y8650 | 3.5 | 28.2 | 2.2 | 0.3 | 2.9 | 3.8 | 18.8 | 2.4 | 2.1 | 2.4 | 0.6 | 0.6 | 2.1 | 56.1 | 5.3 | 15.8 |

EXAMPLE 5

Generation Of *Yarrowia lipolytica* Strain Y9028 To Produce About 54.5% EPA Of Total Fatty Acids ("TFAs"] With 39.6% Total Lipid Content

The present Example describes the construction of strain Y9028, derived from *Yarrowia lipolytica* ATCC #20362, capable of producing about 54.5% EPA relative to the total lipids with 39.6% total lipid content ["TFAs % DCW"] via expression of a Δ9 elongase/Δ8 desaturase pathway. The development of strain Y9028 (FIG. 2) required the construction of strains Y2224, Y4001, Y4001 U, Y4036, Y4036U and L135 (described in Example 1), strains L135U9 and Y8002 (described in Example 2), strains Y8006U6, Y8069, Y8069U, Y8154, Y8154U, Y8269 and Y8269U (described in Example 3), strains Y8412U6 and Y8647 (described in Example 4) and strain Y8467U.

Generation Of Strain Y8647U *(Ura3-)*

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8647 (Example 4) in a manner similar to that described for pZKUM transformation of strain Y8006 (Example 3). A total of 12 transformants were grown and identified to possess a *Ura*- phenotype.

GC analyses showed that there were 30.2%, 29.2%, 28.1% and 29.9% EPA of TFAs in pZKUM-transformant strains #1, #3, #4 and #12, respectively. These four strains were designated as strains Y8647U1, Y8647U2, Y8647U3, and Y8647U6, respectively (collectively, Y8647U).

Generation Of Strain Y9028

Construct pZP2-85m98F (FIG. 7B; SEQ ID NO:135) was generated to integrate one Δ8 desaturase gene, one DGLA synthase and one Δ5 desaturase gene into the *Yarrowia* Pox2 locus (GenBank Accession No. AJ001300) of strain Y8647U3 to thereby enable higher level production of EPA. The pZP2-85m98F plasmid contained the following components:

Table 13: Description of Plasmid pZP2-85m98F (SEQ ID NO:135)

| RE Sites And Nucleotides Within SEQ ID NO:135 | Description Of Fragment And Chimeric Gene Components |
|---|---|
| *Asc*I/*Bsi*WI (5986-5176) | 810 bp 5' portion of *Yarrowia Pox2* gene (GenBank Accession No. AJ001300) |
| *Pac*I/*Sph*I (9349-8694) | 655 bp 3' portion of *Yarrowia Pox2* gene (GenBank Accession No. AJ001300) |
| *Pme*I/*Swa*I (2493-5020) | EXP1::EgD5SM::Lip1, comprising:<br>• EXP1: *Yarrowia lipolytica* export protein (EXP1) promoter (labeled as "EXP" in Figure; Intl. App. Pub. No. WO 2006/052870);<br>• EgD5SM: Synthetic mutant Δ5 desaturase (SEQ ID NO:71; U.S. Pat. Pub. No. 2010-0075386-A1), derived from *Euglena gracilis* (U.S. Patent 7,678,560);<br>• Lip1: Lip1 terminator sequence from *Yarrowia Lip1* gene (GenBank Accession No. Z50020) |
| *Cla*I/*Pme*I (1-2493) | GPD::EaD8S::Pex16, comprising:<br>• GPD: *Yarrowia lipolytica* GPD promoter (U.S. Patent 7,259,255);<br>• EaD8S: codon-optimized Δ8 desaturase gene (SEQ ID NO:63), derived from *Euglena anabaena* (U.S. Pat. Appl. Pub. No. 2008-0254521-A1);<br>• Pex16: Pex16 terminator sequence from *Yarrowia Pex16* gene (GenBank Accession No. U75433) |
| *Sal*I/*Eco*RI (14170 -12551) | *Yarrowia* Ura3 gene (GenBank Accession No. AJ306421) |

(continued)

| RE Sites And Nucleotides Within SEQ ID NO:135 | Description Of Fragment And Chimeric Gene Components |
|---|---|
| *Eco*RI/*Pac*I (12551-9349) | YAT1::EgD9eS/EgD8M::Aco, comprising: • YAT1: *Yarrowia lipolytica* YAT1 promoter (labeled as "YAT" in Figure; U.S. Pat. Appl. Pub. No. 2006/0094102-A1); • EgD9eS/EgD8M: gene fusion comprising a codon-optimized Δ9 elongase derived from *Euglena gracilis* ("EgD9eS"), a linker, and the synthetic mutant Δ8 desaturase derived from *Euglena gracilis* ("EgD8M") (SEQ ID NO:8) (labeled as individually as "EgD9eS", "Linker" and "EgD8M" in Figure; U.S. Pat. Appl. Pub. No. 2008-0254191-A1); • Aco: Aco terminator sequence from *Yarrowia Aco* gene (GenBank Accession No. AJ001300) |

The pZP2-85m98F plasmid was digested with *Asc*I/*Sph*I, and then used for transformation of strains of Y8647U1, Y8647U2, Y8647U3 and Y8647U6, individually, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 48 strains of Y8647U1 transformed with pZP2-85m98F produced 49-52% EPA of TFAs. Two strains (i.e., #30 and #31) that produced about 52.6% and 52.1% EPA of TFAs were designated as Y9024 and Y9025, respectively.

Most of the selected 60 strains of Y8647U2 transformed with pZP2-85m98F produced 49-51.9% EPA of TFAs. Strain #6 produced about 52% EPA of TFAs and was designated as Y9026.

Most of the selected 60 strains of Y8647U3 transformed with pZP2-85m98F produced 50-52.2% EPA of TFAs. Six strains (i.e., #5, #6, #14, #15, #20 and #34) that produced about 53.2%, 53.7%, 54.0%, 52.9%, 53.4% and 52.3% EPA of TFAs were designated as Y9027, Y9028, Y9029, Y9030, Y9031 and Y9032, respectively.

Similarly, GC analyses showed that most of the selected 48 strains of Y8647U6 transformed with pZP2-85m98F produced 50-52.1% EPA of TFAs. Two strains (i.e., #27 and #44) that produced about 52.2% and 52.8% EPA of TFAs were designated as Y9033 and Y9034, respectively.

Knockout of the Pox2 locus (GenBank Accession No. AJ001300) in strains Y9024, Y9025, Y9026, Y9027, Y9028, Y9029, Y9030, Y9031, Y9032, Y9033 and Y9034 was not confirmed in any of these EPA strains, produced by transformation with pZP2-85m98F.

The final genotype of these strains with respect to wildtype *Yarrowia lipolytica* ATCC #20362 was *Ura+, Pex3-, unknown 1-, unknown 2-, unknown 3-, unknown 4-, unknown 5-, unknown6-, unknown 7-*, *unknown 8-, unknown9-,* YAT1::ME3S::Pex16, GPD::ME3S::Pex20, YAT1::ME3S::Lip1, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16 (2 copies),

YAT1::E389D9eS/EgD8M::Lip1, YAT1::EgD9eS/EgD8M::Aco, FBAINm::EaD9eS/EaD8S::Lip2, GPD::FmD12::Pex20, YAT1::FmD12::Oct, EXP1::FmD12S::Aco, GPDIN::FmD12::Pex16, EXP1::EgDSM::Pex16, FBAIN::EgD5SM::;Pex20, GPDIN::EgD5SM::Aco, GPM::EgD5SM::Oct, EXP1::EgD5SM::Lip1, YAT1::EaD5SM::Oct, FBAINm::PaD17::Aco, EXP1::PaD17::Pex16, YAT1::PaD17S::Lip1, YAT1::YlCPT::Aco, YAT1::MCS::Lip1.

Analysis Of Total Lipid Content And Composition By Flask Assay

Cells from YPD plates of strains Y9028, Y9029 and Y9031 were grown and analyzed for total lipid content and composition, according to the General Methods.

Table 14 below summarizes the total dry cell weight of the cells ["DCW'], the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

Table 14: Total Lipid Content And Composition In *Yarrowia* Strains Y9028, Y9029 and Y9031 By Flask Assay

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| **Y9028** | **3.3** | **39.6** | **1.3** | **0.2** | **2.1** | **4.4** | **19.8** | **1.7** | **3.2** | **2.5** | **0.8** | **0.7** | **1.9** | **54.5** | **6.1** | **21.6** |
| Y9029 | 3.2 | 38.4 | 1.3 | 0.3 | 1.7 | 4.4 | 19.8 | 1.5 | 3.2 | 3.3 | 0.9 | 0.7 | 2.4 | 53.8 | 6.0 | 20.7 |
| Y9031 | 3.3 | 38.6 | 1.3 | 0.3 | 1.8 | 4.7 | 20.1 | 1.7 | 3.2 | 3.2 | 0.9 | 0.8 | 2.6 | 52.3 | 6.3 | 20.2 |

EXAMPLE 6

Generation Of *Yarrowia lipolytica* Strains Y9481 And Y9502, Producing At Least About 57% EPA Of Total Fatty Acids ["TFAs"] With At Least About 35% Total Lipid Content

The present Example describes the construction of strains Y9481 and Y9502, derived from *Yarrowia lipolytica* ATCC #20362 and expressing a Δ9 elongase/Δ8 desaturase pathway. Strain Y9481 is capable of producing about 60.9% EPA relative to the total lipids with 35% total lipid content ["TFAs % DCW"], while strain Y9502 is capable of producing about 57% EPA relative to the total lipids with 37.1 % TFAs % DCW.

The development of strains Y9481 and Y9502 (FIG. 2) required the construction of strains Y2224, Y4001, Y4001 U, Y4036, Y4036U and L135 (described in Example 1), strains L135U9 and Y8002 (described in Example 2), strains Y8006U6, Y8069, Y8069U, Y8154, Y8154U, Y8269 and Y8269U (described in Example 3), strains Y8412U6 and Y8647 (described in Example 4), strains Y8467U and Y9028 (described in Example 5) and strain Y9028U.

Generation Of Strain Y9028U *(Ura3-)*

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y9028 (Example 5) in a manner similar to that described for pZKUM transformation of strain Y8006 (Example 3). A total of 8 transformants were grown and identified to possess a *Ura-* phenotype.

GC analyses showed that there were 24.1%, 24.9%, 24.5% and 24.5% EPA of TFAs in pZKUM-transformant strains #1, #3, #4, and #5, respectively. These four strains were designated as strains Y9028U1, Y9028U2, Y9028U3, and Y9028U4, respectively (collectively, Y9028U).

Components Of Integration Vector pZK16-ML8N

Construct pZK16-ML8N (FIG. 8A; SEQ ID NO:136) was generated to integrate one Δ8 desaturase gene, one malonyl-CoA synthetase gene, and one lysophosphatidic acid acyltransferase gene ["LPAAT"] into the *Yarrowia* YALI0B14795p locus (GenBank Accession No. XM_500900) of strain Y9028U2. The pZK16-ML8N plasmid contained the following components:

Table 15: Description of Plasmid pZK16-ML8N (SEQ ID NO:136)

| RE Sites And Nucleotides Within SEQ ID NO:136 | Description Of Fragment And Chimeric Gene Components |
|---|---|
| *Asc*I/*Bsi*WI (1905-1) | 1904 bp 5' portion of YALI0B14795p locus (GenBank Accession No. XM_500900, labeled as "Y8716-5"' in Figure) |
| *Pac*I/*Sph*I (6414-4613) | 1801 bp 3' portion of YALI0B14795p locus (GenBank Accession No. XM_500900, labeled as "Y8716-3"' in Figure) |
| *Swa*I/*Bsi*WI (12920 - 1) | YAT1::EgD8M::Pex20, comprising:<br>• YAT1: *Yarrowia lipolytica* YAT1 promoter (labeled as "YAT" in Figure; U.S. Pat. Appl. Pub. No. 2006-0094102-A1);<br>• EgD8M: Synthetic mutant Δ8 desaturase (SEQ ID NO:59; U.S. Patent 7,709,239), derived from *Euglena gracilis* ("EgD8S"; U.S. Patent 7,256,033);<br>• Pex20: Pex20 terminator sequence from *Yarrowia Pex20* gene (GenBank Accession No. AF054613) |
| *Pme*I/*Swa*I (10534-12920) | FBA::MCS::Lip1, comprising:<br>• FBA: *Yarrowia lipolytica* FBA promoter (U.S. Patent 7,202,356);<br>• MCS: codon-optimized malonyl-CoA synthetase gene (SEQ ID NO:41), derived from *Rhizobium leguminosarum* bv. viciae 3841 (U.S. Pat. Appl. Pub. No. 2010-0159558-A1);<br>• Lip1: Lip1 terminator sequence from *Yarrowia Lip1* gene (GenBank Accession No. Z50020) |

(continued)

| RE Sites And Nucleotides Within SEQ ID NO:136 | Description Of Fragment And Chimeric Gene Components |
|---|---|
| *Cla*I/*Pme*I (8515-10534) | YAT1::MaLPAAT1S::Pex16, comprising:<br>• YAT1: *Yarrowia lipolytica* YAT1 promoter (labeled as "YAT" in Figure; U.S. Pat. Appl. Pub. No. 2006-0094102-A1);<br>• MaLPMT1S: codon-optimized lysophosphatidic acid acyltransferase gene (SEQ ID NO:35), derived from *Mortierella alpina* (U.S. Pat. Appl. Pub. No. 2006-0115881-A1; U.S. Pat. Appl. Pub. No. 2009-0325265-A1);<br>• Pex16: Pex16 terminator sequence from *Yarrowia Pex16* gene (GenBank Accession No. U75433) |
| *Sal*I/*Eco*RI (8065 - 6446) | *Yarrowia* Ura3 gene (GenBank Accession No. AJ306421) |

Components Of Integration Vector pZK16-ML

Construct pZK16-ML (FIG. 8B; SEQ ID NO:137) was generated to integrate one malonyl-CoA synthetase gene and one lysophosphatidic acid acyltransferase gene ["LPAAT"] into the *Yarrowia* YALI0B14795p locus (GenBank Accession No. XM_500900) of strain Y9028U2. The components of the pZK16-ML plasmid are identical to those of pZK16-ML8N *(supra);* however, the chimeric YAT1::EgD8M::Pex20 gene of pZK16-ML8N is lacking.

Generation Of Strains Y9481 And Y9502

The pZK16-ML8N plasmid and pZK16-ML plasmid were each individually digested with *Asc*I/*Sph*I, and then used separately for transformation of strain Y9028U2, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains of Y9028U2 with pZK16-ML8N produced 50-55.4% EPA of TFAs. Fifteen strains (i.e., #8, #18, #21, #24, #29, #48, #60, #66, #68, #75, #76, #78, #90, #95 and #96) that produced about 58.1%, 61.4%, 56.2%, 58.1%, 57.5%, 57.0%, 55.9%, 57.6%, 57.8%, 55.5%, 57.6%, 58.1%, 57.1%, 56.2% and 58.6% EPA of TFAs were designated as Y9472, Y9473, Y9474, Y9475, Y9476, Y9477, Y9478, Y9479, Y9480, Y9481, Y9482, Y9483, Y9484, Y9485 and Y9486, respectively.

The final genotype of these pZK16-ML8N transformant strains with respect to wildtype *Yarrowia lipolytica* ATCC #20362 was *Ura+, Pex3-, unknown 1-, unknown 2-, unknown 3-, unknown 4-, unknown 5-, unknown6-, unknown 7-, unknown 8-, unknown9-, unknown 10-,* YAT1::ME3S::Pex16, GPD::ME3S::Pex20, YAT1::ME3S::Lip1, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, YAT1::EgD8M::Pex20, GPD::EaD8S::Pex16 (2 copies), YAT1::E389D9eS/EgD8M::Lip1, YAT1::EgD9eS/EgD8M::Aco, FBAINm::EaD9eS/EaD8S::Lip2, GPD::FmD12::Pex20, YAT1::FmD12::Oct, EXP1::FmD12S::Aco, GPDIN::FmD12::Pex16, EXP1::EgD5M::Pex16, FBAIN::EgD5SM::Pex20, GPDIN::EgD5SM::Aco, GPM::EgD5SM::Oct, EXP1::EgD5SM::Lip1, YAT1::EaD5SM::Oct, FBAINm::PaD17::Aco, EXP1::PaD17::Pex16, YAT1 ::PaD17S::Lip1, YAT1::YICPT::Aco, YAT1::MCS::Lip1, FBA::MCS::Lip1, YAT1::MaLPAAT1S::Pex16.

Similarly, GC analyses showed that most of the selected 96 strains of Y9028U2 with pZK16-ML produced 51-55.5% EPA of TFAs. Sixteen strains (i.e., #4, #8, #15, #16, #39, #44, #46, #63, #66, #80, #85, #86, #88, #89, #90 and #96) that produced about 56.5%, 57.4%, 56.8%, 57.0%, 56.4%, 57.3%, 58.2%, 55.6%, 57.8%, 55.6%, 57.6%, 56.8%, 55.8%, 56.4%, 56.1% and 57% EPA of TFAs were designated as Y9496, Y9497, Y9498, Y9499, Y9500, Y9501, Y9502, Y9503, Y9504, Y9505, Y9506, Y9507, Y9508, Y9509, Y9510 and Y9511, respectively.

The final genotype of these pZK16-ML transformant strains with respect to wildtype *Yarrowia lipolytica* ATCC #20362 was *Ura+, Pex3-, unknown 1-, unknown* 2-, *unknown 3-, unknown 4-, unknown 5-, unknown6-, unknown 7-, unknown 8-, unknown9-, unknown 10-,* YAT1::ME3S::Pex16, GPD::ME3S::Pex20, YAT1::ME3S::Lip1, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16 (2 copies), YAT1::E389D9eS/EgD8M::Lip1, YAT1::EgD9eS/EgD8M::Aco, FBAINm::EaD9eS/EaD8S::Lip2, GPD::FmD12::Pex20, YAT1::FmD12::Oct, EXP1::FmD12S::Aco, GPDIN::FmD12::Pex16, EXP1::EgD5M::Pex16, FBAIN::EgD5SM::Pex20, GPDIN::EgD5SM::Aco, GPM::EgD5SM::Oct, EXP1::EgD5SM::Lip1, YAT1::EaD5SM::Oct, FBAINm::PaD17::Aco, EXP1::PaD17::Pex16, YAT1 ::PaD17S::Lip1, YAT1::YICPT::Aco, YAT1::MCS::Lip1, FBA::MCS::Lip1, YAT1::MaLPAAT1S::Pex16.

Knockout of the YALIOB14795p locus (GenBank Accession No. XM_500900) in strains Y9472, Y9473, Y9474, Y9475, Y9476, Y9477, Y9478, Y9479, Y9480, Y9481, Y94782, Y9483, Y9484, Y9485, Y9486, Y9496, Y9497, Y9498, Y9499, Y9500, Y9501, Y9502, Y9503, Y9504, Y9505, Y9506, Y9507, Y9508, Y9509, Y9510 and Y9511 was not confirmed in any of these EPA strains, produced by transformation with pZK16-MLBN or pZK16-ML.

Analysis Of Total Lipid Content And Composition By Flask Assay

Cells from YPD plates of strains Y9477, Y9481, Y9486, Y9497, Y9502, Y9504, Y9508 and Y9510 were grown and analyzed for total lipid content and composition, according to the General Methods.

Table 16 below summarizes the total dry cell weight of the cells ["DCW'], the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

Table 16: Total Lipid Content And Composition In *Yarrowia* Strains Y9477, Y9481, Y9486, Y9497, Y9502, Y9504, Y9508 and Y9510 By Flask Assay

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y9477 | 3.2 | 32.6 | 2.6 | 0.5 | 3.4 | 4.8 | 10.0 | 0.5 | 2.5 | 3.7 | 1.0 | 0.5 | 2.1 | 61.4 | 6.9 | 20.0 |
| **Y9481** | **3.1** | **35.0** | **2.5** | **0.5** | **3.1** | **4.7** | **11.0** | **0.6** | **2.6** | **3.6** | **0.9** | **0.5** | **2.1** | **60.9** | **6.8** | **21.3** |
| Y9486 | 3.1 | 32.2 | 2.1 | 0.7 | 1.8 | 4.2 | 11.9 | 0.6 | 2.9 | 4.2 | 1.2 | 0.7 | 2.4 | 60.3 | 6.7 | 19.4 |
| | | | | | | | | | | | | | | | | |
| Y9497 | 3.7 | 33.7 | 2.4 | 0.5 | 3.2 | 4.6 | 11.3 | 0.8 | 3.1 | 3.6 | 0.9 | 0.7 | 2.3 | 58.7 | 7.1 | 19.8 |
| **Y9502** | **3.8** | **37.1** | **2.5** | **0.5** | **2.9** | **5.0** | **12.7** | **0.9** | **3.5** | **3.3** | **0.8** | **0.7** | **2.4** | **57.0** | **7.5** | **21.3** |
| Y9504 | 3.7 | 33.7 | 2.2 | 0.5 | 3.0 | 4.5 | 11.3 | 0.7 | 2.9 | 3.5 | 0.9 | 0.7 | 2.3 | 59.9 | 7.1 | 20.1 |
| Y9508 | 3.7 | 34.9 | 2.3 | 0.5 | 2.7 | 4.4 | 13.1 | 0.9 | 2.9 | 3.3 | 0.9 | 0.7 | 2.3 | 58.7 | 7.3 | 20.5 |
| Y9510 | 3.6 | 35.1 | 2.5 | 0.5 | 2.7 | 4.4 | 11.7 | 0.7 | 2.9 | 3.7 | 0.9 | 0.7 | 2.3 | 58.9 | 7.8 | 20.7 |

EXAMPLE 7

Generation Of *Yarrowia lipolytica* Strain Y8672 To Produce About 61.8% EPA Of Total Fatty Acids ["TFAs"] With 26.5% Total Lipid Content

The present Example describes the construction of strain Y8672, derived from *Yarrowia lipolytica* ATCC #20362, capable of producing about 61.8% EPA relative to the total lipids with 26.5% total lipid content ["TFAs % DCW"] via expression of a Δ9 elongase/Δ8 desaturase pathway. The development of strain Y8672 (FIG. 9) required the construction of strains Y2224, Y4001, Y4001 U, Y4036, Y4036U and L135 (described in Example 1), strains L135U9 and Y8002 (described in Example 2), strains Y8006U6, Y8069, Y8069U (described in Example 3) and strains Y8145, Y8145U, Y8259, Y8259U, Y8367 and Y8367U.

Generation Of Strain Y8145 To Produce About 48.5% EPA Of TFAs

Construct pZKL2-5m89C (FIG. 10; SEQ ID NO:138) was generated to integrate one Δ5 desaturase gene, one Δ9 elongase gene, one Δ8 desaturase gene, and one *Y. lipolytica* diacylglycerol cholinephosphotransferase gene (CPT1) into the *Lip2* loci (GenBank Accession No. AJ012632) of strain Y8069U3 (Example 3) to thereby enable higher level production of EPA. The pZKL2-5m89C plasmid contained the following components:

Table 17: Description of Plasmid pZKL2-5m89C (SEQ ID NO:138)

| RE Sites And Nucleotides Within SEQ ID NO:138 | Description Of Fragment And Chimeric Gene Components |
|---|---|
| *AscI*/*BsiWI* (730-1) | 722 bp 5' portion of *Yarrowia Lip2* gene (labeled as "Lip2.5N" in Figure; GenBank Accession No. AJ012632) |
| *PacI*/*SphI* (4141-3438) | 697 bp 3' portion of *Yarrowia Lip2* gene (labeled as "Lip2.3N" in Figure; GenBank Accession No. AJ012632) |
| *SwaI*/*BsiWI* (13143-1) | GPD::YICPT1::Aco, comprising:<br>• GPD: *Yarrowia lipolytica* GPD promoter (U.S. Patent 7,259,255);<br>• YICPT1: *Yarrowia lipolytica* diacylglycerol cholinephosphotransferase gene (SEQ ID NO:37) (Intl. App. Pub. No. WO 2006/052870);<br>• Aco: Aco terminator sequence from *Yarrowia Aco* gene (GenBank Accession No. AJ001300) |
| *PmeI*/*SwaI* | FBAIN::EgD8M::Lip1 comprising: |
| (10506-13143) | • FBAIN: *Yarrowia lipolytica* FBAIN promoter (U.S. Patent 7,202,356);<br>• EgD8M: Synthetic mutant Δ8 desaturase (SEQ ID NO:59; U.S. Patent 7,709,239), derived from *Euglena gracilis* ("EgD8S"; U.S. Patent 7,256,033);<br>• Lip1: Lip1 terminator sequence from *Yarrowia Lip1* gene (GenBank Accession No. Z50020) |
| *PmeI*/*ClaI* (10506-8650) | YAT1::EgD9eS::Lip2, comprising:<br>• YAT1: *Yarrowia lipolytica* YAT1 promoter (labeled as "YAT" in Figure; U.S. Pat. Appl. Pub. No. 2006-0094102-A1);<br>• EgD9eS: codon-optimized Δ9 elongase gene (SEQ ID NO:45), derived from *Euglena gracilis* (U.S. Patent 7,645,604);<br>• Lip2: Lip2 terminator sequence from *Yarrowia Lip2* gene (GenBank Accession No. AJ012632) |
| *ClaI*/*EcoRI* (8650-6581) | *Yarrorvia* Ura3 gene (GenBank Accession No. AJ306421) |

(continued)

| RE Sites And Nucleotides Within SEQ ID NO:138 | Description Of Fragment And Chimeric Gene Components |
|---|---|
| *Eco*RI/*Pac*I (6581-4141) | YAT1::EgD5SM::ACO, comprising:<br>• YAT1: *Yarrowia lipolytica* YAT1 promoter (labeled as "YAT" in Figure; U.S. Pat. Appl. Pub. No. 2006-0094102-A1);<br>• EgD5SM: Synthetic mutant Δ5 desaturase (SEQ ID NO:71; U.S. Pat. Pub. No. 2010-0075386-A1), derived from *Euglena gracilis* (U.S. Patent 7,678,560);<br>• Aco: Aco terminator sequence from *Yarrowia Aco* gene (GenBank Accession No. AJ001300) |

The pZKL2-5m89C plasmid was digested with *Asc*I/*Sph*I, and then used for transformation of strain Y8069U3 according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced 38-44.5% EPA of TFAs. Four strains (i.e., #10, #50, #70 and #89) that produced about 45.1%, 45.6%, 45.0% and 45.6% EPA of TFAs were designated as Y8143, Y8144, Y8145 and Y8146, respectively. Knockout of the *Lip2* loci (GenBank Accession No. AJ012632) was not confirmed in these EPA strains.

The final genotype of strains Y8143, Y8144, Y8145 and Y8146 with respect to wildtype *Yarrowia lipolytica* ATCC #20362 was *Ura+, Pex3-, unknown 1-, unknown 2-, unknown 3-, unknown 4-, unknown 5-, Leu+, Lys+,* YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, YAT1::EgD9eS::Lip2, FBAINm::EgD8M::Pex20, FBAIN::EgD8M::Lip1, EXP1::EgD8M::Pex16, FBAIN::EgD5SM::Pex20, YAT1::EgD5SM::Aco, EXP1::EgD5M::Pex16, YAT1::EaD5SM::Oct, YAT1::PaD17S::Lip1, EXP1::PaD17::Pex16, FBAINm::PaD17::Aco, GPD::YlCPT1::Aco.

Analysis Of Total Lipid Content And Composition By Flask Assay

Cells from YPD plates of strains Y8143, Y8144, Y8145 and Y8146 were grown and analyzed for total lipid content and composition, according to the General Methods.

Table 18: Total Lipid Content And Composition In *Yarrowia* Strains Y8143, Y8144, Y8145 and Y8146 By Flask Assay

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8143 | 4.6 | 22.3 | 4.2 | 1.5 | 1.4 | 3.6 | 18.1 | 2.6 | 1.7 | 1.6 | 0.6 | 2.2 | 1.6 | 50.3 | 11.6 | 11.2 |
| Y8144 | 4.3 | 23 | 4.0 | 1.5 | 1.4 | 3.3 | 18.0 | 2.6 | 1.8 | 1.7 | 0.7 | 2.3 | 1.6 | 50.6 | 11.5 | 11.6 |
| **Y8145** | **4.6** | **23.1** | **4.3** | **1.7** | **1.4** | **4.8** | **18.6** | **2.8** | **2.2** | **1.5** | **0.6** | **2.2** | **1.5** | **48.5** | **9.9** | **11.2** |
| Y8146 | 4.5 | 23.8 | 4.3 | 1.7 | 1.4 | 4.8 | 18.7 | 2.8 | 2.0 | 1.5 | 0.6 | 2.2 | 1.5 | 48.3 | 11.2 | 11.5 |

Generation Of Strain Y8145U *(Ura3-)*

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8145 in a manner similar to that described for pZKUM transformation of strain Y8006 (Example 3). A total of 8 transformants were grown and identified to possess a Ura- phenotype.

GC analyses showed that there were 22.5%, 22.6% and 23.4% EPA of TFAs in pZKUM-transformant strains #5, #6 and #7, respectively. These three strains were designated as strains Y8145U1, Y8145U2 and Y8145U3, respectively (collectively, Y8145U).

Generation Of Y8259 Strain To Produce About 53.9% EPA Of TFAs

Construct pZKL1-2SR9G85 (Example 3, FIG. 6A; SEQ ID NO:132) was generated to integrate one DGLA synthase gene, one Δ12 desaturase gene and one Δ5 desaturase gene into the *Lip1* loci (GenBank Accession No. Z50020) of strain Y8145U to thereby enable higher level production of EPA.

The pZKL1-2SR9G85 plasmid was digested with AscI/SphI, and then used for transformation of strain Y8145U1, in a manner similar to that described for pZKL1-2SR9G85 transformation of strain Y8154U1 (Example 3). The cells were subjected to fatty acid analysis, according to the General Methods.

GC analyses showed that most of the selected 96 strains produced 40-44.0% EPA of total lipids. Five strains (i.e., #7, #14, #48, #56 and #60) that produced about 45.2%, 47%, 44.4%, 44.3% and 45.2% EPA of TFAs were designated as Y8255, Y8256, Y8257, Y8258 and Y8259, respectively. Knockout of the *Lip1* loci (GenBank Accession No. Z50020) was not confirmed in these EPA strains.

The final genotype of these strains with respect to wildtype *Yarrowia lipolytica* ATCC #20362 was *Ura+, Pex3-, unknown 1-, unknown 2-, unknown 3-, unknown 4-, unknown* 5-, *unknown 6-, Leu+, Lys+,* YAT1::ME3S::Pex16, GPD::FmD12::Pex20, YAT1::FmD12::Oct, EXP1::FmD12S::ACO, GPAT::EgD9e::Lip2, FBAINm::EgD9eS::Lip2, EXP1::EgD9eS::Lip1, YAT1::EgD9eS::Lip2, FBAINm::EgD8M::Pex20, FBAIN::EgD8M::Lip1, EXP1::EgD8M::Pex16, YAT1::E389S/EgD8M::Lip1, FBAIN::EgD5SM::Pex20, YAT1::EgD5SM::Aco, GPM::EgD5SM::Oct, EXP1::EgDSM::Pex16, YAT1::EaD5SM::Oct; YAT1::PaD17S::Lip1, EXP1::PaD17::Pex16, FBAINm::PaD17::Aco, GPD::YlCPT1::Aco.

*Yarrowia lipolytica* strain Y8259 was deposited with the American Type Culture Collection on May 14, 2009 and bears the designation ATCC PTA-10027.

Analysis Of Total Lipid Content And Composition By Flask Assay

Cells from YPD plates of strains Y8256 and Y8259 were grown and analyzed for total lipid content and composition, according to the General Methods.

Table 19: Total Lipid Content And Composition In *Yarrowia* Strains Y8256 and Y8259 By Flask Assay

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8256 | 4.0 | 20.1 | 3.5 | 1.4 | 1.3 | 3.8 | 18.8 | 2.0 | 2.1 | 1.6 | 0.8 | 2.1 | 1.7 | 49.9 | 11.0 | 10.0 |
| **Y8259** | **4.7** | **20.5** | **3.5** | **1.3** | **1.3** | **4.8** | **16.9** | **2.3** | **1.9** | **1.7** | **0.6** | **1.8** | **1.6** | **53.9** | **8.4** | **11.0** |

Generation Of Strain Y8259U *(Ura3-)*

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8259 in a manner similar to that described for pZKUM transformation of strain Y8006 (Example 3). A total of 8 transformants were grown and identified to possess a *Ura-* phenotype.

GC analyses showed that there was 26.6% EPA of TFAs in pZKUM-transformant strain #3. This strain was designated as strain Y8259U.

Generation Of Y8367 Strain To Produce about 58.3% EPA Of TFAs

Construct pZP2-85m98F (Example 5, FIG. 7B; SEQ ID NO:135) was generated to integrate one Δ8 desaturase gene, one DGLA synthase, and one Δ5 desaturase gene into the *Yarrowia* Pox2 locus (GenBank Accession No. AJ001300) of strain Y8259U to thereby enable higher level production of EPA.

The pZP2-85m98F plasmid was digested with *AscI/SphI,* and then used for transformation of strain Y8259U, in a manner similar to that described for pZP2-85m98F transformation of strain Y8647U3 (Example 5). The cells were subjected to fatty acid analysis, according to the General Methods.

GC analyses showed that most of the selected 96 strains of Y8259U with pZP2-85m98F produced 41-46% EPA of TFAs. Four strains (i.e., #26, #33, #77 and #81) that produced about 46.7%, 46.5%, 47.4% and 46.9% EPA of TFAs were designated as Y8367, Y8368, Y8369 and Y8370, respectively. Knock out of the Pox2 locus (GenBank Accession No. AJ001300) was not confirmed in these EPA strains.

The final genotype of strains Y8367, Y8368, Y8369 and Y8370 with respect to wildtype *Yarrowia lipolytica* ATCC #20362 was *Ura+, Pex3-, unknown 1-, unknown* 2-, *unknown 3-, unknown 4-, unknown* 5-, *unknown 6-, unknown 7-, Leu+, Lys+,* YAT1::ME3S::Pex16, GPD::FmD12::Pex20, YAT1::FmD12::Oct, EXP1::FmD12S::ACO, GPAT::EgD9e::Lip2, FBAINm::EgD9eS::Lip2, EXP1::EgD9eS::Lip1, YAT1::EgD9eS::Lip2, FBAINm::EgD8M::Pex20, FBAIN::EgD8M::Lip1, EXP1::EgD8M::Pex16, GPD::EaD8S::Pex16, YAT1::E389D9eS/EgD8M::Lip1, YAT1::EgD9eS/EgD8M::Aco, FBAIN::EgD5SM::Pex20, YAT1::EgD5SM::Aco, GPM::EgD5SM::Oct, EXP1::EgD5M::Pex16, EXP1::EgD5SM::Lip1, YAT1::EaD5SM::Oct, YAT1::PaD17S::Lip1, EXP1::PaD17::Pex16, FBAINm::PaD17::Aco, GPD::YlCPT1::Aco.

Analysis Of Total Lipid Content And Composition By Flask Assay

Cells from YPD plates of strains Y8367, Y8368, Y8369 and Y8370 were grown and analyzed for total lipid content and composition, according to the General Methods.

Table 20: Total Lipid Content And Composition In *Yarrowia* Strains Y8367, Y8368, Y8369 and Y8370 By Flask Assay

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| **Y8367** | **3.6** | **18.4** | **3.7** | **1.2** | **1.1** | **3.4** | **14.2** | **1.1** | **1.5** | **1.7** | **0.8** | **2.1** | **1.0** | **58.3** | **9.9** | **10.7** |
| Y8368 | 4.7 | 19.2 | 3.0 | 1.4 | 1.3 | 4.3 | 17.9 | 1.3 | 2.4 | 2.8 | 1.0 | 1.8 | 1.9 | 52.5 | 8.4 | 10.1 |
| Y8369 | 3.5 | 19.7 | 3.7 | 1.2 | 1.6 | 4.2 | 15.6 | 1.8 | 1.7 | 1.9 | 0.6 | 1.7 | 1.6 | 55.8 | 8.6 | 11.0 |
| Y8370 | 4.0 | 23.3 | 3.4 | 1.1 | 1.4 | 4.0 | 15.7 | 1.9 | 1.7 | 1.9 | 0.6 | 1.8 | 1.5 | 56.4 | 8.6 | 13.1 |

Generation Of Strain Y8367U (*Ura3*-)

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8367 in a manner similar to that described for pZKUM transformation of strain Y8006 (Example 3). A total of 8 transformants were grown and identified to possess a *Ura*- phenotype.

GC analyses showed that there were 25.6%, 25.5% and 25.4% EPA of TFAs in pZKUM-transformant strains #2, #3 and #6, respectively. These three strains were designated as strains Y8367U1, Y8367U2 and Y8367U3, respectively (collectively, Y8367U).

Generation Of Y8672 strain To Produce about 61.8% EPA Of TFAs

Construct pZSCP-Ma83 (Example 3, FIG. 6B; SEQ ID NO:133) was generated to integrate one Δ8 desaturase gene, one $C_{16/18}$ elongase gene and one malonyl-CoA synthetase gene into the SCP2 loci (GenBank Accession No. XM_503410) of strain Y8637U to thereby enable higher level production of EPA.

The pZSCP-Ma83 plasmid was digested with *Asc*I/*Sph*I, and then used for transformation of strain Y8367U1, in a manner similar to that described for pZSCP-Ma83 transformation of strain Y8269U1 (Example 3). The cells were subjected to fatty acid analysis, according to the General Methods.

GC analyses showed that most of the selected 96 strains of Y8367U1 with pZSCP-Ma83 produced 46-52.5% EPA of TFAs. Eight strains (i.e., #8, #40, #43, #44, #61, #63, #68 and #70) that produced about 53.2%, 52.8%, 52.7%, 52.9%, 53.0%, 52.6%, 53.1% and 52.7% EPA of TFAs were designated as Y8666, Y8667, Y8668, Y8669, Y8670, Y8671, Y8672 and Y8673, respectively. Knockout of the SCP2 loci (GenBank Accession No. XM_503410) was not confirmed in these EPA strains.

The final genotype of strains Y8666, Y8667, Y8668, Y8669, Y8670, Y8671, Y8672 and Y8673 with respect to wildtype *Yarrowia lipolytica* ATCC #20362 was *Ura+, Pex3-, unknown 1-, unknown* 2-, *unknown 3-*, *unknown 4-, unknown 5-, unknown 6-, unknown 7-, unknown 8-, Leu+, Lys*+, YAT1::ME3S::Pex16, GPD::ME3S::Pex20, GPD::FmD12::Pex20, YAT1::FmD12::Oct, EXP1::FmD12S::ACO, GPAT::EgD9e::Lip2, FBAINm::EgD9eS::Lip2, EXP1::EgD9eS::Lip1, YAT1::EgD9eS::Lip2, FBAINm::EgD8M::Pex20, FBAIN::EgD8M::Lip1, EXP1::EgD8M::Pex16, GPD::EaD8S::Pex16 (2 copies), YAT1::E389D9eS/EgD8M::Lip1, YAT1::EgD9eS/EgD8M::Aco, FBAIN::EgD5SM::Pex20, YAT1::EgD5SM::Aco, GPM::EgD5SM::Oct, EXP1::EgD5M::Pex16, EXP1::EgD5SM::Lip1, YAT1::EaD5SM::Oct, YAT1::PaD17S::Lip1, EXP1::PaD17::Pex16, FBAINm::PaD17::Aco, GPD::YlCPT1::Aco, YAT1::MCS::Lip1.

Analysis Of Total Lipid Content And Composition By Flask Assay

Cells from YPD plates of strains Y8666, Y8669, Y8679 and Y8672 were grown and analyzed for total lipid content and composition, according to the General Methods.

Table 21: Total Lipid Content And Composition In *Yarrowia* Strains Y8666, Y8669, Y8670 And Y8672 By Flask Assay

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8666 | 3.2 | 25.2 | 2.3 | 0.3 | 2.3 | 4.1 | 15.1 | 1.3 | 1.7 | 1.4 | 0.7 | 0.6 | 1.3 | 62.2 | 6.7 | 15.6 |
| Y8669 | 3.2 | 26.4 | 2.3 | 0.3 | 2.3 | 4.1 | 15.7 | 1.4 | 1.8 | 1.6 | 0.7 | 0.5 | 1.1 | 61.5 | 6.7 | 16.3 |
| Y8670 | 3.2 | 27.3 | 1.9 | 0.4 | 3.4 | 4.3 | 17.0 | 1.5 | 2.2 | 1.7 | 0.6 | 0.5 | 1.1 | 60.9 | 4.5 | 16.6 |
| **Y8672** | **3.3** | **26.5** | **2.3** | **0.4** | **2.0** | **4.0** | **16.1** | **1.4** | **1.8** | **1.6** | **0.7** | **0.4** | **1.1** | **61.8** | **6.4** | **16.4** |

EXAMPLE 8

Construction Of Various Expression Vectors Comprising Different LPLAT ORFs

The present example describes the construction of a series of vectors, each comprising a LPLAT ORF, suitable for expression in *Yarrowia lipolytica.* LPLAT ORFs included the *Saccharomyces cerevisiae* Ale1, *Yarrowia lipolytica* Ale1, *Mortierella alpina* LPAAT1, *Yarrowia lipolytica* LPAAT1 and *Caenorhabditis elegans* LPCAT. Example 9 describes the results obtained following transformation of these vectors into *Yarrowia lipolytica* strain Y8406U.

Origin Of LPLATs

A variety of LPLATs have been identified in the patent and open literature, but the functionality of these genes has not been previously directly compared. Table 22 summarizes publicly available LPLATs (i.e., ScAle1, ScLPAAT, MaLPAAT1 and CeLPCAT) and LPLAT orthologs identified herein (i.e., YlAle1 and YlLPAAT1) that are utilized in the present Example, following codon-optimization of heterologous genes for expression in *Yarrowia lipolytica* (*infra*).

Table 22: LPLATs Functionally Characterized

| LPLAT | Organism | ORF Designation | References | SEQ ID NO |
|---|---|---|---|---|
| Ale1 | *Saccharomyces cerevisiae* * | ORF "YOR175C" or "ScAle1" | GenBank Accession No. NP_014818; U.S. Pat. Appl. Pub. No. 20080145867 (and corresponding to Intl. App. Pub. No. WO 2008/076377); Intl. App. Pub. No. WO 2009/001315 | 14, 15 |
| | *Yarrowia lipolytica* | "YALI0F19514p" or "YlAle1" | GenBank Accession No. XP_505624; Intl. App. Pub. No. WO 2009/001315 | 16, 17 |
| LPAAT | *Saccharomyces cerevisiae* | ORF "YDL052C" or "ScLPAAT" | GenBank Accession No. NP_010231 | 32 |
| | *Mortierella alpina* | "MaLPAAT1" | U.S. Pat. Appl. Pub. No. 2006-0115881-A1; U.S. Pat. Appl. Pub. No. 2009-0325265-A1 | 28,29 |
| | *Yarrowia lipolytica* | "YALI0E18964g" or "YlLPAAT1" | GenBank Accession No. XP_504127; U.S. Patent 7,189,559 | 30, 31 |
| LPCAT | *Caenorhabditis elegans** | "clone T06E8.1" or "CeLPCAT" | GenBank Accession No. CAA98276; Intl. App. Pub. No. WO 2004/076617 (corresponding to U.S. Pat. Appl. Pub. No. 2006-0168687-A1) | 24,25 |

*The *Saccharomyces cerevisiae Ale1* and *Caenorhabditis elegans* LPCAT were used as comparative Examples.

More specifically, the ScLPAAT (SEQ ID NO:32) and ScAle1 (SEQ ID NO:15) protein sequences were used as queries to identify orthologs from the public *Y. lipolytica* protein database of the "Yeast project *Genolevures"* (Center for Bioinformatics, LaBRI, Talence Cedex, France) (see also Dujon, B. et al., Nature, 430(6995):35-44 (2004)). Based on analysis of the best hits, the Ale1 and LPAAT orthologs from *Yarrowia lipolytica* are identified herein as YlAle1 (SEQ ID NO:17) and YlLPAAT (SEQ ID NO:31), respectively. The identiy of YlAle1 and YlLPAAT1 as orthologs of ScAle1 and ScLPAAT, respectively, was further confirmed by doing a reciprocal BLAST, i.e., using SEQ ID NOs:17 and 31 as a query against the *Saccharomyces cerevisiae* public protein database to find ScAle1 and ScLPAAT, repectively, as the best hits.

The LPLAT proteins identified above as ScAle1 (SEQ ID NO:15), YlAle1 (SEQ ID NO:17), ScLPAAT (SEQ ID NO:32), MaLPAAT1 (SEQ ID NO:29), YlLPAAT1 (SEQ ID NO:31) and CeLPCAT (SEQ ID NO:25) were aligned using the method of Clustal W (slow, accurate, Gonnet option; Thompson et al., Nucleic Acids Res., 22:4673-4680 (1994)) of the MegAlign™ program (version 8.0.2) of the LASERGENE bioinformatics computing suite (DNASTAR, Inc., Madison, WI). This resulted in creation of Table 23, where percent similarity is shown in the upper triangle of the Table while percent divergence is shown in the lower triangle.

Table 23: Percent Identity And Percent Divergence Among Various LPLATs

| YlLPAAT1 | CeLPCAT | MaLPAAT1 | ScAle1 | ScLPAAT | YlAle1 | |
|---|---|---|---|---|---|---|
| -- | 26.6 | 34.0 | 9.6 | 43.9 | 11.7 | YlLPAAT1 |
| 184.3 | -- | 36.4 | 11.3 | 32.4 | 14.5 | CeLPCAT |
| 137.5 | 126.4 | -- | 11.1 | 34.6 | 15.0 | MaLPAAT1 |
| 545.0 | 442.0 | 456.0 | -- | 13.5 | 45.0 | ScAle1 |
| 97.9 | 145.7 | 134.5 | 365.0 | -- | 15.6 | ScLPAAT |
| 426.0 | 339.0 | 330.0 | 94.3 | 317.0 | -- | YlAle1 |

The percent identities revealed by this method allowed determination of the minimum percent identity between each of the LPAAT polypeptides and the minimum percent identity between each of the Ale1 polypeptides. The range of identity between LPAAT polypeptides was 34.0% identity (MaLPAAT1 and Y1LPAAT1) to 43.9% identity (ScLPAAT and YlLPAAT1), while identity between the ScAle1 and YlAle1 polypeptides was 45%.

Membrane Bound *O*-Acyltransferase ["MBOAT"] Family Motifs: Orthologs of the ScAle1 protein sequence (SEQ ID NO:15) were identified by conducting a National Center for Biotechnology Information ["NCBI"] BLASTP 2.2.20 (protein-protein Basic Local Alignment Search Tool; Altschul et al., Nucleic Acids Res., 25:3389-3402 (1997); and Altschul et al., FEBS J., 272:5101-5109 (2005)) search using ScAle1 (SEQ ID NO:15) as the query sequence against fungal proteins in the "nr" protein database (comprising all non-redundant GenBank CDS translations, sequences derived from the 3-dimensional structure from Brookhaven Protein Data Bank ["PDB"], sequences included in the last major release of the SWISS-PROT protein sequence database, PIR and PRF excluding those environmental samples from WGS projects) using default parameters (expect threshold = 10; word size = 3; scoring parameters matrix = BLOSUM62; gap costs: existence = 11, extension = 1). The following hits were obtained:

Table 24: Fungal Orthologs Of ScAle1 (SEQ ID NO:15) Based On BLAST Analysis

| Gen Bank Acession No. | Species |
|---|---|
| NP_014818.1 | *Saccharomyces cerevisiae* |
| XP_001643411.1 | *Vanderwaltozyma polyspora* DSM 70294 |
| XP_448977.1 | *Candida glabrata* |
| XP_455985.1 | *Kluyveromyces lactis* |
| NP_986937.1 | *Ashbya gossypii* ATCC10895 |
| XP_001385654.2 | *Pichia stipitis* CBS 6054 |
| XP_001487052.1 | *Pichia guilliermondii* ATCC 6260 |
| EDK36331.2 | *Pichia guilliermondii* ATCC 6260 |
| XP_001525914.1 | *Lodderomyces elongisporus* NRRL YB-4239 |
| XP_461358.1 | *Debaryomyces hansenii* CBS767 |
| XP_713184.1 | *Candida albicans* SC5314 |
| XP_001645053.1 | *Vanderwaltozyma polyspora* DSM 70294 |
| XP_505624.1 | *Yarrowia lipolytica* |
| XP_001805526.1 | *Phaeosphaeria nodorum* SN15 |
| XP_001598340.1 | *Sclerotinia sclerotiorum* 1980 |
| XP_001907785.1 | *Podospora anserina* |
| XP_001931658.1 | *Pyrenophora tritici-repentis* Pt-1C-BFP |
| XP_001560657.1 | *Botryotinia fuckeliana* B05.*10* |
| XP_963006.1 | *Neurospora crassa* OR74A |
| XP_364011.2 | *Magnaporthe grisea* 70-15 |

(continued)

| Gen Bank Acession No. | Species |
|---|---|
| XP_001209647.1 | *Aspergillus terreus* NIH2624 |
| XP_001822945.1 | *Aspergillus oryzae* RIB40 |
| XP_001257694.1 | *Neosartorya fischeri* NRRL 181 |
| XP_747591.2 | *Aspergillus fumigatus* Af293 |
| XP_001270060.1 | *Aspergillus clavatus* NRRL 1 |
| NP_596779.1 | *Schizosaccharomyces pombe* |
| XP_001396584.1 | *Aspergillus niger* |
| XP_001229385.1 | *Chaetomium globosum* CBS 148.51 |
| XP_001248887.1 | *Coccidioides immitis* RS |
| XP_664134.1 | *Aspergillus nidulans* FGSC A4 |
| XP_566668.1 | *Cryptococcus neoformans var. neoformans* JEC21 |
| XP_001839338.1 | *Coprinopsis cinerea okayama* 7#130 |
| XP_757554.1 | *Ustilago maydis* 521 |

The yeast and fungal protein sequences of Table 24 were aligned using DNASTAR. Multiple sequence alignments and percent identity calculations were performed using the Clustal W method of alignment (*supra*).

More specifically, default parameters for multiple protein alignment using the Clustal W method of alignment correspond to: GAP PENALTY=10, GAP LENGTH PENALTY=0.2, Delay Divergent Seqs(%)=30, DNA Transition Weight=0.5, Protein Weight Matrix=Gonnet Series, DNA Weight Matrix=IUB with the 'slow-accurate' option. The resulting alignment was analyzed to determine the presence or absence of the non-plant motifs for Ale1 homologs, as identified in U.S. Pat. Appl. Pub. No. 2008-0145867-A1. Specifically, these include: M-[V/I]-[L/I]-xxK-[L/V/I]-xxxxxxDG (SEQ ID NO:102), RxKYYxxWxxx-[E/D]-[A/G]xxxxGxG-[F/Y]-xG (SEQ ID NO:103), $EX_{11}WNX_2$-[T/V]-$X_2W$ (SEQ ID NO:21) and SAxWHGxxPGYxx-[T/F]-F (SEQ ID NO:104), wherein X encodes any amino acid residue. The His residue in SEQ ID NO:104 has been reported to be a likely active site residue within the protein.

Only one motif, i.e., $EX_{11}WNX_2$-[T/V]-$X_2W$ (SEQ ID NO:21), was completely conserved in all 33 of the organisms aligned. The remaining M-[V/I]-[L/I]-xxK-[L/V/I]-xxxxxxDG (SEQ ID NO:102), RxKYYxx-Wxxx-[E/D]-[A/G]xxxxGxG-[F/Y]-xG (SEQ ID NO:103) and SAxWHGxxPGYxx-[T/F]-F (SEQ ID NO:104) motifs were only partially conserved. Thus, these motifs were appropriately truncated to fit with 0 mismatch (i.e., SAxWHG [SEQ ID NO:20]), 1 mismatch (i.e., RxKYYxxW [SEQ ID NO:19]), or 2 mismatches (i.e., M(V/I)(L/I)xxK(LVI) [SEQ ID NO:18]) for the purposes of the present methodologies.

1-Acyl-*sn*-Glycerol-3-Phosphate Acyltransferase ["LPAAT"] Family Motifs: Analysis of the protein alignment comprising ScLPAAT (SEQ ID NO:18), MaLPAAT1 (SEQ ID NO:15) and YlLPAAT1 (SEQ ID NO:17) revealed that the 1-acyl-sn-glycerol-3-phosphate acyltransferase family motif EGTR (SEQ ID NO:20) was present in each of the LPAAT orthologs. On this basis, MaLPAAT1 was identified as a likely LPAAT, that was clearly distinguishable from the *Morteriella alpina* LPAAT-like proteins disclosed in Intl. App. Pub. No. WO 2004/087902 (i.e., SEQ ID NOs:93 and 95).

It is noteworthy that the EGTR (SEQ ID NO:20) motif, while lacking in the LPCAT sequences in Intl. App. Pub. No. WO 2004/087902, is present in CeLPCAT (SEQ ID NO:2). It appears that other residues distinguish LPAAT and LPCAT sequences in LPAAT-like proteins. One such residue could be the extension of the EGTR (SEQ ID NO:20) motif. Specifically, whereas the EGTR motif in ScLPAAT (SEQ ID NO:18), MaLPAAT1 (SEQ ID NO:15) and YlLPAAT1 (SEQ ID NO:17) is immediately followed by a serine residue, the EGTR motif in CeLPCAT is immediately followed by an asparagine residue. In contrast, the two LPCATs in Intl. App. Pub. No. WO 2004/087902 have a valine substituted for the arginine residue in the EGTR motif and the motif is immediately followed by a valine residue.

Construction Of pY201, Comprising A Codon-Optimized *Saccharomyces cerevisiae* Ale1 Gene

The *Saccharomyces cerevisiae* ORF designated as "ScAle1" (SEQ ID NO:14) was optimized for expression in *Yarrowia lipolytica,* by DNA 2.0 (Menlo Park, CA). In addition to codon optimization, 5' Pci1 and 3' *Not*1 cloning sites were introduced within the synthetic gene (i.e., ScAle1S; SEQ ID NO:22). None of the modifications in the ScAle1S gene changed the amino acid sequence of the encoded protein (i.e., the protein sequence encoded by the codon-optimized gene [i.e., SEQ ID NO:23] is identical to that of the wildtype protein sequence [i.e., SEQ ID NO:15]). ScAle1S was cloned into pJ201 (DNA 2.0) to result in pJ201:ScAle1S.

A 1863 bp *Pci*1/*Not*1 fragment comprising ScAle1S was excised from pJ201:ScAle1S and used to create pY201 (SEQ ID NO:139; Table 25; FIG. 10A). In addition to comprising a chimeric YAT1::ScAle1S::Lip1 gene, pY201 also contains a *Y. lipolytica* URA3 selection marker flanked by LoxP sites for subsequent removal, if needed, by Cre recombinase-mediated recombination. Both the YAT1::ScAle1S::Lip1 chimeric gene and the URA3 gene were flanked by fragments having homology to 5' and 3' regions of the *Y. lipolytica* Pox3 gene to facilitate integration by double homologous recombination, although integration into *Y. lipolytica* is known to usually occur without homologous recombination. Thus, construct pY201 thereby contained the following components:

Table 25: Description of Plasmid pY201 (SEQ ID NO:139)

| RE Sites And Nucleotides Within SEQ ID NO:139 | Description Of Fragment And Chimeric Gene Components |
|---|---|
| *Bsi*W1/*Sbf*1 (1-1706 bp) | LoxP::*Ura*3::LoxP, comprising:<br>• LoxP sequence (SEQ ID NO: 140)<br>• *Yarrowia lipolytica* Ura3 gene (GenBank Accession No. AJ306421);<br>• LoxP sequence (SEQ ID NO:140) |
| *Sbf*1/*Sph*1 (1706-3043 bp) | 3' portion of *Yarrowia lipolytica* POX3 Acyl-CoA oxidase 3 (GenBank Accession No. YALI0D24750g) (i.e., bp 2215-3038 in pY201) |
| *Sph*1/*Asc*1 (3043-5743 bp) | • *ColE1* plasmid origin of replication;<br>• Ampicillin-resistance gene ($Amp^R$) for selection in *E. coli* (i.e., bp 3598-4758 [complementary] in pY201);<br>• *E. coli* f1 origin of replication |
| *Asc*I/*Bsi*WI (5743-6513 bp) | 5' portion of *Yarrowia lipolytica* POX3 Acyl-CoA oxidase 3 (GenBank Accession No. YALI0D24750g) (i.e., bp 5743-6512 in pY201) |
| *Bsi*WI/ *Bsi*WI (6514-1 bp)<br><br>[a *Not*1 site, located between ScAle1S and Lip1 is present at bp 9154 bp] | YAT1::ScAle1S::Lip1, comprising:<br>• YAT1: *Yarrowia lipolytica* YAT1 promoter (U.S. Pat. Appl. Pub. No. 2006/0094102-A1) (i.e., bp 6514-7291 in pY201)<br>• ScAle1S: codon-optimized Ale1 (SEQ ID NO:22) derived from *Saccharomyces cerevisiae* YOR175C (i.e., bp 7292-9151 in pY201; labeled as "Sc LPCATs ORF" in Figure);<br>• Lip1: Lip1 terminator sequence from *Yarrowia Lip1* gene (GenBank Accession No. Z50020) (i.e., bp 9160-9481 pY201; labeled as "Lip1-3'" in Figure) |

Construction Of pY168, Comprising A *Yarrowia lipolytica* Ale1 Gene

The *Yarrowia lipolytica* ORF designated as "YlAle1" (GenBank Accession No. XP_505624; SEQ ID NO:16) was amplified by PCR from *Yarrowia lipolytica* ATCC #20362 cDNA library using PCR primers 798 and 799 (SEQ ID NOs:141 and 142, respectively). Additionally, the YAT promoter was amplified by PCR primers 800 and 801 (SEQ ID NOs:143 and 144, respectively) from pY201 (SEQ ID NO:139). Since the primer pairs were designed to create two PCR products having some overlap with one another, a YAT1::YlAle1 fusion fragment was then amplified by overlapping PCR using primers 798 and 801 (SEQ ID NOs:141 and 144, respectively) and the two PCR fragments as template. The PCR was carried out in a RoboCycler Gradient 40 PCR machine (Stratagene) using the manufacturer's recommendations and Pfu Ultra™ High-Fidelity DNA Polymerase (Stratagene, Cat. No. 600380). Amplification was carried out as follows: initial denaturation at 95 °C for 4 min, followed by 30 cycles of denaturation at 95 °C for 30 sec, annealing at 55 °C for 1 min, and elongation at 72 °C for 1 min. A final elongation cycle at 72 °C for 10 min was carried out, followed by reaction termination at 4 °C.

The PCR product comprising the YAT1::Yl Ale1 fusion fragment was gel purified and digested with *Cla*I/*Not*I. This *Cla*1-*Not*1 fragment was ligated into pY201 that had been similarly digested (thereby removing the YAT1::ScAle1S fragment) to create pY168 (SEQ ID NO:145), comprising a chimeric YAT1::YlAle1::Lip1 gene. The DNA sequence of the *Yarrowia* Ale1 ORF was confirmed by DNA sequencing. The components present in pY168 (FIG. 10B; SEQ ID NO:145) are identical to those present in pY201, with the exception of the YAT1::YlAle1::Lip1 gene in pY168, instead of the YAT1::ScAle1S::Lip1 gene in pY201 (FIG. 10A). Note that YlAle1 is labeled as "Yl LPCAT" in FIG. 10B.

Construction Of pY208, Comprising A *Mortierella alpina* LPAAT1 Gene

The *Mortierella alpina* ORF designated as "MaLPAAT1" (SEQ ID NO:28) was optimized for expression in *Yarrowia lipolytica,* by DNA 2.0 (Menlo Park, CA). In addition to codon optimization, 5' *Pci*1 and 3' *Not*1 cloning sites were introduced within the synthetic gene (i.e., MaLPAAT1S; SEQ ID NO:35). None of the modifications in the MaLPAAT1S gene changed the amino acid sequence of the encoded protein (i.e., the protein sequence encoded by the codon-optimized gene [i.e., SEQ ID NO:36] is identical to that of the wildtype protein sequence [i.e., SEQ ID NO:29]). MaLPMT1S was cloned into pJ201 (DNA 2.0) to result in pJ201:MaLPAAT1S.

A 945 bp *Pci*1/*Not*1 fragment comprising MaLPAAT1S was excised from pJ201:MaLPAAT1S and used to create pY208 (SEQ ID NO:146), in a 3-way ligation with two fragments of pY201 (SEQ ID NO:139). Specifically, the MaLPAAT1 fragment was ligated with a 3530 bp *Sph*-*Not*I pY201 fragment and a 4248 bp *Nco*I-*Sph*I pY201 fragment to result in pY208. The components present in pY208 (FIG. 11A; SEQ ID NO:146) are identical to those present in pY201, with the exception of the YAT1::MaLPAAT1S::Lip1 gene in pY208, instead of the YAT1::ScAle1S::Lip1 gene in pY201 (FIG. 10A).

Construction Of pY207, Comprising A *Yarrowia lipolytica* LPAAT1 Gene

A putative LPAAT1 from *Yarrowia lipolytica* (designated herein as "YlLPAAT1"; SEQ ID NOs:30 and 31) was described in U.S. Patent 7,189,559 and GenBank Accession No. XP_504127. The protein is annotated as "similar to uniprot|P33333 *Saccharomyces cerevisiae* YDL052c SLC1 fatty acyltransferase".

The YlLPAAT1 ORF (SEQ ID NO:30) was amplified by PCR using a *Yarrowia lipolytica* ATCC #20362 cDNA library as a template and PCR primers 856 and 857 (SEQ ID NOs:147 and 148, respectively). The PCR was conducted using the same components and conditions as described above for amplification of the YAT1::Yl Ale1 fusion fragment, prior to synthesis of pY168.

The PCR product comprising the YlLPAAT1 ORF was digested with *Pci*I and *Not*I and then utilized in a 3-way ligation with two fragments from pY168. Specifically, the YlLPAAT1 fragment was ligated with a 3530 bp *Sph*-*Not*I pY168 fragment and a 4248 bp *Nco*I-*Sph*I pY168 fragment, to produce pY207, comprising a chimeric YAT1::YlLPAAT1::Lip1 gene. The *Y. lipolytica* LPAAT1 ORF was confirmed by DNA sequencing. The components present in pY207 (FIG. 11B; SEQ ID NO:149) are identical to those present in pY201, with the exception of the chimeric YAT1::YlLPAAT1::Lip1 gene in pY207, instead of the YAT1::ScAle1S::Lip1 gene in pY201 (FIG. 10A). Note that YlLPAAT1 is labeled as "Yl LPAT1 ORF" in FIG. 11B.

Construction Of pY175, Comprising A *Caenorhabditis elegans* LPCAT Gene

The *Caenorhabditis elegans* ORF designated as "CeLPCAT" (SEQ ID NO:24) was optimized for expression in *Yarrowia lipolytica,* by GenScript Corporation (Piscataway, NJ). In addition to codon optimization, 5' *Nco*1 and 3' *Not*1 cloning sites were introduced within the synthetic gene (i.e., CeLPCATS; SEQ ID NO:26). None of the modifications in the CeLPCATS gene changed the amino acid sequence of the encoded protein (i.e., the protein sequence encoded by the codon-optimized gene [i.e., SEQ ID NO:27] is identical to that of the wildtype protein sequence [i.e., SEQ ID NO:25]).

A *Nco*1-*Not*1 fragment comprising CeLPCATS was used to create pY175 (SEQ ID NO:150), in a 3-way ligation with two fragments from pY168 (SEQ ID NO:145). Specifically, the *Nco*1-*Not*1 fragment comprising CeLPCATS was ligated with a 3530 bp *Sph*-*Not*I pY168 fragment and a 4248 bp *Nco*I-*Sph*I pY168 fragment to result in pY175. The components present in pY175 (FIG. 12A; SEQ ID NO:150) are identical to those present in pY201, with the exception of the YAT1::CeLPCATS::Lip1 gene in pY175, instead of the YAT1::ScAle1S::Lip1 gene in pY201 (FIG. 10A). Note that CeLPCATS is labeled as "Ce.LPCATsyn" in FIG. 12A.

EXAMPLE 9

Functional Characterization Of Different LPLATs In EPA-Producing *Yarrowia lipolytica* Strain Y8406

*Yarrowia lipolytica* strain Y8406U, producing EPA, was used to functionally characterize the effects of overexpression of the *Saccharomyces cerevisiae* Ale1, *Yarrowia lipolytica* Ale1, *Mortierella alpina* LPAAT1, *Yarrowia lipolytica* LPAAT1 and *Caenorhabditis elegans* LPCAT, following their stable integration into the *Yarrowia* host chromosome. This was in spite of the host containing its native LPLATs, i.e., Ale1 and LPAAT1.

Transformation And Growth

To disrupt the *Ura3* gene, construct pZKUM (FIG. 5A; SEQ ID NO:130; described in Table 15 of U.S. Pat. Appl.

Pub. No. 2009-0093543-A1) was used to integrate a *Ura3* mutant gene into the *Ura3* gene of strain Y8406 in a manner similar to that described for pZKUM transformation of strain Y8006 (Example 3). Several transformants were grown and identified to possess a *Ura*- phenotype.

GC analyses showed that there were 26.1% EPA of FAMEs in pZKUM-transformant strains #4 and #5. These two strains were designated as strains Y8406U1 and Y8406U2, respectively (collectively, Y8406U).

*Yarrowia lipolytica* strain Y8406U was then individually transformed with linear *Sph*I-*Asc*I fragments of the integrating vectors described in Example 8, wherein each LPLAT was under the control of the *Yarrowia* YAT1 promoter. Specifically, vectors pY201 (YAT1::ScAle1S::Lip1), pY168 (YAT1::YlAle1::Lip1), pY208 (YAT1::MaLPAAT1S::Lip1), pY207 (YAT1::YILPAAT1::Lip1) and pY175 (YAT1::CeLPCATS::Lip1) were transformed according to the General Methods.

Each transformation mix was plated on MM agar plates. Several resultant URA+ transformants were picked and inoculated into 3 mL FM medium (Biomyx Cat. No. CM-6681, Biomyx Technology, San Diego, CA) containing per L: 6.7 g Difco Yeast Nitrogen Base without amino acids, 5 g Yeast Extract, 6 g $KH_2PO_4$, 2 g $K_2HPO_4$, 1.5 g $MgSO_4 \cdot 7H_20$, 1.5 mg thiamine·HCl, and 20 g glucose. After 2 days growth on a shaker at 200 rpm and 30 °C, the cultures were harvested by centrifugation and resuspended in 3 mL HGM medium (Cat. No. 2G2080, Teknova Inc., Hollister, CA) containing 0.63% monopotassium phosphate, 2.7% dipotassium phosphate, 8.0% glucose, adjusted to pH 7.5. After 5 days growth on a shaker at 200 rpm and at 30 °C, 1 mL aliquots of the cultures were harvested by centrifugation and analyzed by GC. Specifically, the cultured cells were collected by centrifugation for 1 min at 13,000 rpm, total lipids were extracted, and fatty acid methyl esters ["FAMEs"] were prepared by trans-esterification, and subsequently analyzed with a Hewlett-Packard 6890 GC (General Methods).

Based on the fatty acid composition of the 3 mL cultures, selected transformants were further characterized. Specifically, clones #5 and #11 of strain Y8406U transformed with expression vector pY201 (comprising ScAle1S) were selected and designated as "Y8406U::ScAle1 S-5" and "Y8406U::ScAle1S-11", respectively; clone #16 of strain Y8406U transformed with expression vector pY168 (comprising YlAle1) was selected and designated as "Y8406U::YlAle1"; clone #8 of strain Y8406U transformed with expression vector pY208 (comprising MaLPMT1S) was selected and designated as "Y8406U::MaLPAAT1S"; clone #21 of strain Y8406U transformed with expression vector pY207 (comprising YILPAAT1) was selected and designated as "Y8406U::YILPAAT1"; and clone #23 of strain Y8406U transformed with expression vector pY175 (comprising CeLPCATS) was selected and designated as "Y8406U::CeLPCATS". Additionally, strain Y8406 (a Ura+ strain that was parent to strain Y8406U (Ura-)) was used as a control.

Each selected transformant and the control was streaked onto MM agar plates. Then, one loop of freshly streaked cells was inoculated into 3 mL FM medium and grown overnight at 250 rpm and 30 °C. The $OD_{600nm}$ was measured and an aliquot of the cells were added to a final $OD_{600nm}$ of 0.3 in 25 mL FM medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for GC analysis (*supra*) and 10 mL dried for dry cell weight ["DCW"] determination.

For DCW determination, 10 mL culture was harvested by centrifugation for 5 min at 4000 rpm in a Beckman GH-3.8 rotor in a Beckman GS-6R centrifuge. The pellet was resuspended in 25 mL of water and re-harvested as above. The washed pellet was re-suspended in 20 mL of water and transferred to a pre-weighed aluminum pan. The cell suspension was dried overnight in a vacuum oven at 80 °C. The weight of the cells was determined.

Lipid Content, Fatty Acid Composition And Conversion Efficiencies

A total of four separate experiments were conducted under identical conditions. Experiment 1 compared control strain Y8406 versus strain Y8406U::ScAle1S-5. Experiment 2 compared control strain Y8406 versus strain Y8406U::YlAle1. Experiment 3 compared control strain Y8406 versus strain Y8406U::YlAle1, strain Y8406U::ScAle1S-11, and strain Y8406U::MaLPAAT1S. Experiment 4 compared control strain Y8406 versus strain Y8406U::MaLPAAT1S, strain Y8406U::YILPAAT1 and strain Y8406U::CeLPCATS.

In each experiment, the lipid content, fatty acid composition and EPA as a percent of the DCW are quantified for 1, 2 or 3 replicate cultures ["Replicates"] of the control Y8406 strain and the transformant Y8406U strain(s). Additionally, data for each Y8406U transformant is presented as a % of the Y8406 control. Table 26 below summarizes the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA and EPA.

Table 27 summarizes the conversion efficiency of each desaturase and the Δ9 elongase functioning in the PUFA biosynthetic pathway and which are required for EPA production. Specifically, the Δ12 desaturase conversion efficiency ["Δ12 CE"], Δ8 desaturase conversion efficiency ["Δ8 CE"], Δ5 desaturase conversion efficiency ["Δ5 CE"], Δ17 desaturase conversion efficiency ["Δ17 CE"] and Δ9 elongation conversion efficiency ["Δ9e CE"] are provided for each control Y8406 strain and the transformant Y8406U strain(s); data for each Y8406U transformant is presented as a % of the Y8406 control. Conversion efficiency was calculated according to the formula:

$$product(s)/(product(s)+substrate)*100,$$

where product includes both product and product derivatives.

Table 26: Lipid Content And Composition In LPCAT Transformant Strains Of *Yarrowia lipolytica* Y8406

| Expt. | Strain | Repli cates | TFA % DCW | % TFAs | | | | | | | | | | | | EPA % DCW |
|---|---|---|---|---|---|---|---|---|---|---|---|---|---|---|---|---|
| | | | | 16: 0 | 16: 1 | 18: 0 | 18: 1 | 18:2 | ALA | EDA | DGLA | ARA | ERA | ETA | EPA | |
| 1 | Y8406 | AVG.3 | 17.6 | 3.8 | 0.7 | 3.3 | 6.4 | 22.6 | 2.5 | 2.8 | 2.2 | 0.5 | 1.9 | 2.0 | 48.9 | 8.6 |
| | Y8406U:: ScAle1S-5 | AVG.3 | 18.3 | 4.2 | 0.7 | 3.5 | 5.7 | 15.1 | 0.6 | 3.3 | 3.7 | 0.8 | 1.8 | 2.3 | 56.9 | 10.4 |
| | | % Ctrl | 104 | 111 | 100 | 106 | 89 | 67 | 24 | 118 | 168 | 160 | 95 | 115 | 116 | 121 |
| 2 | Y8406 | AVG.3 | 23.2 | 3.5 | 0.6 | 3.3 | 6.4 | 22.3 | 2.7 | 2.6 | 2.1 | 0.5 | 1.6 | 2.0 | 49.9 | 11.6 |
| | Y8406U:: YIAle1 | AVG.3 | 22.3 | 3.8 | 0.7 | 2.9 | 3.9 | 12.7 | 0.4 | 3.0 | 3.8 | 0.8 | 1.6 | 2.4 | 60.9 | 13.6 |
| | | % Ctrl | 96 | 109 | 117 | 88 | 61 | 57 | 15 | 115 | 181 | 160 | 100 | 120 | 122 | 117 |
| 3 | Y8406 | 1 | 26.1 | 2.7 | 0.7 | 2.8 | 6.5 | 20.5 | 2.5 | 3.2 | 2.3 | 0.7 | 0.8 | 0.0 | 50.8 | 13.3 |
| | Y8406U:: YIAle1 | AVG.2 | 23.3 | 3.3 | 0.7 | 2.4 | 3.6 | 12.1 | 0.5 | 3.2 | 3.5 | 0.9 | 0.0 | 2.3 | 62.2 | 14.5 |
| | | % Ctrl | 89 | 122 | 100 | 86 | 55 | 59 | 20 | 100 | 152 | 129 | 0 | na | 122 | 109 |
| | Y8406U:: ScAle1S-11 | AVG.2 | 28.0 | 3.0 | 0.7 | 3.0 | 5.5 | 13.1 | 0.6 | 3.5 | 3.8 | 0.9 | 0.0 | 2.4 | 58.5 | 16.4 |
| | | % Ctrl | 107 | 111 | 100 | 107 | 85 | 64 | 24 | 109 | 165 | 129 | 0 | na | 115 | 123 |
| | Y8406U:: MaLPAAT1S | AVG.2 | 23.7 | 4.4 | 0.8 | 4.2 | 6.6 | 11.2 | 0.7 | 2.7 | 3.7 | 0.9 | 0.0 | 2.5 | 57.0 | 13.5 |
| | | % Ctrl | 91 | 163 | 114 | 150 | 102 | 55 | 28 | 84 | 161 | 129 | 0 | na | 112 | 102 |
| 4 | Y8406 | AVG.2 | 27.9 | 2.8 | 0.6 | 3.1 | 6.2 | 20.6 | 2.9 | 2.9 | 2.0 | 0.6 | 0.7 | 2.0 | 49.4 | 13.8 |
| | Y8406U:: MaLPAAT1S | AVG.2 | 25.2 | 4.8 | 0.8 | 4.8 | 6.9 | 11.6 | 0.8 | 2.5 | 3.0 | 0.7 | 0.0 | 2.3 | 55.3 | 14.0 |
| | | % Ctrl | 90 | 171 | 133 | 155 | 111 | 56 | 28 | 86 | 150 | 117 | 0 | 115 | 112 | 101 |
| | Y8406U:: YILPAAT1 | AVG.2 | 25.2 | 3.7 | 0.7 | 4.2 | 6.2 | 13.0 | 1.2 | 2.3 | 2.6 | 0.6 | 0.0 | 2.2 | 56.7 | 14.3 |
| | | % Ctrl | 90 | 132 | 117 | 135 | 100 | 63 | 41 | 79 | 130 | 100 | 0 | 110 | 115 | 104 |
| | Y8406U:: CeLPCATS | AVG.2 | 24.7 | 3.8 | 0.6 | 4.6 | 7.1 | 13.9 | 1.6 | 2.3 | 2.6 | 0.6 | 0.4 | 2.2 | 53.6 | 13.2 |
| | | % Ctrl | 89 | 136 | 100 | 148 | 115 | 67 | 55 | 79 | 130 | 100 | 57 | 110 | 109 | 96 |

Table 27: Desaturase And Elongase Conversion Efficiency In LPCAT Transformant Strains Of *Yarrowia lipolytica* Y8406

| Expt. | Strain | Replicates | Δ12 CE | Δ9e CE | Δ8 CE | Δ5 CE | Δ17 CE |
|---|---|---|---|---|---|---|---|
| 1 | Y8406 | AVG.3 | 93 | 70 | 92 | 92 | 90 |
| | Y8406U::ScAle1S-5 | AVG.3 | 94 | 81 | 93 | 91 | 89 |
| | | % Ctrl | 101 | 116 | 101 | 98 | 98 |
| 2 | Y8406 | AVG.3 | 93 | 70 | 93 | 93 | 91 |
| | Y8406U::YlAle1 | AVG.3 | 96 | 85 | 94 | 91 | 90 |
| | | % Ctrl | 103 | 121 | 101 | 98 | 98 |
| 3 | Y8406 | 1 | 93 | 72 | 93 | 96 | 89 |
| | Y8406U::YlAle1 | AVG.2 | 96 | 85 | 96 | 92 | 89 |
| | | % Ctrl | 104 | 119 | 103 | 96 | 100 |
| | Y8406U::ScAle1S-11 | AVG.2 | 94 | 83 | 95 | 91 | 88 |
| | | % Ctrl | 101 | 117 | 102 | 95 | 99 |
| | Y8406U::MaLPAAT1S | AVG.2 | 92 | 85 | 96 | 90 | 89 |
| | | % Ctrl | 100 | 119 | 103 | 94 | 100 |
| 4 | Y8406 | AVG.2 | 93 | 71 | 94 | 93 | 91 |
| | Y8406U::MaLPAAT1S | AVG.2 | 92 | 84 | 96 | 91 | 90 |
| | | % Ctrl | 99 | 118 | 102 | 99 | 100 |
| | Y8406U::YILPAAT1 | AVG.2 | 93 | 82 | 96 | 92 | 92 |
| | | % Ctrl | 100 | 115 | 103 | 100 | 101 |
| | Y8406U::CeLPCATS | AVG.2 | 92 | 80 | 96 | 92 | 91 |
| | | % Ctrl | 99 | 113 | 102 | 99 | 100 |

Based on the data concerning Experiments 1, 2 and 3 in Table 26 and Table 27, overexpression of LPLAT in EPA strains Y8406U::ScAle1S-5, Y8406U::ScAle1S-11, Y8406U::YlAle1 and Y8406U::MaLPAAT1S results in significant reduction (to 67% or below of the control) of the concentration of LA (18:2) as a weight % of TFAs ["LA % TFAs"], an increase (to at least 12% of the control) in the concentration of EPA as a weight % of TFAs ["EPA % TFAs"], and an increase (to at least 16% of the control) in the conversion efficiency of the Δ9 elongase. Compared to Y8406U::ScAle1 S-5 and Y8406U::ScAle1S-11, Y8406U::YlAle1 has lower LA % TFAs, higher EPA % TFAs, better Δ9 elongation conversion efficiency, and slightly lower TFAs % DCW and EPA % DCW. Y8406U::YlAle1 and Y8406U::MaLPAAT1S are similar except overexpression of MaLPAAT1S resulted in lower LA % TFAs, EPA % TFAs, and EPA % DCW.

Experiment 4 shows that overexpression of LPLAT in EPA strains Y8406U::YlAle1, Y8406U::MaLPAAT1S and Y8406U::CeLPCATS results in significant reduction (to 67% or below of the control) of LA % TFAs, an increase (to at least 9% of the control) in EPA % TFAs, and an increase (to at least 13% of the control) in the conversion efficiency of the Δ9 elongase. Compared to Y8406U::CeLPCATS, Y8406U::YLPAAT1 and Y8406U::MaLPAAT1S both have lower LA % TFAs, higher EPA % TFAs, higher EPA % DCW, and slightly better TFAs % DCW. Y8406U::YILPAAT1 and Y8406U::MaLPAAT1 S are similar except overexpression of MaLPAAT1S results in lower LA % TFAs, slightly lower EPA % TFAs and EPA % DCW, and slightly better Δ9 elongase conversion efficiency.

It is well known in the art that most desaturations occur at the *sn*-2 position of phospholipids, while fatty acid elongations occur on acyl-CoAs. Furthermore, ScAle1S, YlAle1, MaLPAAT1S and YILPAAT1 were expected to only incorporate acyl groups from the acyl-CoA pool into the sn-2 position of lysophospholipids, such as lysophosphatidic acid ["LPA"] and lysophosphatidylcholine ["LPC"]. Thus, it was expected that expression of ScAle1S, YlAle1, MaLPAAT1S, and YILPAAT1 would result in improved desaturations due to improved substrate availability in phospholipids, and not result in improved elongations that require improved substrate availability in the CoA pool. Our data (*supra*) shows that unexpectedly, expression of ScAle1S, YlAle1, MaLPAAT1S and YILPAAT1 significantly improved the Δ9 elongase conversion efficiency in strains of *Yarrowia* producing EPA but did not improve the desaturations (measured as Δ12 desaturase conversion efficiency, Δ8 desaturase conversion efficiency, Δ5 desaturase conversion efficiency or Δ17 desaturase conversion efficiency).

CeLPCAT was previously shown to improve Δ6 elongation conversion efficiency in *Saccharomyces cerevisiae* fed LA or GLA (Intl App. Pub. No. WO 2004/076617). This was attributed to its reversible LPCAT activity that released fatty acids from phospholipids into the CoA pool. An improvement in Δ9 elongation conversion efficiency in an oleaginous microbe, such as *Yarrowia lipolytica,* engineered for high level LC-PUFA production in the absence of feeding fatty acids was not contemplated in Intl. App. Pub. No. WO 2004/076617.

Futhermore, expression of ScAle1S, YlAle1, MaLPAAT1S, YlLPAAT1 and CeLPCATS did not significantly alter either the level of PUFAs accumulated or the total lipid content in strains of *Yarrowia* producing EPA.

Previous studies have shown that both Δ6 elongation and Δ9 elongation are bottlenecks in long chain PUFA biosynthesis due to poor transfer of acyl groups between phospholipid and acyl-CoA pools. Based on the improved Δ9 elongase conversion efficiency resulting from overexpression of LPLATs, demonstrated above, it is anticipated that the LPLATs described herein and their orthologs, such as ScLPAAT, will also improve Δ6 elongation conversion efficiency.

EXAMPLE 10

Construction Of Expression Vectors Comprising LPAAT ORFs And An Autonomously Replicating Sequence

The present example describes the construction of vectors comprising autonomously replicating sequences ["ARS"] and LPAAT ORFs suitable for LPAAT gene expression without integration in *Yarrowia lipolytica.* ORFs included the *Saccharomyces cerevisiae* LPAAT encoding SEQ ID NO:32 and the *Yarrowia lipolytica* LPAAT1 encoding SEQ ID NO:31. Example 11 describes the results obtained following transformation of these vectors into *Y. lipolytica.*

Construction Of pY222, Comprising A Codon-Optimized *Saccharomyces cerevisiae* LPAAT Gene

The *Saccharomyces cerevisiae* ORF designated as "ScLPAAT" (SEQ ID NO:32) was optimized for expression in *Yarrowia lipolytica,* by DNA 2.0 (Menlo Park, CA). In addition to codon optimization, 5' *Pci1* and 3' Not1 cloning sites were introduced within the synthetic gene (i.e., ScLPAATS; SEQ ID NO:151). None of the modifications in the ScLPAATS gene changed the amino acid sequence of the encoded protein (i.e., the protein sequence encoded by the codon-optimized gene [i.e., SEQ ID NO:152] is identical to that of the wildtype protein sequence [i.e., SEQ ID NO:32]). ScLPAATS was cloned into pJ201 (DNA 2.0) to result in pJ201:ScLPAATS.

A 926 bp *Pci*1/*Not*1 fragment comprising ScLPAATS was excised from pJ201:ScLPAATS and cloned into *Nco*I-*Not*1 cut pYAT-DG2-1 to create pY222 (SEQ ID NO:153; Table 28; FIG. 14A). Thus, pY222 contained the following components:

Table 28: Description of Plasmid pY222 (SEQ ID NO:153)

| RE Sites And Nucleotides Within SEQ ID NO:153 | Description Of Fragment And Chimeric Gene Components |
|---|---|
| *Sal*1/*Swa*I (1-2032) | YAT1::ScLPAATS::Lip1, comprising:<br>• YAT1: *Yarrowia lipolytica* YAT1 promoter (U.S. Pat. Appl. Pub. No. 2006/0094102-A1);<br>• ScLPAATS: codon-optimized ScLPAATS (SEQ ID NO:151) (labeled as "Sc LPAATs ORF" in Figure);<br>• Lip1: Lip1 terminator sequence from *Yarrowia Lip1* gene (GenBank Accession No. Z50020) (labeled as "Lip1-3'" in Figure) |
| *Swa*I/*Ava*I (2032-4946) | • *ColE1* plasmid origin of replication;<br>• Ampicillin-resistance gene ($Amp^R$) for selection in *E. coli*;<br>• *E. coli* f1 origin of replication |
| *Ava*I-*Sph*I (4946-6330) | *Yarrowia lipolytica* centromere and autonomously replicating sequence ["ARS"] 18 locus |
| *Sph*I-*Sal*I (6330-1) | *Yarrowia lipolytica* URA3 gene (GenBank Accession No. AJ306421) |

Construction Of pY177, Comprising A *Yarrowia lipolytica* LPAAT1 Gene

The *Yarrowia lipolytica* centromere and autonomously replicating sequence ["ARS"] was amplified by standard PCR using primer 869 (SEQ ID NO:154) and primer 870 (SEQ ID NO:155), with plasmid pYAT-DG2-1 as template. The PCR product was digested with *Asc*I/*Avr*II and cloned into *Asc*I-*Avr*II digested pY207 (SEQ ID NO:149; Example 8) to create pY177 (SEQ ID NO:156; Table 29; FIG. 14B). Thus, the components present in pY177 are identical to those in pY207 (FIG. 12A), except for the replacement of the 373 bp pY207 sequence between *Asc*I and *Avr*II with the 1341 bp sequence containg ARS. More specifically, pY177 contained the following components:

Table 29: Description of Plasmid pY177 (SEQ ID NO:156)

| RE Sites And Nucleotides Within SEQ ID NO:156 | Description Of Fragment And Chimeric Gene Components |
|---|---|
| *Bsi*W1/*Sbf*1 (1-1706 bp) | LoxP::*Ura3*::LoxP, comprising:<br>• LoxP sequence (SEQ ID NO:140)<br>• *Yarrowia lipolytica* Ura3 gene (GenBank Accession No. AJ306421);<br>• LoxP sequence (SEQ ID NO:140) |
| *Sbf*1/*Sph*1 (1706-3043 bp) | 3' portion of *Yarrowia lipolytica* POX3 Acyl-CoA oxidase 3 (GenBank Accession No. YALI0D24750g) |
| *Sph*I/*Asc*I (3043-5743 bp) | • *ColE1* plasmid origin of replication;<br>• Ampicillin-resistance gene ($Amp^R$) for selection in *E. coli;*<br>• *E. coli* f1 origin of replication |
| *Asc*I/*Bsi*WI (5743-6513 bp) | 5' portion of *Yarrowia lipolytica* POX3 Acyl-CoA oxidase 3 (GenBank Accession No. YALI0D24750g) |
| *Asc*I/*Avr*II (5743-7084 bp) | *Yarrouvia lipolytica* centromere and autonomously replicating sequence ["ARS"] 18 locus |
| *Avr*II/*Bsi*WI (7084-7481 bp) | 5' portion of *Yarrowia lipolytica* POX3 Acyl-CoA oxidase 3 (GenBank Accession No. YALI0D24750g) |
| BsiWI/ *Bsi*WI (7481-1 bp) | YAT1::YlLPAAT1::Lip1, comprising:<br>• YAT1: *Yarrowia lipolytica* YAT1 promoter (U.S. Pat. Appl. Pub. No. 2006/0094102-A1);<br>• YlLPAAT1: *Yarrowia lipolytica* LPAAT1 ("YALI0E18964g"; GenBank Accession No. XP_504127) (SEQ ID NO:30) (labeled as "Yl LPAT1 ORF" in Figure);<br>• Lip1: Lip1 terminator sequence from *Yarrowia Lip1* gene (GenBank Accession No. Z50020) (labeled as "Lip1-3'" in Figure) |

EXAMPLE 11

Functional Characterization Of Different LPAATs In EPA-Producing *Yarrowia lipolytica* Strain Y8406

*Yarrowia lipolytica* strain Y8406U, producing EPA, was used to functionally characterize the effects of expression of the *Saccharomyces cerevisiae* LPAATS (SEQ ID NO:151) and *Yarrowia lipolytica* LPAAT1 (SEQ ID NO:30) without integration on self-replicating plasmids. This was in spite of the host containing its native LPAATs.

Transformation And Growth

*Yarrowia lipolytica* strain Y8406U (Example 9) was individually transformed with uncut plasmids from Example 10. Specifically, vectors pY177 (YAT1::YlLPAAT1::Lip1) [SEQ ID NO:156] and pY222 (YAT1::ScLPAATS::Lip1) [SEQ ID NO:153] were transformed according to the General Methods.

Each transformation mix was plated on MM agar plates. Several resultant URA+ transformants were picked and inoculated into 3 mL CSM-U medium (Teknova Cat. No. C8140, Teknova Inc., Hollister, CA), wherein CSM-U medium refers to CM Broth with glucose minus uracil containing 0.13% amino acid dropout powder minus uracil, 0.17% yeast nitrogen base, 0.5% $(NH_4)_2SO_4$, and 2.0% glucose. After 2 days growth on a shaker at 200 rpm and 30 °C, the cultures were harvested by centrifugation and resuspended in 3 mL HGM medium (Cat. No. 2G2080, Teknova Inc.).

After 5 days growth on a shaker, 1 mL aliquots of the cultures were harvested and analyzed by GC, as described in Example 9.

Based on the fatty acid composition of the 3 mL cultures, selected transformants were further characterized by flask assay. Specifically, clones #5 and #6 of strain Y8406U transformed with expression vector pY222 (comprising ScLPAATS) were selected and designated as "Y8406U::ScLPAATS-5" and "Y8406U::ScLPAATS-6", respectively; clone #1 of strain Y8406U transformed with expression vector pY177 (comprising YILPAAT1) was selected and designated as "Y8406U::YILPAAT1". Additionally, strain Y8406 (a Ura+ strain that was parent to strain Y8406U (Ura-)) was used as a control.

Each selected transformant and the control was streaked onto MM agar plates. Then, one loop of freshly streaked cells was inoculated into 3 mL CSM-U medium and grown overnight at 250 rpm and 30 °C. The $OD_{600nm}$ was measured and an aliquot of the cells were added to a final $OD_{600nm}$ of 0.3 in 25 mL CSM-U medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for GC analysis and 10 mL dried for dry cell weight ["DCW"] determination, as described in Example 9.

Lipid Content, Fatty Acid Composition And Conversion Efficiencies

The lipid content, fatty acid composition and EPA as a percent of the DCW are quantified for 2 replicate cultures ["Replicates"] of the control Y8406 strain and the transformant Y8406U strain(s). Additionally, data for each Y8406U transformant is presented as a % of the Y8406 control. Table 30 below summarizes the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA and EPA.

Table 31 summarizes the conversion efficiency of each desaturase and the Δ9 elongase functioning in the PUFA biosynthetic pathway and which are required for EPA production, in a manner identical to that described in Example 9.

Table 30: Lipid Content And Composition In ScLPAATS and YlLPAAT1 Transformant Strains Of *Yarrowia lipolytica* Y8406

| Strain | Repli cates | TFA % DCW | % TFAs | | | | | | | | | | | | EPA % DCW |
|---|---|---|---|---|---|---|---|---|---|---|---|---|---|---|---|
| | | | 16: 0 | 16: 1 | 18: 0 | 18: 1 | 18:2 | ALA | EDA | DGLA | ARA | ERA | ETA | EPA | |
| Y8406 | AVG.2 | 22.0 | 2 | 0 | 2 | 4 | 19 | 2 | 3 | 4 | 1 | 2 | 3 | 55 | 12 |
| Y8406U:: YlLPAAT1 | AVG.2 | 24.6 | 2 | 1 | 2 | 6 | 14 | 1 | 3 | 5 | 1 | 2 | 3 | 55 | 14 |
| | % Ctrl | 112 | 98 | 153 | 102 | 148 | 76 | 50 | 120 | 144 | 101 | 109 | 123 | 101 | 113 |
| Y8406U:: ScLPAATS-5 | AVG.2 | 21.6 | 3 | 1 | 3 | 6 | 14 | 1 | 3 | 4 | 1 | 2 | 3 | 57 | 12 |
| | % Ctrl | 98 | 131 | 137 | 125 | 131 | 74 | 56 | 100 | 117 | 86 | 101 | 108 | 104 | 102 |
| Y8406U:: ScLPAATS-6 | AVG.2 | 21.4 | 3 | 1 | 3 | 5 | 14 | 1 | 3 | 4 | 1 | 2 | 3 | 58 | 12 |
| | % Ctrl | 97 | 125 | 133 | 121 | 124 | 72 | 52 | 97 | 119 | 88 | 102 | 111 | 106 | 103 |

Table 31: Desaturase And Elongase Conversion Efficiency In ScLPAATS and YlLPAAT1 Transformant Strains Of *Yarrowia lipolytica* Y8406

| Strain | Replicates | Δ12 CE | Δ9e CE | Δ8 CE | Δ5 CE | Δ17 CE |
|---|---|---|---|---|---|---|
| Y8406 | AVG.2 | 95 | 77 | 92 | 90 | 92 |
| Y8406U::YlLPAAT1 | AVG.2 | 93 | 82 | 92 | 87 | 90 |
| | % Ctrl | 98 | 107 | 99 | 97 | 98 |
| Y8406U::ScLPAATS-5 | AVG.2 | 94 | 83 | 93 | 89 | 92 |
| | % Ctrl | 98 | 108 | 100 | 99 | 100 |
| Y8406U::ScLPAATS-6 | AVG.2 | 94 | 83 | 93 | 89 | 92 |
| | % Ctrl | 99 | 109 | 101 | 99 | 100 |

Based on the data in Table 30 and Table 31 above, overexpression of both ScLPAATS and YlLPAAT1 in EPA strains Y8406U::YlLPAAT1, Y8406U::ScLPAATS-5 and Y8406U::ScLPAATS-6 resulted in reduction (to 76% or below of the control) of the concentration of LA (18:2) as a weight % of TFAs ["LA % TFAs"], and an increase (to at least 7% of the control) in the conversion efficiency of the Δ9 elongase. ScLPAATS and YlLPAAT1 have a similar effect on lipid profile.

The results obtained above were then compared to those obtained in Example 9, although different means were utilized to characterize the LPLATs. Specifically, in Example 9, linearized DNA carrying the LPLATs were transformed by chromosomal integration, since the vectors lacked ARS sequences. This resulted in stable integrations and the strains were grown in the relatively rich, non-selective FM growth medium during both preculture and 2 days growth prior to being transferred to HGM.

In Example 11, the functional characterization of YlLPAAT1 and ScLPAATS was done on a replicating plasmid. Thus, *Yarrowia lipolytica* strain Y8406 was transformed with circular DNA carrying each LPAAT and ARS sequence. To maintain these plasmids and assay gene expression without integration, it was necessary to grow the transformants on selective medium (i.e., CSM-U medium) during both preculture and 2 days growth prior to being transferred to HGM.

These differences described above can contribute to differences in lipid profile and content, as illustrated by the expression of YlLPAAT1 in Examples 9 and 11. The change over control in LA % TFAs, EPA % TFAs, and Δ9 elongase conversion efficiency were 63%, 115%, and 115%, respectively, upon expression of YlLPAAT in Example 9, whereas the change over control in LA % TFAs, EPA % TFAs, and Δ9 elongase conversion efficiency were were 76%, 101%, and 107%, respectively, upon expression of YlLPAAT in Example 11. Thus, the improvements in Δ9 elongation and LC-PUFA biosynthesis in Example 11 are minimized when compared to those observed in Example 9. These differences can be attributed to the "position effects" of chromosomal integration and/or different growth conditions.

Since the improvements in LC-PUFA biosynthesis (measured as reduction in LA % TFAs, increase in EPA % TFAs and increase in Δ9 elongase conversion efficiency) are similar for both ScLPAATS and YlLPAAT when transformed in Yarrowia lipolytica strain Y8406 on a replicating plasmid, it is anticipated that both LPLAATs will also function similarly when stably integrated into the host chromosome. Thus, ScLPAATS will likely improve the lipid profile in a manner similar to that observed in Example 9, when YlLPAAT1 was stably integrated into the host chromosome.

## Claims

1. An extracted, unconcentrated oil comprising:

   (a) at least 50 weight percent of eicosapentaenoic acid measured as a weight percent of total fatty acids; and
   (b) a ratio of at least 3.1 of eicosapentaenoic acid, measured as a weight percent of total fatty acids, to linoleic acid, measured as a weight percent of total fatty acids.

2. The oil of claim 1, wherein said oil is a microbial oil.

3. The oil of any of claims 1 or 2, wherein said oil is from an oleaginous yeast, and preferably wherein said oleaginous yeast is *Yarrowia,* e.g. *Yarrowia lipolytica.*

4. The oil of any of claims 1 to 3, wherein the ratio of eicosapentaenoic acid to linoleic acid in the oil is at least 3.5.

5. The oil of any of claims 1 to 3, wherein the ratio of eicosapentaenoic acid to linoleic acid in the oil is at least 4.5.

6. The oil of any of claims 1 to 5, wherein the oil comprises at least 55 or at least 60 weight percent of eicosapentaenoic acid.

7. The oil of any of claims 1 to 6, wherein said oil is extracted from fermented recombinant *Yarrowia* sp. cells engineered for the production of eicosapentaenoic acid, wherein said cells comprise:

   (a) at least one multizyme which comprises a polypeptide having at least one delta-9 elongase linked to at least one delta-8 desaturase;
   (b) at least one peroxisome biogenesis factor protein whose expression has been down-regulated; and (c) at least one recombinant construct comprising a nucleotide sequence encoding an enzyme selected from the group consisting of malonyl CoA synthetase and acyl-CoA lysophospholipid acyltransferase.

8. The oil of claim 7, wherein said cells comprise at least one recombinant construct comprising a nucleotide sequence encoding malonyl CoA synthetase.

9. The oil of claim 8, wherein the malonyl CoA synthetase consists essentially of a sequence selected from the group consisting of SEQ ID NO:40 and SEQ ID NO:42.

10. The oil of claim 7, wherein said cells comprise at least one recombinant construct comprising a nucleotide sequence encoding an acyl-CoA lysophospholipid acyltransferase.

11. The oil of claim 10, wherein the acyl-CoA lysophospholipid acyltransferase consists essentially of the sequence selected from the group consisting of SEQ ID NO:15, SEQ ID NO:17, SEQ ID NO:25, SEQ ID NO:29, SEQ ID NO:31 and SEQ ID NO:32.

12. The oil of claim 7, wherein the multizyme linker is selected from the group consisting of: SEQ ID NO:1, SEQ ID NO:2, SEQ ID NO:3, SEQ ID NO:4, SEQ ID NO:5, SEQ ID NO:6 and SEQ ID NO:7.

13. The oil of claim 7,wherein the multizyme consists essentially of the sequence selected from the group consisting of: SEQ ID NO:9, SEQ ID NO:11 and SEQ ID NO:13.

14. The oil of any of claims 1 to 7, wherein said oil is extracted from a recombinant *Yarrowia* sp. host cell using a process selected from the group consisting of: extraction with organic solvents, sonication and supercritical fluid extraction.

**Patentansprüche**

1. Extrahiertes, nicht konzentriertes Öl, umfassend:

   (a) mindestens 50 Gewichtsprozent Eicosapentaensäure, gemessen als Gewichtsprozent der Gesamtfettsäuren; und
   (b) ein Verhältnis von mindestens 3,1 von Eicosapentaensäure, gemessen als Gewichtsprozent der Gesamtfettsäuren, zu Linolsäure, gemessen als Gewichtsprozent der Gesamtfettsäuren.

2. Öl nach Anspruch 1, wobei das Öl ein mikrobielles Öl ist.

3. Öl nach einem der Ansprüche 1 oder 2, wobei das Öl von einer ölhaltigen Hefe ist, und wobei vorzugsweise die ölhaltige Hefe *Yarrowia,* z. B. *Yarrowia lipolytica,* ist.

4. Öl nach einem der Ansprüche 1 bis 3, wobei das Verhältnis von Eicosapentaensäure zu Linolsäure im Öl mindestens 3,5 ist.

5. Öl nach einem der Ansprüche 1 bis 3, wobei das Verhältnis von Eicosapentaensäure zu Linolsäure im Öl mindestens 4,5 ist.

6. Öl nach einem der Ansprüche 1 bis 5, wobei das Öl mindestens 55 oder mindestens 60 Gewichtsprozent Eicosapentaensäure umfasst.

7. Öl nach einem der Ansprüche 1 bis 6, wobei das Öl aus fermentierten rekombinanten *Yarrowia*-Sp.-Zellen extrahiert ist, die für die Produktion von Eicosapentaensäure konstruiert sind, wobei die Zellen umfassen:

   (a) zumindest ein Multizym, das ein Polypeptid umfasst, das zumindest eine Delta-9-Elongase, verknüpft mit zumindest einer Delta-8-Desaturase, aufweist;
   (b) zumindest ein Peroxysom-Biogenesefaktor-Protein, dessen Expression herunterreguliert worden ist; und
   (c) zumindest ein rekombinantes Konstrukt, umfassend eine Nukleotidsequenz, die für ein Enzym codiert, das aus der Gruppe, bestehend aus Malonyl-CoA-Synthetase und Acyl-CoA-Lysophospholipid-Acyltransferase, gewählt ist.

8. Öl nach Anspruch 7, wobei die Zellen zumindest ein rekombinantes Konstrukt umfassen, das eine für Malonyl-CoA-Synthetase codierende Nukleotidsequenz umfasst.

9. Öl nach Anspruch 8, wobei die Malonyl-CoA-Synthetase im Wesentlichen aus einer Sequenz besteht, die aus der Gruppe gewählt ist, die aus SEQ ID NO: 40 und SEQ ID NO: 42 besteht.

10. Öl nach Anspruch 7, wobei die Zellen zumindest ein rekombinantes Konstrukt umfassen, das eine Nukleotidsequenz umfasst, die für eine Acyl-CoA-Lysophospholipid-Acyltransferase codiert.

11. Öl nach Anspruch 10, wobei die Acyl-CoA-Lysophospholipid-Acyltransferase im Wesentlichen aus der Sequenz besteht, die aus der Gruppe gewählt ist, die besteht aus SEQ ID NO: 15, SEQ ID NO: 17, SEQ ID NO: 25, SEQ ID NO: 29, SEQ ID NO: 31 und SEQ ID NO: 32.

12. Öl nach Anspruch 7, wobei der Multizym-Linker aus der Gruppe gewählt ist, die besteht aus: SEQ ID NO: 1, SEQ ID NO: 2, SEQ ID NO: 3, SEQ ID NO: 4, SEQ ID NO: 5, SEQ ID NO: 6 und SEQ ID NO: 7.

13. Öl nach Anspruch 7, wobei das Multizym im Wesentlichen aus der Sequenz besteht, die aus der Gruppe gewählt ist, die besteht aus: SEQ ID NO: 9, SEQ ID NO: 11 und SEQ ID NO: 13.

14. Öl nach einem der Ansprüche 1 bis 7, wobei das Öl aus einer rekombinanten *Yarrowia*-Sp.-Wirtszelle unter Verwendung eines Vorgangs extrahiert wird, der aus der Gruppe gewählt ist, die besteht aus: Extraktion mit organischen Lösemitteln, Beschallung und Extraktion mit überkritischem Fluid.

## Revendications

1. Huile extraite, non concentrée comprenant:

(a) au moins 50 pour-cent en poids d'acide eicosapentaénoïque mesuré sous forme de pourcentage en poids de total d'acides gras; et
(b) un rapport d'au moins 3,1 de l'acide eicosapentaénoïque, mesuré sous forme de pourcentage en poids de total d'acides gras, sur l'acide linoléique, mesuré sous forme de pourcentage en poids de total d'acides gras.

2. Huile selon la revendication 1, où ladite huile est une huile microbienne.

3. Huile selon l'une quelconque des revendications 1 ou 2, où ladite huile est issue d'une levure oléagineuse et de préférence où ladite levure oléagineuse est *Yarrowia,* par ex. *Yarrowia lipolytica.*

4. Huile selon l'une quelconque des revendications 1 à 3, dans laquelle le rapport de l'acide eicosapentaénoïque sur l'acide linoléique dans l'huile est d'au moins 3,5.

5. Huile selon l'une quelconque des revendications 1 à 3, dans laquelle le rapport de l'acide eicosapentaénoïque sur l'acide linoléique dans l'huile est d'au moins 4,5.

6. Huile selon l'une quelconque des revendications 1 à 5, où l'huile comprend au moins 55 ou au moins 60 pour-cent en poids d'acide eicosapentaénoïque.

7. Huile selon l'une quelconque des revendications 1 à 6, où ladite huile est extraite à partir de cellules de *Yarrowia* sp. recombinantes fermentées mises au point pour la production d'acide eicosapentaénoïque, dans laquelle lesdites cellules comprennent:

(a) au moins une multizyme qui comprend un polypeptide avec au moins une delta-9 élongase liée à au moins une delta-8 désaturase;
(b) au moins une protéine de facteur de biogénèse de peroxysomes dont l'expression a été sous-régulée; et
(c) au moins une construction recombinante comprenant une séquence de nucléotides codant pour une enzyme choisie dans le groupe constitué de malonyl-CoA synthétase et d'acyl-CoA lysophospholipide acyltransférase.

8. Huile selon la revendication 7, dans laquelle lesdites cellules comprennent au moins une construction recombinante comprenant une séquence de nucléotides codant pour une malonyl-CoA synthétase.

9. Huile selon la revendication 8, dans laquelle la malonyl-CoA synthétase est constituée essentiellement d'une séquence choisie dans le groupe constitué de la SEQ ID NO:40 et la SEQ ID NO:42.

10. Huile selon la revendication 7, dans laquelle lesdites cellules comprennent au moins une construction recombinante comprenant une séquence de nucléotides codant pour une acyl-CoA lysophospholipide acyltransférase.

11. Huile selon la revendication 10, dans laquelle l'acyl-CoA lysophospholipide acyltransférase est constituée essentiellement d'une séquence choisie dans le groupe constitué de la SEQ ID NO:15, la SEQ ID NO:17, la SEQ ID NO:25, la SEQ ID NO:29, la SEQ ID NO:31 et la SEQ ID NO:32.

12. Huile selon la revendication 7, dans laquelle le lieur multizyme est choisi dans le groupe constitué de: la SEQ ID NO:1, la SEQ ID NO:2, la SEQ ID NO:3, la SEQ ID NO:4, la SEQ ID NO:5, la SEQ ID NO:6 et la SEQ ID NO:7.

13. Huile selon la revendication 7, dans laquelle la multizyme est constituée essentiellement de la séquence choisie dans le groupe constitué de: la SEQ ID NO:9, la SEQ ID NO:11 et la SEQ ID NO:13.

14. Huile selon l'une quelconque des revendications 1 à 7, où ladite huile est extraite à partir d'une cellule hôte de *Yarrowia* sp. recombinante en utilisant un procédé choisi dans le groupe constitué de: une extraction avec des solvants organiques, une sonication et une extraction avec un fluide supercritique.

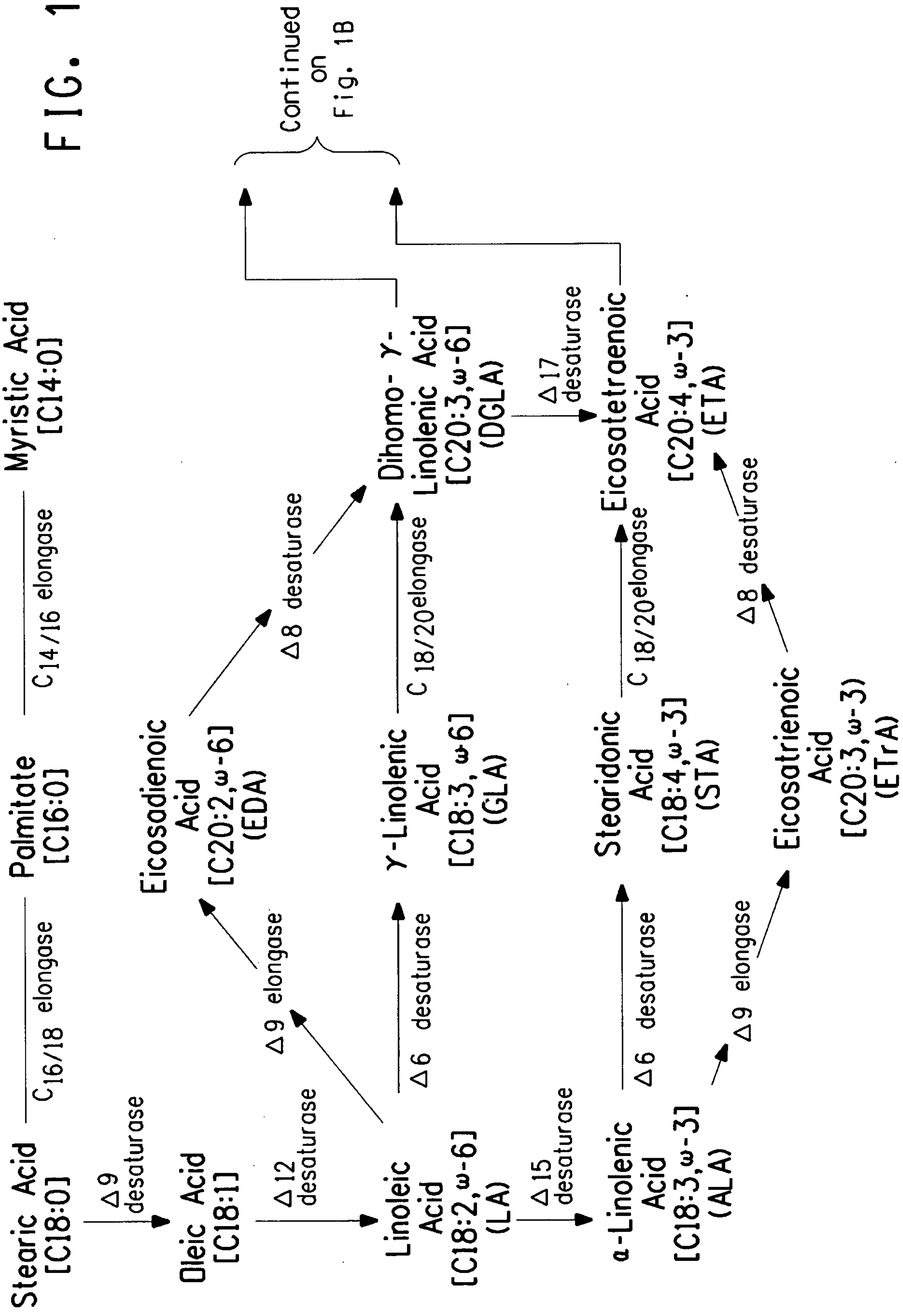
Stearic Acid [C18:0]
C16/18 elongase
Palmitate [C16:0]
C14/16 elongase
Myristic Acid [C14:0]
Δ9 desaturase
Oleic Acid [C18:1]
Δ12 desaturase
Linoleic Acid [C18:2,ω-6] (LA)
Δ9 elongase
Eicosadienoic Acid [C20:2,ω-6] (EDA)
Δ8 desaturase
Δ6 desaturase
γ-Linolenic Acid [C18:3, ω-6] (GLA)
C 18/20 elongase
Dihomo- γ-Linolenic Acid [C20:3,ω-6] (DGLA)
Δ15 desaturase
α-Linolenic Acid [C18:3,ω-3] (ALA)
Δ6 desaturase
Stearidonic Acid [C18:4,ω-3] (STA)
C 18/20 elongase
Δ17 desaturase
Eicosatetraenoic Acid [C20:4,ω-3] (ETA)
Δ9 elongase
Eicosatrienoic Acid [C20:3,ω-3] (ETrA)
Δ8 desaturase
Continued on Fig. 1B

FIG. 1A

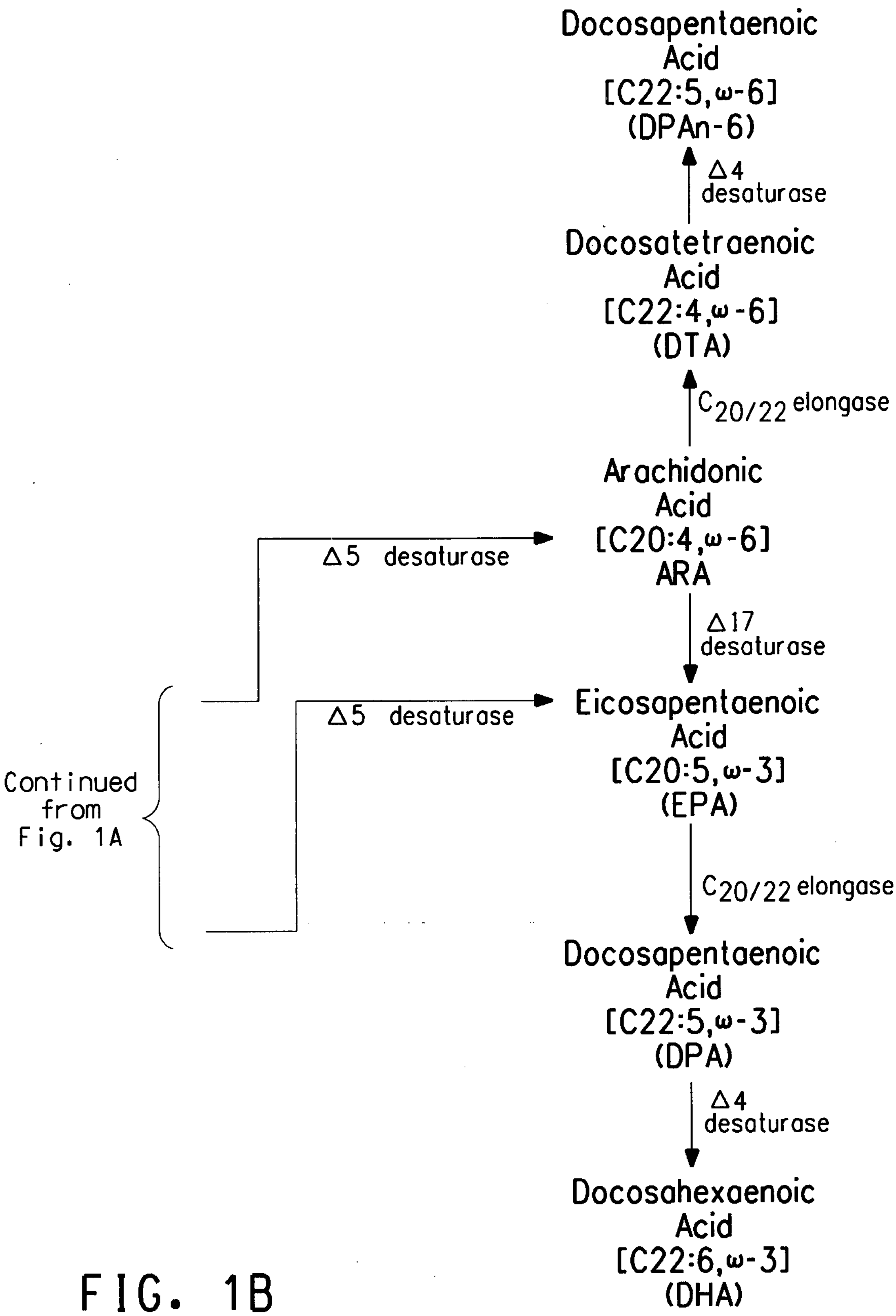
Docosapentaenoic Acid [C22:5,ω-6] (DPAn-6)
Δ4 desaturase
Docosatetraenoic Acid [C22:4,ω-6] (DTA)
C20/22 elongase
Arachidonic Acid [C20:4,ω-6] ARA
Δ5 desaturase
Δ17 desaturase
Δ5 desaturase
Eicosapentaenoic Acid [C20:5,ω-3] (EPA)
Continued from Fig. 1A
C20/22 elongase
Docosapentaenoic Acid [C22:5,ω-3] (DPA)
Δ4 desaturase
Docosahexaenoic Acid [C22:6,ω-3] (DHA)

FIG. 1B

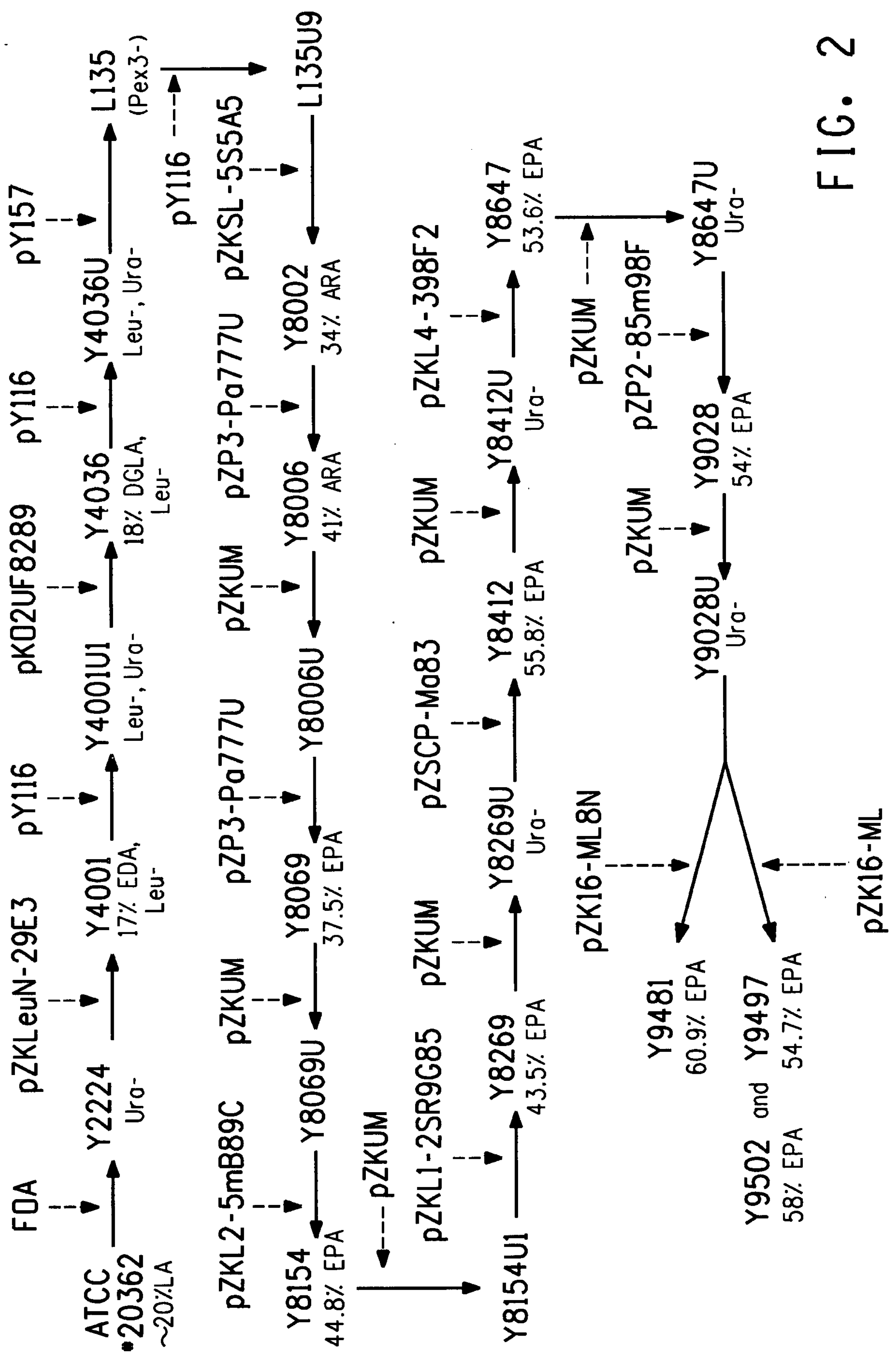
ATCC #20362 ~20%LA
FOA
Y2224 Ura-
pZKLeuN-29E3
Y4001 17% EDA, Leu-
pY116
Y4001U1 Leu-, Ura-
pKO2UF8289
Y4036 18% DGLA, Leu-
pY116
Y4036U Leu-, Ura-
pY157
L135 (Pex3-)
pY116
L135U9
pZKSL-5S5A5
Y8002 34% ARA
pZP3-Pa777U
Y8006 41% ARA
pZKUM
Y8006U
pZP3-Pa777U
Y8069 37.5% EPA
pZKUM
Y8069U
pZKL2-5mB89C
Y8154 44.8% EPA
pZKUM
Y8154U1
pZKL1-2SR9G85
Y8269 43.5% EPA
pZKUM
Y8269U Ura-
pZSCP-Ma83
Y8412 55.8% EPA
pZKUM
Y8412U Ura-
pZKL4-398F2
Y8647 53.6% EPA
pZKUM
Y8647U Ura-
pZP2-85m98F
Y9028 54% EPA
pZKUM
Y9028U Ura-
pZK16-ML8N
pZK16-ML
Y9481 60.9% EPA
Y9502 58% EPA and Y9497 54.7% EPA

FIG. 2

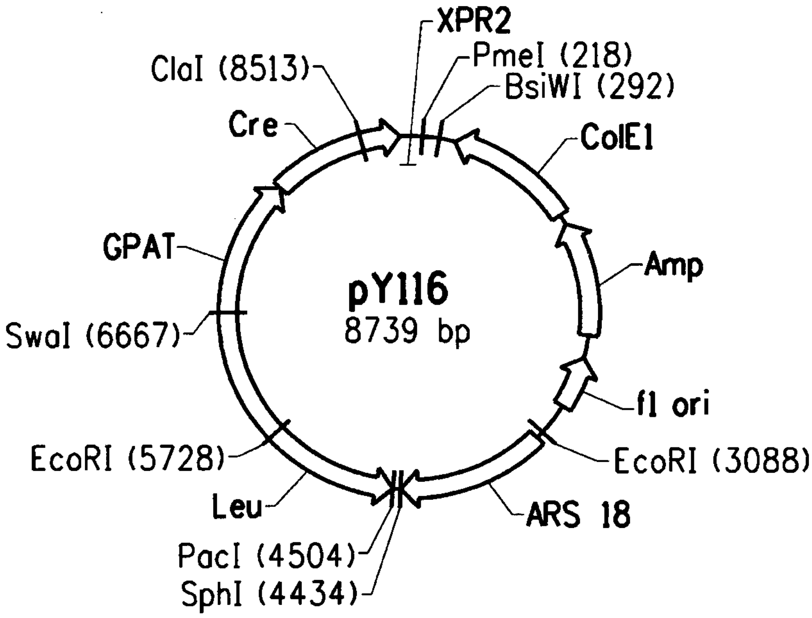
XPR2
PmeI (218)
BsiWI (292)
ClaI (8513)
Cre
ColE1
GPAT
Amp
pY116
8739 bp
SwaI (6667)
f1 ori
EcoRI (5728)
EcoRI (3088)
Leu
ARS 18
PacI (4504)
SphI (4434)

FIG. 3

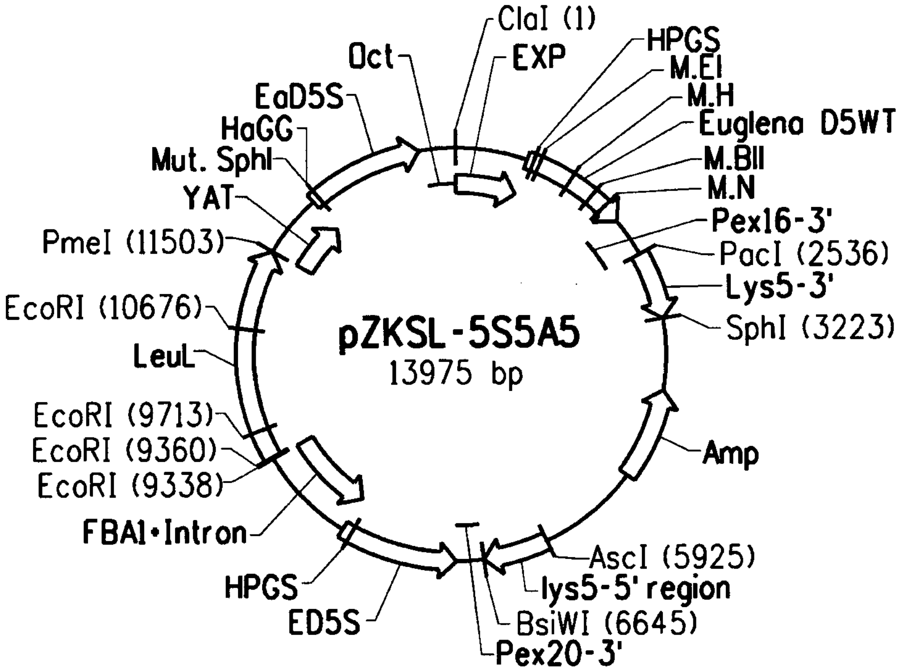

ClaI (1)
EXP
Oct
EaD5S
HaGG
Mut. SphI
YAT
PmeI (11503)
EcoRI (10676)
LeuL
EcoRI (9713)
EcoRI (9360)
EcoRI (9338)
FBA1•Intron
HPGS
ED5S
HPGS
M.EI
M.H
Euglena D5WT
M.BII
M.N
Pex16-3'
PacI (2536)
Lys5-3'
SphI (3223)
pZKSL-5S5A5
13975 bp
Amp
AscI (5925)
lys5-5' region
BsiWI (6645)
Pex20-3'


FIG. 4A

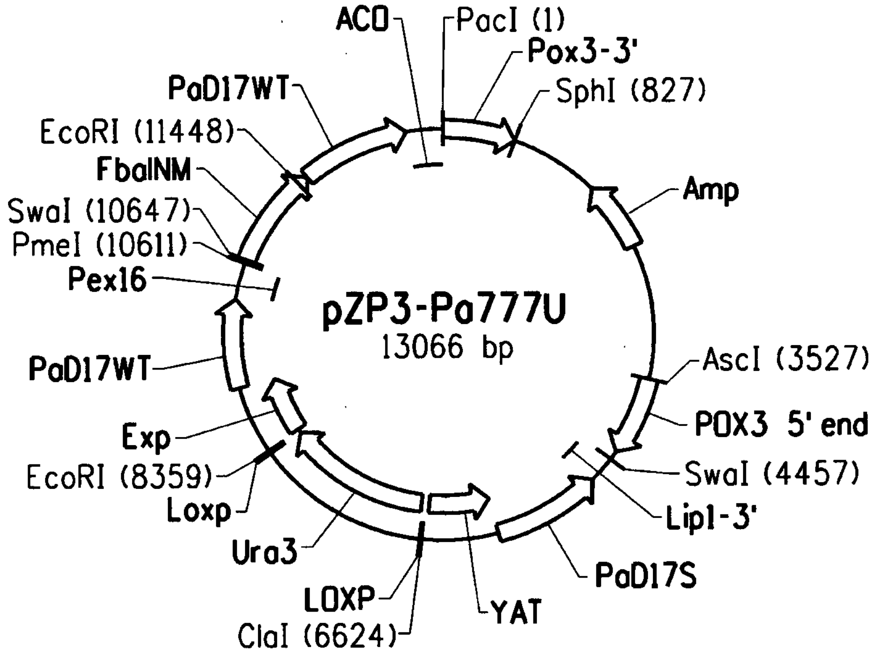

ACO
PacI (1)
Pox3-3'
SphI (827)
PaD17WT
EcoRI (11448)
FbaINM
SwaI (10647)
PmeI (10611)
Pex16
PaD17WT
Exp
EcoRI (8359)
Loxp
Ura3
LOXP
ClaI (6624)
pZP3-Pa777U
13066 bp
Amp
AscI (3527)
POX3 5' end
SwaI (4457)
Lip1-3'
PaD17S
YAT


FIG. 4B

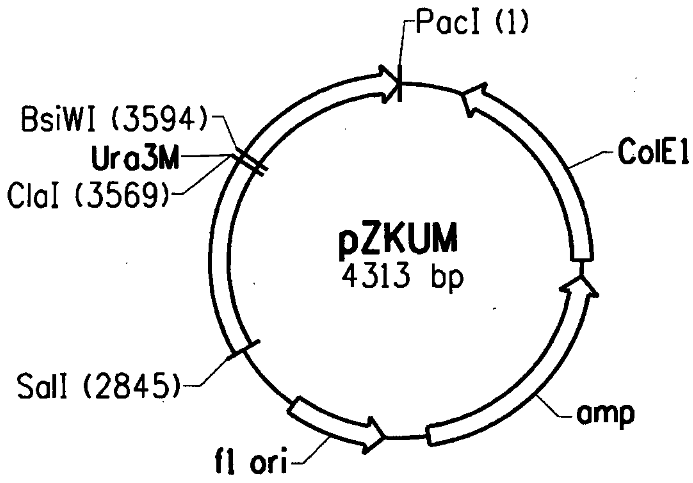
PacI (1)
ColE1
BsiWI (3594)
Ura3M
ClaI (3569)
pZKUM
4313 bp
SalI (2845)
amp
f1 ori

FIG. 5A

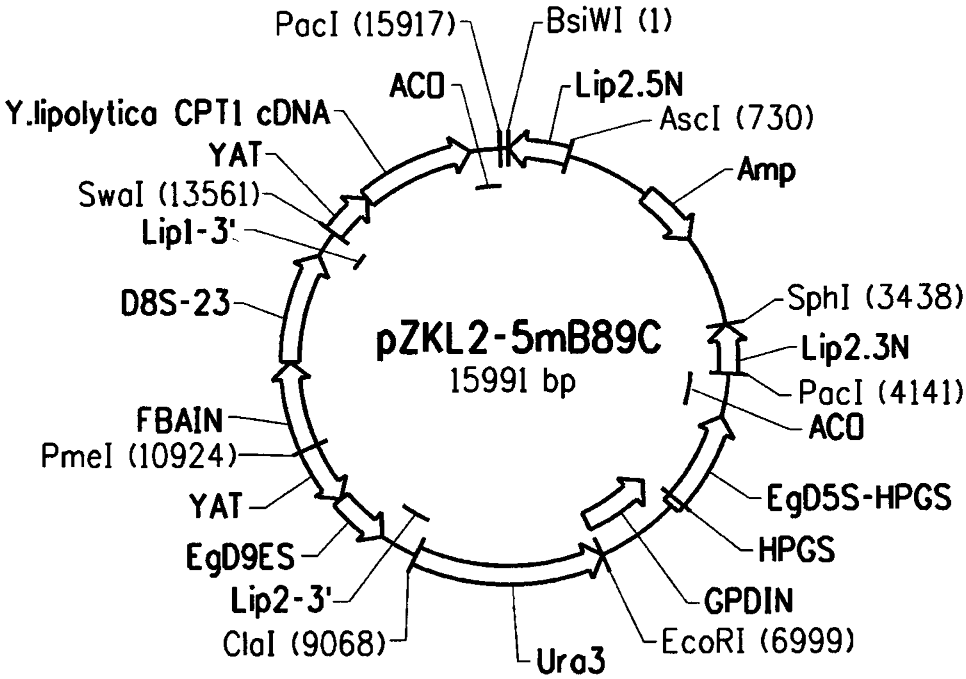
PacI (15917)
BsiWI (1)
ACO
Lip2.5N
AscI (730)
Y.lipolytica CPT1 cDNA
YAT
Amp
SwaI (13561)
Lip1-3'
D8S-23
SphI (3438)
pZKL2-5mB89C
15991 bp
Lip2.3N
PacI (4141)
FBAIN
ACO
PmeI (10924)
YAT
EgD5S-HPGS
EgD9ES
HPGS
Lip2-3'
GPDIN
ClaI (9068)
EcoRI (6999)
Ura3

FIG. 5B

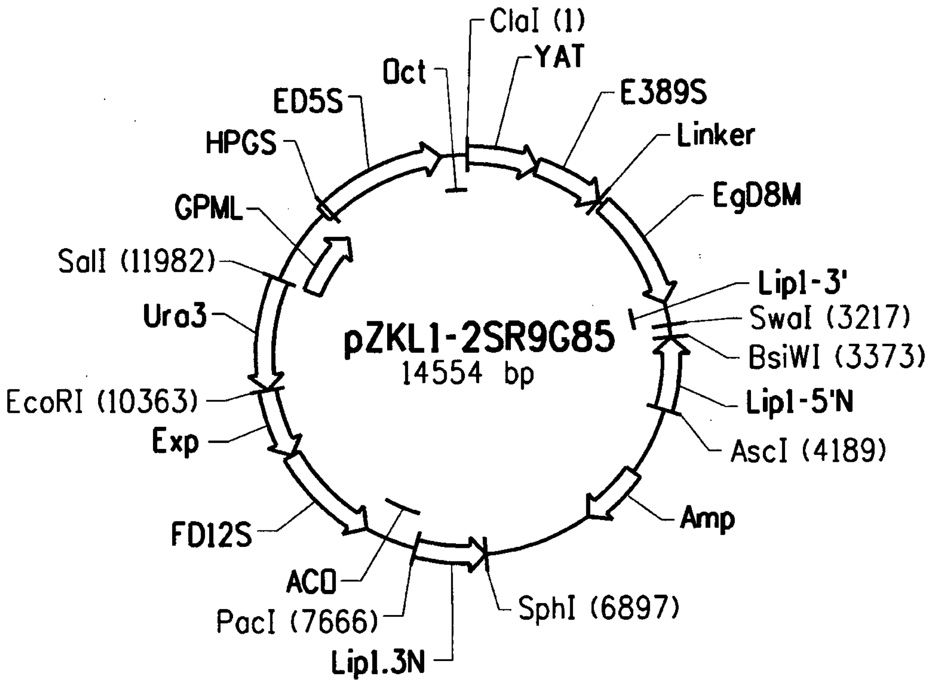
ClaI (1)
YAT
E389S
Linker
EgD8M
Lip1-3'
SwaI (3217)
BsiWI (3373)
Lip1-5'N
AscI (4189)
Amp
SphI (6897)
Lip1.3N
PacI (7666)
ACO
FD12S
Exp
EcoRI (10363)
Ura3
SalI (11982)
GPML
HPGS
ED5S
Oct
pZKL1-2SR9G85
14554 bp

FIG. 6A

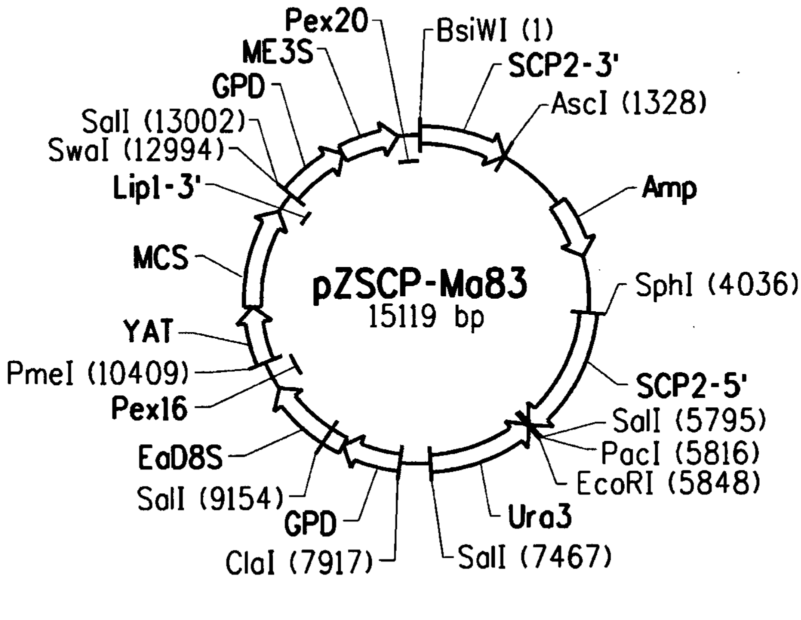
BsiWI (1)
SCP2-3'
AscI (1328)
Amp
SphI (4036)
SCP2-5'
SalI (5795)
PacI (5816)
EcoRI (5848)
Ura3
SalI (7467)
ClaI (7917)
GPD
SalI (9154)
EaD8S
Pex16
PmeI (10409)
YAT
MCS
Lip1-3'
SwaI (12994)
SalI (13002)
GPD
ME3S
Pex20
pZSCP-Ma83
15119 bp

FIG. 6B

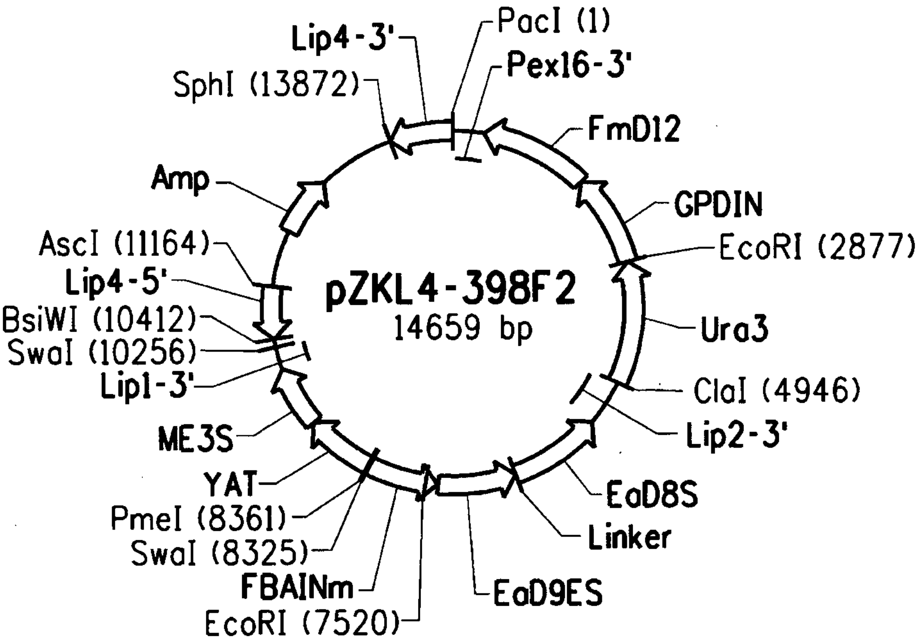
PacI (1)
Pex16-3'
FmD12
GPDIN
EcoRI (2877)
Ura3
ClaI (4946)
Lip2-3'
EaD8S
Linker
EaD9ES
EcoRI (7520)
FBAINm
SwaI (8325)
PmeI (8361)
YAT
ME3S
Lip1-3'
SwaI (10256)
BsiWI (10412)
Lip4-5'
AscI (11164)
Amp
SphI (13872)
Lip4-3'
pZKL4-398F2
14659 bp

FIG. 7A

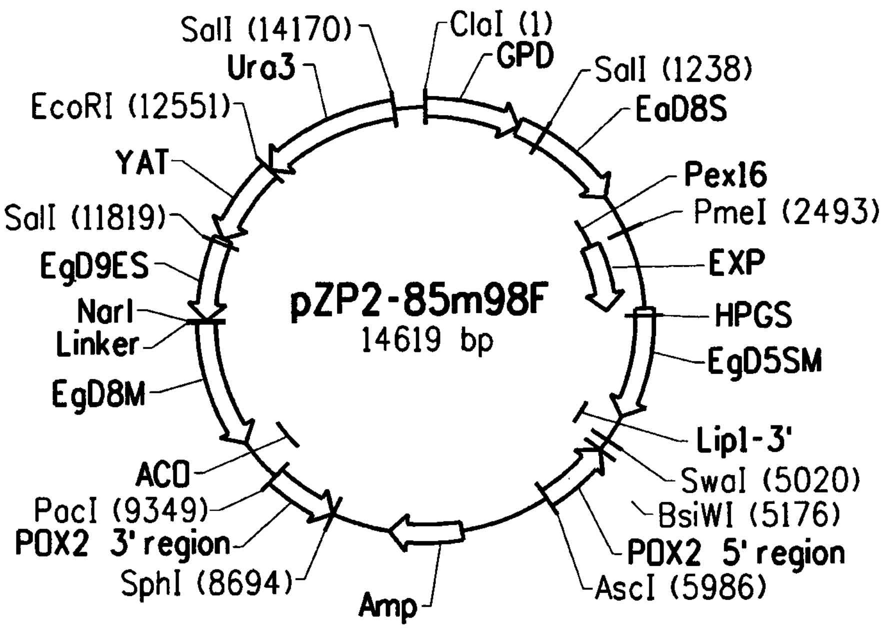
ClaI (1)
GPD
SalI (1238)
EaD8S
Pex16
PmeI (2493)
EXP
HPGS
EgD5SM
Lip1-3'
SwaI (5020)
BsiWI (5176)
POX2 5' region
AscI (5986)
Amp
SphI (8694)
POX2 3' region
PacI (9349)
ACO
EgD8M
Linker
NarI
EgD9ES
SalI (11819)
YAT
EcoRI (12551)
Ura3
SalI (14170)
pZP2-85m98F
14619 bp

FIG. 7B

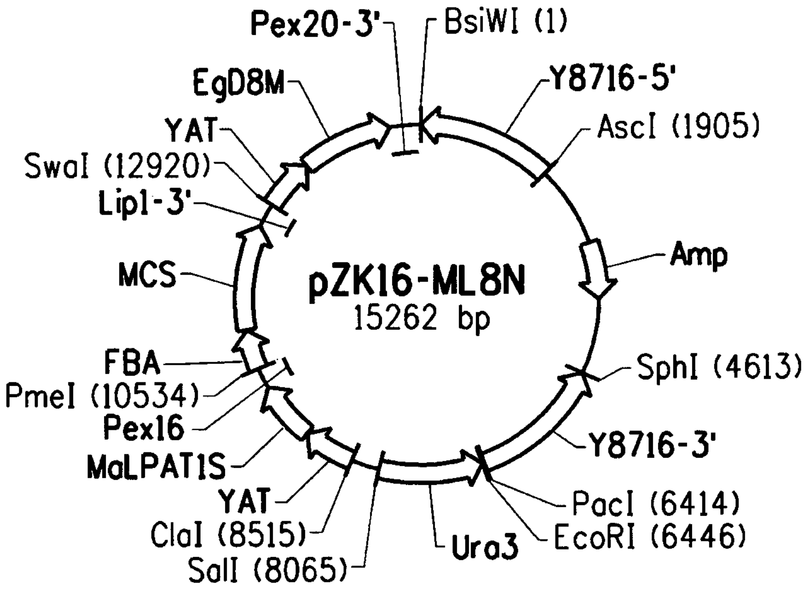
Pex20-3'
BsiWI (1)
EgD8M
Y8716-5'
YAT
AscI (1905)
SwaI (12920)
Lip1-3'
Amp
MCS
pZK16-ML8N
15262 bp
FBA
SphI (4613)
PmeI (10534)
Pex16
Y8716-3'
MaLPAT1S
YAT
PacI (6414)
ClaI (8515)
EcoRI (6446)
Ura3
SalI (8065)

FIG. 8A

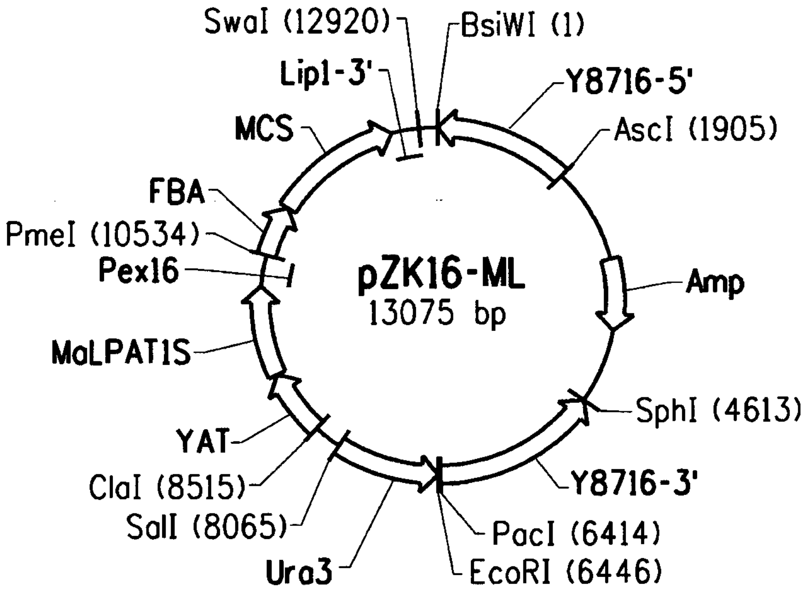
SwaI (12920)
BsiWI (1)
Lip1-3'
Y8716-5'
MCS
AscI (1905)
FBA
PmeI (10534)
Pex16
pZK16-ML
13075 bp
Amp
MaLPAT1S
SphI (4613)
YAT
Y8716-3'
ClaI (8515)
SalI (8065)
PacI (6414)
Ura3
EcoRI (6446)

FIG. 8B

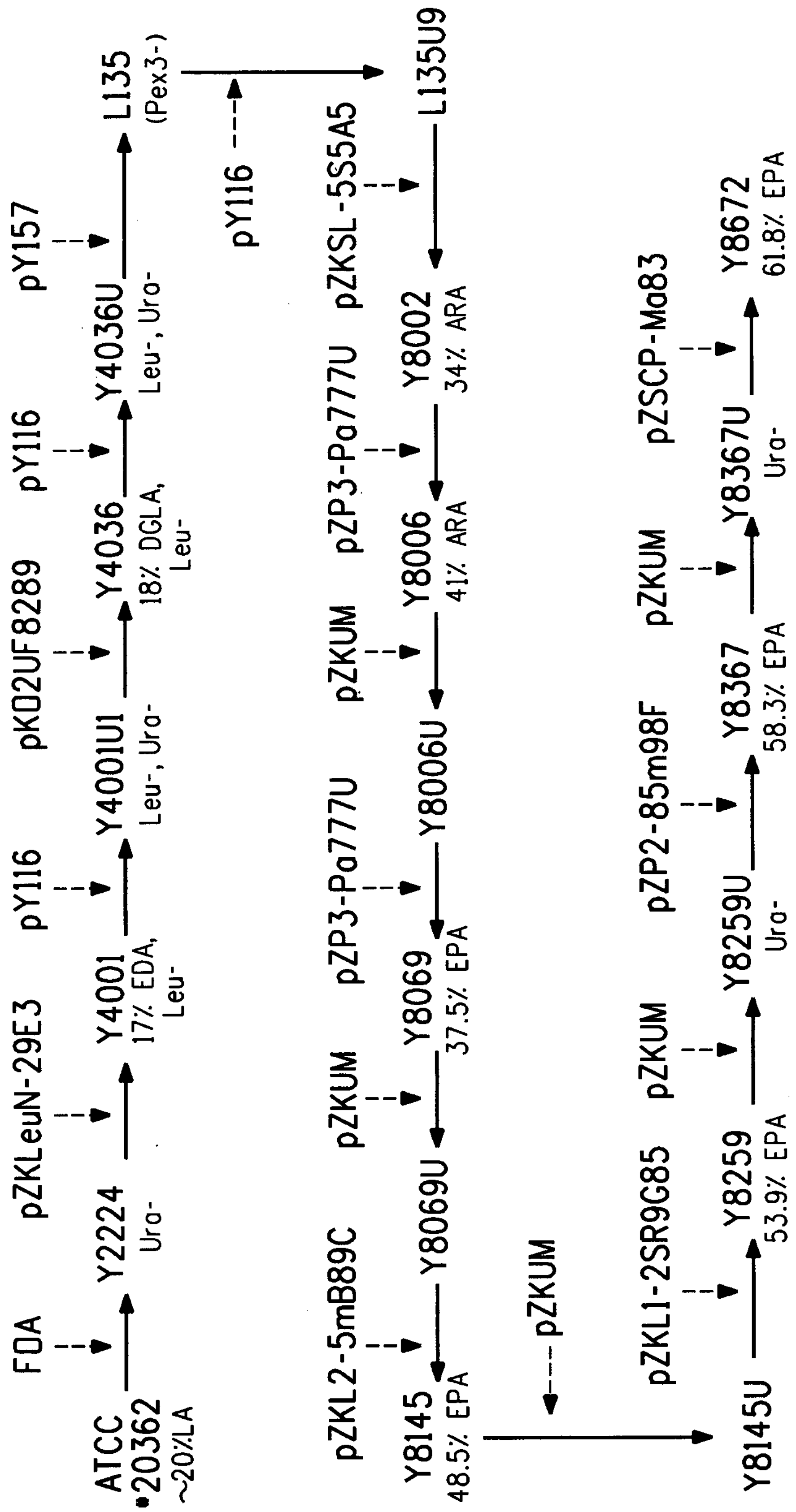
ATCC
#20362
~20%LA
FOA
Y2224
Ura-
pZKLeuN-29E3
Y4001
17% EDA,
Leu-
pY116
Y4001U1
Leu-, Ura-
pKO2UF8289
Y4036
18% DGLA,
Leu-
pY116
Y4036U
Leu-, Ura-
pY157
L135
(Pex3-)
pY116
L135U9
pZKSL-5S5A5
Y8002
34% ARA
pZP3-Pa777U
Y8006
41% ARA
pZKUM
Y8006U
pZP3-Pa777U
Y8069
37.5% EPA
pZKUM
Y8069U
pZKL2-5mB89C
Y8145
48.5% EPA
pZKUM
Y8145U
pZKL1-2SR9G85
Y8259
53.9% EPA
pZKUM
Y8259U
Ura-
pZP2-85m98F
Y8367
58.3% EPA
pZKUM
Y8367U
Ura-
pZSCP-Ma83
Y8672
61.8% EPA

FIG. 9

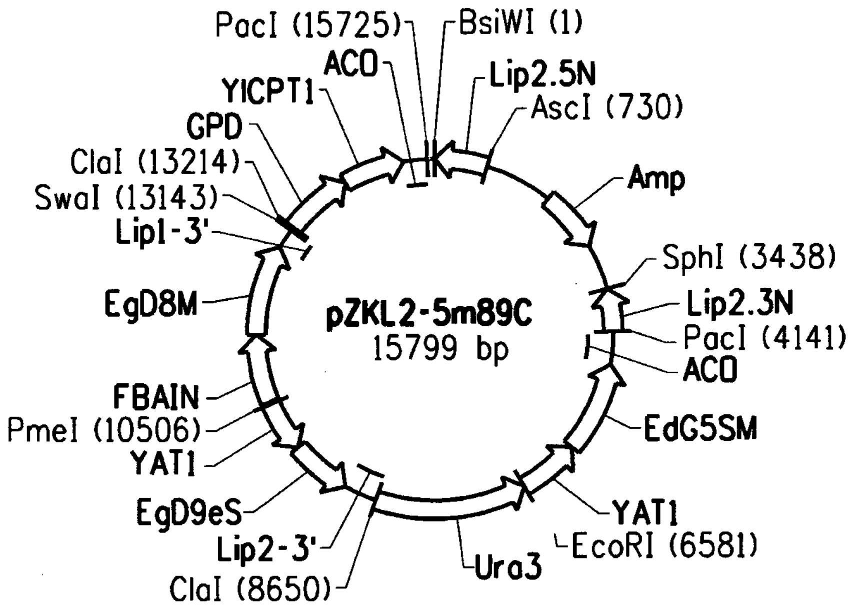


FIG. 10A

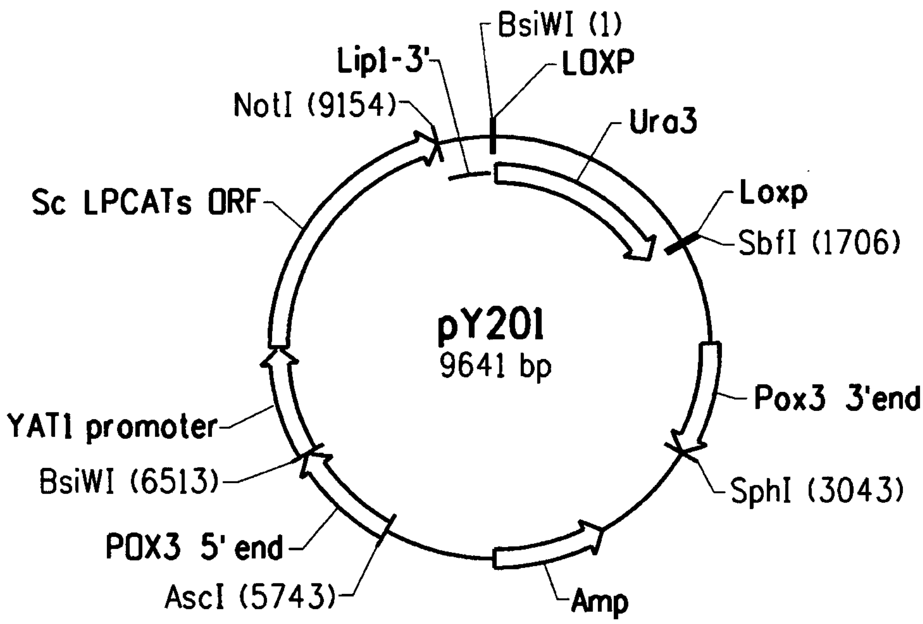


FIG. 10B

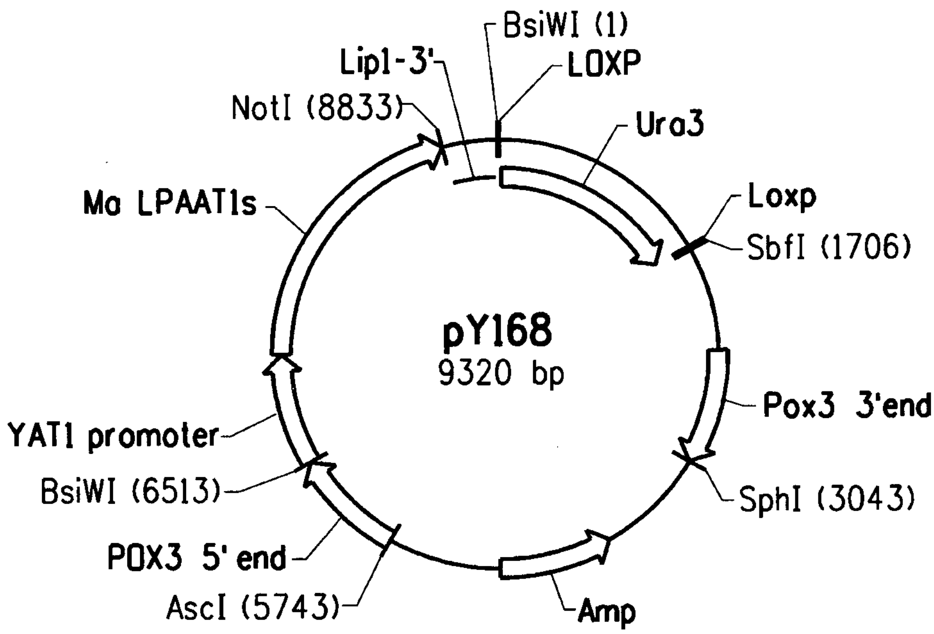


FIG. 11A

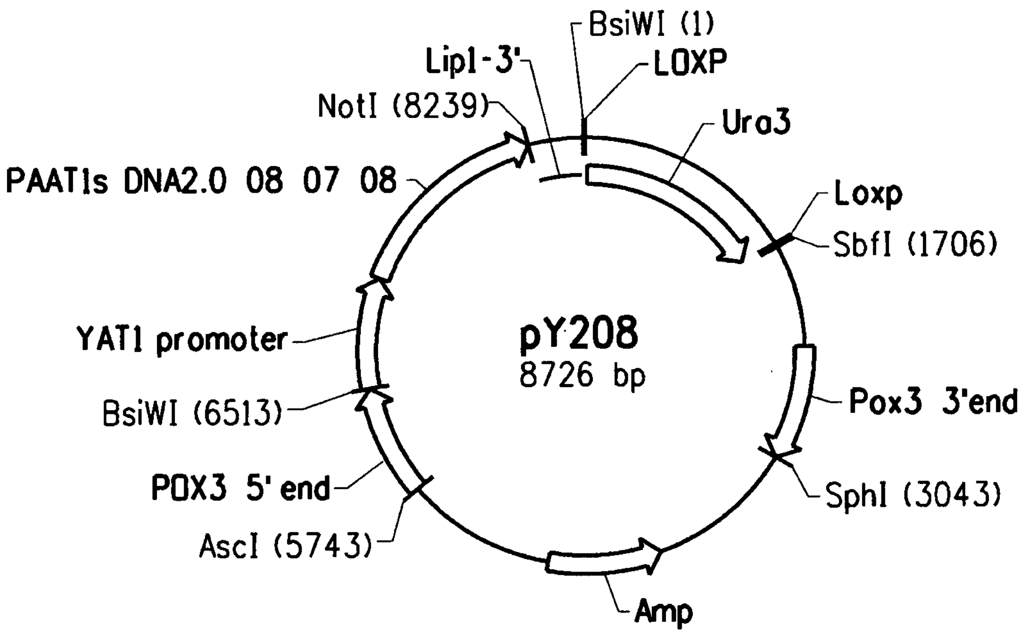


FIG. 11B

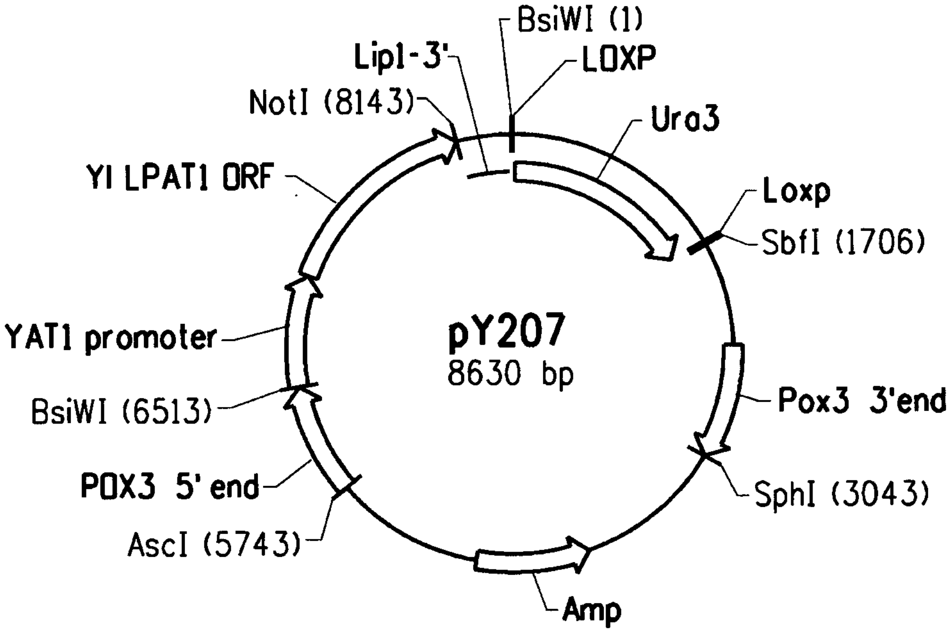


FIG. 12A

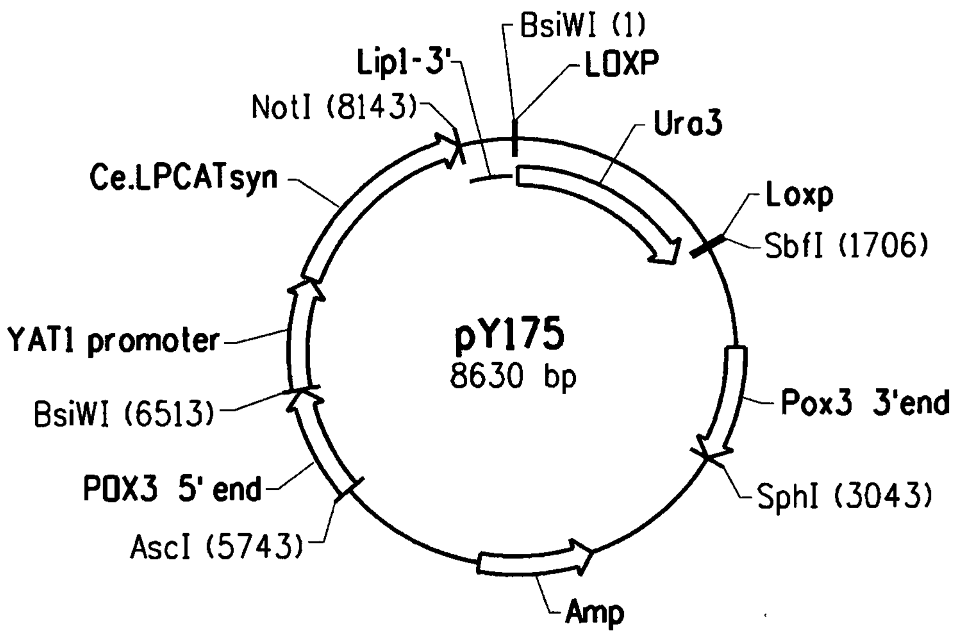


FIG. 12B

| Strain | LA % TFAs | EPA % TFAs | Ratio EPA/ LA |
|---|---|---|---|
| Y8404 | 20.40 | 51.10 | 2.50 |
| Y8405 | 20.50 | 51.40 | 2.51 |
| Y8406 | 20.30 | 51.20 | 2.52 |
| Y8407 | 20.50 | 51.50 | 2.51 |
| Y8408 | 20.20 | 51.20 | 2.53 |
| Y8409 | 20.60 | 51.00 | 2.48 |
| Y8410 | 20.50 | 50.90 | 2.48 |
| Y8411 | 19.90 | 52.90 | 2.66 |
| Y8412 | 19.00 | 55.80 | 2.94 |
| Y8413 | 19.90 | 52.40 | 2.63 |
| Y8414 | 19.90 | 49.40 | 2.48 |
| Y8415 | 16.00 | 59.00 | 3.69 |
| Y8647 | 20.30 | 53.60 | 2.64 |
| Y8648 | 18.60 | 56.70 | 3.05 |
| Y8649 | 18.80 | 55.80 | 2.97 |
| Y8650 | 18.80 | 56.10 | 2.98 |
| Y9028 | 19.80 | 54.50 | 2.75 |
| Y9029 | 19.80 | 53.80 | 2.72 |
| Y9031 | 20.10 | 52.30 | 2.60 |
| Y9477 | 10.00 | 61.40 | 6.14 |

| Strain | LA % TFAs | EPA % TFAs | Ratio EPA/LA |
|---|---|---|---|
| Y9481 | 11.00 | 60.90 | 5.54 |
| Y9486 | 11.90 | 60.30 | 5.07 |
| Y9497 | 11.30 | 58.70 | 5.19 |
| Y9502 | 12.70 | 57.00 | 4.49 |
| Y9504 | 11.30 | 59.90 | 5.30 |
| Y9508 | 13.10 | 58.70 | 4.48 |
| Y9510 | 11.70 | 58.90 | 5.03 |
| Y8143 | 18.10 | 50.30 | 2.78 |
| Y8144 | 18.00 | 50.60 | 2.81 |
| Y8145 | 18.60 | 48.50 | 2.61 |
| Y8146 | 18.70 | 48.30 | 2.58 |
| Y8256 | 18.80 | 49.90 | 2.65 |
| Y8259 | 16.90 | 53.90 | 3.19 |
| Y8367 | 14.20 | 58.30 | 4.11 |
| Y8368 | 17.90 | 52.50 | 2.93 |
| Y8369 | 15.60 | 55.80 | 3.58 |
| Y8370 | 15.70 | 56.40 | 3.59 |
| Y8666 | 15.10 | 62.20 | 4.12 |
| Y8669 | 15.70 | 61.50 | 3.92 |
| Y8670 | 17.00 | 60.90 | 3.58 |
| Y8672 | 16.10 | 61.80 | 3.84 |

FIG. 13

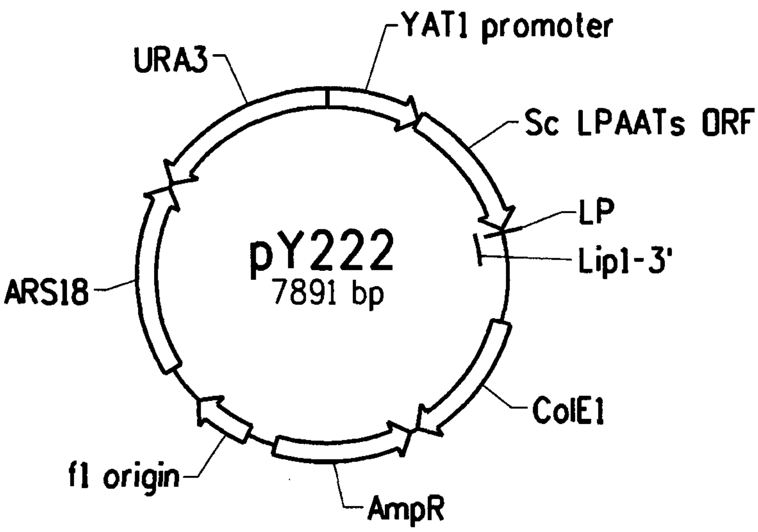


FIG. 14A

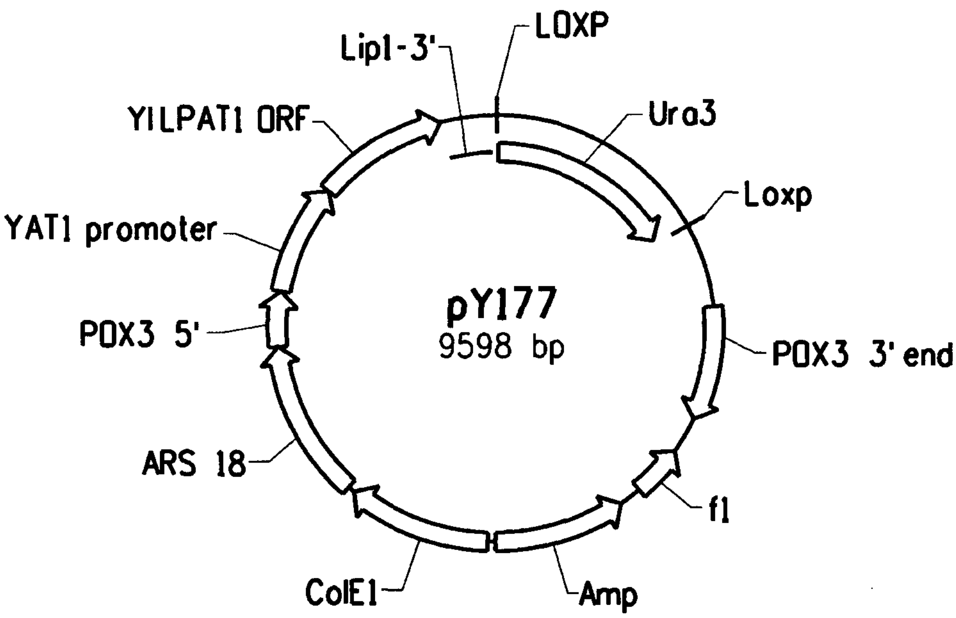


FIG. 14B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090093543 A1
- US 7238482 B
- US 20060115881 A1
- US 20080145867 A1
- US 20100075386 A1
- WO 2005047480 A
- US 20080254191 A1
- US 7645604 B
- US 7709239 B
- US 20080254522 A1
- US 20080254521 A1
- WO 2004076617 A
- WO 2006052870 A
- US 7125672 B
- WO 2008128241 A
- US 7256033 B
- WO 2008124194 A
- US 7678560 B
- US 7695950 B
- US 20080274521 A1
- US 7465793 B
- US 7556949 B
- US 7504259 B
- US 7470532 B
- US 20100159558 A1
- WO 2004076617 A2, Renz, A.
- WO 2004087902 A2
- WO 2004087902 A
- WO 2006069936 A
- WO 2009001315 A
- WO 2009014140 A
- WO 2010147900 A
- WO 2009046248 A
- US 4880741 A
- US 5071764 A
- US 7259255 B
- US 4910141 A
- US 64192909 A
- US 6797303 B
- US 5648564 A
- US 6280747 B
- US 4670285 A
- US 61292915 A
- US 61295347 B
- US 20080254191 A
- WO 2008073367 A
- US 7202356 B
- US 20060094102 A1
- US 7459546 B
- US 20090325265 A1
- US 20080145867 A
- WO 2008076377 A
- US 7189559 B
- US 20060168687 A1
- WO 61187366 A
- WO 61187368 A
- WO 61187359 A
- WO 2008124048 A
- WO 2007061742 A
- US 20070117190 A1
- WO 2006012325 A
- WO 2006012326 A
- WO 2008073271 A
- US 20080138868 A1
- WO 2007136671 A
- US 20070292924 A1
- WO 2007136646 A
- US 20070271632 A1
- WO 2008137532 A
- WO 2007123999 A
- US 20070249026 A1
- WO 2008054565 A
- US 20080125326 A1
- WO 2005047485 A
- WO 2007046817 A
- US 20070087420 A1

**Non-patent literature cited in the description**


- **IBANEZ GONZALEZ et al.** *Journal of the American Oil Chemists' Society,* 2001, vol. 78 (3), 277-285
- **ROBLES MEDINA et al.** *Journal of Biotechnology,* 1999, vol. 70, 379-391
- **TAMAKI, H. et al.** *J. Biol. Chem.,* 2007, vol. 282, 34288-34298
- **STÅHL, U. et al.** *FEBS Letters,* 2008, vol. 582, 305-309
- **CHEN, Q et al.** *FEBS Letters,* 2007, vol. 581, 5511-5516
- **BENGHEZAL, M. et al.** *J. Biol. Chem.,* 2007, vol. 282, 30845-30855
- **RIEKHOF et al.** *J. Biol. Chem.,* 2007, vol. 282, 28344-28352
- **HISHIKAWA et al.** *Proc. Natl. Acad. Sci. U.S.A.,* 2008, vol. 105, 2830-2835
- **KIM, Y.S. ; S.K. BANG.** *J. Biol. Chem.,* 1985, vol. 260, 5098-5104
- **KIM, Y.S. ; H.Z. CHAE.** *Biochem. J.,* 1991, vol. 273, 511-516
- **KIM, Y.S. ; S.W. KANG.** *Biochem. J,* 1994, vol. 297, 327-333
- **DISTEL et al.** *J. Cell Biol.,* 1996, vol. 135, 1-3
- **KIEL, J. A. K. W. et al.** *Traffic,* 2006, vol. 7, 1291-1303
- **WEETE.** Fungal Lipid Biochemistry. Plenum, 1980
- **YONGMANITCHAI ; WARD.** *Appl. Environ. Microbiol.,* 1991, vol. 57, 419-25
- **ALTSCHUL, S. F. et al.** *J. Mol. Biol.,* 1993, vol. 215, 403-410
- **ALTSCHUL et al.** *J. Mol. Biol.,* 1990, vol. 215, 403-410
- Comput. Methods Genome Res. **W. R. PEARSON.** Proc. Int. Symp. Plenum, 1992, 111-20
- **SAMBROOK, J. ; FRITSCH, E. F. ; MANIATIS, T.** Molecular Cloning: A Laboratory Manual. Cold Spring Harbor Laboratory, 1989
- **SILHAVY, T. J. ; BENNAN, M. L. ; ENQUIST, L. W.** Experiments with Gene Fusions. Cold Spring Harbor Laboratory: Cold Spring Harbor, 1984
- **AUSUBEL, F. M. et al.** Current Protocols in Molecular Biology. Greene Publishing Assoc. and Wiley-Interscience, 1987
- **KENNEDY ; WEISS.** *J. Biol. Chem.,* 1956, vol. 222, 193-214
- **LANDS, W.E.** *J. Biol. Chem.,* 1958, vol. 231, 883-888
- **STYMNE S. ; A.K. STOBART.** *Biochem. J.,* 1984, vol. 223 (2), 305-314
- *J. Biol. Chem.,* 2009, vol. 284 (1), 1-5
- *J. Lipid Res.,* 2009, vol. 50, S46-S51
- **SHINDOU et al.** *Biochem. Biophys. Res. Comm.,* 2009, vol. 383, 320-325
- **LEWIN, T.W. et al.** *Biochemistry,* 1999, vol. 38, 5764-5771
- **YAMASHITA et al.** *Biochim. Biophys. Acta,* 2007, vol. 1771, 1202-1215
- **DOMERGUE, F. et al.** *J. Bio. Chem.,* 2003, vol. 278, 35115
- **ABBADI, A. et al.** *The Plant Cell,* 2004, vol. 16, 2734-2748
- *Methods in Enzymology,* 1991, vol. 194, 186-187
- **CHEN, D. C. et al.** *Appl. Microbiol. Biotechnol.,* 1997, vol. 48 (2), 232-235
- **JURETZEK et al.** *Yeast,* 2001, vol. 18, 97-113
- **SOUTHERN.** *J. Mol. Biol.,* 1975, vol. 98, 503
- **KROCZEK.** *J. Chromatogr. Biomed. Appl.,* 1993, vol. 618 (1-2), 133-145
- **NAKAHARA, T. et al.** Ind. Appl. Single Cell Oils. 1992, 61-97
- **JACOBS.** *Critical Reviews in Biotechnology,* 1992, vol. 12 (5/6), 463-491
- **A. SINGH ; O. WARD.** *Adv. Appl. Microbiol.,* 1997, vol. 45, 271-312
- **GILLIES, P.** The New Science of Omega-3 Fatty Acids: Differential Nutritional Pharmacology. *Texas Human Nutrition Conference,* February 2010
- **T. J. SILHAVY ; M. L. BENNAN ; L. W. ENQUIST.** Experiments with Gene Fusions. Cold Spring Harbor Laboratory, 1984
- Manual of Methods for General Bacteriology. American Society for Microbiology, 1994
- **THOMAS D. BROCK.** Biotechnology: A Textbook of Industrial Microbiology. Sinauer Associates, 1989
- **BLIGH, E. G. ; DYER, W. J.** *Can. J. Biochem. Physiol.,* 1959, vol. 37, 911-917
- **ROUGHAN, G. ; NISHIDA I.** *Arch Biochem Biophys.,* 1990, vol. 276 (1), 38-46
- **DUJON, B. et al.** *Nature,* 2004, vol. 430 (6995), 35-44
- **THOMPSON et al.** *Nucleic Acids Res,* 1994, vol. 22, 4673-4680
- **ALTSCHUL et al.** *Nucleic Acids Res.,* 1997, vol. 25, 3389-3402
- **ALTSCHUL et al.** *FEBS J.,* 2005, vol. 272, 5101-5109
- **ATSUSHI YAMASHITA ; HIROKI NAKANISHIA ; HIROSHI SUZUKIA ; RYO KAMATAA ; KEN TANAKAA ; KEIZO WAKUA ; TAKAYUKI SUGIURA.** Topology of acyltransferase motifs and substrate specificity and accessibility in 1-acyl-sn-glycero-3-phosphate acyltransferase 1. *Biochimica et Biophysica Acta,* 17 July 2007, vol. 1771 (9), 1202-1215